# EUROPEAN PATENT SPECIFICATION

(11) **EP 4 251 624 B1**
(45) Date of publication and mention of the grant of the patent: **29.10.2025**
(21) Application number: 21827727.5
(22) Date of filing: 22.11.2021
(51) Int. Cl.: C07D 471/04, A61K 31/4355, A61K 31/437, A61K 31/501, A61P 35/00, C07D 491/048, C07D 491/147, C07D 471/14, C07D 498/04, C07D 401/14, C07D 401/12, C07D 495/04

(54) **TRICYCLIC CARBOXAMIDE DERIVATIVES AS PRMT5 INHIBITORS**
TRIZYKLISCHE CARBOXAMIDDERIVATIVE ALS PRMT5 INHIBITOREN
DERIVES DE CARBOXAMIDE TRICYCLIQUE COMME DES INHIBITEURS DE PRMT5

(30) Priority: 24.11.2020 US 202063117937 P
(43) Date of publication of application: 04.10.2023
(73) Proprietor: Amgen Inc., Thousand Oaks, CA 91320-1799 (US)
(72) Inventor: AMEGADZIE, Albert, Thousand Oaks, California 91320-1799 (US); BEYLKIN, Diane Jennifer, Thousand Oaks, California 91320-1799 (US); BOOKER, Shon, Thousand Oaks, California 91320-1799 (US); BOURBEAU, Matthew Paul, Thousand Oaks, California 91320-1799 (US); BUTLER, John R., Thousand Oaks, California 91320-1799 (US); GLAD, Sanne Omholt Schroder, Thousand Oaks, California 91320-1799 (US); KOHN, Todd J., Thousand Oaks, California 91320-1799 (US); LANMAN, Brian Alan, Thousand Oaks, California 91320-1799 (US); LI, Kexue, Thousand Oaks, California (US); LIU, Qingyian, Thousand Oaks, California 91320-1799 (US); LOPEZ, Patricia, Thousand Oaks, California 91320-1799 (US); MANONI, Francesco, Thousand Oaks, Thousand Oaks (US); NAVARATNE, Primali Vasundera, Thousand Oaks, California 91320-1799 (US); PETTUS, Liping H., Thousand Oaks, California 91320-1799 (US); RAHIMOFF, Rene, Thousand Oaks, California 91320-1799 (US); TAMAYO, Nuria A., Thousand Oaks, California 91320-1799 (US); VESTERGAARD, Mikkel, Thousand Oaks, California 91320-1799 (US); WANG, Hui-Ling, Thousand Oaks, California 91320-1799 (US); WEIRES, Nicholas Anthony, Thousand Oaks, California 91320-1799 (US)
(74) Representative: Grünecker Patent- und Rechtsanwälte PartG mbB
(86) International application number: PCT/US2021/060332
(87) International publication number: WO 2022/115377

(56) References cited:
- WO-A1-2017/153515
- ELAYNE CHAN-PENEBRE ET AL: "A selective inhibitor of PRMT5 with in vivo and in vitro potency in MCL models", NATURE CHEMICAL BIOLOGY, vol. 11, no. 6, 27 April 2015 (2015-04-27), New York, pages 432 - 437, XP055297240, ISSN: 1552-4450, DOI: 10.1038/nchembio.1810

## Description

### BACKGROUND OF THE INVENTION

Epigenetic regulation of gene expression is an important biological determinant of protein production and cellular differentiation and plays a significant pathogenic role in a number of human diseases.

Epigenetic regulation involves heritable modification of genetic material without changing its nucleotide sequence. Typically, epigenetic regulation is mediated by selective and reversible modification (*e.g.,* methylation) of DNA and proteins (*e.g.,* histones) that control the conformational transition between transcriptionally active and inactive states of chromatin. These covalent modifications can be controlled by enzymes such as methyltransferases (e.g., PRMT5), many of which are associated with specific genetic alterations that can cause human disease. PRMT5 plays a role in diseases such as proliferative disorders, metabolic disorders, and blood disorders.

The homozygous deletion of tumor suppressor genes is a key driver of cancer, frequently resulting in the collateral loss of passenger genes located in close genomic proximity to the tumor suppressor. Deletion of these passenger genes can create therapeutically tractable vulnerabilities that are specific to tumor cells. Homozygous deletion of the chromosome 9p21 locus, which harbors the well-known tumor suppressor CDKN2A (cyclin dependent kinase inhibitor 2A), occurs in 15% of all tumors and frequently includes the passenger gene MTAP (methylthioadenosine phosphorylase), a key enzyme in the methionine and adenine salvage pathways. Deletion of MTAP results in accumulation of its substrate, methylthioadenosine (MTA). MTA shares close structural similarity to S-adenosylmethionine (SAM), the substrate methyl donor for the type II methyltransferase PRMT5. Elevated MTA levels, driven by loss of MTAP, selectively compete with SAM for binding to PRMT5, placing the methyltransferase in a hypomorphic state, vulnerable to further PRMT5 inhibition. Multiple genome scale shRNA drop out screens performed in large tumor cell line panels have identified a strong correlation between MTAP loss and cell line dependency on PRMT5, further highlighting the strength of this metabolic vulnerability. However, PRMT5 is a known cell essential gene and conditional PRMT5 knockout and siRNA knockdown studies suggest that significant liabilities could be associated with inhibiting PRMT5 in normal tissues (e.g., pan-cytopenia, infertility, skeletal muscle loss, cardiac hypertrophy). Therefore, novel strategies are required to exploit this metabolic vulnerability and preferentially target PRMT5 in MTAP null tumors while sparing PRMT5 in normal tissues (MTAP WT). Targeting PRMT5 with an MTA-cooperative small molecule inhibitor could preferentially target the MTA bound state of PRMT5, enriched in MTAP null tumor cells, while providing an improved therapeutic index over normal cells where MTAP is intact and MTA levels are low.

### SUMMARY OF THE INVENTION

In one aspect, the invention provides a compound of Formula I 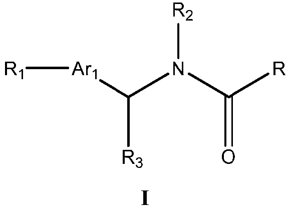 a tautomer thereof, a stereoisomer thereof, or a pharmaceutically acceptable salt of any of the foregoing. In one aspect, R is a tricycle independently selected from the formulae IA.

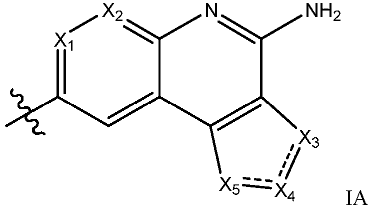

In another aspect, R can be a tricycle independently selected from the formulae IB 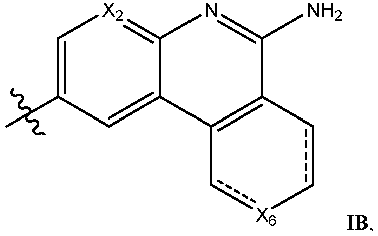 The invention provides that 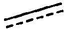 can be a single or double bond.

In one aspect, X¹, X² and X⁶ can be in each instance N, provided that both X¹ and X² cannot be N at the same time. In another aspect X¹, X² and X⁶ can be C. In one embodiment, if X¹ is C, it can be optionally substituted with halo. In a further aspect, halo could be Cl.
The invention further provides that X³, X⁴ and X⁵ can be at each instance optionally substituted C. In another aspect, X³, X⁴ and X⁵ can be at each instance optionally substituted O. In a further aspect, X³, X⁴ and X⁵ can be at each instance optionally substituted N. In a further aspect, X³, X⁴ and X⁵ can be at each instance optionally substituted and S. The invention provides that the substituents can be independently selected from C₁₋₃ alkyl, C₁₋₃ alkyl(OH), wherein alkyl can be optionally substituted with halo;

In one aspect of the invention, R³ in each instance can be H. In another aspect of the invention, R³ in each instance can be C₁₋₃ alkyl. In a further aspect, R³ can be methyl.

The invention provides that Ar¹ can be a six membered optionally substituted aryl. In another aspect, Ar¹ can be a six membered optionally substituted heteroaryl. In one embodiment, Ar¹ can be 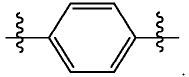 In another embodiment, Ar¹ can be 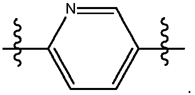 In a further aspect, Ar¹ can be 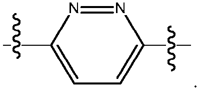 In another aspect, Ar¹ can be 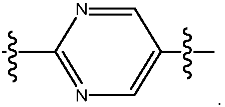 In yet another embodiment, Ar¹ can be 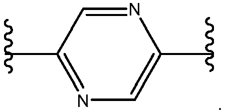 In another embodiment, Ar¹ can be 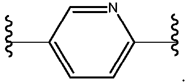 The invention provides that the Ar¹ substituents can be independently selected from C₁₋₃ alkyl. In another aspect, the substituents can be independently selected from -OC₁₋₃ alkyl. In a further aspect, the substituents can be independently selected from halo.

The invention provides that R¹ in each instance can be H. In another aspect, R¹ can be halo. In a further aspect, R¹ can be an optionally substituted C₁₋₃ alkyl. The substituents can be selected from halo and -CN. In a further aspect, R¹ can be an optionally substituted -O-C₁₋₃ alkyl. The substituents can be halo. In a further aspect, R¹ can be an optionally substituted -C(O)OC₁₋₃ alkyl, wherein C₁₋₃ alkyl can be optionally substituted with halo, and morpholinyl.

The invention provides that R² in each instance can be an optionally substituted C₁₋₈ alkyl. The C₁₋₈ alkyl substituents can be selected from halo, hydroxy, amino, -O- C₁₋₃ alkyl or -CN.

In a further aspect, R² in each instance can be an optionally substituted 5 or 6 membered cycle or heterocycle. The 5 or 6 membered cycle or heterocycle substituents can be hydroxy, amino, an optionally substituted C₁₋₆ alkyl, wherein the substituents are selected from halo. In a further aspect, R² can be an optionally substituted C₁₋₆ alkyl-O- C₁₋₃ alkyl, wherein the substituents are selected from halo. In another aspect, R² can be an optionally substituted 5,6,7,8-tetrahydro-[1,2,4]triazolo[1,5-a]pyridinyl. In another aspect, R² can be an C₁₋₃ alkyl-heterocyclyl, wherein the heterocyclyl can be an optionally substituted 3,4-dihydro-2H-pyrano[2,3-c]pyridinyl or pyradazinyl or triazolyl or pyrimidinyl or tetrahydrofuranyl or 1H-pyrrolo[2,3-b]pyridinyl or cyclohexyl. The substituents in each instance can be C₁₋₃ alkyl, -CN, or halo, or an optionally substituted C₁₋₆ alkyl-O- C₁₋₃ alkyl. In the latter case the substituents can be selected from halo; optionally substituted phenyl, wherein in turn the phenyl substituents can be selected from halo or C₁₋₃ alkyl.

The invention provides compounds of, the tautomer thereof, the stereoisomer thereof, or the pharmaceutically acceptable salt of any of the foregoing, wherein R¹ can be a tricycle of formulae IA. In another aspect, R¹ can be a tricycle of formulae IB.
In one aspect, when the compound is a tricycle of formulae IA, X¹ and X² can be both C. In another aspect, one of X¹ and X² can be C and another N. In the following embodiment, if X¹ is C, it can be unsubstituted or substituted with halo. In a further aspect, X² can be N.

The invention further provides compounds, the tautomer thereof, the stereoisomer thereof, or the pharmaceutically acceptable salt of any of the foregoing, wherein R can be a tricycle of the formulae **IA1**

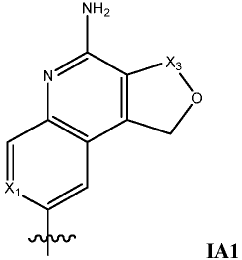

In one aspect, X³ can be C, unsubstituted or substituted with one or more methyl.

The invention further provides compounds, the tautomer thereof, the stereoisomer thereof, or the pharmaceutically acceptable salt of any of the foregoing, wherein R can be a tricycle of the formula **IA2**

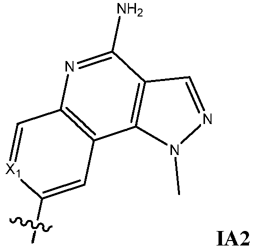

In one aspect of the compounds of the invention R³ can be H. in another aspect, R³ can be methyl.

The invention further provides compounds, the tautomer thereof, the stereoisomer thereof, or the pharmaceutically acceptable salt of any of the foregoing, wherein R² is an optionally substituted C₁₋₈ alkyl. In one embodiment, R² can be an optionally substituted methyl, ethyl, isopropyl, or cycloC₁₋₆alkyl.

All possible combinations between aspects and embodiments as disclosed above are comprised in the present invention.

The invention further provides compounds, the tautomer thereof, the stereoisomer thereof, or the pharmaceutically acceptable salt of any of the foregoing, wherein the compound is selected from the following:
In one aspect, the compound can be selected from 4-amino-N-(cyclopropylmethyl)-N-((5-(trifluoromethyl)-2-pyridinyl)methyl)-1,3-dihydrofuro[3,4-c]quinoline-8-carboxamide;
4-amino-N-((5-cyano-2-pyridinyl)methyl)-N-(2-propanyl)-1,3-dihydrofuro [3,4-c] quinoline-8-carboxamide;
4-amino-N-((6-cyclopropyl-3-pyridinyl)methyl)-N-(2-propanyl)-1,3-dihydrofuro [3,4-c] quinoline-8-carboxamide;
4-amino-N-((2R)-1-methoxy-2-propanyl)-N-((6-(trifluoromethyl)-3-pyridazinyl)methyl)-1,3-dihydrofuro[3,4-c]quinoline-8-carboxamide;
4-amino-N-((2S)-1-methoxy-2-propanyl)-N-((6-(trifluoromethyl)-3-pyridazinyl)methyl)-1,3-dihydrofuro[3,4-c]quinoline-8-carboxamide;
(3R)-4-amino-N-((2R)-1-methoxy-2-propanyl)-3-methyl-N-((6-(trifluoromethyl)-3-pyridazinyl)methyl)-1,3-dihydrofuro[3,4-c]quinoline-8-carboxamide;
(3R)-4-amino-N-((2S)-1-methoxy-2-propanyl)-3-methyl-N-((6-(trifluoromethyl)-3-pyridazinyl)methyl)-1,3-dihydrofuro[3,4-c]quinoline-8-carboxamide;
4-amino-N-((6-chloro-3-pyridazinyl)methyl)-N-((2R)-1-methoxy-2-propanyl)-1,3-dihydrofuro[3,4-c]quinoline-8-carboxamide;
4-amino-N-((6-chloro-3-pyridazinyl)methyl)-N-((2R)-1-methoxy-2-propanyl)-1,3-dihydrofuro[3,4-c][1,7]naphthyridine-8-carboxamide;
(3R)-4-amino-N-((6-chloro-3-pyridazinyl)methyl)-N-((2R)-1-methoxy-2-propanyl)-3-methyl-1,3-dihydrofuro[3,4-c]quinoline-8-carboxamide;
(3R)-4-amino-N-((6-chloro-3-pyridazinyl)methyl)-N-((2R)-1-methoxy-2-propanyl)-3-methyl-1,3-dihydrofuro[3,4-c] [1,7]naphthyridine-8-carboxamide;
(3S)-4-amino-N-(bicyclo[1.1.1]pentan-1-yl)-3-methyl-N-((5-(trifluoromethyl)-2-pyridinyl)methyl)-1,3-dihydrofuro[3,4-c] [1,7]naphthyridine-8-carboxamide;
(3R)-4-amino-3-methyl-N-(2-propanyl)-N-((5-(trifluoromethyl)-2-pyridinyl)methyl)-1,3-dihydrofuro[3,4-c][1,7]naphthyridine-8-carboxamide;
(3R)-4-amino-N-(bicyclo[1.1.1]pentan-1-yl)-3-methyl-N-((5-(trifluoromethyl)-2-pyridinyl)methyl)-1,3-dihydrofuro[3,4-c][1,7]naphthyridine-8-carboxamide;
(3R)-4-amino-N-((5-cyano-2-pyridinyl)methyl)-N,3-dimethyl-1,3-dihydrofuro[3,4-c][1,7]naphthyridine-8-carboxamide;
(3R)-4-amino-3-methyl-N-(2-propanyl)-N-((6-(trifluoromethyl)-3-pyridazinyl)methyl)-1,3-dihydrofuro[3,4-c][1,7]naphthyridine-8-carboxamide;
(3R)-4-amino-N-((2R)-1-methoxy-2-propanyl)-3-methyl-N-((6-(trifluoromethyl)-3-pyridazinyl)methyl)-1,3-dihydrofuro[3,4-c] [1,7]naphthyridine-8-carboxamide;
(3R)-4-amino-N-((2S)-1-methoxy-2-propanyl)-3-methyl-N-((6-(trifluoromethyl)-3-pyridazinyl)methyl)-1,3-dihydrofuro[3,4-c][1,7]naphthyridine-8-carboxamide;
(3R)-4-amino-N-cyclopropyl-3-methyl-N-((5-(trifluoromethyl)-2-pyridinyl)methyl)-1,3-dihydrofuro[3,4-c][1,7]naphthyridine-8-carboxamide;
(3R)-4-amino-N-((5-cyano-2-pyridinyl)methyl)-N-cyclopropyl-3-methyl-1,3-dihydrofuro[3,4-c][1,7]naphthyridine-8-carboxamide;
(3R)-4-amino-N,3-dimethyl-N-((6-(trifluoromethyl)-3-pyridazinyl)methyl)-1,3-dihydrofuro[3,4-c][1,7]naphthyridine-8-carboxamide;
(3S)-4-amino-N-cyclopropyl-3-methyl-N-((5-(trifluoromethyl)-2-pyridinyl)methyl)-1,3-dihydrofuro[3,4-c][1,7]naphthyridine-8-carboxamide;
(3S)-4-amino-N,3-dimethyl-N-((6-(trifluoromethyl)-3-pyridazinyl)methyl)-1,3-dihydrofuro[3,4-c][1,7]naphthyridine-8-carboxamide;
(3R)-4-amino-3-methyl-N-(2,2,2-trifluoroethyl)-N-((5-(trifluoromethyl)-2-pyridinyl)methyl)-1,3-dihydrofuro[3,4-c][1,7]naphthyridine-8-carboxamide;
4-amino-N-(cyclopropylmethyl)-N-((5-(trifluoromethyl)-2-pyridinyl)methyl)-1,3-dihydrofuro[3,4-c][1,8]naphthyridine-8-carboxamide;
4-amino-N-((2R)-1-methoxy-2-propanyl)-N-((5-(trifluoromethyl)-2-pyridinyl)methyl)-1,3-dihydrofuro[3,4-c][1,8]naphthyridine-8-carboxamide;
4-amino-N-((5-cyclopropyl-2-pyridinyl)methyl)-N-methyl-1,3-dihydrofuro[3,4-c]quinoline-8-carboxamide;
4-amino-N-((5-cyano-2-pyridinyl)methyl)-N-methyl-1,3-dihydrofuro[3,4-c]quinoline-8-carboxamide;
4-amino-N-((5-cyano-2-pyridinyl)methyl)-N-(cyclopropylmethyl)-1,3-dihydrofuro[3,4-c]quinoline-8-carboxamide;
(3R)-4-amino-N-((6-bromo-3-pyridazinyl)methyl)-N-(cyclopropylmethyl)-3-methyl-1,3-dihydrofuro[3,4-c]quinoline-8-carboxamide;
4-amino-N-((6-bromo-3-pyridazinyl)methyl)-N-methyl-1,3-dihydrofuro[3,4-c]quinoline-8-carboxamide;
(3R)-4-amino-N-((5-bromo-2-pyridinyl)methyl)-N,3-dimethyl-1,3-dihydrofuro[3,4-c]quinoline-8-carboxamide;
(3R)-4-amino-N-((6-cyclopropyl-3-pyridazinyl)methyl)-3-methyl-N-((1R)-1-(2-pyrimidinyl)ethyl)-1,3-dihydrofuro[3,4-c]quinoline-8-carboxamide;
4-amino-N-((5-bromo-2-pyridinyl)methyl)-N-methyl-1,3-dihydrofuro[3,4-c]quinoline-8-carboxamide;
(3R)-4-amino-N-((5-cyano-2-pyridinyl)methyl)-N-(cyclopropylmethyl)-3-methyl-1,3-dihydrofuro[3,4-c]quinoline-8-carboxamide;
(3R)-4-amino-N-((6-bromo-3-pyridazinyl)methyl)-3-methyl-N-(2-propanyl)-1,3-dihydrofuro[3,4-c]quinoline-8-carboxamide;
(3R)-4-amino-N-(cyclopropylmethyl)-3-methyl-N-((6-(trifluoromethyl)-3-pyridazinyl)methyl)-1,3-dihydrofuro[3,4-c]quinoline-8-carboxamide;
(3R)-4-amino-N-(cyclopropylmethyl)-3-methyl-N-((5-(trifluoromethyl)-2-pyridinyl)methyl)-1,3-dihydrofuro[3,4-c]quinoline-8-carboxamide;
4-amino-N-((6-bromo-3-pyridazinyl)methyl)-N-(2-propanyl)-1,3-dihydrofuro [3,4-c] quinoline-8-carboxamide;
4-amino-N-(cyclopropylmethyl)-N-((6-(trifluoromethyl)-3 -pyridazinyl)methyl)- 1,3 -dihydrofuro [3,4-c]quinoline-8-carboxamide;
4-amino-N-((1-methylcyclopropyl)methyl)-N-((5-(trifluoromethyl)-2-pyridinyl)methyl)-1,3-dihydrofuro[3,4-c]quinoline-8-carboxamide;
(3R)-4-amino-N-(2,2-dimethylpropyl)-3-methyl-N-((5-(trifluoromethyl)-2-pyridinyl)methyl)-1,3-dihydrofuro[3,4-c]quinoline-8-carboxamide;
(3R)-4-amino-3-methyl-N-((1-methylcyclopropyl)methyl)-N-((5-(trifluoromethyl)-2-pyridinyl)methyl)-1,3-dihydrofuro[3,4-c]quinoline-8-carboxamide;
4-amino-N-(2,2-dimethylpropyl)-3,3-dimethyl-N-((5-(trifluoromethyl)-2-pyridinyl)methyl)-1,3-dihydrofuro[3,4-c]quinoline-8-carboxamide;
4-amino-3,3-dimethyl-N-((1-methylcyclopropyl)methyl)-N-((5-(trifluoromethyl)-2-pyridinyl)methyl)-1,3-dihydrofuro[3,4-c]quinoline-8-carboxamide;
4-amino-N-(2,2-dimethylpropyl)-N-((5-(trifluoromethyl)-2-pyridinyl)methyl)-1,3-dihydrofuro[3,4-c]quinoline-8-carboxamide;
4-amino-N-cyclopropyl-N-((5-cyclopropyl-2-pyridinyl)methyl)-1,3-dihydrofuro [3,4-c] quinoline-8-carboxamide;
4-amino-N-methyl-N-((5-(trifluoromethyl)-2-pyridinyl)methyl)-1,3-dihydrofuro[3,4-c][1,7]naphthyridine-8-carboxamide;
4-amino-7-fluoro-N-(2-propanyl)-N-((5-(trifluoromethyl)-2-pyridinyl)methyl)-1,3-dihydrofuro[3,4-c]quinoline-8-carboxamide;
4-amino-N-((5-cyano-2-pyridinyl)methyl)-N-(2-propanyl)-1,3-dihydrofuro[3,4-c] [1,7]naphthyridine-8-carboxamide;
4-amino-N-((5-cyano-2-pyridinyl)methyl)-N-cyclopropyl-1,3-dihydrofuro[3,4-c][1,7]naphthyridine-8-carboxamide;
4-amino-N-cyclopropyl-N-((5-cyclopropyl-2-pyridinyl)methyl)-1,3-dihydrofuro [3,4-c][1,7]naphthyridine-8-carboxamide;
4-amino-N-((5-cyclopropyl-2-pyridinyl)methyl)-N-methyl-1,3-dihydrofuro[3,4-c][1,7]naphthyridine-8-carboxamide;
(3R)-4-amino-N-((6-cyclopropyl-3-pyridinyl)methyl)-3-methyl-N-(2-propanyl)-1,3-dihydrofuro[3,4-c]quinoline-8-carboxamide;
(3R)-4-amino-N-cyclopropyl-3-methyl-N-((5-(trifluoromethyl)-2-pyridinyl)methyl)-1,3-dihydrofuro[3,4-c]quinoline-8-carboxamide;
4-amino-N-methyl-N-((6-(trifluoromethyl)-3-pyridazinyl)methyl)-1,3-dihydrofuro[3,4-c]quinoline-8-carboxamide;
(3R)-4-amino-N-cyclopropyl-3-methyl-N-((6-(trifluoromethyl)-3-pyridazinyl)methyl)-1,3-dihydrofuro[3,4-c]quinoline-8-carboxamide;
(3R)-4-amino-N-((5-cyano-2-pyridinyl)methyl)-3-methyl-N-(2-propanyl)-1,3-dihydrofuro[3,4-c]quinoline-8-carboxamide;
(3R)-4-amino-N-((5-cyano-2-pyridinyl)methyl)-N-cyclopropyl-3-methyl-1,3-dihydrofuro[3,4-c]quinoline-8-carboxamide;
(3R)-4-amino-N-((5-cyclopropyl-2-pyridinyl)methyl)-N,3-dimethyl-1,3-dihydrofuro[3,4-c]quinoline-8-carboxamide;
4-amino-N-cyclopropyl-7-fluoro-N-((6-(trifluoromethyl)-3-pyridazinyl)methyl)-1,3-dihydrofuro[3,4-c]quinoline-8-carboxamide;
4-amino-7-fluoro-N-(2-propanyl)-N-((6-(trifluoromethyl)-3-pyridazinyl)methyl)-1,3-dihydrofuro[3,4-c]quinoline-8-carboxamide;
4-amino-7-fluoro-N-methyl-N-((6-(trifluoromethyl)-3-pyridazinyl)methyl)-1,3-dihydrofuro[3,4-c]quinoline-8-carboxamide;
(3R)-4-amino-N-ethyl-3-methyl-N-((5-(trifluoromethyl)-2-pyridinyl)methyl)-1,3-dihydrofuro[3,4-c]quinoline-8-carboxamide;
4-amino-N-ethyl-N-((5-(trifluoromethyl)-2-pyridinyl)methyl)-1,3-dihydrofuro[3,4-c] [1,7]naphthyridine-8-carboxamide;
4-amino-N-ethyl-N-((5-(trifluoromethyl)-2-pyridinyl)methyl)-1,3-dihydrofuro[3,4-c]quinoline-8-carboxamide;
4-amino-N-((6-cyclopropyl-3-pyridinyl)methyl)-7-fluoro-N-(2-propanyl)-1,3-dihydrofuro[3,4-c]quinoline-8-carboxamide;
4-amino-N-((5-cyclopropyl-2-pyridinyl)methyl)-N-(2-propanyl)-1,3-dihydrofuro [3,4-c] quinoline-8-carboxamide;
4-amino-N-cyclopropyl-N-((5-(trifluoromethyl)-2-pyridinyl)methyl)-1,3-dihydrofuro[3,4-c][1,7]naphthyridine-8-carboxamide;
4-amino-N-(2-propanyl)-N-((6-(trifluoromethyl)-3-pyridazinyl)methyl)-1,3-dihydrofuro[3,4-c][1,7]naphthyridine-8-carboxamide;
4-amino-N-((5-cyano-2-pyridinyl)methyl)-N-methyl-1,3-dihydrofuro[3,4-c][1,7]naphthyridine-8-carboxamide;
(3R)-4-amino-N-cyclopropyl-N-((5-cyclopropyl-2-pyridinyl)methyl)-3-methyl-1,3-dihydrofuro[3,4-c]quinoline-8-carboxamide;
4-amino-N-cyclopropyl-7-fluoro-N-((5 -(trifluoromethyl)-2-pyridinyl)methyl)- 1,3 -dihydrofuro [3,4-c]quinoline-8-carboxamide;
4-amino-N-cyclopropyl-N-((5-cyclopropyl-2-pyridinyl)methyl)-7-fluoro-1,3-dihydrofuro[3,4-c]quinoline-8-carboxamide;
4-amino-N-((5-cyclopropyl-2-pyridinyl)methyl)-7-fluoro-N-(2-propanyl)-1,3-dihydrofuro[3,4-c]quinoline-8-carboxamide;
4-amino-N-((5-cyclopropyl-2-pyridinyl)methyl)-7-fluoro-N-methyl-1,3-dihydrofuro [3,4-c] quinoline-8-carboxamide;
4-amino-N-((6-ethoxy-3-pyridazinyl)methyl)-N-((2R)-1-methoxy-2-propanyl)-1,3-dihydrofuro[3,4-c]quinoline-8-carboxamide;
(3R)-4-amino-N-((6-ethoxy-3-pyridazinyl)methyl)-N-((2R)-1-methoxy-2-propanyl)-3-methyl-1,3-dihydrofuro[3,4-c]quinoline-8-carboxamide;
4-amino-N-methyl-N-((6-(trifluoromethyl)-3-pyridazinyl)methyl)-1,3-dihydrofuro[3,4-c][1,7]naphthyridine-8-carboxamide;
(3R)-4-amino-N,3-dimethyl-N-((5 -(trifluoromethyl)-2-pyridinyl)methyl)- 1,3-dihydrofuro [3,4-c]quinoline-8-carboxamide;
(3R)-4-amino-N-((5-cyclopropyl-2-pyridinyl)methyl)-3-methyl-N-(2-propanyl)-1,3-dihydrofuro[3,4-c]quinoline-8-carboxamide;
4-amino-N-(bicyclo[1.1.1]pentan-1-yl)-N-((6-ethoxy-3-pyridazinyl)methyl)-1,3-dihydrofuro[3,4-c][1,7]naphthyridine-8-carboxamide;
4-amino-7-chloro-N-((5-cyclopropyl-2-pyridinyl)methyl)-N-(2-propanyl)-1,3-dihydrofuro[3,4-c]quinoline-8-carboxamide;
(3R)-4-amino-N-(bicyclo[1.1.1]pentan-1-yl)-3-methyl-N-((5-(trifluoromethyl)-2-pyridinyl)methyl)-1,3-dihydrofuro[3,4-c]quinoline-8-carboxamide;
4-amino-N-(bicyclo[1.1.1]pentan-1-yl)-7-fluoro-N-((5-(trifluoromethyl)-2-pyridinyl)methyl)-1,3-dihydrofuro[3,4-c]quinoline-8-carboxamide;
4-amino-N-cyclopropyl-N-((6-(trifluoromethyl)-3-pyridazinyl)methyl)-1,3-dihydrofuro[3,4-c][1,7]naphthyridine-8-carboxamide;
4-amino-N-((6-cyclopropyl-3-pyridinyl)methyl)-N-(2-propanyl)-1,3-dihydrofuro[3,4-c][1,7]naphthyridine-8-carboxamide;
(3R)-4-amino-3-methyl-N-(2-propanyl)-N-((5-(trifluoromethyl)-2-pyridinyl)methyl)-1,3-dihydrofuro[3,4-c]quinoline-8-carboxamide;
4-amino-N-((5-cyclopropyl-2-pyridinyl)methyl)-N-(2-propanyl)-1,3-dihydrofuro[3,4-c][1,7]naphthyridine-8-carboxamide;
(3R)-4-amino-N-((5-cyano-2-pyridinyl)methyl)-N,3-dimethyl-1,3-dihydrofuro[3,4-c]quinoline-8-carboxamide;
4-amino-N-(bicyclo[1.1.1]pentan-1-yl)-N-((6-ethoxy-3-pyridazinyl)methyl)-1,3-dihydrofuro[3,4-c]quinoline-8-carboxamide;
4-amino-N-((6-ethoxy-3-pyridazinyl)methyl)-N-((2R)-1-methoxy-2-propanyl)-1,3-dihydrofuro[3,4-c][1,7]naphthyridine-8-carboxamide;
4-amino-7-chloro-N-methyl-N-((6-(trifluoromethyl)-3-pyridazinyl)methyl)-1,3-dihydrofuro[3,4-c]quinoline-8-carboxamide;
4-amino-N-((2R)-1-methoxy-2-propanyl)-N-((6-(trifluoromethyl)-3-pyridazinyl)methyl)-1,3-dihydrofuro[3,4-c][1,7]naphthyridine-8-carboxamide;
4-amino-N-((2S)-1-methoxy-2-propanyl)-N-((6-(trifluoromethyl)-3-pyridazinyl)methyl)-1,3-dihydrofuro[3,4-c][1,7]naphthyridine-8-carboxamide;
4-amino-N-(2-propanyl)-N-((6-(trifluoromethyl)-3-pyridazinyl)methyl)-1,3-dihydrofuro [3,4-c] quinoline-8-carboxamide;
4-amino-7-chloro-N-((5-cyano-2-pyridinyl)methyl)-N-(2-propanyl)-1,3-dihydrofuro[3,4-c]quinoline-8-carboxamide;
(3R)-4-amino-3-methyl-N-(2-propanyl)-N-((6-(trifluoromethyl)-3-pyridazinyl)methyl)-1,3-dihydrofuro[3,4-c]quinoline-8-carboxamide;
4-amino-7-chloro-N-(2-propanyl)-N-((6-(trifluoromethyl)-3-pyridazinyl)methyl)- 1,3 -dihydrofuro [3,4-c]quinoline-8-carboxamide;
4-amino-N-(bicyclo[1.1.1]pentan-1-yl)-7-chloro-N-((5-(trifluoromethyl)-2-pyridinyl)methyl)-1,3-dihydrofuro[3,4-c]quinoline-8-carboxamide;
(3R)-4-amino-N-(cyclopropylmethyl)-3-methyl-N-((5-(trifluoromethyl)-2-pyridinyl)methyl)-1,3-dihydrofuro[3,4-c][1,7]naphthyridine-8-carboxamide;
(3R)-4-amino-N-((6-ethoxy-3-pyridazinyl)methyl)-N-((2R)-1-methoxy-2-propanyl)-3-methyl-1,3-dihydrofuro[3,4-c][1,7]naphthyridine-8-carboxamide;
4-amino-N-((1-methylcyclopropyl)methyl)-N-((5-(trifluoromethyl)-2-pyridinyl)methyl)-1,3-dihydrofuro[3,4-c][1,7]naphthyridine-8-carboxamide;
4-amino-N-methyl-N-(4-(trifluoromethyl)benzyl)-1,3-dihydrofuro[3,4-c]quinoline-8-carboxamide;
4-amino-N-methyl-N-((6-(trifluoromethyl)-3-pyridinyl)methyl)-1,3-dihydrofuro [3,4-c] quinoline-8-carboxamide;
4-amino-7-chloro-N-((6-cyclopropyl-3-pyridinyl)methyl)-N-(2-propanyl)-1,3-dihydrofuro[3,4-c]quinoline-8-carboxamide;
(3R)-4-amino-N-(cyclopropylmethyl)-3-methyl-N-((6-(trifluoromethyl)-3-pyridazinyl)methyl)-1,3-dihydrofuro[3,4-c][1,7]naphthyridine-8-carboxamide;
(3S)-4-amino-N-((6-cyclopropyl-3-pyridinyl)methyl)-3-methyl-N-(2-propanyl)-1,3-dihydrofuro[3,4-c][1,7]naphthyridine-8-carboxamide;
(3R)-4-amino-N-((5-cyano-2-pyridinyl)methyl)-3-methyl-N-(2-propanyl)-1,3-dihydrofuro[3,4-c][1,7]naphthyridine-8-carboxamide;
(3R)-4-amino-N-cyclopropyl-3-methyl-N-((6-(trifluoromethyl)-3-pyridazinyl)methyl)-1,3-dihydrofuro[3,4-c][1,7]naphthyridine-8-carboxamide;
(3R)-4-amino-N-((5-cyclopropyl-2-pyridinyl)methyl)-3-methyl-N-(2-propanyl)-1,3-dihydrofuro[3,4-c][1,7]naphthyridine-8-carboxamide;
(3 S)-4-amino-N-((5 -cyclopropyl-2-pyridinyl)methyl)-3-methyl-N-(2-propanyl)- 1,3-dihydrofuro [3,4-c][1,7]naphthyridine-8-carboxamide;
(3R)-4-amino-N-ethyl-3-methyl-N-((5-(trifluoromethyl)-2-pyridinyl)methyl)-1,3-dihydrofuro[3,4-c][1,7]naphthyridine-8-carboxamide;
(3R)-4-amino-N-(bicyclo[1.1.1]pentan-1-yl)-N-((6-ethoxy-3-pyridazinyl)methyl)-3-methyl-1,3-dihydrofuro[3,4-c][1,7]naphthyridine-8-carboxamide;
(3R)-4-amino-N-((6-bromo-3-pyridazinyl)methyl)-N-((2R)-1-methoxy-2-propanyl)-3-methyl-1,3-dihydrofuro[3,4-c][1,7]naphthyridine-8-carboxamide;
(3R)-4-amino-N,3-dimethyl-N-((5-(trifluoromethyl)-2-pyridinyl)methyl)-1,3-dihydrofuro[3,4-c][1,7]naphthyridine-8-carboxamide;
(3R)-4-amino-N-((6-cyclopropyl-3-pyridinyl)methyl)-3-methyl-N-(2-propanyl)-1,3-dihydrofuro[3,4-c][1,7]naphthyridine-8-carboxamide;
methyl 4-(6-((((4-amino-1,3-dihydrofuro[3,4-c]quinolin-8-yl)carbonyl)(methyl)amino)methyl)-3-pyridinyl)-1-piperazinecarboxylate;
(3S)-4-amino-N-((5-cyclopropyl-2-pyridinyl)methyl)-N,3-dimethyl-1,3-dihydrofuro[3,4-c][1,7]naphthyridine-8-carboxamide;
(3R)-4-amino-N-((5-cyclopropyl-2-pyridinyl)methyl)-N,3-dimethyl-1,3-dihydrofuro[3,4-c][1,7]naphthyridine-8-carboxamide;
(3R)-4-amino-N-cyclopropyl-N-((5-cyclopropyl-2-pyridinyl)methyl)-3-methyl-1,3-dihydrofuro[3,4-c][1,7]naphthyridine-8-carboxamide;
(3S)-4-amino-N-cyclopropyl-N-((5-cyclopropyl-2-pyridinyl)methyl)-3-methyl-1,3-dihydrofuro[3,4-c][1,7]naphthyridine-8-carboxamide;
4-amino-N-(cyclopropylmethyl)-N-((6-(difluoromethoxy)-3-pyridazinyl)methyl)-1,3-dihydrofuro[3,4-c][1,7]naphthyridine-8-carboxamide;
4-amino-N-ethyl-7-fluoro-N-((5-(trifluoromethyl)-2-pyridinyl)methyl)-1,3-dihydrofuro [3,4-c] quinoline-8-carboxamide;
4-amino-7-chloro-N-ethyl-N-((5-(trifluoromethyl)-2-pyridinyl)methyl)-1,3-dihydrofuro[3,4-c]quinoline-8-carboxamide;
(3R)-4-amino-N-((5-chloro-2-pyridinyl)methyl)-N-ethyl-3-methyl-1,3-dihydrofuro[3,4-c]quinoline-8-carboxamide;
4-amino-N-ethyl-3-methyl-N-((5-(trifluoromethyl)-2-pyridinyl)methyl)-3H-pyrazolo[3,4-c]quinoline-8-carboxamide;
(3R)-4-amino-N-((5-chloro-2-pyridinyl)methyl)-3-methyl-N-(2-propanyl)-1,3-dihydrofuro[3,4-c]quinoline-8-carboxamide;
(3R)-4-amino-N-(cyclopropylmethyl)-N-((6-(difluoromethoxy)-3-pyridazinyl)methyl)-3-methyl-1,3-dihydrofuro[3,4-c][1,7]naphthyridine-8-carboxamide;
(3S)-4-amino-N-(cyclopropylmethyl)-N-((6-(difluoromethoxy)-3-pyridazinyl)methyl)-3-methyl-1,3-dihydrofuro[3,4-c][1,7]naphthyridine-8-carboxamide;
4-amino-N-(cyclopropylmethyl)-N-((6-(difluoromethoxy)-3-pyridazinyl)methyl)-1,3-dihydrofuro[3,4-c]quinoline-8-carboxamide;
4-amino-N-ethyl-7-fluoro-3-methyl-N-((5-(trifluoromethyl)-2-pyridinyl)methyl)-3H-pyrazolo[3,4-c]quinoline-8-carboxamide;
5-amino-N-ethyl-N-((5-(trifluoromethyl)-2-pyridinyl)methyl)benzo[c][2,6]naphthyridine-9-carboxamide;
4-amino-N-((5-(3,6-dihydro-2H-pyran-4-yl)-2-pyridinyl)methyl)-N-methyl-1,3-dihydrofuro[3,4-c]quinoline-8-carboxamide;
methyl 6-((((4-amino-1,3-dihydrofuro[3,4-c]quinolin-8-yl)carbonyl)(methyl)amino)methyl)-3',6'-dihydro[3,4'-bipyridine]-1'(2'H)-carboxylate;
5-oxo-N-(2-propanyl)-N-((5-(trifluoromethyl)-2-pyridinyl)methyl)-5,6-dihydropyrazolo[1,5-c]quinazoline-9-carboxamide;
4-amino-1,3-dimethyl-N-(2-propanyl)-N-((5-(trifluoromethyl)-2-pyridinyl)methyl)-1H-pyrazolo [4,3-c]quinoline-8-carboxamide;
(3R)-4-amino-N-((5-bromo-2-pyridinyl)methyl)-N,3-dimethyl-1,3-dihydrofuro[3,4-c][1,7]naphthyridine-8-carboxamide;
(3S)-4-amino-N-((5-bromo-2-pyridinyl)methyl)-N,3-dimethyl-1,3-dihydrofuro[3,4-c][1,7]naphthyridine-8-carboxamide;
(3R)-4-amino-3-methyl-N-(2-propanyl)-N-((5-(trifluoromethyl)-2-pyrazinyl)methyl)-1,3-dihydrofuro[3,4-c]quinoline-8-carboxamide;
4-amino-7-fluoro-N-((1R)-1-(2-pyrimidinyl)ethyl)-N-((5-(trifluoromethyl)-2-pyridinyl)methyl)-1,3-dihydrofuro[3,4-c]quinoline-8-carboxamide;
4-amino-N-((5-cyano-2-pyridinyl)methyl)-7-fluoro-N-((1R)-1-(2-pyrimidinyl)ethyl)-1,3-dihydrofuro[3,4-c]quinoline-8-carboxamide;
4-amino-N-((6-bromo-3-pyridazinyl)methyl)-7-fluoro-N-((1R)-1-(3-fluoro-2-pyridinyl)ethyl)-1,3-dihydrofuro[3,4-c]quinoline-8-carboxamide;
4-amino-N-((5-(difluoromethyl)-2-pyridinyl)methyl)-N-((1R)-1-(2-pyrimidinyl)ethyl)-1,3-dihydrofuro[3,4-c]quinoline-8-carboxamide;
4-amino-N-((5-chloro-2-pyridinyl)methyl)-N-((1R)-1-(2-pyrimidinyl)ethyl)-1,3-dihydrofuro[3,4-c]quinoline-8-carboxamide;
4-amino-N-((1S)-1-(2-pyrimidinyl)ethyl)-N-((6-(trifluoromethyl)-3-pyridazinyl)methyl)-1,3-dihydrofuro[3,4-c]quinoline-8-carboxamide;
4-amino-N-((1R)-1-(2-pyrimidinyl)ethyl)-N-((6-(trifluoromethyl)-3-pyridazinyl)methyl)-1,3-dihydrofuro[3,4-c]quinoline-8-carboxamide;
4-amino-N-((1R)-1-(3-fluoro-2-pyridinyl)ethyl)-N-((6-(trifluoromethyl)-3-pyridazinyl)methyl)-1,3-dihydrofuro[3,4-c]quinoline-8-carboxamide;
4-amino-N-cyclopropyl-N-((5-(trifluoromethyl)-2-pyridinyl)methyl)-1,3-dihydrofuro [3,4-c] quinoline-8-carboxamide;
4-amino-N-(1,3-dimethoxy-2-propanyl)-N-((5-(trifluoromethyl)-2-pyridinyl)methyl)-1,3-dihydrofuro[3,4-c]quinoline-8-carboxamide;
4-amino-N-((2R)-1-methoxy-2-propanyl)-N-((5-(trifluoromethyl)-2-pyridinyl)methyl)-1,3-dihydrofuro[3,4-c]quinoline-8-carboxamide;
4-amino-N-((6-methoxy-3-pyridazinyl)methyl)-N-((1R)-1-(2-pyrimidinyl)ethyl)-1,3-dihydrofuro[3,4-c]quinoline-8-carboxamide;
4-amino-N-((8R)-5,6,7,8-tetrahydro[1,2,4]triazolo[1,5-a]pyridin-8-yl)-N-((5-(trifluoromethyl)-2-pyridinyl)methyl)-1,3-dihydrofuro[3,4-c]quinoline-8-carboxamide;
4-amino-N-((8S)-5,6,7,8-tetrahydro[1,2,4]triazolo[1,5-a]pyridin-8-yl)-N-((5-(trifluoromethyl)-2-pyridinyl)methyl)-1,3-dihydrofuro[3,4-c]quinoline-8-carboxamide;
4-amino-N-((5-cyano-2-pyridinyl)methyl)-N-((8R)-5,6,7,8-tetrahydro[1,2,4]triazolo[1,5-a]pyridin-8-yl)-1,3-dihydrofuro[3,4-c]quinoline-8-carboxamide;
4-amino-N-((5-cyano-2-pyridinyl)methyl)-N-((8S)-5,6,7,8-tetrahydro[1,2,4]triazolo[1,5-a]pyridin-8-yl)-1,3-dihydrofuro[3,4-c]quinoline-8-carboxamide;
4-amino-N-(4-(3-oxetanyl)benzyl)-N-((1R)-1-(2-pyrimidinyl)ethyl)-1,3-dihydrofuro[3,4-c]quinoline-8-carboxamide;
4-amino-N-(4-(3-oxetanyl)benzyl)-N-((1S)-1-(2-pyrimidinyl)ethyl)-1,3-dihydrofuro[3,4-c]quinoline-8-carboxamide;
4-amino-N-methyl-N-((5-(trifluoromethyl)-2-pyridinyl)methyl)-1,3-dihydrofuro [3,4-c] quinoline-8-carboxamide;
4-amino-N-(2-propanyl)-N-((5-(trifluoromethyl)-2-pyridinyl)methyl)-1,3-dihydrofuro [3,4-c] quinoline-8-carboxamide;
4-amino-N-(cyclopropylmethyl)-N-((6-(4-morpholinyl)-3-pyridazinyl)methyl)-1,3-dihydrofuro[3,4-c]quinoline-8-carboxamide;
4-amino-N-((1R)-1-(2-pyrimidinyl)ethyl)-N-((6-(2,2,2-trifluoroethoxy)-3-pyridazinyl)methyl)-1,3-dihydrofuro[3,4-c]quinoline-8-carboxamide;
4-amino-N-((5-cyano-2-pyridinyl)methyl)-N-cyclopropyl-1,3-dihydrofuro[3,4-c]quinoline-8-carboxamide;
4-amino-N-((5-bromo-2-pyridinyl)methyl)-N-cyclopropyl-1,3-dihydrofuro[3,4-c]quinoline-8-carboxamide;
4-amino-N-cyclopropyl-N-((5-(difluoromethyl)-2-pyridinyl)methyl)-1,3-dihydrofuro [3,4-c] quinoline-8-carboxamide;
4-amino-N-((1-cyanocyclopropyl)methyl)-N-((5-cyano-2-pyridinyl)methyl)-1,3-dihydrofuro[3,4-c]quinoline-8-carboxamide;
4-amino-N-((6-cyclopropyl-3-pyridazinyl)methyl)-N-((1R)-1-(2-pyrimidinyl)ethyl)-1,3-dihydrofuro[3,4-c]quinoline-8-carboxamide;
4-amino-N-((3R,4S)-3-methoxytetrahydro-2H-pyran-4-yl)-N-((5-(trifluoromethyl)-2-pyridinyl)methyl)-1,3-dihydrofuro[3,4-c]quinoline-8-carboxamide;
4-amino-N-((3S,4R)-3-methoxytetrahydro-2H-pyran-4-yl)-N-((5-(trifluoromethyl)-2-pyridinyl)methyl)-1,3-dihydrofuro[3,4-c]quinoline-8-carboxamide;
4-amino-N-((3R,4R)-4-methoxytetrahydro-2H-pyran-3-yl)-N-((5-(trifluoromethyl)-2-pyridinyl)methyl)-1,3-dihydrofuro[3,4-c]quinoline-8-carboxamide;
4-amino-N-((3S,4S)-4-methoxytetrahydro-2H-pyran-3-yl)-N-((5-(trifluoromethyl)-2-pyridinyl)methyl)-1,3-dihydrofuro[3,4-c]quinoline-8-carboxamide;
4-amino-7-chloro-N-((5-cyano-2-pyridinyl)methyl)-N-cyclopropyl-1,3-dihydrofuro [3,4-c] quinoline-8-carboxamide;
4-amino-N-((6-chloro-5-methoxy-2-pyridinyl)methyl)-N-(2-propanyl)-1,3-dihydrofuro[3,4-c]quinoline-8-carboxamide;
4-amino-N-((6-bromo-3-pyridazinyl)methyl)-N-((2R)-1-methoxy-2-propanyl)-1,3-dihydrofuro[3,4-c]quinoline-8-carboxamide;
4-amino-7-chloro-N-(cyclopropylmethyl)-N-((5-(trifluoromethyl)-2-pyridinyl)methyl)-1,3-dihydrofuro[3,4-c]quinoline-8-carboxamide;
(3R)-4-amino-3-methyl-N-(2-propanyl)-N-((5-(trifluoromethoxy)-2-pyridinyl)methyl)-1,3-dihydrofuro[3,4-c]quinoline-8-carboxamide;
(3R)-4-amino-N-((5-(difluoromethyl)-2-pyridinyl)methyl)-3-methyl-N-((1R)-1-(1-methyl-1H-1,2,4-triazol-3-yl)ethyl)-1,3-dihydrofuro[3,4-c]quinoline-8-carboxamide;
(3R)-4-amino-3-methyl-N-(1-methyl-1H-pyrazol-4-yl)-N-((5-(trifluoromethyl)-2-pyridinyl)methyl)-1,3-dihydrofuro[3,4-c]quinoline-8-carboxamide;
(3R)-4-amino-N-((6-bromo-3-pyridazinyl)methyl)-N-((2R)-1-methoxy-2-propanyl)-3-methyl-1,3-dihydrofuro[3,4-c]quinoline-8-carboxamide;
4-amino-N-(3,4-dihydro-2H-pyrano[2,3-c]pyridin-6-ylmethyl)-N-((1R)-1-(2-pyrimidinyl)ethyl)-1,3-dihydrofuro[3,4-c]quinoline-8-carboxamide;
4-amino-7-chloro-N-methyl-N-((5-(trifluoromethyl)-2-pyridinyl)methyl)-1,3-dihydrofuro [3,4-c]quinoline-8-carboxamide;
4-amino-N-(bicyclo[1.1.1]pentan-1-yl)-N-((5-(trifluoromethyl)-2-pyridinyl)methyl)-1,3-dihydrofuro[3,4-c]quinoline-8-carboxamide;
4-amino-7-chloro-N-((5-cyano-2-pyridinyl)methyl)-N-methyl-1,3-dihydrofuro [3,4-c] quinoline-8-carboxamide;
4-amino-N-((5-cyano-2-pyridinyl)methyl)-N-((2R)-1-fluoro-2-propanyl)-1,3-dihydrofuro[3,4-c]quinoline-8-carboxamide;
4-amino-N-((5-cyano-2-pyridinyl)methyl)-N-((2S)-1-fluoro-2-propanyl)-1,3-dihydrofuro[3,4-c]quinoline-8-carboxamide;
4-amino-7-chloro-N-((5-cyano-2-pyridinyl)methyl)-N-(cyclopropylmethyl)-1,3-dihydrofuro[3,4-c]quinoline-8-carboxamide;
4-amino-7-fluoro-N-methyl-N-((5-(trifluoromethyl)-2-pyridinyl)methyl)-1,3-dihydrofuro[3,4-c] quinoline-8-carboxamide;
4-amino-N-((5 -cyano-2-pyridinyl)methyl)-N-cyclopropyl-7-fluoro- 1,3 -dihydrofuro [3,4-c] quinoline-8-carboxamide;
4-amino-N-((5-cyano-2-pyridinyl)methyl)-7-fluoro-N-(2-propanyl)-1,3-dihydrofuro [3,4-c] quinoline-8-carboxamide;
4-amino-7-chloro-N-((5-cyclopropyl-2-pyridinyl)methyl)-N-methyl-1,3-dihydrofuro[3,4-c]quinoline-8-carboxamide;
4-amino-N-((5-cyano-2-pyridinyl)methyl)-7-fluoro-N-methyl-1,3-dihydrofuro [3,4-c] quinoline-8-carboxamide;
4-amino-7-chloro-N-cyclopropyl-N-((5 -cyclopropyl-2-pyridinyl)methyl)- 1,3 -dihydrofuro [3,4-c]quinoline-8-carboxamide;
(3R)-4-amino-3-methyl-N-(2,2,2-trifluoroethyl)-N-((5-(trifluoromethyl)-2-pyridinyl)methyl)-1,3-dihydrofuro[3,4-c]quinoline-8-carboxamide;
(3R)-4-amino-N-(bicyclo[1.1.1]pentan-1-yl)-N-((6-ethoxy-3-pyridazinyl)methyl)-3-methyl-1,3-dihydrofuro[3,4-c]quinoline-8-carboxamide;
4-amino-N-(2,2,2-trifluoroethyl)-N-((5-(trifluoromethyl)-2-pyridinyl)methyl)-1,3-dihydrofuro[3,4-c]quinoline-8-carboxamide;
4-amino-7-chloro-N-(2-propanyl)-N-((5-(trifluoromethyl)-2-pyridinyl)methyl)-1,3-dihydrofuro[3,4-c]quinoline-8-carboxamide;
(3R)-4-amino-N,3-dimethyl-N-((6-(trifluoromethyl)-3-pyridazinyl)methyl)-1,3-dihydrofuro[3,4-c]quinoline-8-carboxamide;
4-amino-N-(2,2,2-trifluoroethyl)-N-((5-(trifluoromethyl)-2-pyridinyl)methyl)-1,3-dihydrofuro[3,4-c][1,7]naphthyridine-8-carboxamide;
4-amino-7-chloro-N-((2R)-1-methoxy-2-propanyl)-N-((6-(trifluoromethyl)-3-pyridazinyl)methyl)-1,3-dihydrofuro[3,4-c]quinoline-8-carboxamide;
4-amino-7-chloro-N-((2S)-1-methoxy-2-propanyl)-N-((6-(trifluoromethyl)-3-pyridazinyl)methyl)-1,3-dihydrofuro[3,4-c]quinoline-8-carboxamide;
4-amino-7-fluoro-N-((2R)-1-methoxy-2-propanyl)-N-((6-(trifluoromethyl)-3-pyridazinyl)methyl)-1,3-dihydrofuro[3,4-c]quinoline-8-carboxamide;
4-amino-7-fluoro-N-((2S)-1-methoxy-2-propanyl)-N-((6-(trifluoromethyl)-3-pyridazinyl)methyl)-1,3-dihydrofuro[3,4-c]quinoline-8-carboxamide;
(3R)-4-amino-3-methyl-N-(tetrahydro-2H-pyran-4-yl)-N-((5-(trifluoromethyl)-2-pyridinyl)methyl)-1,3-dihydrofuro[3,4-c]quinoline-8-carboxamide;
4-amino-7-chloro-N-cyclopropyl-N-((6-(trifluoromethyl)-3-pyridazinyl)methyl)-1,3-dihydrofuro[3,4-c]quinoline-8-carboxamide;
6-amino-N-(1-methyl-1H-pyrazol-4-yl)-N-((5-(trifluoromethyl)-2-pyridinyl)methyl)-8,9-dihydro-7H-cyclopenta[c][1,7]naphthyridine-2-carboxamide;
6-amino-N-((5-(difluoromethyl)-2-pyridinyl)methyl)-N-(1-methyl-1H-pyrazol-4-yl)-8,9-dihydro-7H-cyclopenta[c][1,7]naphthyridine-2-carboxamide;
4-amino-3-methyl-N-(1-methylcyclopropyl)-N-((5-(trifluoromethyl)-2-pyridinyl)methyl)-3H-pyrazolo[3,4-c]quinoline-8-carboxamide;
4-amino-3-methyl-N-(1-methyl-1H-pyrazol-4-yl)-N-((5-(trifluoromethyl)-2-pyridinyl)methyl)-3H-pyrazolo[3,4-c]quinoline-8-carboxamide;
4-amino-7-fluoro-N-(2-propanyl)-N-((5-(trifluoromethyl)-2-pyrazinyl)methyl)-1,3-dihydrofuro[3,4-c]quinoline-8-carboxamide;
4-amino-N-ethyl-7-fluoro-N-((5-(trifluoromethyl)-2-pyrazinyl)methyl)-1,3-dihydrofuro[3,4-c]quinoline-8-carboxamide;
4-amino-7-chloro-N-ethyl-N-((5-(trifluoromethyl)-2-pyrazinyl)methyl)-1,3-dihydrofuro[3,4-c]quinoline-8-carboxamide;
4-amino-7-chloro-N-(2-propanyl)-N-((5-(trifluoromethyl)-2-pyrazinyl)methyl)-1,3-dihydrofuro[3,4-c]quinoline-8-carboxamide;
(3R)-4-amino-N-ethyl-3-methyl-N-((5-(trifluoromethyl)-2-pyrazinyl)methyl)-1,3-dihydrofuro[3,4-c]quinoline-8-carboxamide;
4-amino-N-cyclobutyl-7-fluoro-N-((6-(trifluoromethyl)-3-pyridazinyl)methyl)-1,3-dihydrofuro[3,4-c]quinoline-8-carboxamide;
4-amino-7-methoxy-N-methyl-N-((5-(trifluoromethyl)-2-pyridinyl)methyl)-1,3-dihydrofuro[3,4-c]quinoline-8-carboxamide;
4-amino-N, 7-dimethyl-N-((5-(trifluoromethyl)-2-pyridinyl)methyl)-1,3-dihydrofuro [3,4-c] quinoline-8-carboxamide;
4-amino-N-cyclobutyl-7-fluoro-3-methyl-N-((6-(trifluoromethyl)-3-pyridazinyl)methyl)-3H-pyrazolo[3,4-c]quinoline-8-carboxamide;
4-amino-7-chloro-N-cyclobutyl-N-((6-(trifluoromethyl)-3 -pyridazinyl)methyl)- 1,3 -dihydrofuro [3,4-c]quinoline-8-carboxamide;
4-amino-7-fluoro-3-methyl-N-(1-methyl-1H-pyrazol-4-yl)-N-((5-(trifluoromethyl)-2-pyridinyl)methyl)-3H-pyrazolo[3,4-c]quinoline-8-carboxamide;
4-amino-1-methyl-N-(1-methyl-1H-pyrazol-4-yl)-N-((5-(trifluoromethyl)-2-pyridinyl)methyl)-1H-pyrazolo[4,3-c] [1,7]naphthyridine-8-carboxamide;
4-amino-N-cyclopropyl-7-fluoro-3-methyl-N-((1R)-1-(5-(trifluoromethyl)-2-pyridinyl)ethyl)-3H-pyrazolo[3,4-c]quinoline-8-carboxamide;
4-amino-N-cyclobutyl-N-((6-(difluoromethoxy)-3-pyridazinyl)methyl)-7-fluoro-1,3-dihydrofuro[3,4-c]quinoline-8-carboxamide;
4-amino-N-methyl-N-(4-(pentafluoro-lambda~6~-sulfanyl)benzyl)-1,3-dihydrofuro[3,4-c]quinoline-8-carboxamide;
4-amino-N-methyl-N-(4-(pentafluoro-lambda~6~-sulfanyl)benzyl)-1,3-dihydrofuro[3,4-c][1,7]naphthyridine-8-carboxamide;
4-amino-7-fluoro-N-methyl-N-(4-(pentafluoro-lambda~6~-sulfanyl)benzyl)-1,3-dihydrofuro[3,4-c]quinoline-8-carboxamide;
4-amino-7-chloro-N-methyl-N-(4-(pentafluoro-lambda~6~-sulfanyl)benzyl)-1,3-dihydrofuro[3,4-c]quinoline-8-carboxamide;
(3R)-4-amino-N,3-dimethyl-N-(4-(pentafluoro-lambda~6~-sulfanyl)benzyl)-1,3-dihydrofuro[3,4-c]quinoline-8-carboxamide;
4-amino-7-fluoro-N,3-dimethyl-N-(4-(pentafluoro-lambda~6~-sulfanyl)benzyl)-3H-pyrazolo[3,4-c]quinoline-8-carboxamide;
4-amino-N-((5-fluoro-2-pyridinyl)methyl)-N,1,7-trimethyl-1H-pyrazolo[4,3-c]quinoline-8-carboxamide;
4-amino-N-((6-cyclopropyl-3-pyridazinyl)methyl)-7-fluoro-N,3-dimethyl-3 H-pyrazolo [3,4-c] quinoline-8-carboxamide;
4-amino-N-((6-cyclopropyl-3-pyridazinyl)methyl)-7-fluoro-N,1-dimethyl-1H-pyrazolo [4,3-c] quinoline-8-carboxamide;
4-amino-7-chloro-N-((6-cyclopropyl-3-pyridazinyl)methyl)-N-methyl-1,3-dihydrofuro[3,4-c]quinoline-8-carboxamide;
4-amino-N,3,7-trimethyl-N-((5-(trifluoromethyl)-2-pyridinyl)methyl)-3H-pyrazolo[3,4-c]quinoline-8-carboxamide;
4-amino-N,1,7-trimethyl-N-((5-(trifluoromethyl)-2-pyridinyl)methyl)-1H-pyrazolo[4,3-c]quinoline-8-carboxamide;
4-amino-N-((5-cyclopropyl-2-pyridinyl)methyl)-7-fluoro-3-methyl-N-(2-propanyl)-3H-pyrazolo[3,4-c]quinoline-8-carboxamide;
4-amino-N-((5-chloro-2-pyridinyl)methyl)-N,1-dimethyl-1H-pyrazolo[4,3-c]quinoline-8-carboxamide;
4-amino-N-((5-(difluoromethyl)-2-pyridinyl)methyl)-N,1-dimethyl-1H-pyrazolo[4,3-c]quinoline-8-carboxamide;
4-amino-N-((5-(difluoromethyl)-2-pyridinyl)methyl)-7-fluoro-N,1-dimethyl-1H-pyrazolo[4,3-c]quinoline-8-carboxamide;
4-amino-N-(3-fluoro-4-(trifluoromethyl)benzyl)-N,1-dimethyl-1H-pyrazolo[4,3-c]quinoline-8-carboxamide;
4-amino-N-((3-fluoro-5-(trifluoromethyl)-2-pyridinyl)methyl)-N,1-dimethyl-1H-pyrazolo[4,3-c]quinoline-8-carboxamide;
4-amino-7-fluoro-N-((3-fluoro-5-(trifluoromethyl)-2-pyridinyl)methyl)-N-methyl-1,3-dihydrofuro[3,4-c]quinoline-8-carboxamide;
4-amino-N,1-dimethyl-N-((6-(1-(trifluoromethyl)-1H-pyrazol-4-yl)-3-pyridazinyl)methyl)-1H-pyrazolo[4,3-c]quinoline-8-carboxamide;
4-amino-N,1-dimethyl-N-((6-(4-(trifluoromethyl)phenyl)-3-pyridazinyl)methyl)-1H-pyrazolo[4,3-c]quinoline-8-carboxamide;
4-amino-N,1-dimethyl-N-((1R)-1-(4'-(trifluoromethyl)[biphenyl]-4-yl)ethyl)-1H-pyrazolo[4,3-c]quinoline-8-carboxamide;
4-amino-N,1-dimethyl-N-((1R)-1-(6-(4-(trifluoromethyl)phenyl)-3-pyridazinyl)ethyl)-1H-pyrazolo[4,3-c]quinoline-8-carboxamide;
4-amino-N,1-dimethyl-N-((1R)-1-(4'-(pentafluoro-lambda~6~-sulfanyl)[biphenyl]-4-yl)ethyl)-1H-pyrazolo[4,3-c]quinoline-8-carboxamide;
4-amino-N-(2-propanyl)-N-((5-(trifluoromethyl)-2-pyridinyl)methyl)-2,3-dihydrofuro[3,2-c][1,7]naphthyridine-8-carboxamide;
4-amino-N-(2-(5-chloro-2-pyridinyl)-2,2-difluoroethyl)-N,1-dimethyl-1H-pyrazolo [4,3-c] quinoline-8-carboxamide;
4-amino-7-fluoro-N-(2-propanyl)-N-((5-(trifluoromethyl)-2-pyridinyl)methyl)-2,3-dihydrofuro[3,2-c]quinoline-8-carboxamide;
4-amino-7-chloro-N-ethyl-N-((5-(trifluoromethyl)-2-pyridinyl)methyl)-1,3-dihydrofuro[3,4-c][1,8]naphthyridine-8-carboxamide;
4-amino-7-fluoro-1,3-dimethyl-N-(2-propanyl)-N-((5-(trifluoromethyl)-2-pyridinyl)methyl)-1H-pyrazolo[4,3-c]quinoline-8-carboxamide;
4-amino-N-((5-cyclopropyl-2-pyridinyl)methyl)-N-ethyl-1,3-dihydrofuro[3,4-c]quinoline-8-carboxamide;
4-amino-N-((5-cyclopropyl-2-pyridinyl)methyl)-N-ethyl-1-methyl-1H-pyrazolo[4,3-c]quinoline-8-carboxamide;
4-amino-N-((5-cyclopropyl-2-pyridinyl)methyl)-N-ethyl-1-methyl-1H-pyrazolo[4,3-c][1,7]naphthyridine-8-carboxamide;
(3R)-4-amino-N-((5-cyclopropyl-2-pyridinyl)methyl)-N-ethyl-3-methyl-1,3-dihydrofuro [3,4-c] quinoline-8-carboxamide;
4-amino-N,1-dimethyl-N-(4-(trifluoromethyl)benzyl)-1H-pyrazolo[4,3-c]quinoline-8-carboxamide;
4-amino-N,1-dimethyl-N-(4-(trifluoromethyl)benzyl)-1H-pyrazolo[4,3-c][1,7]naphthyridine-8-carboxamide;
4-amino-N-((5-cyclopropyl-2-pyridinyl)methyl)-N-ethyl-7-fluoro-1-methyl-1H-pyrazolo[4,3-c]quinoline-8-carboxamide;
4-amino-N-ethyl-1-methyl-N-((5-(trifluoromethyl)-2-pyridinyl)methyl)-1H-pyrazolo[4,3-c]quinoline-8-carboxamide;
4-amino-N-ethyl-3-methyl-N-((5-(trifluoromethyl)-2-pyridinyl)methyl)[1,2]oxazolo[4,5-c]quinoline-8-carboxamide;
4-amino-N-ethyl-1-methyl-N-((6-(trifluoromethyl)-3-pyridazinyl)methyl)-1H-pyrazolo[4,3-c]quinoline-8-carboxamide;
4-amino-N-ethyl-1-methyl-N-((5-(trifluoromethyl)-2-pyridinyl)methyl)-1H-pyrazolo[4,3-c][1,7]naphthyridine-8-carboxamide;
4-amino-N-ethyl-1-methyl-N-((6-(trifluoromethyl)-3-pyridazinyl)methyl)-1H-pyrazolo [4,3-c][1,7]naphthyridine-8-carboxamide;
(3R)-4-amino-N-ethyl-3-methyl-N-((6-(trifluoromethyl)-3-pyridazinyl)methyl)-1,3-dihydrofuro[3,4-c]quinoline-8-carboxamide;
4-amino-7-fluoro-N,1-dimethyl-N-(4-(trifluoromethyl)benzyl)-1H-pyrazolo[4,3-c]quinoline-8-carboxamide;
4-amino-N-ethyl-1,3-dimethyl-N-((5-(trifluoromethyl)-2-pyridinyl)methyl)-1H-pyrazolo[4,3-c][1,7]naphthyridine-8-carboxamide;
4-amino-N-ethyl-7-fluoro-1-methyl-N-((5-(trifluoromethyl)-2-pyridinyl)methyl)-1H-pyrazolo[4,3-c]quinoline-8-carboxamide;
4-amino-N-ethyl-7-fluoro-1-methyl-N-((6-(trifluoromethyl)-3-pyridazinyl)methyl)-1H-pyrazolo[4,3-c]quinoline-8-carboxamide;
(3R)-4-amino-N-ethyl-7-fluoro-3-methyl-N-((5-(trifluoromethyl)-2-pyridinyl)methyl)-1,3-dihydrofuro[3,4-c]quinoline-8-carboxamide;
(3S)-4-amino-N-ethyl-7-fluoro-3-methyl-N-((5-(trifluoromethyl)-2-pyridinyl)methyl)-1,3-dihydrofuro[3,4-c]quinoline-8-carboxamide;
4-amino-7-chloro-N,1-dimethyl-N-(4-(trifluoromethyl)benzyl)-1H-pyrazolo[4,3-c]quinoline-8-carboxamide;
(3R)-4-amino-N-ethyl-7-fluoro-3-methyl-N-((6-(trifluoromethyl)-3-pyridazinyl)methyl)-1,3-dihydrofuro[3,4-c]quinoline-8-carboxamide;
4-amino-N-ethyl-7-fluoro-1,3-dimethyl-N-((5-(trifluoromethyl)-2-pyridinyl)methyl)-1H-pyrazolo[4,3-c]quinoline-8-carboxamide;
4-amino-N,1-dimethyl-N-(4-(pentafluoroethyl)benzyl)-1H-pyrazolo[4,3-c]quinoline-8-carboxamide;
4-amino-N,1-dimethyl-N-(4-(pentafluoroethyl)benzyl)-1H-pyrazolo[4,3-c][1,7]naphthyridine-8-carboxamide;
4-amino-7-fluoro-N,1-dimethyl-N-(4-(pentafluoroethyl)benzyl)-1H-pyrazolo[4,3-c]quinoline-8-carboxamide;
4-amino-N-ethyl-1-methyl-N-(4-(pentafluoro-lambda~6~-sulfanyl)benzyl)-1H-pyrazolo[4,3-c]quinoline-8-carboxamide;
4-amino-N-ethyl-1-methyl-N-(4-(pentafluoro-lambda~6~-sulfanyl)benzyl)-1H-pyrazolo[4,3-c][1,7]naphthyridine-8-carboxamide;
4-amino-N-ethyl-7-fluoro-1-methyl-N-(4-(pentafluoro-lambda~6~-sulfanyl)benzyl)-1H-pyrazolo[4,3-c]quinoline-8-carboxamide;
4-amino-N,1-dimethyl-N-((5-(trifluoromethyl)-2-pyridinyl)methyl)-1H-pyrazolo[4,3-c]quinoline-8-carboxamide;
4-amino-N,1-dimethyl-N-((5-(trifluoromethyl)-2-pyridinyl)methyl)-1H-pyrazolo[4,3-c][1,7]naphthyridine-8-carboxamide;
4-amino-N-cyclopropyl-1-methyl-N-((5-(trifluoromethyl)-2-pyridinyl)methyl)-1H-pyrazolo[4,3-c]quinoline-8-carboxamide;
4-amino-N-cyclopropyl-7-fluoro-1-(2,2,2-trifluoroethyl)-N-((5-(trifluoromethyl)-2-pyridinyl)methyl)-1H-pyrazolo[4,3 -c]quinoline-8-carboxamide;
4-amino-7-fluoro-N,1-dimethyl-N-((6-(trifluoromethyl)-3-pyridazinyl)methyl)-1H-pyrazolo[4,3-c]quinoline-8-carboxamide;
4-amino-N-methyl-7-(trifluoromethyl)-N-((5-(trifluoromethyl)-2-pyridinyl)methyl)-1,3-dihydrofuro[3,4-c]quinoline-8-carboxamide;
4-amino-N,1-dimethyl-N-(4-(pentafluoro-lambda~6~-sulfanyl)benzyl)-1H-pyrazolo[4,3-c]quinoline-8-carboxamide;
4-amino-N,1-dimethyl-N-(4-(pentafluoro-lambda~6~-sulfanyl)benzyl)-1H-pyrazolo[4,3-c][1,7]naphthyridine-8-carboxamide;
4-amino-7-fluoro-N,1-dimethyl-N-(4-(pentafluoro-lambda~6~-sulfanyl)benzyl)-1H-pyrazolo[4,3-c]quinoline-8-carboxamide;
4-amino-7-fluoro-N-(2-hydroxy-4-(pentafluoro-lambda~6~-sulfanyl)benzyl)-N-methyl-1,3-dihydrofuro[3,4-c]quinoline-8-carboxamide;
4-amino-N-((5-cyano-2-pyridinyl)methyl)-N-((2R)-1-fluoro-2-propanyl)-1,3-dihydrofuro[3,4-c]quinoline-8-carboxamide;
4-amino-N-((5-cyano-2-pyridinyl)methyl)-N-((2S)-1-fluoro-2-propanyl)-1,3-dihydrofuro[3,4-c]quinoline-8-carboxamide;
(3R)-4-amino-3-(hydroxymethyl)-N-(2-methylpropyl)-N-((5-(trifluoromethyl)-2-pyridinyl)methyl)-1,3-dihydrofuro[3,4-c]quinoline-8-carboxamide;
(3S)-4-amino-3-(hydroxymethyl)-N-(2-methylpropyl)-N-((5-(trifluoromethyl)-2-pyridinyl)methyl)-1,3-dihydrofuro[3,4-c]quinoline-8-carboxamide;
4-amino-N-((1R)-2-cyano-1-cyclopropylethyl)-N-((5-cyano-2-pyridinyl)methyl)-1,3-dihydrofuro[3,4-c]quinoline-8-carboxamide;
4-amino-N-((1S)-2-cyano-1-cyclopropylethyl)-N-((5-cyano-2-pyridinyl)methyl)-1,3-dihydrofuro[3,4-c]quinoline-8-carboxamide;
4-amino-N-((5-cyano-6-methyl-2-pyridinyl)methyl)-N-((3R,4R)-4-methoxytetrahydro-2H-pyran-3-yl)-1,3-dihydrofuro[3,4-c]quinoline-8-carboxamide;
4-amino-N-((5-cyano-6-methyl-2-pyridinyl)methyl)-N-((3S,4S)-4-methoxytetrahydro-2H-pyran-3-yl)-1,3-dihydrofuro[3,4-c]quinoline-8-carboxamide;
4-amino-N-((3S,4R)-3-methoxytetrahydro-2H-pyran-4-yl)-N-((5-(trifluoromethyl)-2-pyridinyl)methyl)-1,3-dihydrofuro[3,4-c]quinoline-8-carboxamide;
4-amino-N-((3R,4S)-3-methoxytetrahydro-2H-pyran-4-yl)-N-((5-(trifluoromethyl)-2-pyridinyl)methyl)-1,3-dihydrofuro[3,4-c]quinoline-8-carboxamide;
4-amino-N-((3R,4R)-4-methoxytetrahydro-2H-pyran-3-yl)-N-((5-(trifluoromethyl)-2-pyridinyl)methyl)-1,3-dihydrofuro[3,4-c]quinoline-8-carboxamide;
4-amino-N-((3S,4S)-4-methoxytetrahydro-2H-pyran-3-yl)-N-((5-(trifluoromethyl)-2-pyridinyl)methyl)-1,3-dihydrofuro[3,4-c]quinoline-8-carboxamide;
4-amino-N-((5-cyano-6-methyl-2-pyridinyl)methyl)-N-((3R,4R)-4-methoxytetrahydro-2H-pyran-3-yl)-1,3-dihydrofuro[3,4-c]quinoline-8-carboxamide;
(3R)-4-amino-N-cyclopropyl-3-methyl-N-((1R)-1-(5-(trifluoromethyl)-2-pyridinyl)ethyl)-1,3-dihydrofuro[3,4-c][1,7]naphthyridine-8-carboxamide;
(3S)-4-amino-N-cyclopropyl-3-methyl-N-((1R)-1-(5-(trifluoromethyl)-2-pyridinyl)ethyl)-1,3-dihydrofuro[3,4-c][1,7]naphthyridine-8-carboxamide;
4-amino-N-((1S)-1-(5-fluoro-2-pyridinyl)ethyl)-N,1,7-trimethyl-1H-pyrazolo[4,3-c]quinoline-8-carboxamide;
4-amino-N-((1R)-1-(5-fluoro-2-pyridinyl)ethyl)-N,1,7-trimethyl-1H-pyrazolo[4,3-c]quinoline-8-carboxamide;
4-amino-7-chloro-N-((1R)-1-(5-fluoro-2-pyridinyl)ethyl)-N,1-dimethyl-1H-pyrazolo[4,3-c]quinoline-8-carboxamide;
4-amino-7-chloro-N-((1S)-1-(5-fluoro-2-pyridinyl)ethyl)-N,1-dimethyl-1H-pyrazolo[4,3-c]quinoline-8-carboxamide;
4-amino-7-fluoro-N-((1R)-1-(3-fluoro-5-(trifluoromethyl)-2-pyridinyl)ethyl)-N-methyl-1,3-dihydrofuro[3,4-c]quinoline-8-carboxamide;
(3R)-4-amino-N-ethyl-3-(fluoromethyl)-N-((5-(trifluoromethyl)-2-pyridinyl)methyl)-1,3-dihydrofuro[3,4-c]quinoline-8-carboxamide;
(3R)-4-amino-N-((5-bromo-2-pyridinyl)methyl)-N,3-dimethyl-1,3-dihydrofuro[3,4-c] [1,7]naphthyridine-8-carboxamide;
4-amino-N,1-dimethyl-N-((1R)-1-(4-(trifluoromethyl)phenyl)ethyl)-1H-pyrazolo[4,3-c][1,7]naphthyridine-8-carboxamide;
4-amino-N,1-dimethyl-N-((1S)-1-(4-(trifluoromethyl)phenyl)ethyl)-1H-pyrazolo[4,3-c][1,7]naphthyridine-8-carboxamide;
4-amino-N,1-dimethyl-N-((1R)-1-(5-(trifluoromethyl)-2-pyridinyl)ethyl)-1H-pyrazolo[4,3-c]quinoline-8-carboxamide;
4-amino-N,1-dimethyl-N-((1S)-1-(5-(trifluoromethyl)-2-pyridinyl)ethyl)-1H-pyrazolo[4,3-c]quinoline-8-carboxamide;
4-amino-7-fluoro-N,1-dimethyl-N-((1R)-1-(5-(trifluoromethyl)-2-pyridinyl)ethyl)-1H-pyrazolo[4,3-c]quinoline-8-carboxamide;
4-amino-7-fluoro-N,1-dimethyl-N-((1S)-1-(5-(trifluoromethyl)-2-pyridinyl)ethyl)-1H-pyrazolo[4,3-c]quinoline-8-carboxamide;
4-amino-N-((1R)-1-(5-fluoro-2-pyridinyl)ethyl)-N,1-dimethyl-1H-pyrazolo[4,3-c]quinoline-8-carboxamide;
4-amino-7-fluoro-N,1-dimethyl-N-((1R)-1-(6-(trifluoromethyl)-3-pyridazinyl)ethyl)-1H-pyrazolo[4,3-c]quinoline-8-carboxamide;
4-amino-N-((5-cyano-2-pyridinyl)methyl)-N-((1R)-1-(2-pyrimidinyl)ethyl)-1,3-dihydrofuro[3,4-c][1,7]naphthyridine-8-carboxamide;
6-amino-N-(2-methylpropyl)-N-((5-(trifluoromethyl)-2-pyridinyl)methyl)-8,9-dihydro-7H-cyclopenta[c][1,8]naphthyridine-2-carboxamide;
4-amino-N-(cyclopropylmethyl)-N-((5-(trifluoromethyl)-2-pyridinyl)methyl)-1,3-dihydrofuro[3,4-c][1,8]naphthyridine-8-carboxamide;
4-amino-N-((2R)-1-methoxy-2-propanyl)-N-((5-(trifluoromethyl)-2-pyridinyl)methyl)-1,3-dihydrofuro[3,4-c][1,8]naphthyridine-8-carboxamide;
4-amino-N-((1R)-1-(2-pyrimidinyl)ethyl)-N-((5-(trifluoromethyl)-2-pyridinyl)methyl)-2,3-dihydro-1H-cyclopenta[c]quinoline-8-carboxamide;
4-amino-N-(2-methylpropyl)-N-((5-(trifluoromethyl)-2-pyridinyl)methyl)-2,3-dihydro-1H-cyclopenta[c]quinoline-8-carboxamide;
4-amino-N-(2-methylpropyl)-N-((5-(trifluoromethyl)-2-pyridinyl)methyl)-1,3-dihydrofuro[3,4-c][1,8]naphthyridine-8-carboxamide;
4-amino-N-(2-methylpropyl)-N-((5-(trifluoromethyl)-2-pyridinyl)methyl)-1,3-dihydrofuro[3,4-c]quinoline-8-carboxamide;
4-amino-N-((1R)-1-(2-pyrimidinyl)ethyl)-N-((5-(trifluoromethyl)-2-pyridinyl)methyl)-1,3-dihydrofuro[3,4-c][1,8]naphthyridine-8-carboxamide;
4-amino-N-((5-cyano-2-pyridinyl)methyl)-N-((1R)-1-(2-pyrimidinyl)ethyl)-2,3-dihydro-1H-cyclopenta[c]quinoline-8-carboxamide;
4-amino-N-((5-cyano-2-pyridinyl)methyl)-N-((1R)-1-(2-pyrimidinyl)ethyl)-1,3-dihydrofuro[3,4-c]quinoline-8-carboxamide;
4-amino-N-((5-cyano-2-pyridinyl)methyl)-N-((1S)-1-(2-pyrimidinyl)ethyl)-1,3-dihydrofuro[3,4-c]quinoline-8-carboxamide;
4-amino-3-methyl-N-(2-methylpropyl)-N-((5-(trifluoromethyl)-2-pyridinyl)methyl)[1,2]oxazolo[4,5-c]quinoline-8-carboxamide;
4-amino-3-methyl-N-((1R)-1-(2-pyrimidinyl)ethyl)-N-((5-(trifluoromethyl)-2-pyridinyl)methyl)-3H-pyrazolo[3,4-c]quinoline-8-carboxamide;
4-amino-3-methyl-N-((1S)-1-(2-pyrimidinyl)ethyl)-N-((5-(trifluoromethyl)-2-pyridinyl)methyl)-3H-pyrazolo[3,4-c]quinoline-8-carboxamide;
4-amino-3-methyl-N-((1R)-1-(2-pyrimidinyl)ethyl)-N-((5-(trifluoromethyl)-2-pyridinyl)methyl)[1,2]oxazolo[4,5-c]quinoline-8-carboxamide;
4-amino-3-methyl-N-((1S)-1-(2-pyrimidinyl)ethyl)-N-((5-(trifluoromethyl)-2-pyridinyl)methyl)[1,2]oxazolo[4,5-c]quinoline-8-carboxamide;
4-amino-N-(2-methylpropyl)-N-((5-(trifluoromethyl)-2-pyridinyl)methyl)-1,3-dihydrofuro[3,4-c] [1, 7]naphthyridine-8-carboxamide;
4-amino-N-((1R)-1-(2-pyrimidinyl)ethyl)-N-((5-(trifluoromethyl)-2-pyridinyl)methyl)-1,3-dihydrofuro[3,4-c][1,7]naphthyridine-8-carboxamide;
4-amino-N-((5-chloro-2-pyridinyl)methyl)-N-((1R)-1-(2-pyrimidinyl)ethyl)-2,3-dihydro-1H-cyclopenta[c]quinoline-8-carboxamide;
4-amino-N-((5-(difluoromethyl)-2-pyridinyl)methyl)-N-((1R)-1-(2-pyrimidinyl)ethyl)-2,3-dihydro-1H-cyclopenta[c]quinoline-8-carboxamide;
4-amino-N-((6-bromo-3-pyridazinyl)methyl)-N-((1R)-1-(3-fluoro-2-pyridinyl)ethyl)-2,3-dihydro-1H-cyclopenta[c]quinoline-8-carboxamide;
6-amino-N-((5-(difluoromethyl)-2-pyridinyl)methyl)-N-((1R)-1-(2-pyrimidinyl)ethyl)-8,9-dihydro-7H-cyclopenta[c][1,8]naphthyridine-2-carboxamide;
6-amino-N-((1R)-1-(2-pyrimidinyl)ethyl)-N-((5-(trifluoromethyl)-2-pyridinyl)methyl)-8,9-dihydro-7H-cyclopenta[c][1,8]naphthyridine-2-carboxamide;
6-amino-N-((5-chloro-2-pyridinyl)methyl)-N-((1R)-1-(2-pyrimidinyl)ethyl)-8,9-dihydro-7H-cyclopenta[c][1,8]naphthyridine-2-carboxamide;
6-amino-N-((1R)-1-(3-fluoro-2-pyridinyl)ethyl)-N-((6-(trifluoromethyl)-3-pyridazinyl)methyl)-8,9-dihydro-7H-cyclopenta[c] [1,8]naphthyridine-2-carboxamide;
6-amino-N-((1R)-1-(2-pyrimidinyl)ethyl)-N-((5-(trifluoromethyl)-2-pyridinyl)methyl)-2-phenanthridinecarboxamide;
6-amino-N-((5-cyano-2-pyridinyl)methyl)-N-((1R)-1-(2-pyrimidinyl)ethyl)-2-phenanthridinecarboxamide;
5-amino-N-(2-pyrimidinylmethyl)-N-((5-(trifluoromethyl)-2-pyridinyl)methyl)benzo[c][2,6]naphthyridine-9-carboxamide;
5-amino-N-(2-methylpropyl)-N-((5-(trifluoromethyl)-2-pyridinyl)methyl)benzo[c] [2,6]naphthyridine-9-carboxamide;
5-amino-N-((1R)-1-(2-pyrimidinyl)ethyl)-N-((5-(trifluoromethyl)-2-pyridinyl)methyl)benzo[c][2,6]naphthyridine-9-carboxamide;
4-amino-N-((2R)-1-methoxy-2-propanyl)-3-methyl-N-((5-(trifluoromethyl)-2-pyridinyl)methyl)[1,2]oxazolo[4,5-c]quinoline-8-carboxamide;
4-amino-N-((2S)-1-methoxy-2-propanyl)-3-methyl-N-((5-(trifluoromethyl)-2-pyridinyl)methyl)[1,2]oxazolo[4,5-c]quinoline-8-carboxamide;
4-amino-N-((3-fluoro-2-pyridinyl)methyl)-N-((5-(trifluoromethyl)-2-pyridinyl)methyl)thieno[2,3-c]quinoline-8-carboxamide;
4-amino-N-(2-pyrimidinylmethyl)-N-((5 -(trifluoromethyl)-2-pyridinyl)methyl)thieno [2,3 -c] quinoline-8-carboxamide;
4-amino-N-(2-methylpropyl)-N-((5-(trifluoromethyl)-2-pyridinyl)methyl)thieno[2,3-c]quinoline-8-carboxamide;
5-amino-N-((1R)-1-(2-pyrimidinyl)ethyl)-N-((5-(trifluoromethyl)-2-pyridinyl)methyl)pyrimido[4,5-c]quinoline-9-carboxamide;
4-amino-N-((6-bromo-3-pyridazinyl)methyl)-N-((1R)-1-cyclopropyl-2-methoxyethyl)thieno[2,3-c]quinoline-8-carboxamide;
4-amino-N-((6-bromo-3-pyridazinyl)methyl)-N-((1S)-1-cyclopropyl-2-methoxyethyl)thieno[2,3-c]quinoline-8-carboxamide;
4-amino-N-((1R)-1-(2-pyrimidinyl)ethyl)-N-((5-(trifluoromethyl)-2-pyridinyl)methyl)-1,3-dihydrofuro[3,4-c]quinoline-8-carboxamide;
4-amino-N-((1S)-1-(2-pyrimidinyl)ethyl)-N-((5-(trifluoromethyl)-2-pyridinyl)methyl)-1,3-dihydrofuro[3,4-c]quinoline-8-carboxamide;
4-amino-N-((1S)-1-cyclopropyl-2-methoxyethyl)-N-((6-(4-morpholinyl)-3-pyridazinyl)methyl)-1,3-dihydrofuro[3,4-c]quinoline-8-carboxamide;
4-amino-N-((6-ethoxy-3-pyridazinyl)methyl)-N-(2-methylpropyl)-1,3-dihydrofuro[3,4-c]quinoline-8-carboxamide;
4-amino-N-((6-bromo-3-pyridazinyl)methyl)-N-((2R)-1-methoxy-2-propanyl)thieno[2,3-c]quinoline-8-carboxamide;
4-amino-N-((6-bromo-3-pyridazinyl)methyl)-N-(1-methoxy-2-methyl-2-propanyl)-1,3-dihydrofuro[3,4-c]quinoline-8-carboxamide;
4-amino-N-((6-bromo-3-pyridazinyl)methyl)-N-(1-methoxy-2-methyl-2-propanyl)-1,3-dihydrofuro[3,4-c][1,7]naphthyridine-8-carboxamide;
4-amino-N-((6-methoxy-3-pyridazinyl)methyl)-N-((1R)-1-(2-pyrimidinyl)ethyl)-2,3-dihydro-1H-cyclopenta[c]quinoline-8-carboxamide;
4-amino-N-((6-methoxy-3-pyridazinyl)methyl)-N-((1S)-1-(2-pyrimidinyl)ethyl)-2,3-dihydro-1H-cyclopenta[c]quinoline-8-carboxamide;
4-amino-N-((1S)-1-(2-pyrimidinyl)ethyl)-N-((6-(trifluoromethyl)-3-pyridazinyl)methyl)-2,3-dihydro-1H-cyclopenta[c]quinoline-8-carboxamide;
4-amino-N-((1R)-1-(2-pyrimidinyl)ethyl)-N-((6-(trifluoromethyl)-3-pyridazinyl)methyl)-2,3-dihydro-1H-cyclopenta[c]quinoline-8-carboxamide;
4-amino-N-((6-bromo-3-pyridazinyl)methyl)-N-((1S)-1-cyclopropyl-2-methoxyethyl)thieno[2,3-c]quinoline-8-carboxamide;
4-amino-N-((6-bromo-3-pyridazinyl)methyl)-N-((1S)-1-cyclopropyl-2-methoxyethyl)-1,3-dihydrofuro[3,4-c][1,8]naphthyridine-8-carboxamide;
4-amino-N-(cyclopropylmethyl)-N-((5-(trifluoromethyl)-2-pyridinyl)methyl)-1,3-dihydrofuro[3,4-c][1,7]naphthyridine-8-carboxamide;
4-amino-N-((1R)-1-(2-pyrimidinyl)ethyl)-N-((5-(trifluoromethyl)-2-pyridinyl)methyl)-1,3-dihydrofuro[3,4-c]quinoline-8-carboxamide;
6-amino-N-((1R)-1-(2-pyrimidinyl)ethyl)-N-((5-(trifluoromethyl)-2-pyridinyl)methyl)-7,8,9,10-tetrahydro-2-phenanthridinecarboxamide;
4-amino-7-chloro-N-((1R)-1-(2-pyrimidinyl)ethyl)-N-((5-(trifluoromethyl)-2-pyridinyl)methyl)-1,3-dihydrofuro[3,4-c]quinoline-8-carboxamide;
4-amino-7-chloro-N-cyclopropyl-N-((5-(trifluoromethyl)-2-pyridinyl)methyl)-1,3-dihydrofuro[3,4-c]quinoline-8-carboxamide;
4-amino-N-((6-bromo-3-pyridazinyl)methyl)-N-((1R)-1-(2-pyrimidinyl)ethyl)-1,3-dihydrofuro[3,4-c]quinoline-8-carboxamide;
4-amino-N-((5-bromo-2-pyridinyl)methyl)-N-((1R)-1-(2-pyrimidinyl)ethyl)-1,3-dihydrofuro[3,4-c]quinoline-8-carboxamide;
4-amino-N-((5-cyano-2-pyridinyl)methyl)-N-((1R)-1-(2-pyrimidinyl)ethyl)-1,3-dihydrofuro[3,4-c]quinoline-8-carboxamide;
4-amino-N-(3,3-difluorocyclobutyl)-N-((6-(trifluoromethyl)-3-pyridazinyl)methyl)-1,3-dihydrofuro[3,4-c]quinoline-8-carboxamide;
4-amino-N-cyclopropyl-N-((6-(trifluoromethyl)-3-pyridazinyl)methyl)-1,3-dihydrofuro [3,4-c] quinoline-8-carboxamide;
4-amino-N-((5-(difluoromethyl)-2-pyridinyl)methyl)-N-((1R)-1-(1-methyl-1H-1,2,4-triazol-3-yl)ethyl)-1,3-dihydrofuro[3,4-c]quinoline-8-carboxamide;
4-amino-N-(cyclopropylmethyl)-N-((5-(trifluoromethoxy)-2-pyridinyl)methyl)-1,3-dihydrofuro[3,4-c]quinoline-8-carboxamide;
4-amino-N-(2-methylpropyl)-N-((6-(trifluoromethyl)-3-pyridazinyl)methyl)-1,3-dihydrofuro[3,4-c]quinoline-8-carboxamide;
4-amino-N-((6-bromo-3-pyridazinyl)methyl)-N-(2-methylpropyl)-1,3-dihydrofuro[3,4-c]quinoline-8-carboxamide;
4-amino-N-((5-cyano-2-pyridinyl)methyl)-N-((1S)-1-(2-pyrimidinyl)ethyl)-1,3-dihydrofuro[3,4-c]quinoline-8-carboxamide;
4-amino-N-((5-(difluoromethyl)-2-pyridinyl)methyl)-N-((1R)-1-(1-methyl-1H-1,2,4-triazol-3-yl)ethyl)-1,3-dihydrofuro[3,4-c][1,7]naphthyridine-8-carboxamide;
4-amino-N-(3,3-difluorocyclobutyl)-N-((6-(trifluoromethyl)-3-pyridazinyl)methyl)-1,3-dihydrofuro[3,4-c] [1, 7]naphthyridine-8-carboxamide;
4-amino-N-cyclobutyl-N-((6-(trifluoromethyl)-3 -pyridazinyl)methyl)- 1,3-dihydrofuro [3,4-c] quinoline-8-carboxamide;
4-amino-N-(2-methylpropyl)-N-((6-(trifluoromethyl)-3-pyridazinyl)methyl)-1,3-dihydrofuro[3,4-c][1,7]naphthyridine-8-carboxamide;
4-amino-N-((6-bromo-3-pyridazinyl)methyl)-N-(cyclopropylmethyl)-1,3-dihydrofuro[3,4-c][1,7]naphthyridine-8-carboxamide;
4-amino-N-((6-ethoxy-3-pyridazinyl)methyl)-N-(2-methylpropyl)-1,3-dihydrofuro[3,4-c][1,7]naphthyridine-8-carboxamide;
4-amino-N-((6-bromo-3-pyridazinyl)methyl)-N-((1R)-1-(2-pyrimidinyl)ethyl)-1,3-dihydrofuro[3,4-c][1,7]naphthyridine-8-carboxamide;
4-amino-N-((6-bromo-3-pyridazinyl)methyl)-N-(cyclopropylmethyl)-1,3-dihydrofuro [3,4-c] quinoline-8-carboxamide;
4-amino-N-(2-propanyl)-N-((5-(trifluoromethyl)-2-pyridinyl)methyl)-1,3-dihydrofuro[3,4-c][1,7]naphthyridine-8-carboxamide;
4-amino-N-((6-chloro-3-pyridazinyl)methyl)-N-((1R)-1-(2-pyrimidinyl)ethyl)-1,3-dihydrofuro[3,4-c]quinoline-8-carboxamide;
4-amino-N-((5-bromo-6-methyl-2-pyridinyl)methyl)-N-methyl-1,3-dihydrofuro[3,4-c]quinoline-8-carboxamide;
4-amino-N-(2-propanyl)-N-((5-(trifluoromethoxy)-2-pyridinyl)methyl)-1,3-dihydrofuro[3,4-c]quinoline-8-carboxamide;
4-amino-N-((6-chloro-5-cyano-2-pyridinyl)methyl)-N-((1R)-1-(2-pyrimidinyl)ethyl)-1,3-dihydrofuro[3,4-c]quinoline-8-carboxamide;
4-amino-N-((6-bromo-3-pyridazinyl)methyl)-N-((2R)-1-methoxy-2-propanyl)-1,3-dihydrofuro[3,4-c][1,7]naphthyridine-8-carboxamide;
4-amino-N-((6-bromo-3-pyridazinyl)methyl)-N-((2R)-1-methoxy-2-propanyl)-1,3-dihydrofuro[3,4-c][1,8]naphthyridine-8-carboxamide;
4-amino-N-((5-cyano-2-pyridinyl)methyl)-N-(cyclopropylmethyl)-1,3-dihydrofuro[3,4-c] [1, 7]naphthyridine-8-carboxamide;
4-amino-N-((6-chloro-5-methoxy-2-pyridinyl)methyl)-N-(2-propanyl)-1,3-dihydrofuro[3,4-c][1,7]naphthyridine-8-carboxamide;
4-amino-N-((6-bromo-3-pyridazinyl)methyl)-N-(2-propanyl)-1,3-dihydrofuro[3,4-c] [1,7]naphthyridine-8-carboxamide;
4-amino-N-((5-chloro-6-methoxy-2-pyridinyl)methyl)-N-(2-propanyl)-1,3-dihydrofuro[3,4-c][1,7]naphthyridine-8-carboxamide;
4-amino-N-(3,4-dihydro-2H-pyrano[2,3-c]pyridin-6-ylmethyl)-N-((1R)-1-(2-pyrimidinyl)ethyl)-1,3-dihydrofuro[3,4-c][1,7]naphthyridine-8-carboxamide;
4-amino-N-((5-cyano-2-pyridinyl)methyl)-N-((2R)-1-fluoro-2-propanyl)-1,3-dihydrofuro[3,4-c][1,7]naphthyridine-8-carboxamide;
4-amino-N-((5-cyano-2-pyridinyl)methyl)-N-((2S)-1-fluoro-2-propanyl)-1,3-dihydrofuro[3,4-c][1,7]naphthyridine-8-carboxamide;
4-amino-N-(cyclopropylmethyl)-N-((6-(trifluoromethyl)-3 -pyridazinyl)methyl)- 1,3 -dihydrofuro [3,4-c][1,7]naphthyridine-8-carboxamide;
4-amino-N-(bicyclo[1.1.1]pentan-1-yl)-N-((5-(trifluoromethyl)-2-pyridinyl)methyl)-1,3-dihydrofuro[3,4-c][1,7]naphthyridine-8-carboxamide;
4-amino-N-(1-cyclopropyl-1H-pyrazol-4-yl)-N-((5-(trifluoromethyl)-2-pyridinyl)methyl)-1,3-dihydrofuro[3,4-c][1,7]naphthyridine-8-carboxamide;
4-amino-N-((5-(difluoromethyl)-2-pyridinyl)methyl)-N-(1-methyl-1H-pyrazol-4-yl)-1,3-dihydrofuro[3,4-c][1,7]naphthyridine-8-carboxamide;
4-amino-N-methyl-N-(4-(trifluoromethyl)benzyl)-1,3-dihydrofuro[3,4-c][1,7]naphthyridine-8-carboxamide;
4-amino-N-methyl-N-((6-(trifluoromethyl)-3-pyridinyl)methyl)-1,3-dihydrofuro[3,4-c][1,7]naphthyridine-8-carboxamide;
4-amino-N-((5-chloro-2-pyridinyl)methyl)-N-methyl-1,3-dihydrofuro[3,4-c][1,7]naphthyridine-8-carboxamide;
4-amino-N-ethyl-N-((6-(trifluoromethyl)-3-pyridazinyl)methyl)-1,3-dihydrofuro[3,4-c]quinoline-8-carboxamide;
4-amino-N-ethyl-N-((6-(trifluoromethyl)-3-pyridazinyl)methyl)-1,3-dihydrofuro[3,4-c][1,7]naphthyridine-8-carboxamide;
4-amino-N-methyl-N-((1R)-1-(5-(trifluoromethyl)-2-pyridinyl)ethyl)-1,3-dihydrofuro[3,4-c]quinoline-8-carboxamide;
4-amino-N-methyl-N-((1S)-1-(5-(trifluoromethyl)-2-pyridinyl)ethyl)-1,3-dihydrofuro[3,4-c]quinoline-8-carboxamide;
4-amino-N-((5-chloro-2-pyridinyl)methyl)-N-ethyl-1,3-dihydrofuro[3,4-c] [1,7]naphthyridine-8-carboxamide;
4-amino-N-(1-methylcyclopropyl)-N-((5-(trifluoromethyl)-2-pyridinyl)methyl)-1,3-dihydrofuro[3,4-c][1,7]naphthyridine-8-carboxamide;
4-amino-N-methyl-N-((1R)-1-(5-(trifluoromethyl)-2-pyridinyl)ethyl)-1,3-dihydrofuro[3,4-c][1,8]naphthyridine-8-carboxamide;
4-amino-N-methyl-N-((1S)-1-(5-(trifluoromethyl)-2-pyridinyl)ethyl)-1,3-dihydrofuro[3,4-c][1,8]naphthyridine-8-carboxamide;
4-amino-N-((5-chloro-2-pyridinyl)methyl)-N-ethyl-1,3-dihydrofuro[3,4-c]quinoline-8-carboxamide;
4-amino-N-((5-chloro-2-pyridinyl)methyl)-N-methyl-1,3-dihydrofuro[3,4-c]quinoline-8-carboxamide;
4-amino-N-ethyl-N-((6-(trifluoromethyl)-3-pyridinyl)methyl)-1,3-dihydrofuro[3,4-c]quinoline-8-carboxamide;
4-amino-N-ethyl-N-((6-(trifluoromethyl)-3-pyridinyl)methyl)-1,3-dihydrofuro[3,4-c] [1,7]naphthyridine-8-carboxamide;
4-amino-N-ethyl-N-((1R)-1-(5-(trifluoromethyl)-2-pyridinyl)ethyl)-1,3-dihydrofuro[3,4-c][1,8]naphthyridine-8-carboxamide;
4-amino-N-ethyl-N-((1S)-1-(5-(trifluoromethyl)-2-pyridinyl)ethyl)-1,3-dihydrofuro[3,4-c][1,8]naphthyridine-8-carboxamide;
4-amino-N-ethyl-N-(4-(trifluoromethyl)benzyl)-1,3-dihydrofuro[3,4-c]quinoline-8-carboxamide;
4-amino-N-ethyl-N-(4-(trifluoromethyl)benzyl)-1,3-dihydrofuro[3,4-c][1,7]naphthyridine-8-carboxamide;
4-amino-N-((6-bromo-3-pyridazinyl)methyl)-N-ethyl-1,3-dihydrofuro[3,4-c]quinoline-8-carboxamide;
4-amino-N-methyl-N-((1R)-1-(5-(trifluoromethyl)-2-pyridinyl)ethyl)-1,3-dihydrofuro[3,4-c][1,7]naphthyridine-8-carboxamide;
4-amino-N-methyl-N-((1S)-1-(5-(trifluoromethyl)-2-pyridinyl)ethyl)-1,3-dihydrofuro[3,4-c][1,7]naphthyridine-8-carboxamide;
4-amino-N-((6-bromo-3-pyridazinyl)methyl)-N-ethyl-1,3-dihydrofuro[3,4-c] [1,7]naphthyridine-8-carboxamide;
4-amino-N-(1-methyl-1H-pyrazol-4-yl)-N-((6-(trifluoromethyl)-3-pyridazinyl)methyl)-1,3-dihydrofuro[3,4-c][1,7]naphthyridine-8-carboxamide;
4-amino-N-(2,2,2-trifluoroethyl)-N-((6-(trifluoromethyl)-3-pyridazinyl)methyl)-1,3-dihydrofuro[3,4-c][1,7]naphthyridine-8-carboxamide;
4-amino-N-(3-oxetanyl)-N-((6-(trifluoromethyl)-3-pyridazinyl)methyl)-1,3-dihydrofuro [3,4-c] quinoline-8-carboxamide;
4-amino-7-fluoro-N-(1-methyl-1H-pyrazol-4-yl)-N-((5-(trifluoromethyl)-2-pyridinyl)methyl)-1,3-dihydrofuro[3,4-c]quinoline-8-carboxamide;
4-amino-7-chloro-N-(1-methyl-1H-pyrazol-4-yl)-N-((5-(trifluoromethyl)-2-pyridinyl)methyl)-1,3-dihydrofuro[3,4-c]quinoline-8-carboxamide;
4-amino-N,3-dimethyl-N-((1R)-1-(5-(trifluoromethyl)-2-pyridinyl)ethyl)-3H-pyrazolo[3,4-c]quinoline-8-carboxamide;
4-amino-N,3-dimethyl-N-((1S)-1-(5-(trifluoromethyl)-2-pyridinyl)ethyl)-3H-pyrazolo[3,4-c]quinoline-8-carboxamide;
4-amino-N-((5-chloro-2-pyridinyl)methyl)-N-(2-propanyl)-1,3-dihydrofuro[3,4-c]quinoline-8-carboxamide;
4-amino-N-((5-chloro-2-pyridinyl)methyl)-N-(2-propanyl)-1,3-dihydrofuro[3,4-c] [1,7]naphthyridine-8-carboxamide;
4-amino-N-ethyl-7-fluoro-N-((1R)-1-(5-(trifluoromethyl)-2-pyridinyl)ethyl)-1,3-dihydrofuro[3,4-c]quinoline-8-carboxamide;
4-amino-N-ethyl-7-fluoro-N-((1 S)-1-(5-(trifluoromethyl)-2-pyridinyl)ethyl)-1,3-dihydrofuro[3,4-c]quinoline-8-carboxamide;
(3R)-4-amino-N-ethyl-3-methyl-N-((1-methyl-1H-1,2,4-triazol-3-yl)methyl)-1,3-dihydrofuro[3,4-c]quinoline-8-carboxamide;
4-amino-N-cyclobutyl-N-((6-(trifluoromethyl)-3-pyridazinyl)methyl)-1,3-dihydrofuro[3,4-c] [1, 7]naphthyridine-8-carboxamide;
(3R)-4-amino-N-cyclobutyl-3-methyl-N-((6-(trifluoromethyl)-3-pyridazinyl)methyl)-1,3-dihydrofuro[3,4-c]quinoline-8-carboxamide;
4-amino-N-((6-(difluoromethoxy)-3-pyridazinyl)methyl)-N-ethyl-1,3-dihydrofuro[3,4-c]quinoline-8-carboxamide;
4-amino-N-((6-(difluoromethoxy)-3-pyridazinyl)methyl)-N-ethyl-7-fluoro-1,3-dihydrofuro[3,4-c]quinoline-8-carboxamide;
4-amino-7-fluoro-N-((1R)-1-(5-fluoro-2-pyrimidinyl)ethyl)-N-(2-(trifluoromethoxy)ethyl)-1,3-dihydrofuro[3,4-c]quinoline-8-carboxamide;
4-amino-7-fluoro-N-((1S)-1-(5-fluoro-2-pyrimidinyl)ethyl)-N-(2-(trifluoromethoxy)ethyl)-1,3-dihydrofuro[3,4-c]quinoline-8-carboxamide;
(3R)-4-amino-N-((6-(difluoromethoxy)-3-pyridazinyl)methyl)-N-ethyl-3-methyl-1,3-dihydrofuro[3,4-c]quinoline-8-carboxamide;
4-amino-N-((1R)-1-(6-chloro-3-pyridinyl)ethyl)-N-ethyl-1,3-dihydrofuro[3,4-c]quinoline-8-carboxamide;
4-amino-N-((1S)-1-(6-chloro-3-pyridinyl)ethyl)-N-ethyl-1,3-dihydrofuro[3,4-c]quinoline-8-carboxamide;
4-amino-N-((2R)-1-methoxy-2-propanyl)-N-((5-(trifluoromethyl)-2-pyridinyl)methyl)-1,3-dihydrofuro[3,4-c][1,7]naphthyridine-8-carboxamide;
4-amino-7-fluoro-N-(1-(trifluoromethyl)-1H-pyrazol-4-yl)-N-((5-(trifluoromethyl)-2-pyridinyl)methyl)-1,3-dihydrofuro[3,4-c]quinoline-8-carboxamide;
4-amino-N-(cyclopropylmethyl)-7-fluoro-N-((6-(trifluoromethyl)-3-pyridazinyl)methyl)-1,3-dihydrofuro[3,4-c]quinoline-8-carboxamide;
4-amino-N-(cyclopropylmethyl)-7-fluoro-3-methyl-N-((6-(trifluoromethyl)-3-pyridazinyl)methyl)-3H-pyrazolo[3,4-c]quinoline-8-carboxamide;
4-amino-N-(1-(trifluoromethyl)-1H-pyrazol-4-yl)-N-((5-(trifluoromethyl)-2-pyridinyl)methyl)-1,3-dihydrofuro[3,4-c][1,7]naphthyridine-8-carboxamide;
4-amino-N-(2-propanyl)-N-(4-(trifluoromethyl)benzyl)-1,3-dihydrofuro[3,4-c][1,7]naphthyridine-8-carboxamide;
4-amino-N-cyclopropyl-N-(4-(trifluoromethyl)benzyl)-1,3-dihydrofuro[3,4-c][1,7]naphthyridine-8-carboxamide;
4-amino-N-cyclopropyl-N-((1S)-1-(5-(trifluoromethyl)-2-pyridinyl)ethyl)-1,3-dihydrofuro[3,4-c]quinoline-8-carboxamide;
4-amino-N-cyclopropyl-N-((1R)-1-(5-(trifluoromethyl)-2-pyridinyl)ethyl)-1,3-dihydrofuro[3,4-c]quinoline-8-carboxamide;
4-amino-7-chloro-N-((1R)-1-(6-chloro-3-pyridinyl)ethyl)-N-ethyl-1,3-dihydrofuro[3,4-c]quinoline-8-carboxamide;
4-amino-7-chloro-N-((1S)-1-(6-chloro-3-pyridinyl)ethyl)-N-ethyl-1,3-dihydrofuro[3,4-c]quinoline-8-carboxamide;
4-amino-7-chloro-N-ethyl-N-((1R)-1-(5-(trifluoromethyl)-2-pyridinyl)ethyl)-1,3-dihydrofuro[3,4-c]quinoline-8-carboxamide;
4-amino-N-((1R)-1-(6-chloro-3-pyridinyl)ethyl)-N-ethyl-7-fluoro-1,3-dihydrofuro [3,4-c] quinoline-8-carboxamide;
4-amino-N-((1S)-1-(6-chloro-3-pyridinyl)ethyl)-N-ethyl-7-fluoro-1,3-dihydrofuro[3,4-c]quinoline-8-carboxamide;
4-amino-7-fluoro-1-methyl-N-(1-methyl-1H-pyrazol-4-yl)-N-((5-(trifluoromethyl)-2-pyridinyl)methyl)-1H-pyrazolo[4,3-c]quinoline-8-carboxamide;
4-amino-7-chloro-N-(cyclopropylmethyl)-N-((6-(trifluoromethyl)-3-pyridazinyl)methyl)-1,3-dihydrofuro[3,4-c]quinoline-8-carboxamide;
4-amino-N-ethyl-N-((1R)-1-(2-fluoro-4-(trifluoromethyl)phenyl)ethyl)-1,3-dihydrofuro[3,4-c]quinoline-8-carboxamide;
4-amino-N-ethyl-N-((1R)-1-(4-(trifluoromethyl)phenyl)ethyl)-1,3-dihydrofuro[3,4-c]quinoline-8-carboxamide;
4-amino-7-chloro-N-cyclobutyl-N-((1R)-1-(6-(trifluoromethyl)-3-pyridazinyl)ethyl)-1,3-dihydrofuro[3,4-c]quinoline-8-carboxamide;
4-amino-N-cyclobutyl-N-((1R)-1-(6-(trifluoromethyl)-3-pyridazinyl)ethyl)-1,3-dihydrofuro[3,4-c]quinoline-8-carboxamide;
(3R)-4-amino-N-cyclopropyl-3-methyl-N-((1R)-1-(5-(trifluoromethyl)-2-pyridinyl)ethyl)-1,3-dihydrofuro[3,4-c]quinoline-8-carboxamide;
4-amino-N-((6-ethoxy-3-pyridazinyl)methyl)-N-ethyl-7-fluoro-1,3-dihydrofuro[3,4-c]quinoline-8-carboxamide;
4-amino-N-((S)-cyclopropyl(5-(trifluoromethyl)-2-pyridinyl)methyl)-N-ethyl-1,3-dihydrofuro[3,4-c]quinoline-8-carboxamide;
4-amino-N-((6-ethoxy-3-pyridazinyl)methyl)-N-((1R)-1-(2-pyrimidinyl)ethyl)-1,3-dihydrofuro[3,4-c][1,7]naphthyridine-8-carboxamide;
4-amino-7-fluoro-N-(1-methyl-1H-pyrazol-4-yl)-N-((6-(trifluoromethyl)-3-pyridazinyl)methyl)-1,3-dihydrofuro[3,4-c]quinoline-8-carboxamide;
4-amino-7-chloro-N-(1-methyl-1H-pyrazol-4-yl)-N-((6-(trifluoromethyl)-3-pyridazinyl)methyl)-1,3-dihydrofuro[3,4-c]quinoline-8-carboxamide;
4-amino-N-(cyclopropylmethyl)-N-((6-ethoxy-3-pyridazinyl)methyl)-7-fluoro-1,3-dihydrofuro[3,4-c]quinoline-8-carboxamide;
4-amino-N-(cyclopropylmethyl)-N-((6-ethoxy-3-pyridazinyl)methyl)-1,3-dihydrofuro[3,4-c][1,7]naphthyridine-8-carboxamide;
5-amino-N-(2-propanyl)-N-((5-(trifluoromethyl)-2-pyridinyl)methyl)benzo[c] [2,6]naphthyridine-9-carboxamide;
5-amino-N-(2-propanyl)-N-((5-(trifluoromethyl)-2-pyridinyl)methyl)pyrimido [4, 5-c] quinoline-9-carboxamide;
5-amino-N-(2-propanyl)-N-((5-(trifluoromethyl)-2-pyridinyl)methyl)pyrido[4,3-c] [1,7]naphthyridine-9-carboxamide;
4-amino-1-methyl-N-(1-methyl-1H-pyrazol-4-yl)-N-((5-(trifluoromethyl)-2-pyridinyl)methyl)-1H-pyrazolo[4,3-c]quinoline-8-carboxamide;
4-amino-N-((6-ethoxy-3-pyridazinyl)methyl)-3-methyl-N-((1R)-1-(2-pyrimidinyl)ethyl)-3H-pyrazolo[3,4-c]quinoline-8-carboxamide;
4-amino-N-ethyl-N-(2-(4-(trifluoromethyl)phenyl)-2-propanyl)-1,3-dihydrofuro [3,4-c] quinoline-8-carboxamide;
5-amino-N-(2-propanyl)-N-((5-(trifluoromethyl)-2-pyridinyl)methyl)pyrimido[4,5-c] [1,7]naphthyridine-9-carboxamide;
4-amino-N-ethyl-N-((1R)-1-(5-(trifluoromethyl)-2-pyridinyl)propyl)-1,3-dihydrofuro [3,4-c] [1, 7]naphthyridine-8-carboxamide;
4-amino-N-ethyl-7-fluoro-N-((1R)-1-(5-(trifluoromethyl)-2-pyridinyl)ethyl)-1,3-dihydrofuro[3,4-c]quinoline-8-carboxamide;
4-amino-N-ethyl-N-((1R)-1-(6-(trifluoromethyl)-3-pyridazinyl)ethyl)-1,3-dihydrofuro[3,4-c][1,7]naphthyridine-8-carboxamide;
4-amino-N-((R)-cyclopropyl(5-(trifluoromethyl)-2-pyridinyl)methyl)-N-methyl-1,3-dihydrofuro[3,4-c][1,7]naphthyridine-8-carboxamide;
4-amino-N-((S)-cyclopropyl(5-(trifluoromethyl)-2-pyridinyl)methyl)-N-methyl-1,3-dihydrofuro[3,4-c] [1, 7]naphthyridine-8-carboxamide;
4-amino-N-(cyclopropylmethyl)-N-((6-ethoxy-3-pyridazinyl)methyl)-7-fluoro-3-methyl-3H-pyrazolo[3,4-c]quinoline-8-carboxamide;
(3R)-4-amino-N-(cyclopropylmethyl)-N-((6-ethoxy-3-pyridazinyl)methyl)-3-methyl-1,3-dihydrofuro[3,4-c]quinoline-8-carboxamide;
4-amino-N-((R)-cyclopropyl(6-(trifluoromethyl)-3-pyridazinyl)methyl)-N-ethyl-1,3-dihydrofuro[3,4-c][1,7]naphthyridine-8-carboxamide;
(3R)-4-amino-3-methyl-N-((1R)-1-(5-(trifluoromethyl)-2-pyridinyl)ethyl)-1,3-dihydrofuro[3,4-c]quinoline-8-carboxamide;
4-amino-7-fluoro-N,1-dimethyl-N-((5-methyl-2-pyridinyl)methyl)-1H-pyrazolo[4,3-c]quinoline-8-carboxamide;
4-amino-7-fluoro-N-((5-fluoro-2-pyridinyl)methyl)-N,1-dimethyl-1H-pyrazolo[4,3-c]quinoline-8-carboxamide;
4-amino-N-((2,6-difluoro-3-pyridinyl)methyl)-7-fluoro-N,1-dimethyl-1H-pyrazolo[4,3-c]quinoline-8-carboxamide;
4-amino-7-chloro-N-((1R)-1-(5-fluoro-2-pyridinyl)ethyl)-N-methyl-1,3-dihydrofuro[3,4-c]quinoline-8-carboxamide;
4-amino-7-chloro-N-((1S)-1-(5-fluoro-2-pyridinyl)ethyl)-N-methyl-1,3-dihydrofuro[3,4-c]quinoline-8-carboxamide;
4-amino-N-((1R)-1-(5-(difluoromethyl)-2-pyridinyl)ethyl)-N-ethyl-1,3-dihydrofuro[3,4-c][1,7]naphthyridine-8-carboxamide;
4-amino-N-methyl-N-((1R)-1-(5-(trifluoromethyl)-2-pyrazinyl)ethyl)-1,3-dihydrofuro[3,4-c]quinoline-8-carboxamide;
(3R)-4-amino-N-((1R)-1-(5-(difluoromethyl)-2-pyridinyl)ethyl)-N-ethyl-3-methyl-1,3-dihydrofuro[3,4-c]quinoline-8-carboxamide;
4-amino-N,1-dimethyl-N-((1R)-1-(5-(trifluoromethyl)-2-pyrazinyl)ethyl)-1H-pyrazolo[4,3-c]quinoline-8-carboxamide;
(3R)-4-amino-N-ethyl-3-methyl-N-((1R)-1-(5-(trifluoromethyl)-2-pyridinyl)ethyl)-1,3-dihydrofuro[3,4-c]quinoline-8-carboxamide;
(3R)-4-amino-N-ethyl-3-methyl-N-((1R)-1-(6-(trifluoromethyl)-3-pyridazinyl)ethyl)-1,3-dihydrofuro[3,4-c]quinoline-8-carboxamide;
4-amino-7-fluoro-N,3-dimethyl-N-((1R)-1-(6-(trifluoromethyl)-3-pyridazinyl)ethyl)-3H-pyrazolo[3,4-c]quinoline-8-carboxamide;
4-amino-7-fluoro-N,1-dimethyl-N-((1R)-1-(5-(trifluoromethyl)-2-pyrazinyl)ethyl)-1H-pyrazolo[4,3-c]quinoline-8-carboxamide;
4-amino-N-ethyl-7-fluoro-N-((1R)-1-(6-(trifluoromethyl)-3-pyridazinyl)ethyl)-1,3-dihydrofuro[3,4-c]quinoline-8-carboxamide;
4-amino-7-fluoro-N-((2-fluoro-6-(trifluoromethyl)-3-pyridinyl)methyl)-N,1-dimethyl-1H-pyrazolo[4,3-c]quinoline-8-carboxamide;
4-amino-N-((R)-cyclopropyl(6-(trifluoromethyl)-3-pyridazinyl)methyl)-N-ethyl-1,3-dihydrofuro[3,4-c]quinoline-8-carboxamide;
(3R)-4-amino-N-ethyl-7-fluoro-3-methyl-N-((1R)-1-(6-(trifluoromethyl)-3-pyridazinyl)ethyl)-1,3-dihydrofuro[3,4-c]quinoline-8-carboxamide;
(3 S)-4-amino-N-ethyl-7-fluoro-3-methyl-N-((1R)-1-(6-(trifluoromethyl)-3-pyridazinyl)ethyl)-1,3-dihydrofuro[3,4-c]quinoline-8-carboxamide;
4-amino-N,3-dimethyl-N-((1R)-1-(4-(pentafluoroethyl)phenyl)ethyl)-3H-pyrazolo[3,4-c] [1, 7]naphthyridine-8-carboxamide;
4-amino-7-fluoro-N,1-dimethyl-N-((1S)-1-(4-(pentafluoroethyl)phenyl)ethyl)-1H-pyrazolo[4,3-c]quinoline-8-carboxamide;
4-amino-7-fluoro-N,1-dimethyl-N-((1R)-1-(4-(pentafluoroethyl)phenyl)ethyl)-1H-pyrazolo[4,3-c]quinoline-8-carboxamide;
4-amino-7-fluoro-N,1-dimethyl-N-((5-(1-(trifluoromethyl)-1H-pyrazol-4-yl)-2-pyridinyl)methyl)-1H-pyrazolo[4,3-c]quinoline-8-carboxamide;
4-amino-N-cyclopropyl-1-methyl-N-((6-(trifluoromethyl)-3-pyridazinyl)methyl)-1H-pyrazolo[4,3-c]quinoline-8-carboxamide;
4-amino-7-chloro-N,1-dimethyl-N-((5-(trifluoromethyl)-2-pyridinyl)methyl)-1H-pyrazolo[4,3-c]quinoline-8-carboxamide;
4-amino-7-chloro-N,1-dimethyl-N-((6-(trifluoromethyl)-3-pyridazinyl)methyl)-1H-pyrazolo[4,3-c]quinoline-8-carboxamide;
4-amino-7-chloro-N-ethyl-1-methyl-N-((5-(trifluoromethyl)-2-pyridinyl)methyl)-1H-pyrazolo[4,3-c]quinoline-8-carboxamide;
4-amino-7-chloro-N-ethyl-1-methyl-N-((6-(trifluoromethyl)-3-pyridazinyl)methyl)-1H-pyrazolo [4,3-c]quinoline-8-carboxamide;
4-amino-7-chloro-N-cyclopropyl-1-methyl-N-((5-(trifluoromethyl)-2-pyridinyl)methyl)-1H-pyrazolo[4,3-c]quinoline-8-carboxamide;
4-amino-7-chloro-N-cyclopropyl-1-methyl-N-((6-(trifluoromethyl)-3-pyridazinyl)methyl)-1H-pyrazolo[4,3 -c]quinoline-8-carboxamide;
4-amino-1-methyl-N-(2,2,2-trifluoroethyl)-N-((5-(trifluoromethyl)-2-pyridinyl)methyl)-1H-pyrazolo[4,3-c]quinoline-8-carboxamide;
4-amino-7-chloro-N-cyclobutyl-1-methyl-N-((5-(trifluoromethyl)-2-pyridinyl)methyl)-1H-pyrazolo [4,3-c]quinoline-8-carboxamide;
4-amino-7-chloro-N-cyclobutyl-1-methyl-N-((6-(trifluoromethyl)-3-pyridazinyl)methyl)-1H-pyrazolo[4,3 -c]quinoline-8-carboxamide;
4-amino-7-fluoro-1-methyl-N-(2,2,2-trifluoroethyl)-N-((5-(trifluoromethyl)-2-pyridinyl)methyl)-1H-pyrazolo[4,3-c]quinoline-8-carboxamide;
4-amino-N-cyclobutyl-7-fluoro-1-methyl-N-(1,3-oxazol-4-ylmethyl)-1H-pyrazolo[4,3-c]quinoline-8-carboxamide;
4-amino-7-fluoro-N,1-dimethyl-N-((5-(trifluoromethyl)-2-pyridinyl)methyl)-1H-pyrazolo[4,3-c]quinoline-8-carboxamide;
4-amino-N-cyclopropyl-7-fluoro-1-methyl-N-((5-(trifluoromethyl)-2-pyridinyl)methyl)-1H-pyrazolo[4,3-c]quinoline-8-carboxamide;
4-amino-N-cyclopropyl-7-fluoro-1-methyl-N-((6-(trifluoromethyl)-3-pyridazinyl)methyl)-1H-pyrazolo[4,3-c]quinoline-8-carboxamide;
4-amino-7-fluoro-1-methyl-N-(2-propanyl)-N-((5-(trifluoromethyl)-2-pyridinyl)methyl)-1H-pyrazolo[4,3-c]quinoline-8-carboxamide;
4-amino-7-fluoro-N-((2-methoxy-6-(trifluoromethyl)-3-pyridinyl)methyl)-N,1-dimethyl-1H-pyrazolo[4,3-c]quinoline-8-carboxamide;
4-amino-N-cyclobutyl-7-fluoro-1-methyl-N-((5-(trifluoromethyl)-2-pyridinyl)methyl)-1H-pyrazolo[4,3-c]quinoline-8-carboxamide;
4-amino-7-chloro-N-cyclopropyl-3-methyl-N-((5-(trifluoromethyl)-2-pyridinyl)methyl)-3H-pyrazolo[3,4-c]quinoline-8-carboxamide;
4-amino-N-cyclopropyl-7-fluoro-N-((3-fluoro-5-(trifluoromethyl)-2-pyridinyl)methyl)-1-methyl-1H-pyrazolo[4,3 -c]quinoline-8-carboxamide;
4-amino-N,1-dimethyl-7-(trifluoromethyl)-N-((5-(trifluoromethyl)-2-pyridinyl)methyl)-1H-pyrazolo[4,3-c]quinoline-8-carboxamide;
4-amino-N,1-dimethyl-7-(trifluoromethyl)-N-((6-(trifluoromethyl)-3-pyridazinyl)methyl)-1H-pyrazolo[4,3 -c]quinoline-8-carboxamide;
4-amino-N-cyclopropyl-1-methyl-7-(trifluoromethyl)-N-((5-(trifluoromethyl)-2-pyridinyl)methyl)-1H-pyrazolo[4,3 -c]quinoline-8-carboxamide;
4-amino-N-cyclopropyl-1-methyl-7-(trifluoromethyl)-N-((6-(trifluoromethyl)-3-pyridazinyl)methyl)-1H-pyrazolo[4,3 -c]quinoline-8-carboxamide;
4-amino-N-((1R)-1-cyclopropylethyl)-7-fluoro-N-((6-(trifluoromethyl)-3-pyridazinyl)methyl)-1,3-dihydrofuro[3,4-c]quinoline-8-carboxamide;
4-amino-N-((1S)-1-cyclopropylethyl)-7-fluoro-N-((6-(trifluoromethyl)-3-pyridazinyl)methyl)-1,3-dihydrofuro[3,4-c]quinoline-8-carboxamide;
4-amino-N-(cyclopropylmethyl)-7-fluoro-N-((6-(2,2,2-trifluoroethoxy)-3-pyridazinyl)methyl)-1,3-dihydrofuro[3,4-c]quinoline-8-carboxamide;
4-amino-N-(cyclopropylmethyl)-7-fluoro-3-methyl-N-((6-(2,2,2-trifluoroethoxy)-3-pyridazinyl)methyl)-3H-pyrazolo[3,4-c]quinoline-8-carboxamide;
(3R)-4-amino-N-(cyclopropylmethyl)-7-fluoro-3-methyl-N-((6-(2,2,2-trifluoroethoxy)-3-pyridazinyl)methyl)-1,3-dihydrofuro[3,4-c]quinoline-8-carboxamide;
4-amino-N-ethyl-N-((1S)-1-(5-(trifluoromethyl)-2-pyridinyl)ethyl)-1,3-dihydrofuro[3,4-c]quinoline-8-carboxamide;
4-amino-N-ethyl-N-((1R)-1-(5-(trifluoromethyl)-2-pyridinyl)ethyl)-1,3-dihydrofuro[3,4-c] quinoline-8-carboxamide;
4-amino-N-ethyl-N-((1R)-1-(5-(trifluoromethyl)-2-pyridinyl)ethyl)-1,3-dihydrofuro[3,4-c][1,7]naphthyridine-8-carboxamide;
4-amino-N-ethyl-N-((1S)-1-(5-(trifluoromethyl)-2-pyridinyl)ethyl)-1,3-dihydrofuro[3,4-c][1,7]naphthyridine-8-carboxamide;
4-amino-N-(1-methyl-1H-pyrazol-4-yl)-N-((1R)-1-(5-(trifluoromethyl)-2-pyridinyl)ethyl)-1,3-dihydrofuro[3,4-c][1,7]naphthyridine-8-carboxamide;
4-amino-N-(1-methyl-1H-pyrazol-4-yl)-N-((1S)-1-(5-(trifluoromethyl)-2-pyridinyl)ethyl)-1,3-dihydrofuro[3,4-c][1,7]naphthyridine-8-carboxamide;
(3S)-4-amino-3-methyl-N-(1-methyl-1H-pyrazol-4-yl)-N-((5-(trifluoromethyl)-2-pyridinyl)methyl)-1,3-dihydrofuro[3,4-c][1,7]naphthyridine-8-carboxamide;
(3R)-4-amino-3-methyl-N-(1-methyl-1H-pyrazol-4-yl)-N-((5-(trifluoromethyl)-2-pyridinyl)methyl)-1,3-dihydrofuro[3,4-c][1,7]naphthyridine-8-carboxamide;
(3S)-4-amino-N-cyclobutyl-3-methyl-N-((6-(trifluoromethyl)-3-pyridazinyl)methyl)-1,3-dihydrofuro[3,4-c][1,7]naphthyridine-8-carboxamide;
(3R)-4-amino-N-cyclobutyl-3-methyl-N-((6-(trifluoromethyl)-3-pyridazinyl)methyl)-1,3-dihydrofuro[3,4-c][1,7]naphthyridine-8-carboxamide;
4-amino-N-(2-propanyl)-N-((1S)-1-(5-(trifluoromethyl)-2-pyridinyl)ethyl)-1,3-dihydrofuro[3,4-c]quinoline-8-carboxamide;
4-amino-N-(2-propanyl)-N-((1R)-1-(5-(trifluoromethyl)-2-pyridinyl)ethyl)-1,3-dihydrofuro[3,4-c]quinoline-8-carboxamide;
4-amino-7-fluoro-N-((1S)-1-(5-fluoro-2-pyrimidinyl)ethyl)-N-(2-(trifluoromethoxy)ethyl)-1,3-dihydrofuro[3,4-c]quinoline-8-carboxamide;
4-amino-7-fluoro-N-((1R)-1-(5-fluoro-2-pyrimidinyl)ethyl)-N-(2-(trifluoromethoxy)ethyl)-1,3-dihydrofuro[3,4-c]quinoline-8-carboxamide;
4-amino-N-((1R)-1-(4-cyanophenyl)ethyl)-N-ethyl-1,3-dihydrofuro[3,4-c]quinoline-8-carboxamide;
4-amino-N-((1S)-1-(4-cyanophenyl)ethyl)-N-ethyl-1,3-dihydrofuro[3,4-c]quinoline-8-carboxamide;
4-amino-N-((1S)-1-(5-cyano-2-pyridinyl)ethyl)-N-ethyl-1,3-dihydrofuro[3,4-c]quinoline-8-carboxamide;
4-amino-N-((1R)-1-(5-cyano-2-pyridinyl)ethyl)-N-ethyl-1,3-dihydrofuro[3,4-c]quinoline-8-carboxamide;
(3R)-4-amino-3-methyl-N-(2-propanyl)-N-((1R)-1-(5-(trifluoromethyl)-2-pyridinyl)ethyl)-1,3-dihydrofuro[3,4-c]quinoline-8-carboxamide;
(3R)-4-amino-3-methyl-N-(2-propanyl)-N-((1S)-1-(5-(trifluoromethyl)-2-pyridinyl)ethyl)-1,3-dihydrofuro[3,4-c]quinoline-8-carboxamide;
4-amino-7-fluoro-N-(2-propanyl)-N-((1S)-1-(5-(trifluoromethyl)-2-pyridinyl)ethyl)-1,3-dihydrofuro[3,4-c]quinoline-8-carboxamide;
4-amino-7-fluoro-N-(2-propanyl)-N-((1R)-1-(5-(trifluoromethyl)-2-pyridinyl)ethyl)-1,3-dihydrofuro [3,4-c]quinoline-8-carboxamide;
(3R)-4-amino-N-((1S)-1-(5-cyano-2-pyridinyl)ethyl)-N-ethyl-3-methyl-1,3-dihydrofuro[3,4-c]quinoline-8-carboxamide;
(3R)-4-amino-N-((1R)-1-(5-cyano-2-pyridinyl)ethyl)-N-ethyl-3-methyl-1,3-dihydrofuro [3,4-c] quinoline-8-carboxamide;
4-amino-7-chloro-N-(2-propanyl)-N-((1R)-1-(5-(trifluoromethyl)-2-pyridinyl)ethyl)-1,3-dihydrofuro[3,4-c]quinoline-8-carboxamide;
4-amino-7-chloro-N-(2-propanyl)-N-((1S)-1-(5-(trifluoromethyl)-2-pyridinyl)ethyl)-1,3-dihydrofuro[3,4-c]quinoline-8-carboxamide;
4-amino-N-(2-propanyl)-N-((1S)-1-(5-(trifluoromethyl)-2-pyridinyl)ethyl)-1,3-dihydrofuro[3,4-c][1,7]naphthyridine-8-carboxamide;
4-amino-N-(2-propanyl)-N-((1R)-1-(5-(trifluoromethyl)-2-pyridinyl)ethyl)-1,3-dihydrofuro[3,4-c][1,7]naphthyridine-8-carboxamide;
4-amino-N-methyl-N-((1R)-1-(5-(trifluoromethyl)-2-pyridinyl)propyl)-1,3-dihydrofuro[3,4-c] [1, 7]naphthyridine-8-carboxamide;
4-amino-N-methyl-N-((1S)-1-(5-(trifluoromethyl)-2-pyridinyl)propyl)-1,3-dihydrofuro[3,4-c] [1, 7]naphthyridine-8-carboxamide;
(3R)-4-amino-N,3-dimethyl-N-((1R)-1-(5-(trifluoromethyl)-2-pyridinyl)ethyl)-1,3-dihydrofuro[3,4-c]quinoline-8-carboxamide;
(3R)-4-amino-N,3-dimethyl-N-((1S)-1-(5-(trifluoromethyl)-2-pyridinyl)ethyl)-1,3-dihydrofuro[3,4-c]quinoline-8-carboxamide;
4-amino-N-((1R)-1-(3,5-difluoro-2-pyridinyl)ethyl)-7-fluoro-N,1-dimethyl-1H-pyrazolo[4,3-c]quinoline-8-carboxamide;
4-amino-N-methyl-N-((1R)-1-(5-(trifluoromethyl)-2-pyridinyl)ethyl)-1,3-dihydrofuro[3,4-c] [1, 7]naphthyridine-8-carboxamide;
4-amino-N,1-dimethyl-N-((1R)-1-(6-(trifluoromethyl)-3-pyridazinyl)ethyl)-1H-pyrazolo[4,3-c]quinoline-8-carboxamide;
(3R)-4-amino-N,3-dimethyl-N-((1R)-1-(6-(trifluoromethyl)-3-pyridazinyl)ethyl)-1,3-dihydrofuro[3,4-c]quinoline-8-carboxamide;
4-amino-7-fluoro-N-methyl-N-((1R)-1-(4-(trifluoromethyl)phenyl)ethyl)-1,3-dihydrofuro [3,4-c]quinoline-8-carboxamide;
4-amino-7-fluoro-N-methyl-N-((1R)-1-(6-(trifluoromethyl)-3-pyridazinyl)ethyl)-1,3-dihydrofuro[3,4-c]quinoline-8-carboxamide;
(3R)-4-amino-7-fluoro-N,3-dimethyl-N-((1S)-1-(4-(trifluoromethyl)phenyl)ethyl)-1,3-dihydrofuro[3,4-c]quinoline-8-carboxamide;
(3R)-4-amino-7-fluoro-N,3-dimethyl-N-((1R)-1-(4-(trifluoromethyl)phenyl)ethyl)-1,3-dihydrofuro[3,4-c]quinoline-8-carboxamide;
4-amino-N-((1R)-1-(3-fluoro-5-(trifluoromethyl)-2-pyridinyl)ethyl)-N,3-dimethyl-3H-pyrazolo[3,4-c][1, 7]naphthyridine-8-carboxamide;
(3R)-4-amino-7-fluoro-N,3-dimethyl-N-((1R)-1-(6-(trifluoromethyl)-3-pyridazinyl)ethyl)-1,3-dihydrofuro[3,4-c]quinoline-8-carboxamide;
4-amino-7-fluoro-N-((1R)-1-(3-fluoro-5-(trifluoromethyl)-2-pyridinyl)ethyl)-N,1-dimethyl-1H-pyrazolo[4,3-c]quinoline-8-carboxamide;
4-amino-7-fluoro-N-((1S)-1-(3-fluoro-5-(trifluoromethyl)-2-pyridinyl)ethyl)-N,1-dimethyl-1H-pyrazolo[4,3-c]quinoline-8-carboxamide;
4-amino-7-fluoro-N-((1R)-1-(3-fluoro-5-(trifluoromethyl)-2-pyridinyl)ethyl)-N,3-dimethyl-3H-pyrazolo[3,4-c]quinoline-8-carboxamide;
4-amino-7-fluoro-N-((1R)-1-(3-methoxy-5-(trifluoromethyl)-2-pyridinyl)ethyl)-N,3-dimethyl-3H-pyrazolo[3,4-c]quinoline-8-carboxamide;
4-amino-7-fluoro-N-((1R)-1-(3-methoxy-5-(trifluoromethyl)-2-pyridinyl)ethyl)-N, 1-dimethyl-1H-pyrazolo[4,3-c]quinoline-8-carboxamide;
4-amino-N,3-dimethyl-N-((1R)-1-(4-(pentafluoro-lambda~6~-sulfanyl)phenyl)ethyl)-3H-pyrazolo[3,4-c][1,7]naphthyridine-8-carboxamide;
4-amino-7-fluoro-N,1-dimethyl-N-((1S)-1-(4-(pentafluoro-lambda~6~-sulfanyl)phenyl)ethyl)-1H-pyrazolo[4,3-c]quinoline-8-carboxamide;
4-amino-7-fluoro-N,1-dimethyl-N-((1R)-1-(4-(pentafluoro-lambda~6~-sulfanyl)phenyl)ethyl)-1H-pyrazolo[4,3-c]quinoline-8-carboxamide;
4-amino-1-methyl-N-(4-(pentafluoro-lambda~6~-sulfanyl)benzyl)-N-((1R)-1-(5-pyrimidinyl)propyl)-1H-pyrazolo[4,3-c][1,7]naphthyridine-8-carboxamide;
4-amino-1-methyl-N-(4-(pentafluoro-lambda~6~-sulfanyl)benzyl)-N-((1S)-1-(5-pyrimidinyl)propyl)-1H-pyrazolo[4,3-c][1,7]naphthyridine-8-carboxamide;
4-amino-7-fluoro-N-((1R)-1-(5-fluoro-2-pyridinyl)ethyl)-N,1-dimethyl-1H-pyrazolo[4,3-c]quinoline-8-carboxamide;
4-amino-7-fluoro-N-((1S)-1-(5-fluoro-2-pyridinyl)ethyl)-N,1-dimethyl-1H-pyrazolo[4,3-c]quinoline-8-carboxamide;
4-amino-N-ethyl-7-fluoro-N-((1R)-1-(5-fluoro-2-pyridinyl)ethyl)-1-methyl-1H-pyrazolo [4,3-c] quinoline-8-carboxamide;
4-amino-N-ethyl-7-fluoro-N-((1S)-1-(5-fluoro-2-pyridinyl)ethyl)-1-methyl-1H-pyrazolo[4,3-c]quinoline-8-carboxamide;
4-amino-N-cyclopropyl-7-fluoro-1-methyl-N-((1S)-1-(6-(trifluoromethyl)-3-pyridazinyl)ethyl)-1H-pyrazolo[4,3-c]quinoline-8-carboxamide;
4-amino-N-cyclopropyl-7-fluoro-1-methyl-N-((1R)-1-(6-(trifluoromethyl)-3-pyridazinyl)ethyl)-1H-pyrazolo[4,3-c]quinoline-8-carboxamide;
4-amino-N,1-dimethyl-7-(trifluoromethyl)-N-((1R)-1-(6-(trifluoromethyl)-3-pyridazinyl)ethyl)-1H-pyrazolo [4,3-c]quinoline-8-carboxamide;
4-amino-7-chloro-N-cyclopropyl-1-methyl-N-((1S)-1-(6-(trifluoromethyl)-3-pyridazinyl)ethyl)-1H-pyrazolo [4,3-c]quinoline-8-carboxamide;
4-amino-7-chloro-N-cyclopropyl-1-methyl-N-((1R)-1-(6-(trifluoromethyl)-3-pyridazinyl)ethyl)-1H-pyrazolo [4,3-c]quinoline-8-carboxamide;
4-amino-N-(1-methyl-1H-pyrazol-4-yl)-N-(4-(trifluoromethyl)benzyl)-1,3-dihydrofuro[3,4-c][1,7]naphthyridine-8-carboxamide;
4-amino-N-(3-fluorophenyl)-N-((5-(trifluoromethyl)-2-pyridinyl)methyl)-1,3-dihydrofuro[3,4-c]quinoline-8-carboxamide;
4-amino-N-(1-methyl-1H-pyrazol-4-yl)-N-((5-(trifluoromethyl)-2-pyridinyl)methyl)-1,3-dihydrofuro[3,4-c]quinoline-8-carboxamide;
4-amino-N-(1-methyl-1H-pyrazol-4-yl)-N-((5-(trifluoromethyl)-2-pyridinyl)methyl)-1,3-dihydrofuro[3,4-c][1,7]naphthyridine-8-carboxamide;
4-amino-N-(1-methyl-1H-pyrazol-4-yl)-N-((5-(trifluoromethyl)-2-pyridinyl)methyl)-1,3-dihydrofuro[3,4-c][1,8]naphthyridine-8-carboxamide;
5-amino-N-((1R)-1-(2-pyrimidinyl)ethyl)-N-((5-(trifluoromethyl)-2-pyridinyl)methyl)-1,4-dihydro-2H-pyrano[3,4-c]quinoline-9-carboxamide;
4-amino-N-((1R)-1-(2-pyrimidinyl)ethyl)-N-((5-(trifluoromethyl)-2-pyridinyl)methyl)-2,3-dihydrofuro[3,2-c]quinoline-8-carboxamide;
4-amino-3,3-dimethyl-N-(2-methylpropyl)-N-((5-(trifluoromethyl)-2-pyridinyl)methyl)-1,3-dihydrofuro[3,4-c]quinoline-8-carboxamide;
(3R)-4-amino-N-((3-fluoro-2-pyridinyl)methyl)-3-methyl-N-((5-(trifluoromethyl)-2-pyridinyl)methyl)-1,3-dihydrofuro[3,4-c]quinoline-8-carboxamide;
(3S)-4-amino-N-((3-fluoro-2-pyridinyl)methyl)-3-methyl-N-((5-(trifluoromethyl)-2-pyridinyl)methyl)-1,3-dihydrofuro[3,4-c]quinoline-8-carboxamide;
(3R)-4-amino-3-methyl-N-((1R)-1-(tetrahydro-2H-pyran-4-yl)ethyl)-N-((5-(trifluoromethyl)-2-pyridinyl)methyl)-1,3-dihydrofuro[3,4-c]quinoline-8-carboxamide;
(3R)-4-amino-3-methyl-N-((1S)-1-(tetrahydro-2H-pyran-4-yl)ethyl)-N-((5-(trifluoromethyl)-2-pyridinyl)methyl)-1,3-dihydrofuro[3,4-c]quinoline-8-carboxamide;
(3S)-4-amino-3-methyl-N-((1R)-1-(tetrahydro-2H-pyran-4-yl)ethyl)-N-((5-(trifluoromethyl)-2-pyridinyl)methyl)-1,3-dihydrofuro[3,4-c]quinoline-8-carboxamide;
(3S)-4-amino-3-methyl-N-((1S)-1-(tetrahydro-2H-pyran-4-yl)ethyl)-N-((5-(trifluoromethyl)-2-pyridinyl)methyl)-1,3-dihydrofuro[3,4-c]quinoline-8-carboxamide;
(3R)-4-amino-N-((5-cyano-2-pyridinyl)methyl)-3-methyl-N-((1R)-1-(tetrahydro-2H-pyran-4-yl)ethyl)-1,3-dihydrofuro[3,4-c]quinoline-8-carboxamide;
(3R)-4-amino-N-((5-cyano-2-pyridinyl)methyl)-3-methyl-N-((1S)-1-(tetrahydro-2H-pyran-4-yl)ethyl)-1,3-dihydrofuro[3,4-c]quinoline-8-carboxamide;
(3S)-4-amino-N-((5-cyano-2-pyridinyl)methyl)-3-methyl-N-((1R)-1-(tetrahydro-2H-pyran-4-yl)ethyl)-1,3-dihydrofuro[3,4-c]quinoline-8-carboxamide;
(3S)-4-amino-N-((5-cyano-2-pyridinyl)methyl)-3-methyl-N-((1S)-1-(tetrahydro-2H-pyran-4-yl)ethyl)-1,3-dihydrofuro[3,4-c]quinoline-8-carboxamide;
(3R)-4-amino-N-((trans-4-hydroxycyclohexyl)methyl)-3-methyl-N-((5-(trifluoromethyl)-2-pyridinyl)methyl)-1,3-dihydrofuro[3,4-c]quinoline-8-carboxamide;
(3S)-4-amino-N-((trans-4-hydroxycyclohexyl)methyl)-3-methyl-N-((5-(trifluoromethyl)-2-pyridinyl)methyl)-1,3-dihydrofuro[3,4-c]quinoline-8-carboxamide;
(3R)-4-amino-N-((5-cyano-2-pyridinyl)methyl)-N-((trans-4-hydroxycyclohexyl)methyl)-3-methyl-1,3-dihydrofuro[3,4-c]quinoline-8-carboxamide;
(3S)-4-amino-N-((5-cyano-2-pyridinyl)methyl)-N-((trans-4-hydroxycyclohexyl)methyl)-3-methyl-1,3-dihydrofuro[3,4-c]quinoline-8-carboxamide;
(3R)-4-amino-3-methyl-N-(tetrahydro-2H-pyran-4-ylmethyl)-N-((5-(trifluoromethyl)-2-pyridinyl)methyl)-1,3-dihydrofuro[3,4-c]quinoline-8-carboxamide;
(3S)-4-amino-3-methyl-N-(tetrahydro-2H-pyran-4-ylmethyl)-N-((5-(trifluoromethyl)-2-pyridinyl)methyl)-1,3-dihydrofuro[3,4-c]quinoline-8-carboxamide;
(3R)-4-amino-N-((5-cyano-2-pyridinyl)methyl)-3-methyl-N-(tetrahydro-2H-pyran-4-ylmethyl)-1,3-dihydrofuro[3,4-c]quinoline-8-carboxamide;
(3S)-4-amino-N-((5-cyano-2-pyridinyl)methyl)-3-methyl-N-(tetrahydro-2H-pyran-4-ylmethyl)-1,3-dihydrofuro[3,4-c]quinoline-8-carboxamide;
(3R)-4-amino-N-((1R,2R)-2-cyanocyclopentyl)-3-methyl-N-((5-(trifluoromethyl)-2-pyridinyl)methyl)-1,3-dihydrofuro[3,4-c]quinoline-8-carboxamide;
(3R)-4-amino-N-((1R,2S)-2-cyanocyclopentyl)-3-methyl-N-((5-(trifluoromethyl)-2-pyridinyl)methyl)-1,3-dihydrofuro[3,4-c]quinoline-8-carboxamide;
(3R)-4-amino-N-((1S,2R)-2-cyanocyclopentyl)-3-methyl-N-((5-(trifluoromethyl)-2-pyridinyl)methyl)-1,3-dihydrofuro[3,4-c]quinoline-8-carboxamide;
(3R)-4-amino-N-((1S,2S)-2-cyanocyclopentyl)-3-methyl-N-((5-(trifluoromethyl)-2-pyridinyl)methyl)-1,3-dihydrofuro[3,4-c]quinoline-8-carboxamide;
(3S)-4-amino-N-((1R,2R)-2-cyanocyclopentyl)-3-methyl-N-((5-(trifluoromethyl)-2-pyridinyl)methyl)-1,3-dihydrofuro[3,4-c]quinoline-8-carboxamide;
(3S)-4-amino-N-((1R,2S)-2-cyanocyclopentyl)-3-methyl-N-((5-(trifluoromethyl)-2-pyridinyl)methyl)-1,3-dihydrofuro[3,4-c]quinoline-8-carboxamide;
(3S)-4-amino-N-((1S,2R)-2-cyanocyclopentyl)-3-methyl-N-((5-(trifluoromethyl)-2-pyridinyl)methyl)-1,3-dihydrofuro[3,4-c]quinoline-8-carboxamide;
(3S)-4-amino-N-((1S,2S)-2-cyanocyclopentyl)-3-methyl-N-((5-(trifluoromethyl)-2-pyridinyl)methyl)-1,3-dihydrofuro[3,4-c]quinoline-8-carboxamide;
(3R)-4-amino-N-((1R,2R)-2-cyanocyclopentyl)-N-((5-cyano-2-pyridinyl)methyl)-3-methyl-1,3-dihydrofuro[3,4-c]quinoline-8-carboxamide;
(3R)-4-amino-N-((1R,2S)-2-cyanocyclopentyl)-N-((5-cyano-2-pyridinyl)methyl)-3-methyl-1,3-dihydrofuro[3,4-c]quinoline-8-carboxamide;
(3R)-4-amino-N-((1S,2R)-2-cyanocyclopentyl)-N-((5-cyano-2-pyridinyl)methyl)-3-methyl-1,3-dihydrofuro[3,4-c]quinoline-8-carboxamide;
(3R)-4-amino-N-((1S,2S)-2-cyanocyclopentyl)-N-((5-cyano-2-pyridinyl)methyl)-3-methyl-1,3-dihydrofuro[3,4-c]quinoline-8-carboxamide;
(3S)-4-amino-N-((1R,2R)-2-cyanocyclopentyl)-N-((5-cyano-2-pyridinyl)methyl)-3-methyl-1,3-dihydrofuro[3,4-c]quinoline-8-carboxamide;
(3S)-4-amino-N-((1R,2S)-2-cyanocyclopentyl)-N-((5-cyano-2-pyridinyl)methyl)-3-methyl-1,3-dihydrofuro[3,4-c]quinoline-8-carboxamide;
(3S)-4-amino-N-((1S,2R)-2-cyanocyclopentyl)-N-((5-cyano-2-pyridinyl)methyl)-3-methyl-1,3-dihydrofuro[3,4-c]quinoline-8-carboxamide;
(3S)-4-amino-N-((1S,2S)-2-cyanocyclopentyl)-N-((5-cyano-2-pyridinyl)methyl)-3-methyl-1,3-dihydrofuro[3,4-c]quinoline-8-carboxamide;
(3R)-4-amino-3-methyl-N-((3R)-tetrahydro-3-furanyl)-N-((5-(trifluoromethyl)-2-pyridinyl)methyl)-1,3-dihydrofuro[3,4-c]quinoline-8-carboxamide;
(3R)-4-amino-3-methyl-N-((3S)-tetrahydro-3-furanyl)-N-((5-(trifluoromethyl)-2-pyridinyl)methyl)-1,3-dihydrofuro[3,4-c]quinoline-8-carboxamide;
(3S)-4-amino-3-methyl-N-((3R)-tetrahydro-3-furanyl)-N-((5-(trifluoromethyl)-2-pyridinyl)methyl)-1,3-dihydrofuro[3,4-c]quinoline-8-carboxamide;
(3S)-4-amino-3-methyl-N-((3S)-tetrahydro-3-furanyl)-N-((5-(trifluoromethyl)-2-pyridinyl)methyl)-1,3-dihydrofuro[3,4-c]quinoline-8-carboxamide;
(3R)-4-amino-3-methyl-N-((1R,5S,6r)-3-oxabicyclo[3.1.0]hexan-6-yl)-N-((5-(trifluoromethyl)-2-pyridinyl)methyl)-1,3-dihydrofuro[3,4-c]quinoline-8-carboxamide;
(3S)-4-amino-3-methyl-N-((1R,5S,6r)-3-oxabicyclo[3.1.0]hexan-6-yl)-N-((5-(trifluoromethyl)-2-pyridinyl)methyl)-1,3-dihydrofuro[3,4-c]quinoline-8-carboxamide;
(3R)-4-amino-3-methyl-N-((3R,4R)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3R)-4-amino-3-methyl-N-((3R,4S)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3R)-4-amino-3-methyl-N-((3S,4R)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3R)-4-amino-3 -methyl-N-((3 S,4S)-3-methyltetrahydro-2H-pyran-4-yl)-N-((5 -(trifluoromethyl)-2-pyridinyl)methyl)-1,3-dihydrofuro[3,4-c]quinoline-8-carboxamide;
(3S)-4-amino-3-methyl-N-((3R,4R)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3S)-4-amino-3-methyl-N-((3R,4S)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3S)-4-amino-3-methyl-N-((3S,4R)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3S)-4-amino-3-methyl-N-((3S,4S)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3R)-4-amino-N-((3S,4R)-3-methoxytetrahydro-2H-pyran-4-yl)-3-methyl-N-((5-(trifluoromethyl)-2-pyridinyl)methyl)-1,3-dihydrofuro[3,4-c]quinoline-8-carboxamide;
(3S)-4-amino-N-((3S,4R)-3-methoxytetrahydro-2H-pyran-4-yl)-3-methyl-N-((5-(trifluoromethyl)-2-pyridinyl)methyl)-1,3-dihydrofuro[3,4-c]quinoline-8-carboxamide;
(3R)-4-amino-N-((3R,4S)-3-methoxytetrahydro-2H-pyran-4-yl)-3-methyl-N-((5-(trifluoromethyl)-2-pyridinyl)methyl)-1,3-dihydrofuro[3,4-c]quinoline-8-carboxamide;
(3S)-4-amino-N-((3R,4S)-3-methoxytetrahydro-2H-pyran-4-yl)-3-methyl-N-((5-(trifluoromethyl)-2-pyridinyl)methyl)-1,3-dihydrofuro[3,4-c]quinoline-8-carboxamide;
(3R)-4-amino-N-((1R,2R)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3R)-4-amino-N-((1R,2S)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3R)-4-amino-N-((1S,2R)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3R)-4-amino-N-((1S,2S)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3S)-4-amino-N-((1R,2R)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3S)-4-amino-N-((1R,2S)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3S)-4-amino-N-((1S,2R)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3S)-4-amino-N-((1S,2S)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3R)-4-amino-3-methyl-N-((1R)-spiro[2.5]octan-1-yl)-N-((5-(trifluoromethyl)-2-pyridinyl)methyl)-1,3-dihydrofuro[3,4-c]quinoline-8-carboxamide;
(3R)-4-amino-3-methyl-N-((1S)-spiro[2.5]octan-1-yl)-N-((5-(trifluoromethyl)-2-pyridinyl)methyl)-1,3-dihydrofuro[3,4-c]quinoline-8-carboxamide;
(3S)-4-amino-3-methyl-N-((1R)-spiro[2.5]octan-1-yl)-N-((5-(trifluoromethyl)-2-pyridinyl)methyl)-1,3-dihydrofuro[3,4-c]quinoline-8-carboxamide;
(3S)-4-amino-3-methyl-N-((1S)-spiro[2.5]octan-1-yl)-N-((5-(trifluoromethyl)-2-pyridinyl)methyl)-1,3-dihydrofuro[3,4-c]quinoline-8-carboxamide;
(3R)-4-amino-N-((1R,2R)-2-aminocyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1R,2S)-2-aminocyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1S,2R)-2-aminocyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1S,2S)-2-aminocyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1R,2R)-2-aminocyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1R,2S)-2-aminocyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1S,2R)-2-aminocyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1S,2S)-2-aminocyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1R,2R)-2-ethoxycyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1R,2S)-2-ethoxycyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1S,2R)-2-ethoxycyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1S,2S)-2-ethoxycyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1R,2R)-2-ethoxycyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1R,2S)-2-ethoxycyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1S,2R)-2-ethoxycyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1S,2S)-2-ethoxycyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1R)-2,2-dimethylcyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1S)-2,2-dimethylcyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1R)-2,2-dimethylcyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1S)-2,2-dimethylcyclopropyl)-3-methyl-N-((5-(trifluoromethyl)-2-pyridinyl)methyl)-1,3-dihydrofuro[3,4-c]quinoline-8-carboxamide;
(3R)-4-amino-3-methyl-N-((1R)-spiro[2.4]heptan-1-yl)-N-((5-(trifluoromethyl)-2-pyridinyl)methyl)-1,3-dihydrofuro[3,4-c]quinoline-8-carboxamide;
(3R)-4-amino-3-methyl-N-((1S)-spiro[2.4]heptan-1-yl)-N-((5-(trifluoromethyl)-2-pyridinyl)methyl)-1,3-dihydrofuro[3,4-c]quinoline-8-carboxamide;
(3S)-4-amino-3-methyl-N-((1R)-spiro[2.4]heptan-1-yl)-N-((5-(trifluoromethyl)-2-pyridinyl)methyl)-1,3-dihydrofuro[3,4-c]quinoline-8-carboxamide;
(3S)-4-amino-3-methyl-N-((1S)-spiro[2.4]heptan-1-yl)-N-((5-(trifluoromethyl)-2-pyridinyl)methyl)-1,3-dihydrofuro[3,4-c]quinoline-8-carboxamide;
(3R)-4-amino-3-methyl-N-((1R,2R)-2-(trifluoromethyl)cyclopropyl)-N-((5-(trifluoromethyl)-2-pyridinyl)methyl)-1,3-dihydrofuro[3,4-c]quinoline-8-carboxamide;
(3R)-4-amino-3-methyl-N-((1S,2 S)-2-(trifluoromethyl)cyclopropyl)-N-((5-(trifluoromethyl)-2-pyridinyl)methyl)-1,3-dihydrofuro[3,4-c]quinoline-8-carboxamide;
(3S)-4-amino-3-methyl-N-((1R,2R)-2-(trifluoromethyl)cyclopropyl)-N-((5-(trifluoromethyl)-2-pyridinyl)methyl)-1,3-dihydrofuro[3,4-c]quinoline-8-carboxamide;
(3 S)-4-amino-3-methyl-N-((1S,2S)-2-(trifluoromethyl)cyclopropyl)-N-((5-(trifluoromethyl)-2-pyridinyl)methyl)-1,3-dihydrofuro[3,4-c]quinoline-8-carboxamide;
(3R)-4-amino-3-methyl-N-((1R)-1-(2-pyrimidinyl)ethyl)-N-((5-(trifluoromethyl)-2-pyridinyl)methyl)-1,3-dihydrofuro[3,4-c]quinoline-8-carboxamide;
(3S)-4-amino-3-methyl-N-((1R)-1-(2-pyrimidinyl)ethyl)-N-((5-(trifluoromethyl)-2-pyridinyl)methyl)-1,3-dihydrofuro[3,4-c]quinoline-8-carboxamide;
(3R)-4-amino-N-((5-cyano-2-pyridinyl)methyl)-N-(5,6-dihydro-2H-pyran-3-ylmethyl)-3-methyl-1,3-dihydrofuro[3,4-c]quinoline-8-carboxamide;
(3S)-4-amino-N-((5-cyano-2-pyridinyl)methyl)-N-(5,6-dihydro-2H-pyran-3-ylmethyl)-3-methyl-1,3-dihydrofuro[3,4-c]quinoline-8-carboxamide;
(3R)-4-amino-N-((1-cyanocyclopropyl)methyl)-N-((5-cyano-2-pyridinyl)methyl)-3-methyl-1,3-dihydrofuro[3,4-c]quinoline-8-carboxamide;
(3S)-4-amino-N-((1-cyanocyclopropyl)methyl)-N-((5-cyano-2-pyridinyl)methyl)-3-methyl-1,3-dihydrofuro[3,4-c]quinoline-8-carboxamide;
(3R)-4-amino-N-((2-amino-1,3-thiazol-5-yl)methyl)-N-((5-cyano-2-pyridinyl)methyl)-3-methyl-1,3-dihydrofuro[3,4-c]quinoline-8-carboxamide;
(3S)-4-amino-N-((2-amino-1,3-thiazol-5-yl)methyl)-N-((5-cyano-2-pyridinyl)methyl)-3-methyl-1,3-dihydrofuro[3,4-c]quinoline-8-carboxamide;
(3R)-4-amino-N-(5,6-dihydro-2H-pyran-3-ylmethyl)-3-methyl-N-((5-(trifluoromethyl)-2-pyridinyl)methyl)-1,3-dihydrofuro[3,4-c]quinoline-8-carboxamide;
(3S)-4-amino-N-(5,6-dihydro-2H-pyran-3-ylmethyl)-3-methyl-N-((5-(trifluoromethyl)-2-pyridinyl)methyl)-1,3-dihydrofuro[3,4-c]quinoline-8-carboxamide;
(3R)-4-amino-3-methyl-N-(1H-pyrrolo[2,3-b]pyridin-4-ylmethyl)-N-((5-(trifluoromethyl)-2-pyridinyl)methyl)-1,3-dihydrofuro[3,4-c]quinoline-8-carboxamide;
(3S)-4-amino-3-methyl-N-(1H-pyrrolo[2,3-b]pyridin-4-ylmethyl)-N-((5-(trifluoromethyl)-2-pyridinyl)methyl)-1,3-dihydrofuro[3,4-c]quinoline-8-carboxamide;
(3R)-4-amino-N-((1-cyanocyclopropyl)methyl)-3-methyl-N-((5-(trifluoromethyl)-2-pyridinyl)methyl)-1,3-dihydrofuro[3,4-c]quinoline-8-carboxamide;
(3S)-4-amino-N-((1-cyanocyclopropyl)methyl)-3-methyl-N-((5-(trifluoromethyl)-2-pyridinyl)methyl)-1,3-dihydrofuro[3,4-c]quinoline-8-carboxamide;
(3R)-4-amino-N-((2-amino-1,3-thiazol-5-yl)methyl)-3-methyl-N-((5-(trifluoromethyl)-2-pyridinyl)methyl)-1,3-dihydrofuro[3,4-c]quinoline-8-carboxamide;
(3S)-4-amino-N-((2-amino-1,3-thiazol-5-yl)methyl)-3-methyl-N-((5-(trifluoromethyl)-2-pyridinyl)methyl)-1,3-dihydrofuro[3,4-c]quinoline-8-carboxamide;
(3R)-4-amino-3-methyl-N-(2-methylpropyl)-N-((5-(trifluoromethyl)-2-pyridinyl)methyl)-1,3-dihydrofuro[3,4-c]quinoline-8-carboxamide;
(3S)-4-amino-3-methyl-N-(2-methylpropyl)-N-((5-(trifluoromethyl)-2-pyridinyl)methyl)-1,3-dihydrofuro[3,4-c]quinoline-8-carboxamide;
(3R)-4-amino-N-((2R,3R)-3-hydroxy-2-butanyl)-3-methyl-N-((5-(trifluoromethyl)-2-pyridinyl)methyl)-1,3-dihydrofuro[3,4-c]quinoline-8-carboxamide;
(3R)-4-amino-N-((2R,3S)-3-hydroxy-2-butanyl)-3-methyl-N-((5-(trifluoromethyl)-2-pyridinyl)methyl)-1,3-dihydrofuro[3,4-c]quinoline-8-carboxamide;
(3R)-4-amino-N-((2S,3R)-3-hydroxy-2-butanyl)-3-methyl-N-((5-(trifluoromethyl)-2-pyridinyl)methyl)-1,3-dihydrofuro[3,4-c]quinoline-8-carboxamide;
(3R)-4-amino-N-((2S,3S)-3-hydroxy-2-butanyl)-3-methyl-N-((5-(trifluoromethyl)-2-pyridinyl)methyl)-1,3-dihydrofuro[3,4-c]quinoline-8-carboxamide;
(3S)-4-amino-N-((2R,3R)-3-hydroxy-2-butanyl)-3-methyl-N-((5-(trifluoromethyl)-2-pyridinyl)methyl)-1,3-dihydrofuro[3,4-c]quinoline-8-carboxamide;
(3S)-4-amino-N-((2R,3S)-3-hydroxy-2-butanyl)-3-methyl-N-((5-(trifluoromethyl)-2-pyridinyl)methyl)-1,3-dihydrofuro[3,4-c]quinoline-8-carboxamide;
(3S)-4-amino-N-((2S,3R)-3-hydroxy-2-butanyl)-3-methyl-N-((5-(trifluoromethyl)-2-pyridinyl)methyl)-1,3-dihydrofuro[3,4-c]quinoline-8-carboxamide;
(3S)-4-amino-N-((2S,3S)-3-hydroxy-2-butanyl)-3-methyl-N-((5-(trifluoromethyl)-2-pyridinyl)methyl)-1,3-dihydrofuro[3,4-c]quinoline-8-carboxamide;
(3R)-4-amino-N-((1R,3R)-3-hydroxycyclohexyl)-3-methyl-N-((5-(trifluoromethyl)-2-pyridinyl)methyl)-1,3-dihydrofuro[3,4-c]quinoline-8-carboxamide;
(3R)-4-amino-N-((1R,3S)-3-hydroxycyclohexyl)-3-methyl-N-((5-(trifluoromethyl)-2-pyridinyl)methyl)-1,3-dihydrofuro[3,4-c]quinoline-8-carboxamide;
(3R)-4-amino-N-((1S,3R)-3-hydroxycyclohexyl)-3-methyl-N-((5-(trifluoromethyl)-2-pyridinyl)methyl)-1,3-dihydrofuro[3,4-c]quinoline-8-carboxamide;
(3R)-4-amino-N-((1S,3S)-3-hydroxycyclohexyl)-3-methyl-N-((5-(trifluoromethyl)-2-pyridinyl)methyl)-1,3-dihydrofuro[3,4-c]quinoline-8-carboxamide;
(3S)-4-amino-N-((1R,3R)-3-hydroxycyclohexyl)-3-methyl-N-((5-(trifluoromethyl)-2-pyridinyl)methyl)-1,3-dihydrofuro[3,4-c]quinoline-8-carboxamide;
(3S)-4-amino-N-((1R,3S)-3-hydroxycyclohexyl)-3-methyl-N-((5-(trifluoromethyl)-2-pyridinyl)methyl)-1,3-dihydrofuro[3,4-c]quinoline-8-carboxamide;
(3S)-4-amino-N-((1S,3R)-3-hydroxycyclohexyl)-3-methyl-N-((5-(trifluoromethyl)-2-pyridinyl)methyl)-1,3-dihydrofuro[3,4-c]quinoline-8-carboxamide;
(3S)-4-amino-N-((1S,3S)-3-hydroxycyclohexyl)-3-methyl-N-((5-(trifluoromethyl)-2-pyridinyl)methyl)-1,3-dihydrofuro[3,4-c]quinoline-8-carboxamide;
(3R)-4-amino-N-(2-(cis-3-hydroxycyclobutyl)ethyl)-3-methyl-N-((5-(trifluoromethyl)-2-pyridinyl)methyl)-1,3-dihydrofuro[3,4-c]quinoline-8-carboxamide;
(3R)-4-amino-N-(2-(trans-3-hydroxycyclobutyl)ethyl)-3-methyl-N-((5-(trifluoromethyl)-2-pyridinyl)methyl)-1,3-dihydrofuro[3,4-c]quinoline-8-carboxamide;
(3S)-4-amino-N-(2-(cis-3-hydroxycyclobutyl)ethyl)-3-methyl-N-((5-(trifluoromethyl)-2-pyridinyl)methyl)-1,3-dihydrofuro[3,4-c]quinoline-8-carboxamide;
(3S)-4-amino-N-(2-(trans-3-hydroxycyclobutyl)ethyl)-3-methyl-N-((5-(trifluoromethyl)-2-pyridinyl)methyl)-1,3-dihydrofuro[3,4-c]quinoline-8-carboxamide;
(3R)-4-amino-N-((2R)-2-hydroxypropyl)-3-methyl-N-((5-(trifluoromethyl)-2-pyridinyl)methyl)-1,3-dihydrofuro[3,4-c]quinoline-8-carboxamide;
(3R)-4-amino-N-((2S)-2-hydroxypropyl)-3-methyl-N-((5-(trifluoromethyl)-2-pyridinyl)methyl)-1,3-dihydrofuro[3,4-c]quinoline-8-carboxamide;
(3S)-4-amino-N-((2R)-2-hydroxypropyl)-3-methyl-N-((5-(trifluoromethyl)-2-pyridinyl)methyl)-1,3-dihydrofuro[3,4-c]quinoline-8-carboxamide;
(3S)-4-amino-N-((2S)-2-hydroxypropyl)-3-methyl-N-((5-(trifluoromethyl)-2-pyridinyl)methyl)-1,3-dihydrofuro[3,4-c]quinoline-8-carboxamide;
(3R)-4-amino-N-((2R)-3-hydroxy-2-methylpropyl)-3-methyl-N-((5-(trifluoromethyl)-2-pyridinyl)methyl)-1,3-dihydrofuro[3,4-c]quinoline-8-carboxamide;
(3R)-4-amino-N-((2S)-3-hydroxy-2-methylpropyl)-3-methyl-N-((5-(trifluoromethyl)-2-pyridinyl)methyl)-1,3-dihydrofuro[3,4-c]quinoline-8-carboxamide;
(3S)-4-amino-N-((2R)-3-hydroxy-2-methylpropyl)-3-methyl-N-((5-(trifluoromethyl)-2-pyridinyl)methyl)-1,3-dihydrofuro[3,4-c]quinoline-8-carboxamide;
(3S)-4-amino-N-((2S)-3-hydroxy-2-methylpropyl)-3-methyl-N-((5-(trifluoromethyl)-2-pyridinyl)methyl)-1,3-dihydrofuro[3,4-c]quinoline-8-carboxamide;
(3R)-4-amino-3-methyl-N-((2R)-3,3,3-trifluoro-2-hydroxypropyl)-N-((5-(trifluoromethyl)-2-pyridinyl)methyl)-1,3-dihydrofuro[3,4-c]quinoline-8-carboxamide;
(3R)-4-amino-3-methyl-N-((2S)-3,3,3-trifluoro-2-hydroxypropyl)-N-((5-(trifluoromethyl)-2-pyridinyl)methyl)-1,3-dihydrofuro[3,4-c]quinoline-8-carboxamide;
(3S)-4-amino-3-methyl-N-((2R)-3,3,3-trifluoro-2-hydroxypropyl)-N-((5-(trifluoromethyl)-2-pyridinyl)methyl)-1,3-dihydrofuro[3,4-c]quinoline-8-carboxamide;
(3S)-4-amino-3-methyl-N-((2S)-3,3,3-trifluoro-2-hydroxypropyl)-N-((5-(trifluoromethyl)-2-pyridinyl)methyl)-1,3-dihydrofuro[3,4-c]quinoline-8-carboxamide;
(3R)-4-amino-3-methyl-N-((1-methyl-1H-1,2,4-triazol-3-yl)methyl)-N-((5-(trifluoromethyl)-2-pyridinyl)methyl)-1,3-dihydrofuro[3,4-c]quinoline-8-carboxamide;
(3S)-4-amino-3-methyl-N-((1-methyl-1H-1,2,4-triazol-3-yl)methyl)-N-((5-(trifluoromethyl)-2-pyridinyl)methyl)-1,3-dihydrofuro[3,4-c]quinoline-8-carboxamide;
(3R)-4-amino-3-methyl-N-((3R)-tetrahydro-3-furanylmethyl)-N-((5-(trifluoromethyl)-2-pyridinyl)methyl)-1,3-dihydrofuro[3,4-c]quinoline-8-carboxamide;
(3R)-4-amino-3-methyl-N-((3S)-tetrahydro-3-furanylmethyl)-N-((5-(trifluoromethyl)-2-pyridinyl)methyl)-1,3-dihydrofuro[3,4-c]quinoline-8-carboxamide;
(3S)-4-amino-3-methyl-N-((3R)-tetrahydro-3-furanylmethyl)-N-((5-(trifluoromethyl)-2-pyridinyl)methyl)-1,3-dihydrofuro[3,4-c]quinoline-8-carboxamide;
(3 S)-4-amino-3 -methyl-N-((3 S)-tetrahydro-3 -furanylmethyl)-N-((5-(trifluoromethyl)-2-pyridinyl)methyl)-1,3-dihydrofuro[3,4-c]quinoline-8-carboxamide;
(3R)-4-amino-N-((2R)-2-cyanopropyl)-3-methyl-N-((5-(trifluoromethyl)-2-pyridinyl)methyl)-1,3-dihydrofuro[3,4-c]quinoline-8-carboxamide;
(3R)-4-amino-N-((2S)-2-cyanopropyl)-3-methyl-N-((5-(trifluoromethyl)-2-pyridinyl)methyl)-1,3-dihydrofuro[3,4-c]quinoline-8-carboxamide;
(3 S)-4-amino-N-((2R)-2-cyanopropyl)-3-methyl-N-((5-(trifluoromethyl)-2-pyridinyl)methyl)-1,3-dihydrofuro[3,4-c]quinoline-8-carboxamide;
(3 S)-4-amino-N-((2 S)-2-cyanopropyl)-3-methyl-N-((5-(trifluoromethyl)-2-pyridinyl)methyl)-1,3-dihydrofuro[3,4-c]quinoline-8-carboxamide;
(3R)-4-amino-N,3-dimethyl-N-((5-(trifluoromethyl)-2-pyridinyl)methyl)-1,3-dihydrofuro[3,4-c]quinoline-8-carboxamide;
(3 S)-4-amino-N,3-dimethyl-N-((5-(trifluoromethyl)-2-pyridinyl)methyl)-1,3-dihydrofuro [3,4-c] quinoline-8-carboxamide;
(3R)-4-amino-N-ethyl-3-methyl-N-((5-(trifluoromethyl)-2-pyridinyl)methyl)-1,3-dihydrofuro[3,4-c]quinoline-8-carboxamide;
(3S)-4-amino-N-ethyl-3-methyl-N-((5-(trifluoromethyl)-2-pyridinyl)methyl)-1,3-dihydrofuro[3,4-c]quinoline-8-carboxamide;
(3R)-4-amino-3-methyl-N-(2-propanyl)-N-((5-(trifluoromethyl)-2-pyridinyl)methyl)-1,3-dihydrofuro[3,4-c]quinoline-8-carboxamide;
(3S)-4-amino-3-methyl-N-(2-propanyl)-N-((5-(trifluoromethyl)-2-pyridinyl)methyl)-1,3-dihydrofuro[3,4-c]quinoline-8-carboxamide;
4-amino-N,3,3-trimethyl-N-((5-(trifluoromethyl)-2-pyridinyl)methyl)-1,3-dihydrofuro [3,4-c] quinoline-8-carboxamide;
4-amino-N-ethyl-3,3-dimethyl-N-((5-(trifluoromethyl)-2-pyridinyl)methyl)-1,3-dihydrofuro[3,4-c]quinoline-8-carboxamide;
4-amino-3,3-dimethyl-N-(2-propanyl)-N-((5 -(trifluoromethyl)-2-pyridinyl)methyl)- 1,3 -dihydrofuro [3,4-c]quinoline-8-carboxamide;
4-amino-N-cyclopropyl-3,3-dimethyl-N-((5 -(trifluoromethyl)-2-pyridinyl)methyl)- 1,3 -dihydrofuro [3,4-c]quinoline-8-carboxamide; and
4-amino-N-((1R)-1-(3-cyano-5-(trifluoromethyl)-2-pyridinyl)ethyl)-7-fluoro-N,1-dimethyl-1H-pyrazolo[4,3-c]quinoline-8-carboxamide.

In another aspect, the compound can be selected from
(3R)-4-amino-N-ethyl-7-fluoro-3-methyl-N-((1R)-1-(6-(trifluoromethyl)-3-pyridazinyl)ethyl)-1,3-dihydrofuro[3,4-c]quinoline-8-carboxamide;
4-amino-N,1-dimethyl-N-((5-(trifluoromethyl)-2-pyridinyl)methyl)-1H-pyrazolo[4,3-c]quinoline-8-carboxamide;
4-amino-7-fluoro-N-((1R)-1-(3-fluoro-5-(trifluoromethyl)-2-pyridinyl)ethyl)-N,1-dimethyl-1H-pyrazolo[4,3-c]quinoline-8-carboxamide;
4-amino-N,1-dimethyl-N-(4-(pentafluoro-lambda~6~-sulfanyl)benzyl)-1H-pyrazolo[4,3-c][1,7]naphthyridine-8-carboxamide;
4-amino-7-fluoro-N,1-dimethyl-N-((1R)-1-(6-(trifluoromethyl)-3-pyridazinyl)ethyl)-1H-pyrazolo[4,3-c]quinoline-8-carboxamide;
(3R)-4-amino-7-fluoro-N,3-dimethyl-N-((1R)-1-(6-(trifluoromethyl)-3-pyridazinyl)ethyl)-1,3-dihydrofuro[3,4-c]quinoline-8-carboxamide;
4-amino-7-fluoro-N-methyl-N-((1R)-1-(6-(trifluoromethyl)-3-pyridazinyl)ethyl)-1,3-dihydrofuro[3,4-c]quinoline-8-carboxamide;
5-amino-N-(2-propanyl)-N-((5-(trifluoromethyl)-2-pyridinyl)methyl)pyrido[4,3-c] [1,7]naphthyridine-9-carboxamide;
4-amino-N-ethyl-7-fluoro-1-methyl-N-((5-(trifluoromethyl)-2-pyridinyl)methyl)-1H-pyrazolo[4,3-c]quinoline-8-carboxamide;
4-amino-7-chloro-N-((1R)-1-(5-fluoro-2-pyridinyl)ethyl)-N,1-dimethyl-1H-pyrazolo[4,3-c]quinoline-8-carboxamide;
4-amino-N-cyclobutyl-N-((6-(trifluoromethyl)-3 -pyridazinyl)methyl)- 1,3-dihydrofuro [3,4-c] quinoline-8-carboxamide;
4-amino-N-methyl-N-((5-(trifluoromethyl)-2-pyridinyl)methyl)-1,3-dihydrofuro [3,4-c] quinoline-8-carboxamide;
4-amino-N-cyclopropyl-1-methyl-N-((5-(trifluoromethyl)-2-pyridinyl)methyl)-1H-pyrazolo[4,3-c]quinoline-8-carboxamide;
4-amino-N-ethyl-7-fluoro-1-methyl-N-((6-(trifluoromethyl)-3-pyridazinyl)methyl)-1H-pyrazolo[4,3-c]quinoline-8-carboxamide;
4-amino-N-cyclopropyl-7-fluoro-1-methyl-N-((6-(trifluoromethyl)-3-pyridazinyl)methyl)-1H-pyrazolo[4,3-c]quinoline-8-carboxamide;
4-amino-7-fluoro-N,1-dimethyl-N-((1R)-1-(5-(trifluoromethyl)-2-pyridinyl)ethyl)-1H-pyrazolo[4,3-c]quinoline-8-carboxamide;
(3R)-4-amino-N-ethyl-3-methyl-N-((1R)-1-(6-(trifluoromethyl)-3-pyridazinyl)ethyl)-1,3-dihydrofuro[3,4-c]quinoline-8-carboxamide;
4-amino-N-cyclopropyl-7-fluoro-1-methyl-N-((5-(trifluoromethyl)-2-pyridinyl)methyl)-1H-pyrazolo[4,3-c]quinoline-8-carboxamide;
4-amino-7-fluoro-N,1-dimethyl-N-((5-(trifluoromethyl)-2-pyridinyl)methyl)-1H-pyrazolo[4,3-c]quinoline-8-carboxamide and
4-amino-N-ethyl-1-methyl-N-((5-(trifluoromethyl)-2-pyridinyl)methyl)-1H-pyrazolo[4,3-c]quinoline-8-carboxamide.

The reference to methods of treatments in the subsequent paragraphs of this description are to be interpreted as references to compounds, pharmaceutical compositions and medicaments of the present invention for use in a method for treatment of the human (or animal) body by therapy (or for diagnosis).

The invention further provides methods of treating cancer comprising administering to a subject an effective amount of the compound of the invention, the tautomer thereof, the stereoisomer thereof, or the pharmaceutically acceptable salt of any of the foregoing. In one aspect, the cancer is selected from ovarian, lung, lymphoid, glioblastoma, colon, melanoma, gastric, pancreatic or bladder cancer.

The invention further provides pharmaceutical compositions, comprising the compounds of the invention, the tautomer thereof, the stereoisomer thereof, or the pharmaceutically acceptable salt of any of the foregoing or a pharmaceutically acceptable salt thereof, and at least one pharmaceutically acceptable excipient.

The invention also provides methods of treating a cancer, the method comprising administering to a subject an effective amount of the compound of the invention, the tautomer thereof, the stereoisomer thereof, or the pharmaceutically acceptable salt of any of the foregoing. In one aspect, the cancer can be ovarian, lung, lymphoid, glioblastoma, colon, melanoma, gastric, pancreatic or bladder cancer.

The invention also provides the compound of the invention, the tautomer thereof, the stereoisomer thereof, or the pharmaceutically acceptable salt of any of the foregoing for use in a method of treating a cancer, the method comprising administering to a subject an effective amount of such compound. In one aspect, the cancer can be ovarian, lung, lymphoid, glioblastoma, colon, melanoma, gastric, pancreatic or bladder cancer.

The invention also provides the use of the compound of the present invention, the tautomer thereof, the stereoisomer thereof, or the pharmaceutically acceptable salt of any of the foregoing in the manufacture of a medicament for treating a cancer. In one aspect, the cancer is selected from ovarian, lung, lymphoid, glioblastoma, colon, melanoma, gastric, pancreatic or bladder cancer.

Other objects, features and advantages of the invention will become apparent to those skilled in the art from the following description and claims.

### DETAILED DESCRIPTION OF THE INVENTION

### Definitions

As used herein, if any variable occurs more than one time in a chemical formula, its definition on each occurrence is independent of its definition at every other occurrence. If the chemical structure and chemical name conflict, the chemical structure is determinative of the identity of the compound. The compounds of the present disclosure may contain one or more chiral centers and/or double bonds and therefore, may exist as stereoisomers, such as double-bond isomers (*i.e.,* geometric isomers), enantiomers, or diastereomers. Accordingly, any chemical structures within the scope of the specification depicted, in whole or in part, with a relative configuration encompass all possible enantiomers and stereoisomers of the illustrated compounds including the stereoisomerically pure form (*e.g*., geometrically pure, enantiomerically pure or diastereomerically pure) and enantiomeric and stereoisomeric mixtures. Enantiomeric and stereoisomeric mixtures can be resolved into the component enantiomers or stereoisomers using separation techniques or chiral synthesis techniques well known to the skilled artisan.

Certain compounds of the invention may possess asymmetric carbon atoms (optical centers) or double bonds; the racemates, enantiomers, diastereomers, geometric isomers and individual isomers are all intended to be encompassed within the scope of the invention. Furthermore, atropisomers and mixtures thereof such as those resulting from restricted rotation about two aromatic or heteroaromatic rings bonded to one another are intended to be encompassed within the scope of the invention. For example, when substituent is a phenyl group and is substituted with two groups bonded to the C atoms adjacent to the point of attachment to the N atom of the triazole, then rotation of the phenyl may be restricted. In some instances, the barrier of rotation is high enough that the different atropisomers may be separated and isolated.

As used herein and unless otherwise indicated, the term "stereoisomer" or "stereomerically pure" means one stereoisomer of a compound that is substantially free of other stereoisomers of that compound. For example, a stereomerically pure compound having one chiral center will be substantially free of the mirror image enantiomer of the compound. A stereomerically pure compound having two chiral centers will be substantially free of other diastereomers of the compound. A typical stereomerically pure compound comprises greater than about 80% by weight of one stereoisomer of the compound and less than about 20% by weight of other stereoisomers of the compound, more preferably greater than about 90% by weight of one stereoisomer of the compound and less than about 10% by weight of the other stereoisomers of the compound, even more preferably greater than about 95% by weight of one stereoisomer of the compound and less than about 5% by weight of the other stereoisomers of the compound, and most preferably greater than about 97% by weight of one stereoisomer of the compound and less than about 3% by weight of the other stereoisomers of the compound. If the stereochemistry of a structure or a portion of a structure is not indicated with, for example, bold or dashed lines, the structure or portion of the structure is to be interpreted as encompassing all stereoisomers of it. A bond drawn with a wavy line indicates that both stereoisomers are encompassed. This is not to be confused with a wavy line drawn perpendicular to a bond which indicates the point of attachment of a group to the rest of the molecule.

As known by those skilled in the art, certain compounds of the invention may exist in one or more tautomeric forms. Because one chemical structure may only be used to represent one tautomeric form, it will be understood that for convenience, referral to a compound of a given structural formula includes tautomers of the structure represented by the structural formula. Depending on the compound, some compounds may exist primarily in one form more than another. Also, depending on the compound and the energy required to convert one tautomer to the other, some compounds may exist as mixtures at room temperature whereas others may be isolated in one tautomeric form or the other. Examples of other tautomers associated with compounds of the invention are those with a pyridone group (a pyridinyl) for which hydroxypyridine is a tautomer and compounds with a ketone group with the enol tautomer. Examples of these are shown below.

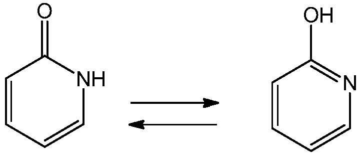

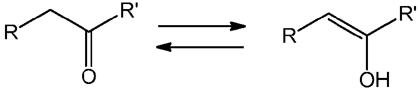

Compounds of the present disclosure include, but are not limited to, compounds of Formula I and all pharmaceutically acceptable forms thereof. Pharmaceutically acceptable forms of the compounds recited herein include pharmaceutically acceptable salts, solvates, crystal forms (including polymorphs and clathrates), chelates, non-covalent complexes, prodrugs, and mixtures thereof. In certain embodiments, the compounds described herein are in the form of pharmaceutically acceptable salts. As used herein, the term "compound" encompasses not only the compound itself, but also a pharmaceutically acceptable salt thereof, a solvate thereof, a chelate thereof, a non-covalent complex thereof, a prodrug thereof, and mixtures of any of the foregoing. In some embodiments, the term "compound" encompasses the compound itself, pharmaceutically acceptable salts thereof, tautomers of the compound, pharmaceutically acceptable salts of the tautomers, and ester prodrugs such as (C₁-C₄)alkyl esters. In other embodiments, the term "compound" encompasses the compound itself, pharmaceutically acceptable salts thereof, tautomers of the compound, pharmaceutically acceptable salts of the tautomers.

Pharmaceutically acceptable salts of the compounds of the present invention include acid addition salts formed with inorganic acids such as hydrochloric, hydrobromic, hydroiodic, phosphoric, metaphosphoric, nitric and sulfuric acids, and with organic acids, such as tartaric, acetic, trifluoroacetic, citric, malic, lactic, fumaric, benzoic, formic, propionic, glycolic, gluconic, maleic, succinic, camphorsulfuric, isothionic, mucic, gentisic, isonicotinic, saccharic, glucuronic, furoic, glutamic, ascorbic, anthranilic, salicylic, phenylacetic, mandelic, embonic (pamoic), methanesulfonic, ethanesulfonic, pantothenic, stearic, sulfinilic, alginic, galacturonic and arylsulfonic, for example benzenesulfonic and p-toluenesulfonic, acids; base addition salts formed with alkali metals and alkaline earth metals and organic bases such as N,N-dibenzylethylenediamine, chloroprocaine, choline, diethanolamine, ethylenediamine, meglumaine (N-methylglucamine), lysine and procaine; and internally formed salts. Suitable salts include those described in P. Heinrich Stahl, Camille G. Wermuth (Eds.), Handbook of Pharmaceutical Salts Properties, Selection and Use; 2002. Salts having a nonpharmaceutically acceptable anion or cation are within the scope of the invention as useful intermediates for the preparation of pharmaceutically acceptable salts and/or for use in non-therapeutic, for example, *in vitro,* situations.

The term "solvate" refers to the compound formed by the interaction of a solvent and a compound. Solvates of a compound includes solvates of all forms of the compound. In certain embodiments, solvents are volatile, non-toxic, and/or acceptable for administration to humans in trace amounts. Suitable solvates are pharmaceutically acceptable solvates, such as hydrates, including monohydrates and hemi-hydrates.

The compounds of the invention may also contain naturally occurring or unnatural proportions of atomic isotopes at one or more of the atoms that constitute such compounds. For example, the compounds may be radiolabeled with radioactive isotopes, such as for example tritium (³H), iodine-125 (¹²⁵I) or carbon-14 (¹⁴C). Radiolabeled compounds are useful as therapeutic or prophylactic agents, research reagents, *e.g.,* assay reagents, and diagnostic agents, *e.g., in vivo* imaging agents. All isotopic variations of the compounds of the invention, whether radioactive or not, are intended to be encompassed within the scope of the invention. For example, if a variable is said or shown to be H, this means that variable may also be deuterium (D) or tritium (T).

"Alkyl" refers to a saturated branched or straight-chain monovalent hydrocarbon group derived by the removal of one hydrogen atom from a single carbon atom of a parent alkane. Typical alkyl groups include, but are not limited to, methyl, ethyl, propyls such as propan-1-yl and propan-2-yl, butyls such as butan-1-yl, butan-2-yl, 2-methyl-propan-1-yl, 2-methyl-propan-2-yl, tert-butyl, and the like. In certain embodiments, an alkyl group comprises 1 to 20 carbon atoms. In some embodiments, alkyl groups include 1 to 10 carbon atoms or 1 to 6 carbon atoms whereas in other embodiments, alkyl groups include 1 to 4 carbon atoms. In still other embodiments, an alkyl group includes 1 or 2 carbon atoms. Branched chain alkyl groups include at least 3 carbon atoms and typically include 3 to 7, or in some embodiments, 3 to 6 carbon atoms. An alkyl group having 1 to 6 carbon atoms may be referred to as a (C₁-C₆)alkyl group and an alkyl group having 1 to 4 carbon atoms may be referred to as a (C₁-C₄)alkyl. This nomenclature may also be used for alkyl groups with differing numbers of carbon atoms. "Alkyl" also includes cycloalkyl, a group wherein the carbons are arranged in the form of the ring. Cycloalkyl includes, but not limited to cyclopropyl, cyclobytyl, cyclpentyl and cyclohexyl.

"Alkenyl" refers to an unsaturated branched or straight-chain hydrocarbon group having at least one carbon-carbon double bond derived by the removal of one hydrogen atom from a single carbon atom of a parent alkene. The group may be in either the Z- or E- form (*cis* or *trans*) about the double bond(s). Typical alkenyl groups include, but are not limited to, ethenyl; propenyls such as prop-1-en-1-yl, prop-1-en-2-yl, prop-2-en-1-yl (allyl), and prop-2-en-2-yl; butenyls such as but-1-en-1-yl, but-1-en-2-yl, 2-methyl-prop-1-en-1-yl, but-2-en-1-yl, but-2-en-1-yl, but-2-en-2-yl, buta-1,3-dien-1-yl, and buta-1,3-dien-2-yl; and the like. In certain embodiments, an alkenyl group has 2 to 20 carbon atoms and in other embodiments, has 2 to 6 carbon atoms. An alkenyl group having 2 to 6 carbon atoms may be referred to as a (C₂-C₆)alkenyl group. "Alkenyl" also includes cycloalkenyl. Cycloalkenyl refers to alkenyls that consist of three or more carbon atoms linked together with at least one carbon-carbon double bond to form a structural ring. Examples include but not limited to cyclopropenyl, cyclobutenyl, cyclopentenyl and cyclohexanyl.

"Alkynyl" refers to an unsaturated branched or straight-chain hydrocarbon having at least one carbon-carbon triple bond derived by the removal of one hydrogen atom from a single carbon atom of a parent alkyne. Typical alkynyl groups include, but are not limited to, ethynyl; propynyl; butynyl, 2-pentynyl, 3-pentynyl, 2-hexynyl, 3-hexynyl and the like. In certain embodiments, an alkynyl group has 2 to 20 carbon atoms and in other embodiments, has 2 to 6 carbon atoms. An alkynyl group having 2 to 6 carbon atoms may be referred to as a -(C₂-C₆)alkynyl group.

"Alkoxy" refers to a radical -OR where R represents an alkyl group as defined herein. Representative examples include, but are not limited to, methoxy, ethoxy, propoxy, butoxy, and the like. Typical alkoxy groups include 1 to 10 carbon atoms, 1 to 6 carbon atoms or 1 to 4 carbon atoms in the R group. Alkoxy groups that include 1 to 6 carbon atoms may be designated as -O-(C₁-C₆) alkyl or as -O-(C₁-C₆ alkyl) groups. In some embodiments, an alkoxy group may include 1 to 4 carbon atoms and may be designated as -O-(C₁-C₄) alkyl or as -O-(C₁-C₄ alkyl) groups group.

"Aryl" refers to a monovalent aromatic hydrocarbon group derived by the removal of one hydrogen atom from a single carbon atom of a parent aromatic ring system. Aryl encompasses monocyclic carbocyclic aromatic rings, for example, benzene. Aryl also encompasses bicyclic carbocyclic aromatic ring systems where each of the rings is aromatic, for example, naphthalene. Aryl groups may thus include fused ring systems where each ring is a carbocyclic aromatic ring. In certain embodiments, an aryl group includes 6 to 10 carbon atoms. Such groups may be referred to as C₆-C₁₀ aryl groups. Aryl, however, does not encompass or overlap in any way with heteroaryl as separately defined below. Hence, if one or more carbocyclic aromatic rings is fused with an aromatic ring that includes at least one heteroatom, the resulting ring system is a heteroaryl group, not an aryl group, as defined herein.

"Carbonyl" refers to the radical -C(O) which may also be referred to as -C(=O) group.

"Carboxy" refers to the radical -C(O)OH which may also be referred to as -C(=O)OH.

"Cyano" refers to the radical -CN.

"Cycloalkyl" refers to a saturated cyclic alkyl group derived by the removal of one hydrogen atom from a single carbon atom of a parent cycloalkane. Typical cycloalkyl groups include, but are not limited to, groups derived from cyclopropane, cyclobutane, cyclopentane, cyclohexane, cycloheptane, cyclooctane, and the like. Cycloalkyl groups may be described by the number of carbon atoms in the ring. For example, a cycloalkyl group having 3 to 8 ring members may be referred to as a (C₃-C₈)cycloalkyl, a cycloalkyl group having 3 to 7 ring members may be referred to as a (C₃-C₇)cycloalkyl and a cycloalkyl group having 4 to 7 ring members may be referred to as a (C₄-C₇)cycloalkyl. In certain embodiments, the cycloalkyl group can be a (C₃-C₁₀)cycloalkyl, a (C₃-C₈)cycloalkyl, a (C₃-C₇)cycloalkyl, a (C₃-C₆)cycloalkyl, or a (C₄-C₇)cycloalkyl group and these may be referred to as C₃-C₁₀ cycloalkyl, C₃-C₈ cycloalkyl, C₃-C₇ cycloalkyl, C₃-C₆ cycloalkyl, or C₄-C₇ cycloalkyl groups using alternative language.

"Heterocyclyl" refers to a cyclic group that includes at least one saturated, partially unsaturated, cyclic ring. Heterocyclyl groups include at least one heteroatom as a ring member. Typical heteroatoms include O, S and N and are independently chosen. Heterocyclyl groups include monocyclic ring systems and bicyclic ring systems. Bicyclic heterocyclyl groups include at least one non-aromatic ring with at least one heteroatom ring member that may be fused to a cycloalkyl ring or may be fused to an aromatic ring where the aromatic ring may be carbocyclic or may include one or more heteroatoms. The point of attachment of a bicyclic heterocyclyl group may be at the non-aromatic cyclic ring that includes at least one heteroatom or at another ring of the heterocyclyl group. For example, a heterocyclyl group derived by removal of a hydrogen atom from one of the 9 membered heterocyclic compounds shown below may be attached to the rest of the molecule at the 5-membered ring or at the 6-membered ring.

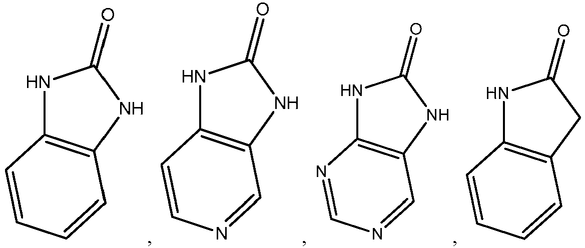

In some embodiments, a heterocyclyl group includes 5 to 10 ring members of which 1, 2, 3 or 4 or 1, 2, or 3 are heteroatoms independently selected from O, S, or N. In other embodiments, a heterocyclyl group includes 3 to 7 ring members of which 1, 2, or 3 heteroatom are independently selected from O, S, or N. In such 3-7 membered heterocyclyl groups, only 1 of the ring atoms is a heteroatom when the ring includes only 3 members and includes 1 or 2 heteroatoms when the ring includes 4 members. In some embodiments, a heterocyclyl group includes 3 or 4 ring members of which 1 is a heteroatom selected from O, S, or N. In other embodiments, a heterocyclyl group includes 5 to 7 ring members of which 1, 2, or 3 are heteroatoms independently selected from O, S, or N. Typical heterocyclyl groups include, but are not limited to, groups derived from epoxides, aziridine, azetidine, imidazolidine, morpholine, piperazine, piperidine, hexahydropyrimidine, 1,4,5,6-tetrahydropyrimidine, pyrazolidine, pyrrolidine, quinuclidine, tetrahydrofuran, tetrahydropyran, benzimidazolone, pyridinone, and the like. Heterocyclyl groups may be fully saturated but may also include one or more double bonds. Examples of such heterocyclyl groups include, but are not limited to, 1,2,3,6-tetrahydropyridinyl, 3,6-dihydro-2H-pyranyl, 3,4-dihydro-2H-pyranyl, 2,5-dihydro-1H-pyrolyl, 2,3-dihydro-1H-pyrolyl, 1H-azirinyl, 1,2-dihydroazetenyl, and the like. Substituted heterocyclyl also includes ring systems substituted with one or more oxo (=O) or oxide (-O⁻) substituents, such as piperidinyl N-oxide, morpholinyl-N-oxide, 1-oxo-1-thiomorpholinyl, pyridinonyl, benzimidazolonyl, benzo[d]oxazol-2(3H)-onyl, 3,4-dihydroisoquinolin-1(2H)-onyl, indolin-onyl, 1H-imidazo[4,5-c]pyridin-2(3H)-onyl, 7H-purin-8(9H)-onyl, imidazolidin-2-onyl, 1H-imidazol-2(3H)-onyl, 1,1-dioxo-1-thiomorpholinyl, and the like.

The term "comprising" is meant to be open ended, *i.e.,* all-encompassing and non-limiting. It may be used herein synonymously with "having" or "including". Comprising is intended to include each and every indicated or recited component or element(s) while not excluding any other components or elements.

"Disease" refers to any disease, disorder, condition, symptom, or indication.

"Halo" or "halogen" refers to a fluoro, chloro, bromo, or iodo group.

"Haloalkyl" refers to an alkyl group in which at least one hydrogen is replaced with a halogen. Thus, the term "haloalkyl" includes monohaloalkyl (alkyl substituted with one halogen atom) and polyhaloalkyl (alkyl substituted with two or more halogen atoms). Representative "haloalkyl" groups include difluoromethyl, 2,2,2-trifluoroethyl, 2,2,2-trichloroethyl, and the like. The term "perhaloalkyl" means, unless otherwise stated, an alkyl group in which each of the hydrogen atoms is replaced with a halogen atom. For example, the term "perhaloalkyl", includes, but is not limited to, trifluoromethyl, pentachloroethyl, 1,1,1-trifluoro-2-bromo-2-chloroethyl, and the like.

"Heteroaryl" refers to a monovalent heteroaromatic group derived by the removal of one hydrogen atom from a single atom of a parent heteroaromatic ring system. Heteroaryl groups typically include 5- to 14-membered, but more typically include 5- to 10-membered aromatic, monocyclic, bicyclic, and tricyclic rings containing one or more, for example, 1, 2, 3, or 4, or in certain embodiments, 1, 2, or 3, heteroatoms chosen from O, S, or N, with the remaining ring atoms being carbon. In monocyclic heteroaryl groups, the single ring is aromatic and includes at least one heteroatom. In some embodiments, a monocyclic heteroaryl group may include 5 or 6 ring members and may include 1, 2, 3, or 4 heteroatoms, 1, 2, or 3 heteroatoms, 1 or 2 heteroatoms, or 1 heteroatom where the heteroatom(s) are independently selected from O, S, or N. In bicyclic aromatic rings, both rings are aromatic. In bicyclic heteroaryl groups, at least one of the rings must include a heteroatom, but it is not necessary that both rings include a heteroatom although it is permitted for them to do so. For example, the term "heteroaryl" includes a 5- to 7-membered heteroaromatic ring fused to a carbocyclic aromatic ring or fused to another heteroaromatic ring. In tricyclic aromatic rings, all three of the rings are aromatic and at least one of the rings includes at least one heteroatom. For fused, bicyclic and tricyclic heteroaryl ring systems where only one of the rings contains one or more heteroatoms, the point of attachment may be at the ring including at least one heteroatom or at a carbocyclic ring. When the total number of S and O atoms in the heteroaryl group exceeds 1, those heteroatoms are not adjacent to one another. In certain embodiments, the total number of S and O atoms in the heteroaryl group is not more than 2. In certain embodiments, the total number of S and O atoms in the aromatic heterocycle is not more than 1. Heteroaryl does not encompass or overlap with aryl as defined above. Examples of heteroaryl groups include, but are not limited to, groups derived from acridine, carbazole, cinnoline, furan, imidazole, indazole, indole, indolizine, isobenzofuran, isochromene, isoindole, isoquinoline, isothiazole, 2H-benzo[d][1,2,3]triazole, isoxazole, naphthyridine, oxadiazole, oxazole, perimidine, phenanthridine, phenanthroline, phenazine, phthalazine, pteridine, purine, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, and the like. In certain embodiments, the heteroaryl group can be between 5 to 20 membered heteroaryl, such as, for example, a 5 to 14 membered or 5 to 10 membered heteroaryl. In certain embodiments, heteroaryl groups can be those derived from thiophene, pyrrole, benzothiophene, 2H-benzo[d][1,2,3]triazole benzofuran, indole, pyridine, quinoline, imidazole, benzimidazole, oxazole, tetrazole, and pyrazine.

"Pharmaceutically acceptable" refers to generally recognized for use in animals, and more particularly in humans.

"Pharmaceutically acceptable salt" refers to a salt of a compound that is pharmaceutically acceptable and that possesses the desired pharmacological activity of the parent compound.

"Pharmaceutically acceptable excipient" refers to a broad range of ingredients that may be combined with a compound or salt of the present invention to prepare a pharmaceutical composition or formulation. Typically, excipients include, but are not limited to, diluents, colorants, vehicles, anti-adherants, glidants, disintegrants, flavoring agents, coatings, binders, sweeteners, lubricants, sorbents, preservatives, and the like.

"Stereoisomer" refers to an isomer that differs in the arrangement of the constituent atoms in space. Stereoisomers that are mirror images of each other and optically active are termed "enantiomers," and stereoisomers that are not mirror images of one another and are optically active are termed "diastereomers."

"Subject" includes mammals and humans. The terms "human" and "subject" are used interchangeably herein.

"Therapeutically effective amount" refers to the amount of a compound that, when administered to a subject for treating a disease, or at least one of the clinical symptoms of a disease or disorder, is sufficient to affect such treatment for the disease, disorder, or symptom. As those skilled in the art will recognize. this amount is typically not limited to a single dose but may comprise multiple dosages over a significant period of time as required to bring about a therapeutic or prophylactic response in the subject. Thus, a "therapeutically effective amount" is not limited to the amount in a single capsule or tablet, but may include more than one capsule or tablet, which is the dose prescribed by a qualified physician or medical care provider. The "therapeutically effective amount" can vary depending on the compound, the disease, disorder, and/or symptoms of the disease or disorder, severity of the disease, disorder, and/or symptoms of the disease or disorder, the age of the subject to be treated, and/or the weight of the subject to be treated. An appropriate amount in any given instance can be readily apparent to those skilled in the art or capable of determination by routine experimentation.

"Treating" or "treatment" of any disease or disorder refers to arresting or ameliorating a disease, disorder, or at least one of the clinical symptoms of a disease or disorder, reducing the risk of acquiring a disease, disorder, or at least one of the clinical symptoms of a disease or disorder, reducing the development of a disease, disorder or at least one of the clinical symptoms of the disease or disorder, or reducing the risk of developing a disease or disorder or at least one of the clinical symptoms of a disease or disorder. "Treating" or "treatment" also refers to inhibiting the disease or disorder, either physically, (e.g., stabilization of a discernible symptom), physiologically, (e.g., stabilization of a physical parameter), or both, or inhibiting at least one physical parameter which may not be discernible to the subject. Further, "treating" or "treatment" refers to delaying the onset of the disease or disorder or at least symptoms thereof in a subject which may be exposed to or predisposed to a disease or disorder even though that subject does not yet experience or display symptoms of the disease or disorder.

In some aspects, the compound may be in a form of a salt. Such salts may be anhydrous or associated with water as a hydrate. In some embodiments, the compound may be in a neutral form as a base or an acid.

Also provided are pharmaceutical compositions that include the compound or the pharmaceutically acceptable salt thereof, the tautomer thereof, the pharmaceutically acceptable salt of the tautomer, the stereoisomer of any of the foregoing, or the mixture thereof according to any one of the examples and at least one pharmaceutically acceptable excipient, carrier or diluent. In some such examples, the compound or the pharmaceutically acceptable salt thereof, the tautomer thereof, the pharmaceutically acceptable salt of the tautomer, the stereoisomer of any of the foregoing, or the mixture thereof according to any one of the aspects is present in an amount effective for the treatment of PRMT5-dependent cancers. In some aspects, the pharmaceutical composition is formulated for oral delivery whereas in other embodiments, the pharmaceutical composition is formulated for intravenous delivery. In some embodiments, the pharmaceutical composition is formulated for oral administration once a day or QD, and in some such formulations is a tablet where the effective amount of the active ingredient ranges from 1 mg to 100 mg, from 5 mg to 80 mg, from 10 mg to 50 mg or from 15 to 30 mg.

In some aspects, the subject is a mammal. In some such aspects, the mammal is a rodent. In other aspects, the mammal is a canine. In still other embodiments, the subject is a primate and, in some such embodiments, is a human.

The pharmaceutical compositions or formulations for the administration of the compounds of this invention may conveniently be presented in unit dosage form and may be prepared by any of the methods well known in the art. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition, the active object compound is included in an amount sufficient to produce the desired effect upon the process or condition of diseases.

The compounds of the invention may be administered via oral, mucosal (including sublingual, buccal, rectal, nasal, or vaginal), parenteral (including subcutaneous, intramuscular, bolus injection, intra-arterial, or intravenous), transdermal, or topical administration. In some aspects, the compounds of the invention are administered via mucosal (including sublingual, buccal, rectal, nasal, or vaginal), parenteral (including subcutaneous, intramuscular, bolus injection, intra-arterial, or intravenous), transdermal, or topical administration. In other aspects, the compounds of the invention are administered via oral administration. In still other embodiments, the compounds of the invention are not administered via oral administration.

The compounds of the invention, the pharmaceutically acceptable salt thereof, the tautomer thereof, the pharmaceutically acceptable salt of the tautomer, the stereoisomer of any of the foregoing, or the mixture thereof may find use in treating a number of conditions.

Compounds and compositions described herein are generally useful for the inhibition of PRMT5. In some aspects, methods of treating PRMT5-mediated disorder in a subject are provided which comprise administering an effective amount of a compound described herein *(e.g.,* a compound of Formula I or a pharmaceutically acceptable salt thereof), to a subject in need of treatment. In certain aspects, the effective amount is a therapeutically effective amount. In certain aspects, the effective amount is a prophylactically effective amount. In certain aspects, the subject is suffering from a PRMT5-mediated disorder (*e.g*., a cancer, for example a lymphoma, breast cancer, or pancreatic cancer). In other aspects, the subject is susceptible to a PRMT5-mediated disorder (*e.g*., a cancer, for example a lymphoma, breast cancer, or pancreatic cancer).

As used herein, the term "PRMTS-mediated disorder" means any disease, disorder, or other pathological condition in which PRMT5 is known to play a role. Accordingly, in some aspects, the present disclosure relates to treating or lessening the severity of one or more diseases in which PRMT5 is known to play a role.

In some aspects, herein provided is a method of inhibiting PRMT5 activity in a subject in need thereof comprising administering to the subject an effective amount of a compound described herein (*e.g.,* a compound of Formula I, or a pharmaceutically acceptable salt thereof, or a pharmaceutical composition thereof.

In further aspects, a compound contemplated by the present invention is useful in treating a proliferative disorder, such as cancer. In certain embodiment, compounds described herein are useful for treating lymphoma. In some embodiments, the lymphoma is mantle cell lymphoma (MCL). In some embodiments, the lymphoma is acute myeloid lymphoma (AML). In some embodiments, the cancer compounds described herein are useful for treating pancreatic cancer. In some aspects, the cancer compounds described herein are useful for treating multiple myeloma (MM). In further embodiments, the cancer compounds described herein are useful for treating breast cancer. The breast cancer can be estrogen receptor negative (ER-) or the breast cancer can be progesterone receptor negative (PR-). In further embodiments, the breast cancer can be HER2 negative. In some embodiments, the breast cancer is estrogen receptor negative, progesterone receptor negative and HER2 negative, also referred to herein as "triple negative breast cancer".

In further aspects, a breast cancer can be a lobular carcinoma in situ (LCIS), a ductal carcinoma in situ (DCIS), an invasive ductal carcinoma (IDC), inflammatory breast cancer, Paget disease of the nipple, Phyllodes tumor, Angiosarcoma, adenoid cystic carcinoma, low-grade adenosquamous carcinoma, medullary carcinoma, mucinous carcinoma, papillary carcinoma, tubular carcinoma, metaplastic carcinoma, micropapary carcinoma, mixed carcinoma, or another breast cancer, including but not limited to triple negative, HER positive, estrogen receptor positive, progesterone receptor positive, HER and estrogen receptor positive, HER and progesterone receptor positive, estrogen and progesterone receptor positive, and HER and estrogen and progesterone receptor positive.

In one embodiment, compounds of the invention are useful for treating pancreatic cancer.

In another embodiment, compounds of the invention are useful for treating NSCLC (non-small cell lung carcinoma. In one embodiment, the NSCLC can be squamous NSCLC. In another embodiment, it can be adenocarcinoma.

In a further aspect, cancer can be GBM. In a further aspect, cancer can be mesothelioma. In one aspect, cancer can be bladder cancer. In another aspect, cancer can be esophageal cancer. In a further aspect, cancer can be melanoma. In one aspect, cancer can be DLBCL, HNSCC or cholangiocarcinoma.

In some aspects, one or more compounds described herein are useful for treating any PRMT5-mediated or PRMTS-responsive proliferative cell disorder, for example a cancer that is PRMT5 responsive.

In one aspect, a cancer that lacks p53 (*e.g.,* a p53 null cancer) is less sensitive to PRMT5 inhibition than a cancer that is p53 positive. Accordingly, a cancer that is PRMT5 responsive can be a p53 positive cancer. The term "p53 positive" refers to a cancer that does not lack p53 expression and/or activity. In some embodiments, one or more compounds described herein are useful for treating a p53 positive cancer. In some aspects, a greater amount of one or more compounds described herein may be required to treat a p53 negative cancer (*e.g*. , a p53 null cancer) than a p53 positive cancer.

In some aspects, the disclosure provides a method for identifying subjects having a cancer that is sensitive to treatment with a PRMT5 inhibitor. In some embodiments, the method comprises obtaining a sample from the subject; detecting the presence or absence of p53; and, identifying the subject as having a cancer that is sensitive to treatment with a PRMT5 inhibitor if p53 is present in the sample. Accordingly, in some embodiments, a subject having a p53 positive cancer is identified as a subject for treatment with a PRMT5 inhibitor. In some embodiments, the method further comprises administering to the subject a composition comprising a PRMT5 inhibitor.

In some embodiments, aspects of the disclosure relate to a method for identifying subjects having a cancer that is insensitive (or that has low sensitivity) to treatment with a PRMT5 inhibitor. In some embodiments, the method comprises obtaining a sample from the subject; detecting the presence or absence of p53; and, identifying the subject as having a cancer that is not sensitive (for example, a cancer that is less sensitive than a p53 positive cancer) to treatment with a PRMT5 inhibitor if p53 is absent from the sample (*e.g.,* if the cancer is a p53 null cancer). In some embodiments, a p53 negative cancer (*e.g., a* p53 null cancer) is treated with a PRMT5 inhibitor, but a greater amount of PRMT5 inhibitor may be required to treat the p53 negative cancer than a p53 positive cancer. However, in some embodiments, a subject having a p53 negative cancer (*e.g.* , a p53 null cancer) is treated with a therapeutic agent that is not a PRMT5 inhibitor.

By "sample" is meant any biological sample derived from the subject, includes but is not limited to, cells, tissues samples, body fluids (including, but not limited to, mucus, blood, plasma, serum, urine, saliva, and semen), cancer cells, and cancer tissues. Detection of the presence or absence of p53 in the sample may be achieved by any suitable method for detecting p53 nucleic acid or protein, for example, nucleic acid sequencing (*e.g.*, DNA or RNA sequencing), quantitative PCR, Western blotting, etc., or any combination of thereof.

It should be appreciated that in some aspects, one or more of the compounds described herein may be useful for treating other types of cancer, including, but not limited to, acoustic neuroma, adenocarcinoma, adrenal gland cancer, anal cancer, angiosarcoma (*e.g*., lymphangiosarcoma, lymphangioendotheliosarcoma, hemangio sarcoma), appendix cancer, benign monoclonal gammopathy, biliary cancer (e.g. , cholangiocarcinoma), bladder cancer, brain cancer (*e.g*., meningioma; glioma, e.g. , astrocytoma, oligodendroglioma; medulloblastoma), bronchus cancer, carcinoid tumor, cervical cancer (e.g. , cervical adenocarcinoma), choriocarcinoma, chordoma, craniopharyngioma, colorectal cancer (e.g., colon cancer, rectal cancer, colorectal adenocarcinoma), epithelial carcinoma, ependymoma, endothelio sarcoma (e.g., Kaposi's sarcoma, multiple idiopathic hemorrhagic sarcoma), endometrial cancer (e.g., uterine cancer, uterine sarcoma), esophageal cancer (e.g. , adenocarcinoma of the esophagus, Barrett' s adenocarinoma), Ewing sarcoma, eye cancer (e.g., intraocular melanoma, retinoblastoma), familiar hypereosinophilia, gall bladder cancer, gastric cancer (e.g. , stomach adenocarcinoma), gastrointestinal stromal tumor (GIST), head and neck cancer (e.g., head and neck squamous cell carcinoma, oral cancer (e.g., oral squamous cell carcinoma (OSCC), throat cancer (e.g., laryngeal cancer, pharyngeal cancer, nasopharyngeal cancer, oropharyngeal cancer)), hematopoietic cancers (e.g., leukemia such as acute lymphocytic leukemia (ALL) (e.g., B-cell ALL, T-cell ALL), acute myelocytic leukemia (AML) (e.g. , B-cell AML, T-cell AML), chronic myelocytic leukemia (CML) (e.g. , B-cell CML, T-cell CML), and chronic lymphocytic leukemia (CLL) (e.g. , B-cell CLL, T-cell CLL), follicular lymphoma, chronic lymphocytic leukemia/small lymphocytic lymphoma (CLL/SLL), marginal zone B-cell lymphomas (e.g. , mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (e.g., "Waldenstrom's macro globulinemia"), hairy cell leukemia (HCL), immunoblastic large cell lymphoma, precursor B -lymphoblastic lymphoma and primary central nervous system (CNS) lymphoma; and T-cell NHL such as precursor T-lymphoblastic lymphoma/leukemia, peripheral T-cell lymphoma (PTCL) (e.g., cutaneous T-cell lymphoma (CTCL) (e.g. , mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma); a mixture of one or more leukemia/lymphoma as described above; and multiple myeloma (MM)), heavy chain disease (e.g., alpha chain disease, gamma chain disease, mu chain disease), hemangioblastoma, inflammatory myofibroblastic tumors, immunocytic amyloidosis, kidney cancer (e.g., nephroblastoma a.k.a. Wilms' tumor, renal cell carcinoma), liver cancer (e.g. , hepatocellular cancer (HCC), malignant hepatoma), lung cancer (e.g., bronchogenic carcinoma, small cell lung cancer (SCLC), non-small cell lung cancer (NSCLC), adenocarcinoma of the lung), leiomyosarcoma (LMS), mastocytosis (e.g. , systemic mastocytosis), myelodysplasia syndrome (MDS), mesothelioma, myeloproliferative disorder (MPD) (e.g., polycythemia Vera (PV), essential thrombocytosis (ET), agnogenic myeloid metaplasia (AMM) a.k.a. myelofibrosis (MF), chronic idiopathic myelofibrosis, chronic myelocytic leukemia (CML), chronic neutrophilic leukemia (CNL), hypereosinophilic syndrome (HES)), neuroblastoma, neurofibroma (e.g. , neurofibromatosis (NF) type 1 or type 2, schwannomatosis), neuroendocrine cancer (e.g., gastroenteropancreatic neuroendoctrine tumor (GEP-NET), carcinoid tumor), osteosarcoma, ovarian cancer (e.g. , cystadenocarcinoma, ovarian embryonal carcinoma, ovarian adenocarcinoma), papillary adenocarcinoma, penile cancer (e.g., Paget' s disease of the penis and scrotum), pinealoma, primitive neuroectodermal tumor (PNT), prostate cancer (e.g., prostate adenocarcinoma), rectal cancer, rhabdomyosarcoma, salivary gland cancer, skin cancer (e.g. , squamous cell carcinoma (SCC), keratoacanthoma (KA), melanoma, basal cell carcinoma (BCC)), small bowel cancer (e.g. , appendix cancer), soft tissue sarcoma (*e.g.*, malignant fibrous histiocytoma (MFH), liposarcoma, malignant peripheral nerve sheath tumor (MPNST), chondrosarcoma, fibrosarcoma, myxosarcoma), sebaceous gland carcinoma, sweat gland carcinoma, synovioma, testicular cancer (*e.g.*, seminoma, testicular embryonal carcinoma), thyroid cancer (*e.g.*, papillary carcinoma of the thyroid, papillary thyroid carcinoma (PTC), medullary thyroid cancer), urethral cancer, vaginal cancer and vulvar cancer (*e.g*. , Paget' s disease of the vulva).

In some aspects, the method of treating cancer in a subject comprises administering a composition comprising a PRMT5 inhibitor to the subject, wherein treatment with the PRMT5 inhibitor inhibits tumor growth of the cancer by more than about 25%, more than about 50%, more than about 75%, more than about 90% (e.g., 25%-50%, 50%-75%, 75%- 90%, or 90%-100% for example). In some embodiments, the method of treating cancer in a subject comprises administering a composition comprising a PRMT5 inhibitor to the subject, wherein methyl mark of the cancer is reduced more than about 50%, more than about 75%, more than about 80% (e.g., 50%-75%, 50%-80%, 80%-90%, 80%-100%, or 90%-100% for example). A methyl mark refers to protein methylation, for example a histone methylation (e.g., methylation of one or more lysines and/or arginines of a histone protein), or DNA methylation (e.g., epigenetic DNA methylation, for example methylated CpG sites). In some embodiments, the methyl mark level of a cell is a measure of the extent to which histones are methylated in the cell (*e.g.,* at one or more particular lysine and/or arginine positions).

Some methods of the invention comprise the administration of a compound of the invention and an additional therapeutic agent (*i.e.,* a therapeutic agent other than a compound of the invention). Thus, the compounds of the invention can be used in combination with at least one other therapeutic agent. Examples of additional therapeutic agents include, but are not limited to, antibiotics, anti-emetic agents, antidepressants, antifungal agents, anti-inflammatory agents, antineoplastic agents, antiviral agents, cytotoxic agents, and other anticancer agents, immunomodulatory agents, alpha-interferons, β-interferons, alkylating agents, hormones, and cytokines. In one embodiment, the invention encompasses administration of an additional therapeutic agent that is used to treat subjects with chronic heart failure or hypertension.

As described above some methods of the invention comprise the administration of a compound of the invention and an additional therapeutic agent (*i.e.,* a therapeutic agent other than a compound of the invention). In some embodiments, the invention encompasses administration of an additional therapeutic agent that is used to treat subjects with acceptable salt thereof, the tautomer thereof, the pharmaceutically acceptable salt of the tautomer, the stereoisomer of any of the foregoing, or the mixture thereof and an additional therapeutic agent such as an inhibitor of the funny current. In some embodiments, the method of use may include two or more additional therapeutic agents.

The invention is further described by reference to the following examples, which are intended to exemplify the claimed invention but not to limit it in any way.

### EXAMPLES

Unless otherwise noted, all materials were obtained from commercial suppliers and were used without further purification.

The following abbreviations are used to refer to various reagents and solvents:

| | |
|---|---|
| AcOH | acetic acid |
| aq or aq. | aqueous |
| Boc | *tert*-butyloxycarbonyl |
| CLND | chemiluminescent nitrogen detection |
| CMPI | 2-Chloro-1-methylpyridinium iodide |
| DAD | diode array detector |
| DCE | 1,2-dichloroethane |
| DCM | dichloromethane |
| DEA | diethylamine |
| DIAD | diisopropyl azodicarboxylate |
| DMA or DMAc | *N,N*-dimethylacetamide |
| DMF | *N,N*-dimethylformamide |
| DMSO | dimethyl sulfoxide |
| dppf | 1,1'-bis(diphenylphosphino)ferrocene |
| EDC·HCl or EDCI | 3-((ethylimino)methyleneamino)-*N,N*-dimethylpropan-1-amonium chloride |
| ESI or ES | electrospray ionization |
| Et | ethyl |
| Et₂O | diethyl ether |
| EtOH | ethyl alcohol |
| EtOAc | ethyl acetate |
| g | grams |
| h | hour |
| HPLC | high pressure liquid chromatography |
| HATU | 1-[bis(dimethylamino)methylene]-1*H*-1,2,3-triazolo[4,5-*b*]pyridinium 3-oxid hexafluorophosphate |
| HBTU | N,N,N',N'-Tetramethyl-O-(1H-benzotriazol-1-yl)uronium hexafluorophosphate, O-(Benzotriazol-1-yl)-N,N,N',N'-tetramethyluronium hexafluorophosphate |
| HOAt | 1-hydroxy-7-azabenzotriazole |
| iPr | isopropyl |
| *i*Pr₂NEt or DIPEA | *N*-ethyl diisopropylamine (Hünig's base) |
| LC MS, LCMS, LC-MS or LC/MS | liquid chromatography mass spectroscopy |
| LG | leaving group (e.g., halogen, mesylate, triflate) |
| LiHMDS | lithium bis(trimethylsilyl)amide |
| m/z | mass divided by charge |
| Me | methyl |
| MeCN/ACN | acetonitrile |
| MeOH | methanol |
| Met | metal species for cross-coupling (e.g., MgX, ZnX, SnR₃, SiR₃, B(OR)₂) |
| mg | milligrams |
| min | minutes |
| mL | milliliters |
| MS | mass spectra |
| MsCl | methanesulfonyl chloride |
| MTBE | *tert-*butyl methyl ether |
| NMP | 1-methyl-2-pyrrolidine |
| *n*-BuLi | n-butyllithium |
| NMR | nuclear magnetic resonance |
| Pd₂(dba)₃ | tris(dibenzylideneacetone)dipalladium (0) |
| Pd(dppf)Cl₂·DCM | [1,1'-bis(diphenylphosphino)ferrocene]dichloropalladium(II), complex with DCM |
| Pd(PPh₃)₄ | tetrakis(triphenylphosphine)palladium(0) |
| Ph | phenyl |
| PG or Prot. group | protecting group |
| Prep | preparative |
| PyBrOP | bromotripyrrolidinophosphonium hexafluorophosphate |
| rbf | round-bottom flask |
| RP-HPLC | reverse phase high pressure liquid chromatography |
| RT or rt | room temperature |
| R.T. | retention time |
| RuPhos | 2-dicyclohexylphosphino-2',6'-diisopropoxybiphenyl |
| sat. or sat'd | saturated |
| SFC | supercritical fluid chromatography |
| *t*-BuOH | *tert*-butanol |
| TEA or Et₃N | triethylamine |
| TEOS | tetraethyl orthosilicate |
| TFA | trifluoroacetic acid |
| THF | tetrahydrofuran |
| TBTU | N,N,N',N'-Tetramethyl-O-(benzotriazol-1-yl)uronium tetrafluoroborate |
| TOF | time of flight |
| UHPLC | ultra-high-performance liquid chromatography |
| Xantphos | 4,5-bis(diphenylphosphino)-9,9-dimethylxanthene |

### General Synthetic Schemes

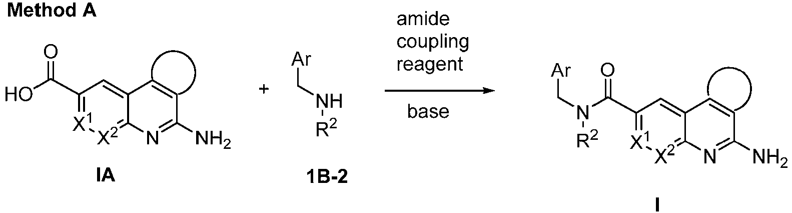

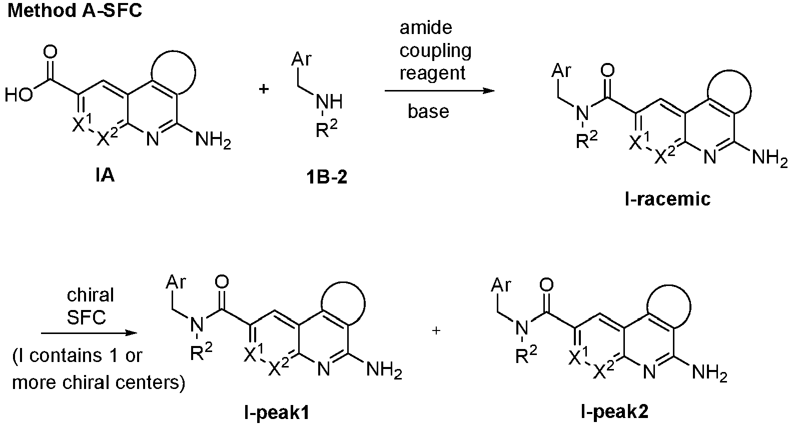

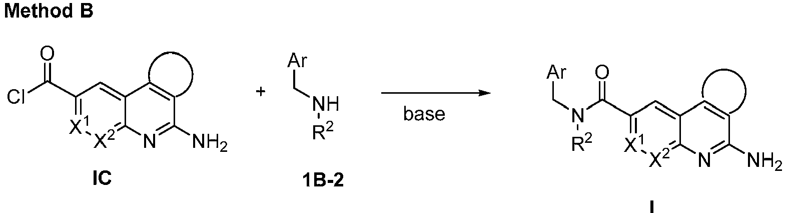

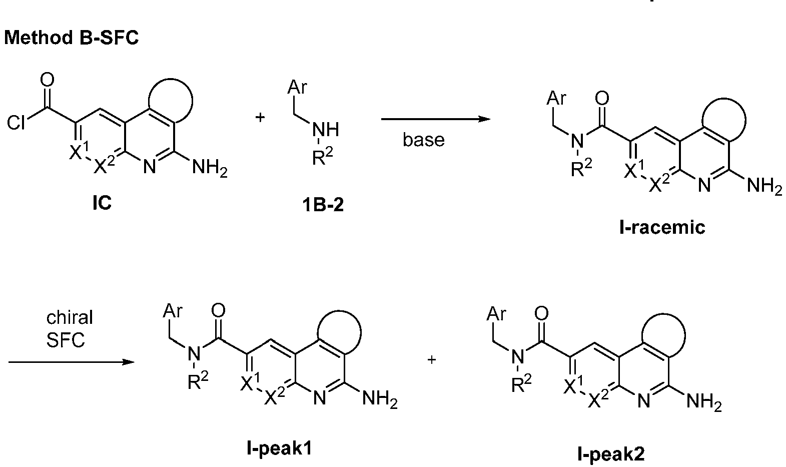

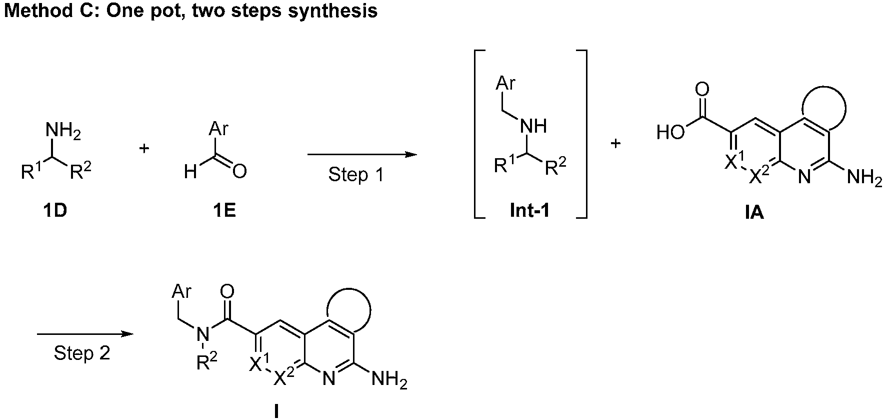

Method A: Compound **I** can be prepared from the reaction of acid **IA** and secondary amine **IB-1** in the presence of a base such as Et₃N or DIPEA, an activating reagent such as HATU or PyBrOP, in a solvent such as DMF or DMAc. If racemic amine or acid is employed in Method A, chiral SFC can be used to separate the stereoisomers, in which case stereochemistry was arbitrarily assigned to each isomer.
Method B: Compound **I** can be prepared from the reaction of acid chloride **IC** and secondary amine **IB** in the presence of a base such as Et₃N or DIPEA or pyridine, in a solvent such as THF or dioxane or DCM or DCE. Alternatively, compound **I** can be prepared from the reaction of acid chloride **IC** and secondary amine **IB** in the presence of DMAP in pyridine. If racemic amine or acid is employed in Method B, chiral SFC can be used to separate the stereoisomers, in which case stereochemistry was arbitrarily assigned to each isomer.
Method C: Compound **I** can be prepared by a small scale one pot, two step protocol as illustrated in General Scheme Method C. Primary amine **1D** can be combined with aldehyde **1E** in the method specified solvents and after imine formation and reduction will yield a secondary amine **(Int-1)** as a crude product. The secondary amine (**Int-1**) was reacted with acid **IA** with the specified coupling reagents to yield product **I** after HPLC purification.

### Analytical U/HPLC

The following equipment was used for analytical UHPLC:
Waters Acquity system equipped with an Acquity BEH C18 (1.7µm, 2.1 x 50 mm) with a linear gradient of a binary solvent system using a flow rate of 0.5 mL/min and DAD at ambient temperature, combined with MS detection SQD I. Linear gradients used (H₂O/CH₃CN/HCO₂H (95/5/0.1% to 0/100/0.1%)).
Agilent Infinity I/II -TOF6230B /CLND Antek 8060 equipped with Acquity BEH C18 (1.7µm, 2.1 x 50 mm) with a linear gradient of a binary solvent system using a flow rate of 0.75 mL/min combined with DAD. Linear gradients used (H₂O/MeOH/HCO₂H (95/5/0.1% to 0/100/0.1%)).

### Preparative HPLC

The following equipment was used for Prep-HPLC: Shimadzu Nexera X2 equipped with a Merck Chromolith SpeedROD RP-18E (5µm, 10 x 100 mm) with a linear gradient of a binary solvent system using a flow rate between 4 and 7 mL/min and UV detection at 254 nm, combined with MS detecting on a Shimadzu LCMS-2020. Linear gradients used (H₂O/MeOH/HCO₂H (95/5/0.1% to 0/100/0.1%)).

### Intermediates

### Intermediate 1: 6-(2,2,2-trifluoroethoxy)pyridazine-3-carbaldehyde

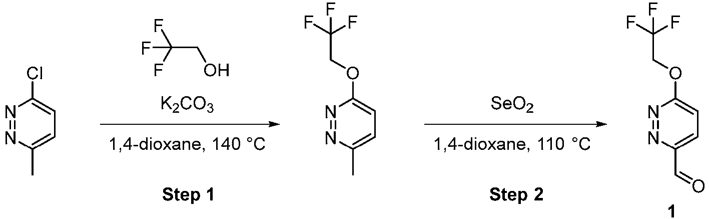

Step 1. A microwave vial was charged with 3-chloro-6-methylpyridazine (1.00 g, 7.78 mmol), potassium carbonate (2.150 g, 15.56 mmol), and 1,4-dioxane (14.0 mL). To the resulting suspension was added 2,2,2-trifluoroethanol (2.334 g, 1.704 mL, 23.34 mmol) and the mixture was heated to 140 °C in the microwave for 14 h. After cooling to 23 °C, the reaction mixture was transferred to a separatory funnel with CH₂Cl₂ (30 mL), H₂O (20 mL), and sat. aq. NH₄Cl (30 mL), the layers were separated, and the aqueous layer was extracted with CH₂Cl₂ (2 x 20 mL). The combined organic layers were dried over anhydrous Na₂SO₄, filtered, and concentrated to dryness. The resulting crude residue was purified by flash chromatography (0 to 100% 3:1 EtOAc:EtOH in heptane) to afford 3-methyl-6-(2,2,2-trifluoroethoxy)pyridazine (662 mg, 3.45 mmol, 44.3 % yield) as a light yellow solid. m/z (ESI): 193.2 (M+H)⁺.

Step 2. A vial was charged with 3-methyl-6-(2,2,2-trifluoroethoxy)pyridazine (662 mg, 3.45 mmol), selenium dioxide (612 mg, 5.51 mmol), and 1,4-dioxane (13.8 mL). The resulting mixture was sparged with nitrogen for 10 min, and the vial was subsequently heated to 110 °C. After 1.5 h, the reaction mixture was allowed to cool to 23 °C and was filtered over a 1 cm pad of Celite (30 mL 3:1 EtOAc:EtOH eluent) and concentrated to dryness. The resulting crude residue was purified by flash chromatograpy (0 to 50% 3:1 EtOAc:EtOH in heptane) to afford 6-(2,2,2-trifluoroethoxy)pyridazine-3-carbaldehyde (**1**, 154.7 mg, 0.751 mmol, 21.8 % yield) as a light yellow solid. m/z (ESI): 207.1 (M+H)⁺.

### Intermediate 2: 2-methyl-N-((5-(trifluoromethyl)pyridin-2-yl)methyl)propan-1-amine.

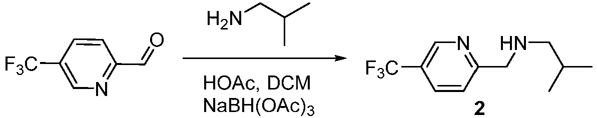

To a mixture of 5-(trifluoromethyl)picolinaldehyde (3.02 g, 17.27 mmol, AstaTech Inc) and isobutylamine (1.48 g, 20.21 mmol, Combi-Blocks Inc.) in DCM (50 mL) at RT was added acetic acid (1.11 g, 18.48 mmol). The mixture was stirred at RT for 30 min then treated with sodium triacetoxyborohydride (5.49 g, 25.9 mmol, Aldrich). The mixture was stirred at RT for 1 h then neutralized with sat'd aqueous Na₂CO₃ solution. The layers were separated, and the aqueous layer was extracted with DCM. The combined organic phase was dried over Na₂SO₄ and concentrated in vacuo. The crude material was purified by silica gel chromatography (0-100% EtOAc/EtOH (3/1) in heptane) to afford 2-methyl-N-((5-(trifluoromethyl)pyridin-2-yl)methyl)propan-1-amine (**2**, 2.81 g, 70% yield) as a brown oil. *m*/*z* (ESI): 233.0 (M+H)⁺. ¹H NMR (400 MHz, CHLOROFORM-d) δ ppm 8.81 (s, 1 H), 7.88 (dd, *J*=8.1, 2.1 Hz, 1 H), 7.50 (d, *J*=8.1 Hz, 1 H), 3.98 (s, 2 H), 2.46 (d, *J*=6.6 Hz, 2 H), 1.73 - 1.84 (m, 2 H), 0.94 (d, *J*=6.6 Hz, 6 H). ¹⁹F NMR (376 MHz, CHLOROFORM-*d*) δ ppm -62.26 (s, 3 F).

Secondary amines in Table 1 were prepared in a manner similar to that described for Intermediate 2.

**Table 1**

| **Int. #** | **Chemical Structure** | **Name** | ***m*/*z* (ESI): (M+H)⁺** |
|---|---|---|---|
| **3** | 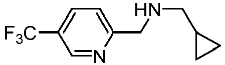 | 1-cyclopropyl-N-((5-(trifluoromethyl)pyridin-2-yl)methyl)methanamine | 231.0 |
| **4** | 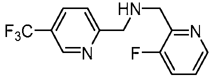 | 1-(3-fluoropyridin-2-yl)-N-((5-(trifluoromethyl)pyridin-2-yl)methyl)methanamine | 286.0 |
| **5** | 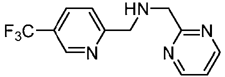 | 1-(pyrimidin-2-yl)-N-((5-(trifluoromethyl)pyridin-2-yl)methyl)methanamine | 269.0 |
| **6** | 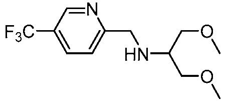 | 1,3-dimethoxy-N-((5-(trifluoromethyl)pyridin-2-yl)methyl)propan-2-amine | 279.2 |
| **7** | 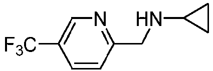 | N-((5-(trifluoromethyl)pyridin-2-yl)methyl)cyclopropanamine | 217 |
| **8** | 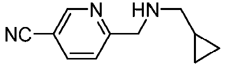 | 6-(((cyclopropylmethyl)amino)methyl)nicotin onitrile | 188 |
| **9** | 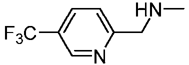 | N-methyl-1-(5-(trifluoromethyl)pyridin-2-yl)methanamine | 191.2 |
| **10** | 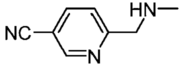 | 6-((methylamino)methyl)nicotinonitrile | 148.2 |
| **11** | 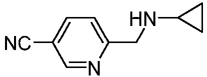 | 6-((cyclopropylamino)methyl)nicotinonitrile | 174.1 |
| **12** | 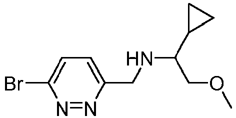 | N-((6-bromopyridazin-3-yl)methyl)-1-cyclopropyl-2-methoxyethan-1-amine | 286 and 288 |
| **13** | 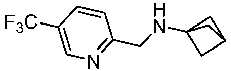 | N-((5-(trifluoromethyl)pyridin-2-yl)methyl)bicyclo[1.1.1]pentan-1-amine | 243.2 |
| **14** | 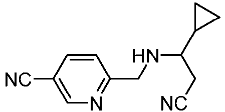 | 6-(((2-cyano-1-cyclopropylethyl)amino)methyl)nicotinonitr ile | 227.1 |
| **15** | 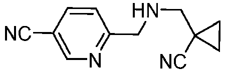 | 6-((((1-cyanocyclopropyl)methyl)amino)methyl)nic otinonitrile | 213.2 |
| **16** | 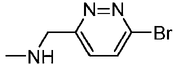 | 1-(6-bromopyridazin-3-yl)-N-methylmethanamine | 202 and 204 |
| **17** | 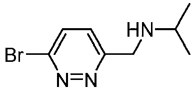 | N-((6-bromopyridazin-3-yl)methyl)propan-2-amine | 230 and 232 |
| **18** | 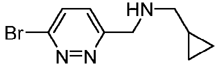 | 1-(6-bromopyridazin-3-yl)-N-(cyclopropylmethyl)methanamine | 242.0 |
| **19** | 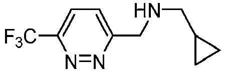 | 1-cyclopropyl-N-((6-(trifluoromethyl)pyridazin-3-yl)methyl)methanamine | 232.1 |
| **20** | 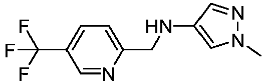 | 1-methyl-N-((5-(trifluoromethyl)pyridin-2-yl)methyl)-1H-pyrazol-4-amine | 257.2 |
| **21** | 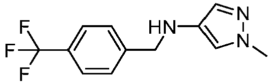 | 1-methyl-N-(4-(trifluoromethyl)benzyl)-1H-pyrazol-4-amine | 256.2 |
| **22** | 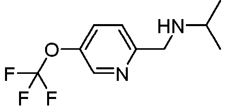 | N-((5-(trifluoromethoxy)pyridin-2-yl)methyl)propan-2-amine | 235.0 |
| **23** | 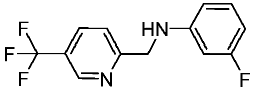 | 3-fluoro-N-((5-(trifluoromethyl)pyridin-2-yl)methyl)aniline | 271.2 |
| **24** | 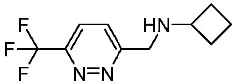 | N-((6-(trifluoromethyl)pyridazin-3-yl)methyl)cyclobutanamine | 232.2 |
| **25** | 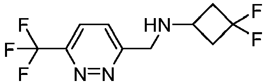 | 3,3-difluoro-N-((6-(trifluoromethyl)pyridazin-3-yl)methyl)cyclobutan-1-amine | 268.2 |
| **26** | 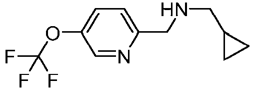 | 1-cyclopropyl-N-((5-(trifluoromethoxy)pyridin-2-yl)methyl)methanamine | 247.1 |
| **27** | 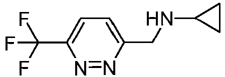 | N-((6-(trifluoromethyl)pyridazin-3-yl)methyl)cyclopropanamine | 218.2 |
| **28** | 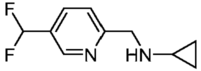 | N-((5-(difluoromethyl)pyridin-2-yl)methyl)cyclopropanamine | 199.1 |
| **29** | 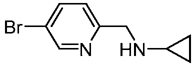 | N-((5-bromopyridin-2-yl)methyl)cyclopropanamine | 227.0 |
| **30** | 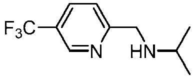 | N-((5-(trifluoromethyl)pyridin-2-yl)methyl)propan-2-amine | 219.1 |
| **31** | 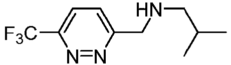 | 2-methyl-N-((6-(trifluoromethyl)pyridazin-3-yl)methyl)propan-1-amine | 234.2 |
| **32** | 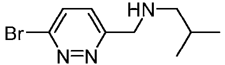 | N-((6-bromopyridazin-3-yl)methyl)-2-methylpropan-1-amine | 244 and 246 |
| **33** | 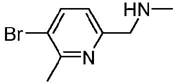 | 1-(5-bromo-6-methylpyridin-2-yl)-N-methylmethanamine | 215.0 and 216.9 |
| **34** | 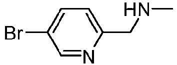 | 1-(5-bromopyridin-2-yl)-N-methylmethanamine | 200.9 and 203.0 |
| **35** | 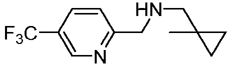 | 1-(1-methylcyclopropyl)-N-((5-(trifluoromethyl)pyridin-2-yl)methyl)methanamine | 245.1 |
| **36** | 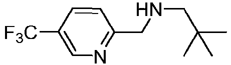 | 2,2-dimethyl-N-((5-(trifluoromethyl)pyridin-2-yl)methyl)propan-1-amine | 247.2 |
| **37** | 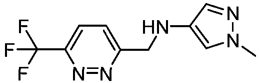 | 1-methyl-N-((6-(trifluoromethyl)pyridazin-3-yl)methyl)-1H-pyrazol-4-amine | 258.1 |
| **38** | 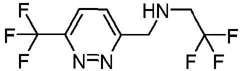 | 2,2,2-trifluoro-N-((6-(trifluoromethyl)pyridazin-3-yl)methyl)ethan-1-amine | 260.05 |
| **39** | 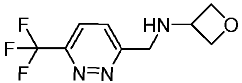 | N-((6-(trifluoromethyl)pyridazin-3-yl)methyl)oxetan-3-amine | 234.1 |
| **40** | 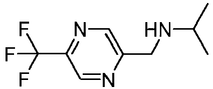 | N-((5-(trifluoromethyl)pyrazin-2-yl)methyl)propan-2-amine | 220.15 |
| **41** | 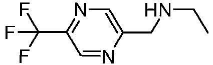 | N-((5-(trifluoromethyl)pyrazin-2-yl)methyl)ethanamine | 206.2 |
| **42** | 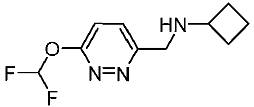 | N-((6-(difluoromethoxy)pyridazin-3-yl)methyl)cyclobutanamine | 230.2 |
| **43** | 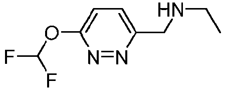 | N-((6-(difluoromethoxy)pyridazin-3-yl)methyl)ethanamine | 204.2 |
| **44** | 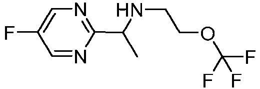 | 1-(5-fluoropyrimidin-2-yl)-N-(2-(trifluoromethoxy)ethyl)ethan-1-amine | 254.2 |
| **45** | 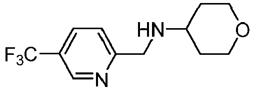 | N-((5-(trifluoromethyl)pyridin-2-yl)methyl)tetrahydro-2H-pyran-4-amine | 261.1 |
| **46** | 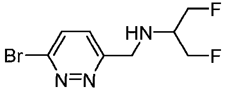 | N-((6-bromopyridazin-3-yl)methyl)-1,3-difluoropropan-2-amine | 266.0 and 268.0 |
| **47** | 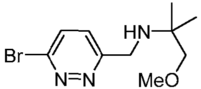 | N-((6-bromopyridazin-3-yl)methyl)-1-methoxy-2-methylpropan-2-amine | 274.0 and 276.0 |
| **48** | 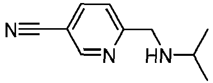 | 6-((isopropylamino)methyl)nicotinonitrile | |
| **49** | 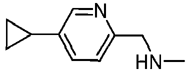 | 1-(5-cyclopropylpyridin-2-yl)-N-methylmethanamine | |
| **50** | 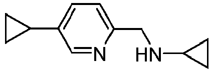 | N-((5-cyclopropylpyridin-2-yl)methyl)cyclopropanamine | |
| **51** | 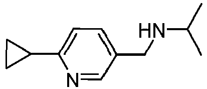 | N-((6-cyclopropylpyridin-3-yl)methyl)propan-2-amine | |
| **52** | 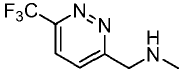 | N-methyl-1-(6-(trifluoromethyl)pyridazin-3-yl)methanamine | |
| **53** | 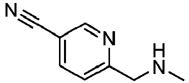 | 6-((methylamino)methyl)nicotinonitrile | |
| **54** | 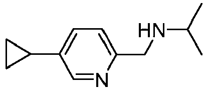 | N-((5-cyclopropylpyridin-2-yl)methyl)propan-2-amine | |
| **55** | 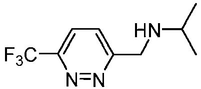 | N-((6-(trifluoromethyl)pyridazin-3-yl)methyl)propan-2-amine | |
| **56** | 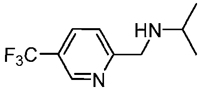 | N-((5-(trifluoromethyl)pyridin-2-yl)methyl)propan-2-amine | |
| **57** | 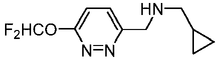 | 1-cyclopropyl-N-((6-(difluoromethoxy)pyridazin-3-yl)methyl)methanamine | 230.0 |
| **58** | 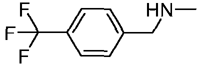 | N-methyl-1-(4-(trifluoromethyl)phenyl)methanamine | 190.0 |
| **59** | 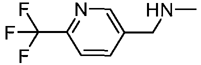 | N-methyl-1-(6-(trifluoromethyl)pyridin-3-yl)methanamine | 191.0 |
| **60** | 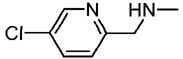 | 1-(5-chloropyridin-2-yl)-N-methylmethanamine | 179.0 (+Na) |
| **61** | 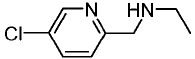 | N-((5-chloropyridin-2-yl)methyl)ethanamine | 171.0 |
| **62** | 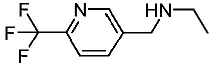 | N-((6-(trifluoromethyl)pyridin-3-yl)methyl)ethanamine | 205.0 |
| **63** | 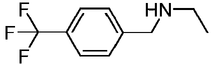 | N-(4-(trifluoromethyl)benzyl)ethanamine | 204.0 |
| **64** | 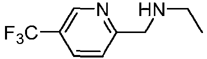 | N-((5-(trifluoromethyl)pyridin-2-yl)methyl)ethanamine | 205.0 |
| **65** | 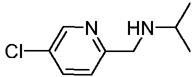 | N-((5-chloropyridin-2-yl)methyl)propan-2-amine | 185.0 |
| **66** | 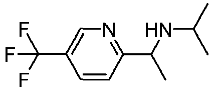 | N-(1-(5-(trifluoromethyl)pyridin-2-yl)ethyl)propan-2-amine | 233.0 |
| **67** | 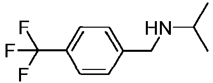 | N-(4-(trifluoromethyl)benzyl)propan-2-amine | 218.0 |
| **68** | 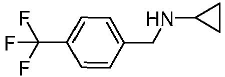 | N-(4-(trifluoromethyl)benzyl)cyclopropanamine | 216.0 |
| **69** | 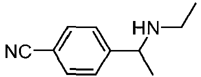 | 4-(1-(ethylamino)ethyl)benzonitrile | 175.0 |
| **70** | 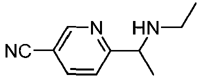 | 6-(1-(ethylamino)ethyl)nicotinonitrile | 176.0 |
| **71** | 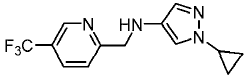 | 1-cyclopropyl-N-((5-(trifluoromethyl)pyridin-2-yl)methyl)-1H-pyrazol-4-amine | 283.0 |
| **72** | 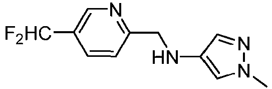 | N-((5-(difluoromethyl)pyridin-2-yl)methyl)-1-methyl-1H-pyrazol-4-amine | 239.2 |
| **73** | 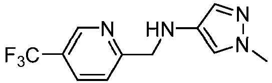 | 1-methyl-N-((5-(trifluoromethyl)pyridin-2-yl)methyl)-1H-pyrazol-4-amine | 257.2 |
| **74** | 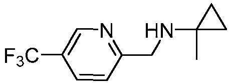 | 1-methyl-N-((5-(trifluoromethyl)pyridin-2-yl)methyl)cyclopropan-1-amine | 231.0 |
| **75** | 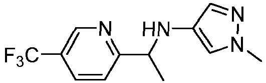 | 1-methyl-N-(1-(5-(trifluoromethyl)pyridin-2-yl)ethyl)-1H-pyrazol-4-amine | 271.2 |
| **76** | 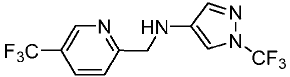 | 1-(trifluoromethyl)-N-((5-(trifluoromethyl)pyridin-2-yl)methyl)-1H-pyrazol-4-amine | 311.0 |
| **77** | 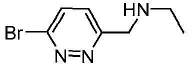 | *N*-((6-bromopyridazin-3-yl)methyl)ethanamine | 216.1 and 218.2 |
| **78** | 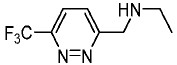 | *N*-((6-(trifluoromethyl)pyridazin-3-yl)methyl)ethanamine | 206.2 |
| **79** | 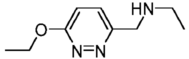 | *N*-((6-ethoxypyridazin-3-yl)methyl)ethanamine | 182.2 |
| **80** | 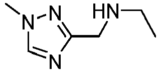 | *N*-((1-methyl-1H-1,2,4-triazol-3-yl)methyl)ethanamine | 141.05 |
| **81** | 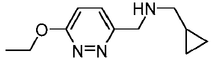 | 1-cyclopropyl-*N*-((6-ethoxypyridazin-3-yl)methyl)methanamine | 208.25 |
| **82** | 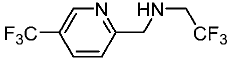 | 2,2,2-trifluoro-N-((5-(trifluoromethyl)pyridin-2-yl)methyl)ethan-1-amine | |
| **83** | 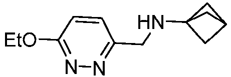 | N-((6-ethoxypyridazin-3-yl)methyl)bicyclo[1.1.1]pentan-1-amine | |
| **84** | 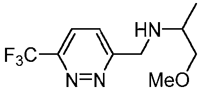 | 1-methoxy-N-((6-(trifluoromethyl)pyridazin-3-yl)methyl)propan-2-amine | |
| **85** | 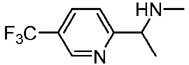 | N-methyl-1-(5-(trifluoromethyl)pyridin-2-yl)ethan-1-amine | 205.1 |
| **86** | 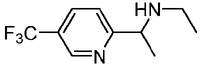 | N-ethyl-1-(5-(trifluoromethyl)pyridin-2-yl)ethan-1-amine | 219.1 |
| **87** | 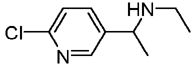 | 1-(6-chloropyridin-3-yl)-N-ethylethan-1-amine | 185.0 |
| **88** | 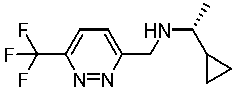 | (*R*)-1-cyclopropyl-N-((6-(trifluoromethyl)pyridazin-3-yl)methyl)ethan-1-amine | 246.1 |
| **89** | 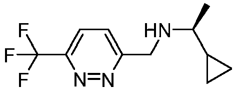 | (*S*)-1-cyclopropyl-N-((6-(trifluoromethyl)pyridazin-3-yl)methyl)ethan-1-amine | 246.3 |
| **90** | 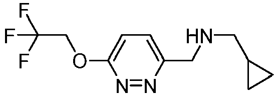 | 1-cyclopropyl-N-((6-(2,2,2-trifluoroethoxy)pyridazin-3-yl)methyl)methanamine | 262.2 |
| **91** | 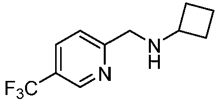 | N-((5-(trifluoromethyl)pyridin-2-yl)methyl)cyclobutanamine | 232.1 |
| **92** | 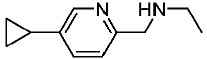 | N-((5-cyclopropylpyridin-2-yl)methyl)ethanamine | 177.2 |
| **93** | 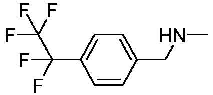 | N-methyl-1-(4-(perfluoroethyl)phenyl)methanamine | 240.1 |
| **94** | 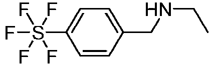 | N-(4-(pentafluoro-l6-sulfaneyl)benzyl)ethanamine | 262.0 |
| **95** | 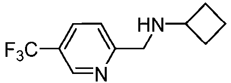 | N-((5-(trifluoromethyl)pyridin-2-yl)methyl)cyclobutanamine | 232.1 |
| **96** | 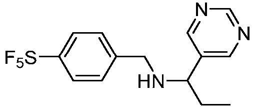 | N-(4-(pentafluoro-l6-sulfaneyl)benzyl)-1-(pyrimidin-5-yl)propan-1-amine | 354.2 |

### Intermediate 97: N-((5-(trifluoromethyl)pyridin-2-yl)methyl)-5,6,7,8-tetrahydro-[1,2,4]triazolo[1,5-a]pyridin-8-amine

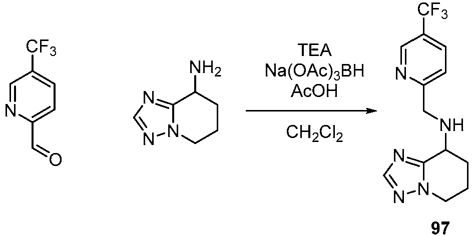

To a stirred mixture of 5-(trifluoromethyl)picolinaldehyde (534 mg, 3.05 mmol, Chemshuttle) and 5,6,7,8-tetrahydro[1,2,4]triazolo[1,5-a]pyridin-8-amine (430 mg, 3.11 mmol, Enamine) in DCM (8 mL) was added triethylamine (315 mg, 0.437 mL, 3.11 mmol, Sigma-Aldrich Corporation) followed, 5 min later, by glacial acetic acid (224 mg, 0.216 mL, 3.73 mmol, Sigma-Aldrich Corporation). The resulting mixture was stirred at rt for 15 min before sodium triacetoxyborohydride (857 mg, 4.05 mmol, Sigma-Aldrich Corporation) was added in one portion as a solid. The resulting mixture was stirred at rt for 25 min. The crude mixture was directly loaded onto a silica gel precolumn (25 g) and subjected to combi-flash column chromatography with a 24-g ISCO gold column eluting with MeOH (with 0.5 % ammonium hydroxide)/DCM (1-20%) to give, after azeotroping with toluene, N-((5-(trifluoromethyl)pyridin-2-yl)methyl)-5,6,7,8-tetrahydro-[1,2,4]triazolo[1,5-a]pyridin-8-amine (350 mg, 1.177 mmol, 37.8 % yield) as a colorless oil. *m*/*z* (ESI): 298.2 (M+H)⁺. ¹H NMR (400 MHz, CHLOROFORM-d) δ 8.78 (d, J=1.05 Hz, 1H), 7.81-7.89 (m, 2H), 7.50 (d, J=8.15 Hz, 1H), 4.09-4.29 (m, 4H), 3.97-4.06 (m, 2H), 2.12-2.33 (m, 2H), 1.83-2.04 (m, 2H). ¹⁹F NMR (376 MHz, CHLOROFORM-d) δ -62.34 (s, 3F).

Secondary amines in Table 2 were prepared in a manner similar to that described for Intermediate **97. 106-109** were derived from commercially available, chiral amines.

**Table 2**

| **Int. #** | **Chemical Structure** | **Name** | ***m*/*z* (ESI): (M+H)⁺** |
|---|---|---|---|
| **98** | 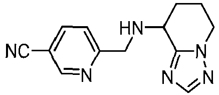 | 6-(((5,6,7,8-tetrahydro-[1,2,4]triazolo[1,5-a]pyridin-8-yl)amino)methyl)nicotinonitrile | 255.1 |
| **99** | 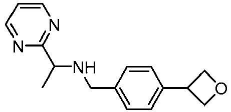 | N-(4-(oxetan-3-yl)benzyl)-1-(pyrimidin-2-yl)ethan-1-amine | 270.2 |
| **100** | 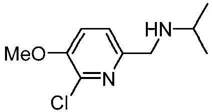 | N-((6-chloro-5-methoxypyridin-2-yl)methyl)propan-2-amine | 215.2 |
| **101** | 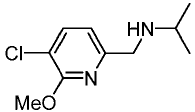 | N-((5-chloro-6-methoxypyridin-2-yl)methyl)propan-2-amine | 215.2 |
| **102** | 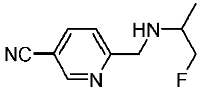 | 6-(((1-fluoropropan-2-yl)amino)methyl)nicotinonitrile | 194.2 |
| **103** | 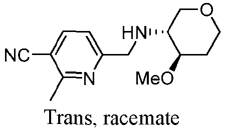 | 6-((((3R,4R)-4-methoxytetrahydro-2H-pyran-3-yl)amino)methyl)-2-methylnicotinonitrile, relative | 262.2 |
| **104** | 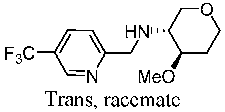 | (3R,4R)-4-methoxy-N-((5-(trifluoromethyl)pyridin-2-yl)methyl)tetrahydro-2H-pyran-3-amine, relative | 291.2 |
| **105** | 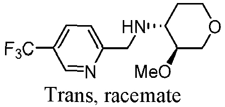 | (3S,4R)-3-methoxy-N-((5-(trifluoromethyl)pyridin-2-yl)methyl)tetrahydro-2H-pyran-4-amine, relative | 291.2 |
| **106** | 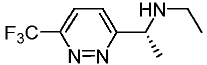 | (R)-N-ethyl-1-(6-(trifluoromethyl)pyridazin-3-yl)ethan-1-amine | 220.1 |
| **107** | 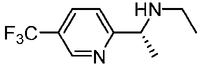 | (R)-N-ethyl-1-(5-(trifluoromethyl)pyridin-2-yl)ethan-1-amine | 219.1 |
| **108** | 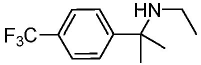 | N-ethyl-2-(4-(trifluoromethyl)phenyl)propan-2-amine | 232.1 |
| **109** | 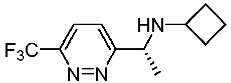 | (R)-N-(1-(6-(trifluoromethyl)pyridazin-3-yl)ethyl)cyclobutanamine | 246.0 |

### Intermediate 110: (R)-1-(pyrimidin-2-yl)-N-((5-(trifluoromethyl)pyridin-2-yl)methyl)ethan-1-amine.

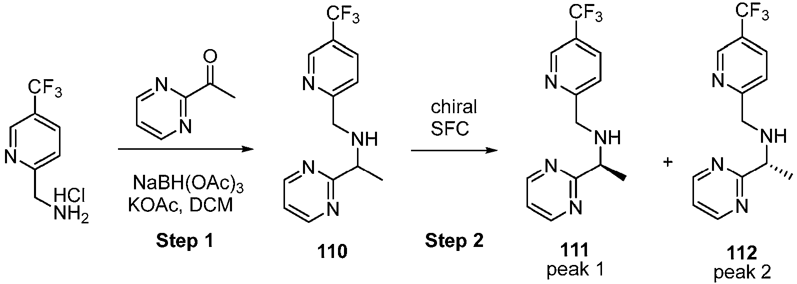

Step 1. To a stirred solution of (5-(trifluoromethyl)pyridin-2-yl)methanamine hydrochloride (115 g, 541 mmol) and 1-(pyrimidin-2-yl)ethan-1-one (76 g, 622 mmol) in DCM (3.5 L) was added potassium acetate (63.7 g, 649 mmol). The mixture was stirred for 30 min then treated with sodium triacetoxyborohydride (149 g, 703 mmol). After stirring for 1.5 h, the reaction mixture was diluted with water (2 L), treated with 1 N HCl (2 L), and extracted with DCM (1 L). The layers were separated. The aqueous layer was treated with 10% sodium hydroxide to adjust the pH to 12 and extracted with DCM (3 x 2 L). The combined organic layer was dried over Na₂SO₄, filtered and concentrated. The crude material was purified by silica gel chromatography (2% MeOH in DCM) to give 1-(pyrimidin-2-yl)-N-((5-(trifluoromethyl)pyridin-2-yl)methyl)ethan-1-amine (**101,** 97 g, 344 mmol, 63% yield) as a brown oil. *m*/*z* (ESI): 283.0 (M+H)⁺. ¹H NMR (400 MHz, Chloroform-d): δ ppm 8.81 (dt, *J =* 2.2, 1.0 Hz, 1 H), 8.74 (d, *J =* 4.9 Hz, 2 H), 7.88 (dd, *J =* 8.2, 2.3 Hz, 1 H), 7.54 (d, *J =* 8.2 Hz, 1 H), 7.20 (t, *J =* 4.9 Hz, 1 H), 4.10 (*q, J =* 6.8 Hz, 1 H), 3.94 (*d, J =* 2.9 Hz, 2 H), 1.53 (*d, J =* 6.8 Hz, 3 H).

Step 2. The racemic secondary amine **110** (44 g) was dissolved in 200 mL of MeOH and subjected to chiral SFC using a Chiralpak AD-H column (250 x30 mm, 5µ) with a mobile phase of 90% Liquid CO₂ and 10% EtOH with 0.5% DEA using a flowrate of 100 mL/ min. The 1^{st} eluting peak was (S)-1-(pyrimidin-2-yl)-N-((5-(trifluoromethyl)pyridin-2-yl)methyl)ethan-1-amine (**111,** 18 g, > 99% *ee*) and the 2^{nd} eluting peak was (R)-1-(pyrimidin-2-yl)-N-((5-(trifluoromethyl)pyridin-2-yl)methyl)ethan-1-amine (**112**, 19 g, > 99% *ee*)*.*

Racemic amines in Table 3 were prepared in a fashion similar to that described above for amine 110. The racemic amines were subjected to chiral SFC to provide enantiomerically pure amines (> 99% *ee*)*.*

**Table 3**

| **Secondary Amines** | **SFC Conditions** | ***m*/*z* (ESI): (M+H)⁺** |
|---|---|---|
| 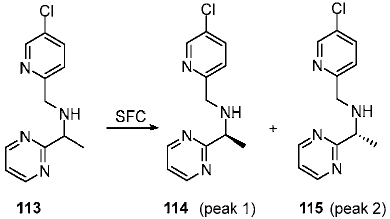 | Chiralpak AD-H column (250×30 mm, 5 µm) using a mobile phase of 75% Liquid CO₂ and 25% EtOH with 0.3% Et₂NH | 249.0 |
| 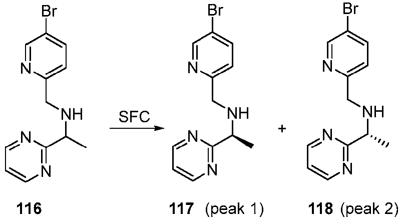 | ChiralPak AD-H column (250×30 mm, 5 µm) with a mobile phase of 70% Liquid CO₂ and 30% EtOH with 0.5% Et₂NH | 293/295 |
| 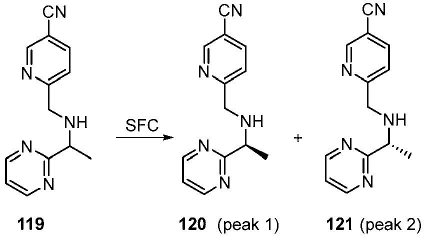 | Chiral Technologies AZ column (250x 21 mm, 5 µm) with a mobile phase of 85% Liquid CO₂ and 15% MeOH with 0.2% TEA | 240.0 |
| 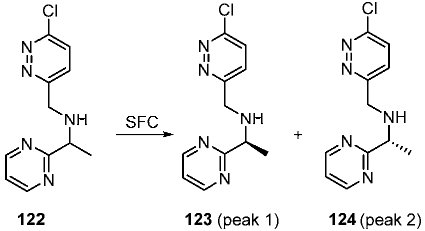 | Chiral Technologies AD column (250 x 21 mm, 5 µm)with a mobile phase 85% Liquid CO₂ and 15% MeOH with 1.0% TEA using a flowrate of 100 mL/min | 249.9 |

### Intermediate 125: (R)-N-((6-methoxypyridazin-3-yl)methyl)-1-(pyrimidin-2-yl)ethan-1-amine.

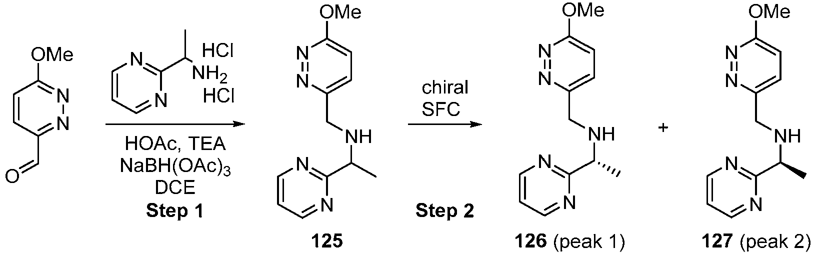

Step 1. A mixture of 6-methoxy-pyridazine-3-carbaldehyde (0.49 g, 3.54 mmol, Princeton BioMolecular Research, Inc.), 1-(pyrimidin-2-yl)ethan-1-amine dihydrochloride (0.73 g, 3.72 mmol, Enamine), 1,2-dichloroethane (30 mL), and acetic acid (0.22 mL, 3.90 mmol) was stirred at RT for 10 min, then sodium triacetoxyborohydride (1.013 g, 4.78 mmol) was added. The mixture was stirred at RT for 30 min then neutralized with saturated aqueous Na₂CO₃ solution. The crude was extracted with DCM. The organic phase was dried over Na₂SO₄ and concentrated in vacuo. The residue was purified by silica gel chromatography (30-100% EtOAc/EtOH (3/1) in heptane) to provide N-((6-methoxypyridazin-3-yl)methyl)-1-(pyrimidin-2-yl)ethan-1-amine (125, 0.84 g, 96% yield) as an orange oil. *m*/*z* (ESI): 246 (M+H)⁺.

Step 2. The racemic amine **125** was subjected to chiral SFC using a Chiral Technologies IC column (250 x 30 mm, 5 µm) with a mobile phase of 70% liquid CO₂ and 30% MeOH with 0.2% TEA using a flowrate of 150 mL/min. The 1^{st} eluting peak was (R)-N-((6-methoxypyridazin-3-yl)methyl)-1-(pyrimidin-2-yl)ethan-1-amine (**126**, 369 mg, > 99% *ee)* . The 2^{nd} eluting peak was (S)-N-((6-methoxypyridazin-3-yl)methyl)-1-(pyrimidin-2-yl)ethan-1-amine (**127**, 374 mg, > 99% *ee*)*.*

Racemic amines in Table 4 were prepared in a fashion similar to that described above for amine **125.** The racemic amines were subjected to chiral SFC to provide enantiomerically pure amines (> 99% *ee*)*.*

**Table 4**

| **Secondary Amines** | **SFC Conditions** | ***m*/*z* (ESI): (M+H)⁺** |
|---|---|---|
| 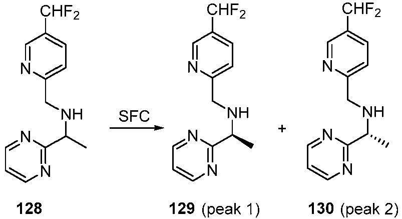 | Chiral Technologies AD column (250x 21 mm, 5 µm) with a mobile phase of 85% Liquid CO₂ and 15% EtOH with 0.2% TEA | 265.1 |
| 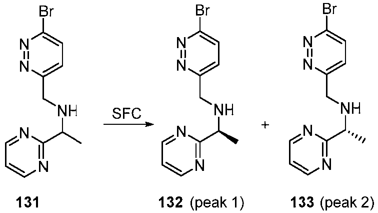 | Chiral Technologies AD column (250 x 30 mm, 5 µm) with a mobile phase of 70% Liquid CO₂ and 30% EtOH with 0.2% TEA | 293.9/ 295.9 |
| 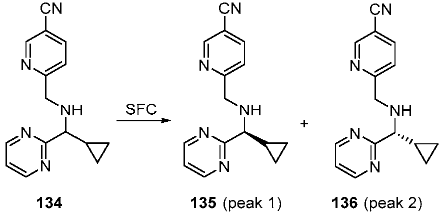 | Chiral Technologies AZ column (250 x 21 mm, 5 µm) with a mobile phase of 75% Liquid CO₂ and 25% EtOH with 0.2% TEA | 266.0 |
| 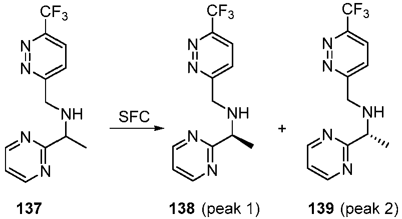 | Chiral Technologies AD column (250 X 21 mm, 5 µm) with a mobile phase of 80% Liquid CO₂ and 20% MeOH with 0.2% TEA using a flowrate of 80 mL/min | 284.1 |
| 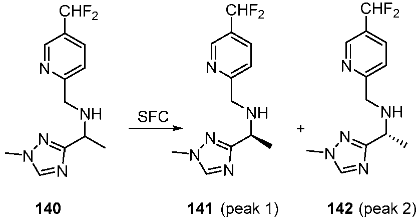 | Chiral IG column (250 X 20 mm, 5 µm) with a mobile phase of 80% Liquid CO₂ and 20% MeOH with 0.1% DEA using a flowrate of 80 mL/min | 268.2 |

Secondary amines in Table 5 were prepared in a manner similar to that described for amine **125** and were derived from commercially available enantiomerically pure reagents.

**Table 5**

| **Int. #** | **Chemical Structure** | **Name** | ***m*/*z* (ESI): (M+H)⁺** |
|---|---|---|---|
| **143** | 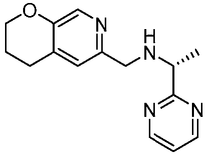 | (R)-N-((3,4-dihydro-2H-pyrano[2,3-c]pyridin-6-yl)methyl)-1-(pyrimidin-2-yl)ethan-1-amine | 271.2 |
| **144** | 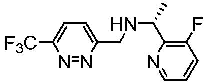 | (R)-1-(3-fluoropyridin-2-yl)-N-((6-(trifluoromethyl)pyridazin-3-yl)methyl)ethan-1-amine | 301.0 |
| **145** | 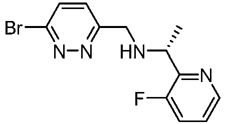 | (R)-N-((6-bromopyridazin-3-yl)methyl)-1-(3-fluoropyridin-2-yl)ethan-1-amine | 311/313 |
| **146** | 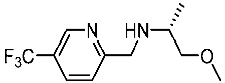 | (R)-1-methoxy-N-((5-(trifluoromethyl)pyridin-2-yl)methyl)propan-2-amine | 249.0 |
| **147** | 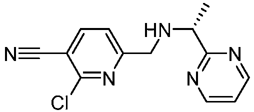 | (R)-2-chloro-6-(((1-(pyrimidin-2-yl)ethyl)amino)methyl)nicotinonitrile | 274.2 |
| **148** | 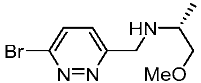 | (R)-N-((6-bromopyridazin-3-yl)methyl)-1-methoxypropan-2-amine | 260.1, 262.0 |
| **149** | 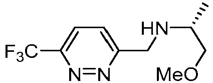 | (R)-1-methoxy-N-((6-(trifluoromethyl)pyridazin-3-yl)methyl)propan-2-amine | |
| **150** | 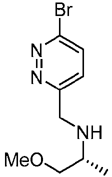 | (R)-N-((6-bromopyridazin-3-yl)methyl)-1-methoxypropan-2-amine | 260.1 and 262.0 |
| **151** | 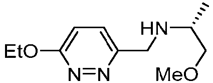 | (R)-N-((6-ethoxypyridazin-3-yl)methyl)-1-methoxypropan-2-amine | |
| **152** | 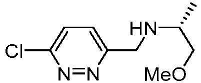 | (R)-N-((6-chloropyridazin-3-yl)methyl)-1-methoxypropan-2-amine | |

### Intermediate 153: N-(1-(5-(trifluoromethyl)pyridin-2-yl)ethyl)cyclopropanamine

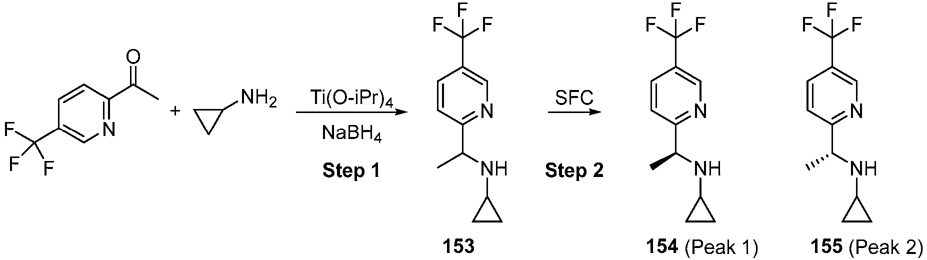

Step 1. To a mixture of 1-(5-(trifluoromethyl)pyridin-2-yl)ethan-1-one (0.71 g, 3.75 mmol, Enamine), 1,2-dichloroethane (20 mL), and cyclopropanamine (0.257 g, 4.50 mmol, Acros) was added titanium (IV) isopropoxide (1.280 g, 1.334 mL, 4.50 mmol, Aldrich). The mixture was stirred at room temperature overnight, then MeOH (2 mL) was added followed by sodium borohydride (0.142 g, 3.75 mmol, Aldrich). The mixture was stirred for 30 min until LCMS showed the product. The mixture was basified with saturated aqueous Na₂CO₃ and extracted with EtOAc. The organic phase was dried over Na₂SO₄, filtered and concentrated in vacuo. The residue was purified by silica gel chromatography: 0-100% EtOAc in heptane. The racemic product (153, 631 mg, 73% yield) was obtained as light-yellow oil. *m*/*z* (ESI): 231 (M+H)⁺

Step 2. The oil was purified by Prep SFC using 2x Chiralpak IG column (250 X 21 mm, 5um) with a mobile phase of 90% Liquid CO₂ and 10% Heptane:EtOH (15:85, v:v) using a flowrate of 70 mL/min. The 1^{st} eluting peak was assigned (S)-N-(1-(5-(trifluoromethyl)pyridin-2-yl)ethyl)cyclopropanamine (**154**, 198 mg, 97.72% *ee*) . The 2^{nd} eluting peak was assigned (R)-N-(1-(5-(trifluoromethyl)pyridin-2-yl)ethyl)cyclopropanamine (**155**, 188 mg, 98.9% *ee*)*.* Absolute stereochemistry was arbitrarily assigned.

Secondary amines in Table 6 were prepared in a manner similar to that described for amine **153**

**Table 6**

| **Int. #** | **Chemical Structure** | **Name** | ***m*/*z* (ESI): (M+H)⁺** |
|---|---|---|---|
| **156** | 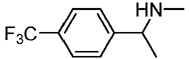 | N-methyl-1-(4-(trifluoromethyl)phenyl)ethan-1-amine | 204.2 |
| **157** | 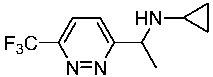 | N-(1-(6-(trifluoromethyl)pyridazin-3-yl)ethyl)cyclopropanamine | 231.2 |
| **158** | 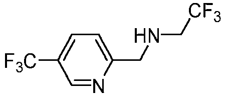 | 2,2,2-trifluoro-N-((5-(trifluoromethyl)pyridin-2-yl)methyl)ethan-1-amine | 259.0 |
| **159** | 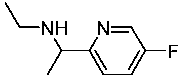 | N-ethyl-1-(5-fluoropyridin-2-yl)ethan-1-amine | 169.2 |
| **160** | 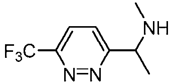 | N-ethyl-1-(6-(trifluoromethyl)pyridazin-3-yl)ethan-1-amine | 206.0 |
| **161** | 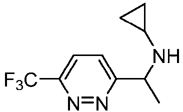 | N-(1-(6-(trifluoromethyl)pyridazin-3-yl)ethyl)cyclopropanamine | 232.2 |
| **162** | 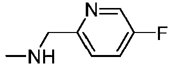 | 1-(5-fluoropyridin-2-yl)-N-methylmethanamine | 141.2 |
| **163** | 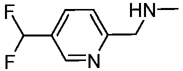 | 1-(5-(difluoromethyl)pyridin-2-yl)-N-methylmethanamine | 173.0 |
| **164** | 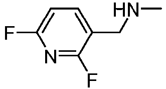 | 1-(2,6-difluoropyridin-3-yl)-N-methylmethanamine | 159.2 |
| **165** | 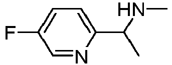 | 1-(5-fluoropyridin-2-yl)-N-methylethan-1-amine | 155.2 |
| **166** | 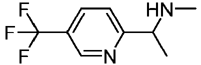 | N-methyl-1-(5-(trifluoromethyl)pyridin-2-yl)ethan-1-amine | 205.0 |
| **167** | 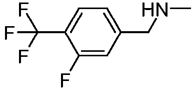 | 1-(3-fluoro-4-(trifluoromethyl)phenyl)-N-methylmethanamine | 208.1 |
| **168** | 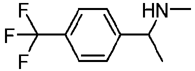 | N-methyl-1-(4-(trifluoromethyl)phenyl)ethan-1-amine | 204.2 |
| **169** | 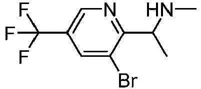 | 1-(3-bromo-5-(trifluoromethyl)pyridin-2-yl)-N-methylethan-1-amine | 283.0 |
| **170** | 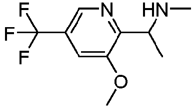 | 1-(3-methoxy-5-(trifluoromethyl)pyridin-2-yl)-N-methylethan-1-amine | 235.0 |

### Intermediate 171: 1-(2-fluoro-6-(trifluoromethyl)pyridin-3-yl)-N-methylmethanamine

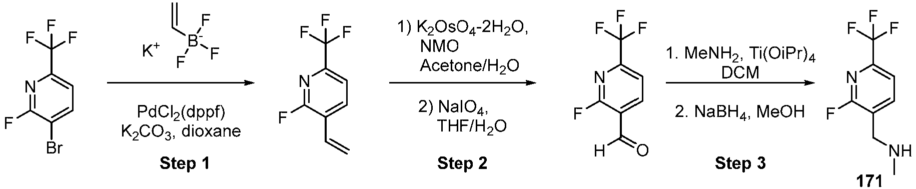

Step 1. A resealable vial was charged with 3-bromo-2-fluoro-6-(trifluoromethyl)pyridine (0.600 g, 2.385 mmol, Combi-Blocks) and potassium vinyltrifluoroborate (0.639 g, 4.77 mmol, Oakwood Products, Inc.) in 1,4-dioxane (5.96 mL) and water (1.988 mL). Then, potassium carbonate (1.319 g, 9.54 mmol, Sigma-Aldrich Corporation) was added to the reaction mixture. The reaction mixture was sparged with Argon (gas) for 5 min, then [1,1'-bis(diphenylphosphino)ferrocene] dichloropalladium (II) (0.044 g, 0.060 mmol, Sigma-Aldrich Corporation) was added to the reaction mixture. The vial was sealed, then the overall reaction mixture was stirred and heated at 80 °C for 16 h. The reaction mixture was diluted with EtOAc and filtered through a pad of celite and concentrated in vacuo. The crude material was absorbed onto a plug of silica gel and purified by chromatography through a Redi-Sep pre-packed silica gel column, eluting with a gradient of 0-15% EtOAc in heptanes, to provide 2-fluoro-6-(trifluoromethyl)-3-vinylpyridine (0.206 g, 1.078 mmol, 45.2 % yield) as light-yellow oil. ¹H NMR (400 MHz, CHLOROFORM-d) δ ppm 8.05 (t, *J*=8.4 Hz, 1 H), 7.59 (dd, *J*=7.7, 1.3 Hz, 1 H), 6.83 (dd, *J*=17.8, 11.3 Hz, 1 H), 6.05 (d, *J*=17.8 Hz, 1 H), 5.67 (d, *J=*11.3 Hz, 1 H). *m*/*z* (ESI): 192.3 (M+H)⁺.

Step 2. To a 100-mL round-bottomed flask was added 2-fluoro-6-(trifluoromethyl)-3-vinylpyridine (1.176 g, 6.15 mmol) in acetone (25.6 mL)/water (5.13 mL) (5:1). To this mixture was added potassium osmate (vi) dihydrate (0.227 g, 0.615 mmol, Acros Organics) and 4-methylmorpholine n-oxide (2.52 g, 21.54 mmol, Sigma-Aldrich Corporation). The overall reaction mixture was allowed to stir under an inert (N₂) atmosphere, while at rt for 45 min. The reaction mixture was quenched with the addition of solid sodium sulfite (700 mg) and allowed the mixture to stir 15 min. The reaction mixture was partially concentrated (to remove acetone) in vacuo. The mixture was diluted with EtOAc and brine. The layers were separated and the aqueous layer was extracted with EtOAc. The organics were combined, dried over MgSO₄, filtered and concentrated in vacuo. The crude residue was used in the next step of the synthesis, without further purification.

The crude diol was diluted with THF (25 mL), then sodium (meta)periodate (3.95 g, 18.46 mmol, Sigma-Aldrich Corporation) and water (3 mL) was added to the mixture. The resulting reaction mixture was allowed to stir under an inert (N₂) atmosphere for 2.5 h. The reaction mixture was diluted with a mixture of EtOAc/Heptane (1:1) (36 mL). The mixture was agitated with sonicator for 1 minute. The mixture was filtered and the filtrate was collected. The mixture was diluted with sat. aq. NaHCO₃ (36 mL). The layers were separated and the aqueous layer was extracted with EtOAc (3x). The combined organic extracts were washed with brine solution (2x), then dried over MgSO₄, filtered and concentrated in vacuo. This material was used in the next step of the synthesis, without further purification, to prevent decompostion. ¹H NMR (400 MHz, CHLOROFORM-d) δ ppm 10.29 - 10.51 (m, 1 H), 8.44 - 8.66 (m, 1 H), 7.67 - 7.88 (m, 1 H).

Step 3. To an oven-dried 100-mL round-bottomed flask was added 2-fluoro-6-(trifluoromethyl)nicotinaldehyde (0.200 g, 1.036 mmol), titanium (IV) isopropoxide (0.368 g, 0.379 mL, 1.295 mmol, Sigma-Aldrich) and methylamine solution, 2.0 M in tetrahydrofuran (1.036 mL, 2.071 mmol, Sigma-Aldrich Corporation) in dichloromethane (2.59 mL). The reaction mixture was stirred at rt overnight. Then, methanol (2.59 mL) was added and the mixture was chilled to 0 °C and sodium borohydride (0.047 g, 1.243 mmol, Sigma-Aldrich) was added slowly to the reaction mixture. The overall reaction mixture was stirred at rt for 16 h, then reaction mixture was concentrated in vacuo. The crude material was absorbed onto a plug of silica gel and purified by chromatography through a Redi-Sep pre-packed silica gel column (40 g), eluting with a gradient of 0-25% MeOH in CH₂Cl₂. This afforded 1-(2-fluoro-6-(trifluoromethyl)pyridin-3-yl)-N-methylmethanamine (0.052 g, 0.250 mmol, 24.12 % yield) as tan oil. ¹H NMR (400 MHz, DMSO-*d*₆) δ ppm 8.24 (t, *J*=8.3 Hz, 1 H), 7.78 - 7.93 (m, 1 H), 3.76 (s, 2 H), 3.29 (br s, 1 H), 2.21 - 2.41 (m, 3 H). *m*/*z* (ESI): 209.2 (M+H)⁺

### Intermediate 172: N-(oxazol-4-ylmethyl)cyclobutanamine

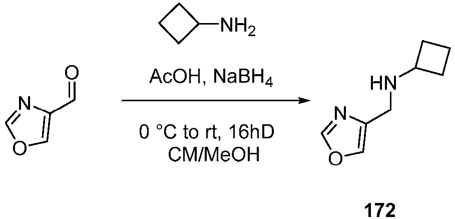

Cyclobutylamine (176 mg, 0.21 mL, 2.47 mmol) and 1,3-oxazole-4-carbaldehyde (240 mg, 2.47 mmol) were dissolved in dichloromethane (5 mL) and acetic acid glacial (29.7 mg, 0.029 mL, 0.494 mmol) was added. The mixture was stirred at rt for 30 minutes, then methanol (5.00 mL) was added and the solution cooled to 0 °C. Sodium borohydride (112 mg, 3.00 mmol) was added portionwise and the mixture was allowed to slowly warm to rt. After 16 h, volatiles were removed in vacuo, the crude product absorbed onto silica gel and the mixture was purified via column chromatography (0 - 20% MeOH/DCM in 8 minutes) to yield N-(oxazol-4-ylmethyl)cyclobutanamine (206 mg, 1.35 mmol, 54.7 % yield) as a colourless oil. *m*/*z* (ESI): 153.3 (M+H)⁺.

Secondary amines in Table 7 were prepared in a manner similar to that described for amine **172**

**Table 7**

| **Int. #** | **Chemical Structure** | **Name** | ***m*/*z* (ESI): (M+H)⁺** |
|---|---|---|---|
| **173** | 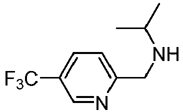 | N-((5-(trifluoromethyl)pyridin-2-yl)methyl)propan-2-amine | 219.1 |
| **174** | 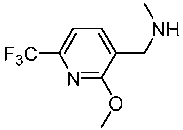 | 1-(2-methoxy-6-(trifluoromethyl)pyridin-3-yl)-N-methylmethanamine | 221.0 |
| **175** | 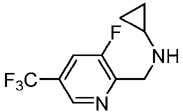 | N-((3-fluoro-5-(trifluoromethyl)pyridin-2-yl)methyl)cyclopropanamine | 235.0 |

### Intermediate 176: 2-((methylamino)methyl)-5-(pentafluoro-l6-sulfaneyl)phenol

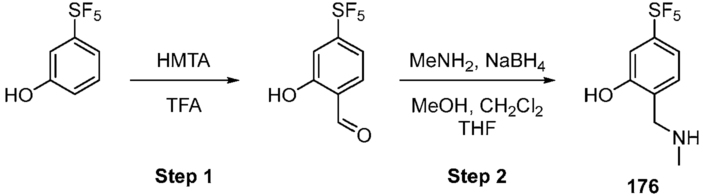

Step 1. To a solution of 3-(pentafluoro-l6-sulfaneyl)phenol (2.5 g, 11.36 mmol, Aurum Pharmatech) in trifluoroacetic acid (20 mL) was add hexamethylenetetramine (HMTA) (2.229 g, 15.90 mmol, Combi-Blocks Inc.) and stirred at 80 °C for 4 h. To the reaction mixture was added water (40 mL) and the reaction was stirred at rt for another 30 min. The reaction mixture was extracted with EtOAc (2 x 40 mL).The organic extract was washed with saturated aqueous NaHCO₃, water and brine and dried over Na₂SO₄. The solution was filtered and concentrated in vacuo to give the crude material as a colorless oil. The crude product was absorbed onto a plug of silica gel and purified by chromatography through a Redi-Sep pre-packed silica gel column (40 g), eluting with a gradient of 0-50% EtOAc in Heptane, to obtain 2-hydroxy-4-(pentafluoro-l6-sulfaneyl)benzaldehyde (1.176 g, 4.74 mmol, 41.7 % yield) as a colorless oil. *m*/*z* (ESI): 248.9 (M+H)⁺. ¹H NMR (400 MHz, CDCl₃) δ 11.06 (s, 1 H), 10.00 (s, 1 H), 7.71 (dd, *J*=8.2,0.8 Hz, 1 H), 7.40 - 7.45 (m, 2 H). ¹⁹F NMR (376 MHz, *DMSO-d*₆) δ ppm 57.10 (s), 56.70 (s)

Step 2. To a solution of 2-hydroxy-4-(pentafluoro-l6-sulfaneyl)benzaldehyde (300 mg, 1.209 mmol) in dichloromethane (5 mL) was treated with methylamine solution, (2.0 M in tetrahydrofuran, 1.813 mL, 3.63 mmol, Sigma Aldrich) and stirred at rt for 3 h. The resulting mixture was concentrated to dryness to give (E)-2-((methylimino)methyl)-5-(pentafluoro-l6-sulfaneyl)phenol as an yellow solid. The above material was dissolved in dichloromethane (5 mL)/ methanol (2 mL) and treated with sodium borohydride (45.7 mg, 1.209 mmol, Sigma Aldrich) at rt and stirred for 30 min. The reaction mixture was diluted with brine and extracted with EtOAc. The organic extract was washed with saturated aqueous NaCl and dried over Na₂SO₄. The solution was filtered and concentrated in vacuo to give the crude material as a yellow oil. The crude product was absorbed onto a plug of silica gel and purified by chromatography through a Redi-Sep pre-packed silica gel column (24 g), eluting with a gradient of 0 % to 100% EtOAc in Heptanes and then 10% MeOH (with 2 M NH₃) in DCM, to obtain 2-((methylamino)methyl)-5-(pentafluoro-l6-sulfaneyl)phenol (142 mg, 0.539 mmol, 44.6 % yield). *m*/*z* (ESI): 264.0 (M+H)⁺. ¹H NMR (400 MHz, *DMSO-d*₆) δ 7.30 (d, *J*=8.4 Hz, 1 H), 7.15 - 7.23 (m, 1 H), 7.11 (d, *J*=2.3 Hz, 1 H), 6.29 (br s, 1 H), 4.14 (s, 1 H), 3.84 (s, 2 H), 2.30 (s, 3 H).

### Intermediate 177: (R)-1-(5-(difluoromethyl)pyridin-2-yl)-N-ethylethan-1-amine

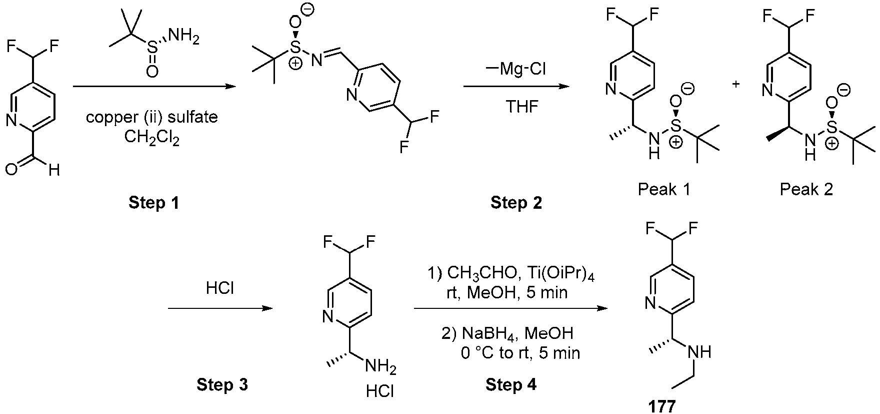

Step 1. To an oven-dried 100-mL round-bottomed flask was added (R)-(+)-2-methyl-2-propanesulfinamide (0.310 g, 2.56 mmol, AK Scientific, Inc.) in dichloromethane (5.12 mL). To this mixture was added copper (ii) sulfate (0.816 g, 5.12 mmol, Sigma-Aldrich Corporation) followed by 5-(difluoromethyl)-2-pyridinecarboxaldehyde (0.402 g, 2.56 mmol, Enamine). The resulting reaction mixture was stirred at rt for 24 h. The reaction mixture was filtered through a pad of Celite and the filter cake was washed with DCM. The filtrate was collected and concentrated in vacuo. The crude material was absorbed onto a plug of silica gel and purified by chromatography through a Redi-Sep pre-packed silica gel column (80 g), eluting with a gradient of 0-30% EtOAc:EtOH (3:1) in heptane, to provide (R,E)-N-((5-(difluoromethyl)pyridin-2-yl)methylene)-2-methylpropane-2-sulfinamide (0.660 g, 2.54 mmol, 99 % yield) as light-yellow solid. ¹H NMR (400 MHz, *DMSO-d*₆) δ ppm 8.97 (s, 1 H), 8.54 (s, 1 H), 8.19 - 8.25 (m, 2 H), 7.25 (t, *J=*55.0 Hz, 1 H), 1.23 (s, 9 H). *m*/*z* (ESI): 261.0 (M+H)⁺.

Step 2. To an oven-dried 100-mL 2-neck round-bottomed flask was added (R,E)-2-methyl-N-((5-(difluoromethyl)pyridin-2-yl)methylene)propane-2-sulfinamide (0.600 g, 2.31 mmol) in tetrahydrofuran (10.78 mL). The reaction mixture was cooled to -78 °C, then methylmagnesium chloride (3.0 M in THF) (1.294 mL, 3.88 mmol, Oakwood Chemicals) was added dropwise to the reaction mixture. After 10 min, the reaction was quenched with the addition of sat. aq. NH₄Cl (5.8 mL) and extracted with EtOAc (3 x 25 mL). The combined organic extracts were dried over MgSO₄, filtered and concentrated in vacuo. The crude material was absorbed onto a plug of silica gel and purified by chromatography through a silica-gel column, eluting in a gradient of 0-80% EtOAc in heptanes, to provide both diastereomers. Peak 1 was arbitrarily assigned as N-((R)-1-(5-(difluoromethyl)pyridin-2-yl)ethyl)-2-methylpropane-2-sulfinamide (0.361 g, 1.306 mmol, 60.6 % yield) as light-yellow solid. ¹H NMR (400 MHz, *DMSO-d*₆) δ ppm 8.68 - 8.74 (m, 1 H), 8.02 (d, *J*=8.5 Hz, 1 H), 7.70 (d, *J*=8.2 Hz, 1 H), 7.14 (t, *J=*55.3 Hz, 1 H), 5.83 (d, *J*=7.9 Hz, 1 H), 4.51 (quin, *J*=7.1 Hz, 1 H), 1.44 (d, *J*=6.9 Hz, 3 H), 1.14 (s, 9 H). *m*/*z* (ESI): 277.1 (M+H)⁺. Peak 2 was arbitrarily assigned as N-((S)-1-(5-(difluoromethyl)pyridin-2-yl)ethyl)-2-methylpropane-2-sulfinamide (0.232 g, 0.840 mmol, 38.9 % yield) as white solid. ¹H NMR (400 MHz, *DMSO-d*₆) δ ppm 8.71 (s, 1 H), 8.02 (d, *J*=8.5 Hz, 1 H), 7.70 (d, *J*=8.2 Hz, 1 H), 7.14 (t, *J=*55.4 Hz, 1 H), 5.83 (d, *J*=7.7 Hz, 1 H), 4.51 (quin, *J*=7.1 Hz, 1 H), 1.44 (d, *J*=6.9 Hz, 3 H), 1.14 (s, 9 H). *m*/*z* (ESI): 277.1 (M+H)⁺.

Step 3. To a 100-mL round-bottomed flask was added (S)-N-((R)-1-(5-(difluoromethyl)pyridin-2-yl)ethyl)-2-methylpropane-2-sulfinamide (0.365 g, 1.321 mmol) and hydrogen chloride solution, 4.0 M in dioxane (0.413 mL, 1.651 mmol, Sigma-Aldrich Corporation) in 1,4-dioxane (8.81 mL). The resulting reaction mixture was stirred at rt for 4 h. The reaction mixture was concentrated in vacuo. The crude residue was carried to the next step of the synthesis, without further purification. *m*/*z* (ESI): 173.0 (M+H)⁺.

Step 4. To a 50-mL round-bottomed flask was added (R)-1-(5-(difluoromethyl)pyridin-2-yl)ethan-1-amine hydrochloride (0.276 g, 1.323 mmol) and acetaldehyde (0.117 g, 0.148 mL, 2.65 mmol, Acros Organics) in methanol (6.61 mL). The reaction mixture was cooled to 0 °C, then titanium (IV) isopropoxide (0.470 g, 0.485 mL, 1.654 mmol, Sigma-Aldrich) was added. The resulting mixture was stirred at rt for 5 min. Then, sodium borohydride (0.300 g, 7.94 mmol, Sigma-Aldrich) was added slowly to the reaction mixture. An additional aliquot of acetaldehyde (0.117 g, 0.148 mL, 2.65 mmol, Acros Organics) was added to the reaction mixture and stirred an additional 10 min. The reaction mixture was quenched with sat. aq. NaHCO₃ (0.5 mL) and stirred 5 min, then it was treated with MgSO₄, and filtered through a pad of Celite. The filtrate was collected and concentrated in vacuo. The crude material was absorbed onto a plug of silica gel and purified by chromatography, eluting with a gradient of 0-30% MeOH in CH₂Cl₂, to provide (R)-1-(5-(difluoromethyl)pyridin-2-yl)-N-ethylethan-1-amine (0.200 g, 0.999 mmol, 76 % yield) as off-white solid. ¹H NMR (400 MHz, *DMSO-d*₆) δ ppm 8.72 - 8.80 (m, 1 H), 8.00 - 8.07 (m, 1 H), 7.60 - 7.69 (m, 1 H), 7.15 (t, *J=*55.3 Hz, 1 H), 7.14 (br t, *J=*55.4 Hz, 1 H), 4.13 - 4.17 (m, 1 H), 2.56 - 2.67 (m, 1 H), 2.41 - 2.49 (m, 1 H), 1.04 - 1.10 (m, 3 H), 0.82 - 0.92 (m, 3 H). *m*/*z* (ESI): 201.1 (M+H)⁺.

Secondary amines in Table 8 were prepared in a manner similar to that described for amine **177.** Secondary amines **178** and **179** were synthesized starting at Step 4 from the commercially available chiral primary amines, (R)-1-[6-(trifluoromethyl)pyridazin-3-yl]ethanamine hydrochloride (CAS# 1948236-91-6) and (R)-1-(5-(trifluoromethyl)pyridin-2-yl)ethan-1-amine hydrochloride (CAS# 1956437-55-0), respectively.

**Table 8**

| **Int. #** | **Chemical Structure** | **Name** | ***m*/*z* (ESI): (M+H)⁺** |
|---|---|---|---|
| **178** | 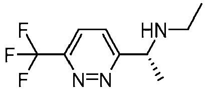 | (R)-N-ethyl-1-(6-(trifluoromethyl)pyridazin-3-yl)ethan-1-amine | 220.1 |
| **179** | 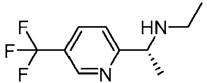 | (R)-N-ethyl-1-(5-(trifluoromethyl)pyridin-2-yl)ethan-1-amine | 219.1 |
| **180** | 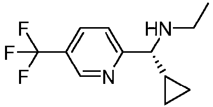 | (R)-N-(cyclopropyl(5-(trifluoromethyl)pyridin-2-yl)methyl)ethanamine | 245.1 |

### Intermediate 181: 1-(3,5-difluoropyridin-2-yl)-N-methylethan-1-amine

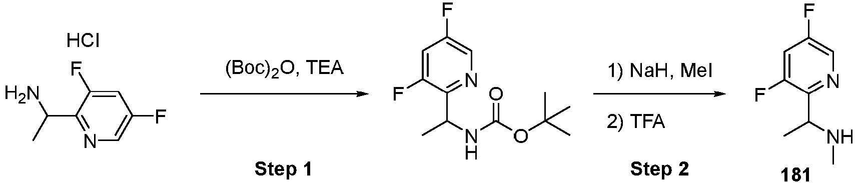

Step 1. To a 100-mL round-bottomed flask was added 1-(3,5-difluoropyridin-2-yl)ethanamine hydrochloride (0.250 g, 1.285 mmol, Combi-Blocks Inc.) and di-tert-butyl dicarbonate (0.421 g, 0.447 mL, 1.927 mmol, Oakwood Products, Inc.) in 1,2-dichloroethane (6.42 mL). Then triethylamine (0.520 g, 0.722 mL, 5.14 mmol, Sigma-Aldrich Corporation) was added to the reaction mixture and the overall mixture was stirred at rt for 2 h. The reaction mixture was diluted with DCM (5 mL) and sat. aq. NaHCO₃ (5 mL). The layers were separated and the aqueous layer was extracted with DCM (3x). The combined organic extracts were dried over MgSO₄, filtered and concentrated in vacuo. The crude material was absorbed onto a plug of silica gel and purified with a gradient of 0-25% EtOAc in heptane, to afford tert-butyl (1-(3,5-difluoropyridin-2-yl)ethyl)carbamate (0.300 g, 1.162 mmol, 90 % yield) as off-white solid. ¹H NMR (400 MHz, *DMSO-d*₆) δ ppm 8.46 (d, J=1.9 Hz, 1 H), 7.87 (t, *J*=9.6 Hz, 1 H), 7.21 (br d, *J=*7.5 Hz, 1 H), 4.81 - 4.98 (m, 1 H), 1.34 (br d, *J*=5.9 Hz, 9 H), 1.31 - 1.33 (m, 3 H). *m*/*z* (ESI): 259.1 (M+H)⁺.

Step 2. To a 100-mL round-bottomed flask was added tert-butyl (1-(3,5-difluoropyridin-2-yl)ethyl)carbamate (0.300 g, 1.162 mmol) in tetrahydrofuran (5.81 mL). The mixture was cooled to 0 °C, then sodium hydride (60% dispersion in mineral oil) (0.058 g, 1.452 mmol, Oakwood Products, Inc.) was added to the reaction mixture. The resulting mixture was stirred at 0 °C for 20 min, then iodomethane (0.198 g, 0.198 mL, 1.394 mmol, Sigma-Aldrich Corporation) was added dropwise to the mixture. The reaction mixture was stirred an additional 20 min, while the temperature was maintained at 0 °C, then the mixture was stirred at rt overnight. The reaction mixture was quenched with MeOH and concentrated in vacuo. The crude material was absorbed onto a plug of silica gel and purified by chromatography through a Redi-Sep pre-packed silica gel column (40 g), eluting with a gradient of 0-30% EtOAc in Heptane, to provide tert-butyl (1-(3,5-difluoropyridin-2-yl)ethyl)(methyl)carbamate (0.287 g, 1.054 mmol, 91 % yield) as light-yellow oil. ¹H NMR (400 MHz, CHLOROFORM-d) δ ppm 8.31 (d, *J*=2.3 Hz, 1 H), 7.18 (ddd, *J*=9.4, 8.3, 2.4 Hz, 1 H), 5.63 (br s, 1 H), 2.75 (br s, 3 H), 1.53 - 1.57 (m, 3 H), 1.47 (s, 9 H). *m*/*z* (ESI): 295.3 (M+Na)⁺.

The residue was dissolved in dichloromethane (5.81 mL) and treated with trifluoroacetic acid (1.324 g, 0.866 mL, 11.62 mmol, Sigma-Aldrich Corporation). The reaction mixture was stirred at rt for 1 h at which time it was concentrated in vacuo. The residue was diluted with DCM, then treated with sat. aq. NaHCO₃. The layers were separated and the aqueous layer was extracted with DCM (3x). The combined organic extracts were dried over MgSO₄, filtered and concentrated in vacuo. This afforded 1-(3,5-difluoropyridin-2-yl)-N-methylethan-1-amine (0.109 g, 0.633 mmol, 54.5 % yield) as light-yellow oil. ¹H NMR (400 MHz, *DMSO-d*₆) δ ppm 8.50 (d, *J*=2.3 Hz, 1 H), 7.87 (ddd, *J*=10.0, 9.2, 2.3 Hz, 1 H), 3.98 (qd, *J*=6.7, 1.4 Hz, 1 H), 3.25 - 3.34 (m, 1 H), 2.14 (s, 3 H), 1.26 - 1.30 (m, 3 H). *m*/*z* (ESI): 173.2 (M+H)⁺.

Secondary amines in Table 9 were prepared in a manner similar to Steps 1-2 described for amine **181.** The chiral primary amines used in Step 1 were synthesized in a manner similar to Step 1-3 from Example **177** above.

**Table 9**

| **Int. #** | **Chemical Structure** | **Name** | ***m*/*z* (ESI): (M+H)⁺** |
|---|---|---|---|
| **182** | 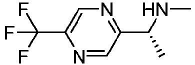 | (R)-N-methyl-1-(5-(trifluoromethyl)pyrazin-2-yl)ethan-1-amine | 206.2 |
| **183** | 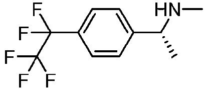 | (R)-N-methyl-1-(4-(perfluoroethyl)phenyl)ethan-1-amine | 254.0 |
| **184** | 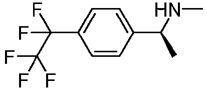 | (S)-N-methyl-1-(4-(perfluoroethyl)phenyl)ethan-1-amine | 254.2 |

### Intermediate 185: 1-(3-fluoro-5-(trifluoromethyl)pyridin-2-yl)-N-methylmethanamine

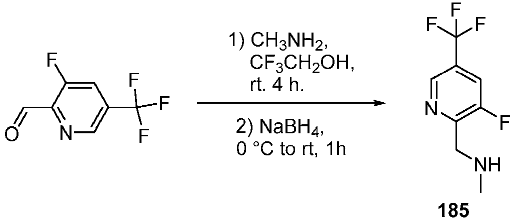

To an oven-dried 100-mL round-bottomed flask was added 3-fluoro-5-(trifluoromethyl)picolinaldehyde (0.300 g, 0.300 mL, 1.554 mmol, Combi-Blocks Inc.) and methylamine solution, 2.0 M in tetrahydrofuran (1.554 mL, 3.11 mmol, Sigma-Aldrich Corporation) in 2,2,2-Trifluoroethanol (4.09 mL). The reaction mixture was stirred at rt for 4 h. Then the mixture was cooled to 0°C, before sodium borohydride (0.071 g, 1.864 mmol, Sigma-Aldrich) was added slowly to the reaction mixture. The overall reaction mixture was stirred at rt for 2 h.. Then the reaction mixture was concentrated in vacuo. The crude material was absorbed onto a plug of silica gel and purified by chromatography through a Redi-Sep pre-packed silica gel column, eluting with a gradient of 0-20% MeOH in DCM, to provide 1-(3-fluoro-5-(trifluoromethyl)pyridin-2-yl)-N-methylmethanamine (0.190 g, 0.913 mmol, 58.8% yield) as yellow oil. *m*/*z* (ESI): 209.2 (M+H)⁺.

Secondary amines in Table 10 were prepared in a manner similar to that described for amine **185.**

**Table 10**

| **Int. #** | **Chemical Structure** | **Name** | ***m*/*z* (ESI): (M+H)⁺** |
|---|---|---|---|
| **186** | 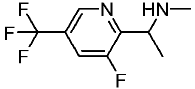 | 1-(3-fluoro-5-(trifluoromethyl)pyridin-2-yl)-N-methylethan-1-amine | 223.0 |
| **187** | 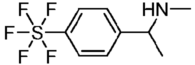 | N-methyl-1-(4-(pentafluoro-l6-sulfaneyl)phenyl)ethan-1-amine | 262.0 |

### Intermediate 188: N-methyl-1-(6-(1-(trifluoromethyl)-1H-pyrazol-4-yl)pyridazin-3-yl)methanamine

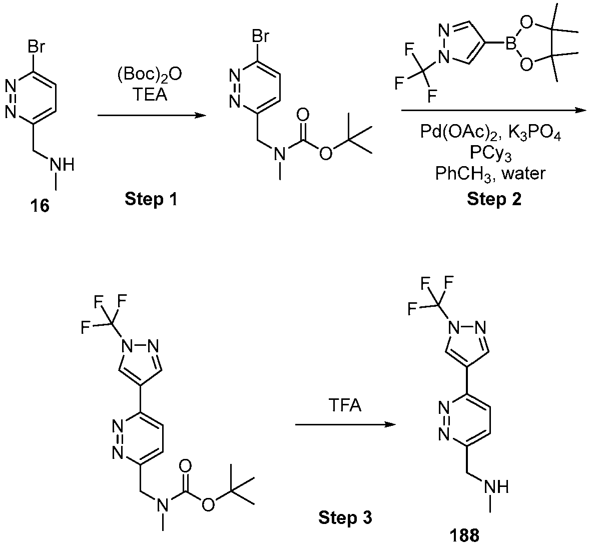

Step 1. To a 100-mL round-bottomed flask was added 1-(6-bromopyridazin-3-yl)-N-methylmethanamine (0.320 g, 1.584 mmol) and di-tert-butyl dicarbonate (0.518 g, 0.552 mL, 2.376 mmol, Oakwood Products, Inc.) in 1,2-dichloroethane (7.92 mL). Then triethylamine (0.641 g, 0.890 mL, 6.33 mmol, Sigma-Aldrich Corporation) was added to the reaction mixture and the overall mixture was stirred at rt for 16 h. The reaction mixture was diluted with DCM (5 mL) and sat. aq. NaHCO₃ (5 mL). The layers were separated and the aqueous layer was extracted with DCM (3x). The combined organic extracts were dried over MgSO₄, filtered and concentrated in vacuo. The crude material was absorbed onto a plug of silica gel and purified with a gradient of 0-60% EtOAc in heptane, to afford tert-butyl ((6-bromopyridazin-3-yl)methyl)(methyl)carbamate (0.400 g, 1.324 mmol, 84 % yield) as light-yellow oil. ¹H NMR (400 MHz, CHLOROFORM-*d*) δ ppm 7.65 (br d, *J*=8.8 Hz, 1 H), 7.30 - 7.51 (m, 1 H), 4.71 (s, 2 H), 2.94 (br s, 3 H), 1.49 (br s, 9 H). *m*/*z* (ESI): 302.0 (M+H)⁺.

Step 2. To a resealable vial, was added tert-butyl ((6-bromopyridazin-3-yl)methyl)(methyl)carbamate (0.200 g, 0.662 mmol), 4-(4,4,5,5-tetramethyl-1,3,2-dioxaborolan-2-yl)-1-(trifluoromethyl)-1h-pyrazole (0.347 g, 0.347 mL, 1.324 mmol, Enamine) and potassium phosphate tribasic (0.421 g, 1.986 mmol, Sigma-Aldrich Corporation) in a mixture of toluene (2.98 mL)/water (0.331 mL). The reaction mixture was sparged with Argon (gas) for 5 min, then tricyclohexylphosphine (0.074 g, 0.265 mmol, Strem Chemicals, Inc.), followed by palladium (II) acetate (0.030 g, 0.132 mmol, Sigma-Aldrich Corporation) was added to the reaction mixture and the vial was sealed. The reaction mixture was stirred and heated at 90 °C for 16 h at which time it was cooled to rt, then diluted with EtOAc and filtered through a pad of Celite. The organic filtrate was collected, then concentrated in vacuo. The crude material was absorbed onto a plug of silica gel and purified by chromatography through a pre-packed silica gel column, eluting with a gradient of 0-45% EtOAc in Heptane, to provide tert-butyl methyl((6-(1-(trifluoromethyl)-1H-pyrazol-4-yl)pyridazin-3-yl)methyl)carbamate (0.078 g, 0.218 mmol, 33.0 % yield) as tan oil.

Step 3. To a 50-mL round-bottomed flask was added tert-butyl methyl((6-(1-(trifluoromethyl)-1H-pyrazol-4-yl)pyridazin-3-yl)methyl)carbamate (0.060 g, 0.168 mmol) and trifluoroacetic acid (0.191 g, 0.191 mL, 1.679 mmol, Apollo Scientific Ltd.) in 1,2-dichloroethane (1.6 mL). The overall mixture was stirred at rt for 16 h. The reaction mixture was concentrated in vacuo. The crude was used in the next step of the synthesis, without further purification. *m*/*z* (ESI): 258.2 (M+H)⁺.

Secondary amines in Table 11 were prepared in a manner similar to that described for amine **188.**

**Table 11**

| **Int. #** | **Chemical Structure** | **Name** | ***m*/*z* (ESI): (M+H)⁺** |
|---|---|---|---|
| **189** | 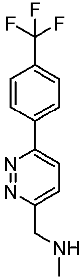 | N-methyl-1-(6-(4-(trifluoromethyl)phenyl)pyridazin-3-yl)methanamine | 268.1 |
| **190** | 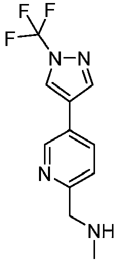 | N-methyl-1-(5-(1-(trifluoromethyl)-1H-pyrazol-4-yl)pyridin-2-yl)methanamine | 257.0 |

### Intermediate 191: (R)-N-methyl-1-(6-(4-(trifluoromethyl)phenyl)pyridazin-3-yl)ethan-1-amine

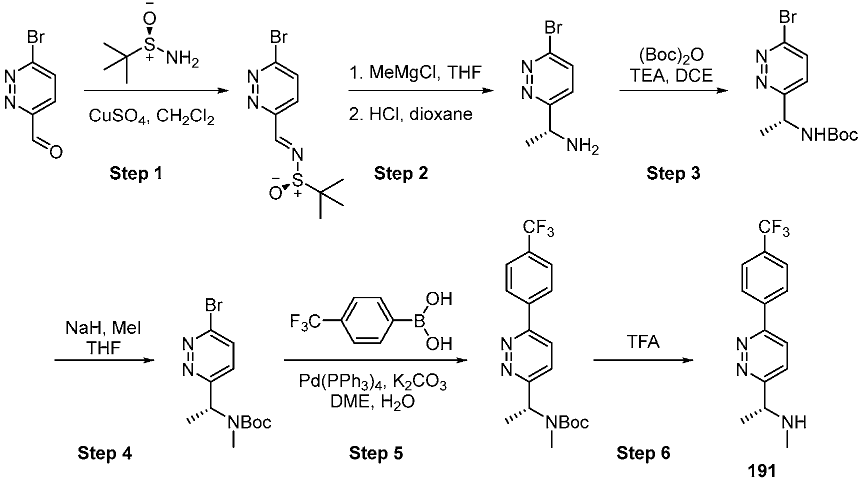

Step 1. To an oven-dried 100-mL round-bottomed flask was added (R)-(+)-2-methyl-2-propanesulfinamide (1.220 g, 10.07 mmol, AK Scientific, Inc.) in dichloromethane (20.13 mL). To this mixture was added copper (ii) sulfate (3.21 g, 20.13 mmol, Sigma-Aldrich Corporation) followed by 6-bromopyridazine-3-carbaldehyde (1.882 g, 10.07 mmol, PharmaBlock Sciences). The resulting reaction mixture was stirred at rt for 24 h at which time it was filtered through a pad of Celite and the filter cake was washed with 1:1 EtOAc:Heptane. The filtrate was collected and concentrated in vacuo. The crude material was triturated from EtOAc and heptane. The solids were collected and dried further in a reduced pressure oven for 2 h. This afforded (R,E)-N-((6-bromopyridazin-3-yl)methylene)-2-methylpropane-2-sulfinamide (1.667 g, 5.74 mmol, 57.1 % yield) as tan solid. ¹H NMR (400 MHz, CHLOROFORM-*d*) δ ppm 9.00 (s, 1 H), 8.04 (br d, *J*=8.8 Hz, 1 H), 7.80 (br d, *J*=8.8 Hz, 1 H), 1.32 (s, 9 H). *m*/*z* (ESI): 314.0 (M+Na)⁺.

Step 2. To an oven-dried 150-mL 3-neck round-bottomed flask, equipped with an internal temperature probe, was added (R,E)-N-((6-bromopyridazin-3-yl)methylene)-2-methylpropane-2-sulfinamide (1.667 g, 5.74 mmol) in tetrahydrofuran (28.7 mL). The reaction mixture was cooled to - 78°C, then methylmagnesium chloride (3.45 mL, 10.34 mmol, Oakwood) was added dropwise to the reaction mixture. (Note: Addition of grignard reagent, was added slow enough where the temperature of reaction mixture did not warm past -70°C) After the addition, the overall reaction mixture was stirred an additional 20 min, while the temperature was maintained at -78°C. Then, the reaction was quenched, while at -70°C, with the addition of sat. aq. NH₄Cl (30 mL). The mixture was warmed to rt and extracted with EtOAc (3 x 100 mL). The combined organic extracts were washed with brine, then dried over MgSO₄, filtered and concentrated in vacuo. The crude material was absorbed onto a plug of silica gel and purified by chromatography through a Interchim (15 micron) silica-gel column (120 grams), eluting with a gradient of 0-100% EtOAc in heptane, then with a gradient of 0-50% EtOAc:EtOH (3:1) in heptane to provide a mixture of both diasteromers (R)-N-((R)-1-(6-bromopyridazin-3-yl)ethyl)-2-methylpropane-2-sulfinamide (0.657 g, 2.146 mmol, 37.3 % yield) as tan solid and (R)-N-((S)-1-(6-bromopyridazin-3-yl)ethyl)-2-methylpropane-2-sulfinamide (0.067 g, 0.219 mmol, 3.81 % yield) as tan solid. Stereochemistry of the major isomer was assigned in analogy to Kuduk, et al. Tet. Lett. 2004, 45 (35), 6641. ¹H NMR (400 MHz, *DMSO-d*₆) δ ppm 7.90 - 8.08 (m, 1 H), 7.76 - 7.85 (m, 1 H), 5.96 (d, *J*=8.4 Hz, 1 H), 4.61 - 4.71 (m, 1 H), 1.50 (d, *J*=6.9 Hz, 3 H), 1.14 (s, 9 H). *m*/*z* (ESI): 306.1 (M+H)⁺

To a 50-mL round-bottomed flask was added (R)-N-((R)-1-(6-bromopyridazin-3-yl)ethyl)-2-methylpropane-2-sulfinamide (0.650 g, 2.123 mmol) and hydrogen chloride solution, 4.0 M in dioxane (0.663 mL, 2.65 mmol, Sigma-Aldrich Corporation) in 1,4-dioxane (10.61 mL). The overall reaction mixture was stirred at rt overnight. The reaction mixture was concentrated in vacuo, and the residue was diluted with heptane and DCM (10:1), then agitated by sonication for 1 min. The precipitate was collected by filtration, then the solids were washed with heptane (3x). This afforded (R)-1-(6-bromopyridazin-3-yl)ethan-1-amine hydrochloride as black crude mixture, which was carried to the next step of the synthesis, without further purification. *m*/*z* (ESI): 202.1 (M+H)⁺

Step 3. To a 100-mL round-bottomed flask was added (R)-1-(6-bromopyridazin-3-yl)ethan-1-amine hydrochloride (0.500 g, 2.096 mmol) and di-tert-butyl dicarbonate (0.686 g, 0.730 mL, 3.14 mmol, Oakwood Products, Inc.) in 1,2-dichloroethane (10.48 mL). Then, triethylamine (1.061 g, 1.473 mL, 10.48 mmol, Sigma-Aldrich Corporation) was added to the reaction mixture and the overall mixture was stirred at rt for 16 h. The reaction mixture was diluted with DCM (5 mL) and sat. aq. NaHCO₃ (5 mL). The layers were separated and the aqueous layer was extracted with DCM (3x). The combined organic extracts were dried over MgSO₄, filtered and concentrated in vacuo. The crude material was absorbed onto a plug of silica gel and purified with a gradient of 0-80% EtOAc in heptane, to afford tert-butyl (R)-(1-(6-bromopyridazin-3-yl)ethyl)carbamate (0.182 g, 0.602 mmol, 28.7 % yield) as tan solid. ¹H NMR (400 MHz, *DMSO-d*₆) δ ppm 7.90 (d, *J*=8.8 Hz, 1 H), 7.71 (d, *J*=9.0 Hz, 1 H), 7.44 - 7.66 (m, 1 H), 4.78 - 4.94 (m, 1 H), 1.38 (br d, *J*=8.8 Hz, 12 H). *m*/*z* (ESI): 302.0 (M+H)⁺.

Step 4. To a 100-mL round-bottomed flask was added tert-butyl (R)-(1-(6-bromopyridazin-3-yl)ethyl)carbamate (0.170 g, 0.563 mmol) in tetrahydrofuran (5.63 mL). The mixture was cooled to 0 °C, then sodium hydride (60% dispersion in mineral oil) (0.028 g, 0.703 mmol, TCI America) was added to the reaction mixture. The resulting mixture was stirred at 0 °C for 20 min, then iodomethane (0.096 g, 0.042 mL, 0.675 mmol, Sigma-Aldrich Corporation) was added dropwise to the mixture. The reaction mixture was stirred an additional 20 min, while temperature maintained at 0 °C, then the mixture was stirred at rt overnight. The reaction mixture was quenched with MeOH and concentrated in vacuo. The crude material was absorbed onto a plug of silica gel and purified by chromatography through a Redi-Sep pre-packed silica gel column (40 g), eluting with a gradient of 0-25% EtOAc in Heptane, to provide tert-butyl (R)-(1-(6-bromopyridazin-3-yl)ethyl)(methyl)carbamate (0.170 g, 0.538 mmol, 96 % yield) as light-yellow oil. ¹H NMR (400 MHz, CHLOROFORM-*d*) δ ppm 8.31 (d, *J*=2.3 Hz, 1 H), 7.18 (ddd, *J*=9.4, 8.3, 2.4 Hz, 1 H), 5.63 (br s, 1 H), 2.75 (br s, 3 H), 1.53 - 1.57 (m, 3 H), 1.47 (s, 9 H). *m*/*z* (ESI): 216.1 (M-Boc+H)⁺

Step 5. A resealable vial was charged with tert-butyl (R)-(1-(6-bromopyridazin-3-yl)ethyl)(methyl)carbamate (0.160 g, 0.506 mmol), b-[4-(trifluoromethyl)phenyl]-boronic acid (0.288 g, 1.518 mmol, AA Blocks) and potassium carbonate (0.210 g, 1.518 mmol, Oakwood Chemicals) in 1,2-dimethoxyethane (2.300 mL)/ water (0.230 mL). The reaction mixture was sparged with Argon for 5 min. Then Pd(PPh₃)₄ (0.117 g, 0.101 mmol, Sigma-Aldrich) was added to the reaction mixture. The vial was sealed, then the reaction mixture was stirred and heated at 90°C for 16 h. The reaction mixture was diluted with EtOAc and brine solution. The layers were separated and the aqueous layer was extracted with EtOAc (3x). The combined organic extract was dried over MgSO₄, filtered and concentrated in vacuo. The crude material was absorbed onto a plug of silica gel and purified by chromatography through a Redi-Sep pre-packed silica gel column (40 g), eluting with a gradient of 0-100% EtOAc in heptane, to provide tert-butyl (R)-methyl(1-(6-(4-(trifluoromethyl)phenyl)pyridazin-3-yl)ethyl)carbamate (0.155 g, 0.406 mmol, 80 % yield) as light-yellow solid. ¹H NMR (400 MHz, CHLOROFORM-*d*) δ ppm 8.16 - 8.47 (m, 1 H), 7.77 - 7.96 (m, 3 H), 7.49 - 7.72 (m, 2 H), 5.07 (s, 1 H), 1.51 (s, 3 H), 1.28 (s, 12 H). *m*/*z* (ESI): 382.1 (M+H)⁺

Step 6. To a 50-mL round-bottomed flask was added tert-butyl (R)-methyl(1-(6-(4-(trifluoromethyl)phenyl)pyridazin-3-yl)ethyl)carbamate (0.140 g, 0.367 mmol) and trifluoroacetic acid (0.419 g, 0.419 mL, 3.67 mmol, Apollo Scientific Ltd.) in 1,2-dichloroethane (3.67 mL). The overall mixture was stirred at rt for 16 h. The reaction mixture was concentrated in vacuo. The crude residue was carried to the next step of the synthesis, without further purification. *m*/*z* (ESI): 282.2 (M+H)⁺

Secondary amines (**192-193**) in Table 12 were prepared in a manner similar to that described for amine **191** starting from Step 3 using the commercially available chiral amine, (R)-1-(4-Bromophenyl)ethylamine (CAS# 45791-36-4).

**Table 12**

| **Int. #** | **Chemical Structure** | **Name** | ***m*/*z* (ESI): (M+H)⁺** |
|---|---|---|---|
| **192** | 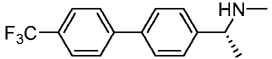 | (R)-N-methyl-1-(4'-(trifluoromethyl)-[1,1'-biphenyl]-4-yl)ethan-1-amine | 280.3 |
| **193** | 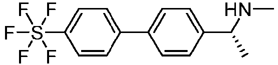 | (R)-N-methyl-1-(4'-(pentafluoro-l6-sulfaneyl)-[1,1'-biphenyl]-4-yl)ethan-1-amine | 338.2 |

### Intermediate 194: 1-(6-cyclopropylpyridazin-3-yl)-N-methylmethanamine

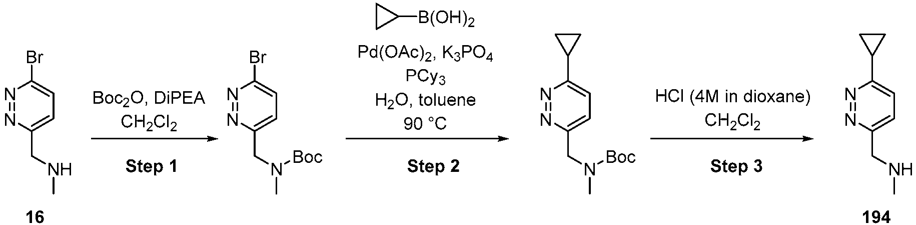

Step 1. 1-(6-bromopyridazin-3-yl)-N-methylmethanamine (16, 176.6 mg, 0.874 mmol) and diisopropylethylamine (226 mg, 305 µL, 1.748 mmol, Sigma-Aldrich Corporation) were stirred in dichloromethane (4370 µL) and Boc anhydride (210 mg, 0.961 mmol, Sigma-Aldrich Corporation) was added. The reaction was stirred at room temp. for 18 hours. The mixture was then partitioned between DCM and water and the layers were separated. The organic layer was washed with brine, dried over MgSO₄, and concentrated. The crude product was then purified by medium pressure chromatography (silica, 10 to 100% EtOAc:Heptanes) to give tert-butyl ((6-bromopyridazin-3-yl)methyl)(methyl)carbamate (204 mg, 0.675 mmol, 77 % yield). *m*/*z* (ESI): 302.0, 304.2 (M+H)⁺. ¹H NMR (400 MHz, CHLOROFORM-d) δ ppm 7.65 (br d, J=7.9 Hz, 1 H), 7.44 (br d, J=7.1 Hz, 1 H), 4.71 (s, 2 H), 2.93 (br s, 3 H), 1.48 (br d, J=5.4 Hz, 9 H)

Step 2. A mixture of tert-butyl ((6-bromopyridazin-3-yl)methyl)(methyl)carbamate (204.6 mg, 0.677 mmol), cyclopropylboronic acid (291 mg, 3.39 mmol) and toluene (2888 µL) was purged with Ar, then potassium phosphate tribasic (431 mg, 2.031 mmol, Alfa Aesar) and water (321 µL) were added and the mixture was stirred for 10 min at rt. Then, tricyclohexylphosphine (38.0 mg, 0.135 mmol, Strem Chemicals, Inc.) and palladium (II) acetate (15.20 mg, 0.068 mmol, Strem Chemicals, Inc.) were added. The mixture was stirred in a sealed vial at 90 °C for 2 hours, then it was filtered through celite and concentrated in vacuo. The crude material was purified by chromatography through a silica gel column, eluting with 0-60% 3:1 EtOAc:EtOH in heptanes and tert-butyl ((6-cyclopropylpyridazin-3-yl)methyl)(methyl)carbamate (90.4 mg, 0.343 mmol, 50.7 % yield) was obtained. *m*/*z* (ESI): 264.2 (M+H)⁺. ¹H NMR (400 MHz, CHLOROFORM-d) δ ppm 7.39 (br d, J=7.5 Hz, 1 H), 7.17 - 7.27 (m, 1 H), 4.69 (s, 2 H), 2.91 (br s, 3 H), 2.11 - 2.22 (m, 1 H), 1.49 (br s, 9 H), 1.09 - 1.24 (m, 4 H)

Step 3. To a solution of tert-butyl ((6-cyclopropylpyridazin-3-yl)methyl)(methyl)carbamate (90.4 mg, 0.343 mmol) in dichloromethane (1248 µL) was added hydrogen chloride solution, 4.0 M in dioxane (687 µL, 2.75 mmol, Sigma-Aldrich Corporation). The solution became a suspension so MeOH was added to make the suspension to a solution again. The mixture was stirred was stirred at rt for 4 h until LCMS showed the product. The mixture was concentrated in vacuo. The product 1-(6-cyclopropylpyridazin-3-yl)-N-methylmethanamine hydrochloride (74.8 mg, 0.375 mmol, 109 % yield) was obtained as light brown solid and used directly in further expermiments. *m*/*z* (ESI): 164.2 (M+H)⁺.

### Intermediate 195: 2-(5-chloropyridin-2-yl)-2,2-difluoro-N-methylethan-1-amine

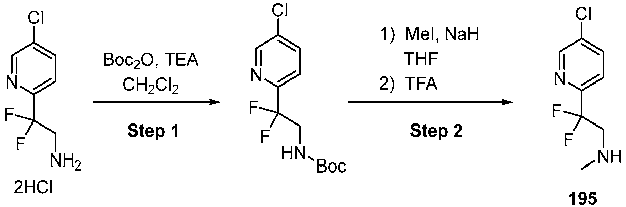

Step 1. To a stirred ice-cooled solution of 2-(5-chloropyridin-2-yl)-2,2-difluoroethan-1-amine dihydrochloride (307 mg, 1.16 mmol, 1.0 equiv, Enamine) and triethylamine (351 mg, 483 µL, 3.47 mmol, 3.0 equiv, Aldrich) in DCM (3.85 mL) was added di-tert-butyl dicarbonate (252 mg, 1.16 mmol, 1 equiv, Aldrich). The resulting mixture was stirred at 0 °C for 15 minutes then to room temperature until completion over 1.5 hours. The crude mixture was directly loaded on a silica gel column and subjected to medium pressure column chromatography eluting with EtOAc/heptane (15 min from 0 to 100%) to give tert-butyl (2-(5-chloropyridin-2-yl)-2,2-difluoroethyl)carbamate (310 mg, 1.06 mmol, 92 % yield). *m*/*z* (ESI): 293.1 (M+H)⁺.

Step 2. To a stirred ice-cooled solution of tert-butyl (2-(5-chloropyridin-2-yl)-2,2-difluoroethyl)carbamate (300 mg, 1.03 mmol, 1.0 equiv) in THF (5.0 mL) was added sodium hydride (60% dispersion) (36.9 mg, 1.54 mmol,1.5 equiv) under nitrogen atmosphere. The resulting mixture was stirred at 0°C for 15 min before methyl iodide (145 mg, 64.1 µL, 1.03 mmol,1.0 equiv) was added via a syringe. The resulting mixture was stirred at 0 °C for 15 minutes and then at ambient temperature for 16 h. The reaction mixture was cooled in an ice bath before quenching with MeOH. The volatiles were removed *in vacuo* and the residue was purified by medium pressure chromatography (silica, 0 to 100% EtOAc:Heptanes) to give tert-butyl (2-(5-chloropyridin-2-yl)-2,2-difluoroethyl)(methyl)carbamate (210 mg, 0.685 mmol, 66.8 % yield). This material was then dissolved in TFA (4.0 mL) and stirred for 25 minutes to completion. The reaction mixture was then concentrated under reduced pressure on the rotovap to give the crude TFA salt. This salt was then dissolved in MeOH and loaded onto an SCX column, eluted with 0 to 2M ammonia in MeOH, and cocentrated to give 2-(5-chloropyridin-2-yl)-2,2-difluoro-N-methylethan-1-amine (127 mg, 0.615 mmol, 60.0 % yield). *m*/*z* (ESI): 207.1 (M+H)⁺.

### Intermediate 196: (R)-1-(pyrimidin-2-yl)-N-((6-(2,2,2-trifluoroethoxy)pyridazin-3-yl)methyl)ethan-1-amine

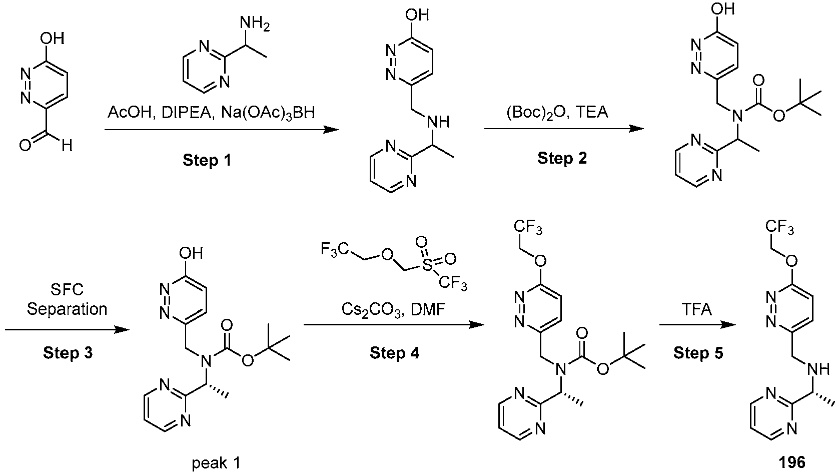

Step 1. To a 150-mL round-bottomed flask was added 1-(pyrimidin-2-yl)ethan-1-amine dihydrochloride (2.79 g, 14.23 mmol, Enamine) in a 1:1 mixture of methanol (21.56 mL)/dichloromethane (21.56 mL). The reaction mixture was cooled to 0 °C, then n,n'-diisopropylethylamine (3.51 g, 4.74 mL, 27.2 mmol, Sigma-Aldrich Corporation) was added to the reaction mixture and stirred 10 min. Then, 3-formyl-6-hydroxypyridazine (1.60 g, 12.93 mmol, Aurum Pharmatech LLC) and acetic acid (0.77 g, 0.74 mL, 12.93 mmol, Sigma-Aldrich Corporation) were added to the mixture, followed by acetic acid (0.77 g, 0.74 mL, 12.93 mmol, Sigma-Aldrich Corporation). The reaction mixture was warmed to rt over 15 min, Then, sodium triacetoxyborohydride (6.85 g, 32.3 mmol, Sigma-Aldrich Corporation) was added and the overall mixture was stirred for 16 h, while under an inert (N₂) atmosphere. Another aliquot of sodium triacetoxyborohydride (6.85 g, 32.3 mmol, Sigma-Aldrich Corporation) was added to the reaction mixture and stirred an additional 16 h. The reaction mixture was filtered through a pad of Celite, then the filter cake was rinsed with 1:1 MeOH:DCM (3x). The filtrate was collected and concentrated in vacuo. The crude material was absorbed onto a plug of silica gel and purified by chromatography through a Redi-Sep pre-packed silica gel column (120 g), eluting with a gradient of 0-35% MeOH in CH₂Cl₂, to provide 6-(((1-(pyrimidin-2-yl)ethyl)amino)methyl)pyridazin-3-ol (1.11 g, 4.84 mmol, 37.4 % yield) as light-yellow solid. *m*/*z* (ESI): 232.1 (M+H)⁺. ¹H NMR (400 MHz, METHANOL-d4) δ ppm 8.78 (d, J=5.0 Hz, 2 H), 7.54 (d, J=9.6 Hz, 1 H), 7.38 (t, J=4.9 Hz, 1 H), 6.92 (d, J=9.6 Hz, 1 H), 4.07 (q, J=6.8 Hz, 1 H), 3.72 (d, J=2.3 Hz, 2 H), 1.98 (s, 1 H), 1.48 (d, J=6.9 Hz, 3 H).

Step 2. To a 150-mL round-bottomed flask was added 6-(((1-(pyrimidin-2-yl)ethyl)amino)methyl)pyridazin-3-ol (1.11 g, 4.80 mmol) and triethylamine (1.45 g, 2.02 mL, 14.40 mmol, Sigma-Aldrich Corporation) in 1,2-dichloroethane (24.00 mL). Then di-tert-butyl dicarbonate (1.57 g, 1.67 mL, 7.20 mmol, Sigma-Aldrich Corporation) was added to the reaction mixture. The overall reaction mixture was stirred and heated at 70 °C for 2 h. The reaction mixture was quenched with sat. aq. NaHCO₃ and the mixture diluted with DCM. The layers were separated, and the aqueous layer was extracted with DCM (3x). The combined organic extracts were dried over MgSO₄, filtered and concentrated in vacuo. The crude material was absorbed onto a plug of silica gel and purified by chromatography through a Redi-Sep pre-packed silica gel column (120 g), eluting with a gradient of 0-80% 3:1 EtOAc:EtOH in heptane, to provide tert-butyl ((6-hydroxypyridazin-3-yl)methyl)(1-(pyrimidin-2-yl)ethyl)carbamate (1.043 g, 3.15 mmol, 65.6 % yield) as white solid. *m*/*z* (ESI): 332.1 (M+H)⁺. ¹H NMR (400 MHz, DMSO-d6) δ ppm 12.77 (s, 1 H), 8.75 (d, J=4.8 Hz, 2 H), 7.37 (t, J=4.8 Hz, 2 H), 6.85 (d, J=9.6 Hz, 1 H), 4.88 - 5.05 (m, 1 H), 4.42 (br s, 2 H), 1.54 (d, J=7.3 Hz, 3 H), 1.15 - 1.34 (m, 9 H).

Step 3. Racemic tert-butyl ((6-hydroxypyridazin-3-yl)methyl)(1-(pyrimidin-2-yl)ethyl)carbamate (1.043 g) was purified via preparative SFC using a Chiral Technologies AD column (250 X 30 mm, 5mm) with a mobile phase of 80% Liquid CO₂ and 20% EtOH with 0.2% TEA using a flowrate of 150 mL/min. The 1^{st} eluting peak was tert-butyl (R)-((6-hydroxypyridazin-3-yl)methyl)(1-(pyrimidin-2-yl)ethyl)carbamate (430 mg, >99% *ee*) . The 2^{nd} eluting peak was tert-butyl (S)-((6-hydroxypyridazin-3-yl)methyl)(1-(pyrimidin-2-yl)ethyl)carbamate (455 mg, 98.8% *ee*)*.* Peak assignment determined by SFC with AD column with 10% EtOH with 0.2% TEA. Peak 1 is the more active enantiomer.

Step 4. To a 50-mL round-bottomed flask was added tert-butyl (R)-((6-hydroxypyridazin-3-yl)methyl)(1-(pyrimidin-2-yl)ethyl)carbamate (0.20 g, 0.62 mmol) and cesium carbonate (0.25 g, 0.78 mmol, Sigma-Aldrich Corporation) in N, N-dimethylformamide (5.23 mL). Then, 2,2,2-trifluoroethyl triflate (0.18 g, 0.78 mmol, Combi-Blocks Inc.) was added to the reaction mixture over 5 min. The resulting reaction mixture was stirred at rt overnight then it was concentrated in vacuo. The crude material was absorbed onto a plug of silica gel and purified by chromatography through a Redi-Sep pre-packed silica gel column (40 g), eluting with a gradient of 0-60% MeOH in CH₂Cl₂, to provide tert-butyl (R)-(1-(pyrimidin-2-yl)ethyl)((6-(2,2,2-trifluoroethoxy)pyridazin-3-yl)methyl)carbamate (0.20 g, 0.48 mmol, 77 % yield) as light-yellow solid. *m*/*z* (ESI): 414.1 (M+H)⁺.

Step 5. To a 50-mL round-bottomed flask was added tert-butyl (R)-(1-(pyrimidin-2-yl)ethyl)((6-(2,2,2-trifluoroethoxy)pyridazin-3-yl)methyl)carbamate (0.10 g, 0.25 mmol) and trifluoracetic acid (1.00 g, 0.65 mL, 8.81 mmol, Sigma-Aldrich Corporation) in dichloromethane (1.25 mL). The resulting reaction mixture was stirred at rt for 1 h. The crude (R)-1-(pyrimidin-2-yl)-N-((6-(2,2,2-trifluoroethoxy)pyridazin-3-yl)methyl)ethan-1-amine was concentrated in vacuo and carried to the next step of the synthesis, without further purification. *m*/*z* (ESI): 314.0 (M+H)⁺.

### Intermediate 197: (R)-N-((6-cyclopropylpyridazin-3-yl)methyl)-1-(pyrimidin-2-yl)ethan-1-amine

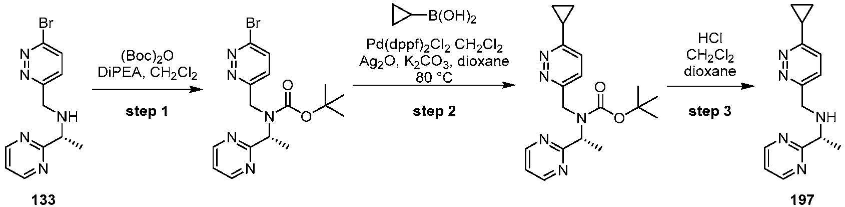

Step 1. (R)-N-((6-bromopyridazin-3-yl)methyl)-1-(pyrimidin-2-yl)ethan-1-amine (133, 0.8 g, 2.72 mmol) and DIPEA (0.703 g, 0.950 mL, 5.44 mmol, Aldrich) were stirred in dichloromethane (13.60 mL) and then di-tert-butyl dicarbonate (0.653 g, 0.695 mL, 2.99 mmol, Oakwood Products, Inc.) was added. The reaction was then stirred at room temp. for 4 hours. An additional 0.5 equiv of Boc₂O were added and after stirring overnight, the mixture was partitioned between 100 mL of DCM and water. The layers were separated. The organic layer was washed with brine, dried over Na₂SO₄, and concentrated. The crude product was purified by flash chromatography (silica, 10 to 100% EtOAc:Heptanes) to give tert-butyl (R)-((6-bromopyridazin-3-yl)methyl)(1-(pyrimidin-2-yl)ethyl)carbamate (1.16 g, 2.94 mmol, 108 % yield). *m*/*z* (ESI): 394, 396 (M+H)⁺.

Step 2. A mixture of tert-butyl (R)-((6-bromopyridazin-3-yl)methyl)(1-(pyrimidin-2-yl)ethyl)carbamate (0.2 g, 0.507 mmol), cyclopropylboronic acid (0.218 g, 2.54 mmol, Combi-Blocks), [1,1'-bis(diphenylphosphino)ferrocene]-dichloropalladium (ii) dichloromethane complex (0.041 g, 0.051 mmol, Oakwood Products, Inc.), silver (i) oxide (0.223 g, 0.964 mmol, Sigma-Aldrich Corporation), potassium carbonate (0.210 g, 1.522 mmol, Acros) and 1,4-dioxane (5 mL) was purged with Ar, then stirred in a sealed vial at 80°C for 4.5 h. Then, the mixture was filtered through Celite and concentrated in vacuo. The crude material was purified by chromatography through a silica gel column, eluting with 0-100% 3/1 EtOAc/EtOH in heptane. tert-Butyl (R)-((6-cyclopropylpyridazin-3-yl)methyl)(1-(pyrimidin-2-yl)ethyl)carbamate (149.2 mg, 0.42 mmol, 83% yield) was obtained as off-white solid and used in the next step. *m*/*z* (ESI): 356.3 (M+H)⁺

Step 3. To a solution of tert-butyl (R)-((6-cyclopropylpyridazin-3-yl)methyl)(1-(pyrimidin-2-yl)ethyl)carbamate (0.14 g, 0.394 mmol) in dichloromethane (4 mL) was added HCl, 4.0 M in dioxane (0.788 mL, 3.15 mmol, Aldrich), causing the solution became a suspension. MeOH was added to make the suspension to a solution again. The mixture was stirred was stirred at rt overnight and then concentrated in vacuo and (R)-N-((6-cyclopropylpyridazin-3-yl)methyl)-1-(pyrimidin-2-yl)ethan-1-amine (155 mg, 0.425 mmol, 108% yield) was obtained as orange solid. *m*/*z* (ESI): 256 (M+H)⁺

### Intermediate 198: 1-cyclopropyl-N-((6-morpholinopyridazin-3-yl)methyl)methanamine

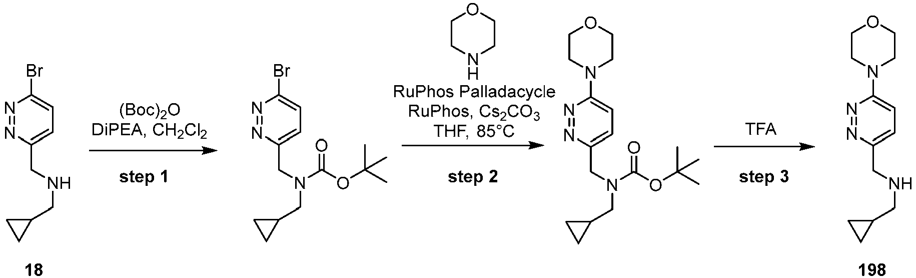

Step 1. 1-(6-bromopyridazin-3-yl)-N-(cyclopropylmethyl)methanamine (**18**, 0.84 g, 3.47 mmol) and DIPEA (0.897 g, 1.21 mL, 6.94 mmol, Aldrich) were stirred in dichloromethane (17.4 mL) and then di-tert-butyl dicarbonate (1.21 g, 1.29 mL, 5.55 mmol, Oakwood Products, Inc.) was added. The reaction was stirred at room temperature overnight. The mixture was then partitioned between 200 mL of DCM and water. The layers were separated. The organic layer was dried over Na₂SO₄ and concentrated to give crude tert-butyl ((6-bromopyridazin-3-yl)methyl)(cyclopropylmethyl)carbamate (1.31 g, 3.83 mmol, 110% yield), that is contaminated with ~30% Boc anhydride side-product. This material was used successfully in the next reaction. *m*/*z* (ESI): 342, 344 (M+H)^{+ 1}H NMR (400 MHz, DMSO-d6) δ ppm 7.11 (br d, J=8.6 Hz, 1 H), 6.76 (d, J=9.0 Hz, 1 H), 4.02 (s, 1 H), 3.96 (s, 1 H), 2.40 - 2.46 (m, 1 H), 0.54 - 0.73 (m, 10 H), 0.25 - 0.29 (m, 2 H), 0.18 (br s, 1 H), -0.36 (br d, J=7.3 Hz, 1 H), -0.61 (br s, 1 H)

Step 2. RuPhos Palladacycle G1 (298 mg, 0.365 mmol, Strem), RuPhos (170 mg, 0.365 mmol, Strem), morpholine (256 µL, 255 mg, 2.92 mmol, Aldrich), cesium carbonate (1.55 g, 4.75 mmol, Aldrich), and tert-butyl ((6-bromopyridazin-3-yl)methyl)(cyclopropylmethyl)carbamate (500 mg, 1.46 mmol) were combined in THF and heated at 85 °C for 2.5 hours. The reaction mixture was then diluted with EtOAc and filtered over a pad of diatomaceous earth. The residue was purified by medium pressure chromatography (silica, 0 to 100% EtOAc:Heptanes) to give tert-butyl (cyclopropylmethyl)((6-morpholinopyridazin-3-yl)methyl)carbamate (430 mg, 1.234 mmol, 84% yield). *m*/*z* (ESI): 349.1 (M+H)^{+ 1}H NMR (400 MHz, CHLOROFORM-d) δ ppm 7.29 - 7.41 (m, 1 H), 6.88 (d, J=9.4 Hz, 1 H), 4.68 (s, 2 H), 3.78 - 3.86 (m, 4 H), 3.55 - 3.63 (m, 4 H), 3.03 - 3.20 (m, 2 H), 1.46 (br s, 9 H), 0.87 - 1.00 (m, 1 H), 0.37 - 0.43 (m, 2 H), 0.17 (br s, 2 H).

Step 3. tert-butyl (cyclopropylmethyl)((6-morpholinopyridazin-3-yl)methyl)carbamate was dissolved in TFA (12.5 mL) and stirred for 15 minutes to completion. The reaction mixture was the concentrated under reduced pressure and the residue was dissolved in MeOH, eluted through an SCX column with 0 to 2M ammonia in MeOH, and concentrated to give 1-cyclopropyl-N-((6-morpholinopyridazin-3-yl)methyl)methanamine (198, 90.0 mg, 0.362 mmol, 24.8 % yield). *m*/*z* (ESI): 249.2 (M+H)⁺

### Intermediate 199: methyl 4-(6-((methylamino)methyl)pyridin-3-yl)pipe159razine-1-carboxylate

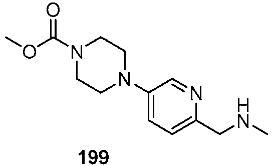

Intermediate 199 was prepared in a fashion similar to that described above for amine **198.** *m*/*z* (ESI): 265.2 (M+H)⁺

### Intermediate 200: (S)-1-Cyclopropyl-2-methoxy-N-((6-morpholinopyridazin-3-yl)methyl)ethan-1-amine

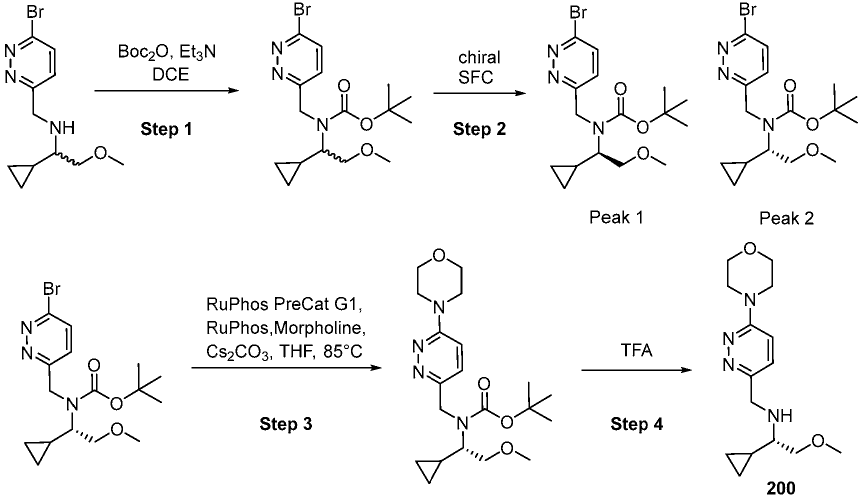

Step 1. To a 100-mL round-bottomed flask was added N-((6-bromopyridazin-3-yl)methyl)-1-cyclopropyl-2-methoxyethan-1-amine (0.075 g, 0.26 mmol) and triethylamine (0.080 g, 0.11 mL, 0.79 mmol, Sigma-Aldrich Corporation) in 1,2-dichloroethane (1.30 mL). Then, di-tert-butyl dicarbonate (0.086 g, 0.091 mL, 0.390 mmol, Sigma-Aldrich Corporation) was added to the reaction mixture. The overall reaction mixture was stirred and heated at 70 °C for 2 h. The reaction mixture was quenched with sat. aq. NaHCO₃ and the mixture diluted with DCM. The layers were separated, and the aqueous layer was extracted with DCM (3x). The combined organic extracts were dried over MgSO₄, filtered and concentrated *in vacuo.* The crude material was absorbed onto a plug of silica gel and purified by chromatography through a Redi-Sep pre-packed silica gel column (12 g), eluting with a gradient of 0-30% 3:1 EtOAc:EtOH in heptane, to provide racemic tert-butyl ((6-bromopyridazin-3-yl)methyl)(1-cyclopropyl-2-methoxyethyl)carbamate (0.085 g, 0.220 mmol, 84 % yield) as tan solid. *m*/*z* (ESI): 386.0 (M+H)⁺.

Step 2. The racemic sample was purified via preparative SFC using a Chiral Technologies IG column (250 X 21 mm, 5mm) with a mobile phase of 90% Liquid CO₂ and 10% iPrOH with 0.2% TEA using a flowrate of 80 mL/min to generate 390 mg of peak 1 with an *ee* of 98% and 380 mg of peak 2 with an ee of 98%. Absolute stereochemistry was assigned arbitrarily for these isomers and the more potent peak 2 was taken forward below as the (S)-isomer.

Step 3. Tert-butyl (*S*)-((6-bromopyridazin-3-yl)methyl)(1-cyclopropyl-2-methoxyethyl)carbamate (peak 2, 170 mg, 0.440 mmol), RuPhos (51.0 mg, 0.110 mmol, Aldrich), RuPhos PreCat G1 (90.0 mg, 0.110 mmol, Strem), cesium carbonate (470 mg, 1.40 mmol, Aldrich) and morpholine (0.077 mL, 0.88 mmol, Spectrum) were combined in degassed THF (2.9 mL) and heated at 85 °C for two hours to completion. The reaction was then cooled and diluted with EtOAc and filtered over a pad of diatomaceous earth. The filtrate was then concentrated and the residue was purified by medium pressure chromatography (silica, 0 to 100% EtOAc:Heptanes to 30 to 100% (3:1 EtOAc:EtOH:Heptanes) to give impure tert-butyl (S)-(1-cyclopropyl-2-methoxyethyl)((6-morpholinopyridazin-3-yl)methyl)carbamate.

Step 4. This material was dissolved in TFA (10 mL) and stirred for 10 minutes. The reaction mixture was then concentrated and the residue was then eluted through an SCX column eluting with 0 to 2M ammonia in MeOH and cocentrated to give (S)-1-cyclopropyl-2-methoxy-N-((6-morpholinopyridazin-3-yl)methyl)ethan-1-amine (110 mg, 0.37 mmol, 83% yield). *m*/*z* (ESI): 293.1 (M+H)⁺.

### Intermediate 201: N-((6-ethoxypyridazin-3-yl)methyl)-2-methylpropan-1-amine

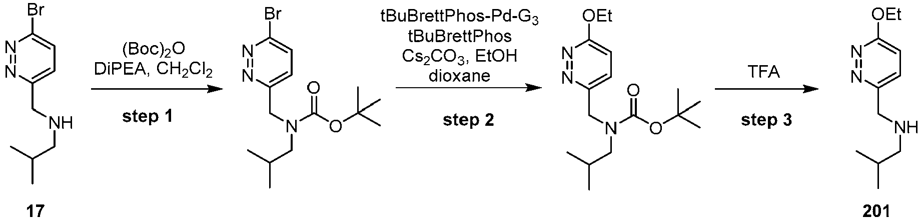

Step 1. N-((6-bromopyridazin-3-yl)methyl)-2-methylpropan-1-amine (**17**, 0.950 g, 3.89 mmol) and DIPEA (1.01 g, 1.36 mL, 7.78 mmol, Aldrich) were stirred in dichloromethane (19.5 mL) and then di-tert-butyl dicarbonate (1.36 g, 1.45 mL, 6.23 mmol, Oakwood Products, Inc.) was added. The reaction was then stirred at room temperature overnight. The mixture was then partitioned between 200 mL of DCM and water. The layers were separated. The organic layer was dried over MgSO₄ and concentrated to give crude tert-butyl ((6-bromopyridazin-3-yl)methyl)(isobutyl)carbamate (1.89 g, 5.49 mmol, 141 % yield) that was ~30% contaminated with Boc anhydride side-product. This material was used directly in the next reaction. *m*/*z* (ESI): 344, 346 (M+H)⁺

Step 2. A re-sealable screw-cap test tube (Tube A) was charged with tBuBrettPhos (170 mg, 0.350 mmol, 0.150 equiv), cesium carbonate (1.10 g, 3.30 mmol, 1.40 equiv), and tert-butyl ((6-bromopyridazin-3-yl)methyl)(isobutyl)carbamate (800 mg, 2.3 mmol, 1.0 equiv). Tube A was evacuated and backfilled with argon (3x), and ethanol (1000 µL, 17.0 mmol, 7.50 equiv) was then added into tube A via syringe. Simultaneously, a re-sealable screw-cap test tube equipped with a Teflon-coated magnetic stir bar (Tube B) was charged with tBuBrettPhos Pd G3 (300 mg, 0.350 mmol, 0.150 equiv). Tube B was then evacuated and backfilled with argon (3x), and 1,4-dioxane (12.0 mL) was added into tube B via syringe. The reaction mixture in tube B was stirred at room temperature for ~1 min to form a homogeneous solution. The pre-catalyst solution from tube B was transferred into tube A via syringe. The resulting reaction mixture in tube A was stirred at room temperature for 20 h. The crude product was diluted with ethyl acetate and concentrated in vacuo with the aid of a rotary evaporator. The crude product residue was purified by flash column chromatography by medium pressure chromatography (silica, 0 to 100% EtOAc:Heptanes) to afford ((6-ethoxypyridazin-3-yl)methyl)(isobutyl)carbamate. *m*/*z* (ESI): 310.1 (M+H)⁺

Step 3. This material was then dissolved in TFA (10 mL) and stirred for 15 minutes to completion. The reaction mixture was then concentrated under reduced pressure and the residue was free based by dissolving in MeOH, eluting through an SCX column eluting with 0 to 2M ammonia in MeOH, and concentrating to give N-((6-ethoxypyridazin-3-yl)methyl)-2-methylpropan-1-amine with about 80% purity that was used successfully in the next reaction. 100 mg was obtained on first pass though SCX column with a trace of TFA present. *m*/*z* (ESI): 210.1 (M+H)^{+ 1}H NMR (400 MHz, CHLOROFORM-d) δ ppm 7.41 - 7.52 (m, 1 H), 6.92 (d, J=9.0 Hz, 1 H), 4.57 (q, J=7.1 Hz, 2 H), 4.00 (s, 2 H), 2.43 - 2.51 (m, 2 H), 1.74 - 1.89 (m, 2 H), 1.45 (t, J=7.1 Hz, 3 H), 0.93 (d, J=6.5 Hz, 6 H)

### Intermediate 202: 1-(5-(3,6-dihydro-2H-pyran-4-yl)pyridin-2-yl)-N-methylmethanamine

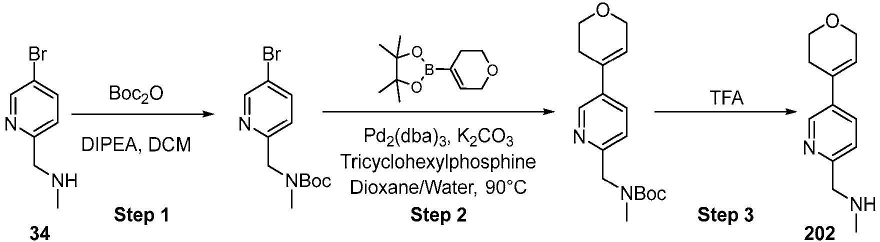

Step 1. 1-(5-Bromopyridin-2-yl)-N-methylmethanamine (0.950 g, 4.72 mmol, **34**) and DIPEA (1.22 g, 1.65 mL, 9.45 mmol, Aldrich) were stirred in dichloromethane (23.6 mL) and the di-tert-butyl dicarbonate (1.65 g, 1.76 mL, 7.56 mmol, Oakwood Products, Inc.) was added. The reaction was then stirred at room temp. overnight to completion. The mixture was then partitioned between 200 mL of DCM and 50 mL of water. The layers were separated. The organic layer was dried over MgSO₄ and concentrated to give crude tert-butyl ((5-bromopyridin-2-yl)methyl)(methyl)carbamate (1.42 g, 4.71 mmol, 100 % yield). *m*/*z* (ESI): 301.2, 303.1 (M+H)⁺.

Steps 2. The 2-(3,6-dihydro-2H-pyran-4-yl)-4,4,5,5-tetramethyl-1,3,2-dioxaborolane (837 mg, 3.98 mmol, Combi-Blocks), tricyclohexylphosphine (112 mg, 0.398 mmol, Strem), tert-butyl ((5-bromopyridin-2-yl)methyl)(methyl)carbamate (0.600 g, 1.99 mmol, From Step 1) and Pd₂(dba)₃ (182 mg, 0.199 mmol, Acros) were slurried in dioxane (7.00 mL) and then sparged with argon. The potassium carbonate (1.30 M solution) (4.14 mL, 5.38 mmol, Aldrich) was then added and the reaction mixture was heated to 90 °C for one hour. The reaction was cooled and then concentrated to a reduced volume. This residue was then taken up in water (30 mL) and extracted with dichloromethane (2 x 80 mL). The combined organic layers were dried over magnesium sulfate and concentrated. The crude product was purified by medium pressure chromatography (silica, 0 to 100% EtOAc:Heptanes) to give tert-butyl ((5-(3,6-dihydro-2H-pyran-4-yl)pyridin-2-yl)methyl)(methyl)carbamate (372 mg, 1.22 mmol, 61.3 % yield).

Step 3. This material was then dissolved in 7 mL of TFA and stirred for 10 minutes resulting in complete deprotection. The reaction mixture was then concentrated and the resulting TFA salt was free based by dissolving in MeOH, eluting through an SCX column using 0 to 2M ammonia in MeOH, and concentrating to give 1-(5-(3,6-dihydro-2H-pyran-4-yl)pyridin-2-yl)-N-methylmethanamine (90.0 mg, 440 mmol, 22.1% yield). *m*/*z* (ESI): 205.2 (M+H)⁺.

### Intermediate 203: 6-((methylamino)methyl)-3',6'-dihydro-[3,4'-bipyridine]-1'(2'H)-carboxylate

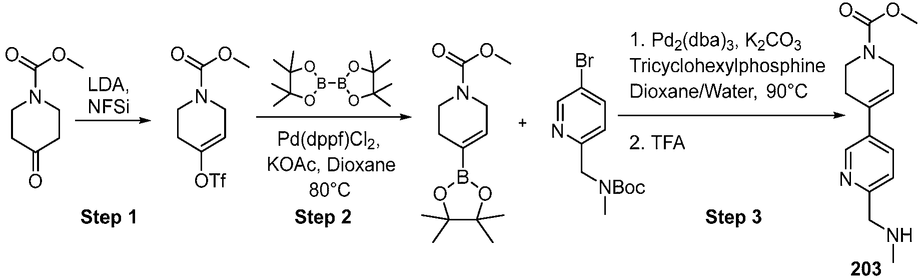

Step 1. Diisopropylamine (185 mg, 0.261 mL, 1.83 mmol, Aldrich) was dissolved in THF (7.00 mL) and cooled to -78 °C. Then, n-butyllithium (2.50 M in hexanes) (0.732 mL, 1.83 mmol, Aldrich) was added dropwise at -78 °C and stirred for 25 minutes. The mixture was raised out of the dry ice bath for 15 minutes then resubmerged. Methyl 4-oxopiperidine-1-carboxylate (250 mg, 1.59 mmol, Combi-Blocks) was then dissolved in THF (4.00 mL) and added slowly to the LDA solution at -78 °C and stirred for 45 minutes. N-phenyl-bis(trifluoromethanesulfonimide) (625 mg, 1.75 mmol, Combi-Blocks) dissolved in THF (5.00 mL) was added slowly and the reaction mixture was allowed to stir overnight while warming to room temperature. The reaction mixture was quenched with water (20 mL) and the mixture was extracted with hexanes (3 x 50 mL). The combined organic layers were washed with brine and dried over magnesium sulfate. The crude product was purified by medium pressure chromatography (silica, 0 to 40% ethyl acetate:hexanes) to give methyl 4-(((trifluoromethyl)sulfonyl)oxy)-3,6-dihydropyridine-1(2H)-carboxylate (249 mg, 0.861 mmol, 54.1% yield). *m*/*z* (ESI): 290.1 (M+H)⁺.

Step 2. The methyl 4-(((trifluoromethyl)sulfonyl)oxy)-3,6-dihydropyridine-1(2H)-carboxylate (230 mg, 0.795 mmol), bis(pinacolato)diboron (242 mg, 0.954 mmol, Aldrich), 1,1'-bis(diphenylphosphino)ferrocene-palladium dichloride (64.9 mg, 0.080 mmol, Strem Chemicals, Inc.), and potassium acetate (312 mg, 3.18 mmol, Aldrich) were added to a flask with dioxane (2.65 mL). This mixture was heated at 80 °C overnight. The reaction mixture was cooled, filtered, and washed with ethyl acetate. The filtrate was concentrated and purified by medium pressure chromatography (silica, 0 to 60% EtOAc : heptanes) to give methyl 4-(4,4,5,5-tetramethyl-1,3,2-dioxaborolan-2-yl)-3,6-dihydropyridine-1(2H)-carboxylate (130 mg, 0.487 mmol, 61.2% yield) *m*/*z* (ESI): 268.2 (M+H)⁺.

Step 3. Methyl 4-(4,4,5,5-tetramethyl-1,3,2-dioxaborolan-2-yl)-3,6-dihydropyridine-1(2H)-carboxylate (133 mg, 0.498 mmol, From Step 2), tricyclohexylphosphine (27.9 mg, 0.100 mmol, Strem), tert-butyl ((5-bromopyridin-2-yl)methyl)(methyl)carbamate (0.150 g, 0.498 mmol, Boc-**3b**4, see Step 1 for intermediate 202) and Pd₂(dba)₃ (45.6 mg, 0.050 mmol, Acros) were slurried in dioxane (1.74 mL) and sparged with argon. Potassium carbonate (1.30 M soln.) (1.03 mL, 1.35 mmol, Aldrich) was added and the reaction mixture was heated to 90°C for one hour. The reaction was cooled and concentrated to a reduced volume. This residue was taken up in water (15 mL) and extracted with dichloromethane (2 x 40 mL). The combined organic layers were dried over magnesium sulfate and concentrated. The crude product was purified by medium pressure chromatography (silica, 0 to 100% EtOAc:Heptanes) to give methyl 6-(((tert-butoxycarbonyl)(methyl)amino)methyl)-3',6'-dihydro-[3,4'-bipyridine]-1'(2'H)-carboxylate (93.0 mg, 0.257 mmol, 51.7 % yield). The material was dissolved in TFA and stirred for 10 minutes to Boc-deprotect. The mixture was concentrated to give the TFA salt of the desired product. The salt was then free based by eluting through an SCX column eluting with 0 to 2M ammonia in methanol and concentrating to give methyl 6-((methylamino)methyl)-3',6'-dihydro-[3,4'-bipyridine]-1'(2'H)-carboxylate (63.0 mg, 0.241 mmol, 48.4 % yield) *m*/*z* (ESI): 262.2 (M+H)⁺.

### Intermediate 204: (R)-N-ethyl-1-(2-fluoro-4-(trifluoromethyl)phenyl)ethan-1-amine'

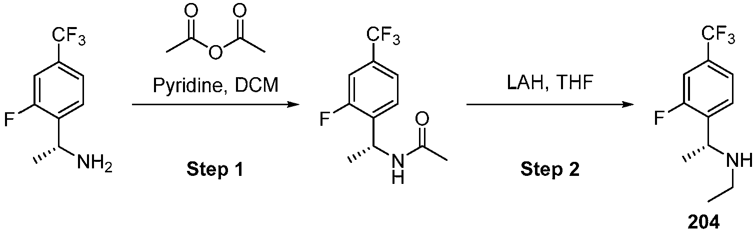

Step 1. To a 100-mL 2-neck round-bottomed flask was added (R)-1-(2-fluoro-4-(trifluoromethyl)phenyl)ethan-1-amine (0.50 g, 2.41 mmol, AP Bioscience) and pyridine (0.27 g, 0.27 mL, 3.38 mmol, Sigma-Aldrich Corporation) in dichloromethane (12 mL). The reaction mixture was cooled to -78 °C, then acetic anhydride (0.30 g, 0.27 mL, 2.90 mmol, Sigma-Aldrich Corporation) was added dropwise to the reaction mixture over 2 min, while under an inert (N2) atmosphere. The ice bath was removed, and the reaction mixture was stirred at rt for 2 h. The reaction mixture was cooled to 0°C, then the reaction mixture was quenched with 1 N HCl (2.4 mL). This mixture was diluted with heptane (20 mL), washed with 1N HCl (6 mL x 2), then sat. aq. NaHCO₃ (12 mL) and brine (12 mL). The organics were collected, then dried over MgSO₄, filtered and concentrated in vacuo, to provide (R)-N-(1-(2-fluoro-4-(trifluoromethyl)phenyl)ethyl)acetamide (0.43 g, 1.73 mmol, 72% yield) as white solid. ¹H NMR (400 MHz, *DMSO-d*₆) δ ppm 8.48 (br d, *J*=7.3 Hz, 1 H), 7.53 - 7.66 (m, 3 H), 5.13 (quin, *J*=7.2 Hz, 1 H), 1.86 (s, 3 H), 1.35 (d, *J*=7.1 Hz, 3 H). *m*/*z* (ESI): 250.0 (M+H)⁺.

Step 2. To a 150-mL round-bottomed flask was added (R)-N-(1-(2-fluoro-4-(trifluoromethyl)phenyl)ethyl)acetamide (0.42 g, 1.69 mmol) in tetrahydrofuran (9 mL). Then lithium aluminum hydride solution, 2.0 M in tetrahydrofuran (2.1 mL, 4.21 mmol, Sigma-Aldrich Corporation) was added slowly to the reaction mixture over 2 min. The resulting reaction mixture was stirred at rt for 1 h, then the mixture was stirred and heated at 55 °C for 5 h. The reaction mixture was diluted with heptane (15 mL) and cooled to 0°C. Then water (0.4 mL) was added to the mixture and stirred 1 min. Then aq. 15% NaOH (0.4 mL) was added to the mixture and stirred 1 min. Then, water (3 x 1.2 mL) was added to the mixture and the resulting mixture was warmed to rt over 15 min. MgSO₄ was added to the mixture and stirred an additional 15 min. The overall reaction mixture was filtered through a pad of Celite, then the filtrate was collected and concentrated in *vacuo.* The crude material was absorbed onto a plug of silica gel and purified by chromatography through a Redi-Sep pre-packed silica gel column (40 g), eluting with a gradient of 0-60% EtOAc:EtOH (3:1) in heptane, to provide (R)-N-ethyl-1-(2-fluoro-4-(trifluoromethyl)phenyl)ethan-1-amine (0.081 g, 0.344 mmol, 20.43 % yield) as light-yellow oil. ¹H NMR (400 MHz, *DMSO-d*₆) δ ppm 7.76 (t, *J*=7.7 Hz, 1 H), 7.53 - 7.61 (m, 2 H), 4.07 (q, *J*=6.6 Hz, 1 H), 2.26 - 2.40 (m, 2 H), 2.08 - 2.17 (m, 1 H), 1.26 (d, *J*=6.7 Hz, 3 H), 0.98 (t, *J*=7.1 Hz, 3 H). *m*/*z* (ESI): 236.1 (M+H)⁺.

### Intermediate 205: (R)-N-ethyl-1-(4-(trifluoromethyl)phenyl)ethan-1-amine

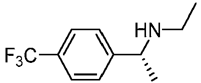

Intermediate 205 was prepared in a manner similar to that described for Intermediate 204. *m*/*z* (ESI): 218.1 (M+H)⁺

### Intermediate 206: Preparation of (S)-N-(cyclopropyl(5-(trifluoromethyl)pyridin-2-yl)methyl)ethanamine

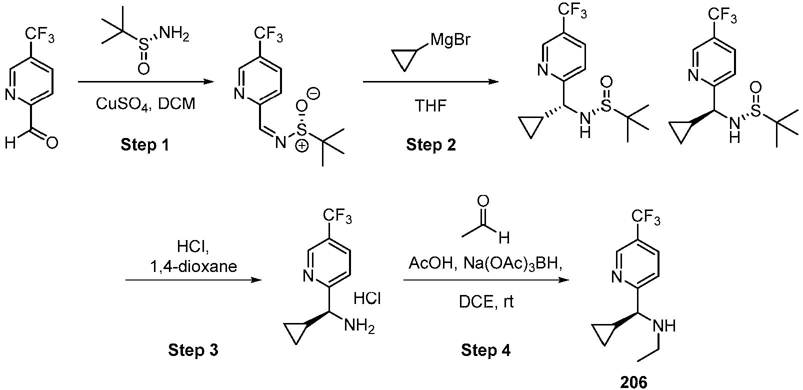

Step 1. To an oven-dried 100-mL round-bottomed flask was added (R)-(+)-2-methyl-2-propanesulfinamide (0.50 g, 4.13 mmol, AK Scientific, Inc.) in dichloromethane (8.25 mL). To this mixture was added copper (ii) sulfate (1.32 g, 8.25 mmol, Sigma-Aldrich Corporation) followed by 5-(trifluoromethyl)picolinaldehyde (0.75 g, 4.13 mmol, J&W Pharmlab). The resulting reaction mixture was stirred at rt for 24 h. The reaction mixture was filtered through a pad of Celite and the filter cake was washed well with DCM. The filtrate was collected and concentrated *in vacuo.* The crude was purified by flash chromatography (silica, 0-20% EtOAc:EtOH (3:1) in heptane), to provide (R,E)-2-methyl-N-((5-(trifluoromethyl)pyridin-2-yl)methylene)propane-2-sulfinamide (1.105 g, 3.97 mmol, 96 % yield) as off-white solid. ¹H NMR (400 MHz, *DMSO-d*₆) δ ppm 9.15 - 9.19 (m, 1 H), 8.56 (s, 1 H), 8.42 (dd, *J*=8.2, 2.3 Hz, 1 H), 8.28 (d, *J*=8.2 Hz, 1 H), 1.23 (s, 9 H). *m*/*z* (ESI): 279.0 (M+H)⁺.

Step 2. To an oven-dried 100-mL 2-neck round-bottomed flask was added (R,E)-2-methyl-N-((5-(trifluoromethyl)pyridin-2-yl)methylene)propane-2-sulfinamide (0.40 g, 1.44 mmol) in tetrahydrofuran (7.19 mL). The reaction mixture was cooled to -78 °C, then cyclopropylmagnesium bromide solution, 0.5 M in THF (5.17 mL, 2.59 mmol, Sigma-Aldrich Corporation) was added dropwise to the reaction mixture. After 10 min, the reaction was quenched with the addition of sat. aq. NH₄Cl (5.8 mL) and extracted with EtOAc (3 x 25 mL). The combined organic extracts were dried over MgSO₄, filtered and concentrated in vacuo. The crude was purified by flash chromatography (silica, 0-60% EtOAc:DCM), to provide both diastereomers with peak 1 being assigned as the (R)-Sulfinamine (0.176 g, 0.55 mmol, 38% yield), a light-yellow oil. ¹H NMR (400 MHz, DMSO*-d*₆) δ ppm 8.91 (s, 1 H), 8.24 (dd, *J*=8.4, 2.3 Hz, 1 H), 7.79 (d, J=8.4 Hz, 1 H), 5.82 (d, *J=*7.5 Hz, 1 H), 3.81 (t, *J*=8.0 Hz, 1 H), 1.12 - 1.24 (m, 10 H), 0.36 - 0.62 (m, 4 H). *m*/*z* (ESI): 321.1 (M+H)⁺. Peak 2 was assigned as the (S)-Sulfinamine (0.099 g, 0.309 mmol, 22 % yield), awhite solid. ¹H NMR (400 MHz, DMSO*-d*₆) δ ppm 8.89 (s, 1 H), 8.24 (dd, *J*=8.4, 2.3 Hz, 1 H), 7.75 (d, *J*=8.4 Hz, 1 H), 5.64 (d, *J*=6.3 Hz, 1 H), 3.74 (dd, *J*=9.0, 6.5 Hz, 1 H), 1.25 - 1.32 (m, 1 H), 1.10 (s, 9 H), 0.59 - 0.64 (m, 1 H), 0.42 - 0.51 (m, 3 H). *m*/*z* (ESI): 321.1 (M+H)⁺. Absolute stereochemistry was assigned to sulfinimine intermediates based on analogy to literature examples (Tetrahedron Letters, S. D. Kuduk et al, 45 (2004) 6641-6643) and to purchased enantiopure amines.

Step 3. To a 50-mL round-bottomed flask was added (S)-Sulfinamide (0.16 g, 0.48 mmol, Peak 2) and hydrogen chloride solution, 4.0 M in dioxane (0.15 mL, 0.61 mmol, Sigma-Aldrich Corporation) in 1,4-dioxane (2.42 mL). The resulting reaction mixture was stirred at rt for 10 min. The reaction mixture was concentrated in vacuo and the crude was carried to the next step of the synthesis, without further purification. *m*/*z* (ESI): 217.0 (M+H)⁺.

Step 4. To a 50-mL round-bottomed flask was added (S)-cyclopropyl(5-(trifluoromethyl)pyridin-2-yl)methanamine hydrochloride (0.12 g, 0.48 mmol) and acetaldehyde (0.03 g, 0.03 mL, 0.61 mmol, Sigma-Aldrich Corporation) in dichloromethane (2.4 mL). Then titanium (IV) isopropoxide (0.17 g, 0.18 mL, 0.60 mmol, Aldrich) was added to the reaction mixture and stirred at rt for 16 h. The mixture was cooled to 0 °C, then methanol (0.16 g, 0.2 mL, 4.83 mmol, Sigma-Aldrich Corporation) was added to the mixture, followed by sodium borohydride (0.02 g, 0.48 mmol, Aldrich) and the resulting reaction mixture was stirred 2 h. The reaction mixture was concentrated in vacuo. The crude was purified by flash chromatography (silica, 0-35% MeOH:DCM), to provide (S)-N-(cyclopropyl(5-(trifluoromethyl)pyridin-2-yl)methyl)ethanamine (0.040 g, 0.164 mmol, 33.9 % yield) as tan solid. *m*/*z* (ESI): 245.1 (M+H)⁺.

Primary and Secondary Amines in Table 13 were prepared in a manner similar to that described for Intermediate 206.

**Table 13**

| **Int. #** | **Chemical Structure** | **Name** | *m*/*z* (ESI): (M+H)⁺ |
|---|---|---|---|
| **207** | 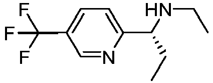 | (R)-N-ethyl-1-(5-(trifluoromethyl)pyridin-2-yl)propan-1-amine | 233.1 |
| **208** | 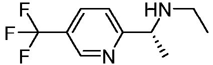 | (R)-N-ethyl-1-(5-(trifluoromethyl)pyridin-2-yl)ethan-1-amine | 219.1 |
| **209** | 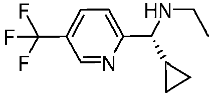 | (R)-N-(cyclopropyl(5-(trifluoromethyl)pyridin-2-yl)methyl)ethanamine | 245.1 |
| **210** | 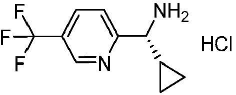 | (R)-cyclopropyl(5-(trifluoromethyl)pyridin-2-yl)methanamine | 217.0 |

### Intermediate 211: N-methyl-1-(5-(trifluoromethyl)pyridin-2-yl)propan-1-amine

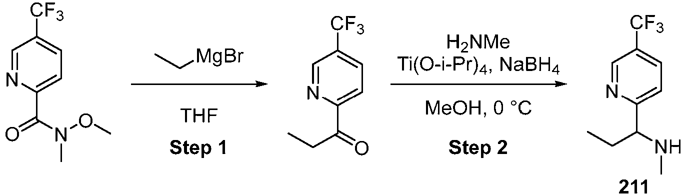

Step 1. To an oven-dried 2-neck 100-mL round-bottomed flask was added N-methoxy-N-methyl-5-(trifluoromethyl)picolinamide (0.29 g, 1.21 mmol, J&W Pharmlab) in tetrahydrofuran (6.1 mL). The reaction mixture was chilled to -78 °C, then ethylmagnesium chloride solution, 2.0 M in THF (1.83 mL, 3.65 mmol, Sigma-Aldrich Corporation) was added dropwise to the reaction mixture. The resulting reaction mixture was stirred for 15 min at -78 °C, then the mixture was quenched with sat. aq. NH₄Cl (6 mL). The mixture was warmed to rt. Then the reaction mixture was diluted with EtOAc (30 mL) and the aqueous layer was extracted with EtOAc (3x). The combined organic extracts were dried over MgSO₄, filtered and concentrated in vacuo. The crude was used without further purification. *m*/*z* (ESI): 204.0 (M+H)⁺.

Step 2. To a 50-mL round-bottomed flask was added 1-(5-(trifluoromethyl)pyridin-2-yl)propan-1-one (0.10 g, 0.49 mmol) and methylamine solution, 2.0 M in tetrahydrofuran (0.37 mL, 0.74 mmol, Sigma-Aldrich Corporation) in methanol (2.5 mL). Then titanium (IV) isopropoxide (0.18 g, 0.18 mL, 0.62 mmol, Sigma-Aldrich) was added to the reaction mixture. The resulting reaction mixture was stirred at rt for 30 min, while under an inert atmosphere. Then the mixture was cooled to 0 °C and sodium borohydride (0.09 g, 2.46 mmol, Sigma-Aldrich) was added slowly to the reaction mixture. The mixture was stirred at rt for 1 h. The reaction mixture was treated with sat.aq. NaHCO₃ (0.5 mL) and the resulting mixture was stirred for 10 min. Then the mixture was diluted with MeOH (2 mL) and filtered through a pad of celite. The filtrate was concentrated in vacuo. This afforded N-methyl-1-(5-(trifluoromethyl)pyridin-2-yl)propan-1-amine as light-yellow solid. The mixture was carried to the next step of the synthesis, without further purification. *m*/*z* (ESI): 219.1 (M+H)⁺.

Secondary Amines in Table 14 were prepared in a manner similar to that described for Intermediate 211.

**Table 14**

| **Int. #** | **Chemical Structure** | **Name** | *m*/*z* (ESI): (M+H)⁺ |
|---|---|---|---|
| **212** | 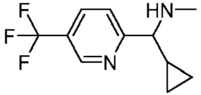 | 1-cyclopropyl-N-methyl-1-(5-(trifluoromethyl)pyridin-2-yl)methanamine | 231.1 |
| **213** | 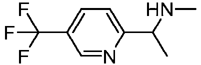 | N-methyl-1-(5-(trifluoromethyl)pyridin-2-yl)ethan-1-amine | 205.1 |

### Intermediate 214: (R)-N-((6-ethoxypyridazin-3-yl)methyl)-1-(pyrimidin-2-yl)ethan-1-amine

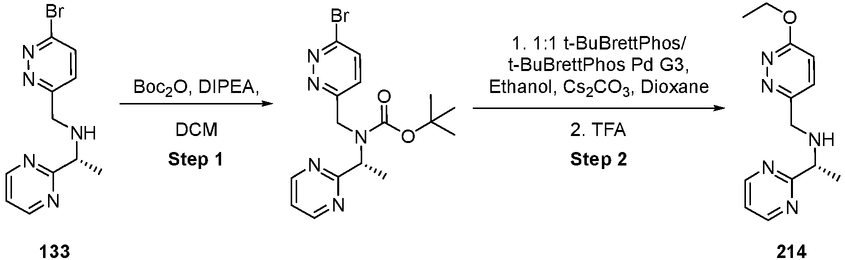

Step 1. (R)-N-((6-bromopyridazin-3-yl)methyl)-1-(pyrimidin-2-yl)ethan-1-amine (**133**, 0.840 g, 3.47 mmol) and DIPEA (0.897 g, 1.21 mL, 6.94 mmol, Aldrich) were stirred in dichloromethane (17.4 mL) and di-tert-butyl dicarbonate (1.21 g, 1.29 mL, 5.55 mmol, Oakwood Products, Inc.) was added. The reaction was then stirred at room temp overnight to completion. The mixture was partitioned between 200 mL of DCM and water. The layers were separated. The organic layer was dried over Na₂SO₄ and concentrated. The crude product was purified by medium pressure chromatography (silica, 0 to 100% EtOAc:Heptnes) to give tert-butyl (R)-((6-bromopyridazin-3-yl)methyl)(1-(pyrimidin-2-yl)ethyl)carbamate (1.61 g, 4.70 mmol, 136 % yield). *m*/*z* (ESI): 394.1, 396.1 (M+H)⁺.

Step 2. t-butylBrettPhos (55.0 mg, 0.110 mmol, Aldrich) was mixed in dioxane (1.0 mL). In a separate flask t-butylBrettPhos Pd G3 (98.0 mg, 0.110 mmol, Aldrich), ethanol (0.300 mL, 5.70 mmol, Aldrich), (R)-((6-bromopyridazin-3-yl)methyl)(1-(pyrimidin-2-yl)ethyl)carbamate (300 mg, 0.76 mmol) and cesium carbonate (350 mg, 1.10 mmol, Aldrich) were slurried in dioxane (2.50 mL). The t-butylBrettPhos mixture was added to the second flask. This slurry was then stirred overnight to completion. The mixture was concentrated under reduced pressure and the residue was purified by medium pressure chromatography (silica, 0 to 100% EtOAc:Heptanes) to give tert-butyl (R)-((6-ethoxypyridazin-3-yl)methyl)(1-(pyrimidin-2-yl)ethyl)carbamate (231 mg, 0.643 mmol, 84.0 % yield). *m*/*z* (ESI): 360.0 (M+H)⁺. This material was dissolved in TFA (10 mL) and stirred for 15 minutes to completion The reaction mixture was concentrated under reduced pressure and the residue was free based by dissolving in MeOH, eluting through an SCX column eluting with 0 to 2M ammonia in MeOH, and concentrating to give (R)-N-((6-ethoxypyridazin-3-yl)methyl)-1-(pyrimidin-2-yl)ethan-1-amine (**214**, 166 mg, 0.640 mmol, 84 % yield). *m*/*z* (ESI): 260.0 (M+H)⁺.

### Intermediate 215: 4-Amino-1,3-dihydrofuro[3,4-c]quinoline-8-carboxylic acid.

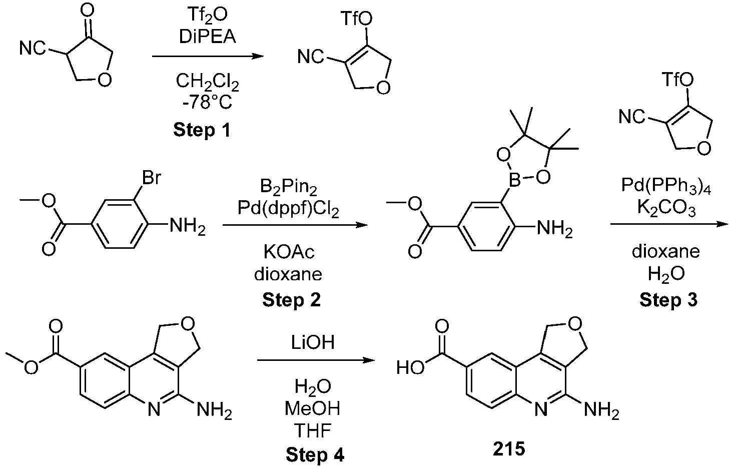

Step 1. To a stirred solution of 4-oxotetrahydrofuran-3-carbonitrile (0.500 g, 4.50 mmol) in dichloromethane (5.00 mL) was added DIPEA (0.943 mL, 5.40 mmol) and the reaction mixture was cooled to -78 °C. Then, triflic anhydride (0.760 mL, 4.50 mmol) was added dropwise at -78 °C for 1 min and the reaction mixture stirred at same temperature for 15 min. After completion of reaction, the reaction mixture was diluted with water, the organic layer was separated, washed with brine (2 x 10 mL), dried over sodium sulfate, and concentrated to give crude 4-cyano-2,5-dihydrofuran-3-yl trifluoromethanesulfonate (1.05 g, 4.32 mmol, 96 % yield), which was used in the next step without further purification.

Step 2. To a 150-mL round-bottomed flask was added methyl 4-amino-3-bromobenzoate (4 g, 17.39 mmol, Combi-Blocks Inc.) and bis(pinacolato)diboron (8.83 g, 34.8 mmol, Frontier Scientific, Inc.) in 1,4-dioxane (58.0 mL). To the solution was then added potassium acetate (5.12 g, 52.2 mmol, Sigma-Aldrich Corporation) and the mixture was degassed by bubbling through with Argon for 5 minutes. Then, [1,1'-bis(diphenylphosphino)ferrocene]dichloropalladium(ii), complex with dichloromethane (1.420 g, 1.739 mmol, Strem Chemicals, Inc.) was added. The reaction was stirred at 100°C. After 18 h the reaction was cooled down and the solid filtered under vacuum and the washed with DCM. The mother liquor was then concentrated to give a semisolid residue. DCM was added, and the solid formed collected by vacuum filtration. The mother liquor concentrated again, and this step was repeated. The desired methyl 4-amino-3-(4,4,5,5-tetramethyl-1,3,2-dioxaborolan-2-yl)benzoate (2.6 g, 9.38 mmol, 54.0 % yield) was isolated as a grey solid. m/z (ESI): 196.1 (M+H)⁺ (boronic acid). ¹H NMR (400 MHz, CHLOROFORM-d) δ ppm 8.33 (d, J=2.1 Hz, 1 H), 7.90 (dd, J=8.6, 2.2 Hz, 1 H), 6.57 (d, J=8.5 Hz, 1 H), 5.20 (br s, 2 H), 3.87 (s, 3 H), 1.37 (s, 12 H).

Step 3. To a stirred solution of 4-cyano-2,5-dihydrofuran-3-yl trifluoromethanesulfonate (10 g, 41.1 mmol) in 1,4-dioxane (200 mL) and water (20.00 mL) was added methyl 4-amino-3-(4,4,5,5-tetramethyl-1,3,2-dioxaborolan-2-yl)benzoate (9.12 g, 32.9 mmol), K₂CO₃ (17.05 g, 123 mmol), and Pd(PPh₃)₄ (4.75 g, 4.11 mmol) under nitrogen purging. Then, the reaction mixture heated at 80 °C for 16 h. The reaction mixture was concentrated, then diluted with ethyl acetate (50 mL) and water (50 mL) and stirred at room temperature for 30 min. Then, the solid formed was filtered and washed with ethyl acetate (50 mL) and 2% MeOH in DCM (50 mL), then dried under vacuum to give methyl 4-amino-1,3-dihydrofuro[3,4-c]quinoline-8-carboxylate (6.6 g, 27.0 mmol, 65.7 % yield) as gray solid. m/z (ESI): 245.3 (M+H)⁺. ¹H NMR (400 MHz, TFA-d) δ ppm 8.59 - 8.67 (2H, m), 7.97 (1H, d, J=9.3 Hz), 5.94 (2H, t, J=3.5 Hz), 5.65 (2H, t, J=3.4 Hz), 4.24 (3H, s). Note: for some heterocycles Pd(dppf)Cl₂ was used in place of Pd(PPh₃)₄.

Step 4. To a stirred solution of methyl 4-amino-1,3-dihydrofuro[3,4-c]quinoline-8-carboxylate (30 g, 123 mmol) in water (300 mL):tetrahydrofuran (300 mL):methanol (300 mL) was added LiOH (11.77 g, 491 mmol) and the reaction mixture was heated at 75 °C for 3 h. The reaction mixture was concentrated and acidified with 1.5 N HCl up to pH 6.0. The solid obtained was filtered, washed with methanol (300 mL), and dried to give 4-amino-1,3-dihydrofuro[3,4-c]quinoline-8-carboxylic acid (28 g, 122 mmol, 99 % yield) as off-white solid. m/z (ESI): 231.2 (M+H)⁺. ¹H NMR (400 MHz, DMSO-d) δ ppm 12.83 (1H, s), 7.88 - 8.30 (2H, m), 7.59 (1H, d, J=8.8 Hz), 7.02 (2H, s), 5.40 (2H, t, J=3.5 Hz), 5.03 (2H, t, J=3.6 Hz).

Acids in Table 15 were prepared in a manner similar to that described for Intermediate **215.**

**Table 15**

| **Int. #** | **Chemical Structure** | **Name** | *m*/*z* (ESI): (M+H)⁺ |
|---|---|---|---|
| **216** | 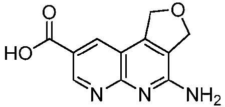 | 4-amino-1,3-dihydrofuro[3,4-c][1,8]naphthyridine-8-carboxylic acid | 232.1 |
| **217** | 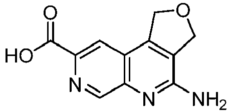 | 4-amino-1,3-dihydrofuro[3,4-c][1,7]naphthyridine-8-carboxylic acid | 232.0 |
| **218** | 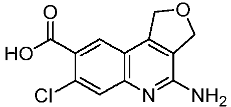 | 4-amino-7-chloro-1,3-dihydrofuro[3,4-c]quinoline-8-carboxylic acid | 264.9 |
| **219** | 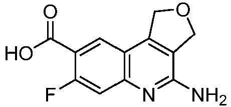 | 4-amino-7-fluoro-1,3-dihydrofuro[3,4-c]quinoline-8-carboxylic acid | 249.0 |
| **220** | 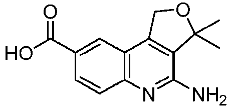 | 4-amino-3,3-dimethyl-1,3-dihydrofuro[3,4-c]quinoline-8-carboxylic acid | 259.1 |
| **221** | 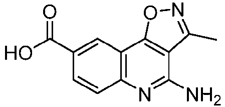 | 4-amino-3 -methylisoxazolo [4,5-c] quinoline-8-carboxylic acid | 244.0 |
| **222** | 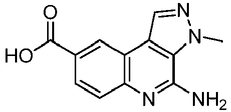 | 4-amino-3-methyl-3H-pyrazolo[3,4-c]quinoline-8-carboxylic acid | 243.1 |
| **223** | 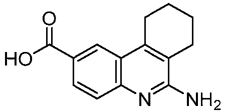 | 6-amino-7,8,9,10-tetrahydrophenanthridine-2-carboxylic acid | 243.2 |
| **224** | 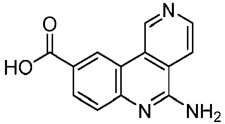 | 5-aminobenzo[c] [2,6]naphthyridine-9-carboxylic acid | 240.1 |
| **225** | 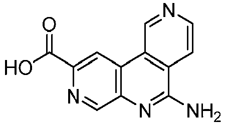 | 5-aminopyrido[4,3-c] [1,7]naphthyridine-9-carboxylic acid | 241.1 |
| **226** | 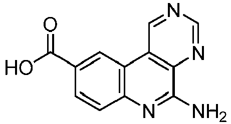 | 5-aminopyrimido[4,5-c]quinoline-9-carboxylic acid | 241.2 |
| **227** | 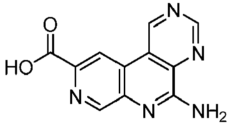 | 5-aminopyrimido[4,5-c] [1,7]naphthyridine-9-carboxylic acid | 241.1 |
| **228** | 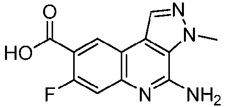 | 4-amino-7-fluoro-3-methyl-3H-pyrazolo[3,4-c]quinoline-8-carboxylic acid | 261.1 |
| **229** | 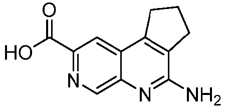 | 6-amino-8,9-dihydro-7H-cyclopenta[c][1,7]naphthyridine-2-carboxylic acid | 230.0 |
| **230** | 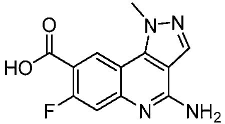 | 4-amino-7-fluoro-1-methyl-1H-pyrazolo[4,3-c]quinoline-8-carboxylic acid | 261.0 |
| **231** | 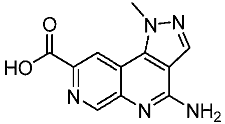 | 4-amino-1-methyl-1H-pyrazolo[4,3-c][1,7]naphthyridine-8-carboxylic acid | 244.0 |
| **232** | 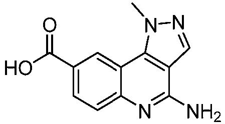 | 4-amino-1-methyl-1H-pyrazolo[4,3-c]quinoline-8-carboxylic acid | 243.0 |
| **233** | 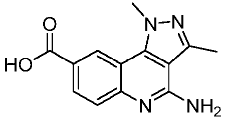 | 4-amino-1,3-dimethyl-1H-pyrazolo[4,3-c]quinoline-8-carboxylic acid | 257.0 |
| **234** | 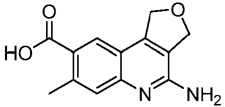 | 4-amino-7-methyl-1,3-dihydrofuro[3,4-c]quinoline-8-carboxylic acid | 245.2 |
| **235** | 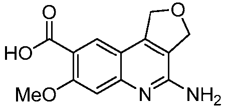 | 4-amino-7-methoxy-1,3-dihydrofuro[3,4-c]quinoline-8-carboxylic acid | 261.0 |
| **236** | 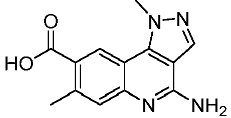 | 4-amino-1,7-dimethyl-1H-pyrazolo[4,3-c]quinoline-8-carboxylic acid | 257.0 |
| **237** | 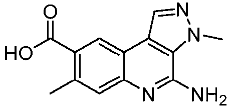 | 4-amino-3,7-dimethyl-3H-pyrazolo[3,4-c]quinoline-8-carboxylic acid | 257.1 |
| **238** | 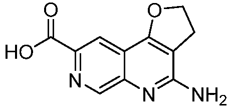 | 4-amino-2,3-dihydrofuro[3,2-c][1,7]naphthyridine-8-carboxylic acid | 232.1 |
| **239** | 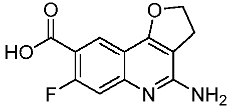 | 4-amino-7-fluoro-2,3-dihydrofuro[3,2-c]quinoline-8-carboxylic acid | 249.1 |
| **240** | 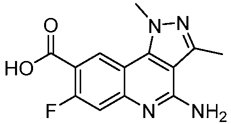 | 4-amino-7-fluoro-1,3-dimethyl-1H-pyrazolo[4,3-c]quinoline-8-carboxylic acid | 275.1 |
| **241** | 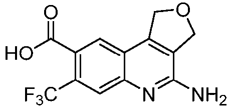 | 4-amino-7-(trifluoromethyl)-1,3-dihydrofuro[3,4-c]quinoline-8-carboxylic acid | 298.9 |
| **242** | 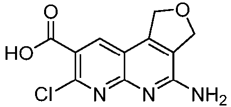 | 4-amino-7-chloro-1,3-dihydrofuro[3,4-c][1,8]naphthyridine-8-carboxylic acid | 265.9 |
| **243** | 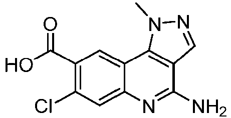 | 4-amino-7-chloro-1-methyl-1H-pyrazolo[4,3-c]quinoline-8-carboxylic acid | 277 |
| **244** | 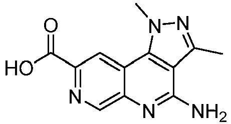 | 4-amino-1,3-dimethyl-1H-pyrazolo[4,3-c][1,7]naphthyridine-8-carboxylic acid | 258.1 |

### Intermediate 245: 6-amino-8,9-dihydro-7H-cyclopenta[c][1,8]naphthyridine-2-carboxylic acid.

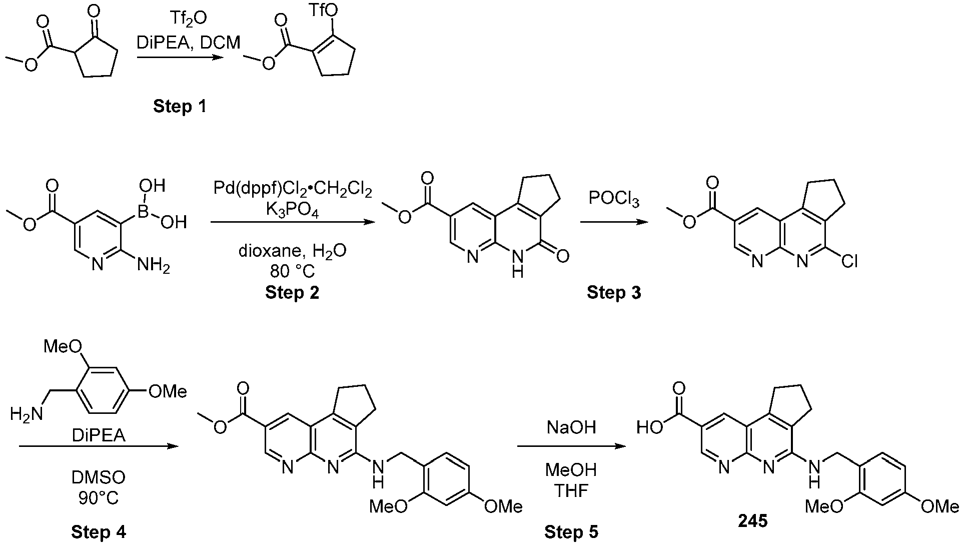

Step 1. A mixture of methyl 2-oxocyclopentanecarboxylate (1.0 g, 0.877 mL, 7.03 mmol, Matrix Scientific) and 1,1'-dimethyltriethylamine (1.000 g, 1.352 mL, 7.74 mmol, Sigma-Aldrich Corporation) in DCM (15 mL) was cooled to -78°C and trifluoromethanesulfonic acid anhydride (7.03 mL, 7.03 mmol, Sigma-Aldrich Corporation) was added. After complete addition, the mixture was stirred at -78°C for 5 min, then the dry ice-bath was removed and stirred at rt. After 15 min, the mixture was concentrated to afford methyl 2-(((trifluoromethyl)sulfonyl)oxy)cyclopent-1-ene-1-carboxylate with quant. yield as a light-yellow solid to be used as is. m/z (ESI): 275 (M+H)⁺.

Step 2. A mixture of methyl 2-(((trifluoromethyl)sulfonyl)oxy)cyclopent-1-ene-1-carboxylate (1.982 g, 7.23 mmol), (2-amino-5-(methoxycarbonyl)pyridin-3-yl)boronic acid (1.70 g, 8.67 mmol), potassium phosphate, tribasic (3.78 g, 21.69 mmol, Acros) and [1,1'-bis(diphenylphosphino)ferrocene]dichloropalladium (ii), complex with dichloromethane (0.177 g, 0.217 mmol, Strem Chemicals, Inc.) in 1,4-dioxane/water (10/0.60 mL) was heated at 80 °C for 1 h at which time it was brought to rt and diluted with EtOAc. A precipitate was formed which corresponded to the desired product. The precipitate was filtered and washed with EtOAc to yield methyl 6-oxo-6,7,8,9-tetrahydro-5H-cyclopenta[c][1,8]naphthyridine-2-carboxylate as a light gray solid with quant. yield. m/z (ESI): 245 (M+H)⁺. ¹H NMR (400 MHz, DMSO-d6) δ ppm 11.93 - 12.58 (m, 1 H), 8.96 (d, J=2.1 Hz, 1 H), 8.33 (d, J=2.1 Hz, 1 H), 3.89 (s, 3 H), 3.13 (br t, J=7.6 Hz, 2 H), 2.78 (br t, J=7.3 Hz, 2 H), 2.08 - 2.18 (m, 2 H).

Step 3. A mixture of methyl 6-oxo-6,7,8,9-tetrahydro-5H-cyclopenta[c][1,8]naphthyridine-2-carboxylate (1.76 g, 7.21 mmol) in POCl₃ (24.68 g, 15 mL, 161 mmol, Aldrich) was heated to reflux for 30 min. The reaction went to completion and was carefully added to cold sat. NaHCO₃ to basify the reaction. After stirring for 15 min, the mixture was extracted with EtOAc and the combined organics were concentrated to afford methyl 6-chloro-8,9-dihydro-7H-cyclopenta[c][1,8]naphthyridine-2-carboxylate as a yellow solid with quant. yield. m/z (ESI): 263 (M+H)⁺.

Step 4. To a suspension of methyl 6-chloro-8,9-dihydro-7H-cyclopenta[c][1,8]naphthyridine-2-carboxylate (1.89 g, 7.19 mmol) in DMSO (15 mL) was added DIPEA (2.79 g, 3.77 mL, 21.58 mmol, Aldrich) followed by the addition of (2,4-dimethoxyphenyl)methanamine (1.564 g, 1.405 mL, 9.35 mmol, Aldrich). The resulting mixture was heated at 90 °C overnight. Then, the reaction was cooled to rt, diluted with water, washed with sat. NH₄Cl and extracted with EtOAc. The combined organics were dried over Na₂SO₄, filtered and concentrated to afford methyl 6-((2,4-dimethoxybenzyl)amino)-8,9-dihydro-7H-cyclopenta[c] [1,8]naphthyridine-2-carboxylate (2.18 g, 5.54 mmol, 77 % yield) as a yellow solid to be used as is. m/z (ESI): 394 (M+H)⁺.

Step 5. To a solution of methyl 6-((2,4-dimethoxybenzyl)amino)-8,9-dihydro-7H-cyclopenta[c] [1,8]naphthyridine-2-carboxylate (2.18 g, 5.54 mmol) in THF/MeOH (10/10 mL) was added NaOH (10 mL, 10.00 mmol) and the resulting solution was heated at 70 °C for 2 h at which time it was brought to rt and acidified with 10 mL 1M HCl. A light yellow precipitate was formed filtered off and azeotropically dried with toluene to afford 6-((2,4-dimethoxybenzyl)amino)-8,9-dihydro-7H-cyclopenta[c][1,8]naphthyridine-2-carboxylic acid hydrochloride (1.44 g, 3.46 mmol, 62.5% yield) as a yellow solid. m/z (ESI): 380.2 (M+H)⁺.

Acids in Table 16 were prepared in a manner similar to that described for Intermediate 245.

**Table 16**

| **Int. #** | **Chemical Structure** | **Name** | *m*/*z* (ESI): (M+H)⁺ |
|---|---|---|---|
| **246** | 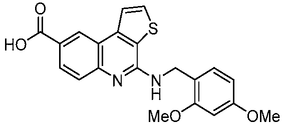 | 4-aminothieno [2,3 -c] quinoline-8-carboxylic acid | 395.0 |
| **247** | 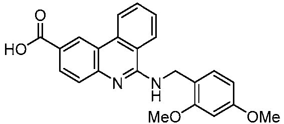 | 6-aminophenanthridine-2-carboxylic acid | 389.2 |
| **248** | 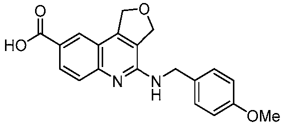 | 4-((4-methoxybenzyl)amino)-1,3-dihydrofuro[3,4-c]quinoline-8-carboxylic acid | 351.0 |
| **249** | 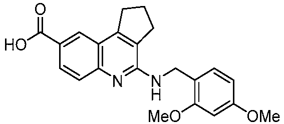 | 4-((2,4-dimethoxybenzyl)amino)-2,3-dihydro-1H-cyclopenta[c]quinoline-8-carboxylic acid | 379.2 |
| **250** | 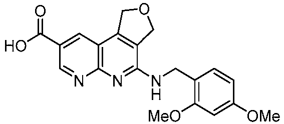 | 4-((2,4-dimethoxybenzyl)amino)-1,3-dihydrofuro[3,4-c][1,8]naphthyridine-8-carboxylic acid | 382.2 |
| **251** | 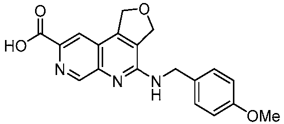 | 4-((4-methoxybenzyl)amino)-1,3-dihydrofuro[3,4-c] [1,7]naphthyridine-8-carboxylic acid | 352.2 |
| **252** | 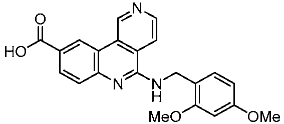 | 5-((2,4-dimethoxybenzyl)amino)benzo[c] [2,6]n aphthyridine-9-carboxylic acid | 390.2 |
| **253** | 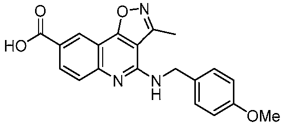 | 4-((4-methoxybenzyl)amino)-3-methylisoxazolo[4,5-c]quinoline-8-carboxylic acid | 364.1 |
| **254** | 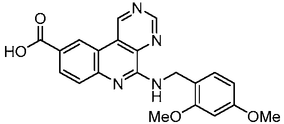 | 5-((2,4-dimethoxybenzyl)amino)pyrimido[4,5-c]quinoline-9-carboxylic acid | 391.2 |
| **255** | 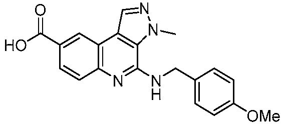 | 4-((4-methoxybenzyl)amino)-3-methyl-3H-pyrazolo[3,4-c]quinoline-8-carboxylic acid | 363.0 |
| **256** | 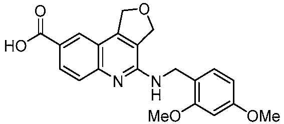 | 4-((2,4-dimethoxybenzyl)amino)-1,3-dihydrofuro[3,4-c]quinoline-8-carboxylic acid | 381.1 |
| **257** | 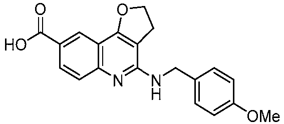 | 4-((4-methoxybenzyl)amino)-2,3-dihydrofuro[3,2-c]quinoline-8-carboxylic acid | 351.2 |
| **258** | 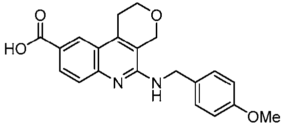 | 5-((4-methoxybenzyl)amino)-1,4-dihydro-2H-pyrano[3,4-c] quinoline-9-carboxylic acid | 365.1 |

### Intermediate 259: 4-amino-3-methyl-1,3-dihydrofuro[3,4-c]quinoline-8-carboxylic acid

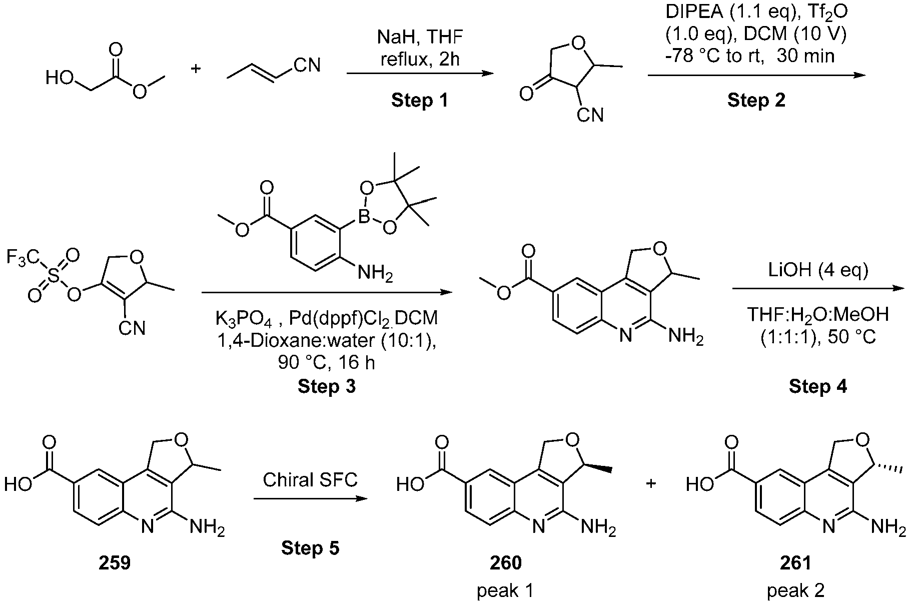

Note: Stereochemistry is arbitrarily assigned

Step 1. To a suspension of sodium hydride (11.10 g, 278 mmol 0.5 equiv., 60% in mineral oil) in anhydrous tetrahydrofuran (250 mL) was added methyl glycolate (42.4 mL, 555 mmol, 1.0 equiv) at room temperature under N₂ atmosphere. To the reaction mixture (E)-but-2-enenitrile (54.5 mL, 666 mmol, 1.2 equiv) was added slowly at 65 °C and stirred for 2h at same temperature. The reaction mixture was cooled and quenched with 2 N NaOH solution (250 mL) and extracted with diethyl ether (500 mL). The aqueous layer was acidified with conc. HCl to adjust the pH to ~1 and extracted with dichloromethane (2 x 500 mL). The combined organic layer was washed with brine (200 mL) and dried over sodium sulfate, filtered and concentrated under reduced pressure. The crude residue was purified by column chromatography over silica gel (230-400 mesh) using 10% ethyl acetate with hexanes as an eluent to 2-methyl-4-oxotetrahydrofuran-3-carbonitrile (22 g, 176 mmol, 32% yield) as a brown solid. *m*/*z* (ESI, Negative): 124.3 [M-1]. 1H NMR (400 MHz, Chloroform-d): δ ppm 4.40 - 4.27 (m, 2 H), 4.26 - 4.19 (m, 1 H), 3.24 - 2.99 (m, 1 H), 1.61 (dd, J=18.6, 6.2 Hz, 3 H).

Step 2. To a stirred solution of 2-methyl-4-oxotetrahydrofuran-3-carbonitrile (25.0 g, 200 mmol, 1.0 equiv) in dichloromethane (500 mL) was added DIPEA (69.8 mL, 400 mmol, 2.0 equiv) and triflic anhydride (47.1 mL, 280 mmol, 1.4 equiv) at -78 °C and stirred at same temperature for 15 min. The reaction mixture was quenched with slow addition of water (250 mL) and after attaining the room temperature, it was extracted with dichloromethane (2 x 500 mL). The combined organic layer was dried over sodium sulfate, filtered and concentrated under reduced pressure. The crude residue was stirred in diethyl ether and filtered. The mother liquor was concentrated under reduced pressure to give of 4-cyano-5-methyl-2,5-dihydrofuran-3-yl trifluoromethanesulfonate (35.0 g, crude) as a light brown adduct. The crude material was used for next step without further purification. *m*/*z:* 257.1 [Not ionized]

Step 3. To a stirred solution of of 4-cyano-5-methyl-2,5-dihydrofuran-3-yl trifluoromethanesulfonate (35 g, 136 mmol, 1.0 equiv) in 1,4-dioxane (1400 mL) and water (70.0 mL), was added methyl 4-amino-3-(4,4,5,5-tetramethyl-1,3,2-dioxaborolan-2-yl)benzoate (37.7 g, 136 mmol, 1.0 equiv) and potassium phosphate (87 g, 408 mmol, 3.0 equiv) under nitrogen atmosphere. The reaction mixture was degassed with nitrogen for 15 min and then PdCl₂(dppf)-DCM adduct (9.96 g, 13.61 mmol, 0.1 equiv) was added and the reaction mixture was heated at 90 °C for 16h. The reaction mass was concentrated under reduced pressure to get crude product. The crude residue was purified by column chromatography over silica gel (60-120 mesh) using 50% ethyl acetate with hexanes as an eluent to give methyl 4-amino-3-methyl-1,3-dihydrofuro[3,4-c]quinoline-8-carboxylate (25 g, 97 mmol, 71% yield) as a brown solid. *m*/*z:* 259.2 (M+H)^{+ 1}H NMR (400 MHz, DMSO-*d*₆): δ 8.11 (d, *J=* 2.0 Hz, 1H), 8.00 (dd,*J* = 8.8, 2.0 Hz, 1H), 7.58 (d, *J=* 8.8 Hz, 1H), 6.87 (s, 2H), 4.11 (q, *J =* 5.3 Hz, 1H), 3.87 (s, 2H), 3.17 (d, *J =* 5.3 Hz, 3H), 1.41 (d, *J =* 5.9 Hz, 3H).

Step 4. To a stirred solution of methyl 4-amino-3-methyl-1,3-dihydrofuro[3,4-c]quinoline-8-carboxylate (26.0 g, 101 mmol, 1.0 equiv) in tetrahydrofuran (130 mL), methanol (78 mL) and water (52 mL), was added lithium hydroxide (9.64 g, 403 mmol, 4.0 equiv) and stirred at 75°C for 4h. LCMS indicated completion of the reaction. The reaction mixture was concentrated under reduced pressure. The crude residue was dissolved in water (100 mL) and filtered to removed insoluble particles. The aqueous layer was acidified with con. HCl (pH 6 to 6.5). The precipitated solid was filtered, washed with water and dried under vacuum to get 4-amino-3-methyl-1,3-dihydrofuro[3,4-c]quinoline-8-carboxylic acid (17.5 g, 71.6 mmol, 71% yield) as an off-white solid. *m*/*z*: 245.1 (M+H)^{+ 1}H NMR(TFA, 400 MHz): δ (ppm) 8.68 (t, *J*=6.2 Hz, 2H), 8.01 (dd, *J*=9.1, 4.2 Hz, 1H), 6.15 (s, 1H), 5.94 (m, 2H), 1.86 (t, *J=*5.4 Hz, 3H)

Step 5. Chiral SFC separation: 44.5 g of racemic 4-amino-3-methyl-1,3-dihydrofuro[3,4-c]quinoline-8-carboxylic acid was separated by chiral SFC to get 14 g of each isomer. Stereochemistry is assigned arbitrarily.

### Separation Information:

| | Key | Value |
|---|---|---|
| 1 | Instrument | SFC 200 |
| 2 | Column | ChiralPak- IC (250x30mm, 5µ) |
| 3 | Mobile Phase | Liquid CO₂: 0.5% DEA in Methanol (40:60) |
| 4 | Flow rate | 100mL/ min |
| 5 | Pressure Drop | 130bar |
| 6 | BPR | 100 bar |
| 7 | UV Detector Wavelength | 210 nm |
| 8 | Dissolution | 14.0 g dissolved in 280mL of 2% of DEA in Methanol |
| 9 | Test Injections | 2.5,1.5,1.8 mL |
| 10 | Processing | NA |
| 11 | Injection Volume | 2.0 mL |
| 12 | Cycle time | 4.14 min |

Racemic acids in Table 17 were prepared in a manner similar to that described for Intermediate **259.**

**Table 17**

| **Acids** | **SFC Conditions** | *m*/*z* (M+H)⁺ |
|---|---|---|
| 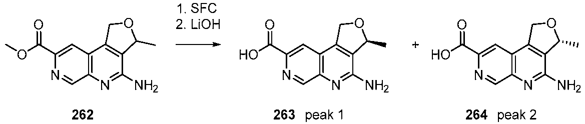 | Chiralpak IG-3, 50×4.6mm I.D., 3um CO₂: MeOH (0.05% isopropylamine, v/v); 95:5 →1:1; 3 min gradient | 246.0 |
| 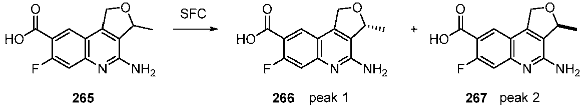 | 1^{st} peak, CHIRALPAK IG column (250 X 50mm, 10□m) with a mobile phase of 75% Liquid CO₂ and 25% MeOH with 0.3% NH₄OH using a flowrate of 200 mL/min | 263.1 |

### Intermediate 268: 4-amino-3-((benzyloxy)methyl)-1,3-dihydrofuro[3,4-c]quinoline-8-carboxylic acid

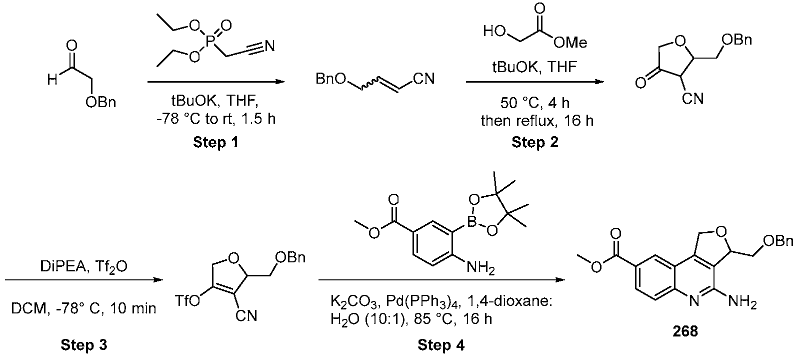

Step 1. To a stirred solution of diethyl (cyanomethyl)phosphonate (130 g, 732 mmol, 1.1 equiv) in tetrahydrofuran (2000 mL) was added potassium tert-butoxide (1 M solution in THF; 732 mL, 732 mmol, 1.1 equiv) at -78 °C and stirred for 30 min. To the reaction mixture, 2-(benzyloxy)acetaldehyde (100 g, 666 mmol, 1.0 equiv) was added at -78 °C and allowed to room temperature for 1 h. After completion, the reaction mixture was quenched with saturated NH₄Cl solution (1500 mL) and extracted with ethyl acetate (2 x 3000 mL). The combined organic layers were washed with brine solution (1000 mL) and dried over anhydrous Na₂SO₄, filtered and concentrated under vacuum. The crude residue was purified by column chromatography over silica gel (60-120 mesh) using 15% ethyl acetate with pet ether as eluent to give 4-(benzyloxy)but-2-enenitrile (100.6 g, 87% yield) as a colorless oil. *m*/*z:* 174.1 (M+H)⁺. ¹H NMR (Chloroform-d, 400 MHz): δ (ppm) proton NMR showed mixture of isomers. 7.47 - 7.32 (m, 5H), 6.80-6.62 (m, 1H), 5.77-5.72 (m, 1H), 4.59 (d, *J =* 2.8 Hz, 2H), 4.18-4.16 (m, 2H).

Step 2. To a stirred solution of potassium tert-butoxide (1 M solution in THF; 289.0 mL, 289 mmol, 1.0 equiv) in tetrahydrofuran (260 mL) was added methyl 2-hydroxyacetate (22.03 mL, 289 mmol, 1.0 equiv) at RT and heated to 50°C under nitrogen atmosphere. To this, 4-(benzyloxy)but-2-enenitrile (50.0 g, 289 mmol, 1.0 equiv) was added and stirred at same temperature for 4 h. The reaction temperature was increased to 70°C and stirred for 16 h. After completion, reaction mixture was cooled to 0 °C and quenched with ice water (500 mL). The resultant solution was washed with diethyl ether (2 x 200 mL) and then acidified with conc. HCl (until pH of ~1-2) and then extracted with DCM (2 x 500 mL). The combined organic layers were dried over anhydrous Na₂SO₄, filtered and concentrated under vacuum. The crude residue was purified by column chromatography over silica gel (60-120 mesh) using 26% ethyl acetate with pet ether as an eluent to give 2-((benzyloxy)methyl)-4-oxotetrahydrofuran-3-carbonitrile (9.2 g, 14% yield) as a colorless oil. LCMS (ESI, Positive) *m*/*z:* 232.0 (M+H)⁺. ¹H NMR (400 MHz, Chloroform-d) δ 7.41-7.28 (m, 5H), 4.83 - 4.60 (m, 2H), 4.52 (dd, *J =* 11.8, 6.6 Hz, 1H), 4.33 (dd, *J =* 17.0, 9.0 Hz, 1H), 4.11 - 3.89 (m, 2H), 3.82 - 3.68 (m, 2H).

Step 3. To a stirred solution of 2-((benzyloxy)methyl)-4-oxotetrahydrofuran-3-carbonitrile (5.8 g, 25.08 mmol, 1.0 equiv) in dichloromethane (116 mL) were added triflic anhydride (6.75 mL, 40.1 mmol, 1.6 equiv) and DIPEA (8.76 mL, 50.2 mmol, 2.0 equiv) at -78 °C under N₂ atmosphere and stirred for 10 min. The reaction mixture was quenched with water (50 mL) and extracted with dichloromethane (2 x 200 mL). The combined organic layers were washed with brine solution (100 mL) and dried over anhydrous Na₂SO₄, filtered and concentrated under reduced pressure. The crude residue was washed with diethyl ether (200 mL) and filtered. The organic layer was concentrated under reduced pressure to give 5-((benzyloxy)methyl)-4-cyano-2,5-dihydrofuran-3-yl trifluoromethane sulfonate (7.65 g) as a light brown liquid, which was taken as such for next step.

Step 4. To a stirred solution of 5-((benzyloxy)methyl)-4-cyano-2,5-dihydrofuran-3-yl trifluoromethane sulfonate (7.65 g, 20.93 mmol, 1.0 equiv) in 1,4-dioxane (232 mL) and water (11.60 mL) were added methyl 4-amino-3-(4,4,5,5-tetramethyl-1,3,2-dioxaborolan-2-yl)benzoate (5.8 g, 20.93 mmol, 1.0 equiv), potassium carbonate (8.68 g, 62.8 mmol, 3.0 equiv) at room temperature. The reaction mixture was purged with N₂ gas for 15 min and then added Pd(PPh₃)₄ (1.209 g, 1.046 mmol, 0.05 equiv). Reaction mixture was heated at 80 °C for 16 h. After completion, the reaction mixture was concentrated under reduced pressure and the crude residue was purified by column chromatography over silica gel (60-120 mesh) using 80% ethyl acetate with pet ether as eluent to give 4-amino-3-((benzyloxy)methyl)-1,3-dihydrofuro[3,4-c]quinoline-8-carboxylate (4.4 g, 58% yield) as an off white solid. *m*/*z:* 365.2 (M+H)⁺. ¹H NMR (400 MHz, *DMSO-d*₆) δ 8.12 (d, *J=* 2.0 Hz, 1H), 8.01 (dd, *J =* 8.9, 2.0 Hz, 1H), 7.59 (d, *J=* 8.8 Hz, 1H), 7.34 - 7.20 (m, 5H), 6.91 (br s, 2H), 5.49 (dq, *J =* 5.6, 3.6, 2.7 Hz, 1H), 5.44 - 5.32 (m, 2H), 4.56 - 4.44 (m, 2H), 3.90 - 3.73 (m, 5H). Ester 268 was treated with LiOH in THF and the lithium salt of 268 was used crude in amide coupling reactions.

### Intermeidate 269: lithium 4-amino-1-methyl-7-(trifluoromethyl)-1H-pyrazolo[4,3-c]quinoline-8-carboxylate hydroxide

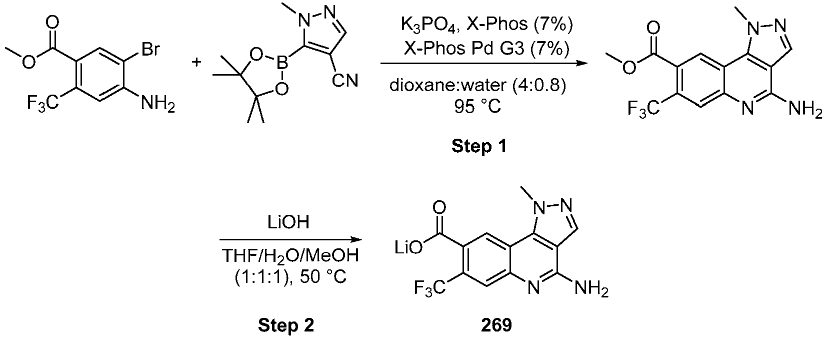

Step 1. K₃PO₄·H₂O (1.08 g, 4.70 mmol, Sigma-Aldrich Corporation), X-Phos (0.08 g, 0.16 mmol, Sigma-Aldrich Corporation), (2-dicyclohexylphosphino-2',4',6'-triisopropyl-1,1'-biphenyl)[2-(2'-amino-1,1'-biphenyl)]palladium (ii) methanesulfonate (0.14 mg, 0.16 mmol, Sigma-Aldrich Corporation), 1-methyl-5-(4,4,5,5-tetramethyl-1,3,2-dioxaborolan-2-yl)-1h-pyrazole-4-carbonitrile (1.10 g, 4.70 mmol, Enamine) and methyl 4-amino-5-bromo-2-(trifluoromethyl)benzoate (0.700 g, 2.349 mmol, Combi Blocks) were suspended in a degassed mixture of water (1.0 mL) and 1,4-dioxane (5.0 mL) and stirred at 60°C overnight and then at 90 °C for 18h. Volatiles were removed *in vacuo* and the crude product was purified via silica column chromatography (0 to 5% MeOH/DCM + 0.5% NH₃/MeOH) to yield methyl 4-amino-1-methyl-7-(trifluoromethyl)-1H-pyrazolo[4,3-c]quinoline-8-carboxylate (0.63 g, 1.94 mmol, 83 % yield) as an slight brownish solid. m/z (ESI): 324.8 (M+H)⁺. ¹H NMR (400 MHz, DMSO-d6) δ ppm 8.71 - 8.76 (m, 1 H), 8.33 - 8.37 (m, 1 H), 7.87 - 7.92 (m, 1 H), 7.54 - 7.61 (m, 2 H), 4.41 - 4.46 (m, 3 H), 3.92 (s, 3 H). ¹⁹F NMR (376 MHz, DMSO-d6) δ ppm -58.06.

Step 2. Methyl 4-amino-1-methyl-7-(trifluoromethyl)-1H-pyrazolo[4,3-c]quinoline-8-carboxylate (0.62 g, 1.90 mmol) and lithium hydroxide (0.91 g, 3.79 mmol, Sigma-Aldrich Corporation) were suspended in methanol (3.0 mL), H₂O (3.0 mL) and THF (3.0 mL) and stirrred at 50 °C for 2 hours. Volatiles of the crude mixture were removed in vacuo and the light brownish solid coevaporated with DCM twice, followed by coevaporation with toluene to give lithium 4-amino-1-methyl-7-(trifluoromethyl)-1H-pyrazolo[4,3-c]quinoline-8-carboxylate hydroxide (585 mg, 1.720 mmol, 91 % yield) that was used in subsequent steps without further purification. m/z (ESI): 310.9 (M+H)⁺. ¹H NMR (400 MHz, DMSO-d6) δ ppm 8.33 (s, 1 H), 8.27 (s, 1 H), 7.68 (s, 1 H), 7.03 (br s, 2 H), 4.38 (s, 3 H). ¹⁹F NMR (376 MHz, DMSO-d6) δ ppm -57.47.

### Intermeidate 270: lithium 4-amino-7-chloro-3-methyl-3H-pyrazolo[3,4-c]quinoline-8-carboxylate hydroxide

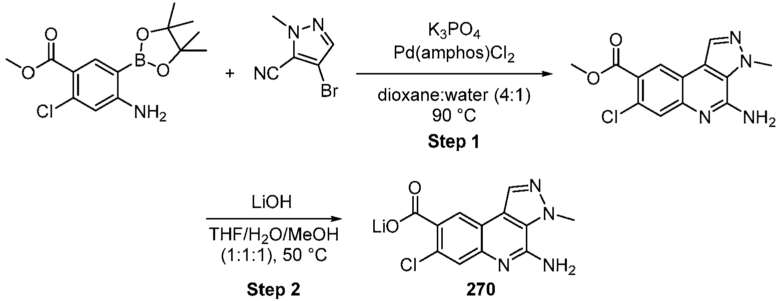

Step 1. 1H-pyrazole-5-carbonitrile, 4-bromo-1-methyl- (273 mg, 1.47 mmol), methyl 4-amino-2-chloro-5-(4,4,5,5-tetramethyl-1,3,2-dioxaborolan-2-yl)benzoate (457 mg, 1.47 mmol), phosphoric acid, tripotassium salt, monohydrate (1.35 g, 5.87 mmol, Sigma-Aldrich Corporation) and Pd(amphos)Cl₂ (72.7 mg, 0.10 mmol) were suspended in degassed water (1.0 mL) and 1,4-dioxane (4.00 mL) and stirred at 90 °C over night, whereas a yellow solid formed. Water (10 mL) was added after the mixture was cooled to rt and the precipitate filtered and washed with DCM, MeOH and acetone. 92 mg of a solid were dried and the organic wash was absorbed onto silica gel and purfied via column chromatography using 0 to 20 % MeOH + 0.5% NH₃ in MeOH/DCM) to yield methyl 4-amino-7-chloro-3-methyl-3H-pyrazolo[3,4-c]quinoline-8-carboxylate (164 mg, 0.564 mmol, 38.5% yield) as a orange solid. m/z (ESI): 291.000 (M+H)⁺. 1H NMR (400 MHz, DMSO-d6) δ ppm 8.52 - 8.63 (m, 2 H), 7.60 (s, 1 H), 7.25 (s, 2 H), 4.35 (s, 3 H), 3.89 (s, 3 H).

Step 2. Methyl 4-amino-7-chloro-3-methyl-3H-pyrazolo[3,4-c]quinoline-8-carboxylate (164 mg, 0.56 mmol) was suspended in water (0.5 mL), methanol (0.5 mL) and tetrahydrofuran (0.5 mL) and then lithium hydroxide hydrate (47.3 mg, 1.13 mmol, Sigma-Aldrich Corporation) was added and the reaction was stirred at 50 °C for 90 minutes. Volatiles were removed in vacuo to yield lithium 4-amino-7-chloro-3-methyl-3H-pyrazolo[3,4-c]quinoline-8-carboxylate hydroxide (170 mg, 0.56 mmol, 98% yield) as a yellow solid. *m*/*z* (ESI): 277.0 (M+H)⁺.

### Intermediate 271: lithium 4-amino-7-fluoro-1-(2,2,2-trifluoroethyl)-1H-pyrazolo[4,3-c]quinoline-8-carboxylate

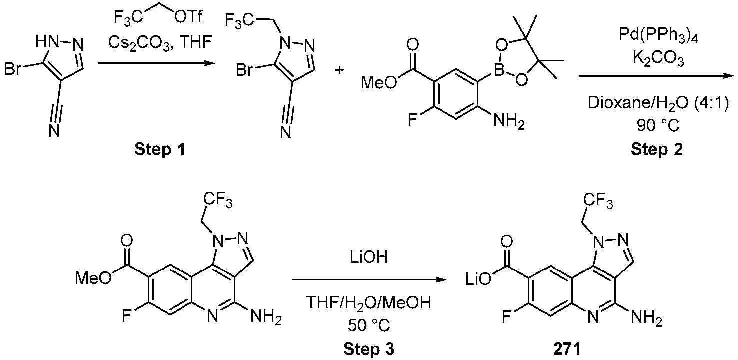

Step 1. To a 100-mL round-bottomed flask was added 5-bromo-1H-pyrazole-4-carbonitrile (1 g, 5.81 mmol, Enamine), cesium carbonate (3.79 g, 11.63 mmol, Sigma-Aldrich Corporation) and 2,2,2-trifluoroethyl triflate (1.687 g, 1.054 mL, 7.27 mmol, Combi-Blocks Inc.) in 1,4-dioxane (17.10 mL). The reaction mixture was stirred at 35 °C for 20 h. Upon completion as determined by LC-MS, the reaction was filtered and concentrated *in-vacuo* to afford the crude product. This was used as it is for the next step without further purification. *m*/*z* (ESI): 253.9 (M+H)⁺

Step 2. To a 25-mL pressure vial was added methyl 4-amino-2-fluoro-5-(4,4,5,5-tetramethyl-1,3,2-dioxaborolan-2-yl)benzoate (534 mg, 1.810 mmol), 5-bromo-1-(2,2,2-trifluoroethyl)-1H-pyrazole-4-carbonitrile (418 mg, 1.646 mmol), anhydrous potassium carbonate (1137 mg, 8.23 mmol, Acros Organics), and tetrakis(triphenylphosphine)palladium (190 mg, 0.165 mmol, Strem) in 1,4-dioxane (4388 µL) and water (1097 µL). The solution was degassed with N₂ for 10 mins and heated at 90 °C for 18 h. Upon completion as determined by LC-MS, the reaction mixture was cooled to room temperature and 5 ml of water was added. The product was filtered, and the precipitate was washed with 5 ml water twice and 5 ml of DCM thrice. The crude product was isolated as a solid and used as it is for the next step without further purification. *m*/*z* (ESI): 343.0 (M+H)⁺

Step 3. To a 20 ml pressure vial was added methyl 4-amino-7-fluoro-1-(2,2,2-trifluoroethyl)-1H-pyrazolo[4,3-c]quinoline-8-carboxylate (544 mg, 1.589 mmol) and lithium hydroxide, monohydrate (133 mg, 3.18 mmol, Sigma-Aldrich Corporation) in tetrahydrofuran (1766 µL), methanol (1766 µL) and water (1766 µL) was stirred at 50 °C for 12 h. Upon completion via LCMS, the reaction mixture was cooled to room temperature and evaporated to dryness and used as it is for the next step. *m*/*z* (ESI): 329.1 (M+H)⁺

### Intermediate 272: 4-amino-3-(fluoromethyl)-1,3-dihydrofuro[3,4-c]quinoline-8-carboxylic acid hydrochloride

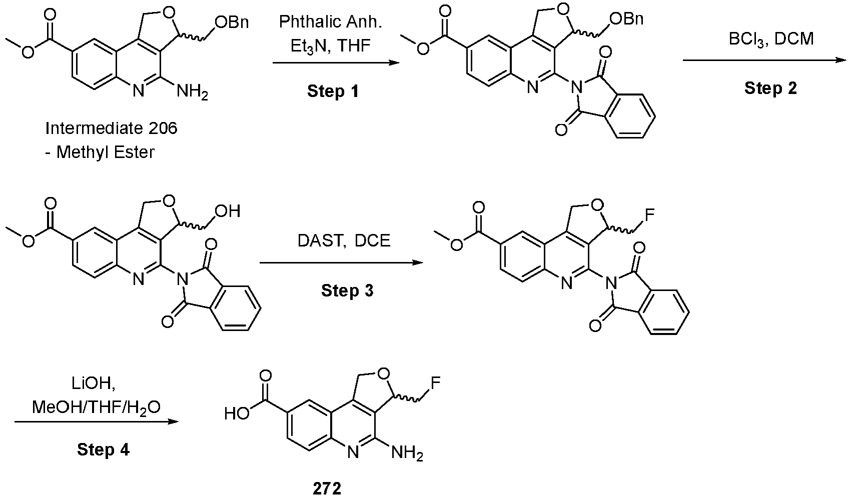

Step 1. Methyl 4-amino-3-((benzyloxy)methyl)-1,3-dihydrofuro[3,4-c]quinoline-8-carboxylate (3.40 g, 9.33 mmol, 1.0 equiv, Intemediate 206-Methyl Ester) was dissolved in tetrahydrofuran (46.7 mL) and triethylamine (4.16 mL, 29.9 mmol, 3.2 equiv, Aldrich) and phthalic anhydride (2.76 g, 18.7 mmol, 2.0 equiv, Aldrich) were added. The reaction mixture was heated at reflux for four days. The reaction was then concentrated to dryness and then taken up in water (100 mL) and DCM (150 mL). The layers were separated and the aqueous layer was extracted with (1 x 200 mL) of DCM. The combined organic layers were dried over MgSO₄, filtered and the crude product was purified by medium pressure chromatography (silica, 0 to 100% EtOAc:Heptanes) to give methyl 3-((benzyloxy)methyl)-4-(1,3-dioxoisoindolin-2-yl)-1,3-dihydrofuro[3,4-c]quinoline-8-carboxylate (3.22 g, 6.51 mmol, 69.8% yield). *m*/*z* (ESI): 495.1 (M+H)⁺.

Step 2. Methyl 3-((benzyloxy)methyl)-4-(1,3-dioxoisoindolin-2-yl)-1,3-dihydrofuro[3,4-c]quinoline-8-carboxylate (4.22 g, 8.53 mmol, 1.0 equiv) was dissolved in dichloromethane (114 mL) and cooled to -78°C, then boron trichloride (1.0 M in DCM) (21.3 mL, 21.3 mmol, 2.5 equiv, Aldrich) was added and the resulting mixture was stirred in a dry ice bath for 1.5 hrs to completion. The slurry was recooled to -78°C and methanol (3.5 mL) was slowly added to quench the reaction mixture. The slurry was removed from the dry ice bath and allowed to slowly warm. The mixture was diluted with water (150 mL) and extracted with EtOAc (2 x 250 mL). The combined organic layers were combined and washed with brine (1 x 100 mL) and dried over MgSO₄. The filtrate was concentrated and then triturated with EtOAc/Hexanes to give the desired methyl 4-(1,3-dioxoisoindolin-2-yl)-3-(hydroxymethyl)-1,3-dihydrofuro[3,4-c]quinoline-8-carboxylate (2.83 g, 7.00 mmol, 82% yield). *m*/*z* (ESI): 405.1 (M+H)⁺.

Step 3. Methyl 4-(1,3-dioxoisoindolin-2-yl)-3-(hydroxymethyl)-1,3-dihydrofuro[3,4-c]quinoline-8-carboxylate (998 mg, 2.47 mmol, 1.0 equiv) was dissolved in DCE (24.7 mL) and DAST (1.31 mL, 9.87 mmol, 4.0 equiv, Aldrich) was added slowly. The resulting mixture was stirred for 1.5 hours to completion. The reaction was quenched by slowly addition of the reaction mixture to 40 mL of satd. NaHCO₃ solution. This mixture was extracted with EtOAc (2 x 100 mL). The combined organic layers were washed with brine (1 x 45 ml) and dried over MgSO₄. The crude product was triturated with EtOAc and the precipitate was filtered and washed to give methyl 4-(1,3-dioxoisoindolin-2-yl)-3-(fluoromethyl)-1,3-dihydrofuro[3,4-c]quinoline-8-carboxylate (690 mg, 1.70 mmol, 68.8% yield). *m*/*z* (ESI): 406.9 (M+H)⁺.

Step 4. Lithium hydroxide, monohydrate (273 mg, 6.50 mmol, 4.0 equiv, Sigma-Aldrich Corporation) was added to a suspension of methyl 4-(1,3-dioxoisoindolin-2-yl)-3-(fluoromethyl)-1,3-dihydrofuro[3,4-c]quinoline-8-carboxylate (660 mg, 1.62 mmol, 1.0 equiv) in MeOH (8.5 mL), THF (8.5 mL) and water (8.5 mL). The mixture was heated to 70 °C for 19 hours then cooled to room temperature. The organic solvent was removed *in vacuo* and the resulting aqueous solution was taken to pH 6 with 5N HCl solution. The resulting suspension was filtered and air dried to give 4-amino-3-(fluoromethyl)-1,3-dihydrofuro[3,4-c]quinoline-8-carboxylic acid hydrochloride (490 mg, 1.64 mmol, 101% yield). *m*/*z* (ESI): 263.1 (M+H)⁺.

### Examples

### Example 300: 4-amino-N-(cyclopropylmethyl)-N-[[5-(trifluoromethyl)-2-pyridyl]methyl]-1,3-dihydrofuro[3,4-c]quinoline-8-carboxamide

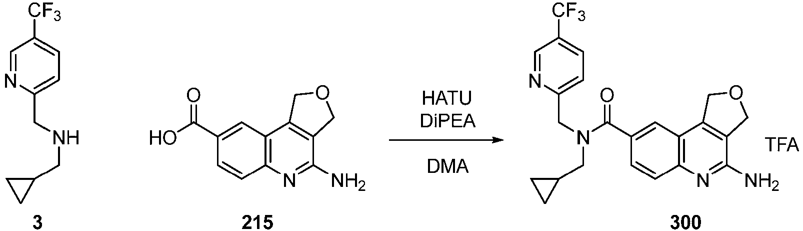

A mixture of 1-cyclopropyl-N-((5-(trifluoromethyl)pyridin-2-yl)methyl)methanamine (**2**, 0.050 g, 0.217 mmol), 4-amino-1,3-dihydrofuro[3,4-c]quinoline-8-carboxylic acid (**165**, 0.057 g, 0.250 mmol), N, N-dimethylacetamide (2 mL), HATU (0.099 g, 0.261 mmol, Combi-Blocks) and diisopropylethylamine (0.112 g, 0.151 mL, 0.869 mmol, Aldrich) was stirred at rt overnight. The mixture was filtered and the crude material was purified by reverse phase prep HPLC (10-70% MeCN in water with 0.1% TFA) to give 4-amino-N-(cyclopropylmethyl)-N-[[5-(trifluoromethyl)-2-pyridyl]methyl]-1,3-dihydrofuro[3,4-c]quinoline-8-carboxamide 2,2,2-trifluoroacetate (105 mg, 0.189 mmol, 87% yield) as a white solid. m/z (ESI): 443 (M+H)⁺. ¹H NMR (400 MHz, METHANOL-d4) δ ppm 8.88 (br s, 1 H), 8.04 - 8.16 (m, 1 H), 7.89 - 7.99 (m, 2 H), 7.46 - 7.88 (m, 2 H), 5.34 - 5.60 (m, 2 H), 4.86 - 5.25 (m, 4 H), 3.33 - 3.60 (m, 2 H), 0.93 - 1.18 (m, 1 H), 0.37 - 0.57 (m, 2 H), 0.00 - 0.31 (m, 2 H). ¹⁹F NMR (377 MHz, METHANOL-d4) δ ppm -63.87 (s, 3 F), -77.15 (s, 3 F).

Compounds in Table 18 were prepared in a manner similar to that described above for example 300 using the indicated amide coupling reagent in the table.

**Table 18**

| **Ex.** | **Structure** | **Name** | **Coupling Reagent** | **m/z (ESI): (M+H)⁺** |
|---|---|---|---|---|
| **301** | 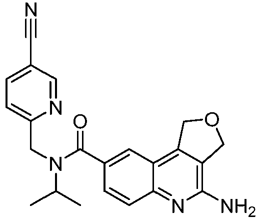 | 4-amino-N-((5-cyano-2-pyridinyl)methyl)-N-(2-propanyl)-1,3-dihydrofuro[3,4-c]quinoline-8-carboxamide | CMPI | 388.2 |
| **302** | 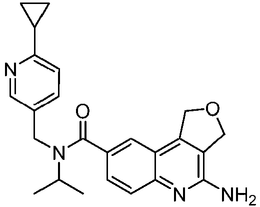 | 4-amino-N-((6-cyclopropyl-3-pyridinyl)methyl)-N-(2-propanyl)-1,3-dihydrofuro[3,4-c]quinoline-8-carboxamide | CMPI | 403.2 |
| **303** | 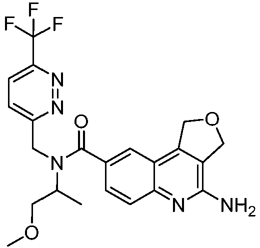 | 4-amino-N-((2R)-1-methoxy-2-propanyl)-N-((6-(trifluoromethyl)-3-pyridazinyl)methyl)-1,3-dihydrofuro[3,4-c]quinoline-8-carboxamide and 4-amino-N-((2S)-1-methoxy-2-propanyl)-N-((6-(trifluoromethyl)-3-pyridazinyl)methyl)-1,3-dihydrofuro[3,4-c]quinoline-8-carboxamide | CMPI | 462.2 |
| **304** | 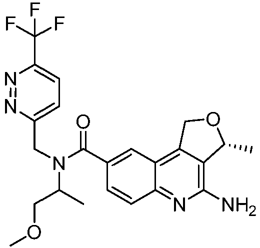 | (3R)-4-amino-N-((2R)-1-methoxy-2-propanyl)-3-methyl-N-((6-(trifluoromethyl)-3-pyridazinyl)methyl)-1,3-dihydrofuro[3,4-c]quinoline-8-carboxamide and (3R)-4-amino-N-((2S)-1-methoxy-2-propanyl)-3-methyl-N-((6-(trifluoromethyl)-3-pyridazinyl)methyl)-1,3-dihydrofuro[3,4-c]quinoline-8-carboxamide | CMPI | 476.2 |
| **305** | 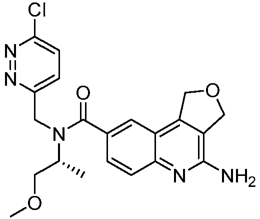 | 4-amino-N-((6-chloro-3-pyridazinyl)methyl)-N-((2R)-1-methoxy-2-propanyl)-1,3-dihydrofuro[3,4-c]quinoline-8-carboxamide | CMPI | 428.2 |
| **306** | 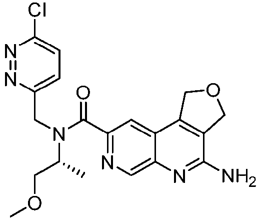 | 4-amino-N-((6-chloro-3-pyridazinyl)methyl)-N-((2R)-1-methoxy-2-propanyl)-1,3-dihydrofuro[3,4-c][1,7]naphthyridine-8-carboxamide | CMPI | 429.1 |
| **307** | 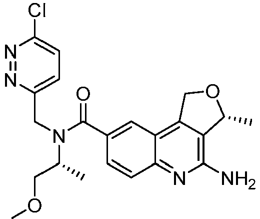 | (3R)-4-amino-N-((6-chloro-3-pyridazinyl)methyl)-N-((2R)-1-methoxy-2-propanyl)-3-methyl-1,3-dihydrofuro[3,4-c]quinoline-8-carboxamide | CMPI | 442.2 |
| **308** | 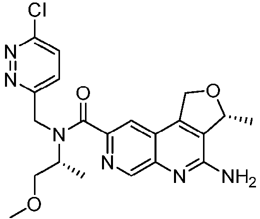 | (3R)-4-amino-N-((6-chloro-3-pyridazinyl)methyl)-N-((2R)-1-methoxy-2-propanyl)-3-methyl-1,3-dihydrofuro[3,4-c][1,7]naphthyridine-8-carboxamide | CMPI | 443.1 |
| **309** | 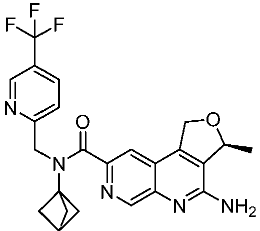 | (3S)-4-amino-N-(bicyclo[1.1.1]pentan-1-yl)-3-methyl-N-((5-(trifluoromethyl)-2-pyridinyl)methyl)-1,3-dihydrofuro[3,4-c][1,7]naphthyridine-8-carboxamide | CMPI | 470.2 |
| **310** | 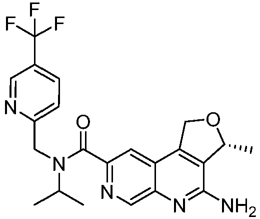 | (3R)-4-amino-3-methyl-N-(2-propanyl)-N-((5-(trifluoromethyl)-2-pyridinyl)methyl)-1,3-dihydrofuro[3,4-c][1,7]naphthyridine-8-carboxamide | CMPI | 446.2 |
| **311** | 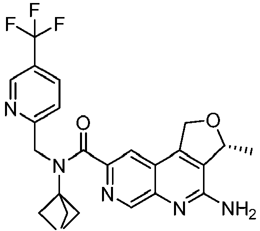 | (3R)-4-amino-N-(bicyclo[1.1.1]pentan-1-yl)-3-methyl-N-((5-(trifluoromethyl)-2-pyridinyl)methyl)-1,3-dihydrofuro[3,4-c][1,7]naphthyridine-8-carboxamide | CMPI | 470.2 |
| **312** | 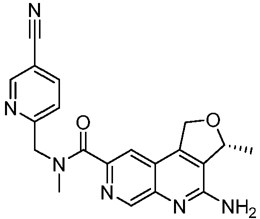 | (3R)-4-amino-N-((5-cyano-2-pyridinyl)methyl)-N,3-dimethyl-1,3-dihydrofuro[3,4-c][1,7]naphthyridine-8-carboxamide | CMPI | 375.1 |
| **313** | 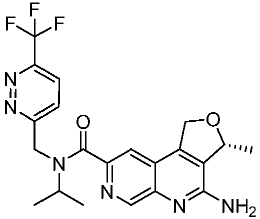 | (3R)-4-amino-3-methyl-N-(2-propanyl)-N-((6-(trifluoromethyl)-3-pyridazinyl)methyl)-1,3-dihydrofuro[3,4-c][1,7]naphthyridine-8-carboxamide | CMPI | 447.2 |
| **314** | 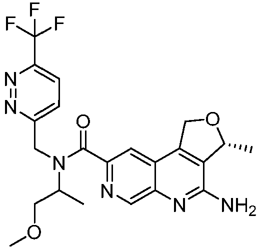 | (3R)-4-amino-N-((2R)-1-methoxy-2-propanyl)-3-methyl-N-((6-(trifluoromethyl)-3-pyridazinyl)methyl)-1,3-dihydrofuro[3,4-c][1,7]naphthyridine-8-carboxamide and (3R)-4-amino-N-((2S)-1-methoxy-2-propanyl)-3-methyl-N-((6-(trifluoromethyl)-3-pyridazinyl)methyl)-1,3-dihydrofuro[3,4-c][1,7]naphthyridine-8-carboxamide | CMPI | 477.2 |
| **315** | 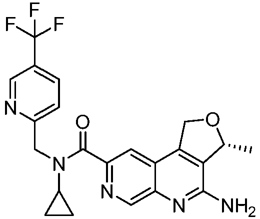 | (3R)-4-amino-N-cyclopropyl-3-methyl-N-((5-(trifluoromethyl)-2-pyridinyl)methyl)-1,3-dihydrofuro[3,4-c][1,7]naphthyridine-8-carboxamide | CMPI | 444.2 |
| **316** | 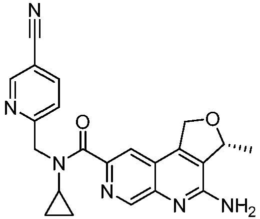 | (3R)-4-amino-N-((5-cyano-2-pyridinyl)methyl)-N-cyclopropyl-3-methyl-1,3-dihydrofuro [3,4-c][1,7]naphthyridine-8-carboxamide | CMPI | 401.2 |
| **317** | 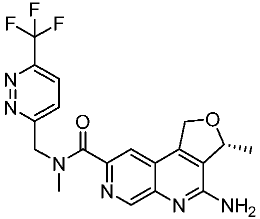 | (3R)-4-amino-N,3-dimethyl-N-((6-(trifluoromethyl)-3-pyridazinyl)methyl)-1,3-dihydrofuro[3,4-c][1,7]naphthyridine-8-carboxamide | CMPI | 419.1 |
| **318** | 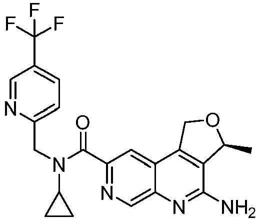 | (3S)-4-amino-N-cyclopropyl-3-methyl-N-((5-(trifluoromethyl)-2-pyridinyl)methyl)-1,3-dihydrofuro[3,4-c][1,7]naphthyridine-8-carboxamide | CMPI | 444.2 |
| **319** | 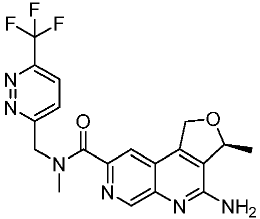 | (3S)-4-amino-N,3-dimethyl-N-((6-(trifluoromethyl)-3-pyridazinyl)methyl)-1,3-dihydrofuro[3,4-c][1,7]naphthyridine-8-carboxamide | CMPI | 419.1 |
| **320** | 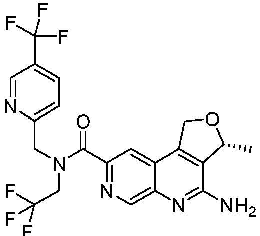 | (3R)-4-amino-3-methyl-N-(2,2,2-trifluoroethyl)-N-((5-(trifluoromethyl)-2-pyridinyl)methyl)-1,3-dihydrofuro[3,4-c][1,7]naphthyridine-8-carboxamide | CMPI | 486.1 |
| **321** | 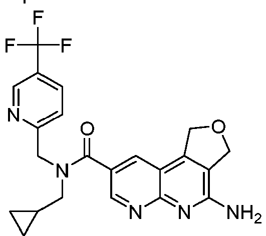 | 4-amino-N-(cyclopropylmethyl)-N-((5-(trifluoromethyl)-2-pyridinyl)methyl)-1,3-dihydrofuro[3,4-c][1,8]naphthyridine-8-carboxamide | DMB | 444.2 |
| **322** | 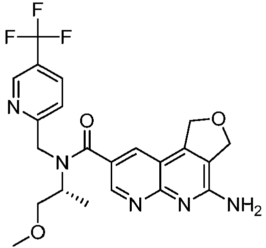 | 4-amino-N-((2R)-1-methoxy-2-propanyl)-N-((5-(trifluoromethyl)-2-pyridinyl)methyl)-1,3-dihydrofuro[3,4-c][1,8]naphthyridine-8-carboxamide | DMB | 462.2 |
| **323** | 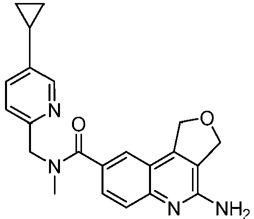 | 4-amino-N-((5-cyclopropyl-2-pyridinyl)methyl)-N-methyl-1,3-dihydrofuro[3,4-c]quinoline-8-carboxamide | EDCI | 375.2 |
| **324** | 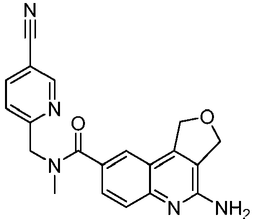 | 4-amino-N-((5-cyano-2-pyridinyl)methyl)-N-methyl-1,3-dihydrofuro[3,4-c]quinoline-8-carboxamide | EDCI | 360.2 |
| **325** | 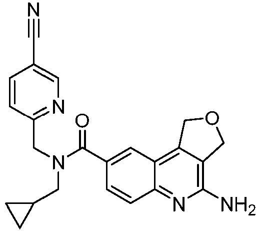 | 4-amino-N-((5-cyano-2-pyridinyl)methyl)-N-(cyclopropylmethyl)-1,3-dihydrofuro[3,4-c]quinoline-8-carboxamide | HATU | 400 |
| **326** | 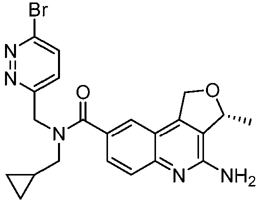 | (3R)-4-amino-N-((6-bromo-3-pyridazinyl)methyl)-N-(cyclopropylmethyl)-3-methyl-1,3-dihydrofuro[3,4-c]quinoline-8-carboxamide | HATU | 468 and 470 |
| **327** | 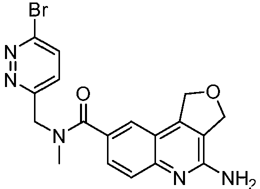 | 4-amino-N-((6-bromo-3-pyridazinyl)methyl)-N-methyl-1,3-dihydrofuro[3,4-c]quinoline-8-carboxamide | HATU | 414 and 416 |
| **328** | 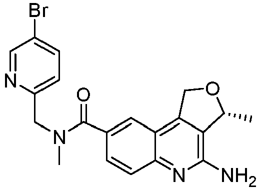 | (3R)-4-amino-N-((5-bromo-2-pyridinyl)methyl)-N,3-dimethyl-1,3-dihydrofuro[3,4-c]quinoline-8-carboxamide | HATU | 427.0 and 429.0 |
| **329** | 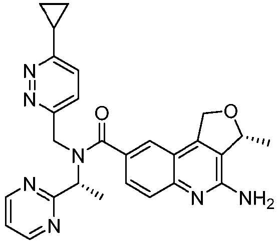 | (3R)-4-amino-N-((6-cyclopropyl-3-pyridazinyl)methyl)-3-methyl-N-((1R)-1-(2-pyrimidinyl)ethyl)-1,3-dihydrofuro[3,4-c]quinoline-8-carboxamide | HATU | 482 |
| **330** | 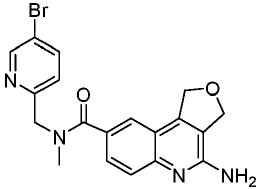 | 4-amino-N-((5-bromo-2-pyridinyl)methyl)-N-methyl-1,3-dihydrofuro[3,4-c]quinoline-8-carboxamide | HATU | 413.0 and 415.0 |
| **331** | 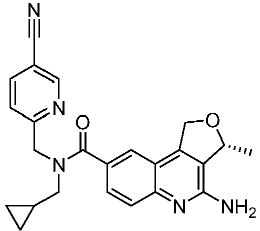 | (3R)-4-amino-N-((5-cyano-2-pyridinyl)methyl)-N-(cyclopropylmethyl)-3-methyl-1,3-dihydrofuro[3,4-c]quinoline-8-carboxamide | HATU | 414 |
| **332** | 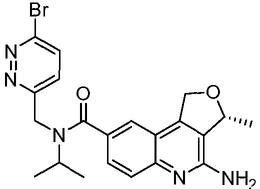 | (3R)-4-amino-N-((6-bromo-3-pyridazinyl)methyl)-3-methyl-N-(2-propanyl)-1,3-dihydrofuro[3,4-c]quinoline-8-carboxamide | HATU | 456 and 458 |
| **333** | 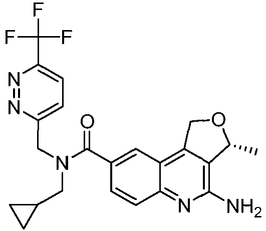 | (3R)-4-amino-N-(cyclopropylmethyl)-3-methyl-N-((6-(trifluoromethyl)-3-pyridazinyl)methyl)-1,3-dihydrofuro[3,4-c]quinoline-8-carboxamide | HATU | 458 |
| **334** | 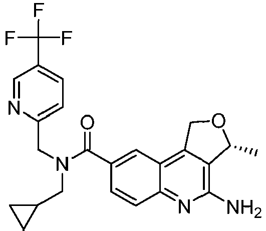 | (3R)-4-amino-N-(cyclopropylmethyl)-3-methyl-N-((5-(trifluoromethyl)-2-pyridinyl)methyl)-1,3-dihydrofuro[3,4-c]quinoline-8-carboxamide | HATU | 457 |
| **335** | 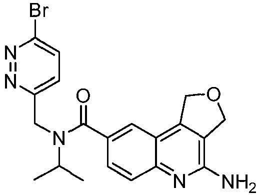 | 4-amino-N-((6-bromo-3-pyridazinyl)methyl)-N-(2-propanyl)-1,3-dihydrofuro[3,4-c]quinoline-8-carboxamide | HATU | 442 and 444 |
| **336** | 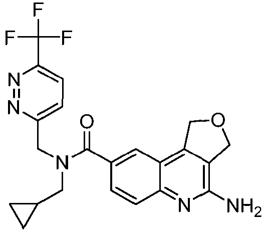 | 4-amino-N-(cyclopropylmethyl)-N-((6-(trifluoromethyl)-3-pyridazinyl)methyl)-1,3-dihydrofuro[3,4-c]quinoline-8-carboxamide | HATU | 444 |
| **337** | 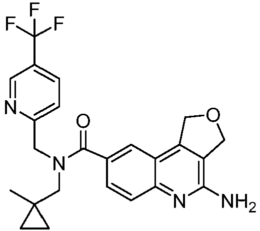 | 4-amino-N-((1-methylcyclopropyl)methyl)-N-((5-(trifluoromethyl)-2-pyridinyl)methyl)-1,3-dihydrofuro[3,4-c]quinoline-8-carboxamide | HATU | 457.2 |
| **338** | 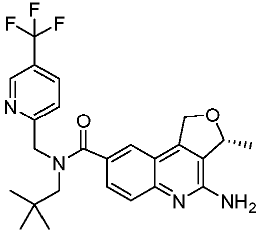 | (3R)-4-amino-N-(2,2-dimethylpropyl)-3-methyl-N-((5-(trifluoromethyl)-2-pyridinyl)methyl)-1,3-dihydrofuro[3,4-c]quinoline-8-carboxamide | HATU | 473.2 |
| **339** | 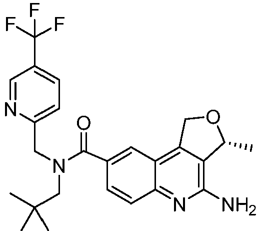 | (3R)-4-amino-3-methyl-N-((1-methylcyclopropyl)methyl)-N-((5-(trifluoromethyl)-2-pyridinyl)methyl)-1,3-dihydrofuro[3,4-c]quinoline-8-carboxamide | HATU | 471.2 |
| **340** | 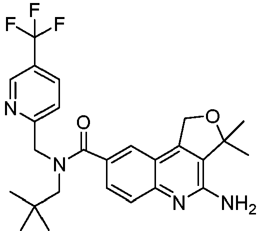 | 4-amino-N-(2,2-dimethylpropyl)-3,3-dimethyl-N-((5-(trifluoromethyl)-2-pyridinyl)methyl)-1,3-dihydrofuro[3,4-c]quinoline-8-carboxamide | HATU | 487.2 |
| **341** | 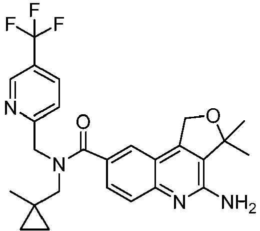 | 4-amino-3,3-dimethyl-N-((1-methylcyclopropyl)methyl)-N-((5-(trifluoromethyl)-2-pyridinyl)methyl)-1,3-dihydrofuro[3,4-c]quinoline-8-carboxamide | HATU | 485.2 |
| **342** | 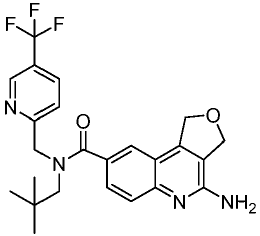 | 4-amino-N-(2,2-dimethylpropyl)-N-((5-(trifluoromethyl)-2-pyridinyl)methyl)-1,3-dihydrofuro[3,4-c]quinoline-8-carboxamide | HATU | 459.2 |
| **343** | 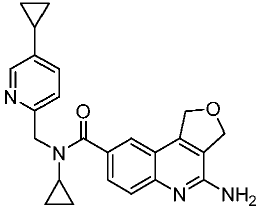 | 4-amino-N-cyclopropyl-N-((5-cyclopropyl-2-pyridinyl)methyl)-1,3-dihydrofuro[3,4-c]quinoline-8-carboxamide | HATU | 401.2 |
| **344** | 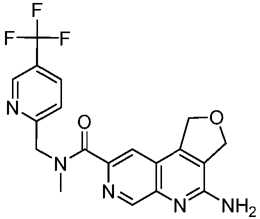 | 4-amino-N-methyl-N-((5-(trifluoromethyl)-2-pyridinyl)methyl)-1,3-dihydrofuro[3,4-c][1,7]naphthyridine-8-carboxamide | HATU | 404.1 |
| **345** | 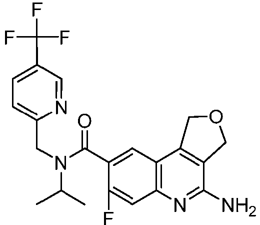 | 4-amino-7-fluoro-N-(2-propanyl)-N-((5-(trifluoromethyl)-2-pyridinyl)methyl)-1,3-dihydrofuro[3,4-c]quinoline-8-carboxamide | HATU | 449.2 |
| **346** | 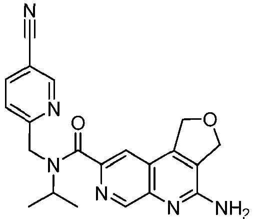 | 4-amino-N-((5-cyano-2-pyridinyl)methyl)-N-(2-propanyl)-1,3-dihydrofuro[3,4-c][1,7]naphthyridine-8-carboxamide | HATU | 389.2 |
| **347** | 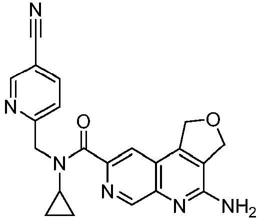 | 4-amino-N-((5-cyano-2-pyridinyl)methyl)-N-cyclopropyl-1,3-dihydrofuro[3,4-c][1,7]naphthyridine-8-carboxamide | HATU | 387.2 |
| **348** | 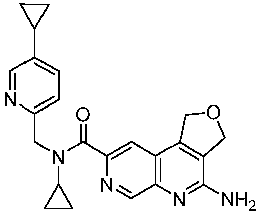 | 4-amino-N-cyclopropyl-N-((5-cyclopropyl-2-pyridinyl)methyl)-1,3-dihydrofuro[3,4-c][1,7]naphthyridine-8-carboxamide | HATU | 402.1 |
| **349** | 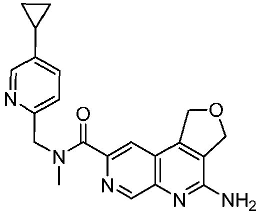 | 4-amino-N-((5-cyclopropyl-2-pyridinyl)methyl)-N-methyl-1,3-dihydrofuro[3,4-c][1,7]naphthyridine-8-carboxamide | HATU | 376.1 |
| **350** | 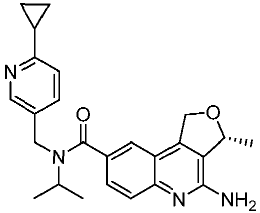 | (3R)-4-amino-N-((6-cyclopropyl-3-pyridinyl)methyl)-3-methyl-N-(2-propanyl)-1,3-dihydrofuro[3,4-c]quinoline-8-carboxamide | HATU | 417.2 |
| **351** | 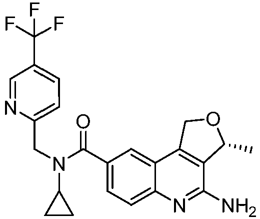 | (3R)-4-amino-N-cyclopropyl-3-methyl-N-((5-(trifluoromethyl)-2-pyridinyl)methyl)-1,3-dihydrofuro[3,4-c]quinoline-8-carboxamide | HATU | 443.1 |
| **352** | 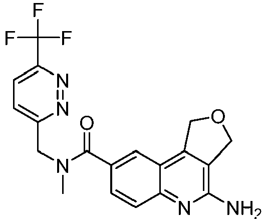 | 4-amino-N-methyl-N-((6-(trifluoromethyl)-3-pyridazinyl)methyl)-1,3-dihydrofuro[3,4-c]quinoline-8-carboxamide | HATU | 404.1 |
| **353** | 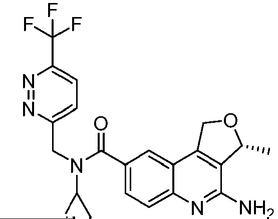 | (3R)-4-amino-N-cyclopropyl-3-methyl-N-((6-(trifluoromethyl)-3-pyridazinyl)methyl)-1,3-dihydrofuro[3,4-c]quinoline-8-carboxamide | HATU | 444.2 |
| **354** | 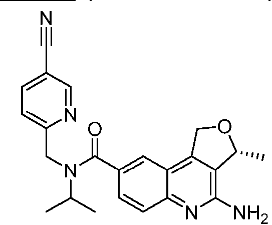 | (3R)-4-amino-N-((5-cyano-2-pyridinyl)methyl)-3-methyl-N-(2-propanyl)-1,3-dihydrofuro[3,4-c]quinoline-8-carboxamide | HATU | 402.2 |
| **355** | 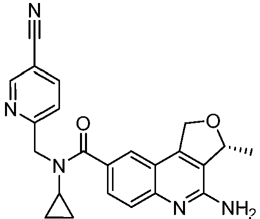 | (3R)-4-amino-N-((5-cyano-2-pyridinyl)methyl)-N-cyclopropyl-3-methyl-1,3-dihydrofuro [3,4-c]quinoline-8-carboxamide | HATU | 400.2 |
| **356** | 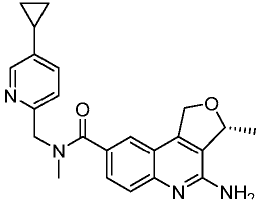 | (3R)-4-amino-N-((5-cyclopropyl-2-pyridinyl)methyl)-N,3-dimethyl-1,3-dihydrofuro[3,4-c]quinoline-8-carboxamide | HATU | 389.2 |
| **357** | 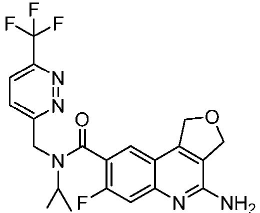 | 4-amino-N-cyclopropyl-7-fluoro-N-((6-(trifluoromethyl)-3-pyridazinyl)methyl)-1,3-dihydrofuro[3,4-c]quinoline-8-carboxamide | HATU | 448.1 |
| **358** | 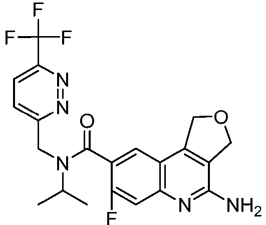 | 4-amino-7-fluoro-N-(2-propanyl)-N-((6-(trifluoromethyl)-3-pyridazinyl)methyl)-1,3-dihydrofuro[3,4-c]quinoline-8-carboxamide | HATU | 450.1 |
| **359** | 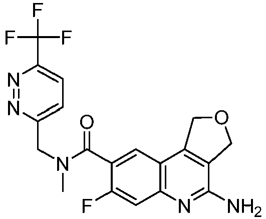 | 4-amino-7-fluoro-N-methyl-N-((6-(trifluoromethyl)-3-pyridazinyl)methyl)-1,3-dihydrofuro[3,4-c]quinoline-8-carboxamide | HATU | 422.1 |
| **360** | 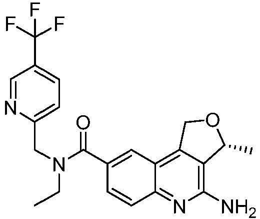 | (3R)-4-amino-N-ethyl-3-methyl-N-((5-(trifluoromethyl)-2-pyridinyl)methyl)-1,3-dihydrofuro[3,4-c]quinoline-8-carboxamide | HATU | 431.2 |
| **361** | 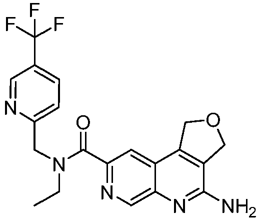 | 4-amino-N-ethyl-N-((5-(trifluoromethyl)-2-pyridinyl)methyl)-1,3-dihydrofuro[3,4-c][1,7]naphthyridine-8-carboxamide | HATU | 418.2 |
| **362** | 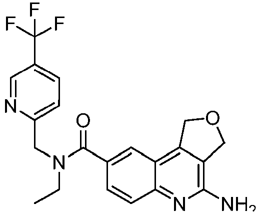 | 4-amino-N-ethyl-N-((5-(trifluoromethyl)-2-pyridinyl)methyl)-1,3-dihydrofuro[3,4-c]quinoline-8-carboxamide | HATU | 417.1 |
| **363** | 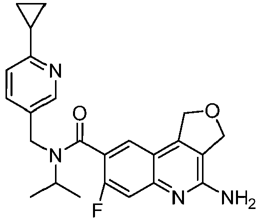 | 4-amino-N-((6-cyclopropyl-3-pyridinyl)methyl)-7-fluoro-N-(2-propanyl)-1,3-dihydrofuro[3,4-c]quinoline-8-carboxamide | HATU | 421.2 |
| **364** | 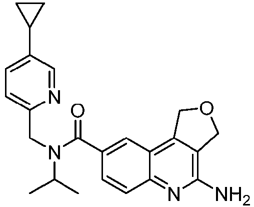 | 4-amino-N-((5-cyclopropyl-2-pyridinyl)methyl)-N-(2-propanyl)-1,3-dihydrofuro[3,4-c]quinoline-8-carboxamide | HATU | 403.2 |
| **365** | 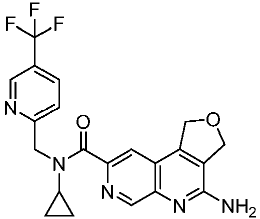 | 4-amino-N-cyclopropyl-N-((5-(trifluoromethyl)-2-pyridinyl)methyl)-1,3-dihydrofuro[3,4-c][1,7]naphthyridine-8-carboxamide | HATU | 430.1 |
| **366** | 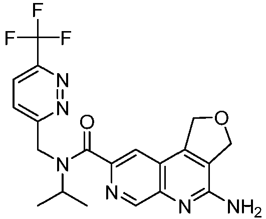 | 4-amino-N-(2-propanyl)-N-((6-(trifluoromethyl)-3-pyridazinyl)methyl)-1,3-dihydrofuro[3,4-c][1,7]naphthyridine-8-carboxamide | HATU | 433.2 |
| **367** | 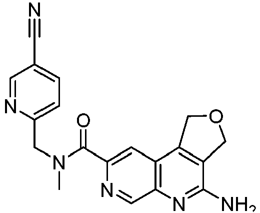 | 4-amino-N-((5-cyano-2-pyridinyl)methyl)-N-methyl-1,3-dihydrofuro[3,4-c][1,7]naphthyridine-8-carboxamide | HATU | 361.1 |
| **368** | 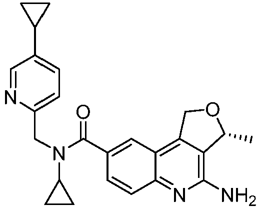 | (3R)-4-amino-N-cyclopropyl-N-((5-cyclopropyl-2-pyridinyl)methyl)-3-methyl-1,3-dihydrofuro[3,4-c]quinoline-8-carboxamide | HATU | 415.2 |
| **369** | 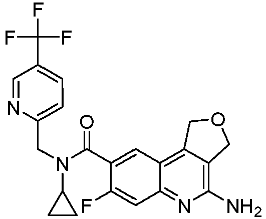 | 4-amino-N-cyclopropyl-7-fluoro-N-((5-(trifluoromethyl)-2-pyridinyl)methyl)-1,3-dihydrofuro[3,4-c]quinoline-8-carboxamide | HATU | 447.1 |
| **370** | 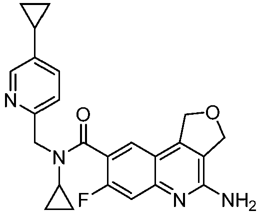 | 4-amino-N-cyclopropyl-N-((5-cyclopropyl-2-pyridinyl)methyl)-7-fluoro-1,3-dihydrofuro[3,4-c]quinoline-8-carboxamide | HATU | 419.2 |
| **371** | 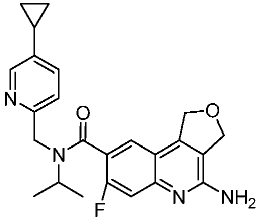 | 4-amino-N-((5-cyclopropyl-2-pyridinyl)methyl)-7-fluoro-N-(2-propanyl)-1,3-dihydrofuro[3,4-c]quinoline-8-carboxamide | HATU | 421.2 |
| **372** | 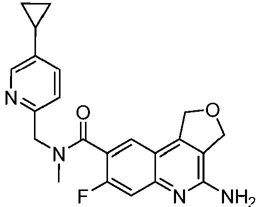 | 4-amino-N-((5-cyclopropyl-2-pyridinyl)methyl)-7-fluoro-N-methyl-1,3-dihydrofuro[3,4-c]quinoline-8-carboxamide | HATU | 393.2 |
| **373** | 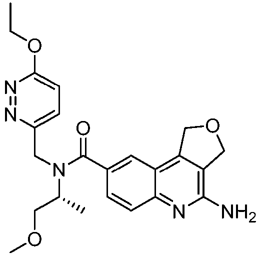 | 4-amino-N-((6-ethoxy-3-pyridazinyl)methyl)-N-((2R)-1-methoxy-2-propanyl)-1,3-dihydrofuro[3,4-c]quinoline-8-carboxamide | HATU | 438.2 |
| **374** | 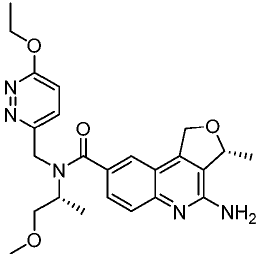 | (3R)-4-amino-N-((6-ethoxy-3-pyridazinyl)methyl)-N-((2R)-1-methoxy-2-propanyl)-3-methyl-1,3-dihydrofuro[3,4-c]quinoline-8-carboxamide | HATU | 452.3 |
| **375** | 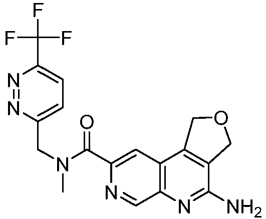 | 4-amino-N-methyl-N-((6-(trifluoromethyl)-3-pyridazinyl)methyl)-1,3-dihydrofuro[3,4-c][1,7]naphthyridine-8-carboxamide | HATU | 405.1 |
| **376** | 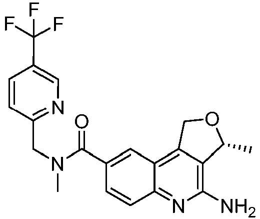 | (3R)-4-amino-N,3-dimethyl-N-((5-(trifluoromethyl)-2-pyridinyl)methyl)-1,3-dihydrofuro[3,4-c]quinoline-8-carboxamide | HATU | 417.1 |
| **377** | 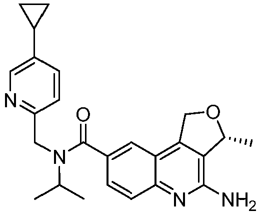 | (3R)-4-amino-N-((5-cyclopropyl-2-pyridinyl)methyl)-3-methyl-N-(2-propanyl)-1,3-dihydrofuro[3,4-c]quinoline-8-carboxamide | HATU | 417.2 |
| **378** | 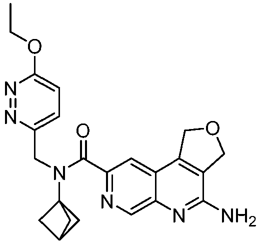 | 4-amino-N-(bicyclo[1.1.1]pentan-1-yl)-N-((6-ethoxy-3-pyridazinyl)methyl)-1,3-dihydrofuro[3,4-c][1,7]naphthyridine-8-carboxamide | HATU | 433.2 |
| **379** | 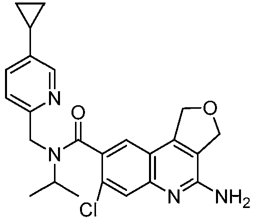 | 4-amino-7-chloro-N-((5-cyclopropyl-2-pyridinyl)methyl)-N-(2-propanyl)-1,3-dihydrofuro[3,4-c]quinoline-8-carboxamide | HATU | 437.2 |
| **380** | 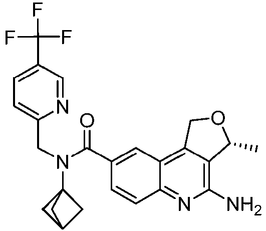 | (3R)-4-amino-N-(bicyclo[1.1.1]pentan-1-yl)-3-methyl-N-((5-(trifluoromethyl)-2-pyridinyl)methyl)-1,3-dihydrofuro[3,4-c]quinoline-8-carboxamide | HATU | 469.2 |
| **381** | 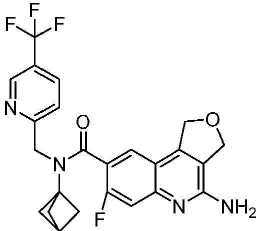 | 4-amino-N-(bicyclo[1.1.1]pentan-1-yl)-7-fluoro-N-((5-(trifluoromethyl)-2-pyridinyl)methyl)-1,3-dihydrofuro[3,4-c]quinoline-8-carboxamide | HATU | 473.2 |
| **382** | 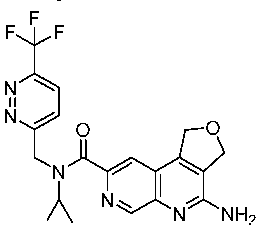 | 4-amino-N-cyclopropyl-N-((6-(trifluoromethyl)-3-pyridazinyl)methyl)-1,3-dihydrofuro[3,4-c][1,7]naphthyridine-8-carboxamide | HATU | 431.1 |
| **383** | 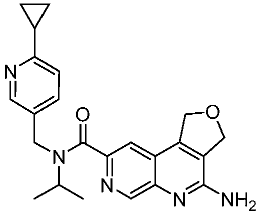 | 4-amino-N-((6-cyclopropyl-3-pyridinyl)methyl)-N-(2-propanyl)-1,3-dihydrofuro[3,4-c][1,7]naphthyridine-8-carboxamide | HATU | 404.2 |
| **384** | 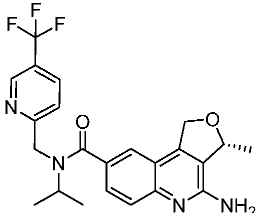 | (3R)-4-amino-3-methyl-N-(2-propanyl)-N-((5-(trifluoromethyl)-2-pyridinyl)methyl)-1,3-dihydrofuro[3,4-c]quinoline-8-carboxamide | HATU | 445.2 |
| **385** | 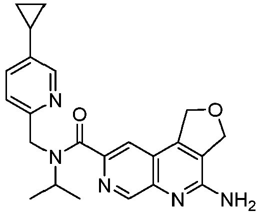 | 4-amino-N-((5-cyclopropyl-2-pyridinyl)methyl)-N-(2-propanyl)-1,3-dihydrofuro[3,4-c][1,7]naphthyridine-8-carboxamide | HATU | 404.2 |
| **386** | 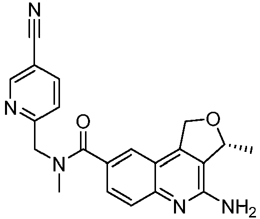 | (3R)-4-amino-N-((5-cyano-2-pyridinyl)methyl)-N,3-dimethyl-1,3-dihydrofuro[3,4-c]quinoline-8-carboxamide | HATU | 374.2 |
| **387** | 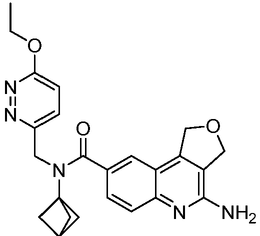 | 4-amino-N-(bicyclo[1.1.1]pentan-1-yl)-N-((6-ethoxy-3-pyridazinyl)methyl)-1,3-dihydrofuro[3,4-c]quinoline-8-carboxamide | HATU | 432.2 |
| **388** | 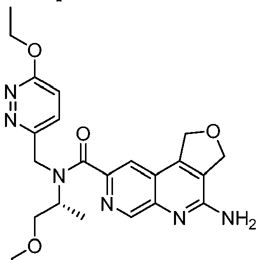 | 4-amino-N-((6-ethoxy-3-pyridazinyl)methyl)-N-((2R)-1-methoxy-2-propanyl)-1,3-dihydrofuro[3,4-c][1,7]naphthyridine-8-carboxamide | HATU | 439.2 |
| **389** | 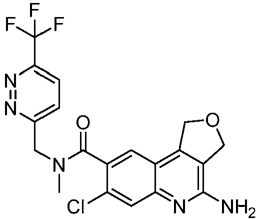 | 4-amino-7-chloro-N-methyl-N-((6-(trifluoromethyl)-3-pyridazinyl)methyl)-1,3-dihydrofuro[3,4-c]quinoline-8-carboxamide | HATU | 438.1 |
| **390** | 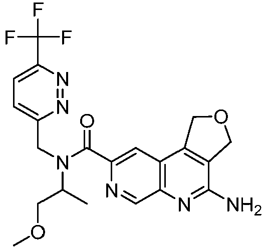 | 4-amino-N-((2R)-1-methoxy-2-propanyl)-N-((6-(trifluoromethyl)-3-pyridazinyl)methyl)-1,3-dihydrofuro[3,4-c][1,7]naphthyridine-8-carboxamide and 4-amino-N-((2S)-1-methoxy-2-propanyl)-N-((6-(trifluoromethyl)-3-pyridazinyl)methyl)-1,3-dihydrofuro[3,4-c][1,7]naphthyridine-8-carboxamide | HATU | 463.2 |
| **391** | 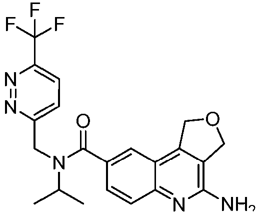 | 4-amino-N-(2-propanyl)-N-((6-(trifluoromethyl)-3-pyridazinyl)methyl)-1,3-dihydrofuro[3,4-c]quinoline-8-carboxamide | HATU | 432.1 |
| **392** | 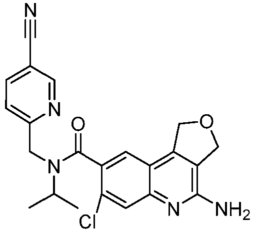 | 4-amino-7-chloro-N-((5-cyano-2-pyridinyl)methyl)-N-(2-propanyl)-1,3-dihydrofuro[3,4-c]quinoline-8-carboxamide | HATU | 422.1 |
| **393** | 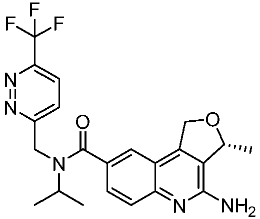 | (3R)-4-amino-3-methyl-N-(2-propanyl)-N-((6-(trifluoromethyl)-3-pyridazinyl)methyl)-1,3-dihydrofuro[3,4-c]quinoline-8-carboxamide | HATU | 446.2 |
| **394** | 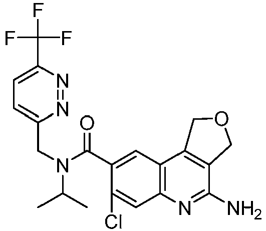 | 4-amino-7-chloro-N-(2-propanyl)-N-((6-(trifluoromethyl)-3-pyridazinyl)methyl)-1,3-dihydrofuro[3,4-c]quinoline-8-carboxamide | HATU | 466.1 |
| **395** | 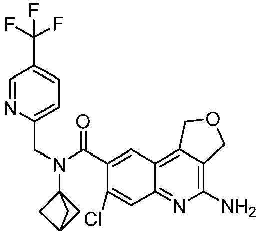 | 4-amino-N-(bicyclo[1.1.1]pentan-1-yl)-7-chloro-N-((5-(trifluoromethyl)-2-pyridinyl)methyl)-1,3-dihydrofuro[3,4-c]quinoline-8-carboxamide | HATU | 489.1 |
| **396** | 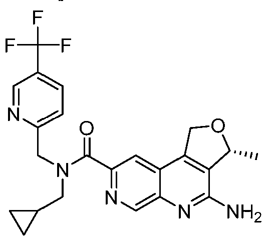 | (3R)-4-amino-N-(cyclopropylmethyl)-3-methyl-N-((5-(trifluoromethyl)-2-pyridinyl)methyl)-1,3-dihydrofuro[3,4-c][1,7]naphthyridine-8-carboxamide | HATU | 458.2 |
| **397** | 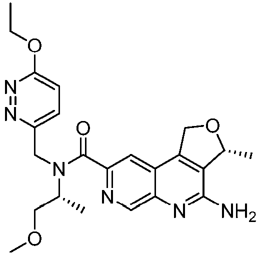 | (3R)-4-amino-N-((6-ethoxy-3-pyridazinyl)methyl)-N-((2R)-1-methoxy-2-propanyl)-3-methyl-1,3-dihydrofuro[3,4-c][1,7]naphthyridine-8-carboxamide | HATU | 453.2 |
| **398** | 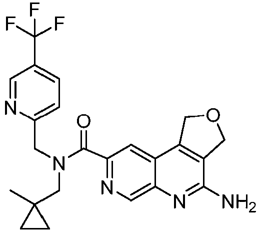 | 4-amino-N-((1-methylcyclopropyl)methyl)-N-((5-(trifluoromethyl)-2-pyridinyl)methyl)-1,3-dihydrofuro[3,4-c][1,7]naphthyridine-8-carboxamide | HATU | 458.1 |
| **399** | 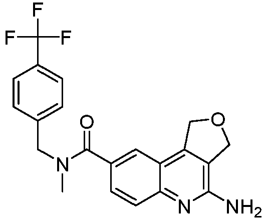 | 4-amino-N-methyl-N-(4-(trifluoromethyl)benzyl)-1,3-dihydrofuro[3,4-c]quinoline-8-carboxamide | HATU | 402 |
| **400** | 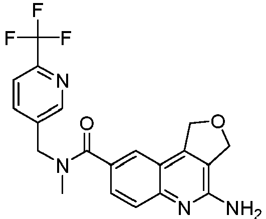 | 4-amino-N-methyl-N-((6-(trifluoromethyl)-3-pyridinyl)methyl)-1,3-dihydrofuro[3,4-c]quinoline-8-carboxamide | HATU | 403 |
| **401** | 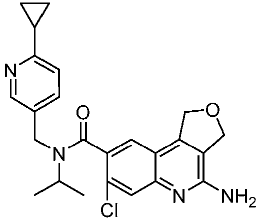 | 4-amino-7-chloro-N-((6-cyclopropyl-3-pyridinyl)methyl)-N-(2-propanyl)-1,3-dihydrofuro[3,4-c]quinoline-8-carboxamide | HATU | 437.2 |
| **402** | 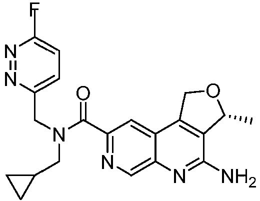 | (3R)-4-amino-N-(cyclopropylmethyl)-3-methyl-N-((6-(trifluoromethyl)-3-pyridazinyl)methyl)-1,3-dihydrofuro[3,4-c][1,7]naphthyridine-8-carboxamide | HATU | 495.2 |
| **403** | 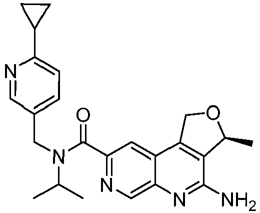 | (3 S)-4-amino-N-((6-cyclopropyl-3-pyridinyl)methyl)-3-methyl-N-(2-propanyl)-1,3-dihydrofuro[3,4-c][1,7]naphthyridine-8-carboxamide | HATU | 418.1 |
| **404** | 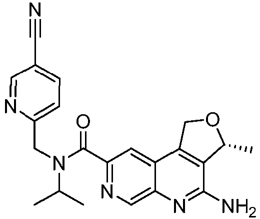 | (3R)-4-amino-N-((5-cyano-2-pyridinyl)methyl)-3-methyl-N-(2-propanyl)-1,3-dihydrofuro[3,4-c][1, 7]naphthyridine-8-carboxamide | HATU | 403.2 |
| **405** | 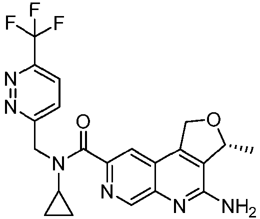 | (3R)-4-amino-N-cyclopropyl-3-methyl-N-((6-(trifluoromethyl)-3-pyridazinyl)methyl)-1,3-dihydrofuro[3,4-c][1,7]naphthyridine-8-carboxamide | HATU | 445.1 |
| **406** | 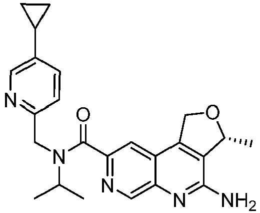 | (3R)-4-amino-N-((5-cyclopropyl-2-pyridinyl)methyl)-3-methyl-N-(2-propanyl)-1,3-dihydrofuro[3,4-c][1,7]naphthyridine-8-carboxamide | HATU | 418.1 |
| **407** | 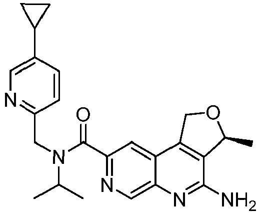 | (3S)-4-amino-N-((5-cyclopropyl-2-pyridinyl)methyl)-3-methyl-N-(2-propanyl)-1,3-dihydrofuro[3,4-c][1,7]naphthyridine-8-carboxamide | HATU | 418.1 |
| **408** | 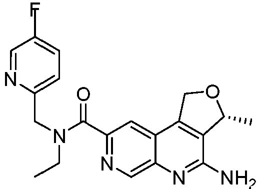 | (3R)-4-amino-N-ethyl-3-methyl-N-((5-(trifluoromethyl)-2-pyridinyl)methyl)-1,3-dihydrofuro[3,4-c][1,7]naphthyridine-8-carboxamide | HATU | 432.2 |
| **409** | 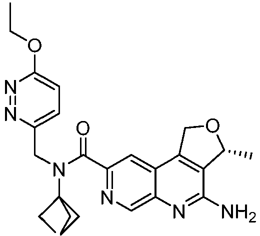 | (3R)-4-amino-N-(bicyclo[1.1.1]pentan-1-yl)-N-((6-ethoxy-3-pyridazinyl)methyl)-3-methyl-1,3-dihydrofuro [3,4-c][1,7]naphthyridine-8-carboxamide | HATU | 447.2 |
| **410** | 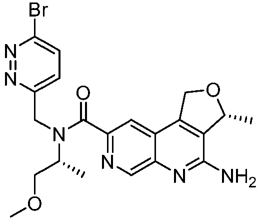 | (3R)-4-amino-N-((6-bromo-3-pyridazinyl)methyl)-N-((2R)-1-methoxy-2-propanyl)-3-methyl-1,3-dihydrofuro[3,4-c][1,7]naphthyridine-8-carboxamide | HATU | 487.1 |
| **411** | 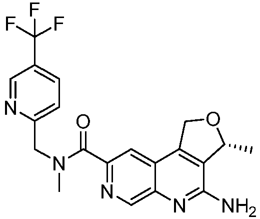 | (3R)-4-amino-N,3-dimethyl-N-((5-(trifluoromethyl)-2-pyridinyl)methyl)-1,3-dihydrofuro[3,4-c][1,7]naphthyridine-8-carboxamide | HATU | 418.1 |
| **412** | 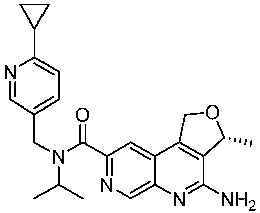 | (3R)-4-amino-N-((6-cyclopropyl-3-pyridinyl)methyl)-3-methyl-N-(2-propanyl)-1,3-dihydrofuro[3,4-c][1,7]naphthyridine-8-carboxamide | HATU | |
| **413** | 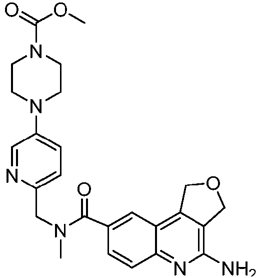 | methyl 4-(6-((((4-amino-1,3-dihydrofuro[3,4-c]quinolin-8-yl)carbonyl)(methyl)amino)methy 1)-3-pyridinyl)-1-piperazinecarboxylate | HATU | 477.1 |
| **414** | 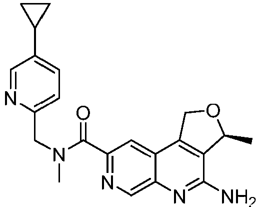 | (3S)-4-amino-N-((5-cyclopropyl-2-pyridinyl)methyl)-N,3-dimethyl-1,3-dihydrofuro [3,4-c][1,7]naphthyridine-8-carboxamide | HATU | 390.2 |
| **415** | 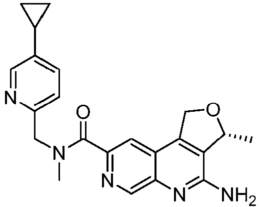 | (3R)-4-amino-N-((5-cyclopropyl-2-pyridinyl)methyl)-N,3-dimethyl-1,3-dihydrofuro[3,4-c][1,7]naphthyridine-8-carboxamide | HATU | 390.2 |
| **416** | 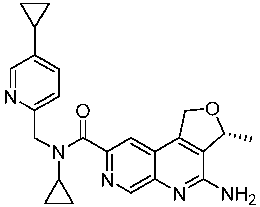 | (3R)-4-amino-N-cyclopropyl-N-((5-cyclopropyl-2-pyridinyl)methyl)-3-methyl-1,3-dihydrofuro[3,4-c][1,7]naphthyridine-8-carboxamide | HATU | 416.1 |
| **417** | 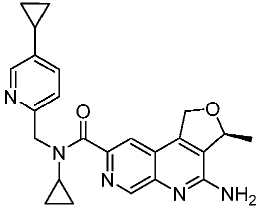 | (3S)-4-amino-N-cyclopropyl-N-((5-cyclopropyl-2-pyridinyl)methyl)-3-methyl-1,3-dihydrofuro[3,4-c][1,7]naphthyridine-8-carboxamide | HATU | |
| **418** | 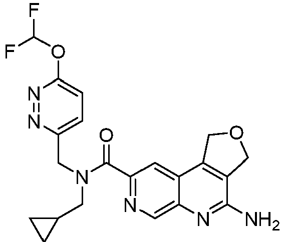 | 4-amino-N-(cyclopropylmethyl)-N-((6-(difluoromethoxy)-3-pyridazinyl)methyl)-1,3-dihydrofuro[3,4-c][1,7]naphthyridine-8-carboxamide | HATU | 443 |
| **419** | 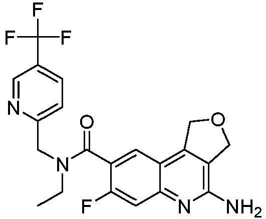 | 4-amino-N-ethyl-7-fluoro-N-((5-(trifluoromethyl)-2-pyridinyl)methyl)-1,3-dihydrofuro[3,4-c]quinoline-8-carboxamide | HATU | 435 |
| **420** | 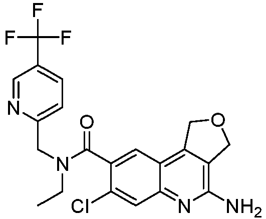 | 4-amino-7-chloro-N-ethyl-N-((5-(trifluoromethyl)-2-pyridinyl)methyl)-1,3-dihydrofuro[3,4-c]quinoline-8-carboxamide | HATU | 451 |
| **421** | 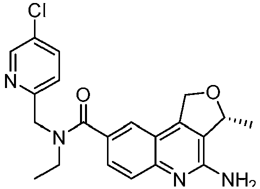 | (3R)-4-amino-N-((5-chloro-2-pyridinyl)methyl)-N-ethyl-3-methyl-1,3-dihydrofuro[3,4-c]quinoline-8-carboxamide | HATU | 397 |
| **422** | 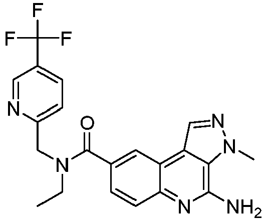 | 4-amino-N-ethyl-3-methyl-N-((5-(trifluoromethyl)-2-pyridinyl)methyl)-3H-pyrazolo[3,4-c]quinoline-8-carboxamide | HATU | 429 |
| **423** | 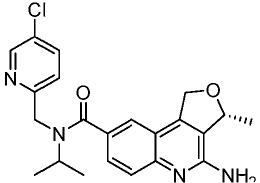 | (3R)-4-amino-N-((5-chloro-2-pyridinyl)methyl)-3-methyl-N-(2-propanyl)-1,3-dihydrofuro[3,4-c]quinoline-8-carboxamide | HATU | 411 |
| **424** | 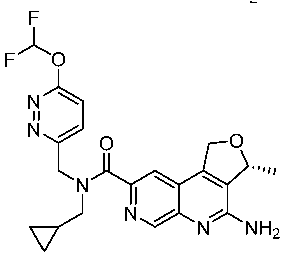 | (3R)-4-amino-N-(cyclopropylmethyl)-N-((6-(difluoromethoxy)-3-pyridazinyl)methyl)-3-methyl-1,3-dihydrofuro[3,4-c][1,7]naphthyridine-8-carboxamide | HATU | 457 |
| **425** | 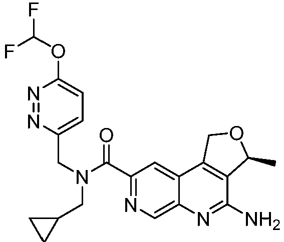 | (3S)-4-amino-N-(cyclopropylmethyl)-N-((6-(difluoromethoxy)-3-pyridazinyl)methyl)-3-methyl-1,3-dihydrofuro[3,4-c][1,7]naphthyridine-8-carboxamide | HATU | 457 |
| **426** | 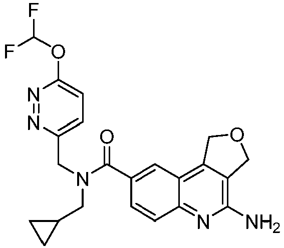 | 4-amino-N-(cyclopropylmethyl)-N-((6-(difluoromethoxy)-3-pyridazinyl)methyl)-1,3-dihydrofuro[3,4-c]quinoline-8-carboxamide | HATU | 442 |
| **427** | 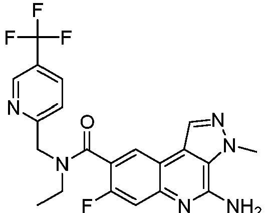 | 4-amino-N-ethyl-7-fluoro-3-methyl-N-((5-(trifluoromethyl)-2-pyridinyl)methyl)-3H-pyrazolo[3,4-c]quinoline-8-carboxamide | HATU | 447 |
| **428** | 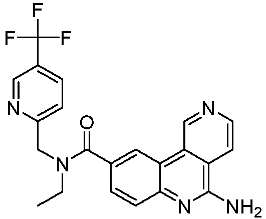 | 5-amino-N-ethyl-N-((5-(trifluoromethyl)-2-pyridinyl)methyl)benzo[c][2,6]na phthyridine-9-carboxamide | HATU | 426 |
| **429** | 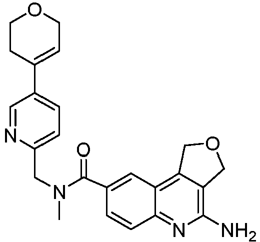 | 4-amino-N-((5-(3,6-dihydro-2H-pyran-4-yl)-2-pyridinyl)methyl)-N-methyl-1,3-dihydrofuro[3,4-c]quinoline-8-carboxamide | HATU | 417.1 |
| **430** | 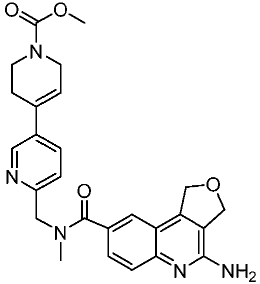 | methyl 6-((((4-amino-1,3-dihydrofuro[3,4-c]quinolin-8-yl)carbonyl)(methyl)amino)methy l)-3',6'-dihydro[3,4'-bipyridine]-1'(2'H)-carboxylate | HATU | 474.1 |
| **431** | 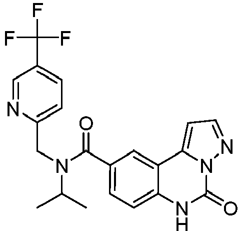 | 5-oxo-N-(2-propanyl)-N-((5-(trifluoromethyl)-2-pyridinyl)methyl)-5,6-dihydropyrazolo[1,5-c]quinazoline-9-carboxamide | HATU | 430.2 |
| **432** | 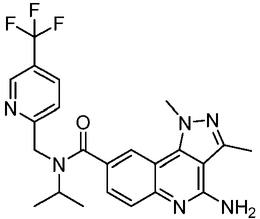 | 4-amino-1,3-dimethyl-N-(2-propanyl)-N-((5-(trifluoromethyl)-2-pyridinyl)methyl)-1H-pyrazolo[4,3-c]quinoline-8-carboxamide | HATU | 457.2 |
| **433** | 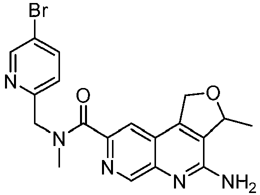 | (3R)-4-amino-N-((5-bromo-2-pyridinyl)methyl)-N,3-dimethyl-1,3-dihydrofuro[3,4-c][1,7]naphthyridine-8-carboxamide and (3S)-4-amino-N-((5-bromo-2-pyridinyl)methyl)-N,3-dimethyl-1,3-dihydrofuro[3,4-c][1,7]naphthyridine-8-carboxamide | HATU | 428 and 430 |
| **434** | 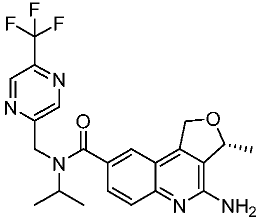 | (3R)-4-amino-3-methyl-N-(2-propanyl)-N-((5-(trifluoromethyl)-2-pyrazinyl)methyl)-1,3-dihydrofuro[3,4-c]quinoline-8-carboxamide | HBTU | 446.2 |
| **435** | 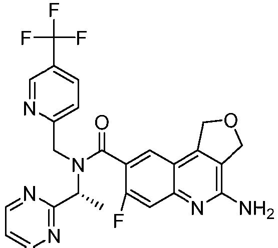 | 4-amino-7-fluoro-N-((1R)-1-(2-pyrimidinyl)ethyl)-N-((5-(trifluoromethyl)-2-pyridinyl)methyl)-1,3-dihydrofuro[3,4-c]quinoline-8-carboxamide | PyBroP | 513.2 |
| **436** | 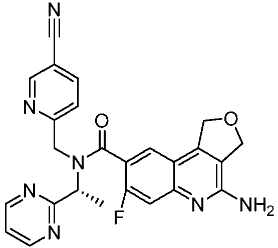 | 4-amino-N-((5-cyano-2-pyridinyl)methyl)-7-fluoro-N-((1R)-1-(2-pyrimidinyl)ethyl)-1,3-dihydrofuro[3,4-c]quinoline-8-carboxamide | PyBroP | 470.2 |
| **437** | 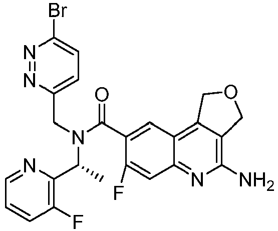 | 4-amino-N-((6-bromo-3-pyridazinyl)methyl)-7-fluoro-N-((1R)-1-(3-fluoro-2-pyridinyl)ethyl)-1,3-dihydrofuro[3,4-c]quinoline-8-carboxamide | PyBroP | 541 and 543 |
| **438** | 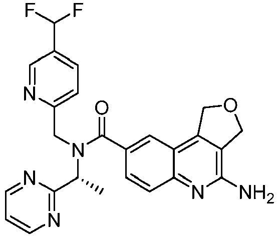 | 4-amino-N-((5-(difluoromethyl)-2-pyridinyl)methyl)-N-((1R)-1-(2-pyrimidinyl)ethyl)-1,3-dihydrofuro[3,4-c]quinoline-8-carboxamide | PyBroP | 477.2 |
| **439** | 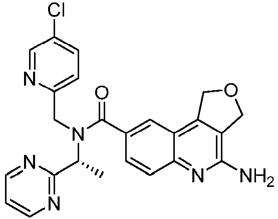 | 4-amino-N-((5-chloro-2-pyridinyl)methyl)-N-((1R)-1-(2-pyrimidinyl)ethyl)-1,3-dihydrofuro[3,4-c]quinoline-8-carboxamide | PyBroP | 461.2 |
| **440** | 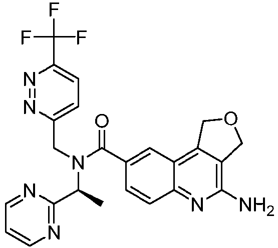 | 4-amino-N-((1S)-1-(2-pyrimidinyl)ethyl)-N-((6-(trifluoromethyl)-3-pyridazinyl)methyl)-1,3-dihydrofuro[3,4-c]quinoline-8-carboxamide | PyBroP | 496.2 |
| **441** | 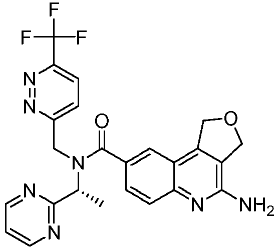 | 4-amino-N-((1R)-1-(2-pyrimidinyl)ethyl)-N-((6-(trifluoromethyl)-3-pyridazinyl)methyl)-1,3-dihydrofuro[3,4-c]quinoline-8-carboxamide | PyBroP | 496.2 |
| **442** | 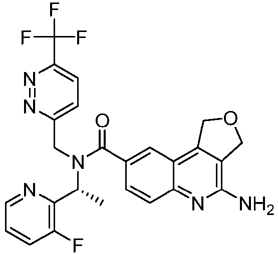 | 4-amino-N-((1R)-1-(3-fluoro-2-pyridinyl)ethyl)-N-((6-(trifluoromethyl)-3-pyridazinyl)methyl)-1,3-dihydrofuro[3,4-c]quinoline-8-carboxamide | PyBroP | 513.2 |
| **443** | 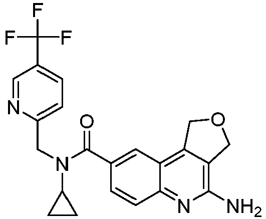 | 4-amino-N-cyclopropyl-N-((5-(trifluoromethyl)-2-pyridinyl)methyl)-1,3-dihydrofuro[3,4-c]quinoline-8-carboxamide | PyBroP | 429.2 |
| **444** | 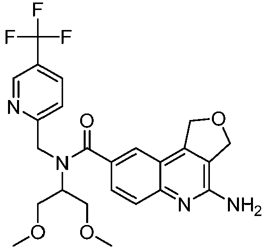 | 4-amino-N-(1,3-dimethoxy-2-propanyl)-N-((5-(trifluoromethyl)-2-pyridinyl)methyl)-1,3-dihydrofuro[3,4-c]quinoline-8-carboxamide | PyBroP | 491.2 |
| **445** | 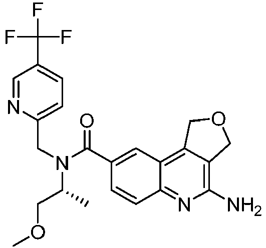 | 4-amino-N-((2R)-1-methoxy-2-propanyl)-N-((5-(trifluoromethyl)-2-pyridinyl)methyl)-1,3-dihydrofuro[3,4-c]quinoline-8-carboxamide | PyBroP | 461.2 |
| **446** | 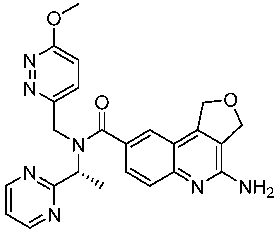 | 4-amino-N-((6-methoxy-3-pyridazinyl)methyl)-N-((1R)-1-(2-pyrimidinyl)ethyl)-1,3-dihydrofuro[3,4-c]quinoline-8-carboxamide | PyBroP | 458.2 |
| **447** | 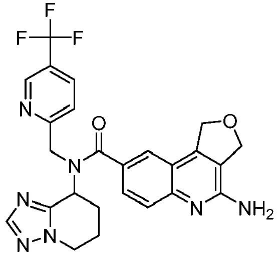 | 4-amino-N-((8R)-5,6,7,8-tetrahydro[1,2,4]triazolo[1,5-a]pyridin-8-yl)-N-((5-(trifluoromethyl)-2-pyridinyl)methyl)-1,3-dihydrofuro[3,4-c]quinoline-8-carboxamide and 4-amino-N-((8S)-5,6,7,8-tetrahydro[1,2,4]triazolo[1,5-a]pyridin-8-yl)-N-((5-(trifluoromethyl)-2-pyridinyl)methyl)-1,3-dihydrofuro[3,4-c]quinoline-8-carboxamide | PyBroP | 510.2 |
| **448** | 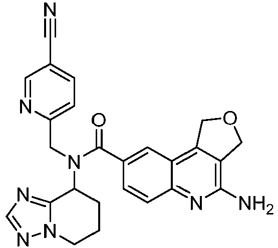 | 4-amino-N-((5-cyano-2-pyridinyl)methyl)-N-((8R)-5,6,7,8-tetrahydro[1,2,4]triazolo[1,5-a]pyridin-8-yl)-1,3-dihydrofuro[3,4-c]quinoline-8-carboxamide and 4-amino-N-((5-cyano-2-pyridinyl)methyl)-N-((8S)-5,6,7,8-tetrahydro[1,2,4]triazolo[1,5-a]pyridin-8-yl)-1,3-dihydrofuro[3,4-c]quinoline-8-carboxamide | PyBroP | 467.2 |
| **449** | 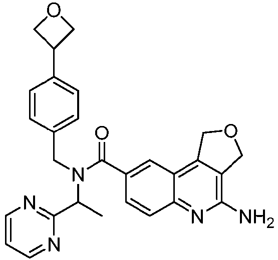 | 4-amino-N-(4-(3-oxetanyl)benzyl)-N-((1R)-1-(2-pyrimidinyl)ethyl)-1,3-dihydrofuro[3,4-c]quinoline-8-carboxamide and 4-amino-N-(4-(3-oxetanyl)benzyl)-N-((1S)-1-(2-pyrimidinyl)ethyl)-1,3-dihydrofuro[3,4-c]quinoline-8-carboxamide | PyBroP | 481.6 |
| **450** | 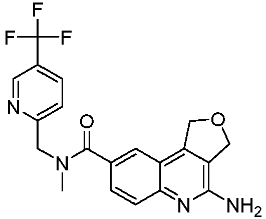 | 4-amino-N-methyl-N-((5-(trifluoromethyl)-2-pyridinyl)methyl)-1,3-dihydrofuro[3,4-c]quinoline-8-carboxamide | PyBroP | 403.4 |
| **451** | 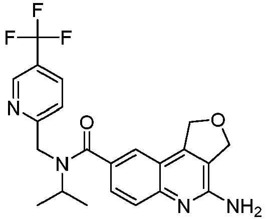 | 4-amino-N-(2-propanyl)-N-((5-(trifluoromethyl)-2-pyridinyl)methyl)-1,3-dihydrofuro[3,4-c]quinoline-8-carboxamide | PyBroP | 431.4 |
| **452** | 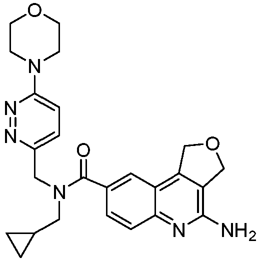 | 4-amino-N-(cyclopropylmethyl)-N-((6-(4-morpholinyl)-3-pyridazinyl)methyl)-1,3-dihydrofuro[3,4-c]quinoline-8-carboxamide | PyBroP | 461 |
| **453** | 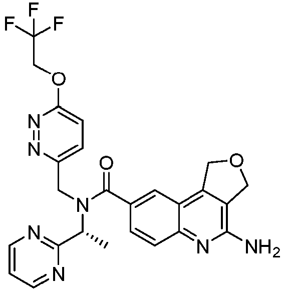 | 4-amino-N-((1R)-1-(2-pyrimidinyl)ethyl)-N-((6-(2,2,2-trifluoroethoxy)-3-pyridazinyl)methyl)-1,3-dihydrofuro[3,4-c]quinoline-8-carboxamide | PyBroP | 526.2 |
| **454** | 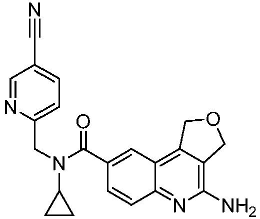 | 4-amino-N-((5-cyano-2-pyridinyl)methyl)-N-cyclopropyl-1,3-dihydrofuro[3,4-c]quinoline-8-carboxamide | PyBroP | 386.1 |
| **455** | 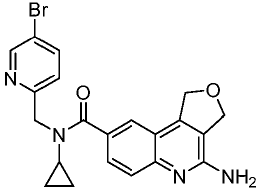 | 4-amino-N-((5-bromo-2-pyridinyl)methyl)-N-cyclopropyl-1,3-dihydrofuro[3,4-c]quinoline-8-carboxamide | PyBroP | 439.0 and 441.0 |
| **456** | 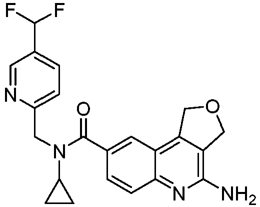 | 4-amino-N-cyclopropyl-N-((5-(difluoromethyl)-2-pyridinyl)methyl)-1,3-dihydrofuro[3,4-c]quinoline-8-carboxamide | PyBroP | 411.2 |
| **457** | 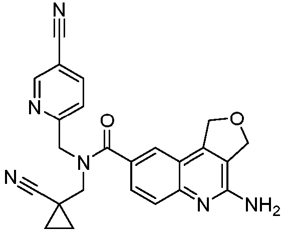 | 4-amino-N-((1-cyanocyclopropyl)methyl)-N-((5-cyano-2-pyridinyl)methyl)-1,3-dihydrofuro[3,4-c]quinoline-8-carboxamide | PyBroP | 425 |
| **458** | 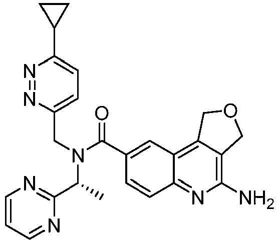 | 4-amino-N-((6-cyclopropyl-3-pyridazinyl)methyl)-N-((1R)-1-(2-pyrimidinyl)ethyl)-1,3-dihydrofuro[3,4-c]quinoline-8-carboxamide | PyBroP | 468 |
| **459** | 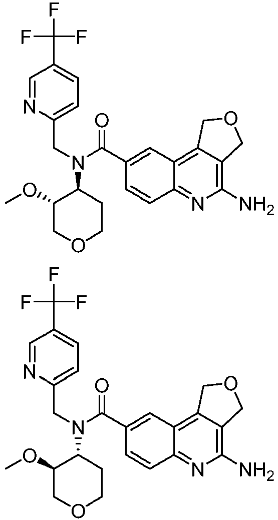 | 4-amino-N-((3R,4S)-3-methoxytetrahydro-2H-pyran-4-yl)-N-((5-(trifluoromethyl)-2-pyridinyl)methyl)-1,3-dihydrofuro[3,4-c]quinoline-8-carboxamide and 4-amino-N-((3S,4R)-3-methoxytetrahydro-2H-pyran-4-yl)-N-((5-(trifluoromethyl)-2-pyridinyl)methyl)-1,3-dihydrofuro[3,4-c]quinoline-8-carboxamide | PyBroP | 503.2 |
| **460** | 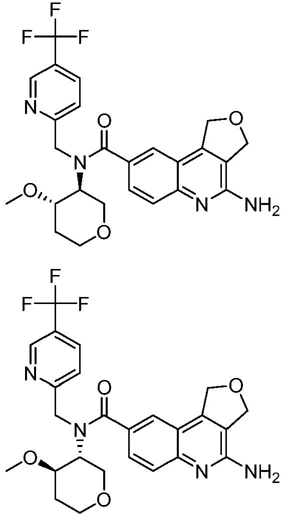 | 4-amino-N-((3R,4R)-4-methoxytetrahydro-2H-pyran-3-yl)-N-((5-(trifluoromethyl)-2-pyridinyl)methyl)-1,3-dihydrofuro[3,4-c]quinoline-8-carboxamide and 4-amino-N-((3S,4S)-4-methoxytetrahydro-2H-pyran-3-yl)-N-((5-(trifluoromethyl)-2-pyridinyl)methyl)-1,3-dihydrofuro[3,4-c]quinoline-8-carboxamide | PyBroP | 503.2 |
| **461** | 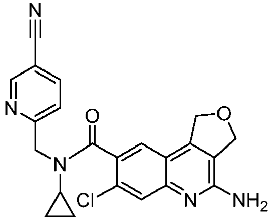 | 4-amino-7-chloro-N-((5-cyano-2-pyridinyl)methyl)-N-cyclopropyl-1,3-dihydrofuro[3,4-c]quinoline-8-carboxamide | PyBroP | 420.2 |
| **462** | 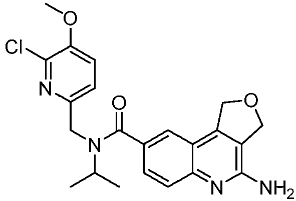 | 4-amino-N-((6-chloro-5-methoxy-2-pyridinyl)methyl)-N-(2-propanyl)-1,3-dihydrofuro[3,4-c]quinoline-8-carboxamide | PyBroP | 427 |
| **463** | 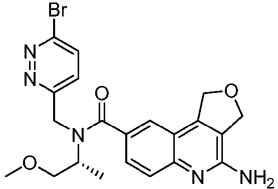 | 4-amino-N-((6-bromo-3-pyridazinyl)methyl)-N-((2R)-1-methoxy-2-propanyl)-1,3-dihydrofuro[3,4-c]quinoline-8-carboxamide | PyBroP | 472.1 and 474.1 |
| **464** | 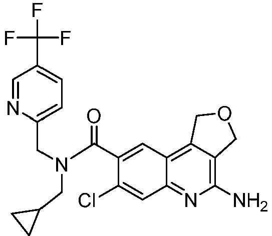 | 4-amino-7-chloro-N-(cyclopropylmethyl)-N-((5-(trifluoromethyl)-2-pyridinyl)methyl)-1,3-dihydrofuro[3,4-c]quinoline-8-carboxamide | PyBroP | 477.2 |
| **465** | 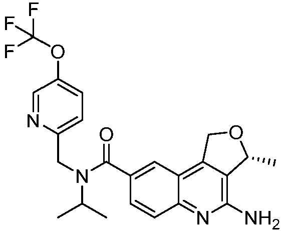 | (3R)-4-amino-3-methyl-N-(2-propanyl)-N-((5-(trifluoromethoxy)-2-pyridinyl)methyl)-1,3-dihydrofuro[3,4-c]quinoline-8-carboxamide | PyBroP | 461.2 |
| **466** | 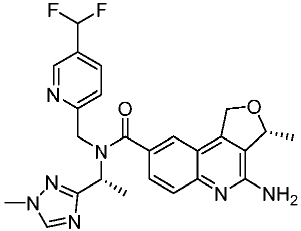 | (3R)-4-amino-N-((5-(difluoromethyl)-2-pyridinyl)methyl)-3-methyl-N-((1R)-1-(1-methyl-1H-1,2,4-triazol-3-yl)ethyl)-1,3-dihydrofuro[3,4-c]quinoline-8-carboxamide | PyBroP | 494 |
| **467** | 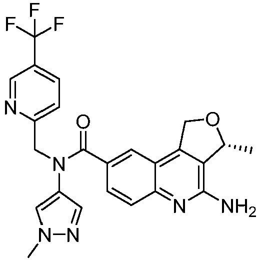 | (3R)-4-amino-3-methyl-N-(1-methyl-1H-pyrazol-4-yl)-N-((5-(trifluoromethyl)-2-pyridinyl)methyl)-1,3-dihydrofuro[3,4-c]quinoline-8-carboxamide | PyBroP | 483.1 |
| **468** | 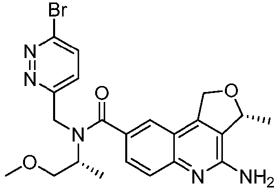 | (3R)-4-amino-N-((6-bromo-3-pyridazinyl)methyl)-N-((2R)-1-methoxy-2-propanyl)-3-methyl-1,3-dihydrofuro[3,4-c]quinoline-8-carboxamide | PyBroP | 486.0 and 488.0 |
| **469** | 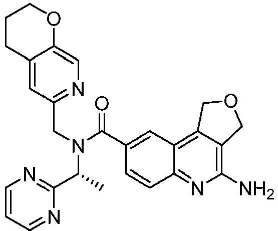 | 4-amino-N-(3,4-dihydro-2H-pyrano[2,3-c]pyridin-6-ylmethyl)-N-((1R)-1-(2-pyrimidinyl)ethyl)-1,3-dihydrofuro[3,4-c]quinoline-8-carboxamide | PyBroP | 483.2 |
| **470** | 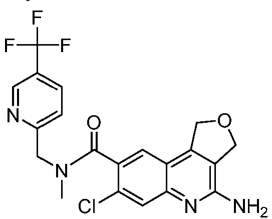 | 4-amino-7-chloro-N-methyl-N-((5-(trifluoromethyl)-2-pyridinyl)methyl)-1,3-dihydrofuro[3,4-c]quinoline-8-carboxamide | PyBroP | 437.2 |
| **471** | 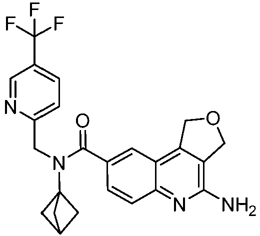 | 4-amino-N-(bicyclo[1.1.1]pentan-1-yl)-N-((5-(trifluoromethyl)-2-pyridinyl)methyl)-1,3-dihydrofuro[3,4-c]quinoline-8-carboxamide | PyBroP | 455.2 |
| **472** | 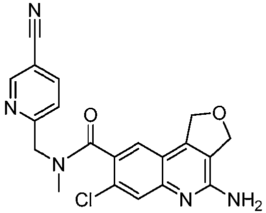 | 4-amino-7-chloro-N-((5-cyano-2-pyridinyl)methyl)-N-methyl-1,3-dihydrofuro[3,4-c]quinoline-8-carboxamide | PyBroP | 394 |
| **473** | 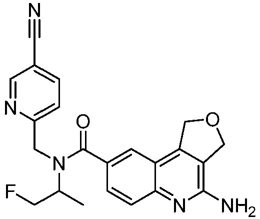 | 4-amino-N-((5-cyano-2-pyridinyl)methyl)-N-((2R)-1-fluoro-2-propanyl)-1,3-dihydrofuro[3,4-c]quinoline-8-carboxamide and 4-amino-N-((5-cyano-2-pyridinyl)methyl)-N-((2S)-1-fluoro-2-propanyl)-1,3-dihydrofuro[3,4-c]quinoline-8-carboxamide | PyBroP | 406.2 |
| **474** | 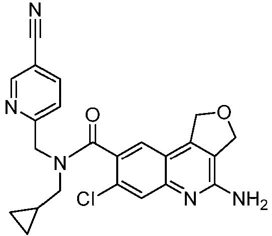 | 4-amino-7-chloro-N-((5-cyano-2-pyridinyl)methyl)-N-(cyclopropylmethyl)-1,3-dihydrofuro[3,4-c]quinoline-8-carboxamide | PyBroP | 434 |
| **475** | 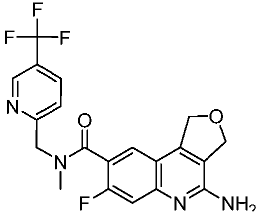 | 4-amino-7-fluoro-N-methyl-N-((5-(trifluoromethyl)-2-pyridinyl)methyl)-1,3-dihydrofuro[3,4-c]quinoline-8-carboxamide | PyBroP | 421.2 |
| **476** | 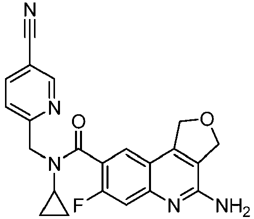 | 4-amino-N-((5-cyano-2-pyridinyl)methyl)-N-cyclopropyl-7-fluoro-1,3-dihydrofuro[3,4-c]quinoline-8-carboxamide | PyBroP | 404.1 |
| **477** | 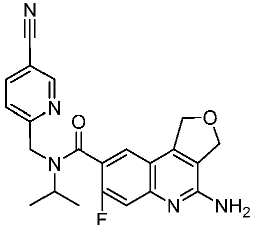 | 4-amino-N-((5-cyano-2-pyridinyl)methyl)-7-fluoro-N-(2-propanyl)-1,3-dihydrofuro[3,4-c]quinoline-8-carboxamide | PyBroP | 406.2 |
| **478** | 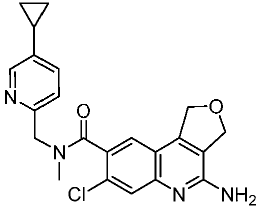 | 4-amino-7-chloro-N-((5-cyclopropyl-2-pyridinyl)methyl)-N-methyl-1,3-dihydrofuro[3,4-c]quinoline-8-carboxamide | PyBroP | 409.1 |
| **479** | 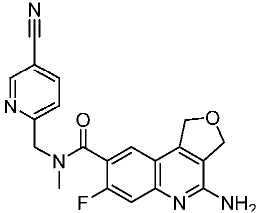 | 4-amino-N-((5-cyano-2-pyridinyl)methyl)-7-fluoro-N-methyl-1,3-dihydrofuro[3,4-c]quinoline-8-carboxamide | PyBroP | 378.1 |
| **480** | 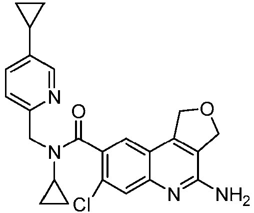 | 4-amino-7-chloro-N-cyclopropyl-N-((5-cyclopropyl-2-pyridinyl)methyl)-1,3-dihydrofuro[3,4-c]quinoline-8-carboxamide | PyBroP | 435.2 |
| **481** | 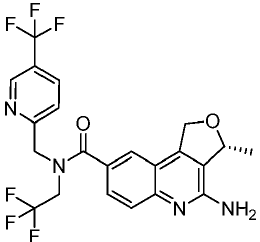 | (3R)-4-amino-3-methyl-N-(2,2,2-trifluoroethyl)-N-((5-(trifluoromethyl)-2-pyridinyl)methyl)-1,3-dihydrofuro[3,4-c]quinoline-8-carboxamide | PyBroP | 485.2 |
| **482** | 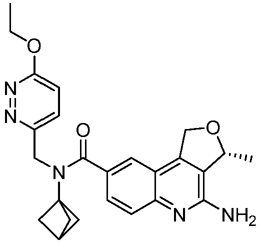 | (3R)-4-amino-N-(bicyclo[1.1.1]pentan-1-yl)-N-((6-ethoxy-3-pyridazinyl)methyl)-3-methyl-1,3-dihydrofuro [3,4-c]quinoline-8-carboxamide | PyBroP | 446.2 |
| **483** | 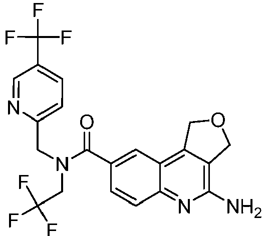 | 4-amino-N-(2,2,2-trifluoroethyl)-N-((5-(trifluoromethyl)-2-pyridinyl)methyl)-1,3-dihydrofuro[3,4-c]quinoline-8-carboxamide | PyBroP | 471.1 |
| **484** | 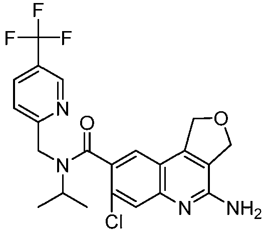 | 4-amino-7-chloro-N-(2-propanyl)-N-((5-(trifluoromethyl)-2-pyridinyl)methyl)-1,3-dihydrofuro[3,4-c]quinoline-8-carboxamide | PyBroP | 465.1 |
| **485** | 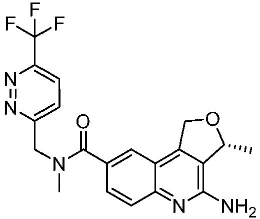 | (3R)-4-amino-N,3-dimethyl-N-((6-(trifluoromethyl)-3-pyridazinyl)methyl)-1,3-dihydrofuro[3,4-c]quinoline-8-carboxamide | PyBroP | 418.1 |
| **486** | 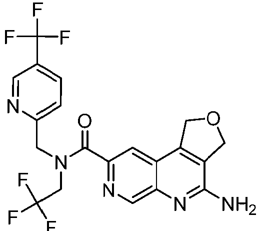 | 4-amino-N-(2,2,2-trifluoroethyl)-N-((5-(trifluoromethyl)-2-pyridinyl)methyl)-1,3-dihydrofuro[3,4-c][1,7]naphthyridine-8-carboxamide | PyBroP | 472.1 |
| **487** | 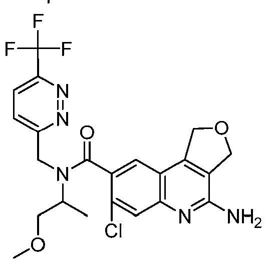 | 4-amino-7-chloro-N-((2R)-1-methoxy-2-propanyl)-N-((6-(trifluoromethyl)-3-pyridazinyl)methyl)-1,3-dihydrofuro[3,4-c]quinoline-8-carboxamide and 4-amino-7-chloro-N-((2S)-1-methoxy-2-propanyl)-N-((6-(trifluoromethyl)-3-pyridazinyl)methyl)-1,3-dihydrofuro[3,4-c]quinoline-8-carboxamide | PyBroP | 496.1 |
| **488** | 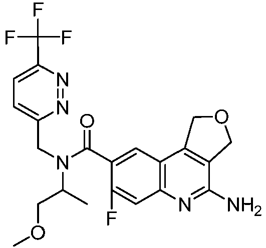 | 4-amino-7-fluoro-N-((2R)-1-methoxy-2-propanyl)-N-((6-(trifluoromethyl)-3-pyridazinyl)methyl)-1,3-dihydrofuro[3,4-c]quinoline-8-carboxamide and 4-amino-7-fluoro-N-((2S)-1-methoxy-2-propanyl)-N-((6-(trifluoromethyl)-3-pyridazinyl)methyl)-1,3-dihydrofuro[3,4-c]quinoline-8-carboxamide | PyBroP | 480.2 |
| **489** | 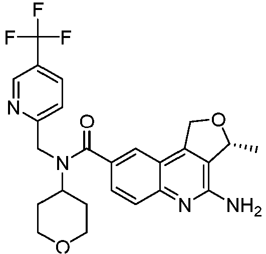 | (3R)-4-amino-3-methyl-N-(tetrahydro-2H-pyran-4-yl)-N-((5-(trifluoromethyl)-2-pyridinyl)methyl)-1,3-dihydrofuro[3,4-c]quinoline-8-carboxamide | PyBroP | 487.2 |
| **490** | 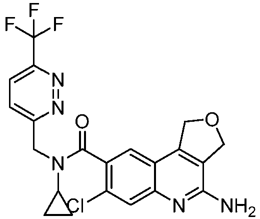 | 4-amino-7-chloro-N-cyclopropyl-N-((6-(trifluoromethyl)-3-pyridazinyl)methyl)-1,3-dihydrofuro[3,4-c]quinoline-8-carboxamide | PyBroP | 464.1 |
| **492** | 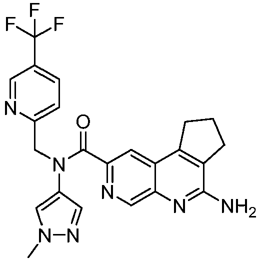 | 6-amino-N-(1-methyl-1H-pyrazol-4-yl)-N-((5-(trifluoromethyl)-2-pyridinyl)methyl)-8,9-dihydro-7H-cyclopenta[c][1,7]naphthyridine-2-carboxamide | PyBroP | 468.2 |
| **493** | 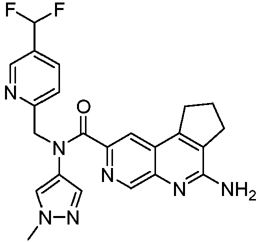 | 6-amino-N-((5-(difluoromethyl)-2-pyridinyl)methyl)-N-(1-methyl-1H-pyrazol-4-yl)-8,9-dihydro-7H-cyclopenta[c][1,7]naphthyridine-2-carboxamide | PyBroP | 450.2 |
| **494** | 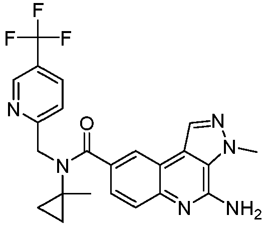 | 4-amino-3-methyl-N-(1-methylcyclopropyl)-N-((5-(trifluoromethyl)-2-pyridinyl)methyl)-3H-pyrazolo[3,4-c]quinoline-8-carboxamide | PyBroP | 455.2 |
| **495** | 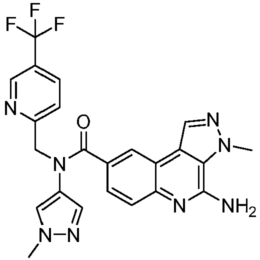 | 4-amino-3-methyl-N-(1-methyl-1H-pyrazol-4-yl)-N-((5-(trifluoromethyl)-2-pyridinyl)methyl)-3H-pyrazolo[3,4-c]quinoline-8-carboxamide | PyBroP | 481.2 |
| **496** | 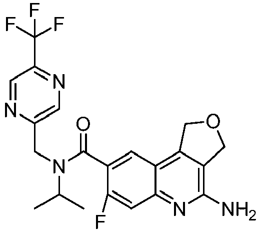 | 4-amino-7-fluoro-N-(2-propanyl)-N-((5-(trifluoromethyl)-2-pyrazinyl)methyl)-1,3-dihydrofuro[3,4-c]quinoline-8-carboxamide | PyBroP | 450.2 |
| **497** | 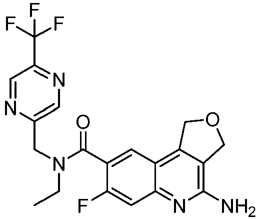 | 4-amino-N-ethyl-7-fluoro-N-((5-(trifluoromethyl)-2-pyrazinyl)methyl)-1,3-dihydrofuro[3,4-c]quinoline-8-carboxamide | PyBroP | 436.15 |
| **498** | 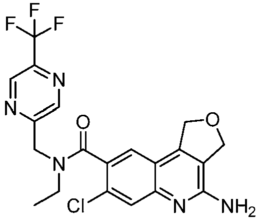 | 4-amino-7-chloro-N-ethyl-N-((5-(trifluoromethyl)-2-pyrazinyl)methyl)-1,3-dihydrofuro[3,4-c]quinoline-8-carboxamide | PyBroP | 452.05 |
| **499** | 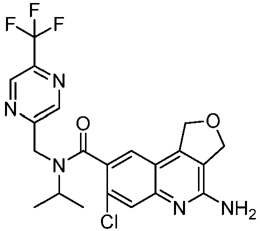 | 4-amino-7-chloro-N-(2-propanyl)-N-((5-(trifluoromethyl)-2-pyrazinyl)methyl)-1,3-dihydrofuro[3,4-c]quinoline-8-carboxamide | PyBroP | 466.2 |
| **500** | 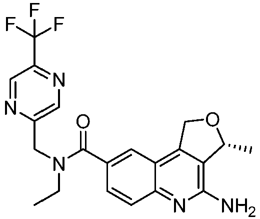 | (3R)-4-amino-N-ethyl-3-methyl-N-((5-(trifluoromethyl)-2-pyrazinyl)methyl)-1,3-dihydrofuro[3,4-c]quinoline-8-carboxamide | PyBroP | 432.2 |
| **501** | 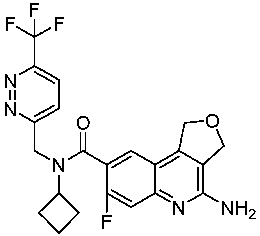 | 4-amino-N-cyclobutyl-7-fluoro-N-((6-(trifluoromethyl)-3-pyridazinyl)methyl)-1,3-dihydrofuro[3,4-c]quinoline-8-carboxamide | PyBroP | 462.2 |
| **502** | 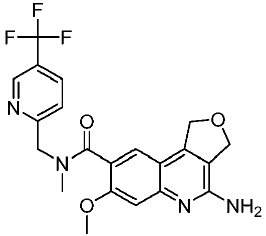 | 4-amino-7-methoxy-N-methyl-N-((5-(trifluoromethyl)-2-pyridinyl)methyl)-1,3-dihydrofuro[3,4-c]quinoline-8-carboxamide | PyBroP | 433.2 |
| **503** | 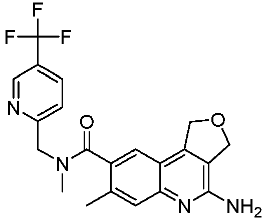 | 4-amino-N,7-dimethyl-N-((5-(trifluoromethyl)-2-pyridinyl)methyl)-1,3-dihydrofuro[3,4-c]quinoline-8-carboxamide | PyBroP | 417 |
| **504** | 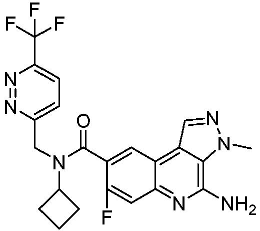 | 4-amino-N-cyclobutyl-7-fluoro-3-methyl-N-((6-(trifluoromethyl)-3-pyridazinyl)methyl)-3H-pyrazolo[3,4-c]quinoline-8-carboxamide | PyBroP | 474.2 |
| **505** | 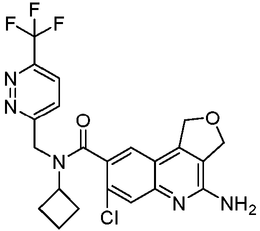 | 4-amino-7-chloro-N-cyclobutyl-N-((6-(trifluoromethyl)-3-pyridazinyl)methyl)-1,3-dihydrofuro[3,4-c]quinoline-8-carboxamide | PyBroP | 478.2 |
| **506** | 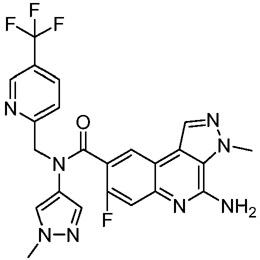 | 4-amino-7-fluoro-3-methyl-N-(1-methyl-1H-pyrazol-4-yl)-N-((5-(trifluoromethyl)-2-pyridinyl)methyl)-3H-pyrazolo[3,4-c]quinoline-8-carboxamide | PyBroP | 499.2 |
| **507** | 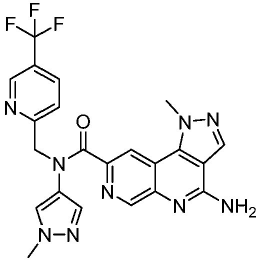 | 4-amino-1-methyl-N-(1-methyl-1H-pyrazol-4-yl)-N-((5-(trifluoromethyl)-2-pyridinyl)methyl)-1H-pyrazolo[4,3-c][1,7]naphthyridine-8-carboxamide | PyBroP | 481.9 |
| **508** | 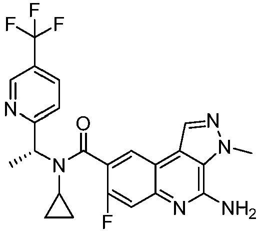 | 4-amino-N-cyclopropyl-7-fluoro-3-methyl-N-((1R)-1-(5-(trifluoromethyl)-2-pyridinyl)ethyl)-3H-pyrazolo[3,4-c]quinoline-8-carboxamide | PyBroP | 473 |
| **509** | 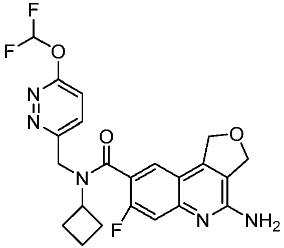 | 4-amino-N-cyclobutyl-N-((6-(difluoromethoxy)-3-pyridazinyl)methyl)-7-fluoro-1,3-dihydrofuro[3,4-c]quinoline-8-carboxamide | TBTU | 460.15 |
| **510** | 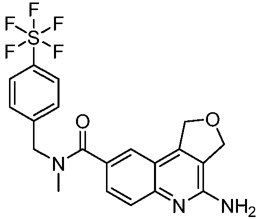 | 4-amino-N-methyl-N-(4-(pentafluoro-lambda~6~-sulfanyl)benzyl)-1,3-dihydrofuro[3,4-c]quinoline-8-carboxamide | TBTU | 460.9 |
| **511** | 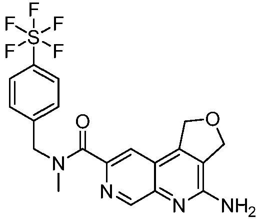 | 4-amino-N-methyl-N-(4-(pentafluoro-lambda~6~-sulfanyl)benzyl)-1,3-dihydrofuro[3,4-c] [1,7]naphthyridine-8-carboxamide | TBTU | 461.8 |
| **512** | 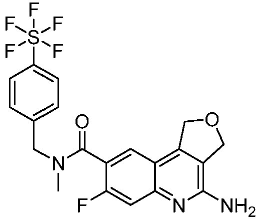 | 4-amino-7-fluoro-N-methyl-N-(4-(pentafluoro-lambda~6~-sulfanyl)benzyl)-1,3-dihydrofuro[3,4-c]quinoline-8-carboxamide | TBTU | 478.7 |
| **513** | 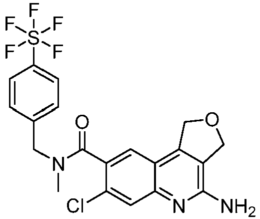 | 4-amino-7-chloro-N-methyl-N-(4-(pentafluoro-lambda~6~-sulfanyl)benzyl)-1,3-dihydrofuro[3,4-c]quinoline-8-carboxamide | TBTU | 494.8 |
| **514** | 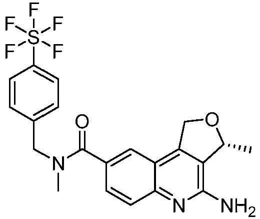 | (3R)-4-amino-N,3-dimethyl-N-(4-(pentafluoro-lambda~6~-sulfanyl)benzyl)-1,3-dihydrofuro[3,4-c]quinoline-8-carboxamide | TBTU | 474.8 |
| **515** | 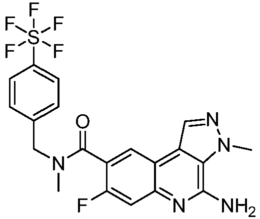 | 4-amino-7-fluoro-N,3-dimethyl-N-(4-(pentafluoro-lambda~6~-sulfanyl)benzyl)-3H-pyrazolo[3,4-c]quinoline-8-carboxamide | TBTU | 490.7 |
| **516** | 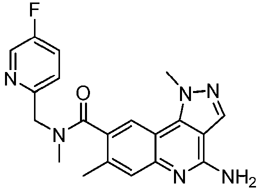 | 4-amino-N-((5-fluoro-2-pyridinyl)methyl)-N,1,7-trimethyl-1H-pyrazolo[4,3-c]quinoline-8-carboxamide | TBTU | 379.2 |
| **517** | 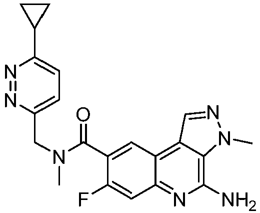 | 4-amino-N-((6-cyclopropyl-3-pyridazinyl)methyl)-7-fluoro-N,3-dimethyl-3H-pyrazolo[3,4-c]quinoline-8-carboxamide | TBTU | 406.2 |
| **518** | 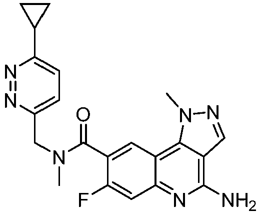 | 4-amino-N-((6-cyclopropyl-3-pyridazinyl)methyl)-7-fluoro-N,1-dimethyl-1H-pyrazolo[4,3-c]quinoline-8-carboxamide | TBTU | 406.2 |
| **519** | 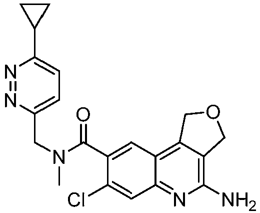 | 4-amino-7-chloro-N-((6-cyclopropyl-3-pyridazinyl)methyl)-N-methyl-1,3-dihydrofuro[3,4-c]quinoline-8-carboxamide | TBTU | 410.2 |
| **520** | 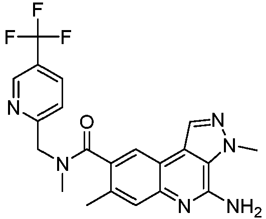 | 4-amino-N,3,7-trimethyl-N-((5-(trifluoromethyl)-2-pyridinyl)methyl)-3H-pyrazolo[3,4-c]quinoline-8-carboxamide | TBTU | 429.2 |
| **521** | 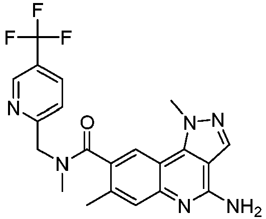 | 4-amino-N,1,7-trimethyl-N-((5-(trifluoromethyl)-2-pyridinyl)methyl)-1H-pyrazolo[4,3-c]quinoline-8-carboxamide | TBTU | 429.2 |
| **522** | 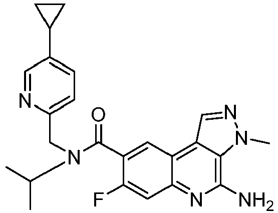 | 4-amino-N-((5-cyclopropyl-2-pyridinyl)methyl)-7-fluoro-3-methyl-N-(2-propanyl)-3H-pyrazolo[3,4-c]quinoline-8-carboxamide | TBTU | 433.0 |
| **523** | 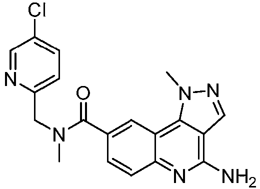 | 4-amino-N-((5-chloro-2-pyridinyl)methyl)-N,1-dimethyl-1H-pyrazolo[4,3-c]quinoline-8-carboxamide | TBTU | 380.9 |
| **524** | 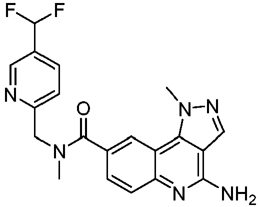 | 4-amino-N-((5-(difluoromethyl)-2-pyridinyl)methyl)-N,1-dimethyl-1H-pyrazolo[4,3-c]quinoline-8-carboxamide | TBTU | 397.0 |
| **525** | 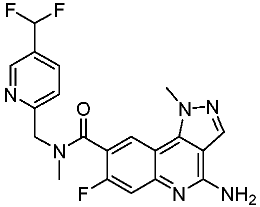 | 4-amino-N-((5-(difluoromethyl)-2-pyridinyl)methyl)-7-fluoro-N,1-dimethyl-1H-pyrazolo[4,3-c]quinoline-8-carboxamide | TBTU | 415.9 |
| **526** | 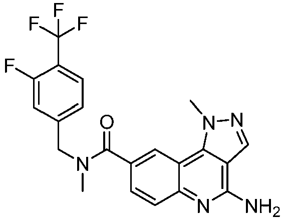 | 4-amino-N-(3-fluoro-4-(trifluoromethyl)benzyl)-N,1-dimethyl-1H-pyrazolo[4,3-c]quinoline-8-carboxamide | TBTU | 432.1 |
| **527** | 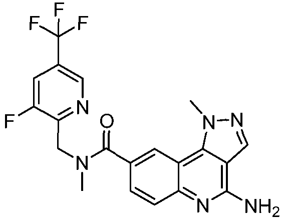 | 4-amino-N-((3-fluoro-5-(trifluoromethyl)-2-pyridinyl)methyl)-N,1-dimethyl-1H-pyrazolo[4,3-c]quinoline-8-carboxamide | TBTU | 433.1 |
| **528** | 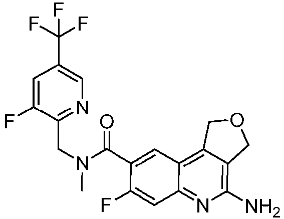 | 4-amino-7-fluoro-N-((3-fluoro-5-(trifluoromethyl)-2-pyridinyl)methyl)-N-methyl-1,3-dihydrofuro[3,4-c]quinoline-8-carboxamide | TBTU | 439.1 |
| **529** | 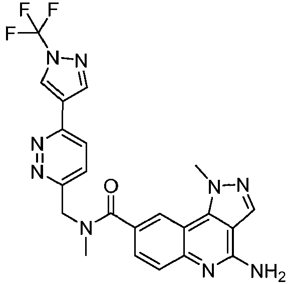 | 4-amino-N,1-dimethyl-N-((6-(1-(trifluoromethyl)-1H-pyrazol-4-yl)-3-pyridazinyl)methyl)-1H-pyrazolo[4,3-c]quinoline-8-carboxamide | TBTU | 482.2 |
| **530** | 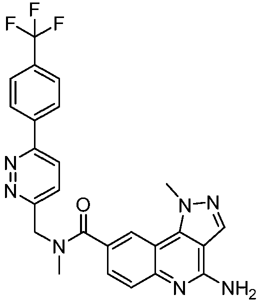 | 4-amino-N,1-dimethyl-N-((6-(4-(trifluoromethyl)phenyl)-3-pyridazinyl)methyl)-1H-pyrazolo[4,3-c]quinoline-8-carboxamide | TBTU | 492.1 |
| **531** | 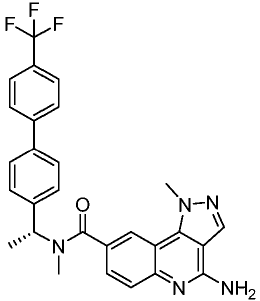 | 4-amino-N,1-dimethyl-N-((1R)-1-(4'-(trifluoromethyl) [biphenyl]-4-yl)ethyl)-1H-pyrazolo[4,3-c]quinoline-8-carboxamide | TBTU | 504.2 |
| **532** | 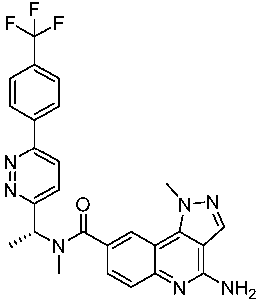 | 4-amino-N,1-dimethyl-N-((1R)-1-(6-(4-(trifluoromethyl)phenyl)-3-pyridazinyl)ethyl)-1H-pyrazolo[4,3-c]quinoline-8-carboxamide | TBTU | 506.2 |
| **533** | 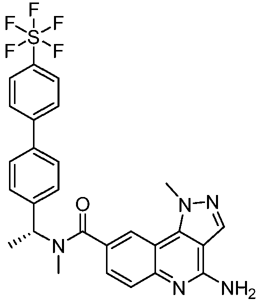 | 4-amino-N,1-dimethyl-N-((1R)-1-(4'-(pentafluoro-lambda~6~-sulfanyl)[biphenyl]-4-yl)ethyl)-1H-pyrazolo[4,3-c]quinoline-8-carboxamide | TBTU | 563.2 |
| **534** | 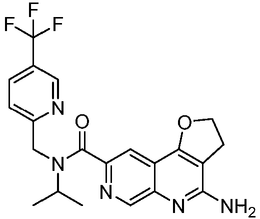 | 4-amino-N-(2-propanyl)-N-((5-(trifluoromethyl)-2-pyridinyl)methyl)-2,3-dihydrofuro[3,2-c][1,7]naphthyridine-8-carboxamide | HATU | 432.1 |
| **535** | 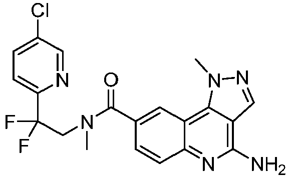 | 4-amino-N-(2-(5-chloro-2-pyridinyl)-2,2-difluoroethyl)-N,1-dimethyl-1H-pyrazolo[4,3-c]quinoline-8-carboxamide | HATU | 431.0 |
| **536** | 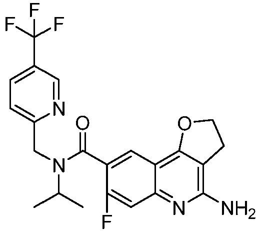 | 4-amino-7-fluoro-N-(2-propanyl)-N-((5-(trifluoromethyl)-2-pyridinyl)methyl)-2,3-dihydrofuro[3,2-c]quinoline-8-carboxamide | HATU | 449.0 |
| **537** | 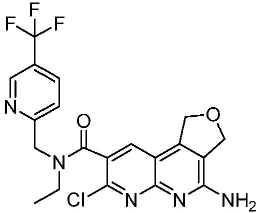 | 4-amino-7-chloro-N-ethyl-N-((5-(trifluoromethyl)-2-pyridinyl)methyl)-1,3-dihydrofuro[3,4-c][1,8]naphthyridine-8-carboxamide | HATU | 451.9 |
| **538** | 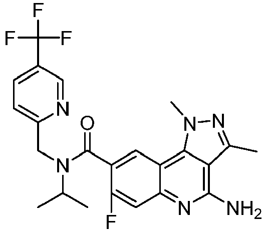 | 4-amino-7-fluoro-1,3-dimethyl-N-(2-propanyl)-N-((5-(trifluoromethyl)-2-pyridinyl)methyl)-1H-pyrazolo[4,3-c]quinoline-8-carboxamide | HATU | 475.1 |
| **539** | 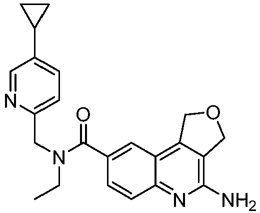 | 4-amino-N-((5-cyclopropyl-2-pyridinyl)methyl)-N-ethyl-1,3-dihydrofuro[3,4-c]quinoline-8-carboxamide | HATU | 389.0 |
| **540** | 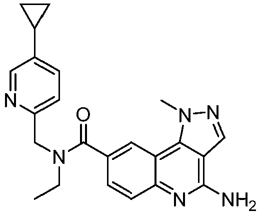 | 4-amino-N-((5-cyclopropyl-2-pyridinyl)methyl)-N-ethyl-1-methyl-1H-pyrazolo[4,3-c]quinoline-8-carboxamide | HATU | 401.0 |
| **541** | 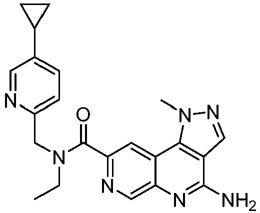 | 4-amino-N-((5-cyclopropyl-2-pyridinyl)methyl)-N-ethyl-1-methyl-1H-pyrazolo[4,3-c][1,7]naphthyridine-8-carboxamide | HATU | 402.0 |
| **542** | 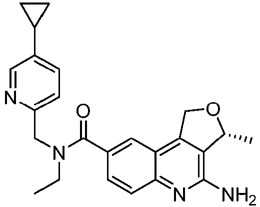 | (3R)-4-amino-N-((5-cyclopropyl-2-pyridinyl)methyl)-N-ethyl-3-methyl-1,3-dihydrofuro[3,4-c]quinoline-8-carboxamide | HATU | 403.0 |
| **543** | 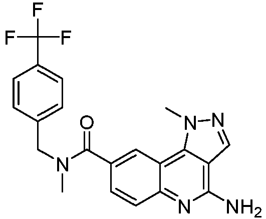 | 4-amino-N,1-dimethyl-N-(4-(trifluoromethyl)benzyl)-1H-pyrazolo[4,3-c]quinoline-8-carboxamide | HATU | 414.0 |
| **544** | 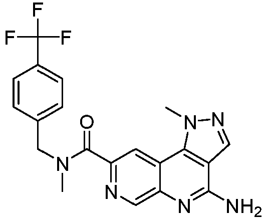 | 4-amino-N,1-dimethyl-N-(4-(trifluoromethyl)benzyl)-1H-pyrazolo[4,3-c][1,7]naphthyridine-8-carboxamide | HATU | 415.0 |
| **545** | 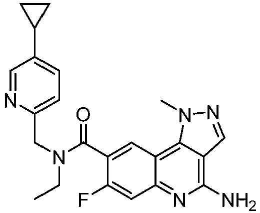 | 4-amino-N-((5-cyclopropyl-2-pyridinyl)methyl)-N-ethyl-7-fluoro-1-methyl-1H-pyrazolo[4,3-c]quinoline-8-carboxamide | HATU | 419.0 |
| **546** | 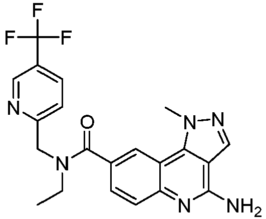 | 4-amino-N-ethyl-1-methyl-N-((5-(trifluoromethyl)-2-pyridinyl)methyl)-1H-pyrazolo[4,3-c]quinoline-8-carboxamide | HATU | 429.0 |
| **547** | 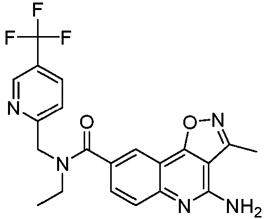 | 4-amino-N-ethyl-3-methyl-N-((5-(trifluoromethyl)-2-pyridinyl)methyl)[1,2]oxazolo[4, 5-c]quinoline-8-carboxamide | HATU | 430.0 |
| **548** | 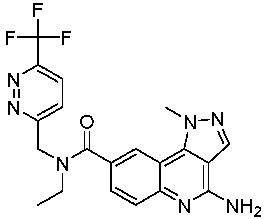 | 4-amino-N-ethyl-1-methyl-N-((6-(trifluoromethyl)-3-pyridazinyl)methyl)-1H-pyrazolo[4,3-c]quinoline-8-carboxamide | HATU | 430.0 |
| **549** | 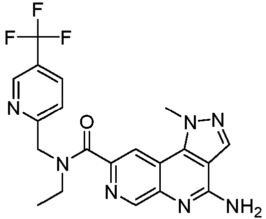 | 4-amino-N-ethyl-1-methyl-N-((5-(trifluoromethyl)-2-pyridinyl)methyl)-1H-pyrazolo[4,3-c][1,7]naphthyridine-8-carboxamide | HATU | 430.0 |
| **550** | 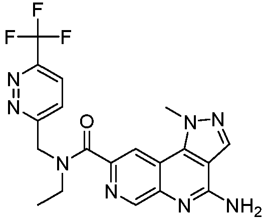 | 4-amino-N-ethyl-1-methyl-N-((6-(trifluoromethyl)-3-pyridazinyl)methyl)-1H-pyrazolo[4,3-c][1,7]naphthyridine-8-carboxamide | HATU | 431.0 |
| **551** | 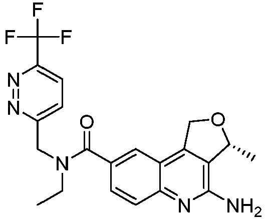 | (3R)-4-amino-N-ethyl-3-methyl-N-((6-(trifluoromethyl)-3-pyridazinyl)methyl)-1,3-dihydrofuro[3,4-c]quinoline-8-carboxamide | HATU | 432.0 |
| **552** | 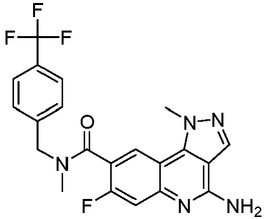 | 4-amino-7-fluoro-N,1-dimethyl-N-(4-(trifluoromethyl)benzyl)-1H-pyrazolo[4,3-c] quinoline-8-carboxamide | HATU | 432.0 |
| **553** | 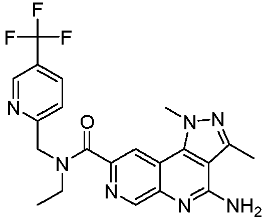 | 4-amino-N-ethyl-1,3-dimethyl-N-((5-(trifluoromethyl)-2-pyridinyl)methyl)-1H-pyrazolo[4,3-c][1,7]naphthyridine-8-carboxamide | HATU | 444.0 |
| **554** | 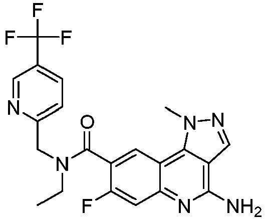 | 4-amino-N-ethyl-7-fluoro-1-methyl-N-((5-(trifluoromethyl)-2-pyridinyl)methyl)-1H-pyrazolo[4,3-c]quinoline-8-carboxamide | HATU | 447.0 |
| **555** | 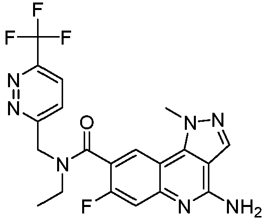 | 4-amino-N-ethyl-7-fluoro-1-methyl-N-((6-(trifluoromethyl)-3-pyridazinyl)methyl)-1H-pyrazolo[4,3-c]quinoline-8-carboxamide | HATU | 448.0 |
| **556** | 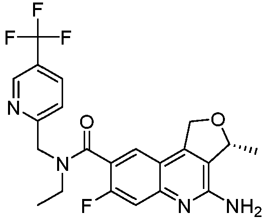 | (3R)-4-amino-N-ethyl-7-fluoro-3-methyl-N-((5-(trifluoromethyl)-2-pyridinyl)methyl)-1,3-dihydrofuro[3,4-c]quinoline-8-carboxamide | HATU | 449.0 |
| **557** | 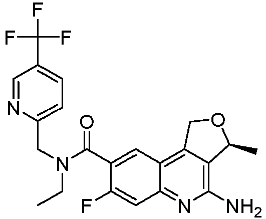 | (3S)-4-amino-N-ethyl-7-fluoro-3-methyl-N-((5-(trifluoromethyl)-2-pyridinyl)methyl)-1,3-dihydrofuro[3,4-c]quinoline-8-carboxamide | HATU | 449.0 |
| **558** | 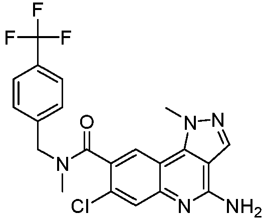 | 4-amino-7-chloro-N,1-dimethyl-N-(4-(trifluoromethyl)benzyl)-1H-pyrazolo [4,3-c] quinoline-8-carboxamide | HATU | 448.0 |
| **559** | 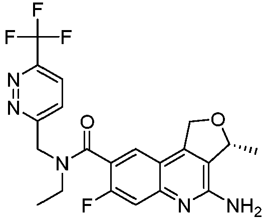 | (3R)-4-amino-N-ethyl-7-fluoro-3-methyl-N-((6-(trifluoromethyl)-3-pyridazinyl)methyl)-1,3-dihydrofuro[3,4-c]quinoline-8-carboxamide | HATU | 450.1 |
| **560** | 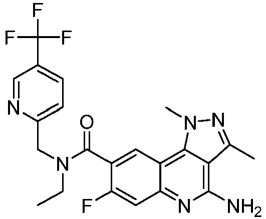 | 4-amino-N-ethyl-7-fluoro-1,3-dimethyl-N-((5-(trifluoromethyl)-2-pyridinyl)methyl)-1H-pyrazolo[4,3-c]quinoline-8-carboxamide | HATU | 461.0 |
| **561** | 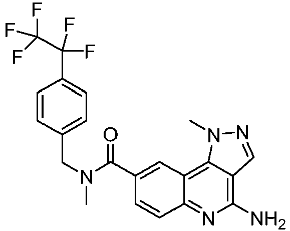 | 4-amino-N,1-dimethyl-N-(4-(pentafluoroethyl)benzyl)-1H-pyrazolo[4,3-c]quinoline-8-carboxamide | HATU | 464.0 |
| **562** | 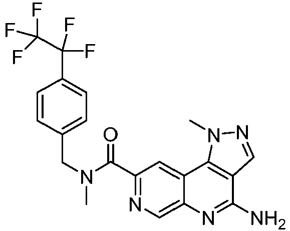 | 4-amino-N,1-dimethyl-N-(4-(pentafluoroethyl)benzyl)-1H-pyrazolo[4,3-c][1,7]naphthyridine-8-carboxamide | HATU | 465.0 |
| **563** | 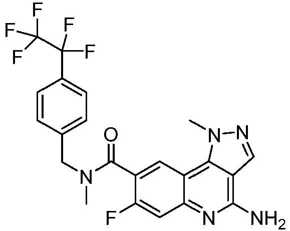 | 4-amino-7-fluoro-N,1-dimethyl-N-(4-(pentafluoroethyl)benzyl)-1H-pyrazolo[4,3-c]quinoline-8-carboxamide | HATU | 482.0 |
| **564** | 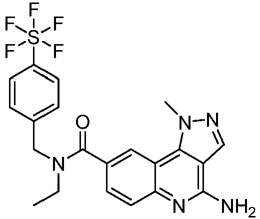 | 4-amino-N-ethyl-1-methyl-N-(4-(pentafluoro-lambda~6~-sulfanyl)benzyl)-1H-pyrazolo[4,3-c]quinoline-8-carboxamide | HATU | 486.0 |
| **565** | 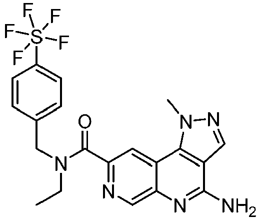 | 4-amino-N-ethyl-1-methyl-N-(4-(pentafluoro-lambda~6~-sulfanyl)benzyl)-1H-pyrazolo[4,3-c][1,7]naphthyridine-8-carboxamide | HATU | 487.0 |
| **566** | 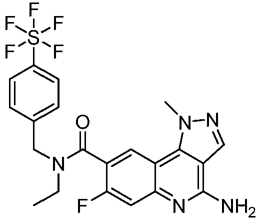 | 4-amino-N-ethyl-7-fluoro-1-methyl-N-(4-(pentafluoro-lambda~6~-sulfanyl)benzyl)-1H-pyrazolo[4,3-c]quinoline-8-carboxamide | HATU | 504.0 |
| **567** | 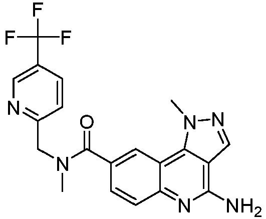 | 4-amino-N,1-dimethyl-N-((5-(trifluoromethyl)-2-pyridinyl)methyl)-1H-pyrazolo[4,3-c]quinoline-8-carboxamide | TBTU | 415.0 |
| **568** | 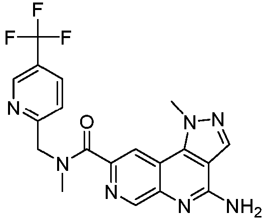 | 4-amino-N,1-dimethyl-N-((5-(trifluoromethyl)-2-pyridinyl)methyl)-1H-pyrazolo[4,3-c][1,7]naphthyridine-8-carboxamide | TBTU | 415.9 |
| **569** | 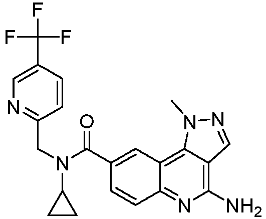 | 4-amino-N-cyclopropyl-1-methyl-N-((5-(trifluoromethyl)-2-pyridinyl)methyl)-1H-pyrazolo[4,3-c]quinoline-8-carboxamide | TBTU | 441.2 |
| **570** | 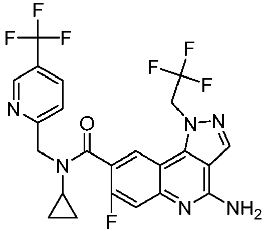 | 4-amino-N-cyclopropyl-7-fluoro-1-(2,2,2-trifluoroethyl)-N-((5-(trifluoromethyl)-2-pyridinyl)methyl)-1H-pyrazolo[4,3-c]quinoline-8-carboxamide | TBTU | 527.2 |
| **571** | 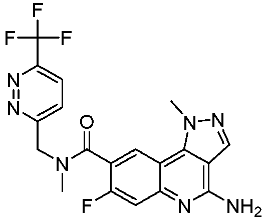 | 4-amino-7-fluoro-N,1-dimethyl-N-((6-(trifluoromethyl)-3-pyridazinyl)methyl)-1H-pyrazolo[4,3-c]quinoline-8-carboxamide | TBTU | 433.9 |
| **572** | 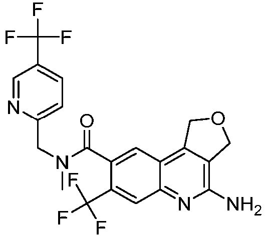 | 4-amino-N-methyl-7-(trifluoromethyl)-N-((5-(trifluoromethyl)-2-pyridinyl)methyl)-1,3-dihydrofuro[3,4-c]quinoline-8-carboxamide | TBTU | 471.1 |
| **573** | 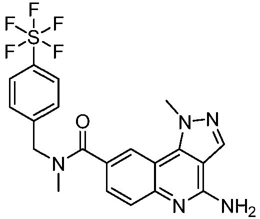 | 4-amino-N,1-dimethyl-N-(4-(pentafluoro-lambda~6~-sulfanyl)benzyl)-1H-pyrazolo[4,3-c]quinoline-8-carboxamide | TBTU | 472.9 |
| **574** | 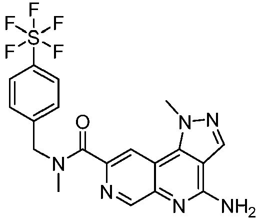 | 4-amino-N,1-dimethyl-N-(4-(pentafluoro-lambda~6~-sulfanyl)benzyl)-1H-pyrazolo[4,3-c][1,7]naphthyridine-8-carboxamide | TBTU | 473.2 |
| **575** | 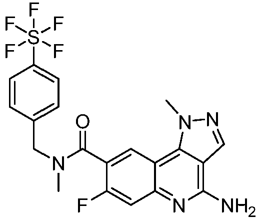 | 4-amino-7-fluoro-N,1-dimethyl-N-(4-(pentafluoro-lambda~6~-sulfanyl)benzyl)-1H-pyrazolo[4,3-c]quinoline-8-carboxamide | TBTU | 490.8 |
| **576** | 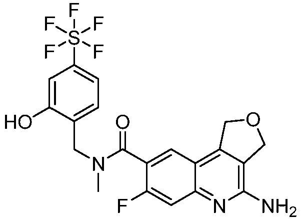 | 4-amino-7-fluoro-N-(2-hydroxy-4-(pentafluoro-lambda~6~-sulfanyl)benzyl)-N-methyl-1,3-dihydrofuro[3,4-c]quinoline-8-carboxamide | TBTU | 494.8 |

### Example 577 and 578: 4-amino-N-((5-cyanopyridin-2-yl)methyl)-N-(1-fluoropropan-2-yl)-1,3-dihydrofuro[3,4-c]quinoline-8-carboxamide

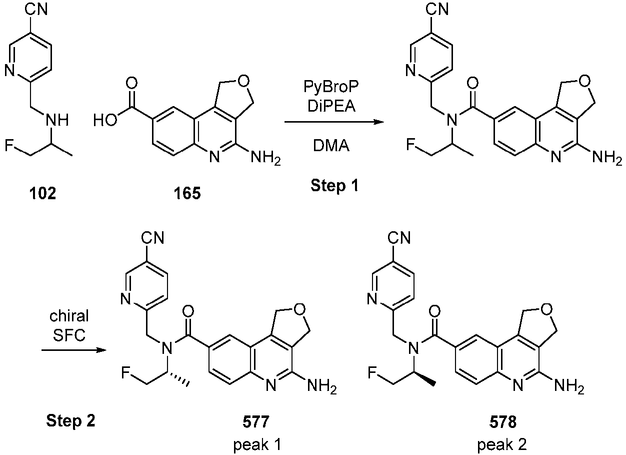

Step 1. To a stirred mixture of 4-amino-1,3-dihydrofuro[3,4-c]quinoline-8-carboxylic acid **(215,** 131 mg, 0.569 mmol), 6-(((1-fluoropropan-2-yl)amino)methyl)nicotinonitrile **(102,** 100 mg, 0.518 mmol) and bromotri(pyrrolidin-1-yl)phosphonium hexafluorophosphate(V) (483 mg, 1.035 mmol, Aldrich) in DMAC (1.5 mL) was added at rt N-ethyl-N-isopropylpropan-2-amine (201 mg, 0.271 mL, 1.553 mmol, Aldrich). The resulting mixture was briefly sonicated and the stirred at rt for 1 h. The crude mixture was directly loaded onto a silica gel precolumn (25 g) and subjected to combi-flash column chromatography on a 12-g ISCO gold column eluting with MeOH/DCM (15 min from 0% to 18%) (2 X) to give two portions of the desired product. The less pure portion was dissolved in DMSO/methanol/TFA and subjected to preparative reverse-phase HPLC (Gemini^{™} Prep C18 10 µm column; Phenomenex; gradient elution of 10 to 75% MeCN in water, where both solvents contain 0.1% TFA 15 min in a 24-min method) to give 140 mg of 4-amino-N-((5-cyanopyridin-2-yl)methyl)-N-(1-fluoropropan-2-yl)-1,3-dihydrofuro[3,4-c]quinoline-8-carboxamide 2,2,2-trifluoroacetate as a white solid. *m*/*z* (ESI): 406.2 (M+H)⁺. ¹H NMR (METHANOL-d4, 400 MHz) δ 8.89 (s, 1H), 8.0-8.3 (m, 1H), 7.4-7.9 (m, 4H), 6.1-6.1 (m, 1H), 5.4-5.6 (m, 2H), 5.1-5.3 (m, 2H), 4.97 (s, 2H), 4.2-4.6 (m, 3H), 1.1-1.5 (m, 3H). ¹⁹F NMR (METHANOL-d4, 376 MHz) δ -221.55 (br s, 1F).

Step 2. The racemate was purified via preparative SFC using a Chiral Technologies OJ column (250 X 21 mm, 5mm) with a mobile phase of 75% Liquid CO₂ and 25% MeOH with 0.2% TEA using a flowrate of 80 mL/min. The more potent (measured by IC₅₀ in HCT116 MTAP null cell viability assay) enantiomer was assigned as the (R)-; the less potent (measured by IC₅₀ in HCT116 MTAP null cell viability assay) enantiomer was assigned as (S)-. The 1^{st} eluting peak was (R)-4-amino-N-((5-cyanopyridin-2-yl)methyl)-N-(1-fluoropropan-2-yl)-1,3-dihydrofuro[3,4-c]quinoline-8-carboxamide **(577,** 54.0 mg, 0.133 mmol, 25.7 % yield). The 2^{nd} eluting peak was (S)-4-amino-N-((5-cyanopyridin-2-yl)methyl)-N-(1-fluoropropan-2-yl)-1,3-dihydrofuro[3,4-c]quinoline-8-carboxamide **(578,** 56.1 mg, 0.138 mmol, 26.7% yield). *m*/*z* (ESI): 406.2 (M+H)⁺. ¹H NMR (DMSO-d6, 500 MHz) δ 8.99 (d, 1H, J=1.8 Hz), 8.26 (br s, 1H), 7.56 (br s, 4H), 6.67 (br s, 2H), 5.34 (br s, 2H), 5.01 (br s, 2H), 4.7-4.9 (m, 2H), 4.2-4.7 (m, 3H), 3.1-3.3 (m, 1H), 1.15 (br s, 3H).

Compounds in Table 19 were prepared in a manner similar to that described for **577** and **578** utilizing the indicated coupling agent.

**Table 19**

| **Ex.** | **Structure** | **Name** | **m/z (ESI): (M+H)⁺** | **Coupling Reagent** | **SFC** |
|---|---|---|---|---|---|
| **579** | 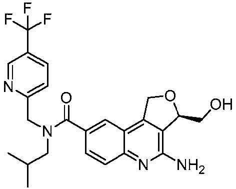 | (3R)-4-amino-3-(hydroxymethyl)-N-(2-methylpropyl)-N-((5-(trifluoromethyl)-2-pyridinyl)methyl)-1,3-dihydrofuro[3,4-c] quinoline-8-carboxamide | 475.2 | HATU | 1st peak, Chiralpak IA column (250 x 21 mm, 5 µm) with a mobile phase of 70% Liquid CO₂ and 30% MeOH with 0.2% DEA using a flowrate of 80 mL/min |
| **580** | 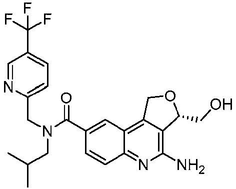 | (3S)-4-amino-3-(hydroxymethyl)-N-(2-methylpropyl)-N-((5-(trifluoromethyl)-2-pyridinyl)methyl)-1,3-dihydrofuro[3,4-c] quinoline-8-carboxamide | 475.2 | HATU | 2nd peak, Chiralpak IA column (250 x 21 mm, 5 µm) with a mobile phase of 70% Liquid CO₂ and 30% MeOH with 0.2% DEA using a flowrate of 80 mL/min |
| **581** | 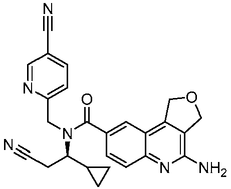 | 4-amino-N-((1R)-2-cyano-1-cyclopropylethyl)-N-((5-cyano-2-pyridinyl)methyl)-1,3-dihydrofuro[3,4-c] quinoline-8-carboxamide | 439.1 | PyBroP | 1st peak, Chiral Tech AS Column (250x21 mm, 5mm) with a mobile phase of 85% liquid CO₂ & 15% MeOH with 0.2% TEA using a flowrate of 80 mL/min |
| **582** | 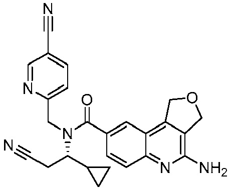 | 4-amino-N-((1S)-2-cyano-1-cyclopropylethyl)-N-((5-cyano-2-pyridinyl)methyl)-1,3-dihydrofuro[3,4-c] quinoline-8-carboxamide | 439.1 | PyBroP | 2nd peak, Chiral Tech AS Column (250x21 mm, 5mm) with a mobile phase of 85% liquid CO₂ & 15% MeOH with 0.2% TEA using a flowrate of 80 mL/min |
| **583** | 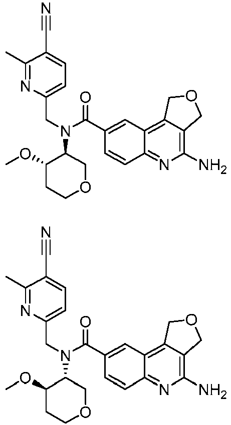 | 4-amino-N-((5-cyano-6-methyl-2-pyridinyl)methyl)-N-((3R,4R)-4-methoxytetrahydro-2H-pyran-3-yl)-1,3-dihydrofuro[3,4-c] quinoline-8-carboxamide and 4-amino-N-((5-cyano-6-methyl-2-pyridinyl)methyl)-N-((3S,4S)-4-methoxytetrahydro-2H-pyran-3-yl)-1,3-dihydrofuro[3,4-c] quinoline-8-carboxamide | 474.2 | PyBroP | 2nd peak, Chiral Technologies AS column (250 X 21 mm, 5mm) with a mobile phase of 80% Liquid CO₂ and 20% MeOH with 0.2% TEA using a flowrate of 80 mL/min |
| **584** | 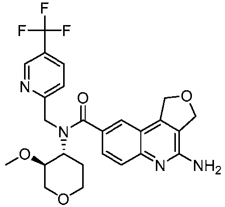 | 4-amino-N-((3S,4R)-3-methoxytetrahydro-2H-pyran-4-yl)-N-((5-(trifluoromethyl)-2-pyridinyl)methyl)-1,3-dihydrofuro[3,4-c] quinoline-8-carboxamide | 503.2 | PyBroP | 1st peak, Chiral Technologies AS column (250 X 21 mm, 5mm) with a mobile phase of 90% Liquid CO₂ and 10% MeOH with 0.2% TEA using a flowrate of 100 mL/min |
| **585** | 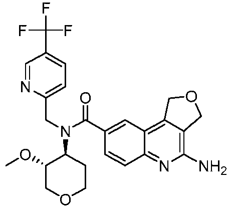 | 4-amino-N-((3R,4S)-3-methoxytetrahydro-2H-pyran-4-yl)-N-((5-(trifluoromethyl)-2-pyridinyl)methyl)-1,3-dihydrofuro[3,4-c] quinoline-8-carboxamide | 503.2 | PyBroP | 2nd peak, Chiral Technologies AS column (250 X 21 mm, 5mm) with a mobile phase of 90% Liquid CO₂ and 10% MeOH with 0.2% TEA using a flowrate of 100 mL/min |
| **586** | 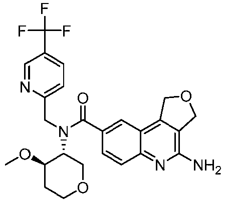 | 4-amino-N-((3R,4R)-4-methoxytetrahydro-2H-pyran-3-yl)-N-((5-(trifluoromethyl)-2-pyridinyl)methyl)-1,3-dihydrofuro[3,4-c] quinoline-8-carboxamide | 503.2 | PyBroP | 1st peak, Chiral Technologies AS column (250 X 21 mm, 5mm) with a mobile phase of 90% Liquid CO₂ and 10% MeOH with 0.2% TEA using a flowrate of 100 mL/min |
| **587** | 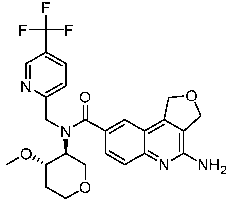 | 4-amino-N-((3S,4S)-4-methoxytetrahydro-2H-pyran-3-yl)-N-((5-(trifluoromethyl)-2-pyridinyl)methyl)-1,3-dihydrofuro[3,4-c] quinoline-8-carboxamide | 503.2 | PyBroP | 2nd peak, Chiral Technologies AS column (250 X 21 mm, 5mm) with a mobile phase of 90% Liquid CO₂ and 10% MeOH with 0.2% TEA using a flowrate of 100 mL/min |
| **588** | 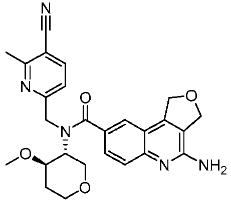 | 4-amino-N-((5-cyano-6-methyl-2-pyridinyl)methyl)-N-((3R,4R)-4-methoxytetrahydro-2H-pyran-3-yl)-1,3-dihydrofuro[3,4-c] quinoline-8-carboxamide | 474.2 | PyBroP | 1st peak, Chiral Technologies AS column (250 X 21 mm, 5mm) with a mobile phase of 80% Liquid CO₂ and 20% MeOH with 0.2% TEA using a flowrate of 80 mL/min |
| **589** | 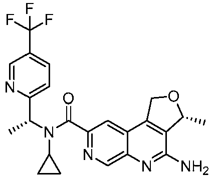 | (3R)-4-amino-N-cyclopropyl-3-methyl-N-((1R)-1-(5-(trifluoromethyl)-2-pyridinyl)ethyl)-1,3-dihydrofuro[3,4-c][1,7]naphthyridine-8-carboxamide | 458 | PyBroP | 1st peak, Chiralpak OD column (250 X 21 mm, 5um) with a mobile phase of 85% Liquid CO₂ and 15% MeOH+TEA using a flowrate of 70 mL/min |
| **590** | 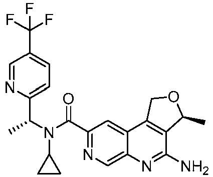 | (3S)-4-amino-N-cyclopropyl-3-methyl-N-((1R)-1-(5-(trifluoromethyl)-2-pyridinyl)ethyl)-1,3-dihydrofuro[3,4-c][1,7]naphthyridine-8-carboxamide | 458 | PyBroP | 2nd peak, Chiralpak OD column (250 X 21 mm, 5um) with a mobile phase of 85% Liquid CO₂ and 15% MeOH+TEA using a flowrate of 70 mL/min |
| **591** | 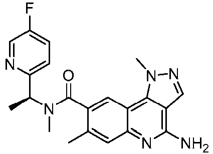 | 4-amino-N-((1S)-1-(5-fluoro-2-pyridinyl)ethyl)-N,1,7-trimethyl-1H-pyrazolo[4,3-c] quinoline-8-carboxamide | 393.2 | TBTU | 2nd peak, Chiralcel OD column (2 x 25 cm, 5 micron) with a mobile phase of 80% Liquid CO₂ and 20% methanol, using a flowrate of 70 mL/min |
| **592** | 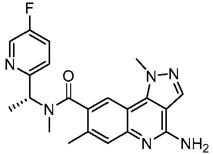 | 4-amino-N-((1R)-1-(5-fluoro-2-pyridinyl)ethyl)-N,1,7-trimethyl-1H-pyrazolo[4,3-c] quinoline-8-carboxamide | 393.2 | TBTU | 1st peak, Chiralcel OD column (2 x 25 cm, 5 micron) with a mobile phase of 80% Liquid CO₂ and 20% methanol, using a flowrate of 70 mL/min |
| **593** | 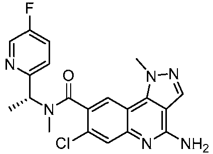 | 4-amino-7-chloro-N-((1R)-1-(5-fluoro-2-pyridinyl)ethyl)-N,1-dimethyl-1H-pyrazolo[4,3-c] quinoline-8-carboxamide | 413.9 | TBTU | 1st peak, Chiralpak AD (3 x 25 cm, 5 micron) with a mobile phase of 65% Liquid CO₂ and 35% isopropanol w/ 0.1% diethylamine, using a flowrate of 70 mL/min |
| **594** | 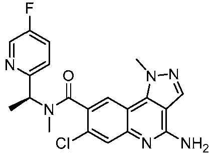 | 4-amino-7-chloro-N-((1S)-1-(5-fluoro-2-pyridinyl)ethyl)-N,1-dimethyl-1H-pyrazolo[4,3-c] quinoline-8-carboxamide | 413.9 | TBTU | 2nd peak, Chiralpak AD (3 x 25 cm, 5 micron) with a mobile phase of 65% Liquid CO₂ and 35% isopropanol w/ 0.1% diethylamine, using a flowrate of 70 mL/min |
| **595** | 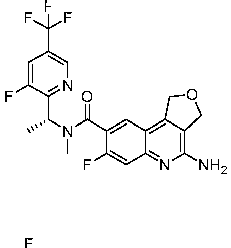 | 4-amino-7-fluoro-N-((1R)-1-(3-fluoro-5-(trifluoromethyl)-2-pyridinyl)ethyl)-N-methyl-1,3-dihydrofuro[3,4-c] quinoline-8-carboxamide | 453.2 | TBTU | 1st peak, Chiralpak AD column (2 x 15 cm, 5 micron) with a mobile phase of 60% Liquid CO₂ and 40% methanol, using a flowrate of 50 mL/min |
| **596** | 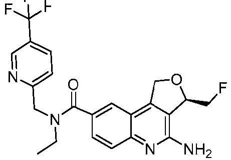 | (3R)-4-amino-N-ethyl-3-(fluoromethyl)-N-((5-(trifluoromethyl)-2-pyridinyl)methyl)-1,3-dihydrofuro[3,4-c] quinoline-8-carboxamide | 449.2 | HATU | 1st peak, Chiralcel OJ column (2 x 25 cm, 5 micron) with a mobile phase of 90% Liquid CO₂ and 10% methanol w/ 0.1% diethylamine using a flowrate of 60 mL/min |
| **597** | 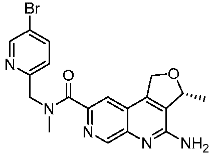 | (3R)-4-amino-N-((5-bromo-2-pyridinyl)methyl)-N,3-dimethyl-1,3-dihydrofuro[3,4-c][1,7]naphthyridine-8-carboxamide | 428.0, 430.0 | HATU | 2nd peak, Chiral Technologies AS column (250 X 21 mm, 5mm) with a mobile phase of 80% Liquid CO₂ and 20% MeOH with 0.2% TEA using a flowrate of 80 mL/min. |
| **598** | 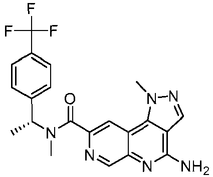 | 4-amino-N,1-dimethyl-N-((1R)-1-(4-(trifluoromethyl)phen yl)ethyl)-1H-pyrazolo[4,3-c][1,7]naphthyridine-8-carboxamide | 429.2 | TBTU | 1st peak, Chiralcel OD column (2 x 25 cm, 5 micron) with a mobile phase of 80% Liquid CO₂ and 20% methanol w/ 0.1% diethylamine using a flowrate of 50 mL/min |
| **599** | 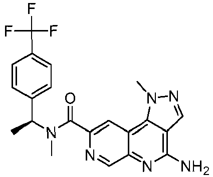 | 4-amino-N,1-dimethyl-N-((1S)-1-(4-(trifluoromethyl)phen yl)ethyl)-1H-pyrazolo[4,3-c][1,7]naphthyridine-8-carboxamide | 429.2 | TBTU | 2nd peak, Chiralcel OD column (2 x 25 cm, 5 micron) with a mobile phase of 80% Liquid CO₂ and 20% methanol w/ 0.1% diethylamine using a flowrate of 50 mL/min |
| **600** | 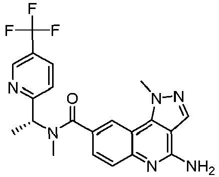 | 4-amino-N,1-dimethyl-N-((1R)-1-(5-(trifluoromethyl)-2-pyridinyl)ethyl)-1H-pyrazolo[4,3-c] quinoline-8-carboxamide | 429. 2 | TBTU | 1st peak, (S,S) Whelk-O column (2 x 25 cm, 5 micron) with a mobile phase of 70% Liquid CO₂ and 30% methanol w/ 0.1% diethylamine using a flowrate of 60 mL/min |
| **601** | 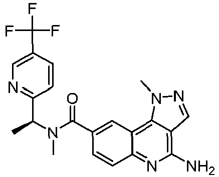 | 4-amino-N,1-dimethyl-N-((1S)-1-(5-(trifluoromethyl)-2-pyridinyl)ethyl)-1H-pyrazolo[4,3-c]quinoline-8-carboxamide | 429. 2 | TBTU | 2nd peak, (S,S) Whelk-O column (2 x 25 cm, 5 micron) with a mobile phase of 70% Liquid CO₂ and 30% methanol w/ 0.1% diethylamine using a flowrate of 60 mL/min |
| **602** | 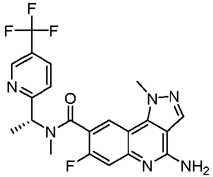 | 4-amino-7-fluoro-N,1-dimethyl-N-((1R)-1-(5-(trifluoromethyl)-2-pyridinyl)ethyl)-1H-pyrazolo[4,3-c] quinoline-8-carboxamide | 447.2 | TBTU | 1st peak, (S,S) Whelk-O column (2 x 25 cm, 5 micron) with a mobile phase of 65% Liquid CO₂ and 35% methanol w/ 0.1% diethylamine using a flowrate of 60 mL/min |
| **603** | 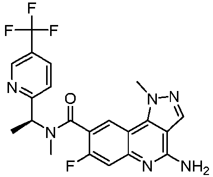 | 4-amino-7-fluoro-N,1-dimethyl-N-((1S)-1-(5-(trifluoromethyl)-2-pyridinyl)ethyl)-1H-pyrazolo[4,3-c] quinoline-8-carboxamide | 447.2 | TBTU | 2nd peak, (S,S) Whelk-O column (2 x 25 cm, 5 micron) with a mobile phase of 65% Liquid CO₂ and 35% methanol w/ 0.1% diethylamine using a flowrate of 60 mL/min |
| **604** | 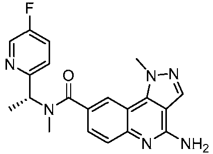 | 4-amino-N-((1R)-1-(5-fluoro-2-pyridinyl)ethyl)-N,1-dimethyl-1H-pyrazolo[4,3-c] quinoline-8-carboxamide | 379.2 | TBTU | 1st peak, Chiralpak AS column (2 x 25 cm, 5 micron) with a mobile phase of 80% Liquid CO₂ and 20% isopropanol w/ 0.1% deithylamine using a flowrate of 60mL/min |
| **605** | 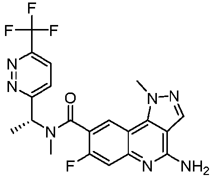 | 4-amino-7-fluoro-N,1-dimethyl-N-((1R)-1-(6-(trifluoromethyl)-3-pyridazinyl)ethyl)-1H-pyrazolo[4,3-c] quinoline-8-carboxamide | 447.9 | TBTU | 1st peak, Regis (S,S) Whelk-01 column (250 X 21 mm, 5mm) with a mobile phase of 60% Liquid CO₂ and 40% MeOH with 0.2% TEA using a flowrate of 70 mL/min |

### Example 606: (R)-4-amino-N-((5-cyanopyridin-2-yl)methyl)-N-(1-(pyrimidin-2-yl)ethyl)-1,3-dihydrofuro[3,4-c][1,7]naphthyridine-8-carboxamide.

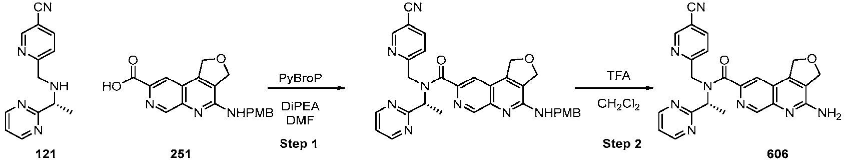

Step 1. To a solution of (R)-6-(((1-(pyrimidin-2-yl)ethyl)amino)methyl)nicotinonitrile **(121,** 0.118 g, 0.495 mmol), 4-((4-methoxybenzyl)amino)-1,3-dihydrofuro[3,4-c][1,7]naphthyridine-8-carboxylic acid hydrochloride **(251,** 0.160 g, 0.413 mmol) and 1,1'-dimethyltriethylamine (0.533 g, 0.721 mL, 4.13 mmol, Sigma-Aldrich Corporation) in DMF (5 mL) was added bromotripyrrolidinophosphonium hexafluorophosphate (0.192 g, 0.413 mmol, Sigma-Aldrich Corporation) and the resulting mixture was heated at 50°C for 1 h. The reaction was brought to rt, diluted with water and sat.NaHCO₃, and extracted with EtOAc (3x). The combined organics were dried over Na₂SO₄, filtered and concentrated. The crude residue was diluted in toluene (3 mL) and concentrated (3x). The residue was then chromatographed on silica gel using 0-50% 3:1 EtOAc/EtOH in heptane to afford (R)-N-((5-cyanopyridin-2-yl)methyl)-4-((4-methoxybenzyl)amino)-N-(1-(pyrimidin-2-yl)ethyl)-1,3-dihydrofuro[3,4-c][1,7]naphthyridine-8-carboxamide (0.112 g, 0.196 mmol, 47.4% yield) as a pale yellow solid. *m*/*z* (ESI): 573.2 (M+H)⁺.

Step 2. To a solution of (R)-N-((5-cyanopyridin-2-yl)methyl)-4-((4-methoxybenzyl)amino)-N-(1-(pyrimidin-2-yl)ethyl)-1,3-dihydrofuro[3,4-c][1,7]naphthyridine-8-carboxamide (0.112 g, 0.196 mmol, 47.4 % yield) in DCM (2 mL) was added TFA (22.20 g, 15 mL, 195 mmol, Aldrich) and the resulting mixture was stirred at 70°C for 24 h. The reaction was washed with 10% Na₂CO₃ and extracted with DCM. The combined organics were concentrated and chromatographed on silica gel using 0-50% 3:1 EtOAc/EtOH in heptane and repurified by HPLC to afford (R)-4-amino-N-((5-cyanopyridin-2-yl)methyl)-N-(1-(pyrimidin-2-yl)ethyl)-1,3-dihydrofuro[3,4-c][1,7]naphthyridine-8-carboxamide as an off-white solid **(606,** 0.070 g, 0.155 mmol, 78.9% yield). *m*/*z* (ESI): 453.2 (M+H)⁺. 1H NMR (400 MHz, DMSO-d6) δ ppm 8.58 - 8.93 (m, 4 H), 8.24 (dd, J=8.3, 2.1 Hz, 1 H), 7.80 (s, 1 H), 7.31 - 7.54 (m, 2 H), 6.97 - 7.12 (m, 2 H), 5.78 - 5.88 (m, 1 H), 5.18 - 5.44 (m, 2 H), 4.96 - 5.09 (m, 3 H), 4.55 (d, J=17.2 Hz, 1 H), 1.54 - 1.72 (m, 3 H).

### Example 607: 6-amino-N-isobutyl-N-((5-(trifluoromethyl)pyridin-2-yl)methyl)-8,9-dihydro-7H-cyclopenta[c][1,8]naphthyridine-2-carboxamide.

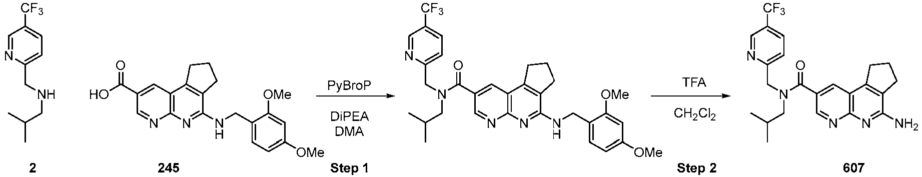

Step 1. To a solution of 2-methyl-N-((5-(trifluoromethyl)pyridin-2-yl)methyl)propan-1-amine **(2,** 0.101 g, 0.435 mmol), 6-((2,4-dimethoxybenzyl)amino)-8,9-dihydro-7H-cyclopenta[c][1,8]naphthyridine-2-carboxylic acid hydrochloride **(245,** 0.217 g, 0.522 mmol) and 1,1'-dimethyltriethylamine (0.562 g, 0.760 mL, 4.35 mmol, Sigma-Aldrich Corporation) in DMA (4 mL) was added bromotripyrrolidinophosphonium hexafluorophosphate (0.203 g, 0.435 mmol, Sigma-Aldrich Corporation) and the resulting mixture was heated at 60°C for 1 h. The reaction was brought to rt, diluted with water, sat. NaHCO₃ and extracted with EtOAc (3x). The combined organics were dried over Na₂SO₄, filtered, and concentrated. The residue was then chromatographed on silica gel using 0-40% 3:1 EtOAc/EtOH in heptane to afford 6-((2,4-dimethoxybenzyl)amino)-N-isobutyl-N-((5-(trifluoromethyl)pyridin-2-yl)methyl)-8,9-dihydro-7H-cyclopenta[c][1,8]naphthyridine-2-carboxamide as a light yellow oil. *m*/*z* (ESI): 594.2 (M+H)⁺.

Step 2. To a solution of 6-((2,4-dimethoxybenzyl)amino)-N-isobutyl-N-((5-(trifluoromethyl)pyridin-2-yl)methyl)-8,9-dihydro-7H-cyclopenta[c][1,8]naphthyridine-2-carboxamide in DCM (2 mL) was added TFA (14.80 g, 10 mL, 130 mmol, Aldrich) and the resulting mixture was heated at 50°C for 1h. The reaction was concentrated, washed with 10% Na₂CO₃ and extracted with DCM. The combined organics were concentrated and chromatographed on silica gel using 0-60% 3:1 EtOAc/EtOH in heptane to afford 6-amino-N-isobutyl-N-((5-(trifluoromethyl)pyridin-2-yl)methyl)-8,9-dihydro-7H-cyclopenta[c][1,8]naphthyridine-2-carboxamide **(607,** 0.035 g, 0.079 mmol, 18.15 % yield) as a white solid. *m*/*z* (ESI): 444.2 (M+H)⁺. ¹H NMR (400 MHz, DMSO-d6) δ ppm 8.98 (s, 1 H), 8.62 - 8.74 (m, 1 H), 8.13 - 8.27 (m, 1 H), 7.86 - 8.11 (m, 1 H), 7.48 (br s, 1 H), 6.58 - 7.06 (m, 2 H), 4.62 - 5.04 (m, 3 H), 2.90 - 3.23 (m, 2 H), 2.82 (br d, J=5.2 Hz, 2 H), 2.10 - 2.28 (m, 2 H), 1.83 - 2.12 (m, 1 H), 0.65 - 1.01 (m, 7 H).

Compounds in Table 20 were prepared in a manner similar to that described above for examples 606 and 607.

**Table 20**

| **Ex.** | **Structure** | **Name** | **m/z (ESI): (M+H)⁺** |
|---|---|---|---|
| **608** | 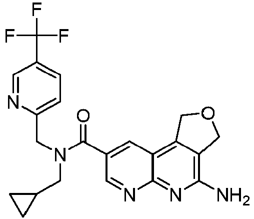 | 4-amino-N-(cyclopropylmethyl)-N-((5-(trifluoromethyl)-2-pyridinyl)methyl)-1,3-dihydrofuro[3,4-c][1,8]naphthyridine-8-carboxamide | 444.2 |
| **609** | 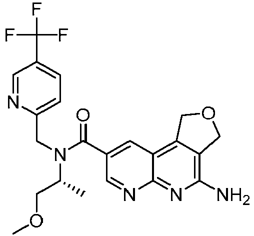 | 4-amino-N-((2R)-1-methoxy-2-propanyl)-N-((5-(trifluoromethyl)-2-pyridinyl)methyl)-1,3-dihydrofuro[3,4-c] [1,8]naphthyridine-8-carboxamide | 462.2 |
| **610** | 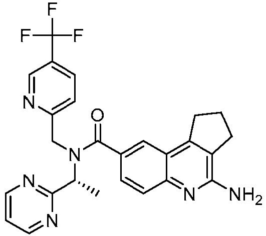 | 4-amino-N-((1R)-1-(2-pyrimidinyl)ethyl)-N-((5-(trifluoromethyl)-2-pyridinyl)methyl)-2,3-dihydro-1H-cyclopenta[c] quinoline-8-carboxamide | 493.0 |
| **611** | 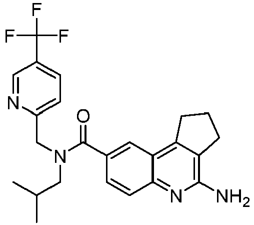 | 4-amino-N-(2-methylpropyl)-N-((5-(trifluoromethyl)-2-pyridinyl)methyl)-2,3-dihydro-1H-cyclopenta[c]quinoline-8-carboxamide | 443.0 |
| **612** | 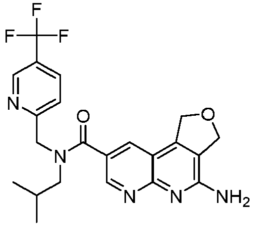 | 4-amino-N-(2-methylpropyl)-N-((5-(trifluoromethyl)-2-pyridinyl)methyl)-1,3-dihydrofuro[3,4-c] [1,8]naphthyridine-8-carboxamide | 446.0 |
| **613** | 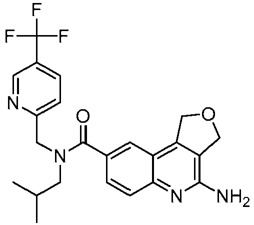 | 4-amino-N-(2-methylpropyl)-N-((5-(trifluoromethyl)-2-pyridinyl)methyl)-1,3-dihydrofuro[3,4-c]quinoline-8-carboxamide | 445.3 |
| **614** | 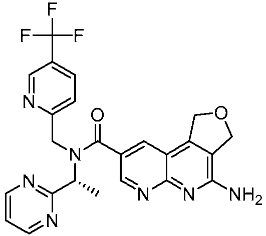 | 4-amino-N-((1R)-1-(2-pyrimidinyl)ethyl)-N-((5-(trifluoromethyl)-2-pyridinyl)methyl)-1,3-dihydrofuro[3,4-c] [1,8]naphthyridine-8-carboxamide | 496.0 |
| **615** | 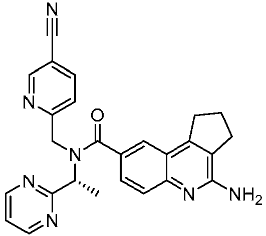 | 4-amino-N-((5-cyano-2-pyridinyl)methyl)-N-((1R)-1-(2-pyrimidinyl)ethyl)-2,3-dihydro-1H-cyclopenta[c]quinoline-8-carboxamide | 450.0 |
| **616** | 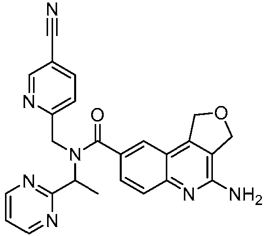 | 4-amino-N-((5-cyano-2-pyridinyl)methyl)-N-((1R)-1-(2-pyrimidinyl)ethyl)-1,3-dihydrofuro[3,4-c]quinoline-8-carboxamide and 4-amino-N-((5-cyano-2-pyridinyl)methyl)-N-((1S)-1-(2-pyrimidinyl)ethyl)-1,3-dihydrofuro[3,4-c]quinoline-8-carboxamide | 452.2 |
| **617** | 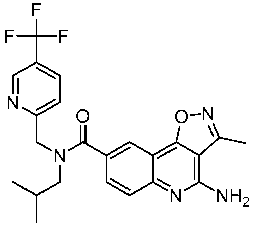 | 4-amino-3-methyl-N-(2-methylpropyl)-N-((5-(trifluoromethyl)-2-pyridinyl)methyl)[1,2]oxazolo[4,5-c]quinoline-8-carboxamide | 458.1 |
| **618** | 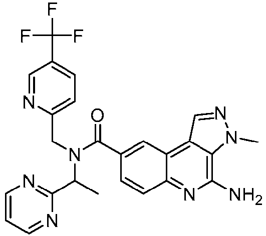 | 4-amino-3-methyl-N-((1R)-1-(2-pyrimidinyl)ethyl)-N-((5-(trifluoromethyl)-2-pyridinyl)methyl)-3H-pyrazolo[3,4-c]quinoline-8-carboxamide and 4-amino-3-methyl-N-((1S)-1-(2-pyrimidinyl)ethyl)-N-((5-(trifluoromethyl)-2-pyridinyl)methyl)-3H-pyrazolo[3,4-c] quinoline-8-carboxamide | 507.2 |
| **619** | 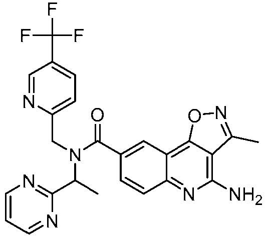 | 4-amino-3-methyl-N-((1R)-1-(2-pyrimidinyl)ethyl)-N-((5-(trifluoromethyl)-2-pyridinyl)methyl)[1,2]oxazolo[4,5-c]quinoline-8-carboxamide and 4-amino-3-methyl-N-((1S)-1-(2-pyrimidinyl)ethyl)-N-((5-(trifluoromethyl)-2-pyridinyl)methyl)[1,2]oxazolo[4,5-c]quinoline-8-carboxamide | 508.2 |
| **620** | 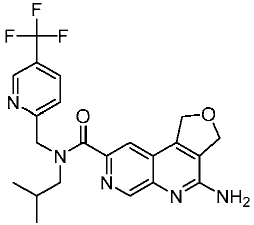 | 4-amino-N-(2-methylpropyl)-N-((5-(trifluoromethyl)-2-pyridinyl)methyl)-1,3-dihydrofuro[3,4-c] [1,7]naphthyridine-8-carboxamide | 446.0 |
| **621** | 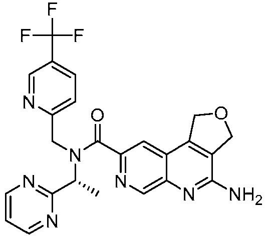 | 4-amino-N-((1R)-1-(2-pyrimidinyl)ethyl)-N-((5-(trifluoromethyl)-2-pyridinyl)methyl)-1,3-dihydrofuro[3,4-c] [1,7]naphthyridine-8-carboxamide | 496.0 |
| **622** | 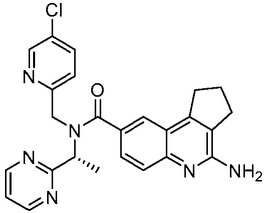 | 4-amino-N-((5-chloro-2-pyridinyl)methyl)-N-((1R)-1-(2-pyrimidinyl)ethyl)-2,3-dihydro-1H-cyclopenta[c]quinoline-8-carboxamide | 459.0 |
| **623** | 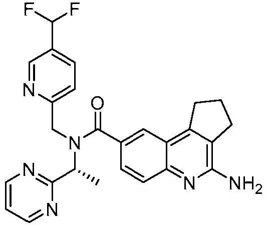 | 4-amino-N-((5-(difluoromethyl)-2-pyridinyl)methyl)-N-((1R)-1-(2-pyrimidinyl)ethyl)-2,3-dihydro-1H-cyclopenta[c]quinoline-8-carboxamide | 475.0 |
| **624** | 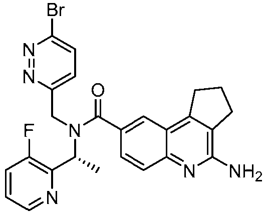 | 4-amino-N-((6-bromo-3-pyridazinyl)methyl)-N-((1R)-1-(3-fluoro-2-pyridinyl)ethyl)-2,3-dihydro-1H-cyclopenta[c]quinoline-8-carboxamide | 521.0 |
| **625** | 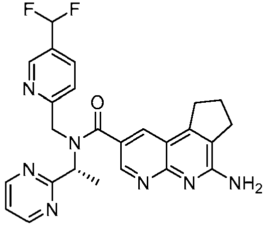 | 6-amino-N-((5-(difluoromethyl)-2-pyridinyl)methyl)-N-((1R)-1-(2-pyrimidinyl)ethyl)-8,9-dihydro-7H-cyclopenta[c] [1,8]naphthyridine-2-carboxamide | 476.0 |
| **626** | 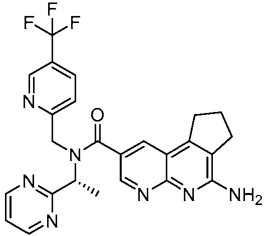 | 6-amino-N-((1R)-1-(2-pyrimidinyl)ethyl)-N-((5-(trifluoromethyl)-2-pyridinyl)methyl)-8,9-dihydro-7H-cyclopenta[c][1,8]naphthyridine-2-carboxamide | 494.0 |
| **627** | 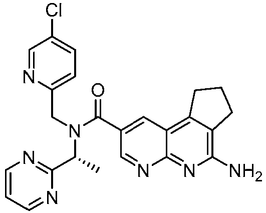 | 6-amino-N-((5-chloro-2-pyridinyl)methyl)-N-((1R)-1-(2-pyrimidinyl)ethyl)-8,9-dihydro-7H-cyclopenta[c] [1,8]naphthyridine-2-carboxamide | 460.0 |
| **628** | 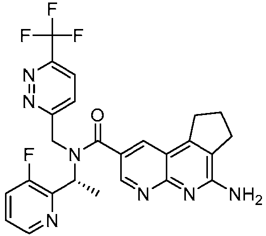 | 6-amino-N-((1R)-1-(3-fluoro-2-pyridinyl)ethyl)-N-((6-(trifluoromethyl)-3-pyridazinyl)methyl)-8,9-dihydro-7H-cyclopenta[c] [1,8]naphthyridine-2-carboxamide | 512.0 |
| **629** | 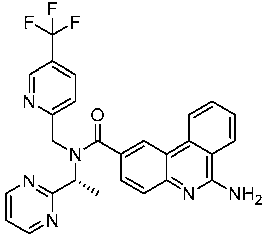 | 6-amino-N-((1R)-1-(2-pyrimidinyl)ethyl)-N-((5-(trifluoromethyl)-2-pyridinyl)methyl)-2-phenanthridinecarboxamide | 503.0 |
| **630** | 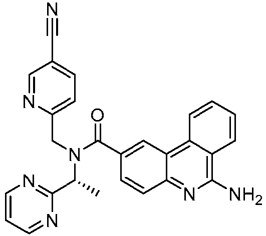 | 6-amino-N-((5-cyano-2-pyridinyl)methyl)-N-((1R)-1-(2-pyrimidinyl)ethyl)-2-phenanthridinecarboxamide | 460.0 |
| **631** | 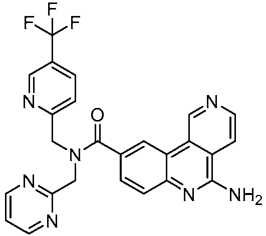 | 5-amino-N-(2-pyrimidinylmethyl)-N-((5-(trifluoromethyl)-2-pyridinyl)methyl)benzo[c] [2,6]naphthyridine-9-carboxamide | 490.0 |
| **632** | 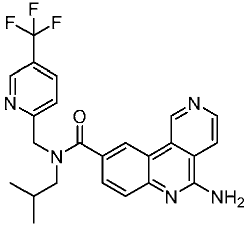 | 5-amino-N-(2-methylpropyl)-N-((5-(trifluoromethyl)-2-pyridinyl)methyl)benzo[c] [2,6]naphthyridine-9-carboxamide | 454.0 |
| **633** | 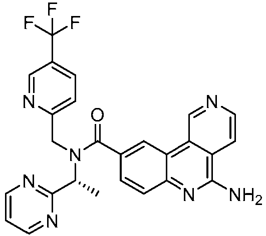 | 5-amino-N-((1R)-1-(2-pyrimidinyl)ethyl)-N-((5-(trifluoromethyl)-2-pyridinyl)methyl)benzo[c] [2,6]naphthyridine-9-carboxamide | 504.0 |
| **634** | 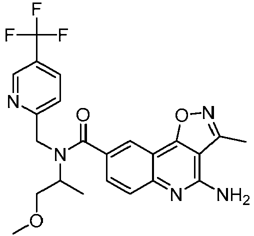 | 4-amino-N-((2R)-1-methoxy-2-propanyl)-3-methyl-N-((5-(trifluoromethyl)-2-pyridinyl)methyl)[1,2]oxazolo[4,5-c]quinoline-8-carboxamide and 4-amino-N-((2S)-1-methoxy-2-propanyl)-3-methyl-N-((5-(trifluoromethyl)-2-pyridinyl)methyl)[1,2]oxazolo[4,5-c]quinoline-8-carboxamide | 474.2 |
| **635** | 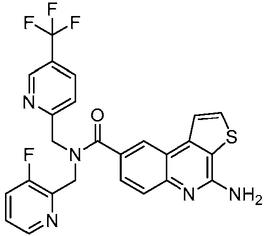 | 4-amino-N-((3-fluoro-2-pyridinyl)methyl)-N-((5-(trifluoromethyl)-2-pyridinyl)methyl)thieno[2,3 - c]quinoline-8-carboxamide | 512.0 |
| **636** | 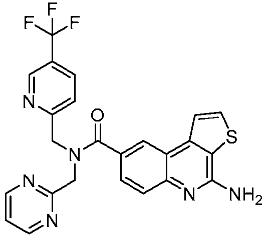 | 4-amino-N-(2-pyrimidinylmethyl)-N-((5-(trifluoromethyl)-2-pyridinyl)methyl)thieno[2,3-c]quinoline-8-carboxamide | 495.0 |
| **637** | 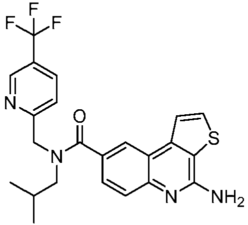 | 4-amino-N-(2-methylpropyl)-N-((5-(trifluoromethyl)-2-pyridinyl)methyl)thieno[2,3-c]quinoline-8-carboxamide | 459.0 |
| **638** | 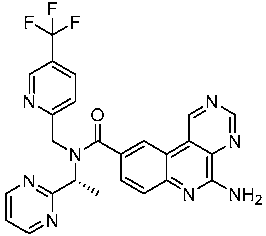 | 5-amino-N-((1R)-1-(2-pyrimidinyl)ethyl)-N-((5-(trifluoromethyl)-2-pyridinyl)methyl)pyrimido[4,5-c] quinoline-9-carboxamide | 505.0 |
| **639** | 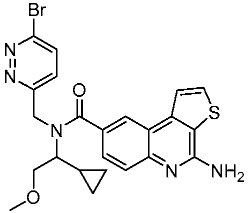 | 4-amino-N-((6-bromo-3-pyridazinyl)methyl)-N-((1R)-1-cyclopropyl-2-methoxyethyl)thieno[2,3-c]quinoline-8-carboxamide and 4-amino-N-((6-bromo-3-pyridazinyl)methyl)-N-((1S)-1-cyclopropyl-2-methoxyethyl)thieno[2,3-c]quinoline-8-carboxamide | 512.0 |
| **640** | 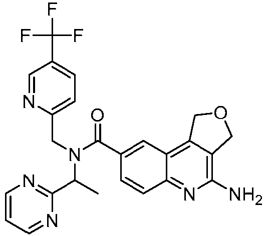 | 4-amino-N-((1R)-1-(2-pyrimidinyl)ethyl)-N-((5-(trifluoromethyl)-2-pyridinyl)methyl)-1,3-dihydrofuro[3,4-c]quinoline-8-carboxamide and 4-amino-N-((1S)-1-(2-pyrimidinyl)ethyl)-N-((5-(trifluoromethyl)-2-pyridinyl)methyl)-1,3-dihydrofuro[3,4-c]quinoline-8-carboxamide | 495.2 |
| **641** | 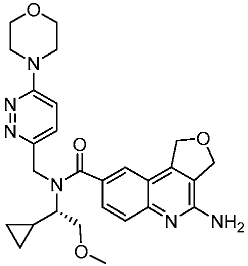 | 4-amino-N-((1S)-1-cyclopropyl-2-methoxyethyl)-N-((6-(4-morpholinyl)-3-pyridazinyl)methyl)-1,3-dihydrofuro[3,4-c]quinoline-8-carboxamide | 505.1 |
| **642** | 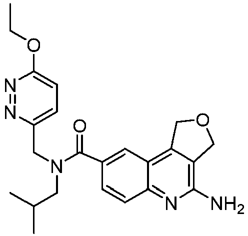 | 4-amino-N-((6-ethoxy-3-pyridazinyl)methyl)-N-(2-methylpropyl)-1,3-dihydrofuro [3,4-c] quinoline-8-carboxamide | 422.1 |
| **643** | 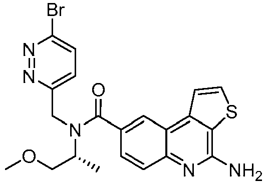 | 4-amino-N-((6-bromo-3-pyridazinyl)methyl)-N-((2R)-1-methoxy-2-propanyl)thieno[2,3-c]quinoline-8-carboxamide | 486.0 and 488.0 |
| **644** | 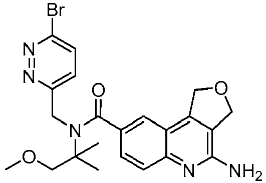 | 4-amino-N-((6-bromo-3-pyridazinyl)methyl)-N-(1-methoxy-2-methyl-2-propanyl)-1,3-dihydrofuro[3,4-c]quinoline-8-carboxamide | 486.0 and 488.0 |
| **645** | 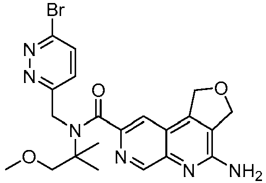 | 4-amino-N-((6-bromo-3-pyridazinyl)methyl)-N-(1-methoxy-2-methyl-2-propanyl)-1,3-dihydrofuro[3,4-c] [1,7]naphthyridine-8-carboxamide | 487.15 and 489.10 |

### Example 646 and 647: (R) or (S)-4-amino-N-((6-methoxypyridazin-3-yl)methyl)-N-(1-(pyrimidin-2-yl)ethyl)-2,3-dihydro-1H-cyclopenta[c]quinoline-8-carboxamide.

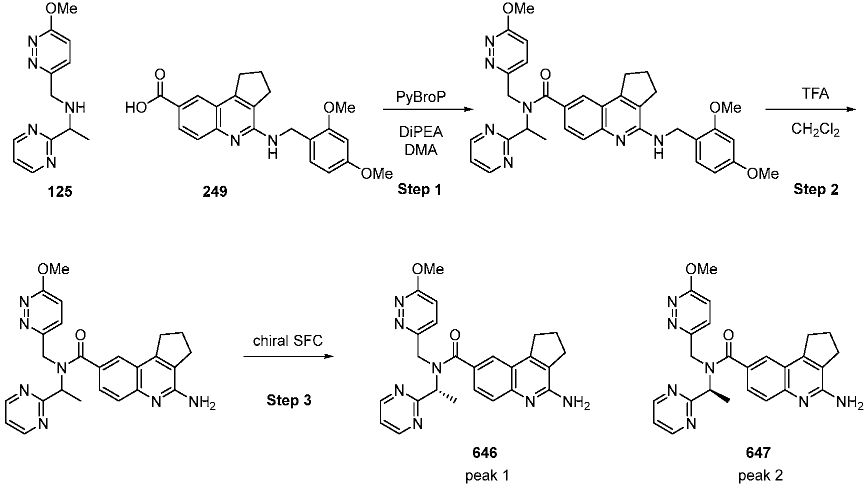

Step 1. To a solution of N-((6-methoxypyridazin-3-yl)methyl)-1-(pyrimidin-2-yl)ethan-1-amine **(125,** 0.150 g, 0.612 mmol), 4-((2,4-dimethoxybenzyl)amino)-2,3-dihydro-1H-cyclopenta[c]quinoline-8-carboxylic acid hydrochloride **(249,** 0.330 g, 0.795 mmol) and 1,1'-dimethyltriethylamine (0.790 g, 1.068 mL, 6.12 mmol, Sigma-Aldrich Corporation) in DMF (5 mL) was added bromotripyrrolidinophosphonium hexafluorophosphate (0.285 g, 0.612 mmol, Sigma-Aldrich Corporation) and the resulting mixture was heated at 50°C for 45 min. The reaction was brought to rt, diluted with water, sat.NaHCO₃ and extracted with EtOAc (3x). The combined organics were dried over Na₂SO₄, filtered and concentrated. The residue was then chromatographed on silica gel using 0-50% 3:1 EtOAc/EtOH in heptane to afford 4-((2,4-dimethoxybenzyl)amino)-N-((6-methoxypyridazin-3-yl)methyl)-N-(1-(pyrimidin-2-yl)ethyl)-2,3-dihydro-1H-cyclopenta[c]quinoline-8-carboxamide as a light yellow solid. *m*/*z* (ESI): 606.2 (M+H)⁺.

Step 2. To a solution of 4-((2,4-dimethoxybenzyl)amino)-N-((6-methoxypyridazin-3-yl)methyl)-N-(1-(pyrimidin-2-yl)ethyl)-2,3-dihydro-1H-cyclopenta[c]quinoline-8-carboxamide in DCM (2 mL) was added TFA (1.5 mL, 19.5 mmol, Aldrich) and the resulting mixture was heated at 50°C for 1 h. The reaction was concentrated, washed with 10% Na₂CO₃ and extracted with DCM. The combined organics were concentrated and chromatographed on silica gel using 0-50% 3:1 EtOAc/EtOH in heptane to afford 4-amino-N-((6-methoxypyridazin-3-yl)methyl)-N-(1-(pyrimidin-2-yl)ethyl)-2,3-dihydro-1H-cyclopenta[c]quinoline-8-carboxamide (0.067 g, 0.147 mmol, 24.05 % yield) as a light yellow solid.

Step 3. The racemic sample was purified via preparative SFC using a Chiral Technologies AD column (250 X 21 mm, 5mm) with a mobile phase of 50% Liquid CO₂ and 50% MeOH with 0.2% TEA using a flowrate of 50 mL/min. The more potent (measured by IC₅₀ in HCT116 MTAP null cell viability assay) enantiomer was assigned as the (*R*)-; the less potent (measured by IC₅₀ in HCT116 MTAP null cell viability assay) enantiomer was assigned as (*S*)-. The 1^{st} eluting peak was (R)-4-amino-N-((6-methoxypyridazin-3-yl)methyl)-N-(1-(pyrimidin-2-yl)ethyl)-2,3-dihydro-1H-cyclopenta[c]quinoline-8-carboxamide **(646,** 0.016 g, 0.035 mmol), isolated as a light brown solid. The 2^{nd} eluting peak was (S)-4-amino-N-((6-methoxypyridazin-3-yl)methyl)-N-(1-(pyrimidin-2-yl)ethyl)-2,3-dihydro-1H-cyclopenta[c]quinoline-8-carboxamide **(647,** 0.015 g, 0.033 mmol), isolated as a light yellow solid. *m*/*z* (ESI): 456 (M+H)⁺. ¹H NMR (400 MHz, DMSO-d6) δ ppm 8.80 (d, J=5.0 Hz, 2 H), 7.71 (br d, J=1.9 Hz, 1 H), 7.47 - 7.62 (m, 3 H), 7.42 (t, J=4.9 Hz, 1 H), 7.14 (d, J=9.1 Hz, 1 H), 6.44 (br s, 2 H), 5.33 - 5.51 (m, 1 H), 4.94 (br d, J=16.0 Hz, 1 H), 4.52 - 4.72 (m, 1 H), 4.00 (s, 3 H), 2.89 - 3.16 (m, 2 H), 2.75 - 2.86 (m, 2 H), 2.16 (br t, J=7.6 Hz, 2 H), 1.60 (d, J=7.0 Hz, 3 H).

Compounds in Table 21 were prepared in a manner similar to that described above for Example **646** and **647.**

**Table 21**

| **Ex.** | **Structure** | **Name** | **m/z (ESI): (M+H)⁺** | **SFC conditions** |
|---|---|---|---|---|
| **648** | 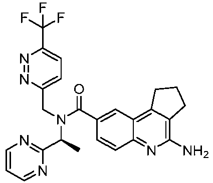 | 4-amino-N-((1S)-1-(2-pyrimidinyl)ethyl)-N-((6-(trifluoromethyl)-3-pyridazinyl)methyl)-2,3-dihydro-1H-cyclopenta[c]quinoline-8-carboxamide | 494 | 1st peak, Chiral Technologies AD column (250 X 21 mm, 5mm) with a mobile phase of 80% Liquid CO₂ and 20% MeOH with 0.2% TEA using a flowrate of 80 mL/min |
| **649** | 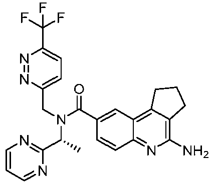 | 4-amino-N-((1R)-1-(2-pyrimidinyl)ethyl)-N-((6-(trifluoromethyl)-3-pyridazinyl)methyl)-2,3-dihydro-1H-cyclopenta[c]quinoline-8-carboxamide | 494 | 2nd peak, Chiral Technologies AD column (250 X 21 mm, 5mm) with a mobile phase of 80% Liquid CO₂ and 20% MeOH with 0.2% TEA using a flowrate of 80 mL/min |
| **650** | 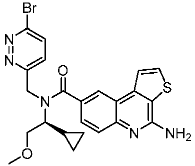 | 4-amino-N-((6-bromo-3-pyridazinyl)methyl)-N-((1S)-1-cyclopropyl-2-methoxyethyl)thieno[2,3-c] quinoline-8-carboxamide | 512 | 1st peak, Chiral Technologies OD column (250 X 21 mm, 5mm) with a mobile phase of 50% Liquid CO₂ and 50% MeOH with 0.2% TEA using a flowrate of 60 mL/min |
| **651** | 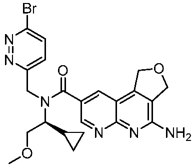 | 4-amino-N-((6-bromo-3-pyridazinyl)methyl)-N-((1S)-1-cyclopropyl-2-methoxyethyl)-1,3-dihydrofuro[3,4-c][1,8]naphthyridine-8-carboxamide | 499 | 1st peak, Chiral Technologies OD column (250 X 21 mm, 5mm) with a mobile phase of 75% Liquid CO₂ and 25% MeOH with 0.2% TEA using a flowrate of 80 mL/min |

### Example 652: 4-amino-N-(cyclopropylmethyl)-N-((5-(trifluoromethyl)pyridin-2-yl)methyl)-1,3-dihydrofuro[3,4-c][1,7]naphthyridine-8-carboxamide.

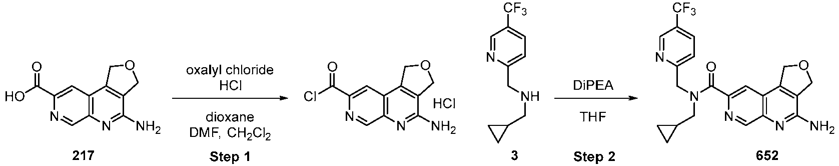

Step 1. To a stirred solution of 4-amino-1,3-dihydrofuro[3,4-c][1,7]naphthyridine-8-carboxylic acid **(217,** 0.500 g, 2.163 mmol) in dichloromethane (5.00 mL) was added HCl (4M in dioxane, 1.622 mL, 6.49 mmol) and the reaction mixture was stirred at room temperature for 30 min. Then, the reaction mixture was concentrated, co-distilled with toluene (3 x 50 mL), and dried. This crude material was taken up in dichloromethane (5.00 mL) and cooled to 0 °C. Oxalyl chloride (1.136 mL, 12.98 mmol) and DMF (0.033 mL, 0.433 mmol) were added dropwise at the same temperature and the reaction mixture was stirred room temperature for 16 h. The reaction mixture was concentrated under reduced pressure in nitrogen atmosphere, and the obtained crude material was triturated with heptane (3 x 5 mL), and dried under nitrogen atmosphere to give 4-amino-1,3-dihydrofuro[3,4-c][1,7]naphthyridine-8-carbonyl chloride hydrochloride (0.500 g, 1.748 mmol, 81 % yield) as yellow solid.

Step 2. To a mixture of 1-cyclopropyl-N-((5-(trifluoromethyl)pyridin-2-yl)methyl)methanamine (3, 0.050 g, 0.217 mmol) , tetrahydrofuran (2 mL) and diisopropylethylamine (0.112 g, 0.151 mL, 0.869 mmol, Aldrich) was added 4-amino-1,3-dihydrofuro[3,4-c][1,7]naphthyridine-8-carbonyl chloride hydrochloride (0.065 g, 0.228 mmol). The mixture was stirred at rt until completion and then concentrated in vacuo. The crude product was purified by silica gel chromatography (0-100% EtOAc/EtOH (3/1) in heptane). 4-amino-N-(cyclopropylmethyl)-N-((5-(trifluoromethyl)pyridin-2-yl)methyl)-1,3-dihydrofuro[3,4-c][1,7]naphthyridine-8-carboxamide **(652,** 49.5 mg, 0.112 mmol, 51.4% yield) was isolated as an off-white solid. *m*/*z* (ESI): 444 (M+H)⁺. ¹H NMR (400 MHz, METHANOL-d4) δ ppm 8.75 - 8.97 (m, 2 H), 8.02 - 8.16 (m, 1 H), 7.82 (br s, 1 H), 7.59 - 7.78 (m, 1 H), 5.31 - 5.49 (m, 2 H), 5.04 - 5.19 (m, 4 H), 3.44 - 3.54 (m, 2 H), 1.06 - 1.31 (m, 1 H), 0.37 - 0.55 (m, 2 H), 0.04 - 0.31 (m, 2 H). ¹⁹F NMR (377 MHz, METHANOL-d4) δ ppm -63.86 (m, 3 F).

Compounds in Table 22 were prepared in a manner similar to that described above for Example **652.**

**Table 22**

| **Ex.** | **Structure** | **Name** | **m/z (ESI): (M+H)⁺** |
|---|---|---|---|
| **653** | 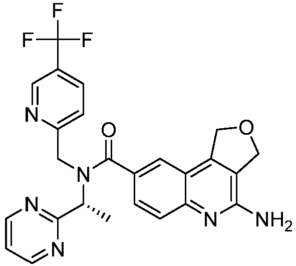 | 4-amino-N-((1R)-1-(2-pyrimidinyl)ethyl)-N-((5-(trifluoromethyl)-2-pyridinyl)methyl)-1,3-dihydrofuro[3,4-c]quinoline-8-carboxamide | 495.1 |
| **654** | 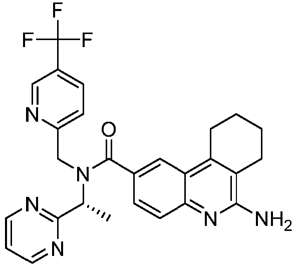 | 6-amino-N-((1R)-1-(2-pyrimidinyl)ethyl)-N-((5-(trifluoromethyl)-2-pyridinyl)methyl)-7,8,9,10-tetrahydro-2-phenanthridinecarboxamide | 507.2 |
| **655** | 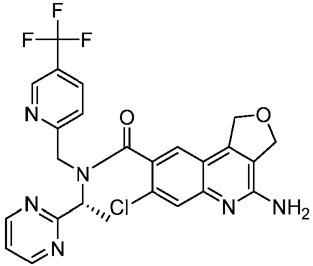 | 4-amino-7-chloro-N-((1R)-1-(2-pyrimidinyl)ethyl)-N-((5-(trifluoromethyl)-2-pyridinyl)methyl)-1,3-dihydrofuro[3,4-c]quinoline-8-carboxamide | 529.1 |
| **656** | 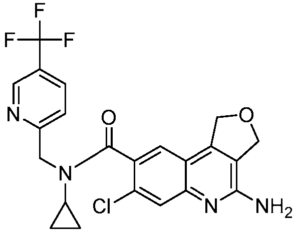 | 4-amino-7-chloro-N-cyclopropyl-N-((5-(trifluoromethyl)-2-pyridinyl)methyl)-1,3-dihydrofuro[3,4-c]quinoline-8-carboxamide | 463 |
| **657** | 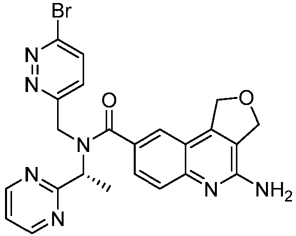 | 4-amino-N-((6-bromo-3-pyridazinyl)methyl)-N-((1R)-1-(2-pyrimidinyl)ethyl)-1,3-dihydrofuro[3,4-c]quinoline-8-carboxamide | 506 and 508 |
| **658** | 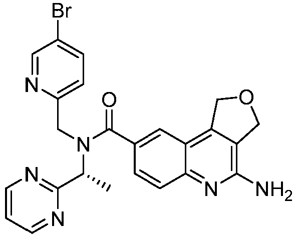 | 4-amino-N-((5-bromo-2-pyridinyl)methyl)-N-((1R)-1-(2-pyrimidinyl)ethyl)-1,3-dihydrofuro[3,4-c]quinoline-8-carboxamide | *505* and 507 |
| **659** | 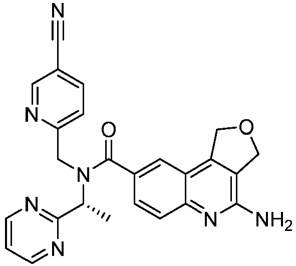 | 4-amino-N-((5-cyano-2-pyridinyl)methyl)-N-((1R)-1-(2-pyrimidinyl)ethyl)-1,3-dihydrofuro[3,4-c]quinoline-8-carboxamide | 452 |
| **660** | 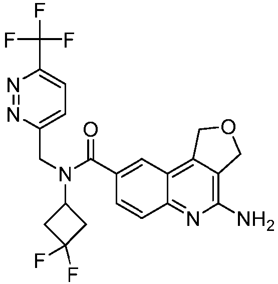 | 4-amino-N-(3,3-difluorocyclobutyl)-N-((6-(trifluoromethyl)-3-pyridazinyl)methyl)-1,3-dihydrofuro[3,4-c]quinoline-8-carboxamide | 481.2 |
| **661** | 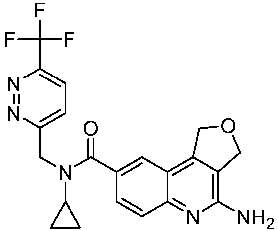 | 4-amino-N-cyclopropyl-N-((6-(trifluoromethyl)-3-pyridazinyl)methyl)-1,3-dihydrofuro[3,4-c]quinoline-8-carboxamide | 430.2 |
| **662** | 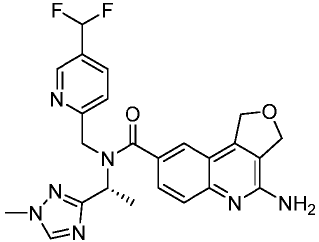 | 4-amino-N-((5-(difluoromethyl)-2-pyridinyl)methyl)-N-((1R)-1-(1-methyl-1H-1,2,4-triazol-3-yl)ethyl)-1,3-dihydrofuro[3,4-c]quinoline-8-carboxamide | 480.3 |
| **663** | 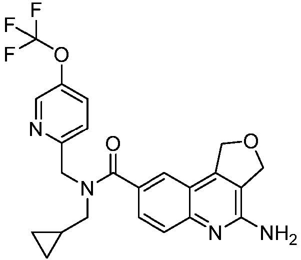 | 4-amino-N-(cyclopropylmethyl)-N-((5-(trifluoromethoxy)-2-pyridinyl)methyl)-1,3-dihydrofuro[3,4-c]quinoline-8-carboxamide | 459.2 |
| **664** | 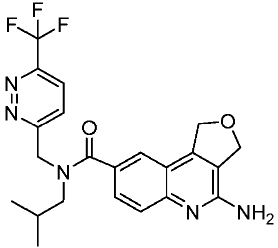 | 4-amino-N-(2-methylpropyl)-N-((6-(trifluoromethyl)-3-pyridazinyl)methyl)-1,3-dihydrofuro[3,4-c]quinoline-8-carboxamide | 446.1 |
| **665** | 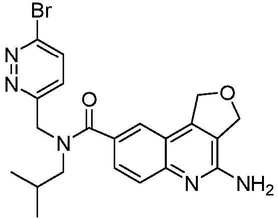 | 4-amino-N-((6-bromo-3-pyridazinyl)methyl)-N-(2-methylpropyl)-1,3-dihydrofuro[3,4-c]quinoline-8-carboxamide | 456 and 458 |
| **666** | 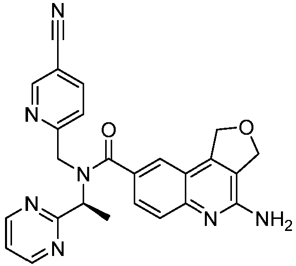 | 4-amino-N-((5-cyano-2-pyridinyl)methyl)-N-((1S)-1-(2-pyrimidinyl)ethyl)-1,3-dihydrofuro[3,4-c]quinoline-8-carboxamide | 452 |
| **667** | 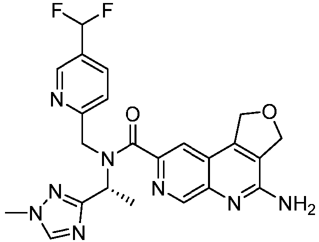 | 4-amino-N-((5-(difluoromethyl)-2-pyridinyl)methyl)-N-((1R)-1-(1-methyl-1H-1,2,4-triazol-3-yl)ethyl)-1,3-dihydrofuro[3,4-c][1,7]naphthyridine-8-carboxamide | 481.2 |
| **668** | 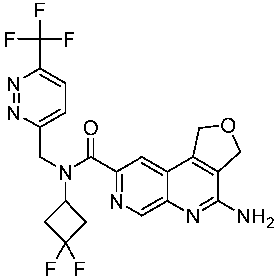 | 4-amino-N-(3,3-difluorocyclobutyl)-N-((6-(trifluoromethyl)-3-pyridazinyl)methyl)-1,3-dihydrofuro[3,4-c][1,7]naphthyridine-8-carboxamide | 481.2 |
| **669** | 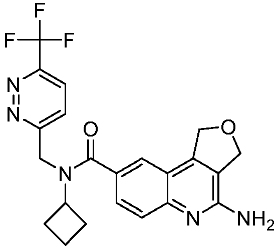 | 4-amino-N-cyclobutyl-N-((6-(trifluoromethyl)-3-pyridazinyl)methyl)-1,3-dihydrofuro[3,4-c]quinoline-8-carboxamide | 444.2 |
| **670** | 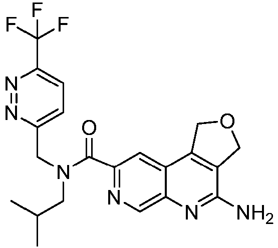 | 4-amino-N-(2-methylpropyl)-N-((6-(trifluoromethyl)-3-pyridazinyl)methyl)-1,3-dihydrofuro[3,4-c][1,7]naphthyridine-8-carboxamide | 447 |
| **671** | 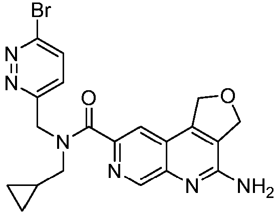 | 4-amino-N-((6-bromo-3-pyridazinyl)methyl)-N-(cyclopropylmethyl)-1,3-dihydrofuro[3,4-c][1,7]naphthyridine-8-carboxamide | 455 and 457 |
| **672** | 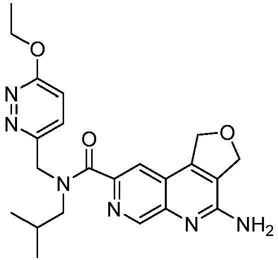 | 4-amino-N-((6-ethoxy-3-pyridazinyl)methyl)-N-(2-methylpropyl)-1,3-dihydrofuro[3,4-c][1,7]naphthyridine-8-carboxamide | 423 |
| **673** | 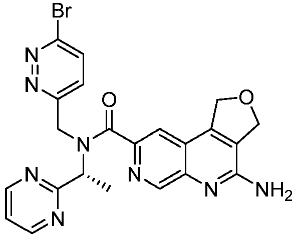 | 4-amino-N-((6-bromo-3-pyridazinyl)methyl)-N-((1R)-1-(2-pyrimidinyl)ethyl)-1,3-dihydrofuro[3,4-c][1,7]naphthyridine-8-carboxamide | 507 and 509 |
| **674** | 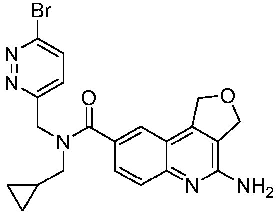 | 4-amino-N-((6-bromo-3-pyridazinyl)methyl)-N-(cyclopropylmethyl)-1,3-dihydrofuro[3,4-c]quinoline-8-carboxamide | 454 and 456 |
| **675** | 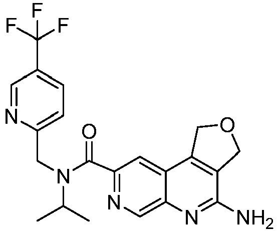 | 4-amino-N-(2-propanyl)-N-((5-(trifluoromethyl)-2-pyridinyl)methyl)-1,3-dihydrofuro[3,4-c][1,7]naphthyridine-8-carboxamide | 432.2 |
| **676** | 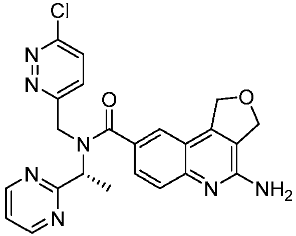 | 4-amino-N-((6-chloro-3-pyridazinyl)methyl)-N-((1R)-1-(2-pyrimidinyl)ethyl)-1,3-dihydrofuro[3,4-c]quinoline-8-carboxamide | 462 |
| **677** | 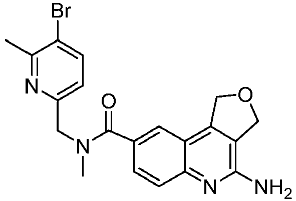 | 4-amino-N-((5-bromo-6-methyl-2-pyridinyl)methyl)-N-methyl-1,3-dihydrofuro[3,4-c]quinoline-8-carboxamide | 427.0 and 428.9 |
| **678** | 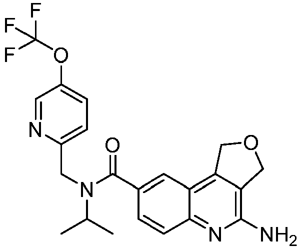 | 4-amino-N-(2-propanyl)-N-((5-(trifluoromethoxy)-2-pyridinyl)methyl)-1,3-dihydrofuro[3,4-c]quinoline-8-carboxamide | |
| **679** | 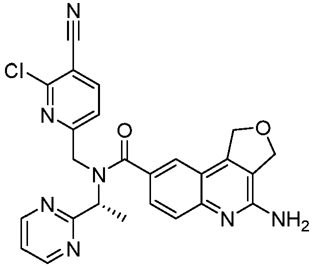 | 4-amino-N-((6-chloro-5-cyano-2-pyridinyl)methyl)-N-((1R)-1-(2-pyrimidinyl)ethyl)-1,3-dihydrofuro[3,4-c]quinoline-8-carboxamide | 486.2 |
| **680** | 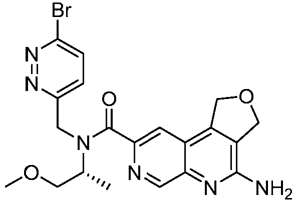 | 4-amino-N-((6-bromo-3-pyridazinyl)methyl)-N-((2R)-1-methoxy-2-propanyl)-1,3-dihydrofuro[3,4-c][1,7]naphthyridine-8-carboxamide | 473.0 and 475.1 |
| **681** | 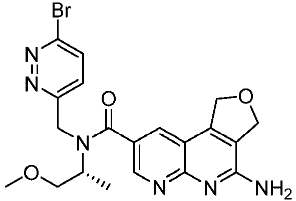 | 4-amino-N-((6-bromo-3-pyridazinyl)methyl)-N-((2R)-1-methoxy-2-propanyl)-1,3-dihydrofuro[3,4-c][1,8]naphthyridine-8-carboxamide | 473.0 and 475.1 |
| **682** | 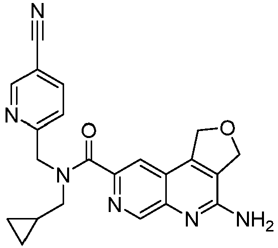 | 4-amino-N-((5-cyano-2-pyridinyl)methyl)-N-(cyclopropylmethyl)-1,3-dihydrofuro[3,4-c][1,7]naphthyridine-8-carboxamide | 401 |
| **683** | 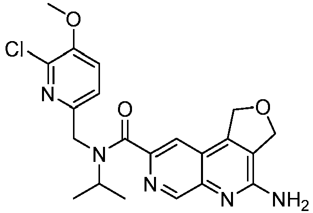 | 4-amino-N-((6-chloro-5-methoxy-2-pyridinyl)methyl)-N-(2-propanyl)-1,3-dihydrofuro[3,4-c][1,7]naphthyridine-8-carboxamide | 428 |
| **684** | 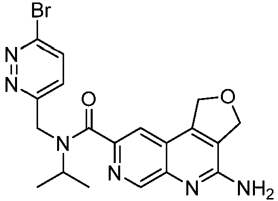 | 4-amino-N-((6-bromo-3-pyridazinyl)methyl)-N-(2-propanyl)-1,3-dihydrofuro[3,4-c][1,7]naphthyridine-8-carboxamide | 443 and 445 |
| **685** | 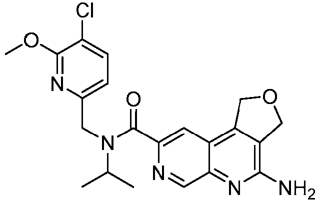 | 4-amino-N-((5-chloro-6-methoxy-2-pyridinyl)methyl)-N-(2-propanyl)-1,3-dihydrofuro[3,4-c][1,7]naphthyridine-8-carboxamide | 428 |
| **686** | 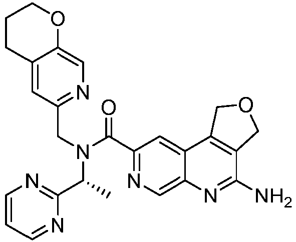 | 4-amino-N-(3,4-dihydro-2H-pyrano[2,3-c]pyridin-6-ylmethyl)-N-((1R)-1-(2-pyrimidinyl)ethyl)-1,3-dihydrofuro[3,4-c][1,7]naphthyridine-8-carboxamide | 484.2 |
| **687** | 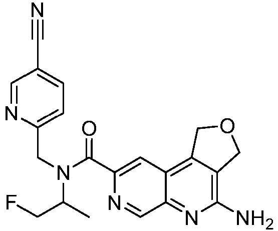 | 4-amino-N-((5-cyano-2-pyridinyl)methyl)-N-((2R)-1-fluoro-2-propanyl)-1,3-dihydrofuro[3,4-c][1,7]naphthyridine-8-carboxamide and 4-amino-N-((5-cyano-2-pyridinyl)methyl)-N-((2S)-1-fluoro-2-propanyl)-1,3-dihydrofuro[3,4-c][1,7]naphthyridine-8-carboxamide | 407.2 |
| **688** | 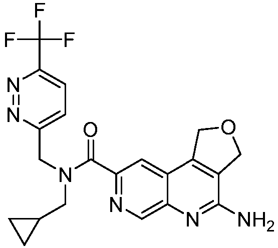 | 4-amino-N-(cyclopropylmethyl)-N-((6-(trifluoromethyl)-3-pyridazinyl)methyl)-1,3-dihydrofuro[3,4-c][1,7]naphthyridine-8-carboxamide | 445 |
| **689** | 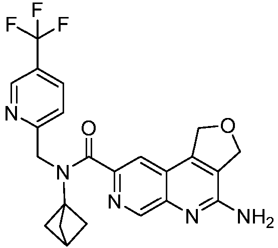 | 4-amino-N-(bicyclo[1.1.1]pentan-1-yl)-N-((5-(trifluoromethyl)-2-pyridinyl)methyl)-1,3-dihydrofuro[3,4-c][1,7]naphthyridine-8-carboxamide | 456.2 |
| **690** | 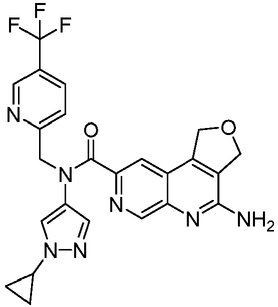 | 4-amino-N-(1-cyclopropyl-1H-pyrazol-4-yl)-N-((5-(trifluoromethyl)-2-pyridinyl)methyl)-1,3-dihydrofuro[3,4-c][1,7]naphthyridine-8-carboxamide | 496 |
| **691** | 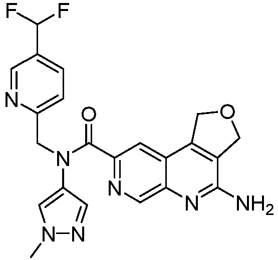 | 4-amino-N-((5-(difluoromethyl)-2-pyridinyl)methyl)-N-(1-methyl-1H-pyrazol-4-yl)-1,3-dihydrofuro[3,4-c][1,7]naphthyridine-8-carboxamide | 452.2 |
| **692** | 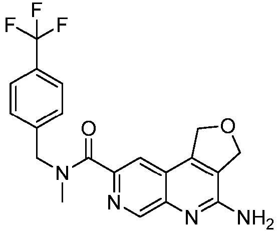 | 4-amino-N-methyl-N-(4-(trifluoromethyl)benzyl)-1,3-dihydrofuro[3,4-c][1,7]naphthyridine-8-carboxamide | 403 |
| **693** | 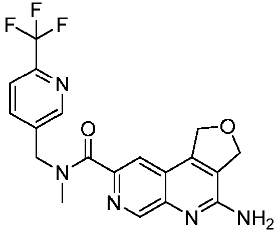 | 4-amino-N-methyl-N-((6-(trifluoromethyl)-3-pyridinyl)methyl)-1,3-dihydrofuro[3,4-c][1,7]naphthyridine-8-carboxamide | 404 |
| **694** | 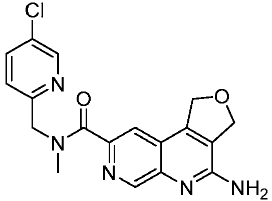 | 4-amino-N-((5-chloro-2-pyridinyl)methyl)-N-methyl-1,3-dihydrofuro[3,4-c][1,7]naphthyridine-8-carboxamide | 370 |
| **695** | 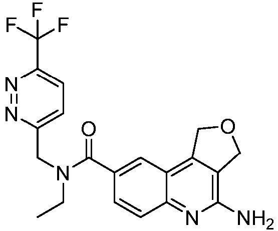 | 4-amino-N-ethyl-N-((6-(trifluoromethyl)-3-pyridazinyl)methyl)-1,3-dihydrofuro[3,4-c]quinoline-8-carboxamide | 418.45 |
| **696** | 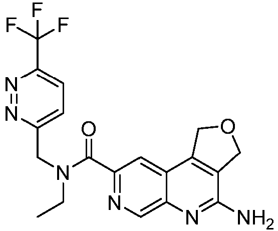 | 4-amino-N-ethyl-N-((6-(trifluoromethyl)-3-pyridazinyl)methyl)-1,3-dihydrofuro[3,4-c][1,7]naphthyridine-8-carboxamide | 419.35 |
| **697** | 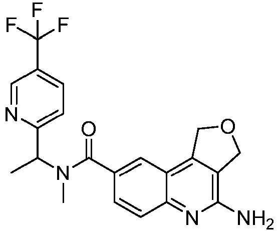 | 4-amino-N-methyl-N-((1R)-1-(5-(trifluoromethyl)-2-pyridinyl)ethyl)-1,3-dihydrofuro[3,4-c]quinoline-8-carboxamide and 4-amino-N-methyl-N-((1S)-1-(5-(trifluoromethyl)-2-pyridinyl)ethyl)-1,3-dihydrofuro[3,4-c]quinoline-8-carboxamide | 417.2 |
| **698** | 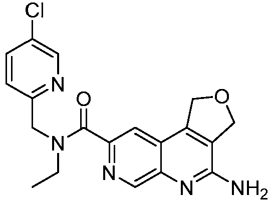 | 4-amino-N-((5-chloro-2-pyridinyl)methyl)-N-ethyl-1,3-dihydrofuro[3,4-c][1,7]naphthyridine-8-carboxamide | 384 |
| **699** | 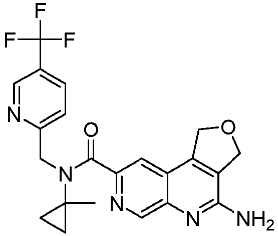 | 4-amino-N-(1-methylcyclopropyl)-N-((5-(trifluoromethyl)-2-pyridinyl)methyl)-1,3-dihydrofuro[3,4-c][1,7]naphthyridine-8-carboxamide | 444.2 |
| **700** | 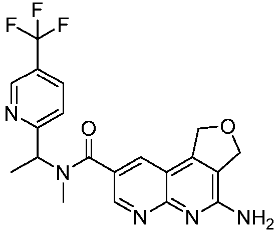 | 4-amino-N-methyl-N-((1R)-1-(5-(trifluoromethyl)-2-pyridinyl)ethyl)-1,3-dihydrofuro[3,4-c] [1,8]naphthyridine-8-carboxamide and 4-amino-N-methyl-N-((1S)-1-(5-(trifluoromethyl)-2-pyridinyl)ethyl)-1,3-dihydrofuro[3,4-c][1,8]naphthyridine-8-carboxamide | 418 |
| **701** | 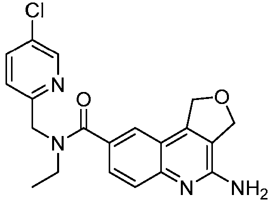 | 4-amino-N-((5-chloro-2-pyridinyl)methyl)-N-ethyl-1,3-dihydrofuro[3,4-c]quinoline-8-carboxamide | 383 |
| **702** | 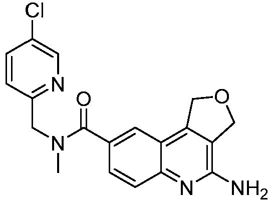 | 4-amino-N-((5-chloro-2-pyridinyl)methyl)-N-methyl-1,3-dihydrofuro[3,4-c]quinoline-8-carboxamide | 369 |
| **703** | 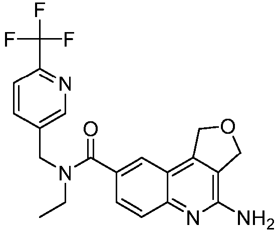 | 4-amino-N-ethyl-N-((6-(trifluoromethyl)-3-pyridinyl)methyl)-1,3-dihydrofuro[3,4-c]quinoline-8-carboxamide | 417 |
| **704** | 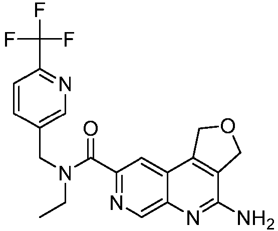 | 4-amino-N-ethyl-N-((6-(trifluoromethyl)-3-pyridinyl)methyl)-1,3-dihydrofuro[3,4-c][1,7]naphthyridine-8-carboxamide | 418 |
| **705** | 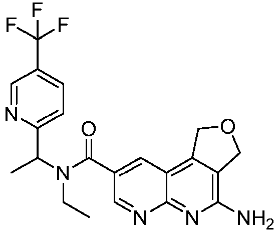 | 4-amino-N-ethyl-N-((1R)-1-(5-(trifluoromethyl)-2-pyridinyl)ethyl)-1,3-dihydrofuro[3,4-c][1,8]naphthyridine-8-carboxamide and 4-amino-N-ethyl-N-((1S)-1-(5-(trifluoromethyl)-2-pyridinyl)ethyl)-1,3-dihydrofuro[3,4-c][1,8]naphthyridine-8-carboxamide | 432 |
| **706** | 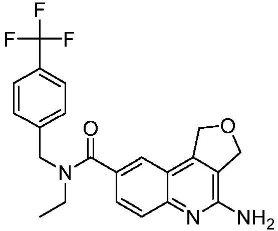 | 4-amino-N-ethyl-N-(4-(trifluoromethyl)benzyl)-1,3-dihydrofuro[3,4-c]quinoline-8-carboxamide | 416 |
| **707** | 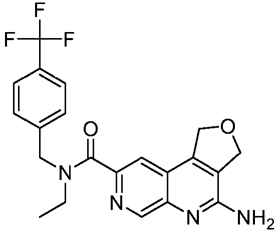 | 4-amino-N-ethyl-N-(4-(trifluoromethyl)benzyl)-1,3-dihydrofuro[3,4-c][1,7]naphthyridine-8-carboxamide | 417 |
| **708** | 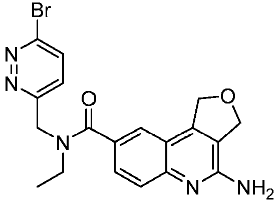 | 4-amino-N-((6-bromo-3-pyridazinyl)methyl)-N-ethyl-1,3-dihydrofuro[3,4-c]quinoline-8-carboxamide | 428.05 and 430.15 |
| **709** | 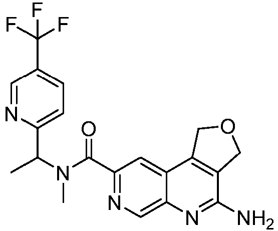 | 4-amino-N-methyl-N-((1R)-1-(5-(trifluoromethyl)-2-pyridinyl)ethyl)-1,3-dihydrofuro[3,4-c][1,7]naphthyridine-8-carboxamide and 4-amino-N-methyl-N-((1S)-1-(5-(trifluoromethyl)-2-pyridinyl)ethyl)-1,3-dihydrofuro[3,4-c][1,7]naphthyridine-8-carboxamide | 418 |
| **710** | 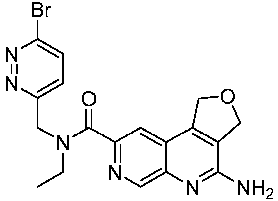 | 4-amino-N-((6-bromo-3-pyridazinyl)methyl)-N-ethyl-1,3-dihydrofuro[3,4-c][1,7]naphthyridine-8-carboxamide | 429.1 and 431.05 |
| **711** | 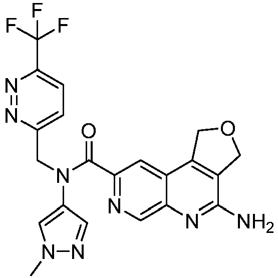 | 4-amino-N-(1-methyl-1H-pyrazol-4-yl)-N-((6-(trifluoromethyl)-3-pyridazinyl)methyl)-1,3-dihydrofuro[3,4-c][1,7]naphthyridine-8-carboxamide | 471.1 |
| **712** | 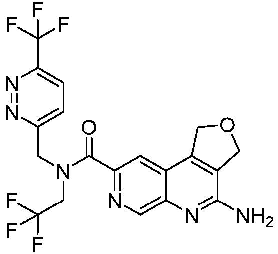 | 4-amino-N-(2,2,2-trifluoroethyl)-N-((6-(trifluoromethyl)-3-pyridazinyl)methyl)-1,3-dihydrofuro[3,4-c][1,7]naphthyridine-8-carboxamide | 473.05 |
| **713** | 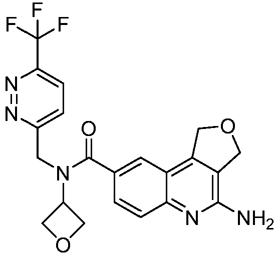 | 4-amino-N-(3-oxetanyl)-N-((6-(trifluoromethyl)-3-pyridazinyl)methyl)-1,3-dihydrofuro[3,4-c]quinoline-8-carboxamide | 446.05 |
| **714** | 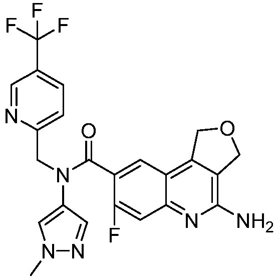 | 4-amino-7-fluoro-N-(1-methyl-1H-pyrazol-4-yl)-N-((5-(trifluoromethyl)-2-pyridinyl)methyl)-1,3-dihydrofuro[3,4-c]quinoline-8-carboxamide | 487.2 |
| **715** | 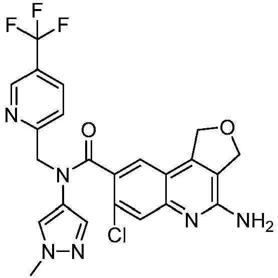 | 4-amino-7-chloro-N-(1-methyl-1H-pyrazol-4-yl)-N-((5-(trifluoromethyl)-2-pyridinyl)methyl)-1,3-dihydrofuro[3,4-c]quinoline-8-carboxamide | 503 |
| **716** | 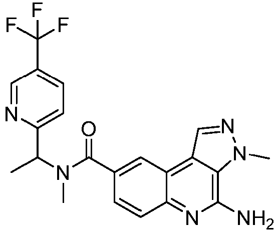 | 4-amino-N,3-dimethyl-N-((1R)-1-(5-(trifluoromethyl)-2-pyridinyl)ethyl)-3H-pyrazolo[3,4-c]quinoline-8-carboxamide and 4-amino-N,3-dimethyl-N-((1S)-1-(5-(trifluoromethyl)-2-pyridinyl)ethyl)-3H-pyrazolo[3,4-c]quinoline-8-carboxamide | 429.2 |
| **717** | 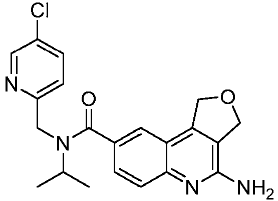 | 4-amino-N-((5-chloro-2-pyridinyl)methyl)-N-(2-propanyl)-1,3-dihydrofuro[3,4-c]quinoline-8-carboxamide | 397 |
| **718** | 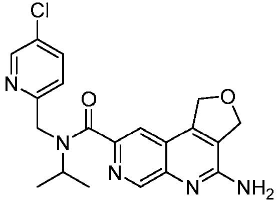 | 4-amino-N-((5-chloro-2-pyridinyl)methyl)-N-(2-propanyl)-1,3-dihydrofuro[3,4-c][1,7]naphthyridine-8-carboxamide | 398 |
| **719** | 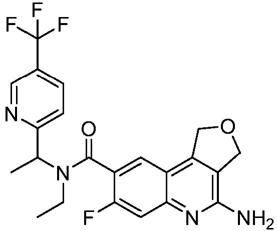 | 4-amino-N-ethyl-7-fluoro-N-((1R)-1-(5-(trifluoromethyl)-2-pyridinyl)ethyl)-1,3-dihydrofuro[3,4-c]quinoline-8-carboxamide and 4-amino-N-ethyl-7-fluoro-N-((1S)-1-(5-(trifluoromethyl)-2-pyridinyl)ethyl)-1,3-dihydrofuro[3,4-c]quinoline-8-carboxamide | 449.1 |
| **720** | 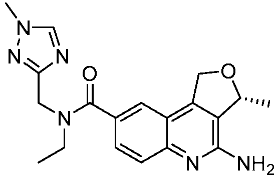 | (3R)-4-amino-N-ethyl-3-methyl-N-((1-methyl-1H-1,2,4-triazol-3-yl)methyl)-1,3-dihydrofuro[3,4-c]quinoline-8-carboxamide | 366.95 |
| **721** | 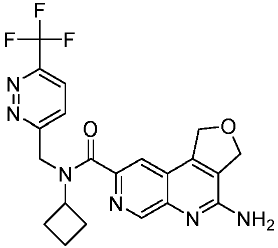 | 4-amino-N-cyclobutyl-N-((6-(trifluoromethyl)-3-pyridazinyl)methyl)-1,3-dihydrofuro[3,4-c][1,7]naphthyridine-8-carboxamide | 445.2 |
| **722** | 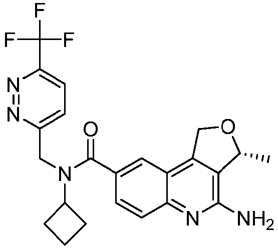 | (3R)-4-amino-N-cyclobutyl-3-methyl-N-((6-(trifluoromethyl)-3-pyridazinyl)methyl)-1,3-dihydrofuro[3,4-c]quinoline-8-carboxamide | 458.2 |
| **723** | 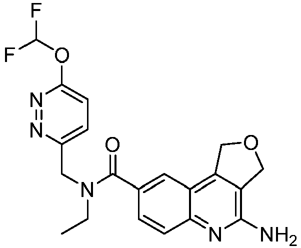 | 4-amino-N-((6-(difluoromethoxy)-3-pyridazinyl)methyl)-N-ethyl-1,3-dihydrofuro[3,4-c]quinoline-8-carboxamide | 416.2 |
| **724** | 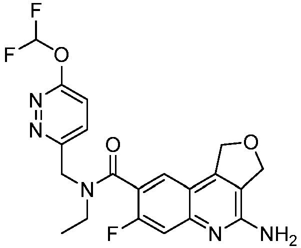 | 4-amino-N-((6-(difluoromethoxy)-3-pyridazinyl)methyl)-N-ethyl-7-fluoro-1,3-dihydrofuro[3,4-c]quinoline-8-carboxamide | 434.05 |
| **725** | 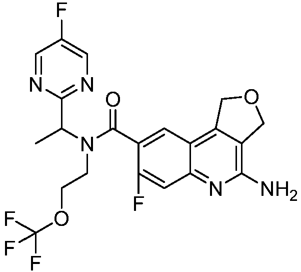 | 4-amino-7-fluoro-N-((1R)-1-(5-fluoro-2-pyrimidinyl)ethyl)-N-(2-(trifluoromethoxy)ethyl)-1,3-dihydrofuro[3,4-c]quinoline-8-carboxamide and 4-amino-7-fluoro-N-((1S)-1-(5-fluoro-2-pyrimidinyl)ethyl)-N-(2-(trifluoromethoxy)ethyl)-1,3-dihydrofuro[3,4-c]quinoline-8-carboxamide | 484.15 |
| **726** | 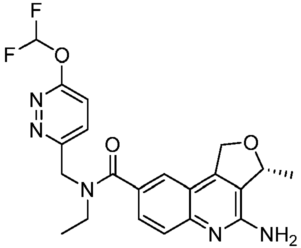 | (3R)-4-amino-N-((6-(difluoromethoxy)-3-pyridazinyl)methyl)-N-ethyl-3-methyl-1,3-dihydrofuro[3,4-c]quinoline-8-carboxamide | 430.05 |
| **727** | 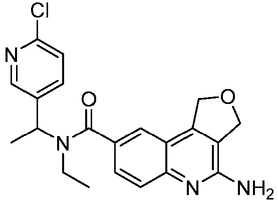 | 4-amino-N-((1R)-1-(6-chloro-3-pyridinyl)ethyl)-N-ethyl-1,3-dihydrofuro[3,4-c]quinoline-8-carboxamide and 4-amino-N-((1S)-1-(6-chloro-3-pyridinyl)ethyl)-N-ethyl-1,3-dihydrofuro[3,4-c]quinoline-8-carboxamide | 397.1 |
| **728** | 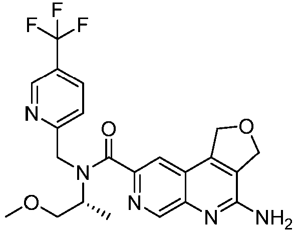 | 4-amino-N-((2R)-1-methoxy-2-propanyl)-N-((5-(trifluoromethyl)-2-pyridinyl)methyl)-1,3-dihydrofuro[3,4-c] [1,7]naphthyridine-8-carboxamide | 462 |
| **729** | 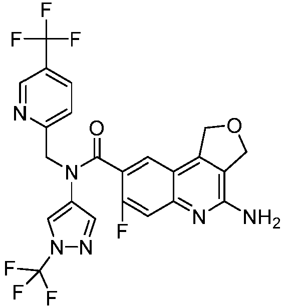 | 4-amino-7-fluoro-N-(1-(trifluoromethyl)-1H-pyrazol-4-yl)-N-((5-(trifluoromethyl)-2-pyridinyl)methyl)-1,3-dihydrofuro[3,4-c]quinoline-8-carboxamide | 541.2 |
| **730** | 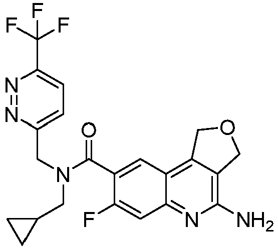 | 4-amino-N-(cyclopropylmethyl)-7-fluoro-N-((6-(trifluoromethyl)-3-pyridazinyl)methyl)-1,3-dihydrofuro[3,4-c]quinoline-8-carboxamide | 462.1 |
| **731** | 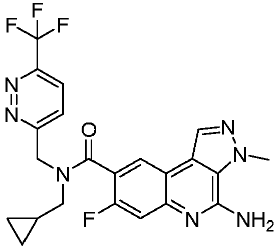 | 4-amino-N-(cyclopropylmethyl)-7-fluoro-3-methyl-N-((6-(trifluoromethyl)-3-pyridazinyl)methyl)-3H-pyrazolo[3,4-c]quinoline-8-carboxamide | 474.1 |
| **732** | 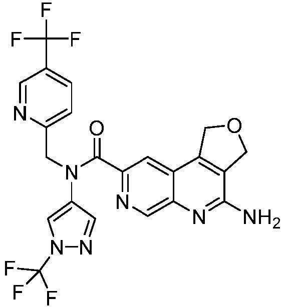 | 4-amino-N-(1-(trifluoromethyl)-1H-pyrazol-4-yl)-N-((5-(trifluoromethyl)-2-pyridinyl)methyl)-1,3-dihydrofuro[3,4-c][1,7]naphthyridine-8-carboxamide | 524 |
| **733** | 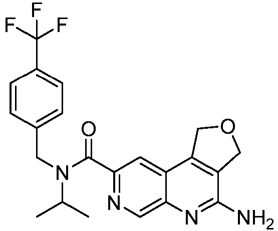 | 4-amino-N-(2-propanyl)-N-(4-(trifluoromethyl)benzyl)-1,3-dihydrofuro[3,4-c][1,7]naphthyridine-8-carboxamide | 431 |
| **734** | 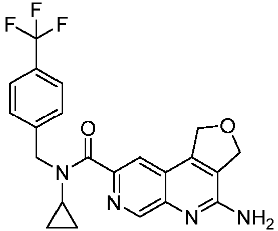 | 4-amino-N-cyclopropyl-N-(4-(trifluoromethyl)benzyl)-1,3-dihydrofuro[3,4-c][1,7]naphthyridine-8-carboxamide | 429 |
| **735** | 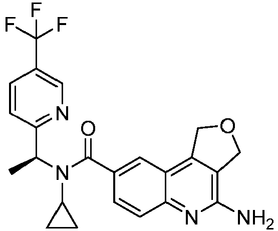 | 4-amino-N-cyclopropyl-N-((1S)-1-(5-(trifluoromethyl)-2-pyridinyl)ethyl)-1,3-dihydrofuro[3,4-c]quinoline-8-carboxamide | 443 |
| **736** | 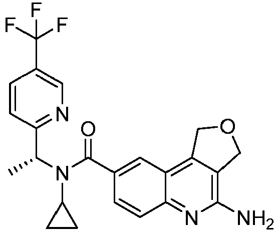 | 4-amino-N-cyclopropyl-N-((1R)-1-(5-(trifluoromethyl)-2-pyridinyl)ethyl)-1,3-dihydrofuro[3,4-c]quinoline-8-carboxamide | 443 |
| **737** | 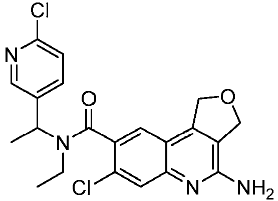 | 4-amino-7-chloro-N-((1R)-1-(6-chloro-3-pyridinyl)ethyl)-N-ethyl-1,3-dihydrofuro[3,4-c]quinoline-8-carboxamide and 4-amino-7-chloro-N-((1S)-1-(6-chloro-3-pyridinyl)ethyl)-N-ethyl-1,3-dihydrofuro[3,4-c]quinoline-8-carboxamide | 431 |
| **738** | 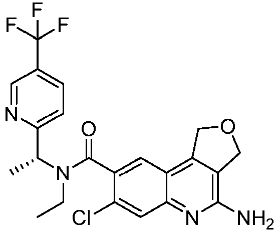 | 4-amino-7-chloro-N-ethyl-N-((1R)-1-(5-(trifluoromethyl)-2-pyridinyl)ethyl)-1,3-dihydrofuro[3,4-c]quinoline-8-carboxamide | 465.1 |
| **739** | 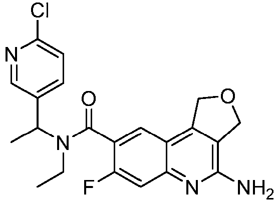 | 4-amino-N-((1R)-1-(6-chloro-3-pyridinyl)ethyl)-N-ethyl-7-fluoro-1,3-dihydrofuro[3,4-c]quinoline-8-carboxamide and 4-amino-N-((1S)-1-(6-chloro-3-pyridinyl)ethyl)-N-ethyl-7-fluoro-1,3-dihydrofuro[3,4-c]quinoline-8-carboxamide | 415 |
| **740** | 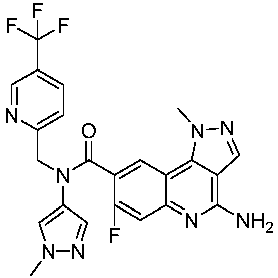 | 4-amino-7-fluoro-1-methyl-N-(1-methyl-1H-pyrazol-4-yl)-N-((5-(trifluoromethyl)-2-pyridinyl)methyl)-1H-pyrazolo[4,3-c]quinoline-8-carboxamide | 499.2 |
| **741** | 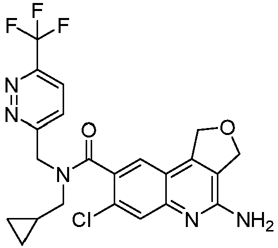 | 4-amino-7-chloro-N-(cyclopropylmethyl)-N-((6-(trifluoromethyl)-3-pyridazinyl)methyl)-1,3-dihydrofuro[3,4-c]quinoline-8-carboxamide | 478 |
| **742** | 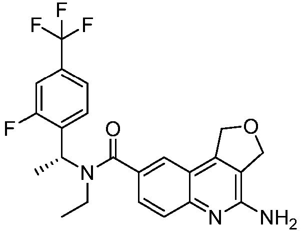 | 4-amino-N-ethyl-N-((1R)-1-(2-fluoro-4-(trifluoromethyl)phenyl)ethyl)-1,3-dihydrofuro[3,4-c]quinoline-8-carboxamide | 448.1 |
| **743** | 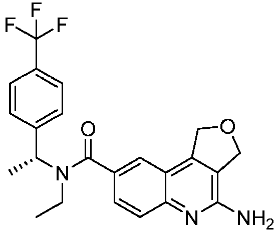 | 4-amino-N-ethyl-N-((1R)-1-(4-(trifluoromethyl)phenyl)ethyl)-1,3-dihydrofuro[3,4-c]quinoline-8-carboxamide | 430.2 |
| **744** | 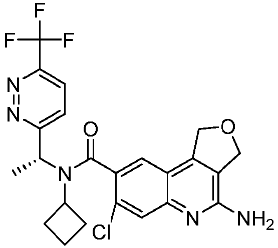 | 4-amino-7-chloro-N-cyclobutyl-N-((1R)-1-(6-(trifluoromethyl)-3-pyridazinyl)ethyl)-1,3-dihydrofuro[3,4-c]quinoline-8-carboxamide | 492.2 |
| **745** | 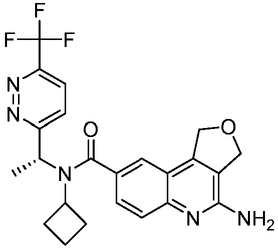 | 4-amino-N-cyclobutyl-N-((1R)-1-(6-(trifluoromethyl)-3-pyridazinyl)ethyl)-1,3-dihydrofuro[3,4-c]quinoline-8-carboxamide | 457.9 |
| **746** | 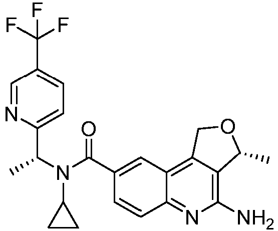 | (3R)-4-amino-N-cyclopropyl-3-methyl-N-((1R)-1-(5-(trifluoromethyl)-2-pyridinyl)ethyl)-1,3-dihydrofuro[3,4-c]quinoline-8-carboxamide | 457 |
| **747** | 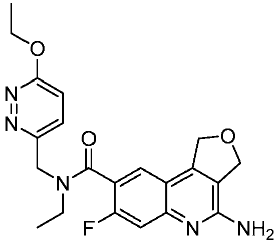 | 4-amino-N-((6-ethoxy-3-pyridazinyl)methyl)-N-ethyl-7-fluoro-1,3-dihydrofuro[3,4-c]quinoline-8-carboxamide | 412.15 |
| **748** | 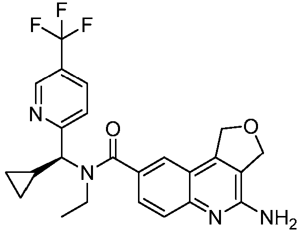 | 4-amino-N-((S)-cyclopropyl(5-(trifluoromethyl)-2-pyridinyl)methyl)-N-ethyl-1,3-dihydrofuro[3,4-c]quinoline-8-carboxamide | 457.2 |
| **749** | 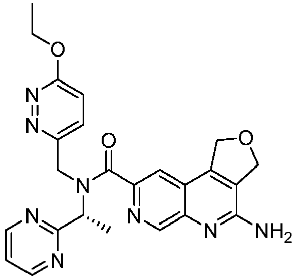 | 4-amino-N-((6-ethoxy-3-pyridazinyl)methyl)-N-((1R)-1-(2-pyrimidinyl)ethyl)-1,3-dihydrofuro[3,4-c][1,7]naphthyridine-8-carboxamide | 473.2 |
| **750** | 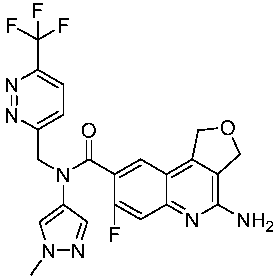 | 4-amino-7-fluoro-N-(1-methyl-1H-pyrazol-4-yl)-N-((6-(trifluoromethyl)-3-pyridazinyl)methyl)-1,3-dihydrofuro[3,4-c]quinoline-8-carboxamide | 488.2 |
| **751** | 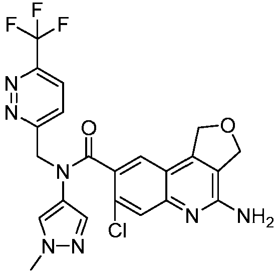 | 4-amino-7-chloro-N-(1-methyl-1H-pyrazol-4-yl)-N-((6-(trifluoromethyl)-3-pyridazinyl)methyl)-1,3-dihydrofuro[3,4-c]quinoline-8-carboxamide | 504.1 |
| **752** | 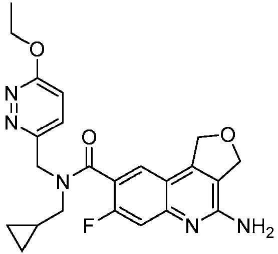 | 4-amino-N-(cyclopropylmethyl)-N-((6-ethoxy-3-pyridazinyl)methyl)-7-fluoro-1,3-dihydrofuro[3,4-c]quinoline-8-carboxamide | 438.2 |
| **753** | 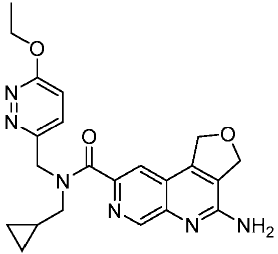 | 4-amino-N-(cyclopropylmethyl)-N-((6-ethoxy-3-pyridazinyl)methyl)-1,3-dihydrofuro[3,4-c][1,7]naphthyridine-8-carboxamide | 421.25 |
| **754** | 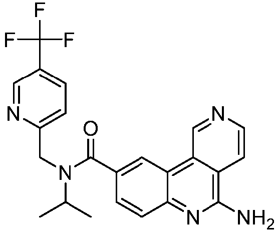 | 5-amino-N-(2-propanyl)-N-((5-(trifluoromethyl)-2-pyridinyl)methyl)benzo[c][2,6]naphthyridin e-9-carboxamide | 440.1 |
| **755** | 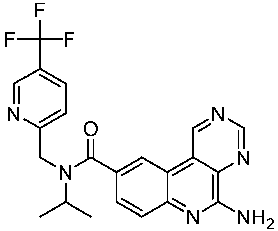 | 5-amino-N-(2-propanyl)-N-((5-(trifluoromethyl)-2-pyridinyl)methyl)pyrimido[4,5-c]quinoline-9-carboxamide | 441.2 |
| **756** | 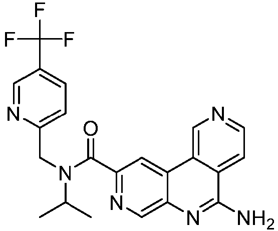 | 5-amino-N-(2-propanyl)-N-((5-(trifluoromethyl)-2-pyridinyl)methyl)pyrido[4,3-c][1,7]naphthyridine-9-carboxamide | 441.1 |
| **757** | 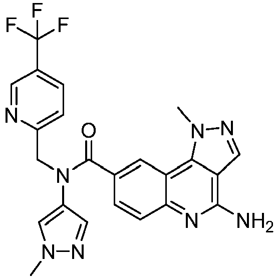 | 4-amino-1-methyl-N-(1-methyl-1H-pyrazol-4-yl)-N-((5-(trifluoromethyl)-2-pyridinyl)methyl)-1H-pyrazolo[4,3-c]quinoline-8-carboxamide | 481 |
| **758** | 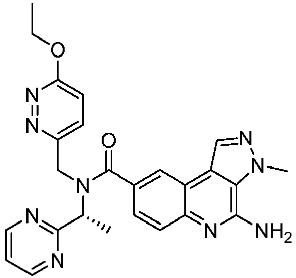 | 4-amino-N-((6-ethoxy-3-pyridazinyl)methyl)-3-methyl-N-((1R)-1-(2-pyrimidinyl)ethyl)-3H-pyrazolo[3,4-c]quinoline-8-carboxamide | 484.25 |
| **759** | 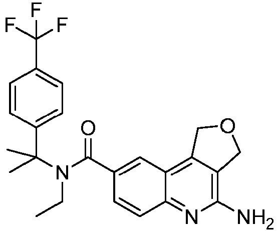 | 4-amino-N-ethyl-N-(2-(4-(trifluoromethyl)phenyl)-2-propanyl)-1,3-dihydrofuro[3,4-c]quinoline-8-carboxamide | 444.1 |
| **760** | 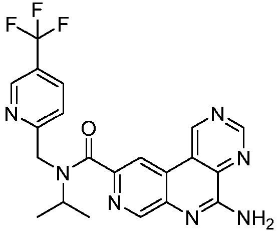 | 5-amino-N-(2-propanyl)-N-((5-(trifluoromethyl)-2-pyridinyl)methyl)pyrimido[4,5-c][1,7]naphthyridine-9-carboxamide | 442.1 |
| **761** | 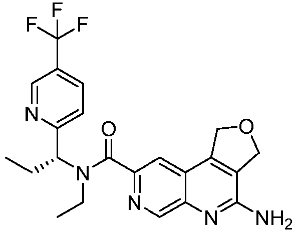 | 4-amino-N-ethyl-N-((1R)-1-(5-(trifluoromethyl)-2-pyridinyl)propyl)-1,3-dihydrofuro[3,4-c][1,7]naphthyridine-8-carboxamide | 446.2 |
| **762** | 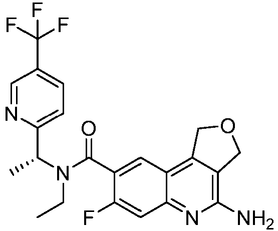 | 4-amino-N-ethyl-7-fluoro-N-((1R)-1-(5-(trifluoromethyl)-2-pyridinyl)ethyl)-1,3-dihydrofuro[3,4-c]quinoline-8-carboxamide | 449 |
| **763** | 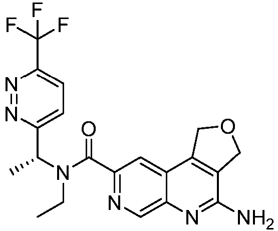 | 4-amino-N-ethyl-N-((1R)-1-(6-(trifluoromethyl)-3-pyridazinyl)ethyl)-1,3-dihydrofuro[3,4-c][1,7]naphthyridine-8-carboxamide | 443 |
| **764** | 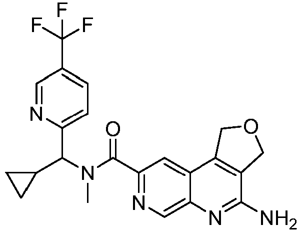 | 4-amino-N-((R)-cyclopropyl(5-(trifluoromethyl)-2-pyridinyl)methyl)-N-methyl-1,3-dihydrofuro[3,4-c][1,7]naphthyridine-8-carboxamide and 4-amino-N-((S)-cyclopropyl(5-(trifluoromethyl)-2-pyridinyl)methyl)-N-methyl-1,3-dihydrofuro[3,4-c][1,7]naphthyridine-8-carboxamide | 444.2 |
| **765** | 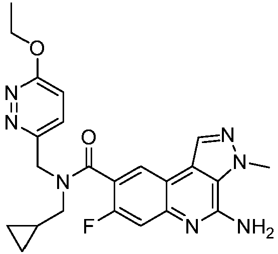 | 4-amino-N-(cyclopropylmethyl)-N-((6-ethoxy-3-pyridazinyl)methyl)-7-fluoro-3-methyl-3H-pyrazolo[3,4-c]quinoline-8-carboxamide | 450.25 |
| **766** | 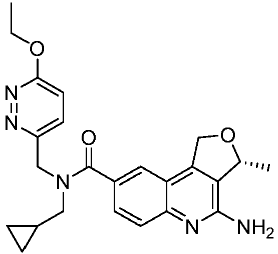 | (3R)-4-amino-N-(cyclopropylmethyl)-N-((6-ethoxy-3-pyridazinyl)methyl)-3-methyl-1,3-dihydrofuro[3,4-c]quinoline-8-carboxamide | 434.2 |
| **767** | 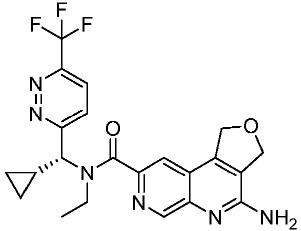 | 4-amino-N-((R)-cyclopropyl(6-(trifluoromethyl)-3-pyridazinyl)methyl)-N-ethyl-1,3-dihydrofuro[3,4-c][1,7]naphthyridine-8-carboxamide | 459.1 |
| **768** | 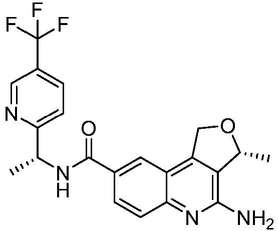 | (3R)-4-amino-3-methyl-N-((1R)-1-(5-(trifluoromethyl)-2-pyridinyl)ethyl)-1,3-dihydrofuro[3,4-c]quinoline-8-carboxamide | 417.2 |
| **769** | 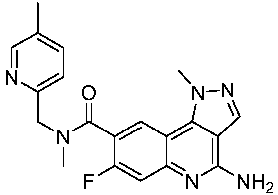 | 4-amino-7-fluoro-N, 1-dimethyl-N-((5-methyl-2-pyridinyl)methyl)-1H-pyrazolo[4,3-c]quinoline-8-carboxamide | 379 |
| **770** | 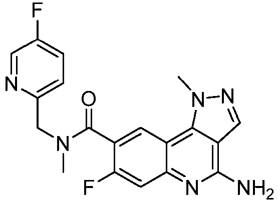 | 4-amino-7-fluoro-N-((5-fluoro-2-pyridinyl)methyl)-N,1-dimethyl-1H-pyrazolo [4,3-c]quinoline-8-carboxamide | 383.2 |
| **771** | 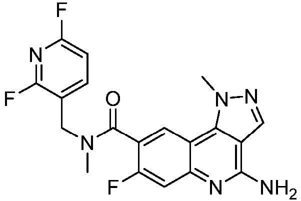 | 4-amino-N-((2,6-difluoro-3-pyridinyl)methyl)-7-fluoro-N,1-dimethyl-1H-pyrazolo[4,3-c]quinoline-8-carboxamide | 401.9 |
| **772** | 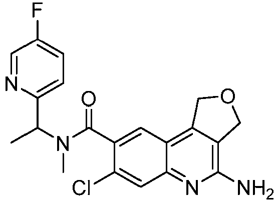 | 4-amino-7-chloro-N-((1R)-1-(5-fluoro-2-pyridinyl)ethyl)-N-methyl-1,3-dihydrofuro[3,4-c]quinoline-8-carboxamide and 4-amino-7-chloro-N-((1S)-1-(5-fluoro-2-pyridinyl)ethyl)-N-methyl-1,3-dihydrofuro[3,4-c]quinoline-8-carboxamide | 401 |
| **773** | 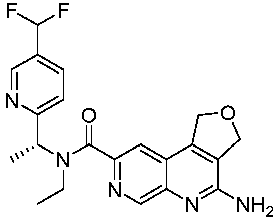 | 4-amino-N-((1R)-1-(5-(difluoromethyl)-2-pyridinyl)ethyl)-N-ethyl-1,3-dihydrofuro[3,4-c][1,7]naphthyridine-8-carboxamide | 414.1 |
| **774** | 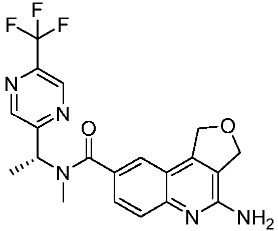 | 4-amino-N-methyl-N-((1R)-1-(5-(trifluoromethyl)-2-pyrazinyl)ethyl)-1,3-dihydrofuro[3,4-c]quinoline-8-carboxamide | 418.1 |
| **775** | 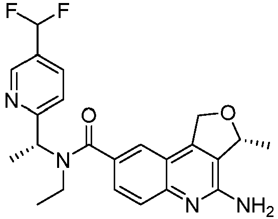 | (3R)-4-amino-N-((1R)-1-(5-(difluoromethyl)-2-pyridinyl)ethyl)-N-ethyl-3-methyl-1,3-dihydrofuro[3,4-c]quinoline-8-carboxamide | 427.1 |
| **776** | 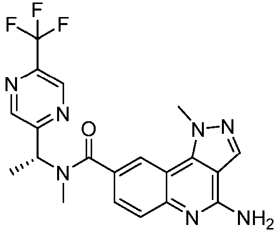 | 4-amino-N,1-dimethyl-N-((1R)-1-(5-(trifluoromethyl)-2-pyrazinyl)ethyl)-1H-pyrazolo [4,3-c]quinoline-8-carboxamide | 430.1 |
| **777** | 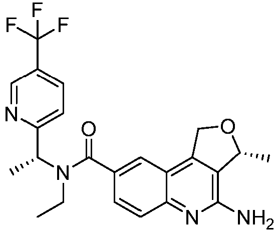 | (3R)-4-amino-N-ethyl-3-methyl-N-((1R)-1-(5-(trifluoromethyl)-2-pyridinyl)ethyl)-1,3-dihydrofuro[3,4-c]quinoline-8-carboxamide | 445.1 |
| **778** | 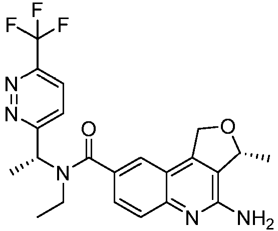 | (3R)-4-amino-N-ethyl-3-methyl-N-((1R)-1-(6-(trifluoromethyl)-3-pyridazinyl)ethyl)-1,3-dihydrofuro[3,4-c]quinoline-8-carboxamide | 446.1 |
| **779** | 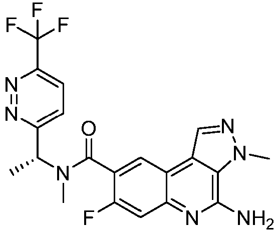 | 4-amino-7-fluoro-N,3-dimethyl-N-((1R)-1-(6-(trifluoromethyl)-3-pyridazinyl)ethyl)-3 H-pyrazolo[3,4-c]quinoline-8-carboxamide | 448.1 |
| **780** | 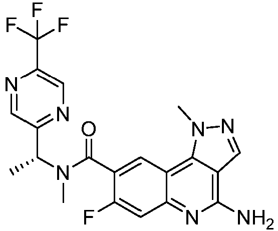 | 4-amino-7-fluoro-N,1-dimethyl-N-((1R)-1-(5-(trifluoromethyl)-2-pyrazinyl)ethyl)-1H-pyrazolo[4,3-c]quinoline-8-carboxamide | 448.1 |
| **781** | 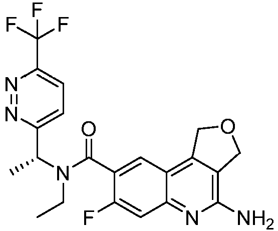 | 4-amino-N-ethyl-7-fluoro-N-((1R)-1-(6-(trifluoromethyl)-3-pyridazinyl)ethyl)-1,3-dihydrofuro[3,4-c]quinoline-8-carboxamide | 450.1 |
| **782** | 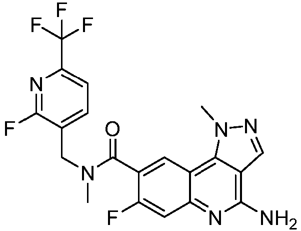 | 4-amino-7-fluoro-N-((2-fluoro-6-(trifluoromethyl)-3-pyridinyl)methyl)-N,1-dimethyl-1H-pyrazolo[4,3-c]quinoline-8-carboxamide | 451.1 |
| **783** | 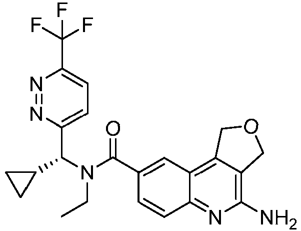 | 4-amino-N-((R)-cyclopropyl(6-(trifluoromethyl)-3-pyridazinyl)methyl)-N-ethyl-1,3-dihydrofuro[3,4-c]quinoline-8-carboxamide | 458.2 |
| **784** | 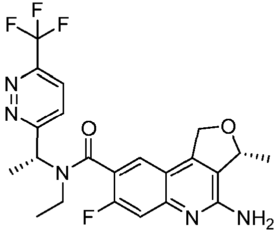 | (3R)-4-amino-N-ethyl-7-fluoro-3-methyl-N-((1R)-1-(6-(trifluoromethyl)-3-pyridazinyl)ethyl)-1,3-dihydrofuro[3,4-c]quinoline-8-carboxamide | 464.1 |
| **785** | 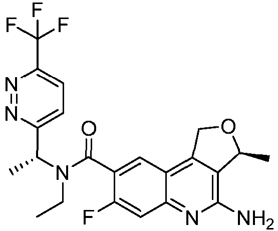 | (3S)-4-amino-N-ethyl-7-fluoro-3-methyl-N-((1R)-1-(6-(trifluoromethyl)-3-pyridazinyl)ethyl)-1,3-dihydrofuro[3,4-c]quinoline-8-carboxamide | 464.1 |
| **786** | 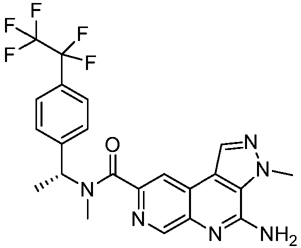 | 4-amino-N,3-dimethyl-N-((1R)-1-(4-(pentafluoroethyl)phenyl)ethyl)-3H-pyrazolo[3,4-c][1,7]naphthyridine-8-carboxamide | 479.1 |
| **787** | 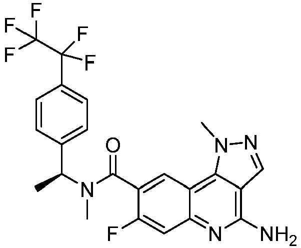 | 4-amino-7-fluoro-N,1-dimethyl-N-((1S)-1-(4-(pentafluoroethyl)phenyl)ethyl)-1H-pyrazolo[4,3-c]quinoline-8-carboxamide | 496.1 |
| **788** | 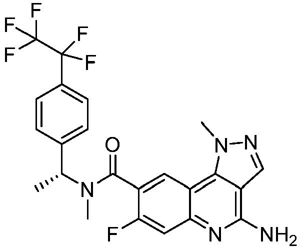 | 4-amino-7-fluoro-N,1-dimethyl-N-((1R)-1-(4-(pentafluoroethyl)phenyl)ethyl)-1H-pyrazolo[4,3-c]quinoline-8-carboxamide | 496.1 |
| **789** | 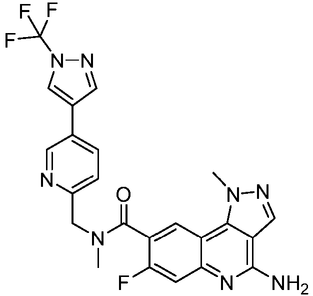 | 4-amino-7-fluoro-N,1-dimethyl-N-((5-(1-(trifluoromethyl)-1H-pyrazol-4-yl)-2-pyridinyl)methyl)-1H-pyrazolo[4,3-c]quinoline-8-carboxamide | 499.4 |
| **790** | 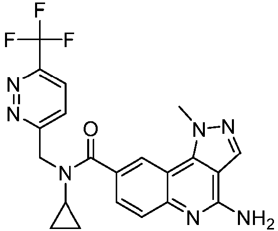 | 4-amino-N-cyclopropyl-1-methyl-N-((6-(trifluoromethyl)-3-pyridazinyl)methyl)-1H-pyrazolo[4,3-c]quinoline-8-carboxamide | 442.1 |
| **791** | 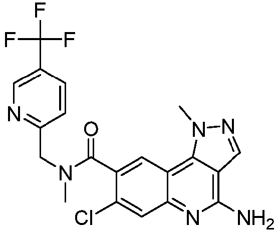 | 4-amino-7-chloro-N,1-dimethyl-N-((5-(trifluoromethyl)-2-pyridinyl)methyl)-1H-pyrazolo[4,3-c]quinoline-8-carboxamide | 449.15 |
| **792** | 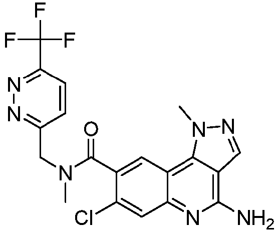 | 4-amino-7-chloro-N,1-dimethyl-N-((6-(trifluoromethyl)-3-pyridazinyl)methyl)-1H-pyrazolo[4,3-c]quinoline-8-carboxamide | 450.1 |
| **793** | 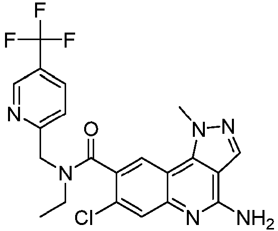 | 4-amino-7-chloro-N-ethyl-1-methyl-N-((5-(trifluoromethyl)-2-pyridinyl)methyl)-1H-pyrazolo[4,3-c]quinoline-8-carboxamide | 463.1 |
| **794** | 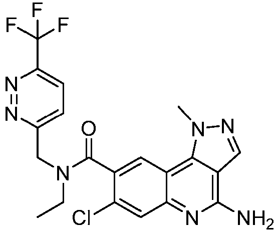 | 4-amino-7-chloro-N-ethyl-1-methyl-N-((6-(trifluoromethyl)-3-pyridazinyl)methyl)-1H-pyrazolo[4,3-c]quinoline-8-carboxamide | 464.2 |
| **795** | 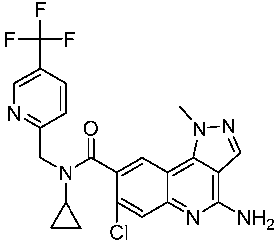 | 4-amino-7-chloro-N-cyclopropyl-1-methyl-N-((5-(trifluoromethyl)-2-pyridinyl)methyl)-1H-pyrazolo[4,3-c]quinoline-8-carboxamide | 475.1 |
| **796** | 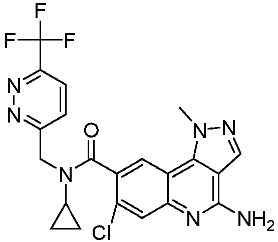 | 4-amino-7-chloro-N-cyclopropyl-1-methyl-N-((6-(trifluoromethyl)-3-pyridazinyl)methyl)-1H-pyrazolo[4,3-c]quinoline-8-carboxamide | 476.2 |
| **797** | 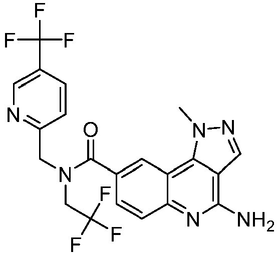 | 4-amino-1-methyl-N-(2,2,2-trifluoroethyl)-N-((5-(trifluoromethyl)-2-pyridinyl)methyl)-1H-pyrazolo[4,3-c]quinoline-8-carboxamide | 483 |
| **798** | 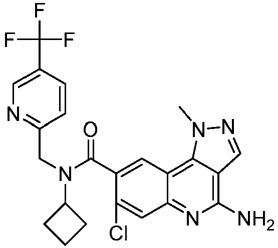 | 4-amino-7-chloro-N-cyclobutyl-1-methyl-N-((5-(trifluoromethyl)-2-pyridinyl)methyl)-1H-pyrazolo[4,3-c]quinoline-8-carboxamide | 489.2 |
| **799** | 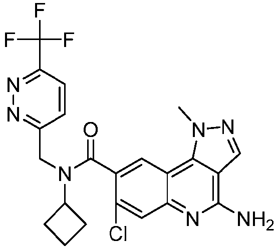 | 4-amino-7-chloro-N-cyclobutyl-1-methyl-N-((6-(trifluoromethyl)-3-pyridazinyl)methyl)-1H-pyrazolo[4,3-c]quinoline-8-carboxamide | 490.15 |
| **800** | 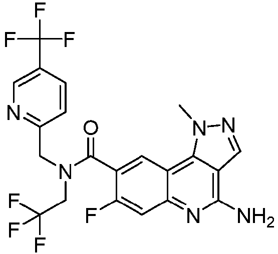 | 4-amino-7-fluoro-1-methyl-N-(2,2,2-trifluoroethyl)-N-((5-(trifluoromethyl)-2-pyridinyl)methyl)-1H-pyrazolo[4,3-c]quinoline-8-carboxamide | 501.1 |
| **801** | 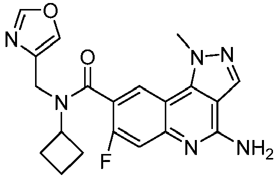 | 4-amino-N-cyclobutyl-7-fluoro-1-methyl-N-(1,3-oxazol-4-ylmethyl)-1H-pyrazolo[4,3-c]quinoline-8-carboxamide | 395 |
| **802** | 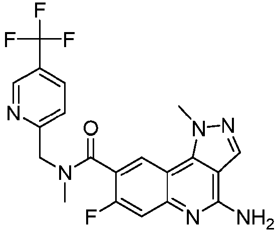 | 4-amino-7-fluoro-N,1-dimethyl-N-((5-(trifluoromethyl)-2-pyridinyl)methyl)-1H-pyrazolo[4,3-c]quinoline-8-carboxamide | 432.9 |
| **803** | 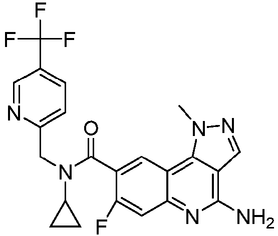 | 4-amino-N-cyclopropyl-7-fluoro-1-methyl-N-((5-(trifluoromethyl)-2-pyridinyl)methyl)-1H-pyrazolo[4,3-c]quinoline-8-carboxamide | 459 |
| **804** | 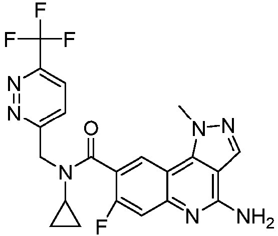 | 4-amino-N-cyclopropyl-7-fluoro-1-methyl-N-((6-(trifluoromethyl)-3-pyridazinyl)methyl)-1H-pyrazolo[4,3-c]quinoline-8-carboxamide | 459.9 |
| **805** | 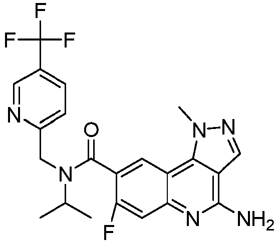 | 4-amino-7-fluoro-1-methyl-N-(2-propanyl)-N-((5-(trifluoromethyl)-2-pyridinyl)methyl)-1H-pyrazolo[4,3-c]quinoline-8-carboxamide | 460.9 |
| **806** | 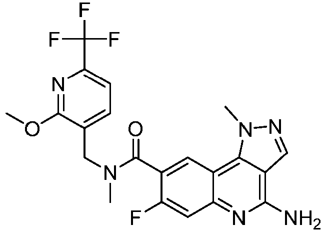 | 4-amino-7-fluoro-N-((2-methoxy-6-(trifluoromethyl)-3-pyridinyl)methyl)-N,1-dimethyl-1H-pyrazolo[4,3-c]quinoline-8-carboxamide | 462.9 |
| **807** | 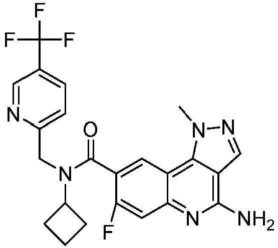 | 4-amino-N-cyclobutyl-7-fluoro-1-methyl-N-((5-(trifluoromethyl)-2-pyridinyl)methyl)-1H-pyrazolo[4,3-c]quinoline-8-carboxamide | 473 |
| **808** | 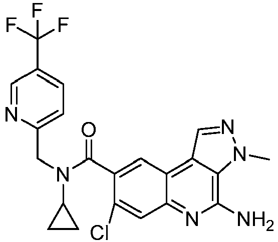 | 4-amino-7-chloro-N-cyclopropyl-3-methyl-N-((5-(trifluoromethyl)-2-pyridinyl)methyl)-3H-pyrazolo[3,4-c]quinoline-8-carboxamide | 474.9 |
| **809** | 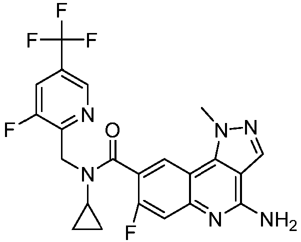 | 4-amino-N-cyclopropyl-7-fluoro-N-((3-fluoro-5-(trifluoromethyl)-2-pyridinyl)methyl)-1-methyl-1H-pyrazolo[4,3-c]quinoline-8-carboxamide | 477 |
| **810** | 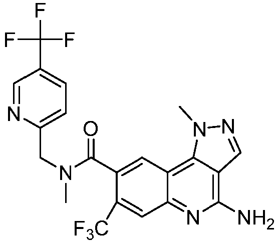 | 4-amino-N,1-dimethyl-7-(trifluoromethyl)-N-((5-(trifluoromethyl)-2-pyridinyl)methyl)-1H-pyrazolo[4,3-c]quinoline-8-carboxamide | 483.2 |
| **811** | 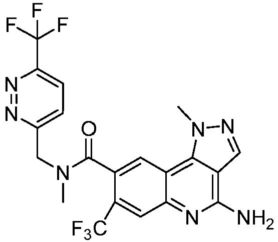 | 4-amino-N,1-dimethyl-7-(trifluoromethyl)-N-((6-(trifluoromethyl)-3-pyridazinyl)methyl)-1H-pyrazolo[4,3-c]quinoline-8-carboxamide | 484.1 |
| **812** | 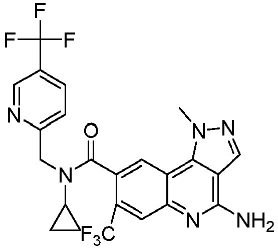 | 4-amino-N-cyclopropyl-1-methyl-7-(trifluoromethyl)-N-((5-(trifluoromethyl)-2-pyridinyl)methyl)-1H-pyrazolo[4,3-c]quinoline-8-carboxamide | 508.8 |
| **813** | 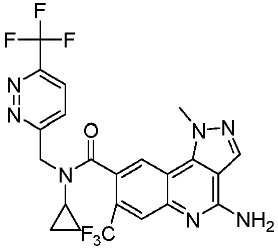 | 4-amino-N-cyclopropyl-1-methyl-7-(trifluoromethyl)-N-((6-(trifluoromethyl)-3-pyridazinyl)methyl)-1H-pyrazolo[4,3-c]quinoline-8-carboxamide | 510.2 |
| **814** | 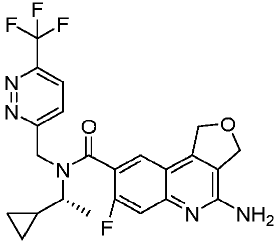 | 4-amino-N-((1R)-1-cyclopropylethyl)-7-fluoro-N-((6-(trifluoromethyl)-3-pyridazinyl)methyl)-1,3-dihydrofuro[3,4-c]quinoline-8-carboxamide | 476.05 |
| **815** | 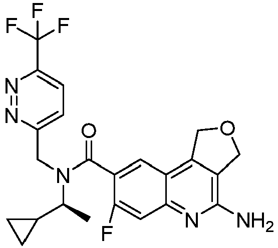 | 4-amino-N-((1S)-1-cyclopropylethyl)-7-fluoro-N-((6-(trifluoromethyl)-3-pyridazinyl)methyl)-1,3-dihydrofuro[3,4-c]quinoline-8-carboxamide | 476.05 |
| **816** | 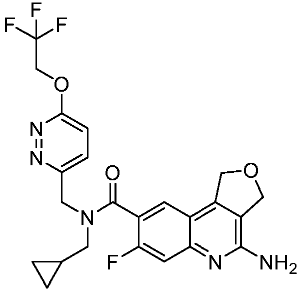 | 4-amino-N-(cyclopropylmethyl)-7-fluoro-N-((6-(2,2,2-trifluoroethoxy)-3-pyridazinyl)methyl)-1,3-dihydrofuro[3,4-c]quinoline-8-carboxamide | 492.1 |
| **817** | 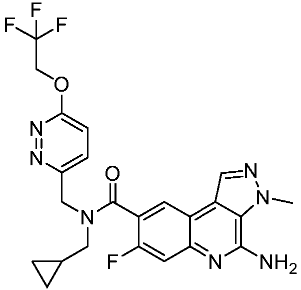 | 4-amino-N-(cyclopropylmethyl)-7-fluoro-3-methyl-N-((6-(2,2,2-trifluoroethoxy)-3-pyridazinyl)methyl)-3H-pyrazolo[3,4-c]quinoline-8-carboxamide | 504.1 |
| **818** | 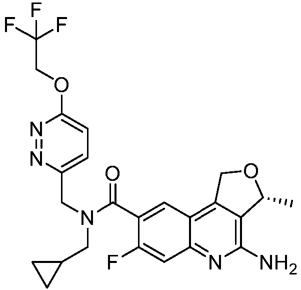 | (3R)-4-amino-N-(cyclopropylmethyl)-7-fluoro-3-methyl-N-((6-(2,2,2-trifluoroethoxy)-3-pyridazinyl)methyl)-1,3-dihydrofuro[3,4-c]quinoline-8-carboxamide | 506.2 |

### Example 819: (S)-4-amino-N-((5-cyanopyridin-2-yl)methyl)-N-(1-fluoropropan-2-yl)-1,3-dihydrofuro[3,4-c][1,7]naphthyridine-8-carboxamide.

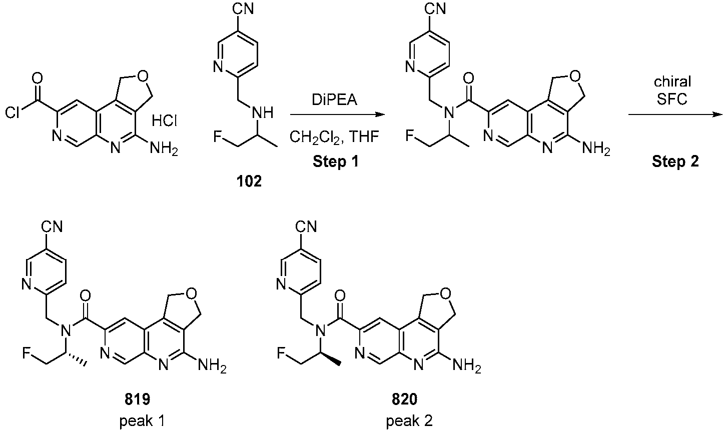

To a stirred ice-cooled solution of 6-(((1-fluoropropan-2-yl)amino)methyl)nicotinonitrile **(102,** 80 mg, 0.41 mmol) and N-ethyl-N-isopropylpropan-2-amine (107 mg, 0.145 mL, 0.828 mmol, Aldrich) in DCM (1 mL) and tetrahydrofuran (1 mL) was added 4-amino-1,3-dihydrofuro[3,4-c][1,7]naphthyridine-8-carbonyl chloride (derived from acid **217**, 119 mg, 0.476 mmol) in one portion as a solid. The resulting mixture was stirred at 0°C for 1 h. The crude mixture was directly loaded on a silica gel precolumn (25 g) and subjected to combi-flash column chromatography on a 12-g ISCO gold column, eluting with MeOH (with 0.5% ammonium hydroxide)/DCM (15 min from 0 to 18%) to give 4-amino-N-((5-cyanopyridin-2-yl)methyl)-N-(1-fluoropropan-2-yl)-1,3-dihydrofuro[3,4-c][1,7]naphthyridine-8-carboxamide (100 mg, 0.246 mmol, 59.4% yield) as a white solid.

The racemic product was purified via preparative SFC using a Chiral Technologies OJ column (250 X 21 mm, 5mm) with a mobile phase of 80% Liquid CO₂ and 20% MeOH with 0.2% TEA using a flowrate of 80 mL/min. Stereochemistry was arbitrarily assigned. The 1^{st} eluting peak was assigned (S)-4-amino-N-((5-cyanopyridin-2-yl)methyl)-N-(1-fluoropropan-2-yl)-1,3-dihydrofuro [3,4-c][1,7]naphthyridine-8-carboxamide (48.6 mg, 0.120 mmol, 28.9% yield), obtained as an off-white solid. The 1^{st} eluting peak was arbitrarily assigned as (S)-4-amino-N-((5-cyanopyridin-2-yl)methyl)-N-(1-fluoropropan-2-yl)-1,3-dihydrofuro[3,4-c][1,7]naphthyridine-8-carboxamide and the 2^{nd} eluting peak was arbitrarily assigned as (R)-4-amino-N-((5-cyanopyridin-2-yl)methyl)-N-(1-fluoropropan-2-yl)-1,3-dihydrofuro[3,4-c][1,7]naphthyridine-8-carboxamide (43.881 mg, 0.108 mmol, 26.1 % yield), obtained as an off-white solid. *m*/*z* (ESI): 407.2 (M+H)⁺. ¹H NMR (DMSO-d6, 500 MHz) δ 8.6-9.1 (m, 2H), 8.1-8.3 (m, 1H), 7.5-7.8 (m, 2H), 6.9-7.2 (m, 2H), 5.2-5.4 (m, 2H), 4.3-5.1 (m, 7H), 3.2-3.3 (m, 1H), 1.20 (br d, 3H, J=6.4 Hz). ¹H NMR (METHANOL-d4 with some CDCl3, 400 MHz) δ 8.7-9.0 (m, 2H), 7.9-8.1 (m, 1H), 7.6-7.8 (m, 2H), 5.3-5.5 (m, 2H), 5.15 (br s, 2H), 4.8-5.1 (m, 2H), 4.7-4.8 (m, 1H), 4.3-4.6 (m, 2H), 1.2-1.4 (m, 3H). ¹⁹F NMR (METHANOL-d4, 376 MHz) δ -227.9--221.0 (m, 1F).

Compounds in Table 23 were prepared in a manner similar to that described above for Example **819** and **820.**

**Table 23**

| **Ex.** | **Structure** | **Name** | **m/z (ESI): (M+H)⁺** | **SFC conditions** |
|---|---|---|---|---|
| **821** | 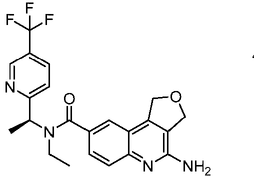 | 4-amino-N-ethyl-N-((1S)-1-(5-(trifluoromethyl)-2-pyridinyl)ethyl)-1,3-dihydrofuro[3,4-c]quinoline-8-carboxamide | 431.2 | 1st peak, Regis (S,S) Whelk-01 column (250 X 21 mm, 5mm) with a mobile phase of 70% Liquid CO₂ and 30% MeOH with 0.2% TEA using a flowrate of 80 mL/min |
| **822** | 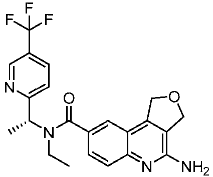 | 4-amino-N-ethyl-N-((1R)-1-(5-(trifluoromethyl)-2-pyridinyl)ethyl)-1,3-dihydrofuro[3,4-c]quinoline-8-carboxamide | 431.2 | 2nd peak, Regis (S,S) Whelk-01 column (250 X 21 mm, 5mm) with a mobile phase of 70% Liquid CO₂ and 30% MeOH with 0.2% TEA using a flowrate of 80 mL/min |
| **823** | 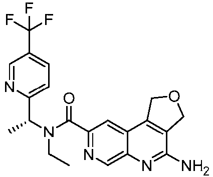 | 4-amino-N-ethyl-N-((1R)-1-(5-(trifluoromethyl)-2-pyridinyl)ethyl)-1,3-dihydrofuro[3,4-c][1,7]naphthyridine-8-carboxamide | 432.2 | 1st peak, Chiralcel OZ-H column (250 X 21 mm, 5µm) with a mobile phase of 65% Liquid CO₂ and 35% MeOH and TEA using a flowrate of 60 mL/min |
| **824** | 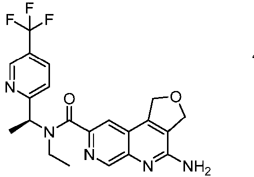 | 4-amino-N-ethyl-N-((1S)-1-(5-(trifluoromethyl)-2-pyridinyl)ethyl)-1,3-dihydrofuro[3,4-c] [1,7]naphthyridine-8-carboxamide | 432.2 | 2nd peak, Chiralcel OZ-H column (250 X 21 mm, 5µm) with a mobile phase of 65% Liquid CO₂ and 35% MeOH and TEA using a flowrate of 60 mL/min |
| **825** | 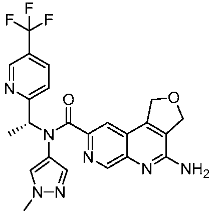 | 4-amino-N-(1-methyl-1H-pyrazol-4-yl)-N-((1R)-1-(5-(trifluoromethyl)-2-pyridinyl)ethyl)-1,3-dihydrofuro[3,4-c][1,7]naphthyridine-8-carboxamide | 484.2 | 1st peak, Regis (S,S) Whelk-01 column (250 X 21 mm) with a mobile phase of 70% Liquid CO₂ and 30% MeOH with 0.2% TEA |
| **826** | 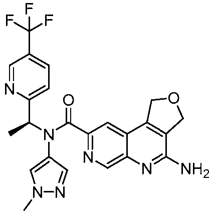 | 4-amino-N-(1-methyl-1H-pyrazol-4-yl)-N-((1S)-1-(5-(trifluoromethyl)-2-pyridinyl)ethyl)-1,3-dihydrofuro[3,4-c][1,7]naphthyridine-8-carboxamide | 484.2 | 2nd peak, Regis (S,S) Whelk-01 column (250 X 21 mm) with a mobile phase of 70% Liquid CO₂ and 30% MeOH with 0.2% TEA |
| **827** | 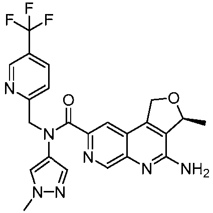 | (3S)-4-amino-3-methyl-N-(1-methyl-1H-pyrazol-4-yl)-N-((5-(trifluoromethyl)-2-pyridinyl)methyl)-1,3-dihydrofuro[3,4-c][1,7]naphthyridine-8-carboxamide | 484.2 | 1st peak, ChiralPak OZ (250 X 21 mm, 5 µm) with a mobile phase of 70% Liquid CO₂ and 30% MeOH with 0.2% TEA |
| **828** | 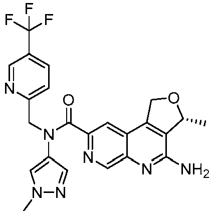 | (3R)-4-amino-3-methyl-N-(1-methyl-1H-pyrazol-4-yl)-N-((5-(trifluoromethyl)-2-pyridinyl)methyl)-1,3-dihydrofuro[3,4-c][1,7]naphthyridine-8-carboxamide | 484.2 | 2nd peak, ChiralPak OZ (250 X 21 mm, 5 µm) with a mobile phase of 70% Liquid CO₂ and 30% MeOH with 0.2% TEA |
| **829** | 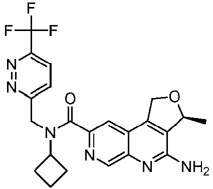 | (3S)-4-amino-N-cyclobutyl-3-methyl-N-((6-(trifluoromethyl)-3-pyridazinyl)methyl)-1,3-dihydrofuro[3,4-c][1,7]naphthyridine-8-carboxamide | 458.8 | 1st peak, Chiral Technologies AS column (250 X 21 mm, 5mm) with a mobile phase of 85% Liquid CO₂ and 15% MeOH with 0.2% TEA using a flowrate of 70 mL/min |
| **830** | 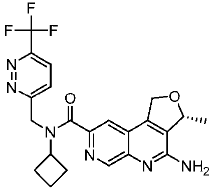 | (3R)-4-amino-N-cyclobutyl-3-methyl-N-((6-(trifluoromethyl)-3-pyridazinyl)methyl)-1,3-dihydrofuro[3,4-c][1,7]naphthyridine-8-carboxamide | 458.8 | 2nd peak, Chiral Technologies AS column (250 X 21 mm, 5mm) with a mobile phase of 85% Liquid CO₂ and 15% MeOH with 0.2% TEA using a flowrate of 70 mL/min |
| **831** | 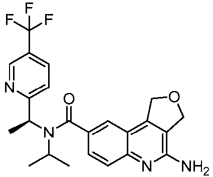 | 4-amino-N-(2-propanyl)-N-((1S)-1-(5-(trifluoromethyl)-2-pyridinyl)ethyl)-1,3-dihydrofuro[3,4-c]quinoline-8-carboxamide | 445.0 | 1st peak, preparative SFC using a Regis (S,S) Whelk-01column (250 X 21 mm, 5mm) with a mobile phase of 70% Liquid CO₂ and 30% MeOH with 0.2% TEA using a flowrate of 80 mL/min |
| **832** | 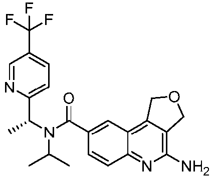 | 4-amino-N-(2-propanyl)-N-((1R)-1-(5-(trifluoromethyl)-2-pyridinyl)ethyl)-1,3-dihydrofuro[3,4-c]quinoline-8-carboxamide | 445.0 | 2nd peak, preparative SFC using a Regis (S,S) Whelk-01column (250 X 21 mm, 5mm) with a mobile phase of 70% Liquid CO₂ and 30% MeOH with 0.2% TEA using a flowrate of 80 mL/min |
| **833** | 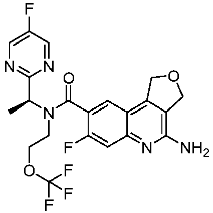 | 4-amino-7-fluoro-N-((1S)-1-(5-fluoro-2-pyrimidinyl)ethyl)-N-(2-(trifluoromethoxy)ethyl)-1,3-dihydrofuro[3,4-c]quinoline-8-carboxamide | 484.2 | 1st peak, Chiral Technologies OX column (250 X 21 mm, 5mm) with a mobile phase of 50% Liquid CO₂ and 50% MeOH with 0.2% TEA using a flowrate of 50 mL/min |
| **834** | 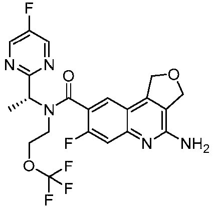 | 4-amino-7-fluoro-N-((1R)-1-(5-fluoro-2-pyrimidinyl)ethyl)-N-(2-(trifluoromethoxy)ethyl)-1,3-dihydrofuro[3,4-c]quinoline-8-carboxamide | 484.2 | 2nd peak, Chiral Technologies OX column (250 X 21 mm, 5mm) with a mobile phase of 50% Liquid CO₂ and 50% MeOH with 0.2% TEA using a flowrate of 50 mL/min |
| **835** | 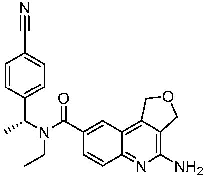 | 4-amino-N-((1R)-1-(4-cyanophenyl)ethyl)-N-ethyl-1,3-dihydrofuro[3,4-c]quinoline-8-carboxamide | 387.0 | 1st peak, Chiral Technologies OX column (250 X 21 mm, 5mm) with a mobile phase of 50% Liquid CO₂ and 50% MeOH with 0.2% TEA using a flowrate of 50 mL/min |
| **836** | 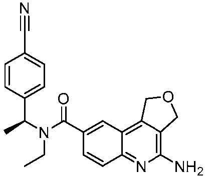 | 4-amino-N-((1S)-1-(4-cyanophenyl)ethyl)-N-ethyl-1,3-dihydrofuro[3,4-c]quinoline-8-carboxamide | 387.0 | 2nd peak, Chiral Technologies OX column (250 X 21 mm, 5mm) with a mobile phase of 50% Liquid CO₂ and 50% MeOH with 0.2% TEA using a flowrate of 50 mL/min |
| **837** | 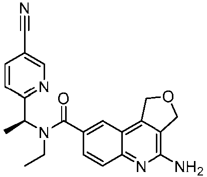 | 4-amino-N-((1S)-1-(5-cyano-2-pyridinyl)ethyl)-N-ethyl-1,3-dihydrofuro[3,4-c]quinoline-8-carboxamide | 388.0 | 1st peak, Chiral Technologies OX column (250 X 21 mm, 5mm) with a mobile phase of 50% Liquid CO₂ and 50% MeOH with 0.2% TEA using a flowrate of 50 mL/min |
| **838** | 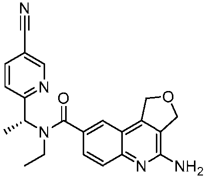 | 4-amino-N-((1R)-1-(5-cyano-2-pyridinyl)ethyl)-N-ethyl-1,3-dihydrofuro[3,4-c]quinoline-8-carboxamide | 388.0 | 2nd peak, Chiral Technologies OX column (250 X 21 mm, 5mm) with a mobile phase of 50% Liquid CO₂ and 50% MeOH with 0.2% TEA using a flowrate of 50 mL/min |
| **839** | 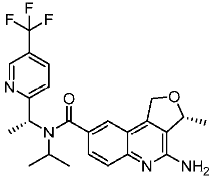 | (3R)-4-amino-3-methyl-N-(2-propanyl)-N-((1R)-1-(5-(trifluoromethyl)-2-pyridinyl)ethyl)-1,3-dihydrofuro[3,4-c]quinoline-8-carboxamide | 459.0 | 1st peak, Chiral Technologies IG column (250 X 21 mm, 5mm) with a mobile phase of 60% Liquid CO₂ and 40% MeOH with 0.2% TEA using a flowrate of 70 mL/min |
| **840** | 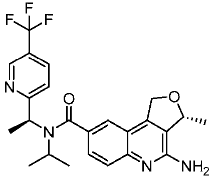 | (3R)-4-amino-3-methyl-N-(2-propanyl)-N-((1S)-1-(5-(trifluoromethyl)-2-pyridinyl)ethyl)-1,3-dihydrofuro[3,4-c]quinoline-8-carboxamide | 459.0 | 2nd peak, Chiral Technologies IG column (250 X 21 mm, 5mm) with a mobile phase of 60% Liquid CO₂ and 40% MeOH with 0.2% TEA using a flowrate of 70 mL/min |
| **841** | 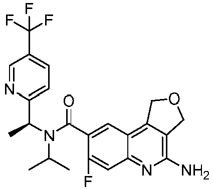 | 4-amino-7-fluoro-N-(2-propanyl)-N-((1S)-1-(5-(trifluoromethyl)-2-pyridinyl)ethyl)-1,3-dihydrofuro[3,4-c]quinoline-8-carboxamide | 463.0 | 1st peak, Purified by Prep SFC using column Whelk-O-S,S (250 X 21 mm, 5µm) with a mobile phase of 75% Liquid CO₂ and 25% MeOH and 0.2%TEA using a flowrate 80 mL/min |
| **842** | 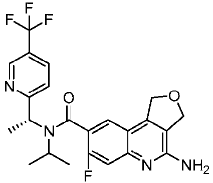 | 4-amino-7-fluoro-N-(2-propanyl)-N-((1R)-1-(5-(trifluoromethyl)-2-pyridinyl)ethyl)-1,3-dihydrofuro[3,4-c]quinoline-8-carboxamide | 463.0 | 2nd peak, Purified by Prep SFC using column Whelk-O-S,S (250 X 21 mm, 5µm) with a mobile phase of 75% Liquid CO₂ and 25% MeOH and 0.2%TEA using a flowrate 80 mL/min |
| **843** | 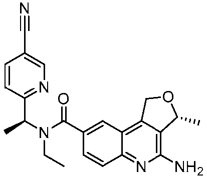 | (3R)-4-amino-N-((1S)-1-(5-cyano-2-pyridinyl)ethyl)-N-ethyl-3-methyl-1,3-dihydrofuro[3,4-c]quinoline-8-carboxamide | 402.0 | 1st peak, Chiral Technologies IG column (250 X 21 mm, 5mm) X 2 with a mobile phase of 85% Liquid CO₂ and 15% MeOH with 0.2% TEA using a flowrate of 70 mL/min |
| **844** | 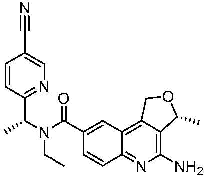 | (3R)-4-amino-N-((1R)-1-(5-cyano-2-pyridinyl)ethyl)-N-ethyl-3-methyl-1,3-dihydrofuro[3,4-c]quinoline-8-carboxamide | 402.0 | 2nd peak, Chiral Technologies IG column (250 X 21 mm, 5mm) X 2 with a mobile phase of 85% Liquid CO₂ and 15% MeOH with 0.2% TEA using a flowrate of 70 mL/min |
| **845** | 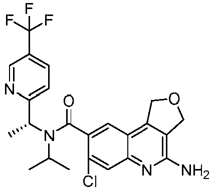 | 4-amino-7-chloro-N-(2-propanyl)-N-((1R)-1-(5-(trifluoromethyl)-2-pyridinyl)ethyl)-1,3-dihydrofuro[3,4-c]quinoline-8-carboxamide | 479.0 | 1st peak, Purified by Prep SFC using column Chiralpak IC (250 X 21 mm, 5µm) with a mobile phase of 65% Liquid CO₂ and 35% MeOH and 0.2%TEA using a flowrate 70 mL/min |
| **846** | 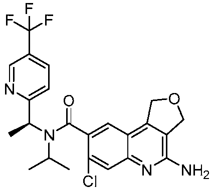 | 4-amino-7-chloro-N-(2-propanyl)-N-((1S)-1-(5-(trifluoromethyl)-2-pyridinyl)ethyl)-1,3-dihydrofuro[3,4-c]quinoline-8-carboxamide | 479.0 | 2nd peak, Purified by Prep SFC using column Chiralpak IC (250 X 21 mm, 5µm) with a mobile phase of 65% Liquid CO₂ and 35% MeOH and 0.2%TEA using a flowrate 70 mL/min |
| **847** | 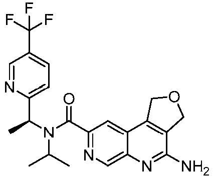 | 4-amino-N-(2-propanyl)-N-((1S)-1-(5-(trifluoromethyl)-2-pyridinyl)ethyl)-1,3-dihydrofuro[3,4-c][1,7]naphthyridine-8-carboxamide | 446.0 | 1st peak, Regis (S,S) Whelk-01 column (250 X 21 mm, 5mm) with a mobile phase of 80% Liquid CO₂ and 20% EtOH with 0.2% TEA using a flowrate of 80 mL/min |
| **848** | 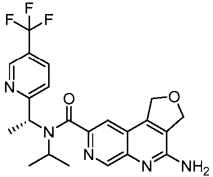 | 4-amino-N-(2-propanyl)-N-((1R)-1-(5-(trifluoromethyl)-2-pyridinyl)ethyl)-1,3-dihydrofuro[3,4-c][1,7]naphthyridine-8-carboxamide | 446.0 | 2nd peak, Regis (S,S) Whelk-01 column (250 X 21 mm, 5mm) with a mobile phase of 80% Liquid CO₂ and 20% EtOH with 0.2% TEA using a flowrate of 80 mL/min |
| **849** | 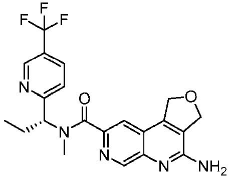 | 4-amino-N-methyl-N-((1R)-1-(5-(trifluoromethyl)-2-pyridinyl)propyl)-1,3-dihydrofuro[3,4-c][1,7]naphthyridine-8-carboxamide | 432.1 | 1st peak, Purified by Prep SFC using column Chiralcel OX (250 X 21 mm, 5µm) with a mobile phase of 70% Liquid CO₂ and 30% MeOH and 0.2%TEA using a flowrate 100 mL/min |
| **850** | 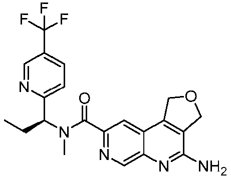 | 4-amino-N-methyl-N-((1S)-1-(5-(trifluoromethyl)-2-pyridinyl)propyl)-1,3-dihydrofuro[3,4-c][1,7]naphthyridine-8-carboxamide | 432.1 | 2nd peak, Purified by Prep SFC using column Chiralcel OX (250 X 21 mm, 5 µm) with a mobile phase of 70% Liquid CO₂ and 30% MeOH and 0.2%TEA using a flowrate 100 mL/min |
| **851** | 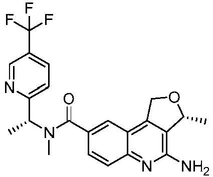 | (3R)-4-amino-N,3-dimethyl-N-((1R)-1-(5-(trifluoromethyl)-2-pyridinyl)ethyl)-1,3-dihydrofuro[3,4-c]quinoline-8-carboxamide | 431.0 | 1st peak, Regis (S,S) Whelk-01 column (250 X 21 mm, 5mm) with a mobile phase of 60% Liquid CO₂ and 40% MeOH with 0.2% TEA using a flowrate of 80 mL/min |
| **852** | 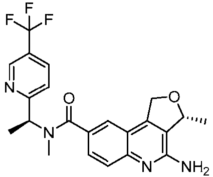 | (3R)-4-amino-N,3-dimethyl-N-((1S)-1-(5-(trifluoromethyl)-2-pyridinyl)ethyl)-1,3-dihydrofuro[3,4-c]quinoline-8-carboxamide | 431.2 | 2nd peak, Regis (S,S) Whelk-01 column (250 X 21 mm, 5mm) with a mobile phase of 60% Liquid CO₂ and 40% MeOH with 0.2% TEA using a flowrate of 80 mL/min |
| **853** | 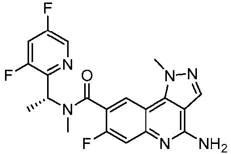 | 4-amino-N-((1R)-1-(3,5-difluoro-2-pyridinyl)ethyl)-7-fluoro-N,1-dimethyl-1H-pyrazolo[4,3-c]quinoline-8-carboxamide | 415 | 2nd peak, Chiralcel OD column (2 x 25 cm, 5 micron) with a mobile phase of 80% Liquid CO₂ and 20% methanol with 0.15% triethylamine using a flowrate of 50 mL/min |
| **854** | 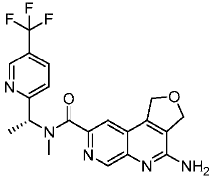 | 4-amino-N-methyl-N-((1R)-1-(5-(trifluoromethyl)-2-pyridinyl)ethyl)-1,3-dihydrofuro[3,4-c][1,7]naphthyridine-8-carboxamide | 418.2 | 1st peak, Chiral Technologies OX column (250 X 21 mm, 5mm) with a mobile phase of 65% Liquid CO₂ and 35% MeOH with 0.2% TEA using a flowrate of 70 mL/min |
| **855** | 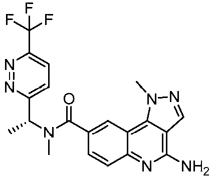 | 4-amino-N,1-dimethyl-N-((1R)-1-(6-(trifluoromethyl)-3-pyridazinyl)ethyl)-1H-pyrazolo[4,3-c]quinoline-8-carboxamide | 430.1 | 1st peak, Chiralpak AD column (21 x 250 mm) with a mobile phase of 75% Liquid CO₂ and 25% EtOH with 0.2% diethylamine using a flowrate of 80 mL/min |
| **856** | 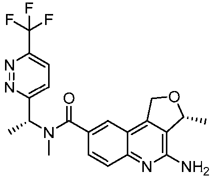 | (3R)-4-amino-N,3-dimethyl-N-((1R)-1-(6-(trifluoromethyl)-3-pyridazinyl)ethyl)-1,3-dihydrofuro[3,4-c]quinoline-8-carboxamide | 432 | 2nd peak, Chiral Technologies OX column (250 X 30 mm, 5mm) with a mobile phase of 80% Liquid CO₂ and 20% MeOH with 0.2% TEA using a flowrate of 170 mL/min |
| **857** | 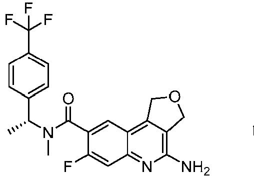 | 4-amino-7-fluoro-N-methyl-N-((1R)-1-(4-(trifluoromethyl)phenyl)e thyl)-1,3-dihydrofuro[3,4-c]quinoline-8-carboxamide | 433.9 | 1st peak, Chiralpak AZ column (21 x 250mm, 5 µm) with a mobile phase of 55% Liquid CO₂ and 45% MeOH with 0.2% DEA using a flowrate of 80 mL/min |
| **858** | 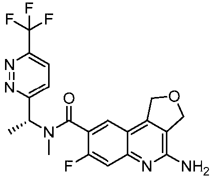 | 4-amino-7-fluoro-N-methyl-N-((1R)-1-(6-(trifluoromethyl)-3-pyridazinyl)ethyl)-1,3-dihydrofuro[3,4-c]quinoline-8-carboxamide | 436.2 | 1st peak, Whelk-O S,S column (250 X 21 mm, 5um) with a mobile phase of 65% Liquid CO₂ and 35% methanol with 0.2%TEA using a flowrate 100 mL/min |
| **859** | 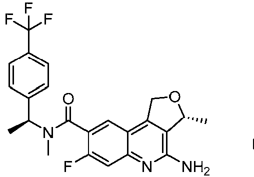 | (3R)-4-amino-7-fluoro-N,3-dimethyl-N-((1S)-1-(4-(trifluoromethyl)phenyl)e thyl)-1,3-dihydrofuro[3,4-c]quinoline-8-carboxamide | 448.1 | 2nd peak, Chiralpak ID column (21 x 250 mm, 5 micron) with a mobile phase of 75% Liquid CO₂ and 25% MeOH with 0.2% diethylamine using a flowrate of 80 mL/min |
| **860** | 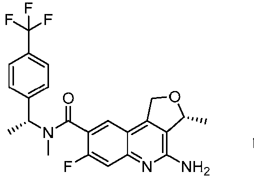 | (3R)-4-amino-7-fluoro-N,3-dimethyl-N-((1R)-1-(4-(trifluoromethyl)phenyl)e thyl)-1,3-dihydrofuro[3,4-c]quinoline-8-carboxamide | 448.1 | 1st peak, Chiralpak ID column (21 x 250 mm, 5 micron) with a mobile phase of 75% Liquid CO₂ and 25% MeOH with 0.2% diethylamine using a flowrate of 80 mL/min |
| **861** | 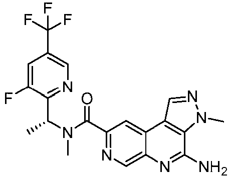 | 4-amino-N-((1R)-1-(3-fluoro-5-(trifluoromethyl)-2-pyridinyl)ethyl)-N,3-dimethyl-3H-pyrazolo[3,4-c][1,7]naphthyridine-8-carboxamide | 448.2 | 1st peak, Lux Cellulose-2 column (2 x 15 cm, 5 micron) with a mobile phase of 65% Liquid CO₂ and 35% methanol with 0.1% diethylamine using a flowrate of 60 mL/min |
| **862** | 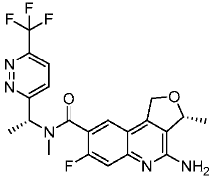 | (3R)-4-amino-7-fluoro-N,3-dimethyl-N-((1R)-1-(6-(trifluoromethyl)-3-pyridazinyl)ethyl)-1,3-dihydrofuro[3,4-c]quinoline-8-carboxamide | 450.2 | 1st peak, Whelk-O S,S column (250 X 21 mm, 5um) with a mobile phase of 70% Liquid CO₂ and 30% methanol with 0.2%TEA using a flowrate 100 mL/min |
| **863** | 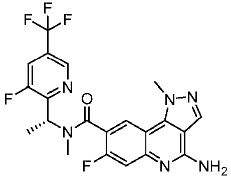 | 4-amino-7-fluoro-N-((1R)-1-(3-fluoro-5-(trifluoromethyl)-2-pyridinyl)ethyl)-N,1-dimethyl-1H-pyrazolo[4,3-c]quinoline-8-carboxamide | 465.1 | 1st peak, (S,S) Whelk-O column (2 x 25 cm, 5 micron) with a mobile phase of 75% Liquid CO₂ and 25% methanol with 0.2 triethylamine using a flowrate of 65 mL/min |
| **864** | 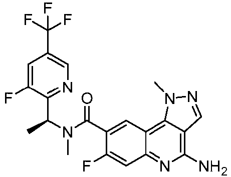 | 4-amino-7-fluoro-N-((1S)-1-(3-fluoro-5-(trifluoromethyl)-2-pyridinyl)ethyl)-N,1-dimethyl-1H-pyrazolo[4,3-c]quinoline-8-carboxamide | 465.1 | 2nd peak, (S,S) Whelk-O column (2 x 25 cm, 5 micron) with a mobile phase of 75% Liquid CO₂ and 25% methanol with 0.2 triethylamine using a flowrate of 65 mL/min |
| **865** | 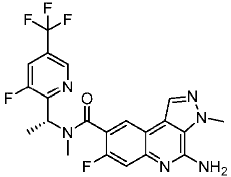 | 4-amino-7-fluoro-N-((1R)-1-(3-fluoro-5-(trifluoromethyl)-2-pyridinyl)ethyl)-N,3-dimethyl-3H-pyrazolo[3,4-c]quinoline-8-carboxamide | 465.1 | 1st peak, Enantiocel AS-5 column (2 x 25 cm, 5 micron) with a mobile phase of 60% Liquid CO₂ and 40% methanol with 0.1% diethyalmine using a flowrate of 60 mL/min |
| **866** | 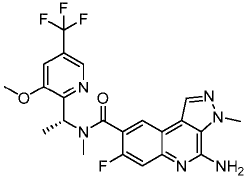 | 4-amino-7-fluoro-N-((1R)-1-(3-methoxy-5-(trifluoromethyl)-2-pyridinyl)ethyl)-N,3-dimethyl-3H-pyrazolo[3,4-c]quinoline-8-carboxamide | 477.2 | 1st peak, Chiralpak IG column (2 x 25 cm, 5 micron) with a mobile phase of 50% Liquid CO₂ and 50% methanol with 0.1% diethylamine using a flowrate of 60 mL/min |
| **867** | 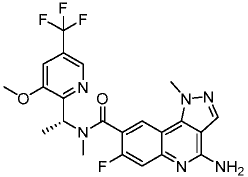 | 4-amino-7-fluoro-N-((1R)-1-(3-methoxy-5-(trifluoromethyl)-2-pyridinyl)ethyl)-N,1-dimethyl-1H-pyrazolo[4,3-c]quinoline-8-carboxamide | 477.2 | 2nd peak, ChiralPak IC column (2 x 15 cm 5 µm) with a mobile phase of 65% Liquid CO₂ and 35% MeOH with 0.2% diethylamine using a flowrate of 60 mL/min |
| **868** | 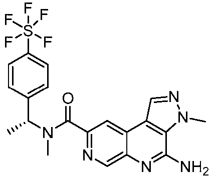 | 4-amino-N,3-dimethyl-N-((1R)-1-(4-(pentafluoro-lambda~6~-sulfanyl)phenyl)ethyl)-3H-pyrazolo[3,4-c][1,7]naphthyridine-8-carboxamide | 487.2 | 1st peak, Chiralcel OD column (2 x 25 cm, 5 micron) with a mobile phase of 65% Liquid CO₂ and 35% methanol with 01.% deithylamine using a flowrate of 60 mL/min |
| **869** | 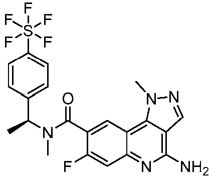 | 4-amino-7-fluoro-N,1-dimethyl-N-((1S)-1-(4-(pentafluoro-lambda~6~-sulfanyl)phenyl)ethyl)-1H-pyrazolo[4,3-c]quinoline-8-carboxamide | 504.1 | 2nd peak, (S,S) Whelk-O column (2 x 25 cm, 5 micron) with a mobile phase of 70% Liquid CO₂ and 30% methanol with 0.2 triethylamine using a flowrate of 65 mL/min |
| **870** | 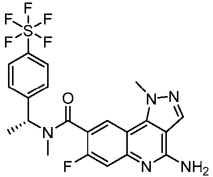 | 4-amino-7-fluoro-N,1-dimethyl-N-((1R)-1-(4-(pentafluoro-lambda~6~-sulfanyl)phenyl)ethyl)-1H-pyrazolo[4,3-c]quinoline-8-carboxamide | 504.1 | 1st peak, (S,S) Whelk-O column (2 x 25 cm, 5 micron) with a mobile phase of 70% Liquid CO₂ and 30% methanol with 0.2 triethylamine using a flowrate of 65 mL/min |
| **871** | 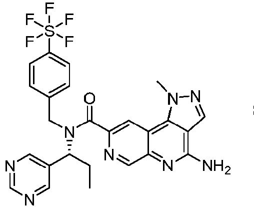 | 4-amino-1-methyl-N-(4-(pentafluoro-lambda~6~-sulfanyl)benzyl)-N-((1R)-1-(5-pyrimidinyl)propyl)-1H-pyrazolo[4,3-c][1,7]naphthyridine-8-carboxamide | 579.2 | 1st peak, Chiralpak IC column (21x150 mm) with a mobile phase of 55% Liquid CO₂ and 45% methanol with 0.2% diethylamine using a flow rate of 80 mL/min |
| **872** | 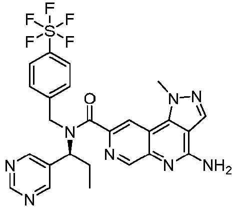 | 4-amino-1-methyl-N-(4-(pentafluoro-lambda~6~-sulfanyl)benzyl)-N-((1S)-1-(5-pyrimidinyl)propyl)-1H-pyrazolo[4,3-c][1,7]naphthyridine-8-carboxamide | 579.2 | 2nd peak, Chiralpak IC column (21x150 mm) with a mobile phase of 55% Liquid CO₂ and 45% methanol with 0.2% diethylamine using a flow rate of 80 mL/min |
| **873** | 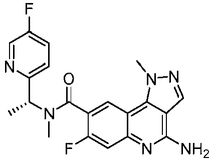 | 4-amino-7-fluoro-N-((1R)-1-(5-fluoro-2-pyridinyl)ethyl)-N,1-dimethyl-1H-pyrazolo[4,3-c]quinoline-8-carboxamide | 397.2 | 1st peak, Chiralpak IC column (2 x 15 cm, 5 micron) with a mobile phase of 60% Liquid CO₂ and 40% isopropanol with 0.1% diethylamine using a flowrate of 65 mL/min |
| **874** | 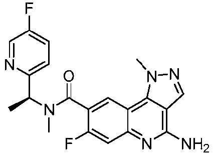 | 4-amino-7-fluoro-N-((1S)-1-(5-fluoro-2-pyridinyl)ethyl)-N,1-dimethyl-1H-pyrazolo[4,3-c]quinoline-8-carboxamide | 397.2 | 2nd peak, Chiralpak IC column (2 x 15 cm, 5 micron) with a mobile phase of 60% Liquid CO₂ and 40% isopropanol with 0.1% diethylamine using a flowrate of 65 mL/min |
| **875** | 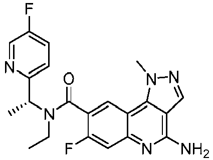 | 4-amino-N-ethyl-7-fluoro-N-((1R)-1-(5-fluoro-2-pyridinyl)ethyl)-1-methyl-1H-pyrazolo[4,3-c]quinoline-8-carboxamide | 411.2 | 1st peak, Chiralpak IC column (2 x 15 cm, 5 micron) with a mobile phase of 55% Liquid CO₂ and 45% isopropanol with 0.1% diethylamine using a flowrate of 65 mL/min |
| **876** | 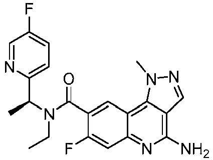 | 4-amino-N-ethyl-7-fluoro-N-((1S)-1-(5-fluoro-2-pyridinyl)ethyl)-1-methyl-1H-pyrazolo[4,3-c]quinoline-8-carboxamide | 411.2 | 2nd peak, Chiralpak IC column (2 x 15 cm, 5 micron) with a mobile phase of 55% Liquid CO₂ and 45% isopropanol with 0.1% diethylamine using a flowrate of 65 mL/min |
| **877** | 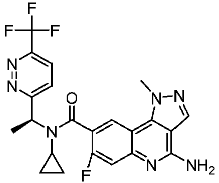 | 4-amino-N-cyclopropyl-7-fluoro-1-methyl-N-((1S)-1-(6-(trifluoromethyl)-3-pyridazinyl)ethyl)-1H-pyrazolo[4,3-c]quinoline-8-carboxamide | 474 | 1st peak, Chiralpak AZ column (21x250 mm) with a mobile phase of 70% Liquid CO₂ and 30% methanol with 0.2% diethylamine using a flow rate of 80 mL/min |
| **878** | 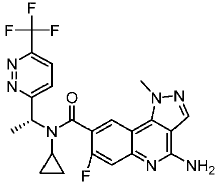 | 4-amino-N-cyclopropyl-7-fluoro-1-methyl-N-((1R)-1-(6-(trifluoromethyl)-3-pyridazinyl)ethyl)-1H-pyrazolo[4,3-c]quinoline-8-carboxamide | 474.2 | 2nd peak, Chiralpak AZ column (21x250 mm) with a mobile phase of 70% Liquid CO₂ and 30% methanol with 0.2% diethylamine using a flow rate of 80 mL/min |
| **879** | 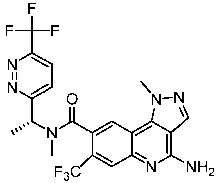 | 4-amino-N,1-dimethyl-7-(trifluoromethyl)-N-((1R)-1-(6-(trifluoromethyl)-3-pyridazinyl)ethyl)-1H-pyrazolo[4,3-c]quinoline-8-carboxamide | 498.2 | 1st peak, Chiralpak IG 21x 500 mm, 5 micron) with a mobile phase of 75% Liquid CO₂ and 25% 2-propanol with 0.2% triethylamine using a flowrate of 55 mL/min |
| **880** | 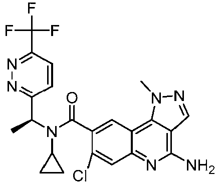 | 4-amino-7-chloro-N-cyclopropyl-1-methyl-N-((1S)-1-(6-(trifluoromethyl)-3-pyridazinyl)ethyl)-1H-pyrazolo[4,3-c]quinoline-8-carboxamide | 490.1 | 2nd peak, (S,S) Whelk-O column (2 x 25 cm, 5 micron) with a mobile phase of 70% Liquid CO₂ and 30% methanol with 0.1% diethylamine using a flowrate of 60 mL/min |
| **881** | 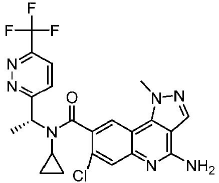 | 4-amino-7-chloro-N-cyclopropyl-1-methyl-N-((1R)-1-(6-(trifluoromethyl)-3-pyridazinyl)ethyl)-1H-pyrazolo [4,3-c]quinoline-8-carboxamide | 490.1 | 1st peak, (S,S) Whelk-O column (2 x 25 cm, 5 micron) with a mobile phase of 70% Liquid CO₂ and 30% methanol with 0.1% diethylamine using a flowrate of 60 mL/min |

### Example 882: 4-amino-N-(1-methyl-1H-pyrazol-4-yl)-N-(4-(trifluoromethyl)benzyl)-1,3-dihydrofuro[3,4-c][1,7]naphthyridine-8-carboxamide 2,2,2-trifluoroacetate.

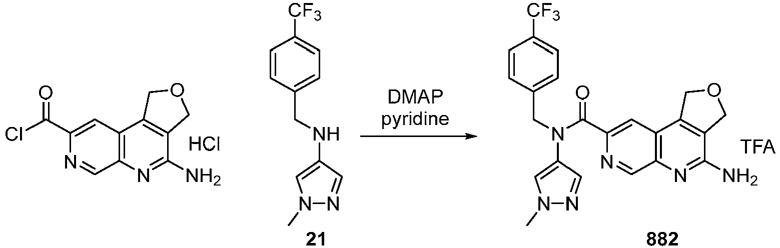

4-amino-1,3-dihydrofuro[3,4-c][1,7]naphthyridine-8-carbonyl chloride hydrochloride (168 mg, 0.588 mmol) prepared as above, was added to a stirred suspension of 1-methyl-N-(4-(trifluoromethyl)benzyl)-1H-pyrazol-4-amine **(21,** 100 mg, 0.392 mmol) and N,N-dimethylpyridin-4-amine (14.36 mg, 0.118 mmol, Aldrich) in pyridine (930 mg, 1000 µL, 11.75 mmol, Sigma-Aldrich Corporation). The mixture was heated in an oil bath for 6 h at 60 °C. The resulting residue was partitioned between 50 mL of EtOAc and 5 mL of 1 N NaOH. The organic layer was concentrated and the residue was purified via reverse phase HPLC (10% to 80% CH_{3C}N in water with 0.1% TFA) to give 4-amino-N-(1-methyl-1H-pyrazol-4-yl)-N-(4-(trifluoromethyl)benzyl)-1,3-dihydrofuro[3,4-c][1,7]naphthyridine-8-carboxamide 2,2,2-trifluoroacetate **(882,** 100 mg, 0.172 mmol, 43.8 % yield) as a brown solid. *m*/*z* (ESI): 469.0 (M+H)⁺. ¹H NMR (METHANOL-d₄, 400 MHz) δ 8.88 (s, 1H), 7.88 (s, 1H), 7.68 (m, 2H), 7.60 (m, 2H), 7.40 (s, 1H), 7.12 (s, 1H), 5.47 (m, 2H), 5.1-5.2 (m, 4H), 3.65 (s, 3H). ¹⁹F NMR (METHANOL-d₄, 376 MHz) δ -64.04 (s, 3F), -77.30 (s, 3F).

Compounds in Table 24 were prepared in a manner similar to that described above for Example **882.**

**Table 24**

| **Ex.** | **Structure** | **Name** | **m/z (ESI): (M+H)⁺** |
|---|---|---|---|
| **883** | 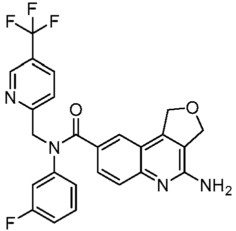 | 4-amino-N-(3-fluorophenyl)-N-((5-(trifluoromethyl)-2-pyridinyl)methyl)-1,3-dihydrofuro[3,4-c]quinoline-8-carboxamide | 483.2 |
| **884** | 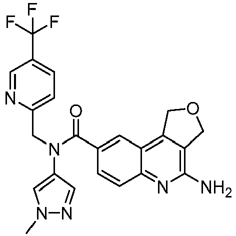 | 4-amino-N-(1-methyl-1H-pyrazol-4-yl)-N-((5-(trifluoromethyl)-2-pyridinyl)methyl)-1,3-dihydrofuro[3,4-c]quinoline-8-carboxamide | 469.2 |
| **885** | 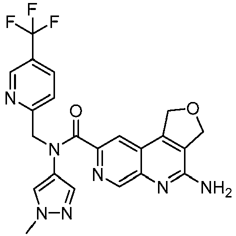 | 4-amino-N-(1-methyl-1H-pyrazol-4-yl)-N-((5-(trifluoromethyl)-2-pyridinyl)methyl)-1,3-dihydrofuro[3,4-c] [1,7]naphthyridine-8-carboxamide | 470.2 |
| **886** | 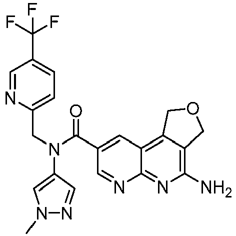 | 4-amino-N-(1-methyl-1H-pyrazol-4-yl)-N-((5-(trifluoromethyl)-2-pyridinyl)methyl)-1,3-dihydrofuro[3,4-c] [1,8]naphthyridine-8-carboxamide | 470.0 |

### Example 887: 5-amino-N-[(1R)-1-pyrimidin-2-ylethyl]-N-[[5-(trifluoromethyl)-2-pyridyl]methyl]-2,4-dihydro-1H-pyrano[3,4-c]quinoline-9-carboxamide.

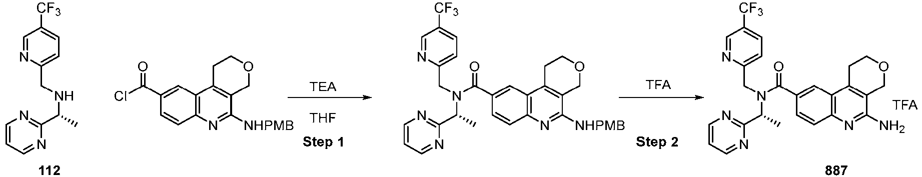

Step 1. (R)-1-(pyrimidin-2-yl)-N-((5-(trifluoromethyl)pyridin-2-yl)methyl)ethan-1-amine **(112,** 0.123 g, 0.436 mmol) and triethylamine (0.240 g, 0.334 mL, 2.376 mmol, Sigma-Aldrich Corporation) were added to a stirred mixture of crude 5-((4-methoxybenzyl)amino)-1,4-dihydro-2H-pyrano[3,4-c]quinoline-9-carbonyl chloride (0.152 g, 0.396 mmol, from acid **258)** in tetrahydrofuran (3 mL). The reaction mixture was stirred at rt for 15 min. The reaction mixture was diluted with EtOAc (75 mL) and washed with saturated aqueous NH₄Cl (50 mL). The organic layer was separated, washed with brine (50 mL), dried over MgSO₄, filtered, and concentrated in vacuo to give crude (R)-5-((4-methoxybenzyl)amino)-N-(1-(pyrimidin-2-yl)ethyl)-N-((5-(trifluoromethyl)pyridin-2-yl)methyl)-1,4-dihydro-2H-pyrano[3,4-c]quinoline-9-carboxamide as a brown oil that was used directly in the next step, assuming 100% yield. *m*/*z* (ESI): 629.1 (M+H)⁺.

Step 2. Crude (R)-5-((4-methoxybenzyl)amino)-N-(1-(pyrimidin-2-yl)ethyl)-N-((5-(trifluoromethyl)pyridin-2-yl)methyl)-1,4-dihydro-2H-pyrano[3,4-c]quinoline-9-carboxamide (249 mg, 0.396 mmol) was mixed in trifluoroacetic acid (1530 mg, 1 mL, 13.42 mmol, Sigma-Aldrich Corporation). The reaction mixture was stirred at 60 °C for 24 h then concentrated and purified on a XBridge column (19x100mm, 5um) using 0.1% NH₄OH in H₂O (A) and ACN (B) as mobile phase. (R)-5-amino-N-(1-(pyrimidin-2-yl)ethyl)-N-((5-(trifluoromethyl)pyridin-2-yl)methyl)-1,4-dihydro-2H-pyrano[3,4-c]quinoline-9-carboxamide 2,2,2-trifluoroacetate (80 mg, 0.129 mmol, 32.5 % yield) was isolated as a brown solid. *m*/*z* (ESI): 509.2 [M + H]⁺.

### Example 888: 4-amino-N-[(1R)-1-pyrimidin-2-ylethyl]-N-[[5-(trifluoromethyl)-2-pyridyl]methyl]-2,3-dihydrofuro[3,2-c]quinoline-8-carboxamide.

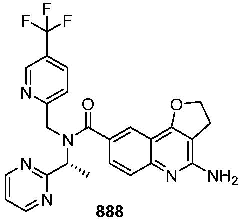

Example 888 was prepared in a manner similar to that described above for Example **887.** *m*/*z* (ESI): 495.0 (M+H)⁺

### Example 889: 2-amino-N-(cyclobutylmethyl)-3-methyl-N-[[5-(trifluoromethyl)-2-pyridyl]methyl]quinoline-6-carboxamide

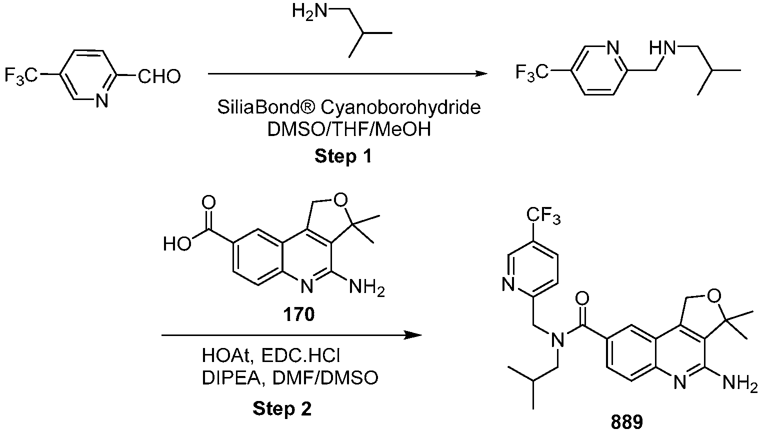

Step 1. A solution of isobutylamine (1 eq, 100 mM in dry DMSO) and a solution of 5-(trifluoromethyl)picolinaldehyde (1 eq, 100 mM in dry DMSO) were mixed together with equal amounts of dry THF and dry MeOH (25 mM final conc) and MS 3Å. The mixture was shaken at rt. Thereafter, SiliaBond^{®} Cyanoborohydride (2.5 eq) was added and the reaction mixture was shaken at rt. The reaction mixture was filtered, and the filter-cake was rinsed with CH₃CN. The combined washings and filtrate were concentrated under reduced pressure to give 2-methyl-N-((5-(trifluoromethyl)pyridin-2-yl)methyl)propan-1-amine.

Step 2. 4-amino-3,3-dimethyl-1,3-dihydrofuro[3,4-c]quinoline-8-carboxylic acid **(170,** 1 eq, 100 mM in dry DMSO), HOAt (1 eq, 100 mM in dry DMSO) and a solution of EDC and DIPEA (100 mM and 200 mM, respectively in dry DMF) were added in sequence to the crude mixture of amine. The reaction was then shaken at rt overnight and then concentrated under reduced pressure to give crude product that was purified by HPLC to yield the final product, 4-amino-N-isobutyl-3,3-dimethyl-N-[[5-(trifluoromethyl)-2-pyridyl|methyl]-1H-furo[3,4-c]quinoline-8-carboxamide with 99 % purity by UV. *m*/*z* (ESI): 473.2 (M+H)⁺.

Compounds in Table 25 were prepared in a manner similar to that described above for Example 889.

**Table 25**

| **Ex.** | **Structure** | **Name** | **m/z (ESI): (M+H)⁺** |
|---|---|---|---|
| **890** | 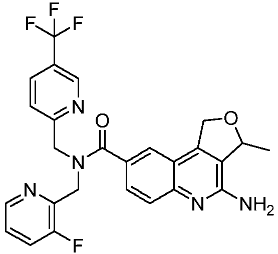 | 4-amino-N-((3-fluoropyridin-2-yl)methyl)-3-methyl-N-((5-(trifluoromethyl)pyridin-2-yl)methyl)-1,3-dihydrofuro[3,4-c] quinoline-8-carboxamide | 512.2 |
| **891** | 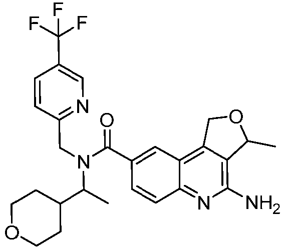 | 4-amino-3-methyl-N-(1-(tetrahydro-2H-pyran-4-yl)ethyl)-N-((5-(trifluoromethyl)pyridin-2-yl)methyl)-1,3-dihydrofuro[3,4-c]quinoline-8-carboxamide | 515.2 |
| **892** | 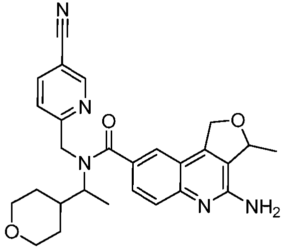 | 4-amino-N-((5-cyanopyridin-2-yl)methyl)-3-methyl-N-(1-(tetrahydro-2H-pyran-4-yl)ethyl)-1,3-dihydrofuro[3,4-c] quinoline-8-carboxamide | 472.2 |
| **893** | 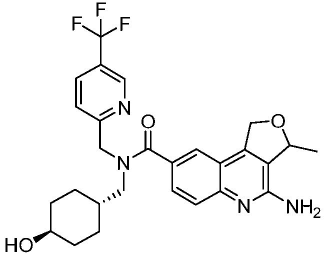 | 4-amino-N-(((1r,4r)-4-hydroxycyclohexyl)methyl)-3-methyl-N-((5-(trifluoromethyl)pyridin-2-yl)methyl)-1,3-dihydrofuro[3,4-c]quinoline-8-carboxamide | 515.2 |
| **894** | 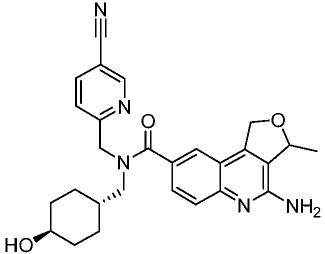 | 4-amino-N-((5-cyanopyridin-2-yl)methyl)-N-(((1r,4r)-4-hydroxycyclohexyl)methyl)-3-methyl-1,3-dihydrofuro[3,4-c]quinoline-8-carboxamide | 472.2 |
| **895** | 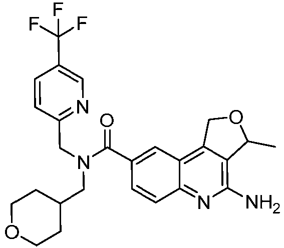 | 4-amino-3-methyl-N-((tetrahydro-2H-pyran-4-yl)methyl)-N-((5-(trifluoromethyl)pyridin-2-yl)methyl)-1,3-dihydrofuro[3,4-c]quinoline-8-carboxamide | 501.2 |
| **896** | 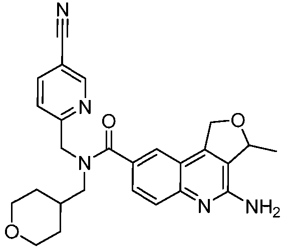 | 4-amino-N-((5-cyanopyridin-2-yl)methyl)-3-methyl-N-((tetrahydro-2H-pyran-4-yl)methyl)-1,3-dihydrofuro[3,4-c]quinoline-8-carboxamide | 458.2 |
| **897** | 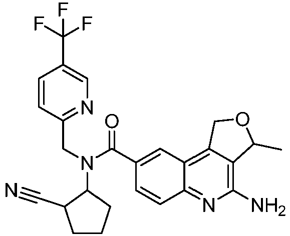 | 4-amino-N-(2-cyanocyclopentyl)-3-methyl-N-((5-(trifluoromethyl)pyridin-2-yl)methyl)-1,3-dihydrofuro[3,4-c]quinoline-8-carboxamide | 496.2 |
| **898** | 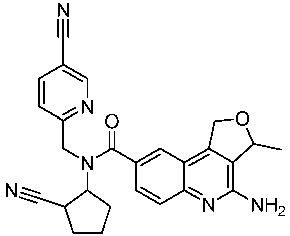 | 4-amino-N-(2-cyanocyclopentyl)-N-((5-cyanopyridin-2-yl)methyl)-3-methyl-1,3-dihydrofuro[3,4-c]quinoline-8-carboxamide | 453.2 |
| **899** | 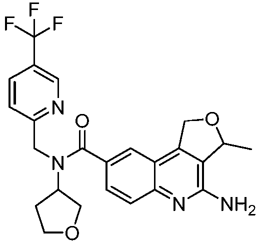 | 4-amino-3-methyl-N-(tetrahydrofuran-3-yl)-N-((5-(trifluoromethyl)pyridin-2-yl)methyl)-1,3-dihydrofuro[3,4-c]quinoline-8-carboxamide | 473.2 |
| **900** | 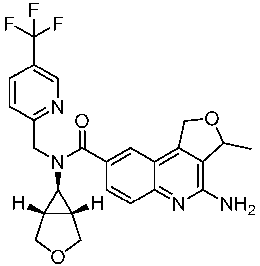 | 4-amino-N-((1R,5S,6r)-3-oxabicyclo[3.1.0]hexan-6-yl)-3-methyl-N-((5-(trifluoromethyl)pyridin-2-yl)methyl)-1,3-dihydrofuro[3,4-c]quinoline-8-carboxamide | 485.2 |
| **901** | 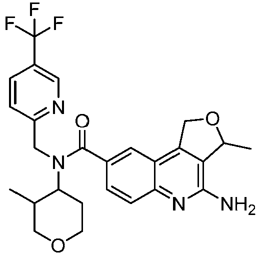 | 4-amino-3-methyl-N-(3-methyltetrahydro-2H-pyran-4-yl)-N-((5-(trifluoromethyl)pyridin-2-yl)methyl)-1,3-dihydrofuro[3,4-c]quinoline-8-carboxamide | 501.2 |
| **902** | 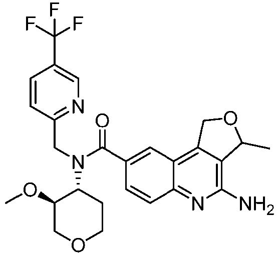 | 4-amino-N-((3S,4R)-3-methoxytetrahydro-2H-pyran-4-yl)-3-methyl-N-((5-(trifluoromethyl)pyridin-2-yl)methyl)-1,3-dihydrofuro[3,4-c]quinoline-8-carboxamide | 517.2 |
| **903** | 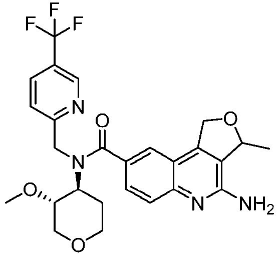 | 4-amino-N-((3R,4S)-3-methoxytetrahydro-2H-pyran-4-yl)-3-methyl-N-((5-(trifluoromethyl)pyridin-2-yl)methyl)-1,3-dihydrofuro[3,4-c]quinoline-8-carboxamide | 517.2 |
| **904** | 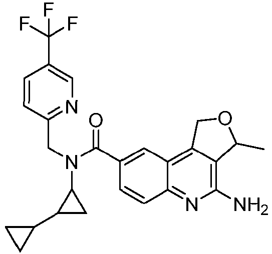 | N-([1,1'-bi(cyclopropan)]-2-yl)-4-amino-3-methyl-N-((5-(trifluoromethyl)pyridin-2-yl)methyl)-1,3-dihydrofuro[3,4-c]quinoline-8-carboxamide | 483.2 |
| **905** | 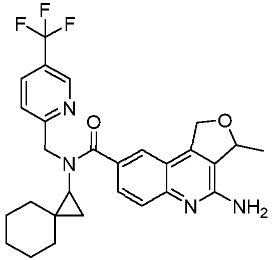 | 4-amino-3-methyl-N-(spiro[2.5]octan-1-yl)-N-((5-(trifluoromethyl)pyridin-2-yl)methyl)-1,3-dihydrofuro[3,4-c]quinoline-8-carboxamide | 511.2 |
| **906** | 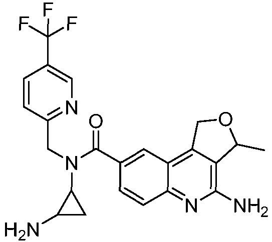 | 4-amino-N-(2-aminocyclopropyl)-3-methyl-N-((5-(trifluoromethyl)pyridin-2-yl)methyl)-1,3-dihydrofuro[3,4-c]quinoline-8-carboxamide | 458.2 |
| **907** | 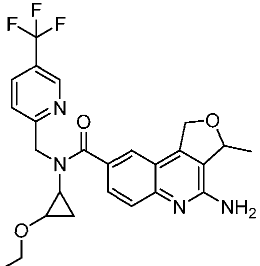 | 4-amino-N-(2-ethoxycyclopropyl)-3-methyl-N-((5-(trifluoromethyl)pyridin-2-yl)methyl)-1,3-dihydrofuro[3,4-c]quinoline-8-carboxamide | 487.2 |
| **908** | 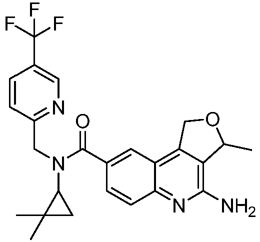 | 4-amino-N-(2,2-dimethylcyclopropyl)-3-methyl-N-((5-(trifluoromethyl)pyridin-2-yl)methyl)-1,3-dihydrofuro[3,4-c]quinoline-8-carboxamide | 471.2 |
| **909** | 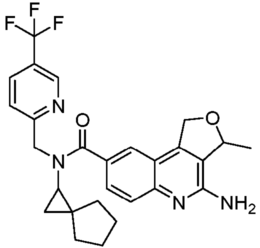 | 4-amino-3-methyl-N-(spiro[2.4]heptan-1-yl)-N-((5-(trifluoromethyl)pyridin-2-yl)methyl)-1,3-dihydrofuro[3,4-c]quinoline-8-carboxamide | 497.2 |
| **910** | 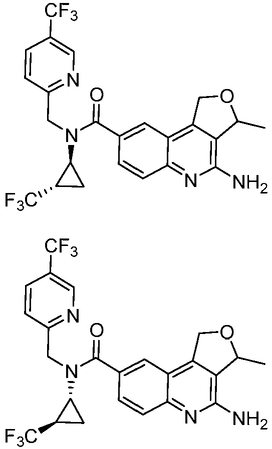 | 4-amino-3-methyl-N-((1R,2R)-2-(trifluoromethyl)cyclopropyl)-N-((5-(trifluoromethyl)-2-pyridinyl)methyl)-1,3-dihydrofuro[3,4-c]quinoline-8-carboxamide and 4-amino-3-methyl-N-((1S,2S)-2-(trifluoromethyl)cyclopropyl)-N-((5-(trifluoromethyl)-2-pyridinyl)methyl)-1,3-dihydrofuro[3,4-c]quinoline-8-carboxamide | 511.2 |
| **911** | 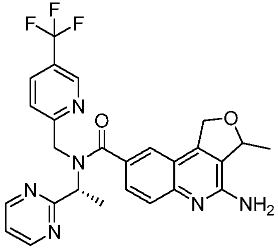 | 4-amino-3-methyl-N-((R)-1-(pyrimidin-2-yl)ethyl)-N-((5-(trifluoromethyl)pyridin-2-yl)methyl)-1,3-dihydrofuro[3,4-c]quinoline-8-carboxamide | 509.6 |
| **912** | 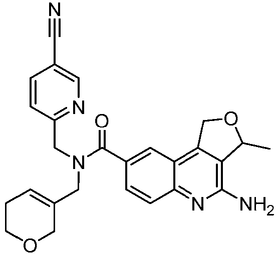 | 4-amino-N-((5-cyanopyridin-2-yl)methyl)-N-((5,6-dihydro-2H-pyran-3-yl)methyl)-3-methyl-1,3-dihydrofuro[3,4-c]quinoline-8-carboxamide | 456.2 |
| **913** | 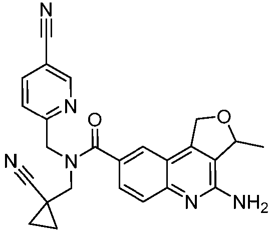 | 4-amino-N-((1-cyanocyclopropyl)methyl)-N-((5-cyanopyridin-2-yl)methyl)-3-methyl-1,3-dihydrofuro[3,4-c]quinoline-8-carboxamide | 439.2 |
| **914** | 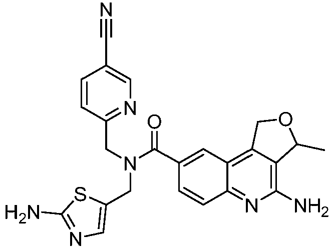 | 4-amino-N-((2-aminothiazol-5-yl)methyl)-N-((5-cyanopyridin-2-yl)methyl)-3-methyl-1,3-dihydrofuro[3,4-c]quinoline-8-carboxamide | 572.2 |
| **915** | 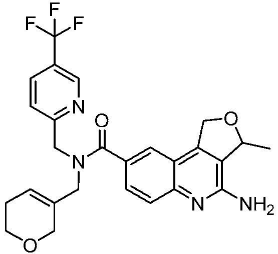 | 4-amino-N-((5,6-dihydro-2H-pyran-3-yl)methyl)-3-methyl-N-((5-(trifluoromethyl)pyridin-2-yl)methyl)-1,3-dihydrofuro[3,4-c]quinoline-8-carboxamide | 499.2 |
| **916** | 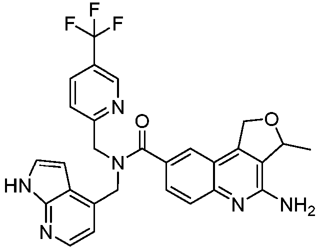 | N-((1H-pyrrolo[2,3-b]pyridin-4-yl)methyl)-4-amino-3-methyl-N-((5-(trifluoromethyl)pyridin-2-yl)methyl)-1,3-dihydrofuro[3,4-c]quinoline-8-carboxamide | 533.2 |
| **917** | 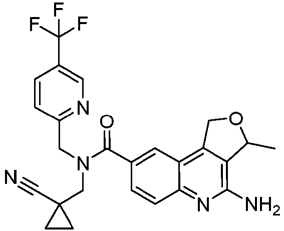 | 4-amino-N-((1-cyanocyclopropyl)methyl)-3-methyl-N-((5-(trifluoromethyl)pyridin-2-yl)methyl)-1,3-dihydrofuro[3,4-c]quinoline-8-carboxamide | 482.2 |
| **918** | 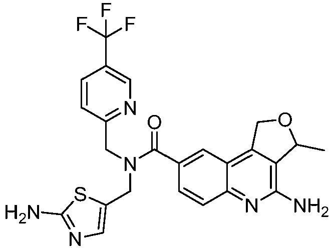 | 4-amino-N-((2-aminothiazol-5-yl)methyl)-3-methyl-N-((5-(trifluoromethyl)pyridin-2-yl)methyl)-1,3-dihydrofuro[3,4-c]quinoline-8-carboxamide | 515.1 |
| **919** | 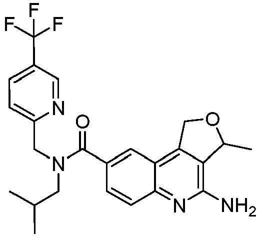 | 4-amino-N-isobutyl-3-methyl-N-((5-(trifluoromethyl)pyridin-2-yl)methyl)-1,3-dihydrofuro[3,4-c]quinoline-8-carboxamide | 459.2 |
| **920** | 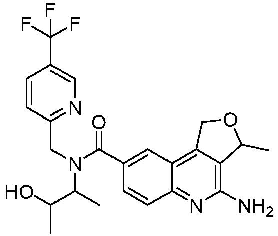 | 4-amino-N-(3-hydroxybutan-2-yl)-3-methyl-N-((5-(trifluoromethyl)pyridin-2-yl)methyl)-1,3-dihydrofuro[3,4-c]quinoline-8-carboxamide | 475.2 |
| **921** | 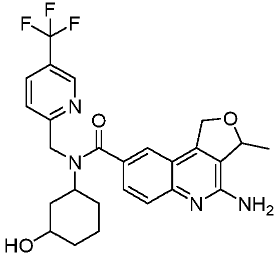 | 4-amino-N-(3-hydroxycyclohexyl)-3-methyl-N-((5-(trifluoromethyl)pyridin-2-yl)methyl)-1,3-dihydrofuro[3,4-c]quinoline-8-carboxamide | 501.2 |
| **922** | 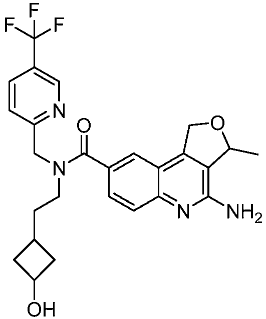 | 4-amino-N-(2-(3-hydroxycyclobutyl)ethyl)-3-methyl-N-((5-(trifluoromethyl)pyridin-2-yl)methyl)-1,3-dihydrofuro[3,4-c]quinoline-8-carboxamide | 501.2 |
| **923** | 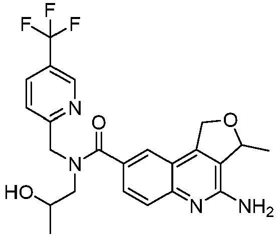 | 4-amino-N-(2-hydroxypropyl)-3-methyl-N-((5-(trifluoromethyl)pyridin-2-yl)methyl)-1,3-dihydrofuro[3,4-c]quinoline-8-carboxamide | 461.2 |
| **924** | 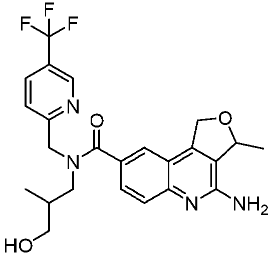 | 4-amino-N-(3-hydroxy-2-methylpropyl)-3-methyl-N-((5-(trifluoromethyl)pyridin-2-yl)methyl)-1,3-dihydrofuro[3,4-c]quinoline-8-carboxamide | 475.2 |
| **925** | 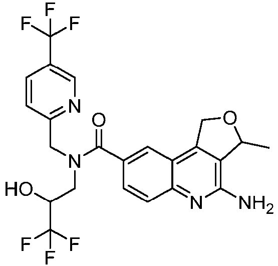 | 4-amino-3-methyl-N-(3,3,3-trifluoro-2-hydroxypropyl)-N-((5-(trifluoromethyl)pyridin-2-yl)methyl)-1,3-dihydrofuro[3,4-c]quinoline-8-carboxamide | 515.2 |
| **926** | 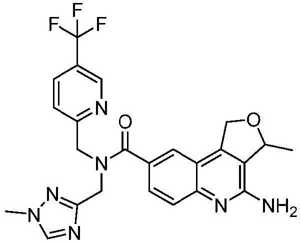 | 4-amino-3-methyl-N-((1-methyl-1H-1,2,4-triazol-3-yl)methyl)-N-((5-(trifluoromethyl)pyridin-2-yl)methyl)-1,3-dihydrofuro[3,4-c]quinoline-8-carboxamide | 498.2 |
| **927** | 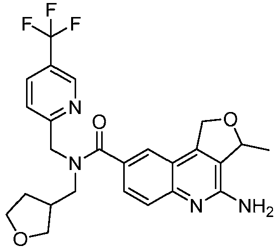 | 4-amino-3-methyl-N-((tetrahydrofuran-3-yl)methyl)-N-((5-(trifluoromethyl)pyridin-2-yl)methyl)-1,3-dihydrofuro[3,4-c]quinoline-8-carboxamide | 487.2 |
| **928** | 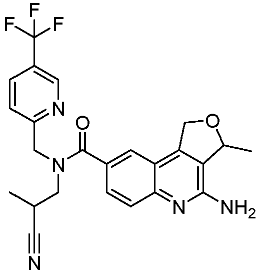 | 4-amino-N-(2-cyanopropyl)-3-methyl-N-((5-(trifluoromethyl)pyridin-2-yl)methyl)-1,3-dihydrofuro[3,4-c]quinoline-8-carboxamide | 470.2 |
| **929** | 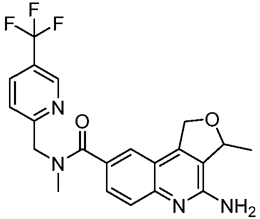 | 4-amino-N,3-dimethyl-N-((5-(trifluoromethyl)pyridin-2-yl)methyl)-1,3-dihydrofuro[3,4-c]quinoline-8-carboxamide | 417.2 |
| **930** | 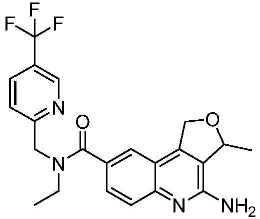 | 4-amino-N-ethyl-3-methyl-N-((5-(trifluoromethyl)pyridin-2-yl)methyl)-1,3-dihydrofuro[3,4-c]quinoline-8-carboxamide | 431.2 |
| **931** | 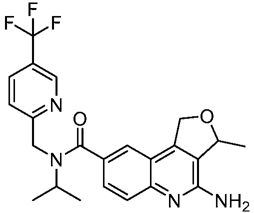 | 4-amino-N-isopropyl-3-methyl-N-((5-(trifluoromethyl)pyridin-2-yl)methyl)-1,3-dihydrofuro[3,4-c]quinoline-8-carboxamide | 445.2 |
| **932** | 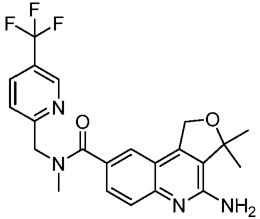 | 4-amino-N,3,3-trimethyl-N-((5-(trifluoromethyl)pyridin-2-yl)methyl)-1,3-dihydrofuro[3,4-c]quinoline-8-carboxamide | 431.2 |
| **933** | 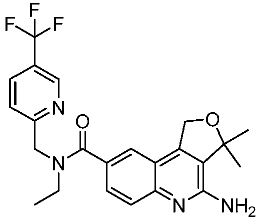 | 4-amino-N-ethyl-3,3-dimethyl-N-((5-(trifluoromethyl)pyridin-2-yl)methyl)-1,3-dihydrofuro[3,4-c]quinoline-8-carboxamide | 445.2 |
| **934** | 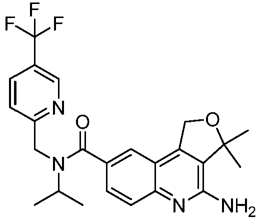 | 4-amino-N-isopropyl-3,3-dimethyl-N-((5-(trifluoromethyl)pyridin-2-yl)methyl)-1,3-dihydrofuro[3,4-c]quinoline-8-carboxamide | 459.2 |
| **935** | 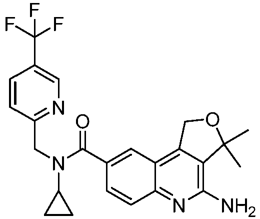 | 4-amino-N-cyclopropyl-3,3-dimethyl-N-((5-(trifluoromethyl)pyridin-2-yl)methyl)-1,3-dihydrofuro[3,4-c]quinoline-8-carboxamide | 457.2 |

### Example 936: 4-amino-N-(1-(3-cyano-5-(trifluoromethyl)pyridin-2-yl)ethyl)-7-fluoro-N,1-dimethyl-1H-pyrazolo[4,3-c]quinoline-8-carboxamide

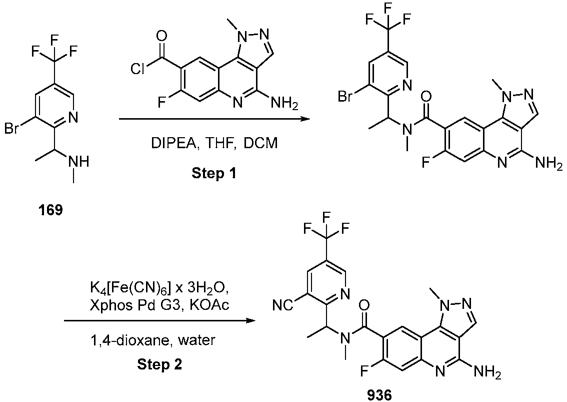

Step 1. To a 50-mL round-bottomed flask was added 1-(3-bromo-5-(trifluoromethyl)pyridin-2-yl)-N-methylethan-1-amine (0.090 g, 0.318 mmol) and n,n-diisoproylethylamine (0.123 g, 0.167 mL, 0.954 mmol, Sigma-Aldrich Corporation) in tetrahydrofuran (1.590 mL) and dichloromethane (1.590 mL). The reaction mixture was cooled to 0 °C, then 4-amino-7-fluoro-1-methyl-1H-pyrazolo[4,3-c]quinoline-8-carbonyl chloride hydrochloride (0.110 g, 0.350 mmol) was added slowly to the reaction mixture. The overall reaction mixture was stirred at rt for 30 min. The reaction mixture was concentrated in vacuo. The crude material was absorbed onto a plug of silica gel and purified by chromatography through a silica-gel column, eluting with a gradient of 0-8% MeOH in CH₂Cl₂, then isocratic at 8% MeOH in CH₂Cl₂, to provide 4-amino-N-(1-(3-bromo-5-(trifluoromethyl)pyridin-2-yl)ethyl)-7-fluoro-N,1-dimethyl-1H-pyrazolo[4,3-c]quinoline-8-carboxamide (0.040 g, 0.076 mmol, 23.95 % yield) as off-white solid. *m*/*z* (ESI): 525.0 (M+H)⁺.

Step 2. A resealable reaction vessel was charged with 4-amino-N-(1-(3-bromo-5-(trifluoromethyl)pyridin-2-yl)ethyl)-7-fluoro-N,1-dimethyl-1H-pyrazolo[4,3-c]quinoline-8-carboxamide (0.040 g, 0.076 mmol), potassium ferrocyanide trihydrate (0.257 g, 0.609 mmol, Toronto Research Chemicals) and potassium acetate (0.022 g, 0.228 mmol, Sigma-Aldrich Corporation) in 1,4-dioxane (0.190 mL) and water (0.190 mL). The reaction mixture was sparged with Argon (gas) for 5 min, then methanesulfonato(2-dicyclohexylphosphino-2',4',6'-tri-i-propyl-1,1'-biphenyl)(2'-amino-1,1'-biphenyl-2-yl)palladium (ii) (XPhos Pd G3) (0.012 g, 0.015 mmol, Strem Chemicals, Inc.) was added to the mixture and the vial was sealed. The mixture was stirred and heated at 100°C for 2 h. The reaction mixture was concentrated in vacuo. The crude material was absorbed onto a plug of silica gel and purified by chromatography through a silica-gel column, eluting with a gradient of 0-8% MeOH in CH₂Cl₂, to provide 4-amino-N-(1-(3-cyano-5-(trifluoromethyl)pyridin-2-yl)ethyl)-7-fluoro-N,1-dimethyl-1H-pyrazolo[4,3-c]quinoline-8-carboxamide (0.020 g, 0.042 mmol, 55.7 % yield) as off-white solid. *m*/*z* (ESI): 472.1 (M+H)⁺.

### HCT116 Proliferation Activity

HCT116 MTAP null and WT cells were seeded in 96-well tissue culture plates in RPMI 1640 media + 10% fetal bovine serum. Plates were incubated overnight at 37°C and 5% CO₂. Cells were then treated with an 8- or 9-point serial dilution of compound, using a top concentration of 1, or 10 µM, 1:3 serial dilution steps and, a DMSO-only control. Cells were incubated in the presence of drug for 6 days. Effects on cell viability were measured with the CellTiter-Glo^{®} Luminescent Cell Viability Assay (Promega) per manufacturer's recommendation. Assay plates were read on an EnVision^{™} Multilabel Reader using the Ultra-Sensitive luminescence module. IC₅₀ values were calculated with GraphPad Prism v 5.01 using symmetrical sigmoidal dose-response least squares fit with Hill slope fixed to -1 and top constrain to 100% or GeneData Screener using a 4-parameter logistic model to fit dose response curves.

Alternatively, compounds could be assayed with a 384 well plate format:

Compounds were pre-spotted into 384 well plates with a 22-point serial dilution of compound, using a top concentration of 10 or 50 µM, 1:2 serial dilution steps and, a DMSO-only control. HCT116 MTAP null and WT cells were then seeded as above and after 6 days effects on cell viability were measured with the CellTiter-Glo^{®} Luminescent Cell Viability Assay (Promega). Assay plates were read as above and IC₅₀ values were calculated with GeneData Screener using a 4-parameter logistic model to fit dose response curves. The reported IC₅₀ represents the value where the curve transits 50% of control.

**Table 26**

| HCT116-MTAP null and WT cell line proliferation | | |
|---|---|---|
| **Ex.** | **HCT-116 MTAP null IC₅₀ (µM)** | **HCT-116 WT IC₅₀ (µM)** |
| **300** | 0.041 | 1.340 |
| **301** | 0.259 | 11.000 |
| **302** | 0.900 | 17.267 |
| **303** | 0.024 | 0.574 |
| **304** | 0.026 | 0.352 |
| **305** | 0.027 | 0.861 |
| **306** | 0.217 | 7.990 |
| **307** | 0.018 | 0.471 |
| **308** | 0.124 | 5.095 |
| **309** | 4.000 | >10 |
| **310** | 0.071 | 4.050 |
| **311** | 0.056 | 2.205 |
| **312** | 1.090 | >10 |
| **313** | 0.200 | 3.680 |
| **314** | 0.254 | 15.200 |
| **315** | 0.137 | 11.060 |
| **316** | 0.570 | 23.650 |
| **317** | 0.432 | 49.700 |
| **318** | 4.930 | 36.300 |
| **319** | 27.700 | >50 |
| **320** | 0.303 | 15.800 |
| **321** | 0.226 | 3.790 |
| **322** | 0.081 | 1.030 |
| **323** | 0.358 | 15.033 |
| **324** | 0.908 | 51.600 |
| **325** | 0.073 | 2.280 |
| **326** | 0.019 | 1.280 |
| **327** | 0.811 | >10 |
| **328** | 0.129 | 19.400 |
| **329** | 0.022 | 0.160 |
| **330** | 0.204 | >10 |
| **331** | 0.028 | 2.300 |
| **332** | 0.120 | 6.095 |
| **333** | 0.024 | 1.408 |
| **334** | 0.010 | 0.509 |
| **335** | 0.361 | |
| **336** | 0.087 | 2.571 |
| **337** | 0.033 | 0.714 |
| **338** | 0.406 | 5.830 |
| **339** | 0.025 | 0.611 |
| **340** | 2.950 | >10 |
| **341** | 0.212 | 3.790 |
| **342** | 1.310 | |
| **343** | 0.270 | 8.240 |
| **344** | 0.244 | >10 |
| **345** | 0.011 | 0.210 |
| **346** | 1.090 | 19.429 |
| **347** | 0.712 | 43.800 |
| **348** | 0.155 | 9.035 |
| **349** | 0.325 | 9.190 |
| **350** | 0.332 | 22.100 |
| **351** | 0.048 | 3.510 |
| **352** | 0.872 | >10 |
| **353** | 0.124 | >10 |
| **354** | 0.623 | 21.400 |
| **355** | 0.171 | 19.000 |
| **356** | 0.092 | 5.360 |
| **357** | 0.115 | 4.270 |
| **358** | 0.121 | 8.161 |
| **359** | 0.226 | 10.200 |
| **360** | 0.053 | 3.072 |
| **361** | 0.173 | 7.753 |
| **362** | 0.076 | 2.210 |
| **363** | 0.036 | 1.180 |
| **364** | 0.058 | 1.760 |
| **365** | 0.076 | 6.500 |
| **366** | 0.175 | 12.500 |
| **367** | 2.280 | >50 |
| **368** | 0.065 | 3.260 |
| **369** | 0.124 | 5.039 |
| **370** | 0.029 | 1.370 |
| **371** | 0.010 | 0.288 |
| **372** | 0.110 | 5.610 |
| **373** | 0.022 | 2.975 |
| **374** | 0.014 | 0.286 |
| **375** | 1.500 | 6.890 |
| **376** | 0.222 | 15.150 |
| **377** | 0.049 | 2.065 |
| **378** | 0.294 | 9.430 |
| **379** | 0.025 | 0.764 |
| **380** | 0.056 | 4.100 |
| **381** | 0.008 | 1.560 |
| **382** | 1.160 | 22.800 |
| **383** | 1.310 | 43.000 |
| **384** | 0.053 | 1.380 |
| **385** | 0.157 | 7.285 |
| **386** | 0.455 | 30.300 |
| **387** | 0.175 | 4.040 |
| **388** | 0.054 | 1.280 |
| **389** | 0.050 | 3.393 |
| **390** | 0.262 | 9.900 |
| **391** | 0.091 | 4.540 |
| **392** | 0.057 | 1.539 |
| **393** | 0.113 | 6.933 |
| **394** | 0.030 | 0.831 |
| **395** | 0.031 | 0.535 |
| **396** | 0.020 | 0.629 |
| **397** | 0.055 | 1.101 |
| **398** | 0.049 | 1.390 |
| **399** | 0.393 | 4.430 |
| **400** | 0.970 | >10 |
| **401** | 0.084 | 3.075 |
| **402** | 0.027 | 2.207 |
| **403** | 10.000 | >10 |
| **404** | 0.180 | 11.400 |
| **405** | 0.362 | >10 |
| **406** | 0.097 | 4.135 |
| **407** | 3.660 | >10 |
| **408** | 0.086 | 3.353 |
| **409** | 0.056 | 5.873 |
| **410** | 0.087 | 3.255 |
| **411** | 0.181 | 13.600 |
| **412** | 0.397 | 25.550 |
| **413** | 0.870 | 20.300 |
| **414** | 5.865 | >50 |
| **415** | 0.449 | 25.132 |
| **416** | 0.062 | 8.233 |
| **417** | 5.610 | 17.600 |
| **418** | 0.171 | 6.337 |
| **419** | 0.152 | 3.793 |
| **420** | 0.248 | 8.358 |
| **421** | 0.116 | 10.035 |
| **422** | 6.740 | 10.675 |
| **423** | 0.064 | 3.980 |
| **424** | 0.032 | 1.340 |
| **425** | 2.710 | >50 |
| **426** | 0.096 | 4.220 |
| **427** | 0.044 | 1.675 |
| **428** | 0.101 | 1.250 |
| **429** | 1.260 | 31.800 |
| **430** | 0.312 | 12.850 |
| **431** | 14.335 | 23.500 |
| **432** | 0.203 | 6.390 |
| **433** | 0.344 | 21.200 |
| **434** | 0.584 | 23.900 |
| **435** | 0.014 | 0.065 |
| **436** | 0.029 | 0.187 |
| **437** | 0.031 | 0.353 |
| **438** | 0.012 | 0.187 |
| **439** | 0.007 | 0.138 |
| **440** | 0.461 | >10 |
| **441** | 0.012 | 0.204 |
| **442** | 0.009 | 0.177 |
| **443** | 0.127 | 7.214 |
| **444** | 0.013 | 0.343 |
| **445** | 0.008 | 0.153 |
| **446** | 0.012 | 0.237 |
| **447** | 0.031 | 0.256 |
| **448** | 0.124 | 2.100 |
| **449** | 0.042 | 1.230 |
| **450** | 0.159 | 11.465 |
| **451** | 0.032 | 4.377 |
| **452** | 0.047 | 1.570 |
| **453** | 0.035 | 0.543 |
| **454** | 0.550 | 17.900 |
| **455** | 0.148 | 4.780 |
| **456** | 0.287 | >10 |
| **457** | 0.132 | 3.175 |
| **458** | 0.026 | 0.361 |
| **459** | 0.029 | 0.305 |
| **460** | 0.019 | 0.287 |
| **461** | 0.069 | 4.500 |
| **462** | 0.105 | 3.935 |
| **463** | 0.014 | 0.440 |
| **464** | 0.007 | 0.240 |
| **465** | 0.031 | 1.230 |
| **466** | 0.028 | 0.217 |
| **467** | 0.014 | 0.332 |
| **468** | 0.008 | 0.208 |
| **469** | 0.025 | 0.550 |
| **470** | 0.041 | 3.023 |
| **471** | 0.043 | 1.161 |
| **472** | 0.267 | 14.900 |
| **473** | 0.809 | >10 |
| **474** | 0.030 | 1.330 |
| **475** | 0.375 | 7.811 |
| **476** | 1.370 | 4.460 |
| **477** | 0.655 | 19.428 |
| **478** | 0.082 | 9.580 |
| **479** | 0.328 | 17.100 |
| **480** | 0.272 | 1.446 |
| **481** | 0.074 | 5.710 |
| **482** | 0.052 | 2.640 |
| **483** | 0.458 | 7.290 |
| **484** | 0.020 | 0.402 |
| **485** | 0.365 | 32.100 |
| **486** | 0.445 | 17.500 |
| **487** | 0.088 | 1.371 |
| **488** | 0.049 | 0.696 |
| **489** | 0.276 | 5.210 |
| **490** | 0.087 | 3.475 |
| **492** | 0.048 | 0.622 |
| **493** | 0.124 | 1.165 |
| **494** | 0.282 | 9.070 |
| **495** | 0.079 | 6.355 |
| **496** | 1.272 | 2.238 |
| **497** | 0.250 | 3.525 |
| **498** | 0.266 | 12.700 |
| **499** | 0.164 | 4.140 |
| **500** | 0.595 | 32.467 |
| **501** | 0.046 | 1.200 |
| **502** | 3.930 | 47.300 |
| **503** | 0.056 | 2.005 |
| **504** | 0.056 | 1.850 |
| **505** | 0.022 | 0.662 |
| **506** | 0.009 | 0.369 |
| **507** | 0.025 | 0.373 |
| **508** | 0.060 | 2.840 |
| **509** | 0.040 | 0.878 |
| **510** | 0.065 | 2.660 |
| **511** | 0.122 | 4.920 |
| **512** | 0.038 | 1.380 |
| **513** | 0.019 | 0.848 |
| **514** | 0.071 | 3.250 |
| **515** | 0.080 | 3.350 |
| **516** | 0.167 | 9.320 |
| **517** | 0.706 | 45.400 |
| **518** | 0.106 | 6.270 |
| **519** | 0.187 | 14.000 |
| **520** | 0.149 | 9.060 |
| **521** | 0.018 | 0.698 |
| **522** | 0.014 | 0.380 |
| **523** | 0.072 | 6.790 |
| **524** | 0.183 | 16.350 |
| **525** | 0.032 | 2.125 |
| **526** | 0.169 | 6.950 |
| **527** | 0.183 | 13.500 |
| **528** | 0.111 | 5.420 |
| **529** | 0.887 | 28.500 |
| **530** | 0.165 | 6.560 |
| **531** | 0.188 | 2.340 |
| **532** | 0.037 | 2.030 |
| **533** | 0.459 | 3.190 |
| **534** | 0.581 | 5.580 |
| **535** | 0.860 | 6.990 |
| **536** | 0.050 | 0.325 |
| **537** | 0.222 | 5.390 |
| **538** | 0.040 | 0.735 |
| **539** | 0.106 | 2.100 |
| **540** | 0.039 | 1.480 |
| **541** | 0.037 | 0.548 |
| **542** | 0.035 | 1.540 |
| **543** | 0.057 | 2.010 |
| **544** | 0.056 | 2.320 |
| **545** | 0.007 | 0.140 |
| **546** | 0.019 | 0.976 |
| **547** | 0.668 | 24.500 |
| **548** | 0.078 | 6.873 |
| **549** | 0.020 | 0.487 |
| **550** | 0.066 | 1.800 |
| **551** | 0.125 | 5.570 |
| **552** | 0.025 | 1.880 |
| **553** | 0.247 | 12.400 |
| **554** | 0.008 | 0.181 |
| **555** | 0.023 | 2.043 |
| **556** | 0.014 | 0.458 |
| **557** | 0.375 | 12.300 |
| **558** | 0.019 | 0.421 |
| **559** | 0.030 | 1.179 |
| **560** | 0.056 | 4.140 |
| **561** | 0.089 | 5.330 |
| **562** | 0.086 | 3.530 |
| **563** | 0.028 | 2.080 |
| **564** | 0.021 | 0.925 |
| **565** | 0.019 | 0.653 |
| **566** | 0.013 | 0.181 |
| **567** | 0.090 | 6.790 |
| **568** | 0.091 | 5.390 |
| **569** | 0.031 | 3.890 |
| **570** | 0.757 | 0.329 |
| **571** | 0.071 | 4.930 |
| **572** | 0.939 | >50 |
| **573** | 0.085 | 3.730 |
| **574** | 0.025 | 0.878 |
| **575** | 0.012 | 0.354 |
| **576** | 0.107 | 2.080 |
| **577** | 3.260 | >10 |
| **578** | 0.338 | 4.930 |
| **579** | 0.348 | 5.620 |
| **580** | 0.321 | 10.800 |
| **581** | 0.809 | 10.200 |
| **582** | 0.036 | 0.558 |
| **583** | 0.173 | 4.815 |
| **584** | 0.014 | 0.164 |
| **585** | 0.149 | 1.980 |
| **586** | 0.012 | 0.150 |
| **587** | 0.076 | 1.710 |
| **588** | 0.079 | 1.950 |
| **589** | 0.029 | 0.815 |
| **590** | 2.230 | >50 |
| **591** | 0.563 | 32.100 |
| **592** | 0.025 | 2.410 |
| **593** | 0.012 | 0.908 |
| **594** | 0.638 | 29.100 |
| **595** | 0.010 | 0.676 |
| **596** | 0.888 | 37.300 |
| **597** | 0.200 | 15.200 |
| **598** | 0.008 | 0.415 |
| **599** | 0.536 | 27.200 |
| **600** | 0.018 | 0.663 |
| **601** | 0.997 | 21.200 |
| **602** | 0.006 | 0.209 |
| **603** | 0.276 | 11.800 |
| **604** | 0.238 | 24.600 |
| **605** | 0.014 | 0.793 |
| **606** | 0.263 | 3.700 |
| **607** | 0.154 | 1.370 |
| **608** | 0.226 | 3.790 |
| **609** | 0.081 | 1.030 |
| **610** | 0.006 | 0.135 |
| **611** | 0.084 | 0.474 |
| **612** | 0.176 | 3.060 |
| **613** | 0.023 | 0.761 |
| **614** | 0.042 | 0.255 |
| **615** | 0.019 | 0.271 |
| **616** | 0.018 | 0.419 |
| **617** | 0.245 | >10 |
| **618** | 0.033 | 0.264 |
| **619** | 0.061 | 1.060 |
| **620** | 0.077 | 3.560 |
| **621** | 0.108 | 1.230 |
| **622** | 0.011 | 0.156 |
| **623** | 0.015 | 0.148 |
| **624** | 0.021 | 0.189 |
| **625** | 0.130 | 1.590 |
| **626** | 0.043 | 0.444 |
| **627** | 0.045 | 0.583 |
| **628** | 0.119 | 1.190 |
| **629** | 0.016 | 0.398 |
| **630** | 0.019 | 0.408 |
| **631** | 0.010 | 0.215 |
| **632** | 0.011 | 0.231 |
| **633** | 0.013 | 0.071 |
| **634** | 0.034 | 0.953 |
| **635** | 0.073 | 1.210 |
| **636** | 0.149 | 2.990 |
| **637** | 0.212 | 3.220 |
| **638** | 0.022 | 0.378 |
| **639** | 0.238 | 3.150 |
| **640** | 0.013 | 0.110 |
| **641** | 0.016 | 0.242 |
| **642** | 0.041 | 1.304 |
| **643** | 0.178 | 1.615 |
| **644** | 2.790 | >10 |
| **645** | 50.000 | >50 |
| **646** | 0.019 | 0.263 |
| **647** | 0.673 | 4.200 |
| **648** | 0.279 | 2.500 |
| **649** | 0.014 | 0.181 |
| **650** | 0.053 | 0.811 |
| **651** | 0.234 | 3.520 |
| **652** | 0.050 | 1.390 |
| **653** | 0.008 | 0.073 |
| **654** | 0.009 | 0.171 |
| **655** | 0.016 | 0.294 |
| **656** | 0.019 | 1.260 |
| **657** | 0.013 | 0.196 |
| **658** | 0.006 | 0.071 |
| **659** | 0.014 | 0.378 |
| **660** | 0.354 | >10 |
| **661** | 0.303 | 26.200 |
| **662** | 0.029 | 0.290 |
| **663** | 0.035 | 1.025 |
| **664** | 0.038 | 1.390 |
| **665** | 0.037 | 1.250 |
| **666** | 0.687 | >10 |
| **667** | 0.929 | 7.130 |
| **668** | 0.304 | >10 |
| **669** | 0.073 | 3.070 |
| **670** | 0.170 | 8.020 |
| **671** | 0.080 | 3.970 |
| **672** | 0.145 | 7.460 |
| **673** | 0.110 | 3.265 |
| **674** | 0.073 | 2.575 |
| **675** | 0.066 | 12.733 |
| **676** | 0.012 | 0.316 |
| **677** | 0.243 | >10 |
| **678** | 0.040 | 1.175 |
| **679** | 0.005 | 0.108 |
| **680** | 0.091 | 2.840 |
| **681** | 0.367 | 6.490 |
| **682** | 0.087 | 3.400 |
| **683** | 0.235 | >10 |
| **684** | 0.342 | >10 |
| **685** | 0.201 | >10 |
| **686** | 0.445 | 8.800 |
| **687** | >10 | >10 |
| **688** | 0.081 | 3.445 |
| **689** | 0.096 | 2.585 |
| **690** | 0.071 | 4.170 |
| **691** | 0.127 | 3.880 |
| **692** | 0.449 | >10 |
| **693** | 2.020 | >10 |
| **694** | 0.415 | >10 |
| **695** | 0.210 | 4.990 |
| **696** | 0.261 | >10 |
| **697** | 0.121 | 4.430 |
| **698** | 0.206 | 4.500 |
| **699** | 0.249 | |
| **700** | 1.100 | >10 |
| **701** | 0.193 | 7.250 |
| **702** | 0.470 | 4.120 |
| **703** | 0.337 | 8.150 |
| **704** | 0.549 | >10 |
| **705** | 0.637 | 28.500 |
| **706** | 0.172 | 3.450 |
| **707** | 0.106 | 2.870 |
| **708** | 0.153 | 8.220 |
| **709** | 0.076 | 4.250 |
| **710** | 0.484 | >10 |
| **711** | 0.148 | 4.780 |
| **712** | 2.000 | >50 |
| **713** | 5.110 | >50 |
| **714** | 0.008 | 0.124 |
| **715** | 0.006 | 0.070 |
| **716** | 0.917 | 33.300 |
| **717** | 0.609 | 7.759 |
| **718** | 0.281 | 16.443 |
| **719** | 1.031 | 21.798 |
| **720** | 14.900 | >50 |
| **721** | 0.209 | 9.410 |
| **722** | 0.085 | 3.350 |
| **723** | 0.157 | 6.020 |
| **724** | 0.054 | 2.440 |
| **725** | 0.854 | >50 |
| **726** | 0.109 | 7.520 |
| **727** | 0.279 | 12.300 |
| **728** | 0.047 | 0.663 |
| **729** | 0.019 | 0.534 |
| **730** | 0.017 | 0.620 |
| **731** | 0.047 | 2.090 |
| **732** | 0.087 | 2.850 |
| **733** | 0.290 | 6.300 |
| **734** | 0.383 | 14.400 |
| **735** | 5.500 | 36.200 |
| **736** | 0.127 | 4.890 |
| **737** | 0.059 | 2.380 |
| **738** | 0.013 | 0.430 |
| **739** | 0.071 | 2.220 |
| **740** | 0.005 | 0.100 |
| **741** | 0.042 | 1.023 |
| **742** | 0.066 | 2.500 |
| **743** | 0.015 | 1.090 |
| **744** | 0.038 | 0.967 |
| **745** | 0.246 | 14.800 |
| **746** | 0.034 | 3.457 |
| **747** | 0.161 | 5.090 |
| **748** | 5.350 | 36.400 |
| **749** | 0.177 | 3.345 |
| **750** | 0.023 | 0.767 |
| **751** | 0.017 | 0.337 |
| **752** | 0.033 | 0.771 |
| **753** | 0.124 | 3.890 |
| **754** | 0.024 | 0.370 |
| **755** | 0.892 | 6.420 |
| **756** | 0.049 | 0.744 |
| **757** | 0.026 | 1.315 |
| **758** | 0.037 | 0.658 |
| **759** | 6.730 | 11.800 |
| **760** | 0.743 | 6.100 |
| **761** | 0.305 | 13.600 |
| **762** | 0.026 | 0.621 |
| **763** | 0.113 | 4.813 |
| **764** | 6.380 | >50 |
| **765** | 0.049 | 1.980 |
| **766** | 0.061 | 1.910 |
| **767** | 2.270 | >50 |
| **768** | 4.230 | >50 |
| **769** | 0.115 | 7.500 |
| **770** | 0.253 | 19.400 |
| **771** | 0.395 | 31.100 |
| **772** | 0.374 | 18.200 |
| **773** | 0.106 | 2.630 |
| **774** | 0.736 | 40.600 |
| **775** | 0.033 | 2.680 |
| **776** | 0.150 | >10 |
| **777** | 0.025 | 1.380 |
| **778** | 0.049 | 2.657 |
| **779** | 0.364 | 28.900 |
| **780** | 0.022 | 1.185 |
| **781** | 0.037 | 1.310 |
| **782** | 0.080 | 7.767 |
| **783** | 0.868 | 16.400 |
| **784** | 0.022 | 0.609 |
| **785** | 0.851 | 19.800 |
| **786** | 0.126 | 7.330 |
| **787** | 0.865 | 8.970 |
| **788** | 0.008 | 0.134 |
| **789** | 0.083 | 6.930 |
| **790** | 0.776 | 47.100 |
| **791** | 0.011 | 0.341 |
| **792** | 0.030 | 2.330 |
| **793** | 0.011 | 0.296 |
| **794** | 0.020 | 0.499 |
| **795** | 0.008 | 0.379 |
| **796** | 0.034 | 1.670 |
| **797** | 0.041 | 0.755 |
| **798** | 0.010 | 0.152 |
| **799** | 0.008 | 0.263 |
| **800** | 0.007 | 0.245 |
| **801** | 0.153 | 13.550 |
| **802** | 0.021 | 1.376 |
| **803** | 0.012 | 0.384 |
| **804** | 0.026 | 1.803 |
| **805** | 0.008 | 0.121 |
| **806** | 0.056 | 3.160 |
| **807** | 0.005 | 0.114 |
| **808** | 0.052 | 3.130 |
| **809** | 0.031 | 1.790 |
| **810** | 0.226 | 13.800 |
| **811** | 0.645 | >50 |
| **812** | 0.237 | 14.100 |
| **813** | 0.676 | 29.300 |
| **814** | 0.044 | 1.170 |
| **815** | 0.018 | 0.301 |
| **816** | 0.078 | 2.490 |
| **817** | 0.028 | 0.689 |
| **818** | 0.007 | 0.161 |
| **819** | | |
| **820** | 7.070 | >10 |
| **821** | 4.190 | >50 |
| **822** | 0.323 | 11.950 |
| **823** | 0.528 | 16.233 |
| **824** | 2.390 | 26.300 |
| **825** | 0.022 | 0.276 |
| **826** | 6.720 | 27.100 |
| **827** | 1.250 | 47.200 |
| **828** | 0.026 | 0.294 |
| **829** | 7.790 | >50 |
| **830** | 0.080 | 2.970 |
| **831** | 2.570 | 31.700 |
| **832** | 0.454 | 12.600 |
| **833** | 2.565 | 27.200 |
| **834** | 0.817 | 19.700 |
| **835** | 0.126 | 3.760 |
| **836** | 3.635 | 38.700 |
| **837** | 7.080 | >50 |
| **838** | 0.161 | 8.590 |
| **839** | 0.213 | 9.520 |
| **840** | 0.638 | 22.950 |
| **841** | 0.613 | 11.650 |
| **842** | 0.115 | 3.275 |
| **843** | 3.355 | >50 |
| **844** | 0.105 | 6.225 |
| **845** | 0.105 | 2.240 |
| **846** | 0.212 | 3.940 |
| **847** | 1.865 | 36.050 |
| **848** | 0.453 | 11.450 |
| **849** | 0.879 | 24.400 |
| **850** | 8.345 | >50 |
| **851** | 0.047 | 4.003 |
| **852** | 3.160 | >50 |
| **853** | 0.167 | 14.350 |
| **854** | 0.086 | 3.280 |
| **855** | 0.164 | 28.850 |
| **856** | 0.087 | 8.480 |
| **857** | 0.026 | 1.090 |
| **858** | 0.041 | 3.333 |
| **859** | 0.583 | 17.500 |
| **860** | 0.017 | 0.849 |
| **861** | 0.205 | 13.600 |
| **862** | 0.031 | 2.195 |
| **863** | 0.007 | 0.340 |
| **864** | 0.618 | 22.700 |
| **865** | 0.059 | 2.810 |
| **866** | 0.309 | 11.300 |
| **867** | 0.157 | 7.840 |
| **868** | 0.059 | 3.840 |
| **869** | 0.627 | 8.430 |
| **870** | 0.007 | 0.162 |
| **871** | 0.025 | 0.078 |
| **872** | 0.075 | 4.500 |
| **873** | 0.066 | 7.317 |
| **874** | 0.929 | 36.000 |
| **875** | 0.025 | 1.415 |
| **876** | 0.334 | 30.450 |
| **877** | 0.756 | 44.100 |
| **878** | 0.010 | 0.709 |
| **879** | 0.181 | 12.300 |
| **880** | 0.137 | 8.310 |
| **881** | 0.007 | 0.370 |
| **882** | 0.062 | 1.340 |
| **883** | 0.020 | 0.514 |
| **884** | 0.027 | 0.733 |
| **885** | 0.025 | 0.596 |
| **886** | 1.690 | >10 |
| **887** | 0.025 | 0.496 |
| **888** | 0.021 | 0.231 |
| **889** | 0.202 | 4.740 |
| **890** | 0.014 | 0.156 |
| **891** | 0.040 | |
| **892** | 0.073 | 1.900 |
| **893** | 0.041 | |
| **894** | 0.180 | |
| **895** | 0.051 | 0.550 |
| **896** | 0.088 | 1.800 |
| **897** | 0.024 | 0.110 |
| **898** | 0.052 | 0.290 |
| **899** | 0.130 | 9.700 |
| **900** | 0.290 | >10 |
| **901** | 0.042 | 0.430 |
| **902** | 0.034 | 0.330 |
| **903** | 0.058 | 4.000 |
| **904** | 0.031 | 1.600 |
| **905** | 0.080 | 4.600 |
| **906** | 0.790 | |
| **907** | 0.040 | >1 |
| **908** | 0.123 | 6.200 |
| **909** | 0.215 | 8.000 |
| **910** | 0.022 | 3.000 |
| **911** | 0.021 | 0.173 |
| **912** | 0.140 | |
| **913** | 0.049 | 1.700 |
| **914** | 0.200 | |
| **915** | 0.029 | 4.200 |
| **916** | 0.006 | 0.200 |
| **917** | 0.017 | 1.563 |
| **918** | 0.054 | >1 |
| **919** | 0.025 | |
| **920** | 0.076 | |
| **921** | 0.093 | |
| **922** | 0.048 | |
| **923** | 0.056 | |
| **924** | 0.043 | |
| **925** | 0.035 | |
| **926** | 0.030 | |
| **927** | 0.069 | |
| **928** | 0.039 | |
| **929** | 0.200 | >1 |
| **930** | 0.061 | >10 |
| **931** | 0.072 | 9.600 |
| **932** | 0.860 | 1.700 |
| **933** | 0.560 | >10 |
| **934** | 0.079 | >1 |
| **935** | 0.630 | >1 |
| **936** | 0.721 | 18.700 |

## Claims

1. A compound of Formula **I** 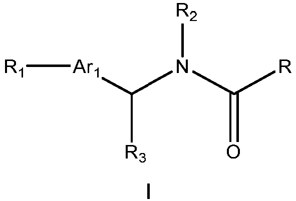 a tautomer thereof, a stereoisomer thereof, or a pharmaceutically acceptable salt of any of the foregoing, wherein:
R is a tricycle independently selected from the formulae **IA** and **IB**: 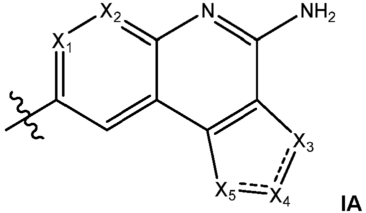 and 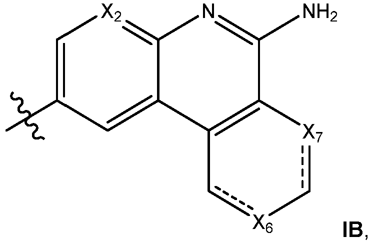
wherein 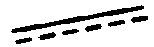 is a single or double bond,
X¹, X², X⁶ and X⁷ are in each instance N or C, wherein both X¹ and X² cannot be N at the same time, and wherein if X¹ is C, it can be optionally substituted with halo;
X³, X⁴ and X⁵ are at each instance independently selected from an optionally substituted C, O, N and S; wherein the substituents are independently selected from C₁₋₃ alkyl, C₁₋₃ alkyl(OH), wherein alkyl can be optionally substituted with halo;
R³ in each instance is independently selected from H or C₁₋₃ alkyl;
Ar¹ is a six membered optionally substituted aryl or heteroaryl independently selected from: 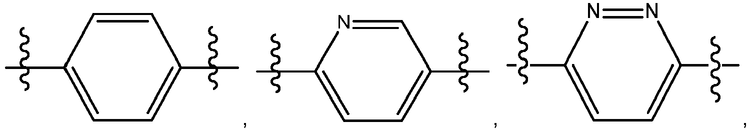 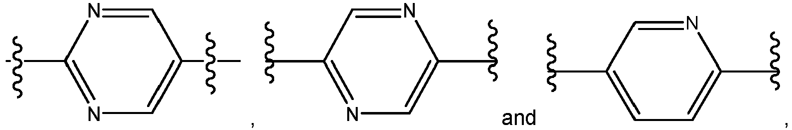
wherein the substituents are independently selected from C₁₋₃ alkyl, -OC₁₋₃ alkyl or halo;
R¹ in each instance is independently selected from H, halo, optionally substituted C₁₋₃ alkyl, wherein the substituents are selected from halo; -CN, optionally substituted -O-C₁₋₃ alkyl, wherein the substituents are selected from halo; -C(O)OC₁₋₃ alkyl, wherein C₁₋₃ alkyl can be optionally substituted with halo, and morpholinyl; and
R² in each instance is independently selected from an optionally substituted C₁₋₈ alkyl, wherein the substituents are selected from halo, hydroxy, amino, -O- C₁₋₃ alkyl or -CN; 5 or 6 membered cycle or heterocycle, optionally substituted with hydroxy, amino, an optionally substituted C₁₋₆ alkyl, wherein the substituents are selected from halo; an optionally substituted C₁₋₆ alkyl-O- C₁₋₃ alkyl, wherein the substituents are selected from halo; 5,6,7,8-tetrahydro-[1,2,4]triazolo[1,5-a]pyridinyl; C₁₋₃ alkyl-heterocyclyl, wherein the heterocyclyl is selected from optionally substituted 3,4-dihydro-2H-pyrano[2,3-c]pyridinyl; pyradazinyl, triazolyl, pyrimidinyl, tetrahydrofuranyl, 1H-pyrrolo[2,3-b]pyridinyl, cyclohexyl; wherein the substituents are selected from C₁₋₃ alkyl, -CN, and halo, or an optionally substituted C₁₋₆ alkyl-O- C₁₋₃ alkyl, wherein the substituents are selected from halo; optionally substituted phenyl, wherein the substituents are selected from halo or C₁₋₃ alkyl.

2. The compound of claim 1, the tautomer thereof, the stereoisomer thereof, or the pharmaceutically acceptable salt of any of the foregoing, wherein R¹ is a tricycle of formulae **IA.**

3. The compound of claim 1, the tautomer thereof, the stereoisomer thereof, or the pharmaceutically acceptable salt of any of the foregoing, wherein R¹ is a tricycle of formulae **IB.**

4. The compound of claim 1, the tautomer thereof, the stereoisomer thereof, or the pharmaceutically acceptable salt of any of the foregoing, wherein R is a tricycle of formula **IA** and X¹ and X² are both C.

5. The compound of claim 4, the tautomer thereof, the stereoisomer thereof, or the pharmaceutically acceptable salt of any of the foregoing, wherein X¹ is unsubstituted or substituted with halo.

6. The compound of claim 1, the tautomer thereof, the stereoisomer thereof, or the pharmaceutically acceptable salt of any of the foregoing, wherein R is a tricycle of formulae **IB** and X² is C or N.

7. The compound of claim 4, the tautomer thereof, the stereoisomer thereof, or the pharmaceutically acceptable salt of any of the foregoing, wherein R is a tricycle of the formulae **IA1** 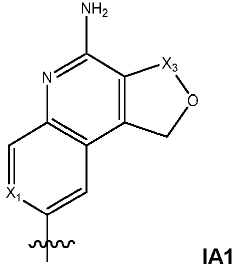 or R is a tricycle of the formulae **IA2**

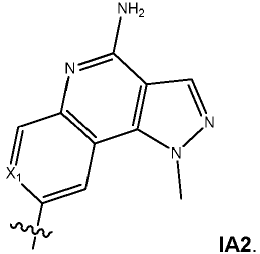

8. The compound of claim 7, the tautomer thereof, the stereoisomer thereof, or the pharmaceutically acceptable salt of any of the foregoing, wherein X³ is C, unsubstituted or substituted with methyl.

9. The compound of claim 1, the tautomer thereof, the stereoisomer thereof, or the pharmaceutically acceptable salt of any of the foregoing, wherein R³ is H or methyl.

10. The compound of claim 7, the tautomer thereof, the stereoisomer thereof, or the pharmaceutically acceptable salt of any of the foregoing, wherein R² is an optionally substituted C₁₋₈ alkyl, preferably an optionally substituted methyl, ethyl, isopropyl, or cycloC₁₋₆ alkyl.

11. The compound of claim 10, the tautomer thereof, the stereoisomer thereof, or the pharmaceutically acceptable salt of any of the foregoing, wherein Ar¹ is

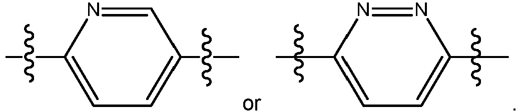

12. The compound of claim 11, wherein Ar¹ is 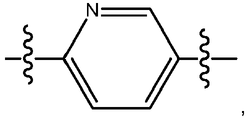 the tautomer thereof, the stereoisomer thereof, or the pharmaceutically acceptable salt of any of the foregoing, wherein R¹ is halo, or optionally substituted C₁₋₃ alkyl or -CN.

13. The compound of claim 1, the tautomer thereof, the stereoisomer thereof, or the pharmaceutically acceptable salt of any of the foregoing, wherein R is a tricycle of formulae **IB** and R¹ is halo, optionally substituted C₁₋₃ alkyl or -CN.

14. A compound, the tautomer thereof, the stereoisomer thereof, or the pharmaceutically acceptable salt of any of the foregoing, wherein the compound is selected from:
4-amino-N-(cyclopropylmethyl)-N-((5-(trifluoromethyl)-2-pyridinyl)methyl)-1,3-dihydrofuro[3,4-c]quinoline-8-carboxamide;
4-amino-N-((5-cyano-2-pyridinyl)methyl)-N-(2-propanyl)-1,3-dihydrofuro[3,4-c]quinoline-8-carboxamide;
4-amino-N-((6-cyclopropyl-3-pyridinyl)methyl)-N-(2-propanyl)-1,3-dihydrofuro[3,4-c]quinoline-8-carboxamide;
4-amino-N-((2R)-1-methoxy-2-propanyl)-N-((6-(trifluoromethyl)-3-pyridazinyl)methyl)-1,3-dihydrofuro[3,4-c]quinoline-8-carboxamide;
4-amino-N-((2S)-1-methoxy-2-propanyl)-N-((6-(trifluoromethyl)-3-pyridazinyl)methyl)-1,3-dihydrofuro[3,4-c]quinoline-8-carboxamide;
(3R)-4-amino-N-((2R)-1-methoxy-2-propanyl)-3-methyl-N-((6-(trifluoromethyl)-3-pyridazinyl)methyl)-1,3-dihydrofuro[3,4-c]quinoline-8-carboxamide;
(3R)-4-amino-N-((2S)-1-methoxy-2-propanyl)-3-methyl-N-((6-(trifluoromethyl)-3-pyridazinyl)methyl)-1,3-dihydrofuro[3,4-c]quinoline-8-carboxamide;
4-amino-N-((6-chloro-3-pyridazinyl)methyl)-N-((2R)-1-methoxy-2-propanyl)-1,3-dihydrofuro[3,4-c]quinoline-8-carboxamide;
4-amino-N-((6-chloro-3-pyridazinyl)methyl)-N-((2R)-1-methoxy-2-propanyl)-1,3-dihydrofuro[3,4-c][1,7]naphthyridine-8-carboxamide;
(3R)-4-amino-N-((6-chloro-3-pyridazinyl)methyl)-N-((2R)-1-methoxy-2-propanyl)-3-methyl-1,3-dihydrofuro[3,4-c]quinoline-8-carboxamide;
(3R)-4-amino-N-((6-chloro-3-pyridazinyl)methyl)-N-((2R)-1-methoxy-2-propanyl)-3-methyl-1,3-dihydrofuro[3,4-c][1,7]naphthyridine-8-carboxamide;
(3S)-4-amino-N-(bicyclo[1.1.1]pentan-1-yl)-3-methyl-N-((5-(trifluoromethyl)-2-pyridinyl)methyl)-1,3-dihydrofuro[3,4-c][1,7]naphthyridine-8-carboxamide;
(3R)-4-amino-3-methyl-N-(2-propanyl)-N-((5-(trifluoromethyl)-2-pyridinyl)methyl)-1,3-dihydrofuro[3,4-c][1,7]naphthyridine-8-carboxamide;
(3R)-4-amino-N-(bicyclo[1.1.1]pentan-1-yl)-3-methyl-N-((5-(trifluoromethyl)-2-pyridinyl)methyl)-1,3-dihydrofuro[3,4-c][1,7]naphthyridine-8-carboxamide;
(3R)-4-amino-N-((5-cyano-2-pyridinyl)methyl)-N,3-dimethyl-1,3-dihydrofuro[3,4-c][1,7]naphthyridine-8-carboxamide;
(3R)-4-amino-3-methyl-N-(2-propanyl)-N-((6-(trifluoromethyl)-3-pyridazinyl)methyl)-1,3-dihydrofuro[3,4-c][1,7]naphthyridine-8-carboxamide;
(3R)-4-amino-N-((2R)-1-methoxy-2-propanyl)-3-methyl-N-((6-(trifluoromethyl)-3-pyridazinyl)methyl)-1,3-dihydrofuro[3,4-c][1,7]naphthyridine-8-carboxamide;
(3R)-4-amino-N-((2S)-1-methoxy-2-propanyl)-3-methyl-N-((6-(trifluoromethyl)-3-pyridazinyl)methyl)-1,3-dihydrofuro[3,4-c][1,7]naphthyridine-8-carboxamide;
(3R)-4-amino-N-cyclopropyl-3-methyl-N-((5-(trifluoromethyl)-2-pyridinyl) methyl)- 1,3-dihydrofuro[3,4-c][1,7]naphthyridine-8-carboxamide;
(3R)-4-amino-N-((5-cyano-2-pyridinyl)methyl)-N-cyclopropyl-3-methyl-1,3-dihydrofuro[3,4-c][1,7]naphthyridine-8-carboxamide;
(3R)-4-amino-N,3-dimethyl-N-((6-(trifluoromethyl)-3-pyridazinyl)methyl)-1,3-dihydrofuro[3,4-c][1,7]naphthyridine-8-carboxamide;
(3S)-4-amino-N-cyclopropyl-3-methyl-N-((5-(trifluoromethyl)-2-pyridinyl)methyl)-1,3-dihydrofuro[3,4-c][1,7]naphthyridine-8-carboxamide;
(3S)-4-amino-N,3-dimethyl-N-((6-(trifluoromethyl)-3-pyridazinyl)methyl)-1,3-dihydrofuro[3,4-c][1,7]naphthyridine-8-carboxamide;
(3R)-4-amino-3-methyl-N-(2,2,2-trifluoroethyl)-N-((5-(trifluoromethyl)-2-pyridinyl)methyl)-1,3-dihydrofuro[3,4-c][1,7]naphthyridine-8-carboxamide;
4-amino-N-(cyclopropylmethyl)-N-((5-(trifluoromethyl)-2-pyridinyl)methyl)-1,3-dihydrofuro[3,4-c][1,8]naphthyridine-8-carboxamide;
4-amino-N-((2R)-1-methoxy-2-propanyl)-N-((5-(trifluoromethyl)-2-pyridinyl)methyl)-1,3-dihydrofuro[3,4-c][1,8]naphthyridine-8-carboxamide;
4-amino-N-((5-cyclopropyl-2-pyridinyl)methyl)-N-methyl-1,3-dihydrofuro[3,4-c]quinoline-8-carboxamide;
4-amino-N-((5-cyano-2-pyridinyl)methyl)-N-methyl-1,3-dihydrofuro[3,4-c]quinoline-8-carboxamide;
4-amino-N-((5-cyano-2-pyridinyl)methyl)-N-(cyclopropylmethyl)-1,3-dihydrofuro[3,4-c]quinoline-8-carboxamide;
(3R)-4-amino-N-((6-bromo-3-pyridazinyl)methyl)-N-(cyclopropylmethyl)-3-methyl-1,3-dihydrofuro[3,4-c]quinoline-8-carboxamide;
4-amino-N-((6-bromo-3-pyridazinyl)methyl)-N-methyl-1,3-dihydrofuro[3,4-c]quinoline-8-carboxamide;
(3R)-4-amino-N-((5-bromo-2-pyridinyl)methyl)-N,3-dimethyl-1,3-dihydrofuro[3,4-c]quinoline-8-carboxamide;
(3R)-4-amino-N-((6-cyclopropyl-3-pyridazinyl)methyl)-3-methyl-N-((1R)-1-(2-pyrimidinyl)ethyl)-1,3-dihydrofuro[3,4-c]quinoline-8-carboxamide;
4-amino-N-((5-bromo-2-pyridinyl)methyl)-N-methyl-1,3-dihydrofuro[3,4-c]quinoline-8-carboxamide;
(3R)-4-amino-N-((5-cyano-2-pyridinyl)methyl)-N-(cyclopropylmethyl)-3-methyl-1,3-dihydrofuro[3,4-c]quinoline-8-carboxamide;
(3R)-4-amino-N-((6-bromo-3-pyridazinyl)methyl)-3-methyl-N-(2-propanyl)-1,3-dihydrofuro[3,4-c]quinoline-8-carboxamide;
(3R)-4-amino-N-(cyclopropylmethyl)-3-methyl-N-((6-(trifluoromethyl)-3-pyridazinyl)methyl)-1,3-dihydrofuro[3,4-c]quinoline-8-carboxamide;
(3R)-4-amino-N-(cyclopropylmethyl)-3-methyl-N-((5-(trifluoromethyl)-2-pyridinyl)methyl)-1,3-dihydrofuro[3,4-c]quinoline-8-carboxamide;
4-amino-N-((6-bromo-3-pyridazinyl)methyl)-N-(2-propanyl)-1,3-dihydrofuro[3,4-c]quinoline-8-carboxamide;
4-amino-N-(cyclopropylmethyl)-N-((6-(trifluoromethyl)-3-pyridazinyl)methyl)-1,3-dihydrofuro[3,4-c]quinoline-8-carboxamide;
4-amino-N-((1-methylcyclopropyl)methyl)-N-((5-(trifluoromethyl)-2-pyridinyl)methyl)-1,3-dihydrofuro[3,4-c]quinoline-8-carboxamide;
(3R)-4-amino-N-(2,2-dimethylpropyl)-3-methyl-N-((5-(trifluoromethyl)-2-pyridinyl)methyl)-1,3-dihydrofuro[3,4-c]quinoline-8-carboxamide;
(3R)-4-amino-3-methyl-N-((1-methylcyclopropyl)methyl)-N-((5-(trifluoromethyl)-2-pyridinyl)methyl)-1,3-dihydrofuro[3,4-c]quinoline-8-carboxamide;
4-amino-N-(2,2-dimethylpropyl)-3,3-dimethyl-N-((5-(trifluoromethyl)-2-pyridinyl)methyl)-1,3-dihydrofuro[3,4-c]quinoline-8-carboxamide;
4-amino-3,3-dimethyl-N-((1-methylcyclopropyl)methyl)-N-((5-(trifluoromethyl)-2-pyridinyl)methyl)-1,3-dihydrofuro[3,4-c]quinoline-8-carboxamide;
4-amino-N-(2,2-dimethylpropyl)-N-((5-(trifluoromethyl)-2-pyridinyl)methyl)-1,3-dihydrofuro[3,4-c]quinoline-8-carboxamide;
4-amino-N-cyclopropyl-N-((5-cyclopropyl-2-pyridinyl)methyl)-1,3-dihydrofuro[3,4-c]quinoline-8-carboxamide;
4-amino-N-methyl-N-((5-(trifluoromethyl)-2-pyridinyl)methyl)-1,3-dihydrofuro[3,4-c][1,7]naphthyridine-8-carboxamide;
4-amino-7-fluoro-N-(2-propanyl)-N-((5-(trifluoromethyl)-2-pyridinyl)methyl)-1,3-dihydrofuro[3,4-c]quinoline-8-carboxamide;
4-amino-N-((5-cyano-2-pyridinyl)methyl)-N-(2-propanyl)-1,3-dihydrofuro[3,4-c][1,7]naphthyridine-8-carboxamide;
4-amino-N-((5-cyano-2-pyridinyl)methyl)-N-cyclopropyl-1,3-dihydrofuro[3,4-c][1,7]naphthyridine-8-carboxamide;
4-amino-N-cyclopropyl-N-((5-cyclopropyl-2-pyridinyl)methyl)-1,3-dihydrofuro[3,4-c][1,7]naphthyridine-8-carboxamide;
4-amino-N-((5-cyclopropyl-2-pyridinyl)methyl)-N-methyl-1,3-dihydrofuro[3,4-c][1,7]naphthyridine-8-carboxamide;
(3R)-4-amino-N-((6-cyclopropyl-3-pyridinyl)methyl)-3-methyl-N-(2-propanyl)-1,3-dihydrofuro[3,4-c]quinoline-8-carboxamide;
(3R)-4-amino-N-cyclopropyl-3-methyl-N-((5-(trifluoromethyl)-2-pyridinyl) methyl)- 1,3-dihydrofuro[3,4-c]quinoline-8-carboxamide;
4-amino-N-methyl-N-((6-(trifluoromethyl)-3-pyridazinyl)methyl)-1,3-dihydrofuro[3,4-c]quinoline-8-carboxamide;
(3R)-4-amino-N-cyclopropyl-3-methyl-N-((6-(trifluoromethyl)-3-pyridazinyl)methyl)-1,3-dihydrofuro[3,4-c]quinoline-8-carboxamide;
(3R)-4-amino-N-((5-cyano-2-pyridinyl)methyl)-3-methyl-N-(2-propanyl)-1,3-dihydrofuro[3,4-c]quinoline-8-carboxamide;
(3R)-4-amino-N-((5-cyano-2-pyridinyl)methyl)-N-cyclopropyl-3-methyl-1,3-dihydrofuro[3,4-c]quinoline-8-carboxamide;
(3R)-4-amino-N-((5-cyclopropyl-2-pyridinyl)methyl)-N,3-dimethyl-1,3-dihydrofuro[3,4-c]quinoline-8-carboxamide;
4-amino-N-cyclopropyl-7-fluoro-N-((6-(trifluoromethyl)-3-pyridazinyl)methyl)-1,3-dihydrofuro[3,4-c]quinoline-8-carboxamide;
4-amino-7-fluoro-N-(2-propanyl)-N-((6-(trifluoromethyl)-3-pyridazinyl)methyl)-1,3-dihydrofuro[3,4-c]quinoline-8-carboxamide;
4-amino-7-fluoro-N-methyl-N-((6-(trifluoromethyl)-3-pyridazinyl)methyl)-1,3-dihydrofuro[3,4-c]quinoline-8-carboxamide;
(3R)-4-amino-N-ethyl-3-methyl-N-((5-(trifluoromethyl)-2-pyridinyl)methyl)-1,3-dihydrofuro[3,4-c]quinoline-8-carboxamide;
4-amino-N-ethyl-N-((5-(trifluoromethyl)-2-pyridinyl)methyl)-1,3-dihydrofuro[3,4-c][1,7]naphthyridine-8-carboxamide;
4-amino-N-ethyl-N-((5-(trifluoromethyl)-2-pyridinyl)methyl)-1,3-dihydrofuro[3,4-c]quinoline-8-carboxamide;
4-amino-N-((6-cyclopropyl-3-pyridinyl)methyl)-7-fluoro-N-(2-propanyl)-1,3-dihydrofuro[3,4-c]quinoline-8-carboxamide;
4-amino-N-((5-cyclopropyl-2-pyridinyl)methyl)-N-(2-propanyl)-1,3-dihydrofuro[3,4-c]quinoline-8-carboxamide;
4-amino-N-cyclopropyl-N-((5-(trifluoromethyl)-2-pyridinyl)methyl)-1,3-dihydrofuro[3,4-c][1,7]naphthyridine-8-carboxamide;
4-amino-N-(2-propanyl)-N-((6-(trifluoromethyl)-3-pyridazinyl)methyl)-1,3-dihydrofuro[3,4-c][1,7]naphthyridine-8-carboxamide;
4-amino-N-((5-cyano-2-pyridinyl)methyl)-N-methyl-1,3-dihydrofuro[3,4-c][1,7]naphthyridine-8-carboxamide;
(3R)-4-amino-N-cyclopropyl-N-((5-cyclopropyl-2-pyridinyl)methyl)-3-methyl-1,3-dihydrofuro[3,4-c]quinoline-8-carboxamide;
4-amino-N-cyclopropyl-7-fluoro-N-((5-(trifluoromethyl)-2-pyridinyl)methyl)-1,3-dihydrofuro[3,4-c]quinoline-8-carboxamide;
4-amino-N-cyclopropyl-N-((5-cyclopropyl-2-pyridinyl)methyl)-7-fluoro-1,3-dihydrofuro[3,4-c]quinoline-8-carboxamide;
4-amino-N-((5-cyclopropyl-2-pyridinyl)methyl)-7-fluoro-N-(2-propanyl)-1,3-dihydrofuro[3,4-c]quinoline-8-carboxamide;
4-amino-N-((5-cyclopropyl-2-pyridinyl)methyl)-7-fluoro-N-methyl-1,3-dihydrofuro[3,4-c]quinoline-8-carboxamide;
4-amino-N-((6-ethoxy-3-pyridazinyl)methyl)-N-((2R)-1-methoxy-2-propanyl)-1,3-dihydrofuro[3,4-c]quinoline-8-carboxamide;
(3R)-4-amino-N-((6-ethoxy-3-pyridazinyl)methyl)-N-((2R)-1-methoxy-2-propanyl)-3-methyl-1,3-dihydrofuro[3,4-c]quinoline-8-carboxamide;
4-amino-N-methyl-N-((6-(trifluoromethyl)-3-pyridazinyl)methyl)-1,3-dihydrofuro[3,4-c][1,7]naphthyridine-8-carboxamide;
(3R)-4-amino-N,3-dimethyl-N-((5-(trifluoromethyl)-2-pyridinyl)m ethyl)- 1,3-dihydrofuro[3,4-c]quinoline-8-carboxamide;
(3R)-4-amino-N-((5-cyclopropyl-2-pyridinyl)methyl)-3-methyl-N-(2-propanyl)-1,3-dihydrofuro[3,4-c]quinoline-8-carboxamide;
4-amino-N-(bicyclo[1.1.1]pentan-1-yl)-N-((6-ethoxy-3-pyridazinyl)methyl)-1,3-dihydrofuro[3,4-c][1,7]naphthyridine-8-carboxamide;
4-amino-7-chloro-N-((5-cyclopropyl-2-pyridinyl)methyl)-N-(2-propanyl)-1,3-dihydrofuro[3,4-c]quinoline-8-carboxamide;
(3R)-4-amino-N-(bicyclo[1.1.1]pentan-1-yl)-3-methyl-N-((5-(trifluoromethyl)-2-pyridinyl)methyl)-1,3-dihydrofuro[3,4-c]quinoline-8-carboxamide;
4-amino-N-(bicyclo[1.1.1]pentan-1-yl)-7-fluoro-N-((5-(trifluoromethyl)-2-pyridinyl)methyl)-1,3-dihydrofuro[3,4-c]quinoline-8-carboxamide;
4-amino-N-cyclopropyl-N-((6-(trifluoromethyl)-3-pyridazinyl)methyl)-1,3-dihydrofuro[3,4-c][1,7]naphthyridine-8-carboxamide;
4-amino-N-((6-cyclopropyl-3-pyridinyl)methyl)-N-(2-propanyl)-1,3-dihydrofuro[3,4-c][1,7]naphthyridine-8-carboxamide;
(3R)-4-amino-3-methyl-N-(2-propanyl)-N-((5-(trifluoromethyl)-2-pyridinyl)methyl)-1,3-dihydrofuro[3,4-c]quinoline-8-carboxamide;
4-amino-N-((5-cyclopropyl-2-pyridinyl)methyl)-N-(2-propanyl)-1,3-dihydrofuro[3,4-c][1,7]naphthyridine-8-carboxamide;
(3R)-4-amino-N-((5-cyano-2-pyridinyl)methyl)-N,3-dimethyl-1,3-dihydrofuro[3,4-c]quinoline-8-carboxamide;
4-amino-N-(bicyclo[1.1.1]pentan-1-yl)-N-((6-ethoxy-3-pyridazinyl)methyl)-1,3-dihydrofuro[3,4-c]quinoline-8-carboxamide;
4-amino-N-((6-ethoxy-3-pyridazinyl)methyl)-N-((2R)-1-methoxy-2-propanyl)-1,3-dihydrofuro[3,4-c][1,7]naphthyridine-8-carboxamide;
4-amino-7-chloro-N-methyl-N-((6-(trifluoromethyl)-3-pyridazinyl)methyl)-1,3-dihydrofuro[3,4-c]quinoline-8-carboxamide;
4-amino-N-((2R)-1-methoxy-2-propanyl)-N-((6-(trifluoromethyl)-3-pyridazinyl)methyl)-1,3-dihydrofuro[3,4-c][1,7]naphthyridine-8-carboxamide;
4-amino-N-((2S)-1-methoxy-2-propanyl)-N-((6-(trifluoromethyl)-3-pyridazinyl)methyl)-1,3-dihydrofuro[3,4-c][1,7]naphthyridine-8-carboxamide;
4-amino-N-(2-propanyl)-N-((6-(trifluoromethyl)-3-pyridazinyl)methyl)-1,3-dihydrofuro[3,4-c]quinoline-8-carboxamide;
4-amino-7-chloro-N-((5-cyano-2-pyridinyl)methyl)-N-(2-propanyl)-1,3-dihydrofuro[3,4-c]quinoline-8-carboxamide;
(3R)-4-amino-3-methyl-N-(2-propanyl)-N-((6-(trifluoromethyl)-3-pyridazinyl)methyl)-1,3-dihydrofuro[3,4-c]quinoline-8-carboxamide;
4-amino-7-chloro-N-(2-propanyl)-N-((6-(trifluoromethyl)-3-pyridazinyl)methyl)-1,3-dihydrofuro[3,4-c]quinoline-8-carboxamide;
4-amino-N-(bicyclo[1.1.1]pentan-1-yl)-7-chloro-N-((5-(trifluoromethyl)-2-pyridinyl)methyl)-1,3-dihydrofuro[3,4-c]quinoline-8-carboxamide;
(3R)-4-amino-N-(cyclopropylmethyl)-3-methyl-N-((5-(trifluoromethyl)-2-pyridinyl)methyl)-1,3-dihydrofuro[3,4-c][1,7]naphthyridine-8-carboxamide;
(3R)-4-amino-N-((6-ethoxy-3-pyridazinyl)methyl)-N-((2R)-1-methoxy-2-propanyl)-3-methyl-1,3-dihydrofuro[3,4-c][1,7]naphthyridine-8-carboxamide;
4-amino-N-((1-methylcyclopropyl)methyl)-N-((5-(trifluoromethyl)-2-pyridinyl)methyl)-1,3-dihydrofuro[3,4-c][1,7]naphthyridine-8-carboxamide;
4-amino-N-methyl-N-(4-(trifluoromethyl)benzyl)-1,3-dihydrofuro[3,4-c]quinoline-8-carboxamide;
4-amino-N-methyl-N-((6-(trifluoromethyl)-3-pyridinyl)methyl)-1,3-dihydrofuro[3,4-c]quinoline-8-carboxamide;
4-amino-7-chloro-N-((6-cyclopropyl-3-pyridinyl)methyl)-N-(2-propanyl)-1,3-dihydrofuro[3,4-c]quinoline-8-carboxamide;
(3R)-4-amino-N-(cyclopropylmethyl)-3-methyl-N-((6-(trifluoromethyl)-3-pyridazinyl)methyl)-1,3-dihydrofuro[3,4-c][1,7]naphthyridine-8-carboxamide;
(3S)-4-amino-N-((6-cyclopropyl-3-pyridinyl)methyl)-3-methyl-N-(2-propanyl)-1,3-dihydrofuro[3,4-c][1,7]naphthyridine-8-carboxamide;
(3R)-4-amino-N-((5-cyano-2-pyridinyl)methyl)-3-methyl-N-(2-propanyl)-1,3-dihydrofuro[3,4-c][1,7]naphthyridine-8-carboxamide;
(3R)-4-amino-N-cyclopropyl-3-methyl-N-((6-(trifluoromethyl)-3-pyridazinyl)methyl)-1,3-dihydrofuro[3,4-c][1,7]naphthyridine-8-carboxamide;
(3R)-4-amino-N-((5-cyclopropyl-2-pyridinyl)methyl)-3-methyl-N-(2-propanyl)-1,3-dihydrofuro[3,4-c][1,7]naphthyridine-8-carboxamide;
(3S)-4-amino-N-((5-cyclopropyl-2-pyridinyl)methyl)-3-methyl-N-(2-propanyl)-1,3-dihydrofuro[3,4-c][1,7]naphthyridine-8-carboxamide;
(3R)-4-amino-N-ethyl-3-methyl-N-((5-(trifluoromethyl)-2-pyridinyl)methyl)-1,3-dihydrofuro[3,4-c][1,7]naphthyridine-8-carboxamide;
(3R)-4-amino-N-(bicyclo[1.1.1]pentan-1-yl)-N-((6-ethoxy-3-pyridazinyl)methyl)-3-methyl-1,3-dihydrofuro[3,4-c][1,7]naphthyridine-8-carboxamide;
(3R)-4-amino-N-((6-bromo-3-pyridazinyl)methyl)-N-((2R)-1-methoxy-2-propanyl)-3-methyl-1,3-dihydrofuro[3,4-c][1,7]naphthyridine-8-carboxamide;
(3R)-4-amino-N,3-dimethyl-N-((5-(trifluoromethyl)-2-pyridinyl)methyl)-1,3-dihydrofuro[3,4-c][1,7]naphthyridine-8-carboxamide;
(3R)-4-amino-N-((6-cyclopropyl-3-pyridinyl)methyl)-3-methyl-N-(2-propanyl)-1,3-dihydrofuro[3,4-c][1,7]naphthyridine-8-carboxamide;
methyl 4-(6-((((4-amino-1,3-dihydrofuro[3,4-c]quinolin-8-yl)carbonyl)(methyl)amino)methyl)-3-pyridinyl)-1-piperazinecarboxylate;
(3S)-4-amino-N-((5-cyclopropyl-2-pyridinyl)methyl)-N,3-dimethyl-1,3-dihydrofuro[3,4-c][1,7]naphthyridine-8-carboxamide;
(3R)-4-amino-N-((5-cyclopropyl-2-pyridinyl)methyl)-N,3-dimethyl-1,3-dihydrofuro[3,4-c][1,7]naphthyridine-8-carboxamide;
(3R)-4-amino-N-cyclopropyl-N-((5-cyclopropyl-2-pyridinyl)methyl)-3-methyl-1,3-dihydrofuro[3,4-c][1,7]naphthyridine-8-carboxamide;
(3S)-4-amino-N-cyclopropyl-N-((5-cyclopropyl-2-pyridinyl)methyl)-3-methyl-1,3-dihydrofuro[3,4-c][1,7]naphthyridine-8-carboxamide;
4-amino-N-(cyclopropylmethyl)-N-((6-(difluoromethoxy)-3-pyridazinyl)methyl)-1,3-dihydrofuro[3,4-c][1,7]naphthyridine-8-carboxamide;
4-amino-N-ethyl-7-fluoro-N-((5-(trifluoromethyl)-2-pyridinyl)methyl)-1,3-dihydrofuro[3,4-c]quinoline-8-carboxamide;
4-amino-7-chloro-N-ethyl-N-((5-(trifluoromethyl)-2-pyridinyl)methyl)-1,3-dihydrofuro[3,4-c]quinoline-8-carboxamide;
(3R)-4-amino-N-((5-chloro-2-pyridinyl)methyl)-N-ethyl-3-methyl-1,3-dihydrofuro[3,4-c]quinoline-8-carboxamide;
4-amino-N-ethyl-3-methyl-N-((5-(trifluoromethyl)-2-pyridinyl)methyl)-3H-pyrazolo[3,4-c]quinoline-8-carboxamide;
(3R)-4-amino-N-((5-chloro-2-pyridinyl)methyl)-3-methyl-N-(2-propanyl)-1,3-dihydrofuro[3,4-c]quinoline-8-carboxamide;
(3R)-4-amino-N-(cyclopropylmethyl)-N-((6-(difluoromethoxy)-3-pyridazinyl)methyl)-3-methyl-1,3-dihydrofuro[3,4-c][1,7]naphthyridine-8-carboxamide;
(3S)-4-amino-N-(cyclopropylmethyl)-N-((6-(difluoromethoxy)-3-pyridazinyl)methyl)-3-methyl-1,3-dihydrofuro[3,4-c][1,7]naphthyridine-8-carboxamide;
4-amino-N-(cyclopropylmethyl)-N-((6-(difluoromethoxy)-3-pyridazinyl)methyl)-1,3-dihydrofuro[3,4-c]quinoline-8-carboxamide;
4-amino-N-ethyl-7-fluoro-3-methyl-N-((5-(trifluoromethyl)-2-pyridinyl)methyl)-3H-pyrazolo[3,4-c]quinoline-8-carboxamide;
5-amino-N-ethyl-N-((5-(trifluoromethyl)-2-pyridinyl)methyl)benzo[c][2,6]naphthyridine-9-carboxamide;
4-amino-N-((5-(3,6-dihydro-2H-pyran-4-yl)-2-pyridinyl)methyl)-N-methyl-1,3-dihydrofuro[3,4-c]quinoline-8-carboxamide;
methyl 6-((((4-amino-1,3-dihydrofuro[3,4-c]quinolin-8-yl)carbonyl)(methyl)amino)methyl)-3',6'-dihydro[3,4'-bipyridine]-1'(2'H)-carboxylate;
5-oxo-N-(2-propanyl)-N-((5-(trifluoromethyl)-2-pyridinyl)methyl)-5,6-dihydropyrazolo[1,5-c]quinazoline-9-carboxamide;
4-amino-1,3-dimethyl-N-(2-propanyl)-N-((5-(trifluoromethyl)-2-pyridinyl)methyl)-1H-pyrazolo[4,3-c]quinoline-8-carboxamide;
(3R)-4-amino-N-((5-bromo-2-pyridinyl)methyl)-N,3-dimethyl-1,3-dihydrofuro[3,4-c][1,7]naphthyridine-8-carboxamide;
(3S)-4-amino-N-((5-bromo-2-pyridinyl)methyl)-N,3-dimethyl-1,3-dihydrofuro[3,4-c][1,7]naphthyridine-8-carboxamide;
(3R)-4-amino-3-methyl-N-(2-propanyl)-N-((5-(trifluoromethyl)-2-pyrazinyl)methyl)-1,3-dihydrofuro[3,4-c]quinoline-8-carboxamide;
4-amino-7-fluoro-N-((1R)-1-(2-pyrimidinyl)ethyl)-N-((5-(trifluoromethyl)-2-pyridinyl)methyl)-1,3-dihydrofuro[3,4-c]quinoline-8-carboxamide;
4-amino-N-((5-cyano-2-pyridinyl)methyl)-7-fluoro-N-((1R)-1-(2-pyrimidinyl)ethyl)-1,3-dihydrofuro[3,4-c]quinoline-8-carboxamide;
4-amino-N-((6-bromo-3-pyridazinyl)methyl)-7-fluoro-N-((1R)-1-(3-fluoro-2-pyridinyl)ethyl)-1,3-dihydrofuro[3,4-c]quinoline-8-carboxamide;
4-amino-N-((5-(difluoromethyl)-2-pyridinyl)methyl)-N-((1R)-1-(2-pyrimidinyl)ethyl)-1,3-dihydrofuro[3,4-c]quinoline-8-carboxamide;
4-amino-N-((5-chloro-2-pyridinyl)methyl)-N-((1R)-1-(2-pyrimidinyl)ethyl)-1,3-dihydrofuro[3,4-c]quinoline-8-carboxamide;
4-amino-N-((1S)-1-(2-pyrimidinyl)ethyl)-N-((6-(trifluoromethyl)-3-pyridazinyl)methyl)-1,3-dihydrofuro[3,4-c]quinoline-8-carboxamide;
4-amino-N-((1R)-1-(2-pyrimidinyl)ethyl)-N-((6-(trifluoromethyl)-3-pyridazinyl)methyl)-1,3-dihydrofuro[3,4-c]quinoline-8-carboxamide;
4-amino-N-((1R)-1-(3-fluoro-2-pyridinyl)ethyl)-N-((6-(trifluoromethyl)-3-pyridazinyl)methyl)-1,3-dihydrofuro[3,4-c]quinoline-8-carboxamide;
4-amino-N-cyclopropyl-N-((5-(trifluoromethyl)-2-pyridinyl)methyl)-1,3-dihydrofuro[3,4-c]quinoline-8-carboxamide;
4-amino-N-(1,3-dimethoxy-2-propanyl)-N-((5-(trifluoromethyl)-2-pyridinyl)methyl)-1,3-dihydrofuro[3,4-c]quinoline-8-carboxamide;
4-amino-N-((2R)-1-methoxy-2-propanyl)-N-((5-(trifluoromethyl)-2-pyridinyl)methyl)-1,3-dihydrofuro[3,4-c]quinoline-8-carboxamide;
4-amino-N-((6-methoxy-3-pyridazinyl)methyl)-N-((1R)-1-(2-pyrimidinyl)ethyl)-1,3-dihydrofuro[3,4-c]quinoline-8-carboxamide;
4-amino-N-((8R)-5,6,7,8-tetrahydro[1,2,4]triazolo[1,5-a]pyridin-8-yl)-N-((5-(trifluoromethyl)-2-pyridinyl)methyl)-1,3-dihydrofuro[3,4-c]quinoline-8-carboxamide;
4-amino-N-((8S)-5,6,7,8-tetrahydro[1,2,4]triazolo[1,5-a]pyridin-8-yl)-N-((5-(trifluoromethyl)-2-pyridinyl)methyl)-1,3-dihydrofuro[3,4-c]quinoline-8-carboxamide;
4-amino-N-((5-cyano-2-pyridinyl)methyl)-N-((8R)-5,6,7,8-tetrahydro[1,2,4]triazolo[1,5-a]pyridin-8-yl)-1,3-dihydrofuro[3,4-c]quinoline-8-carboxamide;
4-amino-N-((5-cyano-2-pyridinyl)methyl)-N-((8S)-5,6,7,8-tetrahydro[1,2,4]triazolo[1,5-a]pyridin-8-yl)-1,3-dihydrofuro[3,4-c]quinoline-8-carboxamide;
4-amino-N-(4-(3-oxetanyl)benzyl)-N-((1R)-1-(2-pyrimidinyl)ethyl)-1,3-dihydrofuro[3,4-c]quinoline-8-carboxamide;
4-amino-N-(4-(3-oxetanyl)benzyl)-N-((1S)-1-(2-pyrimidinyl)ethyl)-1,3-dihydrofuro[3,4-c]quinoline-8-carboxamide;
4-amino-N-methyl-N-((5-(trifluoromethyl)-2-pyridinyl)methyl)-1,3-dihydrofuro[3,4-c]quinoline-8-carboxamide;
4-amino-N-(2-propanyl)-N-((5-(trifluoromethyl)-2-pyridinyl)methyl)-1,3-dihydrofuro[3,4-c]quinoline-8-carboxamide;
4-amino-N-(cyclopropylmethyl)-N-((6-(4-morpholinyl)-3-pyridazinyl)methyl)-1,3-dihydrofuro[3,4-c]quinoline-8-carboxamide;
4-amino-N-((1R)-1-(2-pyrimidinyl)ethyl)-N-((6-(2,2,2-trifluoroethoxy)-3-pyridazinyl)methyl)-1,3-dihydrofuro[3,4-c]quinoline-8-carboxamide;
4-amino-N-((5-cyano-2-pyridinyl)methyl)-N-cyclopropyl-1,3-dihydrofuro[3,4-c]quinoline-8-carboxamide;
4-amino-N-((5-bromo-2-pyridinyl)methyl)-N-cyclopropyl-1,3-dihydrofuro[3,4-c]quinoline-8-carboxamide;
4-amino-N-cyclopropyl-N-((5-(difluoromethyl)-2-pyridinyl)methyl)-1,3-dihydrofuro[3,4-c]quinoline-8-carboxamide;
4-amino-N-((1-cyanocyclopropyl)methyl)-N-((5-cyano-2-pyridinyl)methyl)-1,3-dihydrofuro[3,4-c]quinoline-8-carboxamide;
4-amino-N-((6-cyclopropyl-3-pyridazinyl)methyl)-N-((1R)-1-(2-pyrimidinyl)ethyl)-1,3-dihydrofuro[3,4-c]quinoline-8-carboxamide;
4-amino-N-((3R,4S)-3-methoxytetrahydro-2H-pyran-4-yl)-N-((5-(trifluoromethyl)-2-pyridinyl)methyl)-1,3-dihydrofuro[3,4-c]quinoline-8-carboxamide;
4-amino-N-((3S,4R)-3-methoxytetrahydro-2H-pyran-4-yl)-N-((5-(trifluoromethyl)-2-pyridinyl)methyl)-1,3-dihydrofuro[3,4-c]quinoline-8-carboxamide;
4-amino-N-((3R,4R)-4-methoxytetrahydro-2H-pyran-3-yl)-N-((5-(trifluoromethyl)-2-pyridinyl)methyl)-1,3-dihydrofuro[3,4-c]quinoline-8-carboxamide;
4-amino-N-((3S,4S)-4-methoxytetrahydro-2H-pyran-3-yl)-N-((5-(trifluoromethyl)-2-pyridinyl)methyl)-1,3-dihydrofuro[3,4-c]quinoline-8-carboxamide;
4-amino-7-chloro-N-((5-cyano-2-pyridinyl)methyl)-N-cyclopropyl-1,3-dihydrofuro[3,4-c]quinoline-8-carboxamide;
4-amino-N-((6-chloro-5-methoxy-2-pyridinyl)methyl)-N-(2-propanyl)-1,3-dihydrofuro[3,4-c]quinoline-8-carboxamide;
4-amino-N-((6-bromo-3-pyridazinyl)methyl)-N-((2R)-1-methoxy-2-propanyl)-1,3-dihydrofuro[3,4-c]quinoline-8-carboxamide;
4-amino-7-chloro-N-(cyclopropylmethyl)-N-((5-(trifluoromethyl)-2-pyridinyl)methyl)-1,3-dihydrofuro[3,4-c]quinoline-8-carboxamide;
(3R)-4-amino-3-methyl-N-(2-propanyl)-N-((5-(trifluoromethoxy)-2-pyridinyl)methyl)-1,3-dihydrofuro[3,4-c]quinoline-8-carboxamide;
(3R)-4-amino-N-((5-(difluoromethyl)-2-pyridinyl)methyl)-3-methyl-N-((1R)-1-(1-methyl-1H-1,2,4-triazol-3-yl)ethyl)-1,3-dihydrofuro[3,4-c]quinoline-8-carboxamide;
(3R)-4-amino-3-methyl-N-(1-methyl-1H-pyrazol-4-yl)-N-((5-(trifluoromethyl)-2-pyridinyl)methyl)-1,3-dihydrofuro[3,4-c]quinoline-8-carboxamide;
(3R)-4-amino-N-((6-bromo-3-pyridazinyl)methyl)-N-((2R)-1-methoxy-2-propanyl)-3-methyl-1,3-dihydrofuro[3,4-c]quinoline-8-carboxamide;
4-amino-N-(3,4-dihydro-2H-pyrano[2,3-c]pyridin-6-ylmethyl)-N-((1R)-1-(2-pyrimidinyl)ethyl)-1,3-dihydrofuro[3,4-c]quinoline-8-carboxamide;
4-amino-7-chloro-N-methyl-N-((5-(trifluoromethyl)-2-pyridinyl)methyl)-1,3-dihydrofuro[3,4-c]quinoline-8-carboxamide;
4-amino-N-(bicyclo[1.1.1]pentan-1-yl)-N-((5-(trifluoromethyl)-2-pyridinyl)methyl)-1,3-dihydrofuro[3,4-c]quinoline-8-carboxamide;
4-amino-7-chloro-N-((5-cyano-2-pyridinyl)methyl)-N-methyl-1,3-dihydrofuro[3,4-c]quinoline-8-carboxamide;
4-amino-N-((5-cyano-2-pyridinyl)methyl)-N-((2R)-1-fluoro-2-propanyl)-1,3-dihydrofuro[3,4-c]quinoline-8-carboxamide;
4-amino-N-((5-cyano-2-pyridinyl)methyl)-N-((2S)-1-fluoro-2-propanyl)-1,3-dihydrofuro[3,4-c]quinoline-8-carboxamide;
4-amino-7-chloro-N-((5-cyano-2-pyridinyl)methyl)-N-(cyclopropylmethyl)-1,3-dihydrofuro[3,4-c]quinoline-8-carboxamide;
4-amino-7-fluoro-N-methyl-N-((5-(trifluoromethyl)-2-pyridinyl)methyl)-1,3-dihydrofuro[3,4-c]quinoline-8-carboxamide;
4-amino-N-((5-cyano-2-pyridinyl)methyl)-N-cyclopropyl-7-fluoro-1,3-dihydrofuro[3,4-c]quinoline-8-carboxamide;
4-amino-N-((5-cyano-2-pyridinyl)methyl)-7-fluoro-N-(2-propanyl)-1,3-dihydrofuro[3,4-c]quinoline-8-carboxamide;
4-amino-7-chloro-N-((5-cyclopropyl-2-pyridinyl)methyl)-N-methyl-1,3-dihydrofuro[3,4-c]quinoline-8-carboxamide;
4-amino-N-((5-cyano-2-pyridinyl)methyl)-7-fluoro-N-methyl-1,3-dihydrofuro[3,4-c]quinoline-8-carboxamide;
4-amino-7-chloro-N-cyclopropyl-N-((5-cyclopropyl-2-pyridinyl)methyl)-1,3-dihydrofuro[3,4-c]quinoline-8-carboxamide;
(3R)-4-amino-3-methyl-N-(2,2,2-trifluoroethyl)-N-((5-(trifluoromethyl)-2-pyridinyl)methyl)-1,3-dihydrofuro[3,4-c]quinoline-8-carboxamide;
(3R)-4-amino-N-(bicyclo[1.1.1]pentan-1-yl)-N-((6-ethoxy-3-pyridazinyl)methyl)-3-methyl-1,3-dihydrofuro[3,4-c]quinoline-8-carboxamide;
4-amino-N-(2,2,2-trifluoroethyl)-N-((5-(trifluoromethyl)-2-pyridinyl)methyl)-1,3-dihydrofuro[3,4-c]quinoline-8-carboxamide;
4-amino-7-chloro-N-(2-propanyl)-N-((5-(trifluoromethyl)-2-pyridinyl)methyl)-1,3-dihydrofuro[3,4-c]quinoline-8-carboxamide;
(3R)-4-amino-N,3-dimethyl-N-((6-(trifluoromethyl)-3-pyridazinyl)methyl)-1,3-dihydrofuro[3,4-c]quinoline-8-carboxamide;
4-amino-N-(2,2,2-trifluoroethyl)-N-((5-(trifluoromethyl)-2-pyridinyl)methyl)-1,3-dihydrofuro[3,4-c][1,7]naphthyridine-8-carboxamide;
4-amino-7-chloro-N-((2R)-1-methoxy-2-propanyl)-N-((6-(trifluoromethyl)-3-pyridazinyl)methyl)-1,3-dihydrofuro[3,4-c]quinoline-8-carboxamide;
4-amino-7-chloro-N-((2S)-1-methoxy-2-propanyl)-N-((6-(trifluoromethyl)-3-pyridazinyl)methyl)-1,3-dihydrofuro[3,4-c]quinoline-8-carboxamide;
4-amino-7-fluoro-N-((2R)-1-methoxy-2-propanyl)-N-((6-(trifluoromethyl)-3-pyridazinyl)methyl)-1,3-dihydrofuro[3,4-c]quinoline-8-carboxamide;
4-amino-7-fluoro-N-((2S)-1-methoxy-2-propanyl)-N-((6-(trifluoromethyl)-3-pyridazinyl)methyl)-1,3-dihydrofuro[3,4-c]quinoline-8-carboxamide;
(3R)-4-amino-3-methyl-N-(tetrahydro-2H-pyran-4-yl)-N-((5-(trifluoromethyl)-2-pyridinyl)methyl)-1,3-dihydrofuro[3,4-c]quinoline-8-carboxamide;
4-amino-7-chloro-N-cyclopropyl-N-((6-(trifluoromethyl)-3-pyridazinyl)methyl)-1,3-dihydrofuro[3,4-c]quinoline-8-carboxamide;
6-amino-N-(1-methyl-1H-pyrazol-4-yl)-N-((5-(trifluoromethyl)-2-pyridinyl)methyl)-8,9-dihydro-7H-cyclopenta[c][1,7]naphthyridine-2-carboxamide;
6-amino-N-((5-(difluoromethyl)-2-pyridinyl)methyl)-N-(1-methyl-1H-pyrazol-4-yl)-8,9-dihydro-7H-cyclopenta[c][1,7]naphthyridine-2-carboxamide;
4-amino-3-methyl-N-(1-methylcyclopropyl)-N-((5- (trifluoromethyl)-2-pyridinyl)methyl)-3H-pyrazolo[3,4-c]quinoline-8-carboxamide;
4-amino-3-methyl-N-(1-methyl-1H-pyrazol-4-yl)-N-((5-(trifluoromethyl)-2-pyridinyl)methyl)-3H-pyrazolo[3,4-c]quinoline-8-carboxamide;
4-amino-7-fluoro-N-(2-propanyl)-N-((5-(trifluoromethyl)-2-pyrazinyl)methyl)-1,3-dihydrofuro[3,4-c]quinoline-8-carboxamide;
4-amino-N-ethyl-7-fluoro-N-((5-(trifluoromethyl)-2-pyrazinyl)methyl)-1,3-dihydrofuro[3,4-c]quinoline-8-carboxamide;
4-amino-7-chloro-N-ethyl-N-((5-(trifluoromethyl)-2-pyrazinyl)methyl)-1,3-dihydrofuro[3,4-c]quinoline-8-carboxamide;
4-amino-7-chloro-N-(2-propanyl)-N-((5-(trifluoromethyl)-2-pyrazinyl)methyl)-1,3-dihydrofuro[3,4-c]quinoline-8-carboxamide;
(3R)-4-amino-N-ethyl-3-methyl-N-((5-(trifluoromethyl)-2-pyrazinyl)methyl)-1,3-dihydrofuro[3,4-c]quinoline-8-carboxamide;
4-amino-N-cyclobutyl-7-fluoro-N-((6-(trifluoromethyl)-3-pyridazinyl)methyl)-1,3-dihydrofuro[3,4-c]quinoline-8-carboxamide;
4-amino-7-methoxy-N-methyl-N-((5-(trifluoromethyl)-2-pyridinyl)methyl)-1,3-dihydrofuro[3,4-c]quinoline-8-carboxamide;
4-amino-N,7-dimethyl-N-((5-(trifluoromethyl)-2-pyridinyl)methyl)-1,3-dihydrofuro[3,4-c]quinoline-8-carboxamide;
4-amino-N-cyclobutyl-7-fluoro-3-methyl-N-((6-(trifluoromethyl)-3-pyridazinyl)methyl)-3H-pyrazolo[3,4-c]quinoline-8-carboxamide;
4-amino-7-chloro-N-cyclobutyl-N-((6-(trifluoromethyl)-3-pyridazinyl)methyl)-1,3-dihydrofuro[3,4-c]quinoline-8-carboxamide;
4-amino-7-fluoro-3-methyl-N-(1-methyl-1H-pyrazol-4-yl)-N-((5-(trifluoromethyl)-2-pyridinyl)methyl)-3H-pyrazolo[3,4-c]quinoline-8-carboxamide;
4-amino-1-methyl-N-(1-methyl-1H-pyrazol-4-yl)-N-((5-(trifluoromethyl)-2-pyridinyl)methyl)-1H-pyrazolo[4,3-c][1,7]naphthyridine-8-carboxamide;
4-amino-N-cyclopropyl-7-fluoro-3-methyl-N-((1R)- 1-(5- (trifluoromethyl)-2-pyridinyl)ethyl)-3H-pyrazolo[3,4-c]quinoline-8-carboxamide;
4-amino-N-cyclobutyl-N-((6-(difluoromethoxy)-3-pyridazinyl)methyl)-7-fluoro-1,3-dihydrofuro[3,4-c]quinoline-8-carboxamide;
4-amino-N-methyl-N-(4-(pentafluoro-lambda~6~-sulfanyl)benzyl)-1,3-dihydrofuro[3,4-c]quinoline-8-carboxamide;
4-amino-N-methyl-N-(4-(pentafluoro-lambda~6~-sulfanyl)benzyl)-1,3-dihydrofuro[3,4-c][1,7]naphthyridine-8-carboxamide;
4-amino-7-fluoro-N-methyl-N-(4-(pentafluoro-lambda~6~-sulfanyl)benzyl)-1,3-dihydrofuro[3,4-c]quinoline-8-carboxamide;
4-amino-7-chloro-N-methyl-N-(4-(pentafluoro-lambda~6~-sulfanyl)benzyl)-1,3-dihydrofuro[3,4-c]quinoline-8-carboxamide;
(3R)-4-amino-N,3-dimethyl-N-(4-(pentafluoro-lambda-6~-sulfanyl)benzyl)-1,3-dihydrofuro[3,4-c]quinoline-8-carboxamide;
4-amino-7-fluoro-N,3-dimethyl-N-(4-(pentafluoro-lambda~6~-sulfanyl)benzyl)-3H-pyrazolo[3,4-c]quinoline-8-carboxamide;
4-amino-N-((5-fluoro-2-pyridinyl)methyl)-N,1 ,7-trimethyl-1H-pyrazolo[4,3-c]quinoline-8-carboxamide;
4-amino-N-((6-cyclopropyl-3-pyridazinyl)methyl)-7-fluoro-N,3-dimethyl-3H-pyrazolo[3,4-c]quinoline-8-carboxamide;
4-amino-N-((6-cyclopropyl-3-pyridazinyl)methyl)-7-fluoro-N,1-dimethyl-1H-pyrazolo[4,3-c]quinoline-8-carboxamide;
4-amino-7-chloro-N-((6-cyclopropyl-3-pyridazinyl)methyl)-N-methyl-1,3-dihydrofuro[3,4-c]quinoline-8-carboxamide;
4-amino-N,3,7-trimethyl-N-((5-(trifluoromethyl)-2-pyridinyl)methyl)-3H-pyrazolo[3,4-c]quinoline-8-carboxamide;
4-amino-N,1,7-trimethyl-N-((5-(trifluoromethyl)-2-pyridinyl)methyl)-1H-pyrazolo[4,3-c]quinoline-8-carboxamide;
4-amino-N-((5-cyclopropyl-2-pyridinyl)methyl)-7-fluoro-3-methyl-N-(2-propanyl)-3H-pyrazolo[3,4-c]quinoline-8-carboxamide;
4-amino-N-((5-chloro-2-pyridinyl)methyl)-N,1-dimethyl-1H-pyrazolo[4,3-c]quinoline-8-carboxamide;
4-amino-N-((5-(difluoromethyl)-2-pyridinyl)methyl)-N,1-dimethyl-1H-pyrazolo[4,3-c]quinoline-8-carboxamide;
4-amino-N-((5-(difluoromethyl)-2-pyridinyl)methyl)-7-fluoro-N,1-dimethyl-1H-pyrazolo[4,3-c]quinoline-8-carboxamide;
4-amino-N-(3-fluoro-4-(trifluoromethyl)benzyl)-N,1-dimethyl-1H-pyrazolo[4,3-c]quinoline-8-carboxamide;
4-amino-N-((3-fluoro-5-(trifluoromethyl)-2-pyridinyl)methyl)-N,1-dimethyl-1H-pyrazolo[4,3-c]quinoline-8-carboxamide;
4-amino-7-fluoro-N-((3-fluoro-5-(trifluoromethyl)-2-pyridinyl)methyl)-N-methyl-1,3-dihydrofuro[3,4-c]quinoline-8-carboxamide;
4-amino-N,1-dimethyl-N-((6-(1-(trifluoromethyl)-1H-pyrazol-4-yl)-3-pyridazinyl)methyl)-1H-pyrazolo[4,3-c]quinoline-8-carboxamide;
4-amino-N,1-dimethyl-N-((6-(4-(trifluoromethyl)phenyl)-3-pyridazinyl)methyl)-1H-pyrazolo[4,3-c]quinoline-8-carboxamide;
4-amino-N,1-dimethyl-N-((1R)-1-(4'-(trifluoromethyl)[biphenyl]-4-yl)ethyl)-1H-pyrazolo[4,3-c]quinoline-8-carboxamide;
4-amino-N,1-dimethyl-N-((1R)-1-(6-(4-(trifluoromethyl)phenyl)-3-pyridazinyl)ethyl)-1H-pyrazolo[4,3-c]quinoline-8-carboxamide;
4-amino-N,1-dimethyl-N-((1R)-1-(4'-(pentafluoro-lambda-6~-sulfanyl)[biphenyl]-4-yl)ethyl)-1H-pyrazolo[4,3-c]quinoline-8-carboxamide;
4-amino-N-(2-propanyl)-N-((5-(trifluoromethyl)-2-pyridinyl)methyl)-2,3-dihydrofuro[3,2-c][1,7]naphthyridine-8-carboxamide;
4-amino-N-(2-(5-chloro-2-pyridinyl)-2,2-difluoroethyl)-N,1-dimethyl-1H-pyrazolo[4,3-c]quinoline-8-carboxamide;
4-amino-7-fluoro-N-(2-propanyl)-N-((5-(trifluoromethyl)-2-pyridinyl)methyl)-2,3-dihydrofuro[3,2-c]quinoline-8-carboxamide;
4-amino-7-chloro-N-ethyl-N-((5-(trifluoromethyl)-2-pyridinyl)methyl)-1,3-dihydrofuro[3,4-c][1,8]naphthyridine-8-carboxamide;
4-amino-7-fluoro-1,3-dimethyl-N-(2-propanyl)-N-((5-(trifluoromethyl)-2-pyridinyl)methyl)-1H-pyrazolo[4,3-c]quinoline-8-carboxamide;
4-amino-N-((5-cyclopropyl-2-pyridinyl)methyl)-N-ethyl-1,3-dihydrofuro[3,4-c]quinoline-8-carboxamide;
4-amino-N-((5-cyclopropyl-2-pyridinyl)methyl)-N-ethyl-1-methyl-1H-pyrazolo[4,3-c]quinoline-8-carboxamide;
4-amino-N-((5-cyclopropyl-2-pyridinyl)methyl)-N-ethyl-1-methyl-1H-pyrazolo[4,3-c][1,7]naphthyridine-8-carboxamide;
(3R)-4-amino-N-((5-cyclopropyl-2-pyridinyl)methyl)-N-ethyl-3-methyl-1,3-dihydrofuro[3,4-c]quinoline-8-carboxamide;
4-amino-N,1-dimethyl-N-(4-(trifluoromethyl)benzyl)-1H-pyrazolo[4,3-c]quinoline-8-carboxamide;
4-amino-N,1-dimethyl-N-(4-(trifluoromethyl)benzyl)-1H-pyrazolo[4,3-c][1,7]naphthyridine-8-carboxamide;
4-amino-N-((5-cyclopropyl-2-pyridinyl)methyl)-N-ethyl-7-fluoro-1-methyl-1H-pyrazolo[4,3-c]quinoline-8-carboxamide;
4-amino-N-ethyl-1-methyl-N-((5-(trifluoromethyl)-2-pyridinyl)ymethyl)-1H-pyrazolo[4,3-c]quinoline-8-carboxamide;
4-amino-N-ethyl-3-methyl-N-((5-(trifluoromethyl)-2-pyridinyl)methyl)[1,2]oxazolo[4,5-c]quinoline-8-carboxamide;
4-amino-N-ethyl-1-methyl-N-((6-(trifluoromethyl)-3-pyridazinyl)methyl)-1H-pyrazolo[4,3-c]quinoline-8-carboxamide;
4-amino-N-ethyl-1-methyl-N-((5-(trifluoromethyl)-2-pyridinyl)ymethyl)-1H-pyrazolo[4,3-c][1,7]naphthyridine-8-carboxamide;
4-amino-N-ethyl-1-methyl-N-((6-(trifluoromethyl)-3-pyridazinyl)methyl)-1H-pyrazolo[4,3-c][1,7]naphthyridine-8-carboxamide;
(3R)-4-amino-N-ethyl-3-methyl-N-((6-(trifluoromethyl)-3-pyridazinyl)methyl)-1,3-dihydrofuro[3,4-c]quinoline-8-carboxamide;
4-amino-7-fluoro-N,1-dimethyl-N-(4-(trifluoromethyl)benzyl)-1H-pyrazolo[4,3-c]quinoline-8-carboxamide;
4-amino-N-ethyl-1,3-dimethyl-N-((5-(trifluoromethyl)-2-pyridinyl)methyl)-1H-pyrazolo[4,3-c][1,7]naphthyridine-8-carboxamide;
4-amino-N-ethyl-7-fluoro-1-methyl-N-((5-(trifluoromethyl)-2-pyridinyl)methyl)-1H-pyrazolo[4,3-c]quinoline-8-carboxamide;
4-amino-N-ethyl-7-fluoro-1-methyl-N-((6-(trifluoromethyl)-3-pyridazinyl)methyl)-1H-pyrazolo[4,3-c]quinoline-8-carboxamide;
(3R)-4-amino-N-ethyl-7-fluoro-3-methyl-N-((5-(trifluoromethyl)-2-pyridinyl)methyl)-1,3-dihydrofuro[3,4-c]quinoline-8-carboxamide;
(3S)-4-amino-N-ethyl-7-fluoro-3-methyl-N-((5-(trifluoromethyl)-2-pyridinyl)methyl)-1,3-dihydrofuro[3,4-c]quinoline-8-carboxamide;
4-amino-7-chloro-N,1-dimethyl-N-(4-(trifluoromethyl)benzyl)-1H-pyrazolo[4,3-c]quinoline-8-carboxamide;
(3R)-4-amino-N-ethyl-7-fluoro-3-methyl-N-((6-(trifluoromethyl)-3-pyridazinyl)methyl)-1,3-dihydrofuro[3,4-c]quinoline-8-carboxamide;
4-amino-N-ethyl-7-fluoro-1,3-dimethyl-N-((5-(trifluoromethyl)-2-pyridinyl)methyl)-1H-pyrazolo[4,3-c]quinoline-8-carboxamide;
4-amino-N,1-dimethyl-N-(4-(pentafluoroethyl)benzyl)-1H-pyrazolo[4,3-c]quinoline-8-carboxamide;
4-amino-N,1-dimethyl-N-(4-(pentafluoroethyl)benzyl)-1,7]naphthyridine-8-carboxamide;
4-amino-7-fluoro-N,1-dimethyl-N-(4-(pentafluoroethyl)benzyl)-1H-pyrazolo[4,3-c]quinoline-8-carboxamide;
4-amino-N-ethyl-1-methyl-N-(4-(pentafluoro-lambda-6~-sulfanyl)benzyl)-1H-pyrazolo[4,3-c]quinoline-8-carboxamide;
4-amino-N-ethyl-1-methyl-N-(4-(pentafluoro-lambda-6~-sulfanyl)benzyl)-1H-pyrazolo[4,3-c][1,7]naphthyridine-8-carboxamide;
4-amino-N-ethyl-7-fluoro-1-methyl-N-(4-(pentafluoro-lambda-6~-sulfanyl)benzyl)-1H-pyrazolo[4,3-c]quinoline-8-carboxamide;
4-amino-N,1-dimethyl-N-((5-(trifluoromethyl)-2-pyridinyl)methyl)-1H-pyrazolo[4,3-c]quinoline-8-carboxamide;
4-amino-N,1-dimethyl-N-((5-(trifluoromethyl)-2-pyridinyl)methyl)-1H-pyrazolo[4,3-c][1,7]naphthyridine-8-carboxamide;
4-amino-N-cyclopropyl-1-methyl-N-((5-(trifluoromethyl)-2-pyridinyl)methyl)-1H-pyrazolo[4,3-c]quinoline-8-carboxamide;
4-amino-N-cyclopropyl-7-fluoro-1-(2,2,2-trifluoroethyl)-N-((5-(trifluoromethyl)-2-pyridinyl)methyl)-1H-pyrazolo[4,3-c]quinoline-8-carboxamide;
4-amino-7-fluoro-N,1-dimethyl-N-((6-(trifluoromethyl)-3-pyridazinyl)methyl)-1H-pyrazolo[4,3-c]quinoline-8-carboxamide;
4-amino-N-methyl-7-(trifluoromethyl)-N-((5-(trifluoromethyl)-2-pyridinyl)methyl)-1,3-dihydrofuro[3,4-c]quinoline-8-carboxamide;
4-amino-N,1-dimethyl-N-(4-(pentafluoro-lambda~6~-sulfanyl)benzyl)-1H-pyrazolo[4,3-c]quinoline-8-carboxamide;
4-amino-N,1-dimethyl-N-(4-(pentafluoro-lambda~6~-sulfanyl)benzyl)-1H-pyrazolo[4,3-c][1,7]naphthyridine-8-carboxamide;
4-amino-7-fluoro-N,1-dimethyl-N-(4-(pentafluoro-lambda~6~-sulfanyl)benzyl)-1H-pyrazolo[4,3-c]quinoline-8-carboxamide;
4-amino-7-fluoro-N-(2-hydroxy-4-(pentafluoro-lambda~6~-sulfanyl)benzyl)-N-methyl-1,3-dihydrofuro[3,4-c]quinoline-8-carboxamide;
4-amino-N-((5-cyano-2-pyridinyl)methyl)-N-((2R)-1-fluoro-2-propanyl)-1,3-dihydrofuro[3,4-c]quinoline-8-carboxamide;
4-amino-N-((5-cyano-2-pyridinyl)methyl)-N-((2S)-1-fluoro-2-propanyl)-1,3-dihydrofuro[3,4-c]quinoline-8-carboxamide;
(3R)-4-amino-3-(hydroxymethyl)-N-(2-methylpropyl)-N-((5-(trifluoromethyl)-2-pyridinyl)methyl)-1,3-dihydrofuro[3,4-c]quinoline-8-carboxamide;
(3S)-4-amino-3-(hydroxymethyl)-N-(2-methylpropyl)-N-((5-(trifluoromethyl)-2-pyridinyl)methyl)-1,3-dihydrofuro[3,4-c]quinoline-8-carboxamide;
4-amino-N-((1R)-2-cyano-1-cyclopropylethyl)-N-((5-cyano-2-pyridinyl)methyl)-1,3-dihydrofuro[3,4-c]quinoline-8-carboxamide;
4-amino-N-((1S)-2-cyano-1-cyclopropylethyl)-N-((5-cyano-2-pyridinyl)methyl)-1,3-dihydrofuro[3,4-c]quinoline-8-carboxamide;
4-amino-N-((5-cyano-6-methyl-2-pyridinyl)methyl)-N-((3R,4R)-4-methoxytetrahydro-2H-pyran-3-yl)-1,3-dihydrofuro[3,4-c]quinoline-8-carboxamide;
4-amino-N-((5-cyano-6-methyl-2-pyridinyl)methyl)-N-((3S,4S)-4-methoxytetrahydro-2H-pyran-3-yl)-1,3-dihydrofuro[3,4-c]quinoline-8-carboxamide;
4-amino-N-((3S,4R)-3-methoxytetrahydro-2H-pyran-4-yl)-N-((5-(trifluoromethyl)-2-pyridinyl)methyl)-1,3-dihydrofuro[3,4-c]quinoline-8-carboxamide;
4-amino-N-((3R,4S)-3-methoxytetrahydro-2H-pyran-4-yl)-N-((5-(trifluoromethyl)-2-pyridinyl)methyl)-1,3-dihydrofuro[3,4-c]quinoline-8-carboxamide;
4-amino-N-((3R,4R)-4-methoxytetrahydro-2H-pyran-3-yl)-N-((5-(trifluoromethyl)-2-pyridinyl)methyl)-1,3-dihydrofuro[3,4-c]quinoline-8-carboxamide;
4-amino-N-((3S,4S)-4-methoxytetrahydro-2H-pyran-3-yl)-N-((5-(trifluoromethyl)-2-pyridinyl)methyl)-1,3-dihydrofuro[3,4-c]quinoline-8-carboxamide;
4-amino-N-((5-cyano-6-methyl-2-pyridinyl)methyl)-N-((3R,4R)-4-methoxytetrahydro-2H-pyran-3-yl)-1,3-dihydrofuro[3,4-c]quinoline-8-carboxamide;
(3R)-4-amino-N-cyclopropyl-3-methyl-N-((1R)-1-(5-(trifluoromethyl)-2-pyridinyl)ethyl)-1,3-dihydrofuro[3,4-c][1,7]naphthyridine-8-carboxamide;
(3S)-4-amino-N-cyclopropyl-3-methyl-N-((1R)-1-(5-(trifluoromethyl)-2-pyridinyl)ethyl)-1,3-dihydrofuro[3,4-c][1,7]naphthyridine-8-carboxamide;
4-amino-N-((1S)-1-(5-fluoro-2-pyridinyl)ethyl)-N,1,7-trimethyl-1H-pyrazolo[4,3-c]quinoline-8-carboxamide;
4-amino-N-((1R)-1-(5-fluoro-2-pyridinyl)ethyl)-N,1,7-trimethyl-1H-pyrazolo[4,3-c]quinoline-8-carboxamide;
4-amino-7-chloro-N-((1R)-1-(5-fluoro-2-pyridinyl)ethyl)-N,1-dimethyl-1H-pyrazolo[4,3-c]quinoline-8-carboxamide;
4-amino-7-chloro-N-((1S)-1-(5-fluoro-2-pyridinyl)ethyl)-N,1-dimethyl-1H-pyrazolo[4,3-c]quinoline-8-carboxamide;
4-amino-7-fluoro-N-((1R)-1-(3-fluoro-5-(trifluoromethyl)-2-pyridinyl)ethyl)-N-methyl-1,3-dihydrofuro[3,4-c]quinoline-8-carboxamide;
(3R)-4-amino-N-ethyl-3-(fluoromethyl)-N-((5-(trifluoromethyl)-2-pyridinyl)methyl)-1,3-dihydrofuro[3,4-c]quinoline-8-carboxamide;
(3R)-4-amino-N-((5-bromo-2-pyridinyl)methyl)-N,3-dimethyl-1,3-dihydrofuro[3,4-c][1,7]naphthyridine-8-carboxamide;
4-amino-N,1-dimethyl-N-((1R)-1-(4-(trifluoromethyl)phenyl)ethyl)-1H-pyrazolo[4,3-c][1,7]naphthyridine-8-carboxamide;
4-amino-N,1-dimethyl-N-((1S)-1-(4-(trifluoromethyl)phenyl)ethyl)-1H-pyrazolo[4,3-c][1,7]naphthyridine-8-carboxamide;
4-amino-N,1-dimethyl-N-((1R)-1-(5-(trifluoromethyl)-2-pyridinyl)ethyl)-1H-pyrazolo[4,3-c]quinoline-8-carboxamide;
4-amino-N,1-dimethyl-N-((1S)-1-(5-(trifluoromethyl)-2-pyridinyl)ethyl)-1H-pyrazolo[4,3-c]quinoline-8-carboxamide;
4-amino-7-fluoro-N,1-dimethyl-N-((1R)-1-(5-(trifluoromethyl)-2-pyridinyl)ethyl)-1H-pyrazolo[4,3-c]quinoline-8-carboxamide;
4-amino-7-fluoro-N,1-dimethyl-N-((1S)-1-(5-(trifluoromethyl)-2-pyridinyl)ethyl)-1H-pyrazolo[4,3-c]quinoline-8-carboxamide;
4-amino-N-((1R)-1-(5-fluoro-2-pyridinyl)ethyl)-N,1-dimethyl-1H-pyrazolo[4,3-c]quinoline-8-carboxamide;
4-amino-7-fluoro-N,1-dimethyl-N-((1R)-1-(6-(trifluoromethyl)-3-pyridazinyl)ethyl)-1H-pyrazolo[4,3-c]quinoline-8-carboxamide;
4-amino-N-((5-cyano-2-pyridinyl)methyl)-N-((1R)-1-(2-pyrimidinyl)ethyl)-1,3-dihydrofuro[3,4-c][1,7]naphthyridine-8-carboxamide;
6-amino-N-(2-methylpropyl)-N-((5-(trifluoromethyl)-2-pyridinyl)methyl)-8,9-dihydro-7H-cyclopenta[c][1,8]naphthyridine-2-carboxamide;
4-amino-N-(cyclopropylmethyl)-N-((5-(trifluoromethyl)-2-pyridinyl)methyl)-1,3-dihydrofuro[3,4-c][1,8]naphthyridine-8-carboxamide;
4-amino-N-((2R)-1-methoxy-2-propanyl)-N-((5-(trifluoromethyl)-2-pyridinyl)methyl)-1,3-dihydrofuro[3,4-c][1,8]naphthyridine-8-carboxamide;
4-amino-N-((1R)-1-(2-pyrimidinyl)ethyl)-N-((5-(trifluoromethyl)-2-pyridinyl)methyl)-2,3-dihydro-1H-cyclopenta[c]quinoline-8-carboxamide;
4-amino-N-(2-methylpropyl)-N-((5-(trifluoromethyl)-2-pyridinyl)methyl)-2,3-dihydro-1H-cyclopenta[c]quinoline-8-carboxamide;
4-amino-N-(2-methylpropyl)-N-((5-(trifluoromethyl)-2-pyridinyl)methyl)-1,3-dihydrofuro[3,4-c][1,8]naphthyridine-8-carboxamide;
4-amino-N-(2-methylpropyl)-N-((5-(trifluoromethyl)-2-pyridinyl)methyl)-1,3-dihydrofuro[3,4-c]quinoline-8-carboxamide;
4-amino-N-((1R)-1-(2-pyrimidinyl)ethyl)-N-((5-(trifluoromethyl)-2-pyridinyl)methyl)-1, 3-dihydrofuro[3,4-c][1,8]naphthyridine-8-carboxamide;
4-amino-N-((5-cyano-2-pyridinyl)methyl)-N-((1R)-1-(2-pyrimidinyl)ethyl)-2,3-dihydro-1H-cyclopenta[c]quinoline-8-carboxamide;
4-amino-N-((5-cyano-2-pyridinyl)methyl)-N-((1R)-1-(2-pyrimidinyl)ethyl)-1,3-dihydrofuro[3,4-c]quinoline-8-carboxamide;
4-amino-N-((5-cyano-2-pyridinyl)methyl)-N-((1S)-1-(2-pyrimidinyl)ethyl)-1,3-dihydrofuro[3,4-c]quinoline-8-carboxamide;
4-amino-3-methyl-N-(2-methylpropyl)-N-((5-(trifluoromethyl)-2-pyridinyl)methyl)[1,2]oxazolo[4,5-c]quinoline-8-carboxamide;
4-amino-3-methyl-N-((1R)-1-(2-pyrimidinyl)ethyl)-N-((5-(trifluoromethyl)-2-pyridinyl)methyl)-3H-pyrazolo[3,4-c]quinoline-8-carboxamide;
4-amino-3-methyl-N-((1S)-1-(2-pyrimidinyl)ethyl)-N-((5-(trifluoromethyl)-2-pyridinyl)methyl)-3H-pyrazolo[3,4-c]quinoline-8-carboxamide;
4-amino-3-methyl-N-((1R)-1-(2-pyrimidinyl)ethyl)-N-((5-(trifluoromethyl)-2-pyridinyl)methyl)[1,2]oxazolo[4,5-c]quinoline-8-carboxamide;
4-amino-3-methyl-N-((1S)-1-(2-pyrimidinyl)ethyl)-N-((5-(trifluoromethyl)-2-pyridinyl)methyl)[1,2]oxazolo[4,5-c]quinoline-8-carboxamide;
4-amino-N-(2-methylpropyl)-N-((5-(trifluoromethyl)-2-pyridinyl)methyl)-1,3-dihydrofuro[3,4-c][1,7]naphthyridine-8-carboxamide;
4-amino-N-((1R)-1-(2-pyrimidinyl)ethyl)-N-((5-(trifluoromethyl)-2-pyridinyl)methyl)-1, 3-dihydrofuro[3,4-c][1,7]naphthyridine-8-carboxamide;
4-amino-N-((5-chloro-2-pyridinyl)methyl)-N-((1R)-1-(2-pyrimidinyl)ethyl)-2,3-dihydro-1H-cyclopenta[c]quinoline-8-carboxamide;
4-amino-N-((5-(difluoromethyl)-2-pyridinyl)methyl)-N-((1R)-1-(2-pyrimidinyl)ethyl)-2,3-dihydro-1H-cyclopenta[c]quinoline-8-carboxamide;
4-amino-N-((6-bromo-3-pyridazinyl)methyl)-N-((1R)-1-(3-fluoro-2-pyridinyl)ethyl)-2,3-dihydro-1H-cyclopenta[c]quinoline-8-carboxamide;
6-amino-N-((5-(difluoromethyl)-2-pyridinyl)methyl)-N-((1R)-1-(2-pyrimidinyl)ethyl)-8,9-dihydro-7H-cyclopenta[c][1,8]naphthyridine-2-carboxamide;
6-amino-N-((1R)-1-(2-pyrimidinyl)ethyl)-N-((5-(trifluoromethyl)-2-pyridinyl)methyl)-8,9-dihydro-7H-cyclopenta[c][1,8]naphthyridine-2-carboxamide;
6-amino-N-((5-chloro-2-pyridinyl)methyl)-N-((1R)-1-(2-pyrimidinyl)ethyl)-8,9-dihydro-7H-cyclopenta[c][1,8]naphthyridine-2-carboxamide;
6-amino-N-((1R)-1-(3-fluoro-2-pyridinyl)ethyl)-N-((6-(trifluoromethyl)-3-pyridazinyl)methyl)-8,9-dihydro-7H-cyclopenta[c][1,8]naphthyridine-2-carboxamide;
6-amino-N-((1R)-1-(2-pyrimidinyl)ethyl)-N-((5-(trifluoromethyl)-2-pyridinyl)methyl)-2-phenanthridinecarboxamide;
6-amino-N-((5-cyano-2-pyridinyl)methyl)-N-((1R)-1-(2-pyrimidinyl)ethyl)-2-phenanthridinecarboxamide;
5-amino-N-(2-pyrimidinylmethyl)-N-((5-(trifluoromethyl)-2-pyridinyl)methyl)benzo[c][2,6]naphthyridine-9-carboxamide;
5-amino-N-(2-methylpropyl)-N-((5-(trifluoromethyl)-2-pyridinyl)methyl)benzo[c][2,6]naphthyridine-9-carboxamide;
5-amino-N-((1R)-1-(2-pyrimidinyl)ethyl)-N-((5-(trifluoromethyl)-2-pyridinyl)methyl)benzo[c][2,6]naphthyridine-9-carboxamide;
4-amino-N-((2R)-1-methoxy-2-propanyl)-3-methyl-N-((5-(trifluoromethyl)-2-pyridinyl)methyl)[1,2]oxazolo[4,5-c]quinoline-8-carboxamide;
4-amino-N-((2S)-1-methoxy-2-propanyl)-3-methyl-N-((5-(trifluoromethyl)-2-pyridinyl)methyl)[1,2]oxazolo[4,5-c]quinoline-8-carboxamide;
4-amino-N-((3-fluoro-2-pyridinyl)methyl)-N-((5-(trifluoromethyl)-2-pyridinyl)methyl)thieno[2,3-c]quinoline-8-carboxamide;
4-amino-N-(2-pyrimidinylmethyl)-N-((5-(trifluoromethyl)-2-pyridinyl)methyl)thieno[2,3-c]quinoline-8-carboxamide;
4-amino-N-(2-methylpropyl)-N-((5-(trifluoromethyl)-2-pyridinyl)methyl)thieno[2,3-c]quinoline-8-carboxamide;
5-amino-N-((1R)-1-(2-pyrimidinyl)ethyl)-N-((5-(trifluoromethyl)-2-pyridinyl)methyl)pyrimido[4,5-c]quinoline-9-carboxamide;
4-amino-N-((6-bromo-3-pyridazinyl)methyl)-N-((1R)-1-cyclopropyl-2-methoxyethyl)thieno[2,3-c]quinoline-8-carboxamide;
4-amino-N-((6-bromo-3-pyridazinyl)methyl)-N-((1S)-1-cyclopropyl-2-methoxyethyl)thieno[2, 3-c]quinoline-8-carboxamide;
4-amino-N-((1R)-1-(2-pyrimidinyl)ethyl)-N-((5-(trifluoromethyl)-2-pyridinyl)methyl)-1, 3-dihydrofuro[3,4-c]quinoline-8-carboxamide;
4-amino-N-((1S)-1-(2-pyrimidinyl)ethyl)-N-((5-(trifluoromethyl)-2-pyridinyl)methyl)-1,3-dihydrofuro[3,4-c]quinoline-8-carboxamide;
4-amino-N-((1S)-1-cyclopropyl-2-methoxyethyl)-N-((6-(4-morpholinyl)-3-pyridazinyl)methyl)-1,3-dihydrofuro[3,4-c]quinoline-8-carboxamide;
4-amino-N-((6-ethoxy-3-pyridazinyl)methyl)-N-(2-methylpropyl)-1,3-dihydrofuro[3,4-c]quinoline-8-carboxamide;
4-amino-N-((6-bromo-3-pyridazinyl)methyl)-N-((2R)-1-methoxy-2-propanyl)thieno[2,3-c]quinoline-8-carboxamide;
4-amino-N-((6-bromo-3-pyridazinyl)methyl)-N-(1-methoxy-2-methyl-2-propanyl)-1,3-dihydrofuro[3,4-c]quinoline-8-carboxamide;
4-amino-N-((6-bromo-3-pyridazinyl)methyl)-N-(1-methoxy-2-methyl-2-propanyl)-1,3-dihydrofuro[3,4-c][1,7]naphthyridine-8-carboxamide;
4-amino-N-((6-methoxy-3-pyridazinyl)methyl)-N-((1R)-1-(2-pyrimidinyl)ethyl)-2,3-dihydro-1H-cyclopenta[c]quinoline-8-carboxamide;
4-amino-N-((6-methoxy-3-pyridazinyl)methyl)-N-((1S)-1-(2-pyrimidinyl)ethyl)-2,3-dihydro-1H-cyclopenta[c]quinoline-8-carboxamide;
4-amino-N-((1S)-1-(2-pyrimidinyl)ethyl)-N-((6-(trifluoromethyl)-3-pyridazinyl)methyl)-2,3-dihydro-1H-cyclopenta[c]quinoline-8-carboxamide;
4-amino-N-((1R)-1-(2-pyrimidinyl)ethyl)-N-((6-(trifluoromethyl)-3-pyridazinyl)methyl)-2,3-dihydro-1H-cyclopenta[c]quinoline-8-carboxamide;
4-amino-N-((6-bromo-3-pyridazinyl)methyl)-N-((1S)-1-cyclopropyl-2-methoxyethyl)thieno[2,3-c]quinoline-8-carboxamide;
4-amino-N-((6-bromo-3-pyridazinyl)methyl)-N-((1S)-1-cyclopropyl-2-methoxyethyl)-1,3-dihydrofuro[3,4-c][1,8]naphthyridine-8-carboxamide;
4-amino-N-(cyclopropylmethyl)-N-((5-(trifluoromethyl)-2-pyridinyl)methyl)-1,3-dihydrofuro[3,4-c][1,7]naphthyridine-8-carboxamide;
4-amino-N-((1R)-1-(2-pyrimidinyl)ethyl)-N-((5-(trifluoromethyl)-2-pyridinyl)methyl)-1, 3-dihydrofuro[3,4-c]quinoline-8-carboxamide;
6-amino-N-((1R)-1-(2-pyrimidinyl)ethyl)-N-((5-(trifluoromethyl)-2-pyridinyl)methyl)-7,8,9,10-tetrahydro-2-phenanthridinecarboxamide;
4-amino-7-chloro-N-((1R)-1-(2-pyrimidinyl)ethyl)-N-((5-(trifluoromethyl)-2-pyridinyl)methyl)-1,3-dihydrofuro[3,4-c]quinoline-8-carboxamide;
4-amino-7-chloro-N-cyclopropyl-N-((5-(trifluoromethyl)-2-pyridinyl)methyl)-1,3-dihydrofuro[3,4-c]quinoline-8-carboxamide;
4-amino-N-((6-bromo-3-pyridazinyl)methyl)-N-((1R)-1-(2-pyrimidinyl)ethyl)-1,3-dihydrofuro[3,4-c]quinoline-8-carboxamide;
4-amino-N-((5-bromo-2-pyridinyl)methyl)-N-((1R)-1-(2-pyrimidinyl)ethyl)-1,3-dihydrofuro[3,4-c]quinoline-8-carboxamide;
4-amino-N-((5-cyano-2-pyridinyl)methyl)-N-((1R)-1-(2-pyrimidinyl)ethyl)-1,3-dihydrofuro[3,4-c]quinoline-8-carboxamide;
4-amino-N-(3,3-difluorocyclobutyl)-N-((6-(trifluoromethyl)-3-pyridazinyl)methyl)-1,3-dihydrofuro[3,4-c]quinoline-8-carboxamide;
4-amino-N-cyclopropyl-N-((6-(trifluoromethyl)-3-pyridazinyl)methyl)-1,3-dihydrofuro[3,4-c]quinoline-8-carboxamide;
4-amino-N-((5-(difluoromethyl)-2-pyridinyl)methyl)-N-((1R)-1-(1-methyl-1H-1,2,4-triazol-3-yl)ethyl)-1,3-dihydrofuro[3,4-c]quinoline-8-carboxamide;
4-amino-N-(cyclopropylmethyl)-N-((5-(trifluoromethoxy)-2-pyridinyl)methyl)-1,3-dihydrofuro[3,4-c]quinoline-8-carboxamide;
4-amino-N-(2-methylpropyl)-N-((6-(trifluoromethyl)-3-pyridazinyl)methyl)-1,3-dihydrofuro[3,4-c]quinoline-8-carboxamide;
4-amino-N-((6-bromo-3-pyridazinyl)methyl)-N-(2-methylpropyl)-1,3-dihydrofuro[3,4-c]quinoline-8-carboxamide;
4-amino-N-((5-cyano-2-pyridinyl)methyl)-N-((1S)-1-(2-pyrimidinyl)ethyl)-1,3-dihydrofuro[3,4-c]quinoline-8-carboxamide;
4-amino-N-((5-(difluoromethyl)-2-pyridinyl)methyl)-N-((1R)-1-(1-methyl-1H-1,2,4-triazol-3-yl)ethyl)-1,3-dihydrofuro[3,4-c][1,7]naphthyridine-8-carboxamide;
4-amino-N-(3,3-difluorocyclobutyl)-N-((6-(trifluoromethyl)-3-pyridazinyl)methyl)-1,3-dihydrofuro[3,4-c][1,7]naphthyridine-8-carboxamide;
4-amino-N-cyclobutyl-N-((6-(trifluoromethyl)-3-pyridazinyl)methyl)-1,3-dihydrofuro[3,4-c]quinoline-8-carboxamide;
4-amino-N-(2-methylpropyl)-N-((6-(trifluoromethyl)-3-pyridazinyl)methyl)-1,3-dihydrofuro[3,4-c][1,7]naphthyridine-8-carboxamide;
4-amino-N-((6-bromo-3-pyridazinyl)methyl)-N-(cyclopropylmethyl)-1,3-dihydrofuro[3,4-c][1,7]naphthyridine-8-carboxamide;
4-amino-N-((6-ethoxy-3-pyridazinyl)methyl)-N-(2-methylpropyl)-1,3-dihydrofuro[3,4-c][1,7]naphthyridine-8-carboxamide;
4-amino-N-((6-bromo-3-pyridazinyl)methyl)-N-((1R)-1-(2-pyrimidinyl)ethyl)-1,3-dihydrofuro[3,4-c][1,7]naphthyridine-8-carboxamide;
4-amino-N-((6-bromo-3-pyridazinyl)methyl)-N-(cyclopropylmethyl)-1,3-dihydrofuro[3,4-c]quinoline-8-carboxamide;
4-amino-N-(2-propanyl)-N-((5-(trifluoromethyl)-2-pyridinyl)methyl)-1,3-dihydrofuro[3,4-c][1,7]naphthyridine-8-carboxamide;
4-amino-N-((6-chloro-3-pyridazinyl)methyl)-N-((1R)-1-(2-pyrimidinyl)ethyl)-1,3-dihydrofuro[3,4-c]quinoline-8-carboxamide;
4-amino-N-((5-bromo-6-methyl-2-pyridinyl)methyl)-N-methyl-1,3-dihydrofuro[3,4-c]quinoline-8-carboxamide;
4-amino-N-(2-propanyl)-N-((5-(trifluoromethoxy)-2-pyridinyl)methyl)-1,3-dihydrofuro[3,4-c]quinoline-8-carboxamide;
4-amino-N-((6-chloro-5-cyano-2-pyridinyl)methyl)-N-((1R)-1-(2-pyrimidinyl)ethyl)-1,3-dihydrofuro[3,4-c]quinoline-8-carboxamide;
4-amino-N-((6-bromo-3-pyridazinyl)methyl)-N-((2R)-1-methoxy-2-propanyl)-1,3-dihydrofuro[3,4-c][1,7]naphthyridine-8-carboxamide;
4-amino-N-((6-bromo-3-pyridazinyl)methyl)-N-((2R)-1-methoxy-2-propanyl)-1,3-dihydrofuro[3,4-c][1,8]naphthyridine-8-carboxamide;
4-amino-N-((5-cyano-2-pyridinyl)methyl)-N-(cyclopropylmethyl)-1,3-dihydrofuro[3,4-c][1,7]naphthyridine-8-carboxamide;
4-amino-N-((6-chloro-5-methoxy-2-pyridinyl)methyl)-N-(2-propanyl)-1,3-dihydrofuro[3,4-c][1,7]naphthyridine-8-carboxamide;
4-amino-N-((6-bromo-3-pyridazinyl)methyl)-N-(2-propanyl)-1,3-dihydrofuro[3,4-c][1,7]naphthyridine-8-carboxamide;
4-amino-N-((5-chloro-6-methoxy-2-pyridinyl)methyl)-N-(2-propanyl)-1,3-dihydrofuro[3,4-c][1,7]naphthyridine-8-carboxamide;
4-amino-N-(3,4-dihydro-2H-pyrano[2,3-c]pyridin-6-ylmethyl)-N-((1R)-1-(2-pyrimidinyl)ethyl)-1,3-dihydrofuro[3,4-c][1,7]naphthyridine-8-carboxamide;
4-amino-N-((5-cyano-2-pyridinyl)methyl)-N-((2R)-1-fluoro-2-propanyl)-1,3-dihydrofuro[3,4-c][1,7]naphthyridine-8-carboxamide;
4-amino-N-((5-cyano-2-pyridinyl)methyl)-N-((2S)-1-fluoro-2-propanyl)-1,3-dihydrofuro[3,4-c][1,7]naphthyridine-8-carboxamide;
4-amino-N-(cyclopropylmethyl)-N-((6-(trifluoromethyl)-3-pyridazinyl)methyl)-1,3-dihydrofuro[3,4-c][1,7]naphthyridine-8-carboxamide;
4-amino-N-(bicyclo[1.1.1]pentan-1-yl)-N-((5-(trifluoromethyl)-2-pyridinyl)methyl)-1,3-dihydrofuro[3,4-c][1,7]naphthyridine-8-carboxamide;
4-amino-N-(1-cyclopropyl-1H-pyrazol-4-yl)-N-((5-(trifluoromethyl)-2-pyridinyl)methyl)-1,3-dihydrofuro[3,4-c][1,7]naphthyridine-8-carboxamide;
4-amino-N-((5-(difluoromethyl)-2-pyridinyl)methyl)-N-(1-methyl-1H-pyrazol-4-yl)-1,3-dihydrofuro[3,4-c][1,7]naphthyridine-8-carboxamide;
4-amino-N-methyl-N-(4-(trifluoromethyl)benzyl)-1,3-dihydrofuro[3,4-c][1,7]naphthyridine-8-carboxamide;
4-amino-N-methyl-N-((6-(trifluoromethyl)-3-pyridinyl)methyl)-1,3-dihydrofuro[3,4-c][1,7]naphthyridine-8-carboxamide;
4-amino-N-((5-chloro-2-pyridinyl)methyl)-N-methyl-1,3-dihydrofuro[3,4-c][1,7]naphthyridine-8-carboxamide;
4-amino-N-ethyl-N-((6-(trifluoromethyl)-3-pyridazinyl)methyl)-1,3-dihydrofuro[3,4-c]quinoline-8-carboxamide;
4-amino-N-ethyl-N-((6-(trifluoromethyl)-3-pyridazinyl)methyl)-1,3-dihydrofuro[3,4-c][1,7]naphthyridine-8-carboxamide;
4-amino-N-methyl-N-((1R)-1-(5-(trifluoromethyl)-2-pyridinyl)ethyl)-1,3-dihydrofuro[3,4-c]quinoline-8-carboxamide;
4-amino-N-methyl-N-((1S)-1-(5-(trifluoromethyl)-2-pyridinyl)ethyl)-1,3-dihydrofuro[3,4-c]quinoline-8-carboxamide;
4-amino-N-((5-chloro-2-pyridinyl)methyl)-N-ethyl-1,3-dihydrofuro[3,4-c][1,7]naphthyridine-8-carboxamide;
4-amino-N-(1-methylcyclopropyl)-N-((5-(trifluoromethyl)-2-pyridinyl)methyl)-1,3-dihydrofuro[3,4-c][1,7]naphthyridine-8-carboxamide;
4-amino-N-methyl-N-((1R)-1-(5-(trifluoromethyl)-2-pyridinyl)ethyl)-1,3-dihydrofuro[3,4-c][1,8]naphthyridine-8-carboxamide;
4-amino-N-methyl-N-((1S)-1-(5-(trifluoromethyl)-2-pyridinyl)ethyl)-1,3-dihydrofuro[3,4-c][1,8]naphthyridine-8-carboxamide;
4-amino-N-((5-chloro-2-pyridinyl)methyl)-N-ethyl-1,3-dihydrofuro[3,4-c]quinoline-8-carboxamide;
4-amino-N-((5-chloro-2-pyridinyl)methyl)-N-methyl-1,3-dihydrofuro[3,4-c]quinoline-8-carboxamide;
4-amino-N-ethyl-N-((6-(trifluoromethyl)-3-pyridinyl)methyl)-1,3-dihydrofuro[3,4-c]quinoline-8-carboxamide;
4-amino-N-ethyl-N-((6-(trifluoromethyl)-3-pyridinyl)methyl)-1,3-dihydrofuro[3,4-c][1,7]naphthyridine-8-carboxamide;
4-amino-N-ethyl-N-((1R)-1-(5-(trifluoromethyl)-2-pyridinyl)ethyl)-1,3-dihydrofuro[3,4-c][1,8]naphthyridine-8-carboxamide;
4-amino-N-ethyl-N-((1S)-1-(5-(trifluoromethyl)-2-pyridinyl)ethyl)-1,3-dihydrofuro[3,4-c][1,8]naphthyridine-8-carboxamide;
4-amino-N-ethyl-N-(4-(trifluoromethyl)benzyl)-1,3-dihydrofuro[3,4-c]quinoline-8-carboxamide;
4-amino-N-ethyl-N-(4-(trifluoromethyl)benzyl)-1,3-dihydrofuro[3,4-c][1,7]naphthyridine-8-carboxamide;
4-amino-N-((6-bromo-3-pyridazinyl)methyl)-N-ethyl-1,3-dihydrofuro[3,4-c]quinoline-8-carboxamide;
4-amino-N-methyl-N-((1R)-1-(5-(trifluoromethyl)-2-pyridinyl)ethyl)-1,3-dihydrofuro[3,4-c][1,7]naphthyridine-8-carboxamide;
4-amino-N-methyl-N-((1S)-1-(5-(trifluoromethyl)-2-pyridinyl)ethyl)-1,3-dihydrofuro[3,4-c][1,7]naphthyridine-8-carboxamide;
4-amino-N-((6-bromo-3-pyridazinyl)methyl)-N-ethyl-1,3-dihydrofuro[3,4-c][1,7]naphthyridine-8-carboxamide;
4-amino-N-(1-methyl-1H-pyrazol-4-yl)-N-((6-(trifluoromethyl)-3-pyridazinyl)methyl)-1,3-dihydrofuro[3,4-c][1,7]naphthyridine-8-carboxamide;
4-amino-N-(2,2,2-trifluoroethyl)-N-((6-(trifluoromethyl)-3-pyridazinyl)methyl)-1,3-dihydrofuro[3,4-c][1,7]naphthyridine-8-carboxamide;
4-amino-N-(3-oxetanyl)-N-((6-(trifluoromethyl)-3-pyridazinyl)methyl)-1,3-dihydrofuro[3,4-c]quinoline-8-carboxamide;
4-amino-7-fluoro-N-(1-methyl-1H-pyrazol-4-yl)-N-((5-(trifluoromethyl)-2-pyridinyl)methyl)-1,3-dihydrofuro[3,4-c]quinoline-8-carboxamide;
4-amino-7-chloro-N-(1-methyl-1H-pyrazol-4-yl)-N-((5-(trifluoromethyl)-2-pyridinyl)methyl)-1,3-dihydrofuro[3,4-c]quinoline-8-carboxamide;
4-amino-N,3-dimethyl-N-((1R)-1-(5-(trifluoromethyl)-2-pyridinyl)ethyl)-3H-pyrazolo[3,4-c]quinoline-8-carboxamide;
4-amino-N,3-dimethyl-N-((1S)-1-(5-(trifluoromethyl)-2-pyridinyl)ethyl)-3H-pyrazolo[3,4-c]quinoline-8-carboxamide;
4-amino-N-((5-chloro-2-pyridinyl)methyl)-N-(2-propanyl)-1,3-dihydrofuro[3,4-c]quinoline-8-carboxamide;
4-amino-N-((5-chloro-2-pyridinyl)methyl)-N-(2-propanyl)-1,3-dihydrofuro[3,4-c][1,7]naphthyridine-8-carboxamide;
4-amino-N-ethyl-7-fluoro-N-((1R)-1-(5-(trifluoromethyl)-2-pyridinyl)ethyl)-1,3-dihydrofuro[3,4-c]quinoline-8-carboxamide;
4-amino-N-ethyl-7-fluoro-N-((1S)-1-(5-(trifluoromethyl)-2-pyridinyl)ethyl)-1,3-dihydrofuro[3,4-c]quinoline-8-carboxamide;
(3R)-4-amino-N-ethyl-3-methyl-N-((1-methyl-1H-1,2,4-triazol-3-yl)methyl)-1,3-dihydrofuro[3,4-c]quinoline-8-carboxamide;
4-amino-N-cyclobutyl-N-((6-(trifluoromethyl)-3-pyridazinyl)methyl)-1,3-dihydrofuro[3,4-c][1,7]naphthyridine-8-carboxamide;
(3R)-4-amino-N-cyclobutyl-3-methyl-N-((6-(trifluoromethyl)-3-pyridazinyl)methyl)-1,3-dihydrofuro[3,4-c]quinoline-8-carboxamide;
4-amino-N-((6-(difluoromethoxy)-3-pyridazinyl)methyl)-N-ethyl-1,3-dihydrofuro[3,4-c]quinoline-8-carboxamide;
4-amino-N-((6-(difluoromethoxy)-3-pyridazinyl)methyl)-N-ethyl-7-fluoro-1,3-dihydrofuro[3,4-c]quinoline-8-carboxamide;
4-amino-7-fluoro-N-((1R)-1-(5-fluoro-2-pyrimidinyl)ethyl)-N-(2-(trifluoromethoxy)ethyl)-1,3-dihydrofuro[3,4-c]quinoline-8-carboxamide;
4-amino-7-fluoro-N-((1S)-1-(5-fluoro-2-pyrimidinyl)ethyl)-N-(2-(trifluoromethoxy)ethyl)-1,3-dihydrofuro[3,4-c]quinoline-8-carboxamide;
(3R)-4-amino-N-((6-(difluoromethoxy)-3-pyridazinyl)methyl)-N-ethyl-3-methyl-1,3-dihydrofuro[3,4-c]quinoline-8-carboxamide;
4-amino-N-((1R)-1-(6-chloro-3-pyridinyl)ethyl)-N-ethyl-1,3-dihydrofuro[3,4-c]quinoline-8-carboxamide;
4-amino-N-((1S)-1-(6-chloro-3-pyridinyl)ethyl)-N-ethyl-1,3-dihydrofuro[3,4-c]quinoline-8-carboxamide;
4-amino-N-((2R)-1-methoxy-2-propanyl)-N-((5-(trifluoromethyl)-2-pyridinyl)methyl)-1,3-dihydrofuro[3,4-c][1,7]naphthyridine-8-carboxamide;
4-amino-7-fluoro-N-(1-(trifluoromethyl)-1H-pyrazol-4-yl)-N-((5-(trifluoromethyl)-2-pyridinyl)methyl)-1,3-dihydrofuro[3,4-c]quinoline-8-carboxamide;
4-amino-N-(cyclopropylmethyl)-7-fluoro-N-((6-(trifluoromethyl)-3-pyridazinyl)methyl)-1,3-dihydrofuro[3,4-c]quinoline-8-carboxamide;
4-amino-N-(cyclopropylmethyl)-7-fluoro-3-methyl-N-((6-(trifluoromethyl)-3-pyridazinyl)methyl)-3H-pyrazolo[3,4-c]quinoline-8-carboxamide;
4-amino-N-(1-(trifluoromethyl)-1H-pyrazol-4-yl)-N-((5-(trifluoromethyl)-2-pyridinyl)methyl)-1,3-dihydrofuro[3,4-c][1,7]naphthyridine-8-carboxamide;
4-amino-N-(2-propanyl)-N-(4-(trifluoromethyl)benzyl)-1,3-dihydrofuro[3,4-c][1,7]naphthyridine-8-carboxamide;
4-amino-N-cyclopropyl-N-(4-(trifluoromethyl)benzyl)-1,3-dihydrofuro[3,4-c][1,7]naphthyridine-8-carboxamide;
4-amino-N-cyclopropyl-N-((1S)-1-(5-(trifluoromethyl)-2-pyridinyl)ethyl)-1,3-dihydrofuro[3,4-c]quinoline-8-carboxamide;
4-amino-N-cyclopropyl-N-((1R)-1-(5-(trifluoromethyl)-2-pyridinyl)ethyl)-1,3-dihydrofuro[3,4-c]quinoline-8-carboxamide;
4-amino-7-chloro-N-((1R)-1-(6-chloro-3-pyridinyl)ethyl)-N-ethyl-1,3-dihydrofuro[3,4-c]quinoline-8-carboxamide;
4-amino-7-chloro-N-((1S)-1-(6-chloro-3-pyridinyl)ethyl)-N-ethyl-1,3-dihydrofuro[3,4-c]quinoline-8-carboxamide;
4-amino-7-chloro-N-ethyl-N-((1R)-1-(5-(trifluoromethyl)-2-pyridinyl)ethyl)-1,3-dihydrofuro[3,4-c]quinoline-8-carboxamide;
4-amino-N-((1R)-1-(6-chloro-3-pyridinyl)ethyl)-N-ethyl-7-fluoro-1,3-dihydrofuro[3,4-c]quinoline-8-carboxamide;
4-amino-N-((1S)-1-(6-chloro-3-pyridinyl)ethyl)-N-ethyl-7-fluoro-1,3-dihydrofuro[3,4-c]quinoline-8-carboxamide;
4-amino-7-fluoro-1-methyl-N-(1-methyl-1H-pyrazol-4-yl)-N-((5-(trifluoromethyl)-2-pyridinyl)methyl)-1H-pyrazolo[4,3-c]quinoline-8-carboxamide;
4-amino-7-chloro-N-(cyclopropylmethyl)-N-((6-(trifluoromethyl)-3-pyridazinyl)methyl)-1,3-dihydrofuro[3,4-c]quinoline-8-carboxamide;
4-amino-N-ethyl-N-((1R)-1-(2-fluoro-4-(trifluoromethyl)phenyl)ethyl)-1,3-dihydrofuro[3,4-c]quinoline-8-carboxamide;
4-amino-N-ethyl-N-((1R)-1-(4-(trifluoromethyl)phenyl)ethyl)-1,3-dihydrofuro[3,4-c]quinoline-8-carboxamide;
4-amino-7-chloro-N-cyclobutyl-N-((1R)-1-(6-(trifluoromethyl)-3-pyridazinyl)ethyl)-1,3-dihydrofuro[3,4-c]quinoline-8-carboxamide;
4-amino-N-cyclobutyl-N-((1R)-1-(6-(trifluoromethyl)-3-pyridazinyl)ethyl)-1,3-dihydrofuro[3,4-c]quinoline-8-carboxamide;
(3R)-4-amino-N-cyclopropyl-3-methyl-N-((1R)-1-(5-(trifluoromethyl)-2-pyridinyl)ethyl)-1,3-dihydrofuro[3,4-c]quinoline-8-carboxamide;
4-amino-N-((6-ethoxy-3-pyridazinyl)methyl)-N-ethyl-7-fluoro-1,3-dihydrofuro[3,4-c]quinoline-8-carboxamide;
4-amino-N-((S)-cyclopropyl(5-(trifluoromethyl)-2-pyridinyl)methyl)-N-ethyl-1,3-dihydrofuro[3,4-c]quinoline-8-carboxamide;
4-amino-N-((6-ethoxy-3-pyridazinyl)methyl)-N-((1R)-1-(2-pyrimidinyl)ethyl)-1,3-dihydrofuro[3,4-c][1,7]naphthyridine-8-carboxamide;
4-amino-7-fluoro-N-(1-methyl-1H-pyrazol-4-yl)-N-((6-(trifluoromethyl)-3-pyridazinyl)methyl)-1,3-dihydrofuro[3,4-c]quinoline-8-carboxamide;
4-amino-7-chloro-N-(1-methyl-1H-pyrazol-4-yl)-N-((6-(trifluoromethyl)-3-pyridazinyl)methyl)-1,3-dihydrofuro[3,4-c]quinoline-8-carboxamide;
4-amino-N-(cyclopropylmethyl)-N-((6-ethoxy-3-pyridazinyl)methyl)-7-fluoro-1,3-dihydrofuro[3,4-c]quinoline-8-carboxamide;
4-amino-N-(cyclopropylmethyl)-N-((6-ethoxy-3-pyridazinyl)methyl)-1,3-dihydrofuro[3,4-c][1,7]naphthyridine-8-carboxamide;
5-amino-N-(2-propanyl)-N-((5-(trifluoromethyl)-2-pyridinyl)methyl)benzo[c][2,6]naphthyridine-9-carboxamide;
5-amino-N-(2-propanyl)-N-((5-(trifluoromethyl)-2-pyridinyl)methyl)pyrimido[4,5-c]quinoline-9-carboxamide;
5-amino-N-(2-propanyl)-N-((5-(trifluoromethyl)-2-pyridinyl)methyl)pyrido[4,3-c][1,7]naphthyridine-9-carboxamide;
4-amino-1-methyl-N-(1-methyl-1H-pyrazol-4-yl)-N-((5-(trifluoromethyl)-2-pyridinyl)methyl)-1H-pyrazolo[4,3-c]quinoline-8-carboxamide;
4-amino-N-((6-ethoxy-3-pyridazinyl)methyl)-3-methyl-N-((1R)-1-(2-pyrimidinyl)ethyl)-3H-pyrazolo[3,4-c]quinoline-8-carboxamide;
4-amino-N-ethyl-N-(2-(4-(trifluoromethyl)phenyl)-2-propanyl)-1,3-dihydrofuro[3,4-c]quinoline-8-carboxamide;
5-amino-N-(2-propanyl)-N-((5-(trifluoromethyl)-2-pyridinyl)methyl)pyrimido[4,5-c][1,7]naphthyridine-9-carboxamide;
4-amino-N-ethyl-N-((1R)-1-(5-(trifluoromethyl)-2-pyridinyl)propyl)-1,3-dihydrofuro[3,4-c][1,7]naphthyridine-8-carboxamide;
4-amino-N-ethyl-7-fluoro-N-((1R)-1-(5-(trifluoromethyl)-2-pyridinyl)ethyl)-1,3-dihydrofuro[3,4-c]quinoline-8-carboxamide;
4-amino-N-ethyl-N-((1R)-1-(6-(trifluoromethyl)-3-pyridazinyl)ethyl)-1,3-dihydrofuro[3,4-c][1,7]naphthyridine-8-carboxamide;
4-amino-N-((R)-cyclopropyl(5-(trifluoromethyl)-2-pyridinyl)methyl)-N-methyl-1,3-dihydrofuro[3,4-c][1,7]naphthyridine-8-carboxamide;
4-amino-N-((S)-cyclopropyl(5-(trifluoromethyl)-2-pyridinyl)methyl)-N-methyl-1,3-dihydrofuro[3,4-c][1,7]naphthyridine-8-carboxamide;
4-amino-N-(cyclopropylmethyl)-N-((6-ethoxy-3-pyridazinyl)methyl)-7-fluoro-3-methyl-3H-pyrazolo[3,4-c]quinoline-8-carboxamide;
(3R)-4-amino-N-(cyclopropylmethyl)-N-((6-ethoxy-3-pyridazinyl)methyl)-3-methyl-1,3-dihydrofuro[3,4-c]quinoline-8-carboxamide;
4-amino-N-((R)-cyclopropyl(6-(trifluoromethyl)-3-pyridazinyl)methyl)-N-ethyl-1,3-dihydrofuro[3,4-c][1,7]naphthyridine-8-carboxamide;
(3R)-4-amino-3-methyl-N-((1R)-1-(5-(trifluoromethyl)-2-pyridinyl)ethyl)-1,3-dihydrofuro[3,4-c]quinoline-8-carboxamide;
4-amino-7-fluoro-N,1-dimethyl-N-((5-methyl-2-pyridinyl)methyl)-1H-pyrazolo[4,3-c]quinoline-8-carboxamide;
4-amino-7-fluoro-N-((5-fluoro-2-pyridinyl)methyl)-N,1-dimethyl-1H-pyrazolo[4,3-c]quinoline-8-carboxamide;
4-amino-N-((2,6-difluoro-3-pyridinyl)methyl)-7-fluoro-N,1-dimethyl-1H-pyrazolo[4,3-c]quinoline-8-carboxamide;
4-amino-7-chloro-N-((1R)-1-(5-fluoro-2-pyridinyl)ethyl)-N-methyl-1,3-dihydrofuro[3,4-c]quinoline-8-carboxamide;
4-amino-7-chloro-N-((1S)-1-(5-fluoro-2-pyridinyl)ethyl)-N-methyl-1,3-dihydrofuro[3,4-c]quinoline-8-carboxamide;
4-amino-N-((1R)-1-(5-(difluoromethyl)-2-pyridinyl)ethyl)-N-ethyl-1,3-dihydrofuro[3,4-c][1,7]naphthyridine-8-carboxamide;
4-amino-N-methyl-N-((1R)-1-(5-(trifluoromethyl)-2-pyrazinyl)ethyl)-1,3-dihydrofuro[3,4-c]quinoline-8-carboxamide;
(3R)-4-amino-N-((1R)-1-(5-(difluoromethyl)-2-pyridinyl)ethyl)-N-ethyl-3-methyl-1,3-dihydrofuro[3,4-c]quinoline-8-carboxamide;
4-amino-N,1-dimethyl-N-((1R)-1-(5-(trifluoromethyl)-2-pyrazinyl)ethyl)-1H-pyrazolo[4,3-c]quinoline-8-carboxamide;
(3R)-4-amino-N-ethyl-3-methyl-N-((1R)-1-(5-(trifluoromethyl)-2-pyridinyl)ethyl)-1,3-dihydrofuro[3,4-c]quinoline-8-carboxamide;
(3R)-4-amino-N-ethyl-3-methyl-N-((1R)-1-(6-(trifluoromethyl)-3-pyridazinyl)ethyl)-1,3-dihydrofuro[3,4-c]quinoline-8-carboxamide;
4-amino-7-fluoro-N,3-dimethyl-N-((1R)-1-(6-(trifluoromethyl)-3-pyridazinyl)ethyl)-3H-pyrazolo[3,4-c]quinoline-8-carboxamide;
4-amino-7-fluoro-N,1-dimethyl-N-((1R)-1-(5-(trifluoromethyl)-2-pyrazinyl)ethyl)-1H-pyrazolo[4,3-c]quinoline-8-carboxamide;
4-amino-N-ethyl-7-fluoro-N-((1R)-1-(6-(trifluoromethyl)-3-pyridazinyl)ethyl)-1,3-dihydrofuro[3,4-c]quinoline-8-carboxamide;
4-amino-7-fluoro-N-((2-fluoro-6-(trifluoromethyl)-3-pyridinyl)methyl)-N,1-dimethyl-1H-pyrazolo[4,3-c]quinoline-8-carboxamide;
4-amino-N-((R)-cyclopropyl(6-(trifluoromethyl)-3-pyridazinyl)methyl)-N-ethyl-1,3-dihydrofuro[3,4-c]quinoline-8-carboxamide;
(3R)-4-amino-N-ethyl-7-fluoro-3-methyl-N-((1R)-1-(6-(trifluoromethyl)-3-pyridazinyl)ethyl)-1,3-dihydrofuro[3,4-c]quinoline-8-carboxamide;
(3S)-4-amino-N-ethyl-7-fluoro-3-methyl-N-((1R)-1-(6-(trifluoromethyl)-3-pyridazinyl)ethyl)-1,3-dihydrofuro[3,4-c]quinoline-8-carboxamide;
4-amino-N,3-dimethyl-N-((1R)-1-(4-(pentafluoroethyl)phenyl)ethyl)-3H-pyrazolo[3,4-c][1,7]naphthyridine-8-carboxamide;
4-amino-7-fluoro-N,1-dimethyl-N-((1S)-1-(4-(pentafluoroethyl)phenyl)ethyl)-1H-pyrazolo[4,3-c]quinoline-8-carboxamide;
4-amino-7-fluoro-N,1-dimethyl-N-((1R)-1-(4-(pentafluoroethyl)phenyl)ethyl)-1H-pyrazolo[4,3-c]quinoline-8-carboxamide;
4-amino-7-fluoro-N,1-dimethyl-N-((5-(1-(trifluoromethyl)-1H-pyrazol-4-yl)-2-pyridinyl)methyl)-1H-pyrazolo[4,3-c]quinoline-8-carboxamide;
4-amino-N-cyclopropyl-1-methyl-N-((6-(trifluoromethyl)-3-pyridazinyl)methyl)-1H-pyrazolo[4,3-c]quinoline-8-carboxamide;
4-amino-7-chloro-N,1-dimethyl-N-((5-(trifluoromethyl)-2-pyridinyl)methyl)-1H-pyrazolo[4,3-c]quinoline-8-carboxamide;
4-amino-7-chloro-N,1-dimethyl-N-((6-(trifluoromethyl)-3-pyridazinyl)methyl)-1H-pyrazolo[4,3-c]quinoline-8-carboxamide;
4-amino-7-chloro-N-ethyl-1-methyl-N-((5-(trifluoromethyl)-2-pyridinyl)methyl)-1H-pyrazolo[4,3-c]quinoline-8-carboxamide;
4-amino-7-chloro-N-ethyl-1-methyl-N-((6-(trifluoromethyl)-3-pyridazinyl)methyl)-1H-pyrazolo[4,3-c]quinoline-8-carboxamide;
4-amino-7-chloro-N-cyclopropyl-1-methyl-N-((5-(trifluoromethyl)-2-pyridinyl)methyl)-1H-pyrazolo[4,3-c]quinoline-8-carboxamide;
4-amino-7-chloro-N-cyclopropyl-1-methyl-N-((6-(trifluoromethyl)-3-pyridazinyl)methyl)-1H-pyrazolo[4,3-c]quinoline-8-carboxamide;
4-amino-1-methyl-N-(2,2,2-trifluoroethyl)-N-((5-(trifluoromethyl)-2-pyridinyl)methyl)-1H-pyrazolo[4,3-c]quinoline-8-carboxamide;
4-amino-7-chloro-N-cyclobutyl-1-methyl-N-((5-(trifluoromethyl)-2-pyridinyl)methyl)-1H-pyrazolo[4,3-c]quinoline-8-carboxamide;
4-amino-7-chloro-N-cyclobutyl-1-methyl-N-((6-(trifluoromethyl)-3-pyridazinyl)methyl)-1H-pyrazolo[4,3-c]quinoline-8-carboxamide;
4-amino-7-fluoro-1-methyl-N-(2,2,2-trifluoroethyl)-N-((5-(trifluoromethyl)-2-pyridinyl)methyl)-1H-pyrazolo[4,3-c]quinoline-8-carboxamide;
4-amino-N-cyclobutyl-7-fluoro-1-methyl-N-(1,3-oxazol-4-ylmethyl)-1H-pyrazolo[4,3-c]quinoline-8-carboxamide;
4-amino-7-fluoro-N,1-dimethyl-N-((5-(trifluoromethyl)-2-pyridinyl)methyl)-1H-pyrazolo[4,3-c]quinoline-8-carboxamide;
4-amino-N-cyclopropyl-7-fluoro-1-methyl-N-((5-(trifluoromethyl)-2-pyridinyl)methyl)-1H-pyrazolo[4,3-c]quinoline-8-carboxamide;
4-amino-N-cyclopropyl-7-fluoro-1-methyl-N-((6-(trifluoromethyl)-3-pyridazinyl)methyl)-1H-pyrazolo[4,3-c]quinoline-8-carboxamide;
4-amino-7-fluoro-1-methyl-N-(2-propanyl)-N-((5-(trifluoromethyl)-2-pyridinyl)methyl)-1H-pyrazolo[4,3-c]quinoline-8-carboxamide;
4-amino-7-fluoro-N-((2-methoxy-6-(trifluoromethyl)-3-pyridinyl)methyl)-N,1-dimethyl-1H-pyrazolo[4,3-c]quinoline-8-carboxamide;
4-amino-N-cyclobutyl-7-fluoro-1-methyl-N-((5-(trifluoromethyl)-2-pyridinyl)methyl)-1H-pyrazolo[4,3-c]quinoline-8-carboxamide;
4-amino-7-chloro-N-cyclopropyl-3-methyl-N-((5-(trifluoromethyl)-2-pyridinyl)methyl)-3H-pyrazolo[3,4-c]quinoline-8-carboxamide;
4-amino-N-cyclopropyl-7-fluoro-N-((3-fluoro-5-(trifluoromethyl)-2-pyridinyl)methyl)-1-methyl-1H-pyrazolo[4,3-c]quinoline-8-carboxamide;
4-amino-N,1-dimethyl-7-(trifluoromethyl)-N-((5-(trifluoromethyl)-2-pyridinyl)methyl)-1H-pyrazolo[4,3-c]quinoline-8-carboxamide;
4-amino-N,1-dimethyl-7-(trifluoromethyl)-N-((6-(trifluoromethyl)-3-pyridazinyl)methyl)-1H-pyrazolo[4,3-c]quinoline-8-carboxamide;
4-amino-N-cyclopropyl-1-methyl-7-(trifluoromethyl)-N-((5-(trifluoromethyl)-2-pyridinyl)methyl)-1H-pyrazolo[4,3-c]quinoline-8-carboxamide;
4-amino-N-cyclopropyl-1-methyl-7-(trifluoromethyl)-N-((6-(trifluoromethyl)-3-pyridazinyl)methyl)-1H-pyrazolo[4,3-c]quinoline-8-carboxamide;
4-amino-N-((1R)-1-cyclopropylethyl)-7-fluoro-N-((6-(trifluoromethyl)-3-pyridazinyl)methyl)-1,3-dihydrofuro[3,4-c]quinoline-8-carboxamide;
4-amino-N-((1S)-1-cyclopropylethyl)-7-fluoro-N-((6-(trifluoromethyl)-3-pyridazinyl)methyl)-1,3-dihydrofuro[3,4-c]quinoline-8-carboxamide;
4-amino-N-(cyclopropylmethyl)-7-fluoro-N-((6-(2,2,2-trifluoroethoxy)-3-pyridazinyl)methyl)-1,3-dihydrofuro[3,4-c]quinoline-8-carboxamide;
4-amino-N-(cyclopropylmethyl)-7-fluoro-3-methyl-N-((6-(2,2,2-trifluoroethoxy)-3-pyridazinyl)methyl)-3H-pyrazolo[3,4-c]quinoline-8-carboxamide;
(3R)-4-amino-N-(cyclopropylmethyl)-7-fluoro-3-methyl-N-((6-(2,2,2-trifluoroethoxy)-3-pyridazinyl)methyl)-1,3-dihydrofuro[3,4-c]quinoline-8-carboxamide;
4-amino-N-ethyl-N-((1S)-1-(5-(trifluoromethyl)-2-pyridinyl)ethyl)-1,3-dihydrofuro[3,4-c]quinoline-8-carboxamide;
4-amino-N-ethyl-N-((1R)-1-(5-(trifluoromethyl)-2-pyridinyl)ethyl)-1,3-dihydrofuro[3,4-c]quinoline-8-carboxamide;
4-amino-N-ethyl-N-((1R)-1-(5-(trifluoromethyl)-2-pyridinyl)ethyl)-1,3-dihydrofuro[3,4-c][1,7]naphthyridine-8-carboxamide;
4-amino-N-ethyl-N-((1S)-1-(5-(trifluoromethyl)-2-pyridinyl)ethyl)-1,3-dihydrofuro[3,4-c][1,7]naphthyridine-8-carboxamide;
4-amino-N-(1-methyl-1H-pyrazol-4-yl)-N-((1R)-1-(5-(trifluoromethyl)-2-pyridinyl)ethyl)-1,3-dihydrofuro[3,4-c][1,7]naphthyridine-8-carboxamide;
4-amino-N-(1-methyl-1H-pyrazol-4-yl)-N-((1S)-1-(5-(trifluoromethyl)-2-pyridinyl)ethyl)-1,3-dihydrofuro[3,4-c][1,7]naphthyridine-8-carboxamide;
(3S)-4-amino-3-methyl-N-(1-methyl-1H-pyrazol-4-yl)-N-((5-(trifluoromethyl)-2-pyridinyl)methyl)-1,3-dihydrofuro[3,4-c][1,7]naphthyridine-8-carboxamide;
(3R)-4-amino-3-methyl-N-(1-methyl-1H-pyrazol-4-yl)-N-((5-(trifluoromethyl)-2-pyridinyl)methyl)-1,3-dihydrofuro[3,4-c][1,7]naphthyridine-8-carboxamide;
(3S)-4-amino-N-cyclobutyl-3-methyl-N-((6-(trifluoromethyl)-3-pyridazinyl)methyl)-1,3-dihydrofuro[3,4-c][1,7]naphthyridine-8-carboxamide;
(3R)-4-amino-N-cyclobutyl-3-methyl-N-((6-(trifluoromethyl)-3-pyridazinyl)methyl)-1,3-dihydrofuro[3,4-c][1,7]naphthyridine-8-carboxamide;
4-amino-N-(2-propanyl)-N-((1S)-1-(5-(trifluoromethyl)-2-pyridinyl)ethyl)-1,3-dihydrofuro[3,4-c]quinoline-8-carboxamide;
4-amino-N-(2-propanyl)-N-((1R)-1-(5-(trifluoromethyl)-2-pyridinyl)ethyl)-1,3-dihydrofuro[3,4-c]quinoline-8-carboxamide;
4-amino-7-fluoro-N-((1S)-1-(5-fluoro-2-pyrimidinyl)ethyl)-N-(2-(trifluoromethoxy)ethyl)-1,3-dihydrofuro[3,4-c]quinoline-8-carboxamide;
4-amino-7-fluoro-N-((1R)-1-(5-fluoro-2-pyrimidinyl)ethyl)-N-(2-(trifluoromethoxy)ethyl)-1,3-dihydrofuro[3,4-c]quinoline-8-carboxamide;
4-amino-N-((1R)-1-(4-cyanophenyl)ethyl)-N-ethyl-1,3-dihydrofuro[3,4-c]quinoline-8-carboxamide;
4-amino-N-((1S)-1-(4-cyanophenyl)ethyl)-N-ethyl-1,3-dihydrofuro[3,4-c]quinoline-8-carboxamide;
4-amino-N-((1S)-1-(5-cyano-2-pyridinyl)ethyl)-N-ethyl-1,3-dihydrofuro[3,4-c]quinoline-8-carboxamide;
4-amino-N-((1R)-1-(5-cyano-2-pyridinyl)ethyl)-N-ethyl-1,3-dihydrofuro[3,4-c]quinoline-8-carboxamide;
(3R)-4-amino-3-methyl-N-(2-propanyl)-N-((1R)-1-(5-(trifluoromethyl)-2-pyridinyl)ethyl)-1,3-dihydrofuro[3,4-c]quinoline-8-carboxamide;
(3R)-4-amino-3-methyl-N-(2-propanyl)-N-((1S)-1-(5-(trifluoromethyl)-2-pyridinyl)ethyl)-1,3-dihydrofuro[3,4-c]quinoline-8-carboxamide;
4-amino-7-fluoro-N-(2-propanyl)-N-((1S)-1-(5-(trifluoromethyl)-2-pyridinyl)ethyl)-1,3-dihydrofuro[3,4-c]quinoline-8-carboxamide;
4-amino-7-fluoro-N-(2-propanyl)-N-((1R)-1-(5-(trifluoromethyl)-2-pyridinyl)ethyl)-1,3-dihydrofuro[3,4-c]quinoline-8-carboxamide;
(3R)-4-amino-N-((1S)-1-(5-cyano-2-pyridinyl)ethyl)-N-ethyl-3-methyl-1,3-dihydrofuro[3,4-c]quinoline-8-carboxamide;
(3R)-4-amino-N-((1R)-1-(5-cyano-2-pyridinyl)ethyl)-N-ethyl-3-methyl-1,3-dihydrofuro[3,4-c]quinoline-8-carboxamide;
4-amino-7-chloro-N-(2-propanyl)-N-((1R)-1-(5-(trifluoromethyl)-2-pyridinyl)ethyl)-1,3-dihydrofuro[3,4-c]quinoline-8-carboxamide;
4-amino-7-chloro-N-(2-propanyl)-N-((1S)-1-(5-(trifluoromethyl)-2-pyridinyl)ethyl)-1,3-dihydrofuro[3,4-c]quinoline-8-carboxamide;
4-amino-N-(2-propanyl)-N-((1S)-1-(5-(trifluoromethyl)-2-pyridinyl)ethyl)-1,3-dihydrofuro[3,4-c][1,7]naphthyridine-8-carboxamide;
4-amino-N-(2-propanyl)-N-((1R)-1-(5-(trifluoromethyl)-2-pyridinyl)ethyl)-1,3-dihydrofuro[3,4-c][1,7]naphthyridine-8-carboxamide;
4-amino-N-methyl-N-((1R)-1-(5-(trifluoromethyl)-2-pyridinyl)propyl)-1,3-dihydrofuro[3,4-c][1,7]naphthyridine-8-carboxamide;
4-amino-N-methyl-N-((1S)-1-(5-(trifluoromethyl)-2-pyridinyl) propyl)-1,3-dihydrofuro[3,4-c][1,7]naphthyridine-8-carboxamide;
(3R)-4-amino-N,3-dimethyl-N-((1R)-1-(5-(trifluoromethyl)-2-pyridinyl)ethyl)-1,3-dihydrofuro[3,4-c]quinoline-8-carboxamide;
(3R)-4-amino-N,3-dimethyl-N-((1S)-1-(5-(trifluoromethyl)-2-pyridinyl)ethyl)-1,3-dihydrofuro[3,4-c]quinoline-8-carboxamide;
4-amino-N-((1R)-1-(3,5-difluoro-2-pyridinyl)ethyl)-7-fluoro-N,1-dimethyl-1H-pyrazolo[4,3-c]quinoline-8-carboxamide;
4-amino-N-methyl-N-((1R)-1-(5-(trifluoromethyl)-2-pyridinyl)ethyl)-1,3-dihydrofuro[3,4-c][1,7]naphthyridine-8-carboxamide;
4-amino-N,1-dimethyl-N-((1R)-1-(6-(trifluoromethyl)-3-pyridazinyl)ethyl)-1H-pyrazolo[4,3-c]quinoline-8-carboxamide;
(3R)-4-amino-N,3-dimethyl-N-((1R)-1-(6-(trifluoromethyl)-3-pyridazinyl)ethyl)-1,3-dihydrofuro[3,4-c]quinoline-8-carboxamide;
4-amino-7-fluoro-N-methyl-N-((1R)-1-(4-(trifluoromethyl)phenyl)ethyl)-1 ,3-dihydrofuro[3,4-c]quinoline-8-carboxamide;
4-amino-7-fluoro-N-methyl-N-((1R)-1-(6-(trifluoromethyl)-3-pyridazinyl)ethyl)-1,3-dihydrofuro[3,4-c]quinoline-8-carboxamide;
(3R)-4-amino-7-fluoro-N,3-dimethyl-N-((1S)-1-(4-(trifluoromethyl)phenyl)ethyl)-1,3-dihydrofuro[3,4-c]quinoline-8-carboxamide;
(3R)-4-amino-7-fluoro-N,3-dimethyl-N-((1R)-1-(4-(trifluoromethyl)phenyl)ethyl)-1,3-dihydrofuro[3,4-c]quinoline-8-carboxamide;
4-amino-N-((1R)-1-(3-fluoro-5-(trifluoromethyl)-2-pyridinyl)ethyl)-N,3-dimethyl-3H-pyrazolo[3,4-c][1,7]naphthyridine-8-carboxamide;
(3R)-4-amino-7-fluoro-N,3-dimethyl-N-((1R)-1-(6-(trifluoromethyl)-3-pyridazinyl)ethyl)-1,3-dihydrofuro[3,4-c]quinoline-8-carboxamide;
4-amino-7-fluoro-N-((1R)-1-(3-fluoro-5-(trifluoromethyl)-2-pyridinyl)ethyl)-N,1-dimethyl-1H-pyrazolo[4,3-c]quinoline-8-carboxamide;
4-amino-7-fluoro-N-((1S)-1-(3-fluoro-5-(trifluoromethyl)-2-pyridinyl)ethyl)-N,1-dimethyl-1H-pyrazolo[4,3-c]quinoline-8-carboxamide;
4-amino-7-fluoro-N-((1R)-1-(3-fluoro-5-(trifluoromethyl)-2-pyridinyl)ethyl)-N, 3-dimethyl-3H-pyrazolo[3,4-c]quinoline-8-carboxamide;
4-amino-7-fluoro-N-((1R)-1-(3-methoxy-5- (trifluoromethyl)-2-pyridinyl) ethyl)-N,3-dimethyl-3H-pyrazolo[3,4-c]quinoline-8-carboxamide;
4-amino-7-fluoro-N-((1R)-1-(3-methoxy-5-(trifluoromethyl)-2-pyridinyl)ethyl)-N,1-dimethyl-1H-pyrazolo[4,3-c]quinoline-8-carboxamide;
4-amino-N,3-dimethyl-N-((1R)-1-(4-(pentafluoro-lambda~6~-sulfanyl)phenyl)ethyl)-3H-pyrazolo[3,4-c][1,7]naphthyridine-8-carboxamide;
4-amino-7-fluoro-N,1-dimethyl-N-((1S)-1-(4-(pentafluoro-lambda~6~-sulfanyl)phenyl)ethyl)-1H-pyrazolo[4,3-c]quinoline-8-carboxamide;
4-amino-7-fluoro-N,1-dimethyl-N-((1R)-1-(4-(pentafluoro-lambda-6~-sulfanyl)phenyl)ethyl)-1H-pyrazolo[4,3-c]quinoline-8-carboxamide;
4-amino-1-methyl-N-(4-(pentafluoro-lambda~6~-sulfanyl)benzyl)-N-((1R)-1-(5-pyrimidinyl)propyl)-1H-pyrazolo[4,3-c][1,7]naphthyridine-8-carboxamide;
4-amino-1-methyl-N-(4-(pentafluoro-lambda~6~-sulfanyl)benzyl)-N-((1S)-1-(5-pyrimidinyl)propyl)-1H-pyrazolo[4,3-c][1,7]naphthyridine-8-carboxamide;
4-amino-7-fluoro-N-((1R)-1-(5-fluoro-2-pyridinyl)ethyl)-N,1-dimethyl-1H-pyrazolo[4,3-c]quinoline-8-carboxamide;
4-amino-7-fluoro-N-((1S)-1-(5-fluoro-2-pyridinyl)ethyl)-N,1-dimethyl-1H-pyrazolo[4,3-c]quinoline-8-carboxamide;
4-amino-N-ethyl-7-fluoro-N-((1R)-1-(5-fluoro-2-pyridinyl)ethyl)-1-methyl-1H-pyrazolo[4,3-c]quinoline-8-carboxamide;
4-amino-N-ethyl-7-fluoro-N-((1S)-1-(5-fluoro-2-pyridinyl)ethyl)-1-methyl-1H-pyrazolo[4,3-c]quinoline-8-carboxamide;
4-amino-N-cyclopropyl-7-fluoro-1-methyl-N-((1S)-1-(6-(trifluoromethyl)-3-pyridazinyl)ethyl)-1H-pyrazolo[4,3-c]quinoline-8-carboxamide;
4-amino-N-cyclopropyl-7-fluoro-1-methyl-N-((1R)-1-(6-(trifluoromethyl)-3-pyridazinyl)ethyl)-1H-pyrazolo[4,3-c]quinoline-8-carboxamide;
4-amino-N,1-dimethyl-7-(trifluoromethyl)-N-((1R)-1-(6-(trifluoromethyl)-3-pyridazinyl)ethyl)-1H-pyrazolo[4,3-c]quinoline-8-carboxamide;
4-amino-7-chloro-N-cyclopropyl-1-methyl-N-((1S)-1-(6-(trifluoromethyl)-3-pyridazinyl)ethyl)-1H-pyrazolo[4,3-c]quinoline-8-carboxamide;
4-amino-7-chloro-N-cyclopropyl-1-methyl-N-((1R)-1-(6-(trifluoromethyl)-3-pyridazinyl)ethyl)-1H-pyrazolo[4,3-c]quinoline-8-carboxamide;
4-amino-N-(1-methyl-1H-pyrazol-4-yl)-N-(4-(trifluoromethyl)benzyl)-1,3-dihydrofuro[3,4-c][1,7]naphthyridine-8-carboxamide;
4-amino-N-(3-fluorophenyl)-N-((5-(trifluoromethyl)-2-pyridinyl)methyl)-1,3-dihydrofuro[3,4-c]quinoline-8-carboxamide;
4-amino-N-(1-methyl-1H-pyrazol-4-yl)-N-((5-(trifluoromethyl)-2-pyridinyl)methyl)-1,3-dihydrofuro[3,4-c]quinoline-8-carboxamide;
4-amino-N-(1-methyl-1H-pyrazol-4-yl)-N-((5-(trifluoromethyl)-2-pyridinyl) methyl)-1,3-dihydrofuro[3,4-c][1,7]naphthyridine-8-carboxamide;
4-amino-N-(1-methyl-1H-pyrazol-4-yl)-N-((5-(trifluoromethyl)-2-pyridinyl)methyl)-1,3-dihydrofuro[3,4-c][1,8]naphthyridine-8-carboxamide;
5-amino-N-((1R)-1-(2-pyrimidinyl)ethyl)-N-((5-(trifluoromethyl)-2-pyridinyl)methyl)-1,4-dihydro-2H-pyrano[3,4-c]quinoline-9-carboxamide;
4-amino-N-((1R)-1-(2-pyrimidinyl)ethyl)-N-((5-(trifluoromethyl)-2-pyridinyl)methyl)-2, 3-dihydrofuro[3,2-c]quinoline-8-carboxamide;
4-amino-3,3-dimethyl-N-(2-methylpropyl)-N-((5-(trifluoromethyl)-2-pyridinyl)methyl)-1,3-dihydrofuro[3,4-c]quinoline-8-carboxamide;
(3R)-4-amino-N-((3-fluoro-2-pyridinyl)methyl)-3-methyl-N-((5-(trifluoromethyl)-2-pyridinyl)methyl)-1,3-dihydrofuro[3,4-c]quinoline-8-carboxamide;
(3S)-4-amino-N-((3-fluoro-2-pyridinyl)methyl)-3-methyl-N-((5-(trifluoromethyl)-2-pyridinyl)methyl)-1,3-dihydrofuro[3,4-c]quinoline-8-carboxamide;
(3R)-4-amino-3-methyl-N-((1R)-1-(tetrahydro-2H-pyran-4-yl)ethyl)-N-((5-(trifluoromethyl)-2-pyridinyl)methyl)-1,3-dihydrofuro[3,4-c]quinoline-8-carboxamide;
(3R)-4-amino-3-methyl-N-((1S)-1-(tetrahydro-2H-pyran-4-yl)ethyl)-N-((5-(trifluoromethyl)-2-pyridinyl)methyl)-1,3-dihydrofuro[3,4-c]quinoline-8-carboxamide;
(3S)-4-amino-3-methyl-N-((1R)-1-(tetrahydro-2H-pyran-4-yl)ethyl)-N-((5-(trifluoromethyl)-2-pyridinyl)methyl)-1,3-dihydrofuro[3,4-c]quinoline-8-carboxamide;
(3S)-4-amino-3-methyl-N-((1S)-1-(tetrahydro-2H-pyran-4-yl)ethyl)-N-((5-(trifluoromethyl)-2-pyridinyl)methyl)-1,3-dihydrofuro[3,4-c]quinoline-8-carboxamide;
(3R)-4-amino-N-((5-cyano-2-pyridinyl)methyl)-3-methyl-N-((1R)-1-(tetrahydro-2H-pyran-4-yl)ethyl)-1,3-dihydrofuro[3,4-c]quinoline-8-carboxamide;
(3R)-4-amino-N-((5-cyano-2-pyridinyl)methyl)-3-methyl-N-((1S)-1-(tetrahydro-2H-pyran-4-yl)ethyl)-1,3-dihydrofuro[3,4-c]quinoline-8-carboxamide;
(3S)-4-amino-N-((5-cyano-2-pyridinyl)methyl)-3-methyl-N-((1R)-1-(tetrahydro-2H-pyran-4-yl)ethyl)-1,3-dihydrofuro[3,4-c]quinoline-8-carboxamide;
(3S)-4-amino-N-((5-cyano-2-pyridinyl)methyl)-3-methyl-N-((1S)-1-(tetrahydro-2H-pyran-4-yl)ethyl)-1,3-dihydrofuro[3,4-c]quinoline-8-carboxamide;
(3R)-4-amino-N-((trans-4-hydroxycyclohexyl)methyl)-3-methyl-N-((5-(trifluoromethyl)-2-pyridinyl)methyl)-1,3-dihydrofuro[3,4-c]quinoline-8-carboxamide;
(3S)-4-amino-N-((trans-4-hydroxycyclohexyl)methyl)-3-methyl-N-((5-(trifluoromethyl)-2-pyridinyl)methyl)-1,3-dihydrofuro[3,4-c]quinoline-8-carboxamide;
(3R)-4-amino-N-((5-cyano-2-pyridinyl)methyl)-N-((trans-4-hydroxycyclohexyl)methyl)-3-methyl-1,3-dihydrofuro[3,4-c]quinoline-8-carboxamide;
(3S)-4-amino-N-((5-cyano-2-pyridinyl)methyl)-N-((trans-4-hydroxycyclohexyl)methyl)-3-methyl-1,3-dihydrofuro[3,4-c]quinoline-8-carboxamide;
(3R)-4-amino-3-methyl-N-(tetrahydro-2H-pyran-4-ylmethyl)-N-((5-(trifluoromethyl)-2-pyridinyl)methyl)-1,3-dihydrofuro[3,4-c]quinoline-8-carboxamide;
(3S)-4-amino-3-methyl-N-(tetrahydro-2H-pyran-4-ylmethyl)-N-((5-(trifluoromethyl)-2-pyridinyl)methyl)-1,3-dihydrofuro[3,4-c]quinoline-8-carboxamide;
(3R)-4-amino-N-((5-cyano-2-pyridinyl)methyl)-3-methyl-N-(tetrahydro-2H-pyran-4-ylmethyl)-1,3-dihydrofuro[3,4-c]quinoline-8-carboxamide;
(3S)-4-amino-N-((5-cyano-2-pyridinyl)methyl)-3-methyl-N-(tetrahydro-2H-pyran-4-ylmethyl)-1,3-dihydrofuro[3,4-c]quinoline-8-carboxamide;
(3R)-4-amino-N-((1R,2R)-2-cyanocyclopentyl)-3-methyl-N-((5-(trifluoromethyl)-2-pyridinyl)methyl)-1,3-dihydrofuro[3,4-c]quinoline-8-carboxamide;
(3R)-4-amino-N-((1R,2S)-2-cyanocyclopentyl)-3-methyl-N-((5-(trifluoromethyl)-2-pyridinyl)methyl)-1,3-dihydrofuro[3,4-c]quinoline-8-carboxamide;
(3R)-4-amino-N-((1S,2R)-2-cyanocyclopentyl)-3-methyl-N-((5-(trifluoromethyl)-2-pyridinyl)methyl)-1,3-dihydrofuro[3,4-c]quinoline-8-carboxamide;
(3R)-4-amino-N-((1S,2S)-2-cyanocyclopentyl)-3-methyl-N-((5-(trifluoromethyl)-2-pyridinyl)methyl)-1,3-dihydrofuro[3,4-c]quinoline-8-carboxamide;
(3S)-4-amino-N-((1R,2R)-2-cyanocyclopentyl)-3-methyl-N-((5-(trifluoromethyl)-2-pyridinyl)methyl)-1,3-dihydrofuro[3,4-c]quinoline-8-carboxamide;
(3S)-4-amino-N-((1R,2S)-2-cyanocyclopentyl)-3-methyl-N-((5-(trifluoromethyl)-2-pyridinyl)methyl)-1,3-dihydrofuro[3,4-c]quinoline-8-carboxamide;
(3S)-4-amino-N-((1S,2R)-2-cyanocyclopentyl)-3-methyl-N-((5-(trifluoromethyl)-2-pyridinyl)methyl)-1,3-dihydrofuro[3,4-c]quinoline-8-carboxamide;
(3S)-4-amino-N-((1S,2S)-2-cyanocyclopentyl)-3-methyl-N-((5-(trifluoromethyl)-2-pyridinyl)methyl)-1,3-dihydrofuro[3,4-c]quinoline-8-carboxamide;
(3R)-4-amino-N-((1R,2R)-2-cyanocyclopentyl)-N-((5-cyano-2-pyridinyl)methyl)-3-methyl-1,3-dihydrofuro[3,4-c]quinoline-8-carboxamide;
(3R)-4-amino-N-((1R,2S)-2-cyanocyclopentyl)-N-((5-cyano-2-pyridinyl)methyl)-3-methyl-1,3-dihydrofuro[3,4-c]quinoline-8-carboxamide;
(3R)-4-amino-N-((1S,2R)-2-cyanocyclopentyl)-N-((5-cyano-2-pyridinyl)methyl)-3-methyl-1,3-dihydrofuro[3,4-c]quinoline-8-carboxamide;
(3R)-4-amino-N-((1S,2S)-2-cyanocyclopentyl)-N-((5-cyano-2-pyridinyl)methyl)-3-methyl-1,3-dihydrofuro[3,4-c]quinoline-8-carboxamide;
(3S)-4-amino-N-((1R,2R)-2-cyanocyclopentyl)-N-((5-cyano-2-pyridinyl)methyl)-3-methyl-1,3-dihydrofuro[3,4-c]quinoline-8-carboxamide;
(3S)-4-amino-N-((1R,2S)-2-cyanocyclopentyl)-N-((5-cyano-2-pyridinyl)methyl)-3-methyl-1,3-dihydrofuro[3,4-c]quinoline-8-carboxamide;
(3S)-4-amino-N-((1S,2R)-2-cyanocyclopentyl)-N-((5-cyano-2-pyridinyl)methyl)-3-methyl-1,3-dihydrofuro[3,4-c]quinoline-8-carboxamide;
(3S)-4-amino-N-((1S,2S)-2-cyanocyclopentyl)-N-((5-cyano-2-pyridinyl)methyl)-3-methyl-1,3-dihydrofuro[3,4-c]quinoline-8-carboxamide;
(3R)-4-amino-3-methyl-N-((3R)-tetrahydro-3-furanyl)-N-((5-(trifluoromethyl)-2-pyridinyl)methyl)-1,3-dihydrofuro[3,4-c]quinoline-8-carboxamide;
(3R)-4-amino-3-methyl-N-((3S)-tetrahydro-3-furanyl)-N-((5-(trifluoromethyl)-2-pyridinyl)methyl)-1,3-dihydrofuro[3,4-c]quinoline-8-carboxamide;
(3S)-4-amino-3-methyl-N-((3R)-tetrahydro-3-furanyl)-N-((5-(trifluoromethyl)-2-pyridinyl)methyl)-1,3-dihydrofuro[3,4-c]quinoline-8-carboxamide;
(3S)-4-amino-3-methyl-N-((3S)-tetrahydro-3-furanyl)-N-((5-(trifluoromethyl)-2-pyridinyl)methyl)-1,3-dihydrofuro[3,4-c]quinoline-8-carboxamide;
(3R)-4-amino-3-methyl-N-((1R,5S,6r)-3-oxabicyclo[3.1.0]hexan-6-yl)-N-((5-(trifluoromethyl)-2-pyridinyl)methyl)-1,3-dihydrofuro[3,4-c]quinoline-8-carboxamide;
(3S)-4-amino-3-methyl-N-((1R,5S,6r)-3-oxabicyclo[3.1.0]hexan-6-yl)-N-((5-(trifluoromethyl)-2-pyridinyl)methyl)-1,3-dihydrofuro[3,4-c]quinoline-8-carboxamide;
(3R)-4-amino-3-methyl-N-((3R,4R)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3R)-4-amino-3-methyl-N-((3R,4S)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3R)-4-amino-3-methyl-N-((3S,4R)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3R)-4-amino-3-methyl-N-((3S,4S)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3S)-4-amino-3-methyl-N-((3R,4R)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3S)-4-amino-3-methyl-N-((3R,4S)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3S)-4-amino-3-methyl-N-((3S,4R)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3S)-4-amino-3-methyl-N-((3S,4S)-3-methyltetrahydro-2H-pyran-4-yl)-N-((5-(trifluoromethyl)-2-pyridinyl)methyl)-1,3-dihydrofuro[3,4-c]quinoline-8-carboxamide;
(3R)-4-amino-N-((3S,4R)-3-methoxytetrahydro-2H-pyran-4-yl)-3-methyl-N-((5-(trifluoromethyl)-2-pyridinyl)methyl)-1,3-dihydrofuro[3,4-c]quinoline-8-carboxamide;
(3S)-4-amino-N-((3S,4R)-3-methoxytetrahydro-2H-pyran-4-yl)-3-methyl-N-((5-(trifluoromethyl)-2-pyridinyl)methyl)-1,3-dihydrofuro[3,4-c]quinoline-8-carboxamide;
(3R)-4-amino-N-((3R,4S)-3-methoxytetrahydro-2H-pyran-4-yl)-3-methyl-N-((5-(trifluoromethyl)-2-pyridinyl)methyl)-1,3-dihydrofuro[3,4-c]quinoline-8-carboxamide;
(3S)-4-amino-N-((3R,4S)-3-methoxytetrahydro-2H-pyran-4-yl)-3-methyl-N-((5-(trifluoromethyl)-2-pyridinyl)methyl)-1,3-dihydrofuro[3,4-c]quinoline-8-carboxamide;
(3R)-4-amino-N-((1R,2R)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3R)-4-amino-N-((1R,2S)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3R)-4-amino-N-((1S,2R)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3R)-4-amino-N-((1S,2S)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3S)-4-amino-N-((1R,2R)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3S)-4-amino-N-((1R,2S)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3S)-4-amino-N-((1S,2R)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3S)-4-amino-N-((1S,2S)-[1,1'-bi(cyclopropyl)]-2-yl)-3-methyl-N-((5-(trifluoromethyl)-2-pyridinyl)methyl)-1,3-dihydrofuro[3,4-c]quinoline-8-carboxamide;
(3R)-4-amino-3-methyl-N-((1R)-spiro[2.5]octan-1-yl)-N-((5-(trifluoromethyl)-2-pyridinyl)methyl)-1,3-dihydrofuro[3,4-c]quinoline-8-carboxamide;
(3R)-4-amino-3-methyl-N-((1S)-spiro[2.5]octan-1-yl)-N-((5-(trifluoromethyl)-2-pyridinyl)methyl)-1,3-dihydrofuro[3,4-c]quinoline-8-carboxamide;
(3S)-4-amino-3-methyl-N-((1R)-spiro[2.5]octan-1-yl)-N-((5-(trifluoromethyl)-2-pyridinyl)methyl)-1,3-dihydrofuro[3,4-c]quinoline-8-carboxamide;
(3S)-4-amino-3-methyl-N-((1S)-spiro[2.5]octan-1-yl)-N-((5-(trifluoromethyl)-2-pyridinyl)methyl)-1,3-dihydrofuro[3,4-c]quinoline-8-carboxamide;
(3R)-4-amino-N-((1R,2R)-2-aminocyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1R,2S)-2-aminocyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1S,2R)-2-aminocyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1S,2S)-2-aminocyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1R,2R)-2-aminocyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1R,2S)-2-aminocyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1S,2R)-2-aminocyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1S,2S)-2-aminocyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1R,2R)-2-ethoxycyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1R,2S)-2-ethoxycyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1S,2R)-2-ethoxycyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1S,2S)-2-ethoxycyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1R,2R)-2-ethoxycyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1R,2S)-2-ethoxycyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1S,2R)-2-ethoxycyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1S,2S)-2-ethoxycyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1R)-2,2-dimethylcyclopropyl)-3-methyl-N-((5-(trifluoromethyl)-2-pyridinyl)methyl)-1,3-dihydrofuro[3,4-c]quinoline-8-carboxamide;
(3R)-4-amino-N-((1S)-2,2-dimethylcyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1R)-2,2-dimethylcyclopropyl)-3-methyl-N-((5-(trifluoromethyl)-2-pyridinyl)methyl)-1,3-dihydrofuro[3,4-c]quinoline-8-carboxamide;
(3S)-4-amino-N-((1S)-2,2-dimethylcyclopropyl)-3-methyl-N-((5-(trifluoromethyl)-2-pyridinyl)methyl)-1,3-dihydrofuro[3,4-c]quinoline-8-carboxamide;
(3R)-4-amino-3-methyl-N-((1R)-spiro[2.4]heptan-1-yl)-N-((5-(trifluoromethyl)-2-pyridinyl)methyl)-1,3-dihydrofuro[3,4-c]quinoline-8-carboxamide;
(3R)-4-amino-3-methyl-N-((1S)-spiro[2.4]heptan-1-yl)-N-((5-(trifluoromethyl)-2-pyridinyl)methyl)-1,3-dihydrofuro[3,4-c]quinoline-8-carboxamide;
(3S)-4-amino-3-methyl-N-((1R)-spiro[2.4]heptan-1-yl)-N-((5-(trifluoromethyl)-2-pyridinyl)methyl)-1,3-dihydrofuro[3,4-c]quinoline-8-carboxamide;
(3S)-4-amino-3-methyl-N-((1S)-spiro[2.4]heptan-1-yl)-N-((5-(trifluoromethyl)-2-pyridinyl)methyl)-1,3-dihydrofuro[3,4-c]quinoline-8-carboxamide;
(3R)-4-amino-3-methyl-N-((1R,2R)-2-(trifluoromethyl)cyclopropyl)-N-((5-(trifluoromethyl)-2-pyridinyl)methyl)-1,3-dihydrofuro[3,4-c]quinoline-8-carboxamide;
(3R)-4-amino-3-methyl-N-((1S,2S)-2-(trifluoromethyl)cyclopropyl)-N-((5-(trifluoromethyl)-2-pyridinyl)methyl)-1,3-dihydrofuro[3,4-c]quinoline-8-carboxamide;
(3S)-4-amino-3-methyl-N-((1R,2R)-2-(trifluoromethyl)cyclopropyl)-N-((5-(trifluoromethyl)-2-pyridinyl)methyl)-1,3-dihydrofuro[3,4-c]quinoline-8-carboxamide;
(3S)-4-amino-3-methyl-N-((1S,2S)-2-(trifluoromethyl)cyclopropyl)-N-((5-(trifluoromethyl)-2-pyridinyl)methyl)-1,3-dihydrofuro[3,4-c]quinoline-8-carboxamide;
(3R)-4-amino-3-methyl-N-((1R)-1-(2-pyrimidinyl)ethyl)-N-((5-(trifluoromethyl)-2-pyridinyl)methyl)-1,3-dihydrofuro[3,4-c]quinoline-8-carboxamide;
(3S)-4-amino-3-methyl-N-((1R)-1-(2-pyrimidinyl)ethyl)-N-((5-(trifluoromethyl)-2-pyridinyl)methyl)-1,3-dihydrofuro[3,4-c]quinoline-8-carboxamide;
(3R)-4-amino-N-((5-cyano-2-pyridinyl)methyl)-N-(5,6-dihydro-2H-pyran-3-ylmethyl)-3-methyl-1,3-dihydrofuro[3,4-c]quinoline-8-carboxamide;
(3S)-4-amino-N-((5-cyano-2-pyridinyl)methyl)-N-(5,6-dihydro-2H-pyran-3-ylmethyl)-3-methyl-1,3-dihydrofuro[3,4-c]quinoline-8-carboxamide;
(3R)-4-amino-N-((1-cyanocyclopropyl)methyl)-N-((5-cyano-2-pyridinyl)methyl)-3-methyl-1,3-dihydrofuro[3,4-c]quinoline-8-carboxamide;
(3S)-4-amino-N-((1-cyanocyclopropyl)methyl)-N-((5-cyano-2-pyridinyl)methyl)-3-methyl-1,3-dihydrofuro[3,4-c]quinoline-8-carboxamide;
(3R)-4-amino-N-((2-amino-1,3-thiazol-5-yl)methyl)-N-((5-cyano-2-pyridinyl)methyl)-3-methyl-1,3-dihydrofuro[3,4-c]quinoline-8-carboxamide;
(3S)-4-amino-N-((2-amino-1,3-thiazol-5-yl)methyl)-N-((5-cyano-2-pyridinyl)methyl)-3-methyl-1,3-dihydrofuro[3,4-c]quinoline-8-carboxamide;
(3R)-4-amino-N-(5,6-dihydro-2H-pyran-3-ylmethyl)-3-methyl-N-((5-(trifluoromethyl)-2-pyridinyl)methyl)-1,3-dihydrofuro[3,4-c]quinoline-8-carboxamide;
(3S)-4-amino-N-(5,6-dihydro-2H-pyran-3-ylmethyl)-3-methyl-N-((5-(trifluoromethyl)-2-pyridinyl)methyl)-1,3-dihydrofuro[3,4-c]quinoline-8-carboxamide;
(3R)-4-amino-3-methyl-N-(1H-pyrrolo[2,3-b]pyridin-4-ylmethyl)-N-((5-(trifluoromethyl)-2-pyridinyl)methyl)-1,3-dihydrofuro[3,4-c]quinoline-8-carboxamide;
(3S)-4-amino-3-methyl-N-(1H-pyrrolo[2,3-b]pyridin-4-ylmethyl)-N-((5-(trifluoromethyl)-2-pyridinyl)methyl)-1,3-dihydrofuro[3,4-c]quinoline-8-carboxamide;
(3R)-4-amino-N-((1-cyanocyclopropyl)methyl)-3-methyl-N-((5-(trifluoromethyl)-2-pyridinyl)methyl)-1,3-dihydrofuro[3,4-c]quinoline-8-carboxamide;
(3S)-4-amino-N-((1-cyanocyclopropyl)methyl)-3-methyl-N-((5-(trifluoromethyl)-2-pyridinyl)methyl)-1,3-dihydrofuro[3,4-c]quinoline-8-carboxamide;
(3R)-4-amino-N-((2-amino-1,3-thiazol-5-yl)methyl)-3-methyl-N-((5-(trifluoromethyl)-2-pyridinyl)methyl)-1,3-dihydrofuro[3,4-c]quinoline-8-carboxamide;
(3S)-4-amino-N-((2-amino-1,3-thiazol-5-yl)methyl)-3-methyl-N-((5-(trifluoromethyl)-2-pyridinyl)methyl)-1,3-dihydrofuro[3,4-c]quinoline-8-carboxamide;
(3R)-4-amino-3-methyl-N-(2-methylpropyl)-N-((5-(trifluoromethyl)-2-pyridinyl)methyl)-1,3-dihydrofuro[3,4-c]quinoline-8-carboxamide;
(3S)-4-amino-3-methyl-N-(2-methylpropyl)-N-((5-(trifluoromethyl)-2-pyridinyl)methyl)-1,3-dihydrofuro[3,4-c]quinoline-8-carboxamide;
(3R)-4-amino-N-((2R,3R)-3-hydroxy-2-butanyl)-3-methyl-N-((5-(trifluoromethyl)-2-pyridinyl)methyl)-1,3-dihydrofuro[3,4-c]quinoline-8-carboxamide;
(3R)-4-amino-N-((2R,3S)-3-hydroxy-2-butanyl)-3-methyl-N-((5-(trifluoromethyl)-2-pyridinyl)methyl)-1,3-dihydrofuro[3,4-c]quinoline-8-carboxamide;
(3R)-4-amino-N-((2S,3R)-3-hydroxy-2-butanyl)-3-methyl-N-((5-(trifluoromethyl)-2-pyridinyl)methyl)-1,3-dihydrofuro[3,4-c]quinoline-8-carboxamide;
(3R)-4-amino-N-((2S,3S)-3-hydroxy-2-butanyl)-3-methyl-N-((5-(trifluoromethyl)-2-pyridinyl)methyl)-1,3-dihydrofuro[3,4-c]quinoline-8-carboxamide;
(3S)-4-amino-N-((2R,3R)-3-hydroxy-2-butanyl)-3-methyl-N-((5-(trifluoromethyl)-2-pyridinyl)methyl)-1,3-dihydrofuro[3,4-c]quinoline-8-carboxamide;
(3S)-4-amino-N-((2R,3S)-3-hydroxy-2-butanyl)-3-methyl-N-((5-(trifluoromethyl)-2-pyridinyl)methyl)-1,3-dihydrofuro[3,4-c]quinoline-8-carboxamide;
(3S)-4-amino-N-((2S,3R)-3-hydroxy-2-butanyl)-3-methyl-N-((5-(trifluoromethyl)-2-pyridinyl)methyl)-1,3-dihydrofuro[3,4-c]quinoline-8-carboxamide;
(3S)-4-amino-N-((2S,3S)-3-hydroxy-2-butanyl)-3-methyl-N-((5-(trifluoromethyl)-2-pyridinyl)methyl)-1,3-dihydrofuro[3,4-c]quinoline-8-carboxamide;
(3R)-4-amino-N-((1R,3R)-3-hydroxycyclohexyl)-3-methyl-N-((5-(trifluoromethyl)-2-pyridinyl)methyl)-1,3-dihydrofuro[3,4-c]quinoline-8-carboxamide;
(3R)-4-amino-N-((1R,3S)-3-hydroxycyclohexyl)-3-methyl-N-((5-(trifluoromethyl)-2-pyridinyl)methyl)-1,3-dihydrofuro[3,4-c]quinoline-8-carboxamide;
(3R)-4-amino-N-((1S,3R)-3-hydroxycyclohexyl)-3-methyl-N-((5-(trifluoromethyl)-2-pyridinyl)methyl)-1,3-dihydrofuro[3,4-c]quinoline-8-carboxamide;
(3R)-4-amino-N-((1S,3S)-3-hydroxycyclohexyl)-3-methyl-N-((5-(trifluoromethyl)-2-pyridinyl)methyl)-1,3-dihydrofuro[3,4-c]quinoline-8-carboxamide;
(3S)-4-amino-N-((1R,3R)-3-hydroxycyclohexyl)-3-methyl-N-((5-(trifluoromethyl)-2-pyridinyl)methyl)-1,3-dihydrofuro[3,4-c]quinoline-8-carboxamide;
(3S)-4-amino-N-((1R,3S)-3-hydroxycyclohexyl)-3-methyl-N-((5-(trifluoromethyl)-2-pyridinyl)methyl)-1,3-dihydrofuro[3,4-c]quinoline-8-carboxamide;
(3S)-4-amino-N-((1S,3R)-3-hydroxycyclohexyl)-3-methyl-N-((5-(trifluoromethyl)-2-pyridinyl)methyl)-1,3-dihydrofuro[3,4-c]quinoline-8-carboxamide;
(3S)-4-amino-N-((1S,3S)-3-hydroxycyclohexyl)-3-methyl-N-((5-(trifluoromethyl)-2-pyridinyl)methyl)-1,3-dihydrofuro[3,4-c]quinoline-8-carboxamide;
(3R)-4-amino-N-(2-(cis-3-hydroxycyclobutyl)ethyl)-3-methyl-N-((5-(trifluoromethyl)-2-pyridinyl)methyl)-1,3-dihydrofuro[3,4-c]quinoline-8-carboxamide;
(3R)-4-amino-N-(2-(trans-3-hydroxycyclobutyl)ethyl)-3-methyl-N-((5-(trifluoromethyl)-2-pyridinyl)methyl)-1,3-dihydrofuro[3,4-c]quinoline-8-carboxamide;
(3S)-4-amino-N-(2-(cis-3-hydroxycyclobutyl)ethyl)-3-methyl-N-((5-(trifluoromethyl)-2-pyridinyl)methyl)-1,3-dihydrofuro[3,4-c]quinoline-8-carboxamide;
(3S)-4-amino-N-(2-(trans-3-hydroxycyclobutyl)ethyl)-3-methyl-N-((5-(trifluoromethyl)-2-pyridinyl)methyl)-1,3-dihydrofuro[3,4-c]quinoline-8-carboxamide;
(3R)-4-amino-N-((2R)-2-hydroxypropyl)-3-methyl-N-((5-(trifluoromethyl)-2-pyridinyl)methyl)-1,3-dihydrofuro[3,4-c]quinoline-8-carboxamide;
(3R)-4-amino-N-((2S)-2-hydroxypropyl)-3-methyl-N-((5-(trifluoromethyl)-2-pyridinyl)methyl)-1,3-dihydrofuro[3,4-c]quinoline-8-carboxamide;
(3S)-4-amino-N-((2R)-2-hydroxypropyl)-3-methyl-N-((5-(trifluoromethyl)-2-pyridinyl)methyl)-1,3-dihydrofuro[3,4-c]quinoline-8-carboxamide;
(3S)-4-amino-N-((2S)-2-hydroxypropyl)-3-methyl-N-((5-(trifluoromethyl)-2-pyridinyl)methyl)-1,3-dihydrofuro[3,4-c]quinoline-8-carboxamide;
(3R)-4-amino-N-((2R)-3-hydroxy-2-methylpropyl)-3-methyl-N-((5-(trifluoromethyl)-2-pyridinyl)methyl)-1,3-dihydrofuro[3,4-c]quinoline-8-carboxamide;
(3R)-4-amino-N-((2S)-3-hydroxy-2-methylpropyl)-3-methyl-N-((5-(trifluoromethyl)-2-pyridinyl)methyl)-1,3-dihydrofuro[3,4-c]quinoline-8-carboxamide;
(3S)-4-amino-N-((2R)-3-hydroxy-2-methylpropyl)-3-methyl-N-((5-(trifluoromethyl)-2-pyridinyl)methyl)-1,3-dihydrofuro[3,4-c]quinoline-8-carboxamide;
(3S)-4-amino-N-((2S)-3-hydroxy-2-methylpropyl)-3-methyl-N-((5-(trifluoromethyl)-2-pyridinyl)methyl)-1,3-dihydrofuro[3,4-c]quinoline-8-carboxamide;
(3R)-4-amino-3-methyl-N-((2R)-3,3,3-trifluoro-2-hydroxypropyl)-N-((5-(trifluoromethyl)-2-pyridinyl)methyl)-1,3-dihydrofuro[3,4-c]quinoline-8-carboxamide;
(3R)-4-amino-3-methyl-N-((2S)-3,3,3-trifluoro-2-hydroxypropyl)-N-((5-(trifluoromethyl)-2-pyridinyl)methyl)-1,3-dihydrofuro[3,4-c]quinoline-8-carboxamide;
(3S)-4-amino-3-methyl-N-((2R)-3,3,3-trifluoro-2-hydroxypropyl)-N-((5-(trifluoromethyl)-2-pyridinyl)methyl)-1,3-dihydrofuro[3,4-c]quinoline-8-carboxamide;
(3S)-4-amino-3-methyl-N-((2S)-3,3,3-trifluoro-2-hydroxypropyl)-N-((5-(trifluoromethyl)-2-pyridinyl)methyl)-1,3-dihydrofuro[3,4-c]quinoline-8-carboxamide;
(3R)-4-amino-3-methyl-N-((1-methyl-1H-1,2,4-triazol-3-yl)methyl)-N-((5-(trifluoromethyl)-2-pyridinyl)methyl)-1,3-dihydrofuro[3,4-c]quinoline-8-carboxamide;
(3S)-4-amino-3-methyl-N-((1-methyl-1H-1,2,4-triazol-3-yl)methyl)-N-((5-(trifluoromethyl)-2-pyridinyl)methyl)-1,3-dihydrofuro[3,4-c]quinoline-8-carboxamide;
(3R)-4-amino-3-methyl-N-((3R)-tetrahydro-3-furanylmethyl)-N-((5-(trifluoromethyl)-2-pyridinyl)methyl)-1,3-dihydrofuro[3,4-c]quinoline-8-carboxamide;
(3R)-4-amino-3-methyl-N-((3S)-tetrahydro-3-furanylmethyl)-N-((5-(trifluoromethyl)-2-pyridinyl)methyl)-1,3-dihydrofuro[3,4-c]quinoline-8-carboxamide;
(3S)-4-amino-3-methyl-N-((3R)-tetrahydro-3-furanylmethyl)-N-((5-(trifluoromethyl)-2-pyridinyl)methyl)-1,3-dihydrofuro[3,4-c]quinoline-8-carboxamide;
(3S)-4-amino-3-methyl-N-((3S)-tetrahydro-3-furanylmethyl)-N-((5-(trifluoromethyl)-2-pyridinyl)methyl)-1,3-dihydrofuro[3,4-c]quinoline-8-carboxamide;
(3R)-4-amino-N-((2R)-2-cyanopropyl)-3-methyl-N-((5-(trifluoromethyl)-2-pyridinyl)methyl)-1,3-dihydrofuro[3,4-c]quinoline-8-carboxamide;
(3R)-4-amino-N-((2S)-2-cyanopropyl)-3-methyl-N-((5-(trifluoromethyl)-2-pyridinyl)methyl)-1,3-dihydrofuro[3,4-c]quinoline-8-carboxamide;
(3S)-4-amino-N-((2R)-2-cyanopropyl)-3-methyl-N-((5-(trifluoromethyl)-2-pyridinyl)methyl)-1,3-dihydrofuro[3,4-c]quinoline-8-carboxamide;
(3S)-4-amino-N-((2S)-2-cyanopropyl)-3-methyl-N-((5-(trifluoromethyl)-2-pyridinyl)methyl)-1,3-dihydrofuro[3,4-c]quinoline-8-carboxamide;
(3R)-4-amino-N,3-dimethyl-N-((5-(trifluoromethyl)-2-pyridinyl)methyl)-1,3-dihydrofuro[3,4-c]quinoline-8-carboxamide;
(3S)-4-amino-N,3-dimethyl-N-((5-(trifluoromethyl)-2-pyridinyl)methyl)-1,3-dihydrofuro[3,4-c]quinoline-8-carboxamide;
(3R)-4-amino-N-ethyl-3-methyl-N-((5-(trifluoromethyl)-2-pyridinyl)methyl)-1,3-dihydrofuro[3,4-c]quinoline-8-carboxamide;
(3S)-4-amino-N-ethyl-3-methyl-N-((5-(trifluoromethyl)-2-pyridinyl)methyl)-1,3-dihydrofuro[3,4-c]quinoline-8-carboxamide;
(3R)-4-amino-3-methyl-N-(2-propanyl)-N-((5-(trifluoromethyl)-2-pyridinyl)methyl)-1,3-dihydrofuro[3,4-c]quinoline-8-carboxamide;
(3S)-4-amino-3-methyl-N-(2-propanyl)-N-((5-(trifluoromethyl)-2-pyridinyl)methyl)-1,3-dihydrofuro[3,4-c]quinoline-8-carboxamide;
4-amino-N,3,3-trimethyl-N-((5-(trifluoromethyl)-2-pyridinyl)methyl)-1,3-dihydrofuro[3,4-c]quinoline-8-carboxamide;
4-amino-N-ethyl-3,3-dimethyl-N-((5-(trifluoromethyl)-2-pyridinyl)methyl)-1,3-dihydrofuro[3,4-c]quinoline-8-carboxamide;
4-amino-3,3-dimethyl-N-(2-propanyl)-N-((5-(trifluoromethyl)-2-pyridinyl)methyl)-1,3-dihydrofuro[3,4-c]quinoline-8-carboxamide;
4-amino-N-cyclopropyl-3,3-dimethyl-N-((5-(trifluoromethyl)-2-pyridinyl)methyl)-1,3-dihydrofuro[3,4-c]quinoline-8-carboxamide; and
4-amino-N-((1R)-1-(3-cyano-5-(trifluoromethyl)-2-pyridinyl)ethyl)-7-fluoro-N,1-dimethyl-1H-pyrazolo[4,3-c]quinoline-8-carboxamide.

15. The compound of claim 14, the tautomer thereof, the stereoisomer thereof, or the pharmaceutically acceptable salt of any of the foregoing, wherein the compound is selected from:
(3R)-4-amino-N-ethyl-7-fluoro-3-methyl-N-((1R)-1-(6-(trifluoromethyl)-3-pyridazinyl)ethyl)-1,3-dihydrofuro[3,4-c]quinoline-8-carboxamide;
4-amino-N,1-dimethyl-N-((5-(trifluoromethyl)-2-pyridinyl)methyl)-1H-pyrazolo[4,3-c]quinoline-8-carboxamide;
4-amino-7-fluoro-N-((1R)-1-(3-fluoro-5-(trifluoromethyl)-2-pyridinyl)ethyl)-N,1-dimethyl-1H-pyrazolo[4,3-c]quinoline-8-carboxamide;
4-amino-N,1-dimethyl-N-(4-(pentafluoro-lambda~6~-sulfanyl)benzyl)-1H-pyrazolo[4,3-c][1,7]naphthyridine-8-carboxamide;
4-amino-7-fluoro-N,1-dimethyl-N-((1R)-1-(6-(trifluoromethyl)-3-pyridazinyl)ethyl)-1H-pyrazolo[4,3-c]quinoline-8-carboxamide;
(3R)-4-amino-7-fluoro-N,3-dimethyl-N-((1R)-1-(6-(trifluoromethyl)-3-pyridazinyl)ethyl)-1,3-dihydrofuro[3,4-c]quinoline-8-carboxamide;
4-amino-7-fluoro-N-methyl-N-((1R)-1-(6-(trifluoromethyl)-3-pyridazinyl)ethyl)-1,3-dihydrofuro[3,4-c]quinoline-8-carboxamide;
5-amino-N-(2-propanyl)-N-((5-(trifluoromethyl)-2-pyridinyl)methyl)pyrido[4,3-c][1,7]naphthyridine-9-carboxamide;
4-amino-N-ethyl-7-fluoro-1-methyl-N-((5-(trifluoromethyl)-2-pyridinyl)methyl)-1H-pyrazolo[4,3-c]quinoline-8-carboxamide;
4-amino-7-chloro-N-((1R)-1-(5-fluoro-2-pyridinyl)ethyl)-N, 1-dimethyl-1H-pyrazolo[4,3-c]quinoline-8-carboxamide;
4-amino-N-cyclobutyl-N-((6-(trifluoromethyl)-3-pyridazinyl)methyl)-1,3-dihydrofuro[3,4-c]quinoline-8-carboxamide;
4-amino-N-methyl-N-((5-(trifluoromethyl)-2-pyridinyl)methyl)-1,3-dihydrofuro[3,4-c]quinoline-8-carboxamide;
4-amino-N-cyclopropyl-1-methyl-N-((5-(trifluoromethyl)-2-pyridinyl)methyl)-1H-pyrazolo[4,3-c]quinoline-8-carboxamide;
4-amino-N-ethyl-7-fluoro-1-methyl-N-((6-(trifluoromethyl)-3-pyridazinyl)methyl)-1H-pyrazolo[4,3-c]quinoline-8-carboxamide;
4-amino-N-cyclopropyl-7-fluoro-1-methyl-N-((6-(trifluoromethyl)-3-pyridazinyl)methyl)-1H-pyrazolo[4,3-c]quinoline-8-carboxamide;
4-amino-7-fluoro-N,1-dimethyl-N-((1R)-1-(5-(trifluoromethyl)-2-pyridinyl)ethyl)-1H-pyrazolo[4,3-c]quinoline-8-carboxamide;
(3R)-4-amino-N-ethyl-3-methyl-N-((1R)-1-(6-(trifluoromethyl)-3-pyridazinyl)ethyl)-1,3-dihydrofuro[3,4-c]quinoline-8-carboxamide;
4-amino-N-cyclopropyl-7-fluoro-1-methyl-N-((5-(trifluoromethyl)-2-pyridinyl)methyl)-1H-pyrazolo[4,3-c]quinoline-8-carboxamide;
4-amino-7-fluoro-N,1-dimethyl-N-((5-(trifluoromethyl)-2-pyridinyl)methyl)-1H-pyrazolo[4,3-c]quinoline-8-carboxamide and
4-amino-N-ethyl-1-methyl-N-((5-(trifluoromethyl)-2-pyridinyl)methyl)-1H-pyrazolo[4,3-c]quinoline-8-carboxamide.

16. A pharmaceutical composition, comprising the compound of any of claims 1, 14 or 15, the tautomer thereof, the stereoisomer thereof, or the pharmaceutically acceptable salt of any of the foregoing or a pharmaceutically acceptable salt thereof, and at least one pharmaceutically acceptable excipient.

17. The compound of any of claims 1, 14 or 15, the tautomer thereof, the stereoisomer thereof, or the pharmaceutically acceptable salt of any of the foregoing for use in treating cancer, wherein the cancer is preferably selected from ovarian, lung, lymphoid, glioblastoma, colon, melanoma, gastric, pancreatic or bladder cancer.

## Patentansprüche

1. Eine Verbindung der Formel I 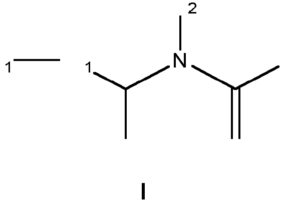 ein Tautomeres davon, ein Stereoisomeres davon oder ein pharmazeutisch verträgliches Salz einer der vorgenannten Verbindungen, wobei:
R ein Trizyklus ist, unabhängig ausgewählt aus den Formeln IA und IB: 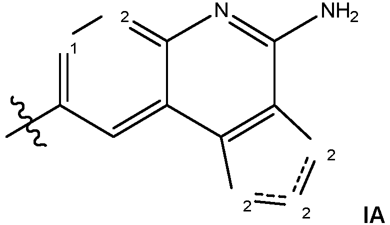 und 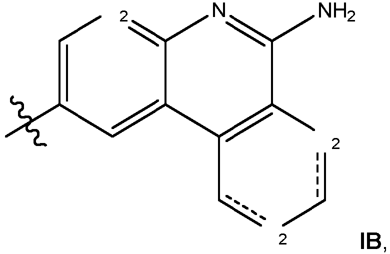
wobei 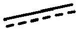 eine Einfach- oder Doppelbindung ist,
X¹, X², X⁶ und X⁷ in jedem Fall N oder C sind, wobei X¹ und X² nicht gleichzeitig N sein können und wobei, wenn X¹ gleich C ist, es gegebenenfalls mit Halogen substituiert sein kann;
X³, X⁴ und X⁵ jeweils unabhängig voneinander ausgewählt sind aus einem gegebenenfalls substituierten C, O, N und S; wobei die Substituenten unabhängig voneinander ausgewählt sind aus C₁₋₃-Alkyl, C₁₋₃-Alkyl(OH), wobei Alkyl gegebenenfalls mit Halogen substituiert sein kann;
R³ in jedem Fall unabhängig ausgewählt ist aus H oder C₁₋₃-Alkyl;
Ar¹ ein sechsgliedriges, gegebenenfalls substituiertes Aryl oder Heteroaryl ist, unabhängig ausgewählt aus: 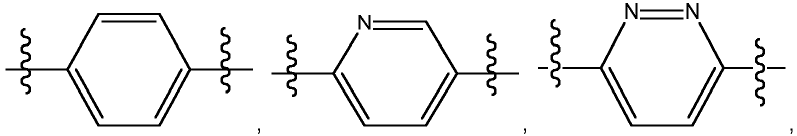 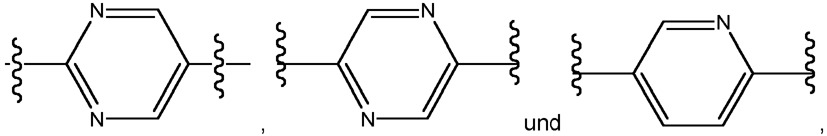
wobei die Substituenten unabhängig voneinander ausgewählt sind aus C₁₋₃-Alkyl, -OC₁₋₃-Alkyl oder Halogen;
R¹ in jedem Fall unabhängig ausgewählt ist aus H, Halogen, gegebenenfalls substituiertem C₁₋₃-Alkyl, wobei die Substituenten ausgewählt sind aus Halogen; -CN, gegebenenfalls substituiertem -O-C₁₋₃-Alkyl, wobei die Substituenten ausgewählt sind aus Halogen; -C(O)OC₁₋₃-Alkyl, wobei C₁₋₃-Alkyl gegebenenfalls mit Halogen substituiert sein kann, und Morpholinyl; und
R² in jedem Fall unabhängig ausgewählt ist aus einem gegebenenfalls substituierten C₁₋₈-Alkyl, wobei die Substituenten ausgewählt sind aus Halogen, Hydroxy, Amino, -O-C₁₋₃-Alkyl oder -CN; 5- oder 6-gliedrigem Zyklus oder Heterozyklus, gegebenenfalls substituiert mit Hydroxy, Amino, einem gegebenenfalls substituierten C₁₋₆-Alkyl, wobei die Substituenten ausgewählt sind aus Halogen; einem gegebenenfalls substituierten C₁₋₆-Alkyl-O- C₁₋₃-Alkyl, wobei die Substituenten ausgewählt sind aus Halogen; 5,6,7,8-Tetrahydro-[1,2,4]triazolo[1,5-a]pyridinyl; C₁₋₃-Alkyl-Heterocyclyl, wobei das Heterocyclyl ausgewählt ist aus gegebenenfalls substituiertem 3,4-Dihydro-2H-pyrano[2,3-c]pyridinyl; Pyradazinyl, Triazolyl, Pyrimidinyl, Tetrahydrofuranyl, 1H-Pyrrolo[2,3-b]pyridinyl, Cyclohexyl; wobei die Substituenten ausgewählt sind aus C₁₋₃-Alkyl, -CN und Halogen oder einem gegebenenfalls substituierten C₁₋₆-Alkyl-O- C₁₋₃-Alkyl, wobei die Substituenten ausgewählt sind aus Halogen; gegebenenfalls substituiertem Phenyl, wobei die Substituenten ausgewählt sind aus Halogen oder C₁₋₃-Alkyl.

2. Die Verbindung nach Anspruch 1, das Tautomer davon, das Stereoisomer davon oder das pharmazeutisch verträgliche Salz einer der vorgenannten Verbindungen, wobei R¹ ein Trizyklus der Formel **IA** ist.

3. Die Verbindung nach Anspruch 1, das Tautomer davon, das Stereoisomer davon oder das pharmazeutisch verträgliche Salz einer der vorgenannten, worin R¹ ein Trizyklus der Formel **IB** ist.

4. Die Verbindung nach Anspruch 1, das Tautomer davon, das Stereoisomer davon oder das pharmazeutisch verträgliche Salz einer der vorstehenden Verbindungen, worin R ein Trizyklus der Formel IA ist und X¹und X² beide C sind.

5. Die Verbindung nach Anspruch 4, das Tautomer davon, das Stereoisomer davon oder das pharmazeutisch verträgliche Salz einer der vorgenannten Verbindungen, wobei X¹ unsubstituiert oder mit Halogen substituiert ist.

6. Die Verbindung nach Anspruch 1, das Tautomer davon, das Stereoisomer davon oder das pharmazeutisch verträgliche Salz einer der vorstehenden Verbindungen, worin R ein Trizyklus der Formel IB ist und X² gleich C oder N ist.

7. Die Verbindung nach Anspruch 4, das Tautomer davon, das Stereoisomer davon oder das pharmazeutisch verträgliche Salz einer der vorgenannten, worin R ein Trizyklus der Formel IA1 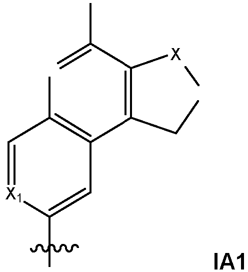 oder R ist ein Trizyklus der Formel IA2 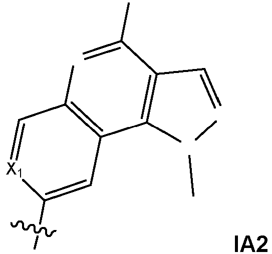 ist.

8. Die Verbindung nach Anspruch 7, das Tautomer davon, das Stereoisomer davon oder das pharmazeutisch verträgliche Salz einer der vorgenannten, worin X³C, unsubstituiert oder mit Methyl substituiert, ist.

9. Die Verbindung nach Anspruch 1, das Tautomer davon, das Stereoisomer davon oder das pharmazeutisch verträgliche Salz einer der vorstehenden Verbindungen, worin R³ H gleich oder Methyl ist.

10. Die Verbindung nach Anspruch 7, das Tautomer davon, das Stereoisomer davon oder das pharmazeutisch verträgliche Salz einer der vorstehenden Verbindungen, wobei R² ein gegebenenfalls substituiertes C₁₋₈-Alkyl, vorzugsweise ein gegebenenfalls substituiertes Methyl, Ethyl, Isopropyl oder CycloC₁₋₆-Alkyl ist.

11. Die Verbindung nach Anspruch 10, das Tautomer davon, das Stereoisomer davon oder das pharmazeutisch verträgliche Salz einer der vorgenannten Verbindungen, wobei Ar¹ 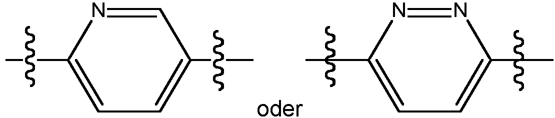 ist.

12. Die Verbindung nach Anspruch 11, worin Ar¹ 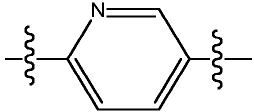 ist, das Tautomer davon, das Stereoisomer davon oder das pharmazeutisch verträgliche Salz einer der vorgenannten Verbindungen, wobei R¹ Halogen oder gegebenenfalls substituiertes C₁₋₃-Alkyl oder -CN ist.

13. Die Verbindung nach Anspruch 1, das Tautomer davon, das Stereoisomer davon oder das pharmazeutisch verträgliche Salz einer der vorstehenden Verbindungen, wobei R ein Trizyklus der Formel IB ist und R¹ Halogen, gegebenenfalls substituiertes C₁₋₃-Alkyl oder -CN ist.

14. Eine Verbindung, das Tautomer davon, das Stereoisomer davon oder das pharmazeutisch verträgliche Salz einer der vorgenannten Verbindungen, wobei die Verbindung ausgewählt ist aus:
4-Amino-N-(cyclopropylmethyl)-N-((5-(trifluormethyl)-2-pyridinyl)methyl)-1,3-dihydrofuro[3,4-c]chinolin-8-carboxamid;
4-Amino-N-((5-Cyano-2-pyridinyl)-methyl)-N-(2-propanyl)-1,3-dihydrofuro[3,4-c]chinolin-8-carboxamid;
4-Amino-N-((6-cyclopropyl-3-pyridinyl)methyl)-N-(2-propanyl)-1,3-dihydrofuro[3,4-c]chinolin-8-carboxamid;
4-Amino-N-((2R)-1-methoxy-2-propanyl)-N-((6-(trifluormethyl)-3-pyridazinyl)methyl)-1,3-dihydrofuro[3,4-c]chinolin-8-carboxamid;
4-Amino-N-((2S)-1-methoxy-2-propanyl)-N-((6-(trifluormethyl)-3-pyridazinyl)methyl)-1,3-dihydrofuro[3,4-c]chinolin-8-carboxamid;
(3R)-4-Amino-N-((2R)-1-methoxy-2-propanyl)-3-methyl-N-((6-(trifluormethyl)-3-pyridazinyl)methyl)-1,3-dihydrofuro[3,4-c]chinolin-8-carboxamid;
(3R)-4-Amino-N-((2S)-1-methoxy-2-propanyl)-3-methyl-N-((6-(trifluormethyl)-3-pyridazinyl)methyl)-1,3-dihydrofuro[3,4-c]chinolin-8-carboxamid;
4-Amino-N-((6-chloro-3-pyridazinyl)methyl)-N-((2R)-1-methoxy-2-propanyl)-1,3-dihydrofuro[3,4-c]chinolin-8-carboxamid;
4-Amino-N-((6-chloro-3-pyridazinyl)methyl)-N-((2R)-1-methoxy-2-propanyl)-1,3-dihydrofuro[3,4-c][1,7]naphthyridin-8-carboxamid;
(3R)-4-Amino-N-((6-chloro-3-pyridazinyl)methyl)-N-((2R)-1-methoxy-2-propanyl)-3-methyl-1,3-dihydrofuro[3,4-c]chinolin-8-carboxamid;
(3R)-4-Amino-N-((6-chloro-3-pyridazinyl)methyl)-N-((2R)-1-methoxy-2-propanyl)-3-methyl-1,3-dihydrofuro[3,4-c][1,7]naphthyridin-8-carboxamid;
(3S)-4-Amino-N-(bicyclo[1.1.1]pentan-1-yl)-3-methyl-N-((5-(trifluormethyl)-2-pyridinyl)methyl)-1,3-dihydrofuro[3,4-c][1,7]naphthyridin-8-carboxamid;
(3R)-4-Amino-3-methyl-N-(2-propanyl)-N-((5-(trifluormethyl)-2-pyridinyl)methyl)-1,3-dihydrofuro[3,4-c][1,7]naphthyridin-8-carboxamid;
(3R)-4-Amino-N-(bicyclo[1.1.1]pentan-1-yl)-3-methyl-N-((5-(trifluormethyl)-2-pyridinyl)methyl)-1,3-dihydrofuro[3,4-c][1,7]naphthyridin-8-carboxamid;
(3R)-4-Amino-N-((5-cyano-2-pyridinyl)methyl)-N,3-dimethyl-1,3-dihydrofuro[3,4-c][1,7]naphthyridin-8-carboxamid;
(3R)-4-Amino-3-methyl-N-(2-propanyl)-N-((6-(trifluormethyl)-3-pyridazinyl)methyl)-1,3-dihydrofuro[3,4-c][1,7]naphthyridin-8-carboxamid;
(3R)-4-Amino-N-((2R)-1-methoxy-2-propanyl)-3-methyl-N-((6-(trifluormethyl)-3-pyridazinyl)methyl)-1,3-dihydrofuro[3,4-c][1,7]naphthyridin-8-carboxamid;
(3R)-4-Amino-N-((2S)-1-methoxy-2-propanyl)-3-methyl-N-((6-(trifluormethyl)-3-pyridazinyl)methyl)-1,3-dihydrofuro[3,4-c][1,7]naphthyridin-8-carboxamid;
(3R)-4-Amino-N-cyclopropyl-3-methyl-N-((5-(trifluormethyl)-2-pyridinyl)methyl)-1,3-dihydrofuro[3,4-c][1,7]naphthyridin-8-carboxamid;
(3R)-4-Amino-N-((5-cyano-2-pyridinyl)methyl)-N-cyclopropyl-3-methyl-1,3-dihydrofuro[3,4-c][1,7]naphthyridin-8-carboxamid;
(3R)-4-Amino-N,3-dimethyl-N-((6-(trifluormethyl)-3-pyridazinyl)methyl)-1,3-dihydrofuro[3,4-c][1,7]naphthyridin-8-carboxamid;
(3S)-4-Amino-N-cyclopropyl-3-methyl-N-((5-(trifluormethyl)-2-pyridinyl)methyl)-1,3-dihydrofuro[3,4-c][1,7]naphthyridin-8-carboxamid;
(3S)-4-Amino-N,3-dimethyl-N-((6-(trifluormethyl)-3-pyridazinyl)methyl)-1,3-dihydrofuro[3,4-c][1,7]naphthyridin-8-carboxamid;
(3R)-4-Amino-3-methyl-N-(2,2,2-trifluorethyl)-N-((5-(trifluormethyl)-2-pyridinyl)methyl)-1,3-dihydrofuro[3,4-c][1,7]naphthyridin-8-carboxamid;
4-Amino-N-(cyclopropylmethyl)-N-((5-(trifluormethyl)-2-pyridinyl)methyl)-1,3-dihydrofuro[3,4-c][1,8]naphthyridin-8-carboxamid;
4-Amino-N-((2R)-1-methoxy-2-propanyl)-N-((5-(trifluormethyl)-2-pyridinyl)methyl)-1,3-dihydrofuro[3,4-c][1,8]naphthyridin-8-carboxamid;
4-Amino-N-((5-Cyclopropyl-2-pyridinyl)methyl)-N-methyl-1,3-dihydrofuro[3,4-c]chinolin-8-carboxamid;
4-Amino-N-((5-Cyano-2-pyridinyl)methyl)-N-methyl-1,3-dihydrofuro[3,4-c]chinolin-8-carboxamid;
4-Amino-N-((5-Cyano-2-pyridinyl)methyl)-N-(cyclopropylmethyl)-1,3-dihydrofuro[3,4-c]chinolin-8-carboxamid;
(3R)-4-Amino-N-((6-bromo-3-pyridazinyl)methyl)-N-(cyclopropylmethyl)-3-methyl-1,3-dihydrofuro[3,4-c]chinolin-8-carboxamid;
4-Amino-N-((6-bromo-3-pyridazinyl)methyl)-N-methyl-1,3-dihydrofuro[3,4-c]chinolin-8-carboxamid;
(3R)-4-Amino-N-((5-bromo-2-pyridinyl)methyl)-N,3-dimethyl-1,3-dihydrofuro[3,4-c]chinolin-8-carboxamid;
(3R)-4-Amino-N-((6-cyclopropyl-3-pyridazinyl)methyl)-3-methyl-N-((1R)-1-(2-pyrimidinyl)ethyl)-1,3-dihydrofuro[3,4-c]chinolin-8-carboxamid;
4-Amino-N-((5-bromo-2-pyridinyl)methyl)-N-methyl-1,3-dihydrofuro[3,4-c]chinolin-8-carboxamid;
(3R)-4-Amino-N-((5-Cyano-2-pyridinyl)methyl)-N-(cyclopropylmethyl)-3-methyl-1,3-dihydrofuro[3,4-c]chinolin-8-carboxamid;
(3R)-4-Amino-N-((6-bromo-3-pyridazinyl)methyl)-3-methyl-N-(2-propanyl)-1,3-dihydrofuro[3,4-c]chinolin-8-carboxamid;
(3R)-4-Amino-N-(cyclopropylmethyl)-3-methyl-N-((6-(trifluormethyl)-3-pyridazinyl)methyl)-1,3-dihydrofuro[3,4-c]chinolin-8-carboxamid;
(3R)-4-Amino-N-(cyclopropylmethyl)-3-methyl-N-((5-(trifluormethyl)-2-pyridinyl)methyl)-1,3-dihydrofuro[3,4-c]chinolin-8-carboxamid;
4-Amino-N-((6-bromo-3-pyridazinyl)methyl)-N-(2-propanyl)-1,3-dihydrofuro[3,4-c]chinolin-8-carboxamid;
4-Amino-N-(cyclopropylmethyl)-N-((6-(trifluormethyl)-3-pyridazinyl)methyl)-1,3-dihydrofuro[3,4-c]chinolin-8-carboxamid;
4-Amino-N-((1-methylcyclopropyl)methyl)-N-((5-(trifluormethyl)-2-pyridinyl)methyl)-1,3-dihydrofuro[3,4-c]chinolin-8-carboxamid;
(3R)-4-Amino-N-(2,2-dimethylpropyl)-3-methyl-N-((5-(trifluormethyl)-2-pyridinyl)methyl)-1,3-dihydrofuro[3,4-c]chinolin-8-carboxamid;
(3R)-4-Amino-3-methyl-N-((1-methylcyclopropyl)methyl)-N-((5-(trifluormethyl)-2-pyridinyl)methyl)-1,3-dihydrofuro[3,4-c]chinolin-8-carboxamid;
4-Amino-N-(2,2-dimethylpropyl)-3,3-dimethyl-N-((5-(trifluormethyl)-2-pyridinyl)methyl)-1,3-dihydrofuro[3,4-c]chinolin-8-carboxamid;
4-Amino-3,3-dimethyl-N-((1-methylcyclopropyl)methyl)-N-((5-(trifluormethyl)-2-pyridinyl)methyl)-1,3-dihydrofuro[3,4-c]chinolin-8-carboxamid;
4-Amino-N-(2,2-dimethylpropyl)-N-((5-(trifluormethyl)-2-pyridinyl)methyl)-1,3-dihydrofuro[3,4-c]chinolin-8-carboxamid;
4-Amino-N-cyclopropyl-N-((5-cyclopropyl-2-pyridinyl)methyl)-1,3-dihydrofuro[3,4-c]chinolin-8-carboxamid;
4-Amino-N-methyl-N-((5-(trifluormethyl)-2-pyridinyl)methyl)-1,3-dihydrofuro[3,4-c][1,7]naphthyridin-8-carboxamid;
4-Amino-7-fluoro-N-(2-propanyl)-N-((5-(trifluormethyl)-2-pyridinyl)methyl)-1,3-dihydrofuro[3,4-c]chinolin-8-carboxamid;
4-Amino-N-((5-Cyano-2-pyridinyl)methyl)-N-(2-propanyl)-1,3-dihydrofuro[3,4-c][1,7]naphthyridin-8-carboxamid;
4-Amino-N-((5-Cyano-2-pyridinyl)methyl)-N-cyclopropyl-1,3-dihydrofuro[3,4-c][1,7]naphthyridin-8-carboxamid;
4-Amino-N-cyclopropyl-N-((5-cyclopropyl-2-pyridinyl)methyl)-1,3-dihydrofuro[3,4-c][1,7]naphthyridin-8-carboxamid;
4-Amino-N-((5-Cyclopropyl-2-pyridinyl)methyl)-N-methyl-1,3-dihydrofuro[3,4-c][1,7]naphthyridin-8-carboxamid;
(3R)-4-Amino-N-((6-cyclopropyl-3-pyridinyl)methyl)-3-methyl-N-(2-propanyl)-1,3-dihydrofuro[3,4-c]chinolin-8-carboxamid;
(3R)-4-Amino-N-cyclopropyl-3-methyl-N-((5-(trifluormethyl)-2-pyridinyl)methyl)-1,3-dihydrofuro[3,4-c]chinolin-8-carboxamid;
4-Amino-N-methyl-N-((6-(Trifluormethyl)-3-pyridazinyl)methyl)-1,3-dihydrofuro[3,4-c]chinolin-8-carboxamid;
(3R)-4-Amino-N-cyclopropyl-3-methyl-N-((6-(Trifluormethyl)-3-pyridazinyl)methyl)-1,3-dihydrofuro[3,4-c]chinolin-8-carboxamid;
(3R)-4-Amino-N-((5-cyano-2-pyridinyl)methyl)-3-methyl-N-(2-propanyl)-1,3-dihydrofuro[3,4-c]chinolin-8-carboxamid;
(3R)-4-Amino-N-((5-Cyano-2-pyridinyl)methyl)-N-cyclopropyl-3-methyl-1,3-dihydrofuro[3,4-c]chinolin-8-carboxamid;
(3R)-4-Amino-N-((5-Cyclopropyl-2-pyridinyl)methyl)-N,3-dimethyl-1,3-dihydrofuro[3,4-c]chinolin-8-carboxamid;
4-Amino-N-cyclopropyl-7-fluoro-N-((6-(trifluormethyl)-3-pyridazinyl)methyl)-1,3-dihydrofuro[3,4-c]chinolin-8-carboxamid;
4-Amino-7-fluoro-N-(2-propanyl)-N-((6-(trifluormethyl)-3-pyridazinyl)methyl)-1,3-dihydrofuro[3,4-c]chinolin-8-carboxamid;
4-Amino-7-fluoro-N-methyl-N-((6-(trifluormethyl)-3-pyridazinyl)methyl)-1,3-dihydrofuro[3,4-c]chinolin-8-carboxamid;
(3R)-4-Amino-N-ethyl-3-methyl-N-((5-(trifluormethyl)-2-pyridinyl)methyl)-1,3-dihydrofuro[3,4-c]chinolin-8-carboxamid;
4-Amino-N-ethyl-N-((5-(Trifluormethyl)-2-pyridinyl)methyl)-1,3-dihydrofuro[3,4-c][1,7]naphthyridin-8-carboxamid;
4-Amino-N-ethyl-N-((5-(Trifluormethyl)-2-pyridinyl)methyl)-1,3-dihydrofuro[3,4-c]chinolin-8-carboxamid;
4-Amino-N-((6-cyclopropyl-3-pyridinyl)methyl)-7-fluoro-N-(2-propanyl)-1,3-dihydrofuro[3,4-c]chinolin-8-carboxamid;
4-Amino-N-((5-Cyclopropyl-2-pyridinyl)methyl)-N-(2-propanyl)-1,3-dihydrofuro[3,4-c]chinolin-8-carboxamid;
4-Amino-N-cyclopropyl-N-((5-(trifluormethyl)-2-pyridinyl)methyl)-1,3-dihydrofuro[3,4-c][1,7]naphthyridin-8-carboxamid;
4-Amino-N-(2-propanyl)-N-((6-(trifluormethyl)-3-pyridazinyl)methyl)-1,3-dihydrofuro[3,4-c][1,7]naphthyridin-8-carboxamid;
4-Amino-N-((5-Cyano-2-pyridinyl)methyl)-N-methyl-1,3-dihydrofuro[3,4-c][1,7]naphthyridin-8-carboxamid;
(3R)-4-Amino-N-cyclopropyl-N-((5-cyclopropyl-2-pyridinyl)methyl)-3-methyl-1,3-dihydrofuro[3,4-c]chinolin-8-carboxamid;
4-Amino-N-cyclopropyl-7-fluoro-N-((5-(trifluormethyl)-2-pyridinyl)methyl)-1,3-dihydrofuro[3,4-c]chinolin-8-carboxamid;
4-Amino-N-cyclopropyl-N-((5-cyclopropyl-2-pyridinyl)methyl)-7-fluoro-1,3-dihydrofuro[3,4-c]chinolin-8-carboxamid;
4-Amino-N-((5-Cyclopropyl-2-pyridinyl)methyl)-7-fluoro-N-(2-propanyl)-1,3-dihydrofuro[3,4-c]chinolin-8-carboxamid;
4-Amino-N-((5-Cyclopropyl-2-pyridinyl)methyl)-7-fluoro-N-methyl-1,3-dihydrofuro[3,4-c]chinolin-8-carboxamid;
4-Amino-N-((6-ethoxy-3-pyridazinyl)methyl)-N-((2R)-1-methoxy-2-propanyl)-1,3-dihydrofuro[3,4-c]chinolin-8-carboxamid;
(3R)-4-Amino-N-((6-ethoxy-3-pyridazinyl)methyl)-N-((2R)-1-methoxy-2-propanyl)-3-methyl-1,3-dihydrofuro[3,4-c]chinolin-8-carboxamid;
4-Amino-N-methyl-N-((6-(Trifluormethyl)-3-pyridazinyl)methyl)-1,3-dihydrofuro[3,4-c][1,7]naphthyridin-8-carboxamid;
(3R)-4-Amino-N,3-dimethyl-N-((5-(trifluormethyl)-2-pyridinyl)methyl)-1,3-dihydrofuro[3,4-c]chinolin-8-carboxamid;
(3R)-4-Amino-N-((5-Cyclopropyl-2-pyridinyl)methyl)-3-methyl-N-(2-propanyl)-1,3-dihydrofuro[3,4-c]chinolin-8-carboxamid;
4-Amino-N-(Bicyclo[1.1.1]pentan-1-yl)-N-((6-ethoxy-3-pyridazinyl)methyl)-1,3-dihydrofuro[3,4-c][1,7]naphthyridin-8-carboxamid;
4-Amino-7-chloro-N-((5-Cyclopropyl-2-pyridinyl)methyl)-N-(2-propanyl)-1,3-dihydrofuro[3,4-c]chinolin-8-carboxamid;
(3R)-4-Amino-N-(bicyclo[1.1.1]pentan-1-yl)-3-methyl-N-((5-(trifluormethyl)-2-pyridinyl)methyl)-1,3-dihydrofuro[3,4-c]chinolin-8-carboxamid;
4-Amino-N-(bicyclo[1.1.1 ]pentan-1-yl)-7-fluoro-N-((5-(trifluormethyl)-2-pyridinyl)methyl)-1,3-dihydrofuro[3,4-c]chinolin-8-carboxamid;
4-Amino-N-cyclopropyl-N-((6-(Trifluormethyl)-3-pyridazinyl)methyl)-1,3-dihydrofuro[3,4-c][1,7]naphthyridin-8-carboxamid;
4-Amino-N-((6-cyclopropyl-3-pyridinyl)methyl)-N-(2-propanyl)-1,3-dihydrofuro[3,4-c][1,7]naphthyridin-8-carboxamid;
(3R)-4-Amino-3-methyl-N-(2-propanyl)-N-((5-(trifluormethyl)-2-pyridinyl)methyl)-1,3-dihydrofuro[3,4-c]chinolin-8-carboxamid;
4-Amino-N-((5-Cyclopropyl-2-pyridinyl)methyl)-N-(2-propanyl)-1,3-dihydrofuro[3,4-c][1,7]naphthyridin-8-carboxamid;
(3R)-4-Amino-N-((5-Cyano-2-pyridinyl)methyl)-N,3-dimethyl-1,3-dihydrofuro[3,4-c]chinolin-8-carboxamid;
4-Amino-N-(bicyclo[1.1.1]pentan-1-yl)-N-((6-ethoxy-3-pyridazinyl)methyl)-1,3-dihydrofuro[3,4-c]chinolin-8-carboxamid;
4-Amino-N-((6-Ethoxy-3-pyridazinyl)methyl)-N-((2R)-1-methoxy-2-propanyl)-1,3-dihydrofuro[3,4-c][1,7]naphthyridin-8-carboxamid;
4-Amino-7-chloro-N-methyl-N-((6-(trifluormethyl)-3-pyridazinyl)methyl)-1,3-dihydrofuro[3,4-c]chinolin-8-carboxamid;
4-Amino-N-((2R)-1-methoxy-2-propanyl)-N-((6-(Trifluormethyl)-3-pyridazinyl)methyl)-1,3-dihydrofuro[3,4-c][1,7]naphthyridin-8-carboxamid;
4-Amino-N-((2S)-1-methoxy-2-propanyl)-N-((6-(trifluormethyl)-3-pyridazinyl)methyl)-1,3-dihydrofuro[3,4-c][1,7]naphthyridin-8-carboxamid;
4-Amino-N-(2-propanyl)-N-((6-(trifluormethyl)-3-pyridazinyl)methyl)-1,3-dihydrofuro[3,4-c]chinolin-8-carboxamid;
4-Amino-7-chloro-N-((5-Cyano-2-pyridinyl)methyl)-N-(2-propanyl)-1,3-dihydrofuro[3,4-c]chinolin-8-carboxamid;
(3R)-4-Amino-3-methyl-N-(2-propanyl)-N-((6-(trifluormethyl)-3-pyridazinyl)methyl)-1,3-dihydrofuro[3,4-c]chinolin-8-carboxamid;
4-Amino-7-chloro-N-(2-propanyl)-N-((6-(trifluormethyl)-3-pyridazinyl)methyl)-1,3-dihydrofuro[3,4-c]chinolin-8-carboxamid;
4-Amino-N-(bicyclo[1.1.1]pentan-1-yl)-7-chloro-N-((5-(trifluormethyl)-2-pyridinyl)methyl)-1,3-dihydrofuro[3,4-c]chinolin-8-carboxamid;
(3R)-4-Amino-N-(cyclopropylmethyl)-3-methyl-N-((5-(trifluormethyl)-2-pyridinyl)methyl)-1,3-dihydrofuro[3,4-c][1,7]naphthyridin-8-carboxamid;
(3R)-4-Amino-N-((6-ethoxy-3-pyridazinyl)methyl)-N-((2R)-1-methoxy-2-propanyl)-3-methyl-1,3-dihydrofuro[3,4-c][1,7]naphthyridin-8-carboxamid;
4-Amino-N-((1-Methylcyclopropyl)methyl)-N-((5-(Trifluormethyl)-2-pyridinyl)methyl)-1,3-dihydrofuro[3,4-c][1,7]naphthyridin-8-carboxamid;
4-Amino-N-Methyl-N-(4-(Trifluormethyl)benzyl)-1,3-dihydrofuro[3,4-c]chinolin-8-carboxamid;
4-Amino-N-methyl-N-((6-(Trifluormethyl)-3-pyridinyl)methyl)-1,3-dihydrofuro[3,4-c]chinolin-8-carboxamid;
4-Amino-7-chloro-N-((6-cyclopropyl-3-pyridinyl)methyl)-N-(2-propanyl)-1,3-dihydrofuro[3,4-c]chinolin-8-carboxamid;
(3R)-4-Amino-N-(cyclopropylmethyl)-3-methyl-N-((6-(trifluormethyl)-3-pyridazinyl)methyl)-1,3-dihydrofuro[3,4-c][1,7]naphthyridin-8-carboxamid;
(3S)-4-Amino-N-((6-cyclopropyl-3-pyridinyl)methyl)-3-methyl-N-(2-propanyl)-1,3-dihydrofuro[3,4-c][1,7]naphthyridin-8-carboxamid;
(3R)-4-Amino-N-((5-Cyano-2-pyridinyl)methyl)-3-methyl-N-(2-propanyl)-1,3-dihydrofuro[3,4-c][1,7]naphthyridin-8-carboxamid;
(3R)-4-Amino-N-cyclopropyl-3-methyl-N-((6-(Trifluormethyl)-3-pyridazinyl)methyl)-1,3-dihydrofuro[3,4-c][1,7]naphthyridin-8-carboxamid;
(3R)-4-Amino-N-((5-cyclopropyl-2-pyridinyl)methyl)-3-methyl-N-(2-propanyl)-1,3-dihydrofuro[3,4-c][1,7]naphthyridin-8-carboxamid;
(3S)-4-Amino-N-((5-cyclopropyl-2-pyridinyl)methyl)-3-methyl-N-(2-propanyl)-1,3-dihydrofuro[3,4-c][1,7]naphthyridin-8-carboxamid;
(3R)-4-Amino-N-ethyl-3-methyl-N-((5-(Trifluormethyl)-2-pyridinyl)methyl)-1,3-dihydrofuro[3,4-c][1,7]naphthyridin-8-carboxamid;
(3R)-4-Amino-N-(bicyclo[1.1.1]pentan-1-yl)-N-((6-ethoxy-3-pyridazinyl)methyl)-3-methyl-1,3-dihydrofuro[3,4-c][1,7]naphthyridin-8-carboxamid;
(3R)-4-Amino-N-((6-bromo-3-pyridazinyl)methyl)-N-((2R)-1-methoxy-2-propanyl)-3-methyl-1,3-dihydrofuro[3,4-c][1,7]naphthyridin-8-carboxamid;
(3R)-4-Amino-N,3-dimethyl-N-((5-(trifluormethyl)-2-pyridinyl)methyl)-1,3-dihydrofuro[3,4-c][1,7]naphthyridin-8-carboxamid;
(3R)-4-Amino-N-((6-cyclopropyl-3-pyridinyl)methyl)-3-methyl-N-(2-propanyl)-1,3-dihydrofuro[3,4-c][1,7]naphthyridin-8-carboxamid;
Methyl-4-(6-((((4-amino-1,3-dihydrofuro[3,4-c]chinolin-8-yl)carbonyl)(methyl)amino)methyl)-3-pyridinyl)-1-piperazincarboxylat;
(3S)-4-Amino-N-((5-cyclopropyl-2-pyridinyl)methyl)-N,3-dimethyl-1,3-dihydrofuro[3,4-c][1,7]naphthyridin-8-carboxamid;
(3R)-4-Amino-N-((5-cyclopropyl-2-pyridinyl)methyl)-N,3-dimethyl-1,3-dihydrofuro[3,4-c][1,7]naphthyridin-8-carboxamid;
(3R)-4-Amino-N-cyclopropyl-N-((5-cyclopropyl-2-pyridinyl)methyl)-3-methyl-1,3-dihydrofuro[3,4-c][1,7]naphthyridin-8-carboxamid;
(3S)-4-Amino-N-cyclopropyl-N-((5-cyclopropyl-2-pyridinyl)methyl)-3-methyl-1,3-dihydrofuro[3,4-c][1,7]naphthyridin-8-carboxamid;
4-Amino-N-(cyclopropylmethyl)-N-((6-(difluormethoxy)-3-pyridazinyl)methyl)-1,3-dihydrofuro[3,4-c][1,7]naphthyridin-8-carboxamid;
4-Amino-N-ethyl-7-fluoro-N-((5-(trifluormethyl)-2-pyridinyl)methyl)-1,3-dihydrofuro[3,4-c]chinolin-8-carboxamid;
4-Amino-7-chloro-N-ethyl-N-((5-(trifluormethyl)-2-pyridinyl)methyl)-1,3-dihydrofuro[3,4-c]chinolin-8-carboxamid;
(3R)-4-Amino-N-((5-chloro-2-pyridinyl)methyl)-N-ethyl-3-methyl-1,3-dihydrofuro[3,4-c]chinolin-8-carboxamid;
4-Amino-N-ethyl-3-methyl-N-((5-(Trifluormethyl)-2-pyridinyl)methyl)-3H-pyrazolo[3,4-c]chinolin-8-carboxamid;
(3R)-4-Amino-N-((5-chloro-2-pyridinyl)methyl)-3-methyl-N-(2-propanyl)-1,3-dihydrofuro[3,4-c]chinolin-8-carboxamid;
(3R)-4-Amino-N-(cyclopropylmethyl)-N-((6-(difluormethoxy)-3-pyridazinyl)methyl)-3-methyl-1,3-dihydrofuro[3,4-c][1,7]naphthyridin-8-carboxamid;
(3S)-4-Amino-N-(cyclopropylmethyl)-N-((6-(difluormethoxy)-3-pyridazinyl)methyl)-3-methyl-1,3-dihydrofuro[3,4-c][1,7]naphthyridin-8-carboxamid;
4-Amino-N-(cyclopropylmethyl)-N-((6-(difluormethoxy)-3-pyridazinyl)methyl)-1,3-dihydrofuro[3,4-c]chinolin-8-carboxamid;
4-Amino-N-ethyl-7-fluoro-3-methyl-N-((5-(trifluormethyl)-2-pyridinyl)methyl)-3H-pyrazolo[3,4-c]chinolin-8-carboxamid;
5-Amino-N-ethyl-N-((5-(Trifluormethyl)-2-pyridinyl)methyl)benzo[c][2,6]naphthyridin-9-carboxamid;
4-Amino-N-((5-(3,6-dihydro-2H-pyran-4-yl)-2-pyridinyl)methyl)-N-methyl-1,3-dihydrofuro[3,4-c]chinolin-8-carboxamid;
Methyl-6-((((4-amino-1,3-dihydrofuro[3,4-c]chinolin-8-yl)carbonyl)(methyl)amino)methyl)-3',6'-dihydro[3,4'-bipyridin]-1'(2'H)-carboxylat;
5-Oxo-N-(2-propanyl)-N-((5-(trifluormethyl)-2-pyridinyl)methyl)-5,6-dihydropyrazolo[1,5-c]chinazolin-9-carboxamid;
4-Amino-1,3-dimethyl-N-(2-propanyl)-N-((5-(trifluormethyl)-2-pyridinyl)methyl)-1H-pyrazolo[4,3-c]chinolin-8-carboxamid;
(3R)-4-Amino-N-((5-bromo-2-pyridinyl)methyl)-N,3-dimethyl-1,3-dihydrofuro[3,4-c][1,7]naphthyridin-8-carboxamid;
(3S)-4-Amino-N-((5-bromo-2-pyridinyl)methyl)-N,3-dimethyl-1,3-dihydrofuro[3,4-c][1,7]naphthyridin-8-carboxamid;
(3R)-4-Amino-3-methyl-N-(2-propanyl)-N-((5-(trifluormethyl)-2-pyrazinyl)methyl)-1,3-dihydrofuro[3,4-c]chinolin-8-carboxamid;
4-Amino-7-fluoro-N-((1R)-1-(2-pyrimidinyl)ethyl)-N-((5-(trifluormethyl)-2-pyridinyl)methyl)-1,3-dihydrofuro[3,4-c]chinolin-8-carboxamid;
4-Amino-N-((5-Cyano-2-pyridinyl)methyl)-7-fluoro-N-((1R)-1-(2-pyrimidinyl)ethyl)-1,3-dihydrofuro[3,4-c]chinolin-8-carboxamid;
4-Amino-N-((6-bromo-3-pyridazinyl)methyl)-7-fluoro-N-((1R)-1-(3-fluoro-2-pyridinyl)ethyl)-1,3-dihydrofuro[3,4-c]chinolin-8-carboxamid;
4-Amino-N-((5-(Difluormethyl)-2-pyridinyl)methyl)-N-((1R)-1-(2-pyrimidinyl)ethyl)-1,3-dihydrofuro[3,4-c]chinolin-8-carboxamid;
4-Amino-N-((5-chloro-2-pyridinyl)methyl)-N-((1R)-1-(2-pyrimidinyl)ethyl)-1,3-dihydrofuro[3,4-c]chinolin-8-carboxamid;
4-Amino-N-((1S)-1-(2-Pyrimidinyl)ethyl)-N-((6-(Trifluormethyl)-3-pyridazinyl)methyl)-1,3-dihydrofuro[3,4-c]chinolin-8-carboxamid;
4-Amino-N-((1R)-1-(2-pyrimidinyl)ethyl)-N-((6-(Trifluormethyl)-3-pyridazinyl)methyl)-1,3-dihydrofuro[3,4-c]chinolin-8-carboxamid;
4-Amino-N-((1R)-1-(3-fluoro-2-pyridinyl)ethyl)-N-((6-(Trifluormethyl)-3-pyridazinyl)methyl)-1,3-dihydrofuro[3,4-c]chinolin-8-carboxamid;
4-Amino-N-cyclopropyl-N-((5-(trifluormethyl)-2-pyridinyl)methyl)-1,3-dihydrofuro[3,4-c]chinolin-8-carboxamid;
4-Amino-N-(1,3-dimethoxy-2-propanyl)-N-((5-(trifluormethyl)-2-pyridinyl)methyl)-1,3-dihydrofuro[3,4-c]chinolin-8-carboxamid;
4-Amino-N-((2R)-1-methoxy-2-propanyl)-N-((5-(trifluormethyl)-2-pyridinyl)methyl)-1,3-dihydrofuro[3,4-c]chinolin-8-carboxamid;
4-Amino-N-((6-methoxy-3-pyridazinyl)methyl)-N-((1R)-1-(2-pyrimidinyl)ethyl)-1,3-dihydrofuro[3,4-c]chinolin-8-carboxamid;
4-Amino-N-((8R)-5,6,7,8-tetrahydro[1,2,4]triazolo[1,5-a]pyridin-8-yl)-N-((5-(Trifluormethyl)-2-pyridinyl)methyl)-1,3-dihydrofuro[3,4-c]chinolin-8-carboxamid;
4-Amino-N-((8S)-5,6,7,8-tetrahydro[1,2,4]triazolo[1,5-a]pyridin-8-yl)-N-((5-(Trifluormethyl)-2-pyridinyl)methyl)-1,3-dihydrofuro[3,4-c]chinolin-8-carboxamid;
4-amino-N-((5-cyano-2-pyridinyl)methyl)-N-((8R)-5,6,7,8-tetrahydro[1,2,4]triazolo[1,5-a]pyridin-8-yl)-1,3-dihydrofuro[3,4-c]quinoline-8-carboxamide;
4-amino-N-((5-cyano-2-pyridinyl)methyl)-N-((8S)-5,6,7,8-tetrahydro[1,2,4]triazolo[1,5-a]pyridin-8-yl)-1,3-dihydrofuro[3,4-c]quinoline-8-carboxamide;
4-Amino-N-(4-(3-oxetanyl)benzyl)-N-((1R)-1-(2-pyrimidinyl)ethyl)-1,3-dihydrofuro[3,4-c]chinolin-8-carboxamid;
4-Amino-N-(4-(3-oxetanyl)benzyl)-N-((1S)-1-(2-pyrimidinyl)ethyl)-1,3-dihydrofuro[3,4-c]chinolin-8-carboxamid;
4-Amino-N-methyl-N-((5-(Trifluormethyl)-2-pyridinyl)methyl)-1,3-dihydrofuro[3,4-c]chinolin-8-carboxamid;
4-Amino-N-(2-propanyl)-N-((5-(trifluormethyl)-2-pyridinyl)methyl)-1,3-dihydrofuro[3,4-c]chinolin-8-carboxamid;
4-Amino-N-(cyclopropylmethyl)-N-((6-(4-morpholinyl)-3-pyridazinyl)methyl)-1,3-dihydrofuro[3,4-c]chinolin-8-carboxamid;
4-Amino-N-((1R)-1-(2-pyrimidinyl)ethyl)-N-((6-(2,2,2-Trifluorethoxy)-3-pyridazinyl)methyl)-1,3-dihydrofuro[3,4-c]chinolin-8-carboxamid;
4-Amino-N-((5-Cyano-2-pyridinyl)methyl)-N-cyclopropyl-1,3-dihydrofuro[3,4-c]chinolin-8-carboxamid;
4-Amino-N-((5-bromo-2-pyridinyl)methyl)-N-cyclopropyl-1,3-dihydrofuro[3,4-c]chinolin-8-carboxamid;
4-Amino-N-cyclopropyl-N-((5-(difluormethyl)-2-pyridinyl)methyl)-1,3-dihydrofuro[3,4-c]chinolin-8-carboxamid;
4-Amino-N-((1-Cyanocyclopropyl)methyl)-N-((5-cyano-2-pyridinyl)methyl)-1,3-dihydrofuro[3,4-c]chinolin-8-carboxamid;
4-Amino-N-((6-cyclopropyl-3-pyridazinyl)methyl)-N-((1R)-1-(2-pyrimidinyl)ethyl)-1,3-dihydrofuro[3,4-c]chinolin-8-carboxamid;
4-Amino-N-((3R,4S)-3-methoxytetrahydro-2H-pyran-4-yl)-N-((5-(Trifluormethyl)-2-pyridinyl)methyl)-1,3-dihydrofuro[3,4-c]chinolin-8-carboxamid;
4-Amino-N-((3S,4R)-3-methoxytetrahydro-2H-pyran-4-yl)-N-((5-(trifluormethyl)-2-pyridinyl)methyl)-1,3-dihydrofuro[3,4-c]chinolin-8-carboxamid;
4-Amino-N-((3R,4R)-4-Methoxytetrahydro-2H-pyran-3-yl)-N-((5-(Trifluormethyl)-2-pyridinyl)methyl)-1,3-dihydrofuro[3,4-c]chinolin-8-carboxamid;
4-Amino-N-((3S,4S)-4-Methoxytetrahydro-2H-pyran-3-yl)-N-((5-(Trifluormethyl)-2-pyridinyl)methyl)-1,3-dihydrofuro[3,4-c]chinolin-8-carboxamid;
4-Amino-7-chloro-N-((5-Cyano-2-pyridinyl)methyl)-N-cyclopropyl-1,3-dihydrofuro[3,4-c]chinolin-8-carboxamid;
4-Amino-N-((6-chloro-5-methoxy-2-pyridinyl)methyl)-N-(2-propanyl)-1,3-dihydrofuro[3,4-c]chinolin-8-carboxamid;
4-Amino-N-((6-bromo-3-pyridazinyl)methyl)-N-((2R)-1-methoxy-2-propanyl)-1,3-dihydrofuro[3,4-c]chinolin-8-carboxamid;
4-Amino-7-chloro-N-(cyclopropylmethyl)-N-((5-(trifluormethyl)-2-pyridinyl)methyl)-1,3-dihydrofuro[3,4-c]chinolin-8-carboxamid;
(3R)-4-Amino-3-methyl-N-(2-propanyl)-N-((5-(trifluormethoxy)-2-pyridinyl)methyl)-1,3-dihydrofuro[3,4-c]chinolin-8-carboxamid;
(3R)-4-Amino-N-((5-(Difluormethyl)-2-pyridinyl)methyl)-3-methyl-N-((1R)-1-(1-methyl-1H-1,2,4-triazol-3-yl)ethyl)-1,3-dihydrofuro[3,4-c]chinolin-8-carboxamid;
(3R)-4-Amino-3-methyl-N-(1-methyl-1H-pyrazol-4-yl)-N-((5-(trifluormethyl)-2-pyridinyl)methyl)-1,3-dihydrofuro[3,4-c]chinolin-8-carboxamid;
(3R)-4-Amino-N-((6-bromo-3-pyridazinyl)methyl)-N-((2R)-1-methoxy-2-propanyl)-3-methyl-1,3-dihydrofuro[3,4-c]chinolin-8-carboxamid;
4-Amino-N-(3,4-dihydro-2H-pyrano[2,3-c]pyridin-6-ylmethyl)-N-((1R)-1-(2-pyrimidinyl)ethyl)-1,3-dihydrofuro[3,4-c]chinolin-8-carboxamid;
4-Amino-7-chloro-N-methyl-N-((5-(trifluormethyl)-2-pyridinyl)methyl)-1,3-dihydrofuro[3,4-c]chinolin-8-carboxamid;
4-Amino-N-(bicyclo[1.1.1]pentan-1-yl)-N-((5-(trifluormethyl)-2-pyridinyl)methyl)-1,3-dihydrofuro[3,4-c]chinolin-8-carboxamid;
4-Amino-7-chloro-N-((5-cyano-2-pyridinyl)methyl)-N-methyl-1,3-dihydrofuro[3,4-c]chinolin-8-carboxamid;
4-Amino-N-((5-Cyano-2-pyridinyl)methyl)-N-((2R)-1-fluoro-2-propanyl)-1,3-dihydrofuro[3,4-c]chinolin-8-carboxamid;
4-Amino-N-((5-Cyano-2-pyridinyl)methyl)-N-((2S)-1-fluoro-2-propanyl)-1,3-dihydrofuro[3,4-c]chinolin-8-carboxamid;
4-Amino-7-chloro-N-((5-cyano-2-pyridinyl)methyl)-N-(cyclopropylmethyl)-1,3-dihydrofuro[3,4-c]chinolin-8-carboxamid;
4-Amino-7-fluoro-N-methyl-N-((5-(trifluormethyl)-2-pyridinyl)methyl)-1,3-dihydrofuro[3,4-c]chinolin-8-carboxamid;
4-Amino-N-((5-Cyano-2-pyridinyl)-methyl)-N-cyclopropyl-7-fluoro-1,3-dihydrofuro[3,4-c]chinolin-8-carboxamid;
4-Amino-N-((5-Cyano-2-pyridinyl)methyl)-7-fluoro-N-(2-propanyl)-1,3-dihydrofuro[3,4-c]chinolin-8-carboxamid;
4-Amino-7-chloro-N-((5-Cyclopropyl-2-pyridinyl)-methyl)-N-methyl-1,3-dihydrofuro[3,4-c]chinolin-8-carboxamid;
4-Amino-N-((5-Cyano-2-pyridinyl)methyl)-7-fluoro-N-methyl-1,3-dihydrofuro[3,4-c]chinolin-8-carboxamid;
4-Amino-7-chloro-N-cyclopropyl-N-((5-Cyclopropyl-2-pyridinyl)methyl)-1,3-dihydrofuro[3,4-c]chinolin-8-carboxamid;
(3R)-4-Amino-3-methyl-N-(2,2,2-trifluorethyl)-N-((5-(trifluormethyl)-2-pyridinyl)methyl)-1,3-dihydrofuro[3,4-c]chinolin-8-carboxamid;
(3R)-4-Amino-N-(bicyclo[1.1.1]pentan-1-yl)-N-((6-ethoxy-3-pyridazinyl)methyl)-3-methyl-1,3-dihydrofuro[3,4-c]chinolin-8-carboxamid;
4-Amino-N-(2,2,2-trifluorethyl)-N-((5-(trifluormethyl)-2-pyridinyl)methyl)-1,3-dihydrofuro[3,4-c]chinolin-8-carboxamid;
4-Amino-7-chloro-N-(2-propanyl)-N-((5-(trifluormethyl)-2-pyridinyl)methyl)-1,3-dihydrofuro[3,4-c]chinolin-8-carboxamid;
(3R)-4-Amino-N,3-dimethyl-N-((6-(trifluormethyl)-3-pyridazinyl)methyl)-1,3-dihydrofuro[3,4-c]chinolin-8-carboxamid;
4-Amino-N-(2,2,2-trifluorethyl)-N-((5-(trifluormethyl)-2-pyridinyl)methyl)-1,3-dihydrofuro[3,4-c][1,7]naphthyridin-8-carboxamid;
4-Amino-7-chloro--N-((2R)-1-methoxy-2-propanyl)-N-((6-(Trifluormethyl)-3-pyridazinyl)methyl)-1,3-dihydrofuro[3,4-c]chinolin-8-carboxamid;
4-Amino-7-chloro-N-((2S)-1-methoxy-2-propanyl)-N-((6-(trifluormethyl)-3-pyridazinyl)methyl)-1,3-dihydrofuro[3,4-c]chinolin-8-carboxamid;
4-Amino-7-fluoro-N-((2R)-1-methoxy-2-propanyl)-N-((6-(trifluormethyl)-3-pyridazinyl)methyl)-1,3-dihydrofuro[3,4-c]chinolin-8-carboxamid;
4-Amino-7-fluoro-N-((2S)-1-methoxy-2-propanyl)-N-((6-(trifluormethyl)-3-pyridazinyl)methyl)-1,3-dihydrofuro[3,4-c]chinolin-8-carboxamid;
(3R)-4-Amino-3-methyl-N-(tetrahydro-2H-pyran-4-yl)-N-((5-(trifluormethyl)-2-pyridinyl)methyl)-1,3-dihydrofuro[3,4-c]chinolin-8-carboxamid;
4-Amino-7-chloro-N-cyclopropyl-N-((6-(Trifluormethyl)-3-pyridazinyl)methyl)-1,3-dihydrofuro[3,4-c]chinolin-8-carboxamid;
6-Amino-N-(1-methyl-1H-pyrazol-4-yl)-N-((5-(trifluormethyl)-2-pyridinyl)methyl)-8,9-dihydro-7H-cyclopenta[c][1,7]naphthyridin-2-carboxamid;
6-Amino-N-((5-(Difluormethyl)-2-pyridinyl)methyl)-N-(1-methyl-1H-pyrazol-4-yl)-8,9-dihydro-7H-cyclopenta[c][1,7]naphthyridin-2-carboxamid;
4-Amino-3-methyl-N-(1-methylcyclopropyl)-N-((5-(trifluormethyl)-2-pyridinyl)methyl)-3H-pyrazolo[3,4-c]chinolin-8-carboxamid;
4-Amino-3-methyl-N-(1-methyl-1H-pyrazol-4-yl)-N-((5-(trifluormethyl)-2-pyridinyl)methyl)-3H-pyrazolo[3,4-c]chinolin-8-carboxamid;
4-Amino-7-fluoro-N-(2-propanyl)-N-((5-(trifluormethyl)-2-pyrazinyl)methyl)-1,3-dihydrofuro[3,4-c]chinolin-8-carboxamid;
4-Amino-N-ethyl-7-fluoro-N-((5-(trifluormethyl)-2-pyrazinyl)methyl)-1,3-dihydrofuro[3,4-c]chinolin-8-carboxamid;
4-Amino-7-chloro-N-ethyl-N-((5-(Trifluormethyl)-2-pyrazinyl)methyl)-1,3-dihydrofuro[3,4-c]chinolin-8-carboxamid;
4-Amino-7-chloro-N-(2-propanyl)-N-((5-(trifluormethyl)-2-pyrazinyl)methyl)-1,3-dihydrofuro[3,4-c]chinolin-8-carboxamid;
(3R)-4-Amino-N-ethyl-3-methyl-N-((5-(Trifluormethyl)-2-pyrazinyl)methyl)-1,3-dihydrofuro[3,4-c]chinolin-8-carboxamid;
4-Amino-N-cyclobutyl-7-fluoro-N-((6-(trifluormethyl)-3-pyridazinyl)methyl)-1,3-dihydrofuro[3,4-c]chinolin-8-carboxamid;
4-Amino-7-methoxy-N-methyl-N-((5-(trifluormethyl)-2-pyridinyl)methyl)-1,3-dihydrofuro[3,4-c]chinolin-8-carboxamid;
4-Amino-N,7-dimethyl-N-((5-(trifluormethyl)-2-pyridinyl)methyl)-1,3-dihydrofuro[3,4-c]chinolin-8-carboxamid;
4-Amino-N-cyclobutyl-7-fluoro-3-methyl-N-((6-(trifluormethyl)-3-pyridazinyl)methyl)-3H-pyrazolo[3,4-c]chinolin-8-carboxamid;
4-Amino-7-chloro-N-cyclobutyl-N-((6-(trifluormethyl)-3-pyridazinyl)methyl)-1,3-dihydrofuro[3,4-c]chinolin-8-carboxamid;
4-Amino-7-fluoro-3-methyl-N-(1-methyl-1H-pyrazol-4-yl)-N-((5-(trifluormethyl)-2-pyridinyl)methyl)-3H-pyrazolo[3,4-c]chinolin-8-carboxamid;
4-Amino-1-methyl-N-(1-methyl-1H-pyrazol-4-yl)-N-((5-(trifluormethyl)-2-pyridinyl)methyl)-1H-pyrazolo[4,3-c][1,7]naphthyridin-8-carboxamid;
4-Amino-N-cyclopropyl-7-fluoro-3-methyl-N-((1R)-1-(5-(Trifluormethyl)-2-pyridinyl)ethyl)-3H-pyrazolo[3,4-c]chinolin-8-carboxamid;
4-Amino-N-cyclobutyl-N-((6-(Difluormethoxy)-3-pyridazinyl)methyl)-7-fluoro-1,3-dihydrofuro[3,4-c]chinolin-8-carboxamid;
4-Amino-N-methyl-N-(4-(Pentafluoro-lambda~6~-sulfanyl)benzyl)-1,3-dihydrofuro[3,4-c]chinolin-8-carboxamid;
4-Amino-N-methyl-N-(4-(Pentafluoro-lambda~6~-sulfanyl)benzyl)-1,3-dihydrofuro[3,4-c][1,7]naphthyridin-8-carboxamid;
4-Amino-7-fluoro-N-methyl-N-(4-(pentafluoro-lambda~6~-sulfanyl)benzyl)-1,3-dihydrofuro[3,4-c]chinolin-8-carboxamid;
4-Amino-7-chloro-N-methyl-N-(4-(Pentafluoro-lambda~6~-sulfanyl)benzyl)-1,3-dihydrofuro[3,4-c]chinolin-8-carboxamid;
(3R)-4-Amino-N,3-dimethyl-N-(4-(pentafluoro-lambda~6~-sulfanyl)benzyl)-1,3-dihydrofuro[3,4-c]chinolin-8-carboxamid;
4-Amino-7-fluoro-N,3-dimethyl-N-(4-(pentafluoro-lambda-6~-sulfanyl)benzyl)-3H-pyrazolo[3,4-c]chinolin-8-carboxamid;
4-Amino-N-((5-fluoro-2-pyridinyl)methyl)-N,1,7-trimethyl-1H-pyrazolo[4,3-c]chinolin-8-carboxamid;
4-Amino-N-((6-cyclopropyl-3-pyridazinyl)methyl)-7-fluoro-N,3-dimethyl-3H-pyrazolo[3,4-c]chinolin-8-carboxamid;
4-Amino-N-((6-cyclopropyl-3-pyridazinyl)methyl)-7-fluoro-N,1-dimethyl-1H-pyrazolo[4,3-c]chinolin-8-carboxamid;
4-Amino-7-chloro-N-((6-cyclopropyl-3-pyridazinyl)methyl)-N-methyl-1,3-dihydrofuro[3,4-c]chinolin-8-carboxamid;
4-Amino-N,3,7-trimethyl-N-((5-(trifluormethyl)-2-pyridinyl)methyl)-3H-pyrazolo[3,4-c]chinolin-8-carboxamid;
4-Amino-N,1,7-trimethyl-N-((5-(trifluormethyl)-2-pyridinyl)methyl)-1H-pyrazolo[4,3-c]chinolin-8-carboxamid;
4-Amino-N-((5-Cyclopropyl-2-pyridinyl)methyl)-7-fluoro-3-methyl-N-(2-propanyl)-3H-pyrazolo[3,4-c]chinolin-8-carboxamid;
4-Amino-N-((5-chloro-2-pyridinyl)methyl)-N,1-dimethyl-1H-pyrazolo[4,3-c]chinolin-8-carboxamid;
4-Amino-N-((5-(Difluormethyl)-2-pyridinyl)methyl)-N,1-dimethyl-1H-pyrazolo[4,3-c]chinolin-8-carboxamid;
4-Amino-N-((5-(difluormethyl)-2-pyridinyl)methyl)-7-fluoro-N,1-dimethyl-1H-pyrazolo[4,3-c]chinolin-8-carboxamid;
4-Amino-N-(3-fluoro-4-(trifluormethyl)benzyl)-N,1-dimethyl-1H-pyrazolo[4,3-c]chinolin-8-carboxamid;
4-Amino-N-((3-fluoro-5-(trifluormethyl)-2-pyridinyl)methyl)-N,1-dimethyl-1H-pyrazolo[4,3-c]chinolin-8-carboxamid;
4-Amino-7-fluoro-N-((3-fluoro-5-(trifluormethyl)-2-pyridinyl)methyl)-N-methyl-1,3-dihydrofuro[3,4-c]chinolin-8-carboxamid;
4-Amino-N,1-dimethyl-N-((6-(1-(trifluormethyl)-1H-pyrazol-4-yl)-3-pyridazinyl)methyl)-1H-pyrazolo[4,3-c]chinolin-8-carboxamid;
4-Amino-N,1-dimethyl-N-((6-(4-(trifluormethyl)phenyl)-3-pyridazinyl)methyl)-1H-pyrazolo[4,3-c]chinolin-8-carboxamid;
4-Amino-N,1-dimethyl-N-((1R)-1-(4'-(trifluormethyl)[biphenyl]-4-yl)ethyl)-1H-pyrazolo[4,3-c]chinolin-8-carboxamid;
4-Amino-N,1-dimethyl-N-((1R)-1-(6-(4-(trifluormethyl)phenyl)-3-pyridazinyl)ethyl)-1H-pyrazolo[4,3-c]chinolin-8-carboxamid;
4-Amino-N,1-dimethyl-N-((1R)-1-(4'-(pentafluoro-lambda~6~-sulfanyl)[biphenyl]-4-yl)ethyl)-1H-pyrazolo[4,3-c]chinolin-8-carboxamid;
4-Amino-N-(2-propanyl)-N-((5-(trifluormethyl)-2-pyridinyl)methyl)-2,3-dihydrofuro[3,2-c][1,7]naphthyridin-8-carboxamid;
4-Amino-N-(2-(5-chloro--2-pyridinyl)-2,2-difluorethyl)-N,1-dimethyl-1H-pyrazolo[4,3-c]chinolin-8-carboxamid;
4-Amino-7-fluoro-N-(2-propanyl)-N-((5-(trifluormethyl)-2-pyridinyl)methyl)-2,3-dihydrofuro[3,2-c]chinolin-8-carboxamid;
4-Amino-7-chloro-N-ethyl-N-((5-(trifluormethyl)-2-pyridinyl)methyl)-1,3-dihydrofuro[3,4-c][1,8]naphthyridin-8-carboxamid;
4-Amino-7-fluoro-1,3-dimethyl-N-(2-propanyl)-N-((5-(trifluormethyl)-2-pyridinyl)methyl)-1H-pyrazolo[4,3-c]chinolin-8-carboxamid;
4-Amino-N-((5-Cyclopropyl-2-pyridinyl)-methyl)-N-ethyl-1,3-dihydrofuro[3,4-c]chinolin-8-carboxamid;
4-Amino-N-((5-Cyclopropyl-2-pyridinyl)-methyl)-N-ethyl-1-methyl-1H-pyrazolo[4,3-c]chinolin-8-carboxamid;
4-Amino-N-((5-Cyclopropyl-2-pyridinyl)methyl)-N-ethyl-1-methyl-1H-pyrazolo[4,3-c][1,7]naphthyridin-8-carboxamid;
(3R)-4-Amino-N-((5-cyclopropyl-2-pyridinyl)methyl)-N-ethyl-3-methyl-1,3-dihydrofuro[3,4-c]chinolin-8-carboxamid;
4-Amino-N,1-dimethyl-N-(4-(trifluormethyl)benzyl)-1H-pyrazolo[4,3-c]chinolin-8-carboxamid;
4-Amino-N,1-dimethyl-N-(4-(trifluormethyl)benzyl)-1H-pyrazolo[4,3-c][1,7]naphthyridin-8-carboxamid;
4-Amino-N-((5-Cyclopropyl-2-pyridinyl)methyl)-N-ethyl-7-fluoro-1-methyl-1H-pyrazolo[4,3-c]chinolin-8-carboxamid;
4-Amino-N-ethyl-1-methyl-N-((5-(trifluormethyl)-2-pyridinyl)methyl)-1H-pyrazolo[4,3-c]chinolin-8-carboxamid;
4-Amino-N-ethyl-3-methyl-N-((5-(Trifluormethyl)-2-pyridinyl)methyl)[1,2]oxazolo[4,5-c]chinolin-8-carboxamid;
4-Amino-N-ethyl-1-methyl-N-((6-(Trifluormethyl)-3-pyridazinyl)methyl)-1H-pyrazolo[4,3-c]chinolin-8-carboxamid;
4-Amino-N-ethyl-1-methyl-N-((5-(trifluormethyl)-2-pyridinyl)methyl)-1H-pyrazolo[4,3-c][1,7]naphthyridin-8-carboxamid;
4-Amino-N-ethyl-1-methyl-N-((6-(Trifluormethyl)-3-pyridazinyl)methyl)-1H-pyrazolo[4,3-c][1,7]naphthyridin-8-carboxamid;
(3R)-4-Amino-N-ethyl-3-methyl-N-((6-(Trifluormethyl)-3-pyridazinyl)methyl)-1,3-dihydrofuro[3,4-c]chinolin-8-carboxamid;
4-Amino-7-fluoro-N,1-dimethyl-N-(4-(trifluormethyl)benzyl)-1H-pyrazolo[4,3-c]chinolin-8-carboxamid;
4-Amino-N-ethyl-1,3-dimethyl-N-((5-(trifluormethyl)-2-pyridinyl)methyl)-1H-pyrazolo[4,3-c][1,7]naphthyridin-8-carboxamid;
4-Amino-N-ethyl-7-fluoro-1-methyl-N-((5-(trifluormethyl)-2-pyridinyl)methyl)-1H-pyrazolo[4,3-c]chinolin-8-carboxamid;
4-Amino-N-ethyl-7-fluoro-1-methyl-N-((6-(trifluormethyl)-3-pyridazinyl)methyl)-1H-pyrazolo[4,3-c]chinolin-8-carboxamid;
(3R)-4-Amino-N-ethyl-7-fluoro-3-methyl-N-((5-(trifluormethyl)-2-pyridinyl)methyl)-1,3-dihydrofuro[3,4-c]chinolin-8-carboxamid;
(3S)-4-Amino-N-ethyl-7-fluoro-3-methyl-N-((5-(trifluormethyl)-2-pyridinyl)methyl)-1,3-dihydrofuro[3,4-c]chinolin-8-carboxamid;
4-Amino-7-chloro-N, 1-dimethyl-N-(4-(trifluormethyl)benzyl)-1H-pyrazolo[4,3-c]chinolin-8-carboxamid;
(3R)-4-Amino-N-ethyl-7-fluoro-3-methyl-N-((6-(trifluormethyl)-3-pyridazinyl)methyl)-1,3-dihydrofuro[3,4-c]chinolin-8-carboxamid;
4-Amino-N-ethyl-7-fluoro-1,3-dimethyl-N-((5-(trifluormethyl)-2-pyridinyl)methyl)-1H-pyrazolo[4,3-c]chinolin-8-carboxamid;
4-Amino-N,1-dimethyl-N-(4-(pentafluorethyl)benzyl)-1H-pyrazolo[4,3-c]chinolin-8-carboxamid;
4-Amino-N,1-dimethyl-N-(4-(pentafluorethyl)benzyl)-1H-pyrazolo[4,3-c][1,7]naphthyridin-8-carboxamid;
4-Amino-7-fluoro-N,1-dimethyl-N-(4-(pentafluorethyl)benzyl)-1H-pyrazolo[4,3-c]chinolin-8-carboxamid;
4-Amino-N-ethyl-1-methyl-N-(4-(Pentafluoro-Lambda-6~-sulfanyl)benzyl)-1H-pyrazolo[4,3-c]chinolin-8-carboxamid;
4-Amino-N-ethyl-1-methyl-N-(4-(Pentafluoro-lambda~6~-sulfanyl)benzyl)-1H-pyrazolo[4,3-c][1,7]naphthyridin-8-carboxamid;
4-Amino-N-ethyl-7-fluoro-1-methyl-N-(4-(pentafluoro-lambda~6~-sulfanyl)benzyl)-1H-pyrazolo[4,3-c]chinolin-8-carboxamid;
4-Amino-N,1-dimethyl-N-((5-(trifluormethyl)-2-pyridinyl)methyl)-1H-pyrazolo[4,3-c]chinolin-8-carboxamid;
4-Amino-N,1-dimethyl-N-((5-(trifluormethyl)-2-pyridinyl)methyl)-1H-pyrazolo[4,3-c][1,7]naphthyridin-8-carboxamid;
4-Amino-N-cyclopropyl-1-methyl-N-((5-(trifluormethyl)-2-pyridinyl)methyl)-1H-pyrazolo[4,3-c]chinolin-8-carboxamid;
4-Amino-N-cyclopropyl-7-fluoro-1-(2,2,2-trifluorethyl)-N-((5-(trifluormethyl)-2-pyridinyl)methyl)-1H-pyrazolo[4,3-c]chinolin-8-carboxamid;
4-Amino-7-fluoro-N,1-dimethyl-N-((6-(trifluormethyl)-3-pyridazinyl)methyl)-1H-pyrazolo[4,3-c]chinolin-8-carboxamid;
4-Amino-N-methyl-7-(trifluormethyl)-N-((5-(trifluormethyl)-2-pyridinyl)methyl)-1,3-dihydrofuro[3,4-c]chinolin-8-carboxamid;
4-Amino-N,1-dimethyl-N-(4-(pentafluoro-lambda-6~-sulfanyl)benzyl)-1H-pyrazolo[4,3-c]chinolin-8-carboxamid;
4-Amino-N,1-dimethyl-N-(4-(Pentafluoro-lambda~6~-sulfanyl)benzyl)-1H-pyrazolo[4,3-c][1,7]naphthyridin-8-carboxamid;
4-Amino-7-fluoro-N,1-dimethyl-N-(4-(pentafluoro-lambda-6~-sulfanyl)benzyl)-1H-pyrazolo[4,3-c]chinolin-8-carboxamid;
4-Amino-7-fluoro-N-(2-hydroxy-4-(pentafluoro-lambda~6~-sulfanyl)benzyl)-N-methyl-1,3-dihydrofuro[3,4-c]chinolin-8-carboxamid;
4-Amino-N-((5-Cyano-2-pyridinyl)methyl)-N-((2R)-1-fluoro-2-propanyl)-1,3-dihydrofuro[3,4-c]chinolin-8-carboxamid;
4-Amino-N-((5-Cyano-2-pyridinyl)methyl)-N-((2S)-1-fluoro-2-propanyl)-1,3-dihydrofuro[3,4-c]chinolin-8-carboxamid;
(3R)-4-Amino-3-(hydroxymethyl)-N-(2-methylpropyl)-N-((5-(trifluormethyl)-2-pyridinyl)methyl)-1,3-dihydrofuro[3,4-c]chinolin-8-carboxamid;
(3S)-4-Amino-3-(hydroxymethyl)-N-(2-methylpropyl)-N-((5-(trifluormethyl)-2-pyridinyl)methyl)-1,3-dihydrofuro[3,4-c]chinolin-8-carboxamid;
4-Amino-N-((1R)-2-cyano-1-cyclopropylethyl)-N-((5-cyano-2-pyridinyl)methyl)-1,3-dihydrofuro[3,4-c]chinolin-8-carboxamid;
4-Amino-N-((1S)-2-cyano-1-cyclopropylethyl)-N-((5-cyano-2-pyridinyl)methyl)-1,3-dihydrofuro[3,4-c]chinolin-8-carboxamid;
4-Amino-N-((5-Cyano-6-methyl-2-pyridinyl)methyl)-N-((3R,4R)-4-methoxytetrahydro-2H-pyran-3-yl)-1,3-dihydrofuro[3,4-c]chinolin-8-carboxamid;
4-Amino-N-((5-Cyano-6-methyl-2-pyridinyl)methyl)-N-((3S,4S)-4-methoxytetrahydro-2H-pyran-3-yl)-1,3-dihydrofuro[3,4-c]chinolin-8-carboxamid;
4-Amino-N-((3S,4R)-3-Methoxytetrahydro-2H-pyran-4-yl)-N-((5-(Trifluormethyl)-2-pyridinyl)methyl)-1,3-dihydrofuro[3,4-c]chinolin-8-carboxamid;
4-Amino-N-((3R,4S)-3-methoxytetrahydro-2H-pyran-4-yl)-N-((5-(trifluormethyl)-2-pyridinyl)methyl)-1,3-dihydrofuro[3,4-c]chinolin-8-carboxamid;
4-Amino-N-((3R,4R)-4-Methoxytetrahydro-2H-pyran-3-yl)-N-((5-(Trifluormethyl)-2-pyridinyl)methyl)-1,3-dihydrofuro[3,4-c]chinolin-8-carboxamid;
4-Amino-N-((3S,4S)-4-Methoxytetrahydro-2H-pyran-3-yl)-N-((5-(Trifluormethyl)-2-pyridinyl)methyl)-1,3-dihydrofuro[3,4-c]chinolin-8-carboxamid;
4-Amino-N-((5-Cyano-6-methyl-2-pyridinyl)methyl)-N-((3R,4R)-4-methoxytetrahydro-2H-pyran-3-yl)-1,3-dihydrofuro[3,4-c]chinolin-8-carboxamid;
(3R)-4-Amino-N-cyclopropyl-3-methyl-N-((1R)-1-(5-(Trifluormethyl)-2-pyridinyl)ethyl)-1,3-dihydrofuro[3,4-c][1,7]naphthyridin-8-carboxamid;
(3S)-4-Amino-N-cyclopropyl-3-methyl-N-((1R)-1-(5-(Trifluormethyl)-2-pyridinyl)ethyl)-1,3-dihydrofuro[3,4-c][1,7]naphthyridin-8-carboxamid;
4-Amino-N-((1S)-1-(5-fluoro-2-pyridinyl)ethyl)-N,1,7-trimethyl-1H-pyrazolo[4,3-c]chinolin-8-carboxamid;
4-Amino-N-((1R)-1-(5-fluoro-2-pyridinyl)ethyl)-N,1,7-trimethyl-1H-pyrazolo[4,3-c]chinolin-8-carboxamid;
4-Amino-7-chloro-N-((1R)-1-(5-fluoro-2-pyridinyl)ethyl)-N,1-dimethyl-1H-pyrazolo[4,3-c]chinolin-8-carboxamid;
4-Amino-7-chloro-N-((1S)-1-(5-fluoro-2-pyridinyl)ethyl)-N,1-dimethyl-1H-pyrazolo[4,3-c]chinolin-8-carboxamid;
4-Amino-7-fluoro-N-((1R)-1-(3-fluoro-5-(trifluormethyl)-2-pyridinyl)ethyl)-N-methyl-1,3-dihydrofuro[3,4-c]chinolin-8-carboxamid;
(3R)-4-Amino-N-ethyl-3-(fluormethyl)-N-((5-(trifluormethyl)-2-pyridinyl)methyl)-1,3-dihydrofuro[3,4-c]chinolin-8-carboxamid;
(3R)-4-Amino-N-((5-bromo-2-pyridinyl)methyl)-N,3-dimethyl-1,3-dihydrofuro[3,4-c][1,7]naphthyridin-8-carboxamid;
4-Amino-N,1-dimethyl-N-((1R)-1-(4-(trifluormethyl)phenyl)ethyl)-1H-pyrazolo[4,3-c][1,7]naphthyridin-8-carboxamid;
4-Amino-N,1-dimethyl-N-((1S)-1-(4-(trifluormethyl)phenyl)ethyl)-1H-pyrazolo[4,3-c][1,7]naphthyridin-8-carboxamid;
4-Amino-N,1-dimethyl-N-((1R)-1-(5-(Trifluormethyl)-2-pyridinyl)ethyl)-1H-pyrazolo[4,3-c]chinolin-8-carboxamid;
4-Amino-N,1-dimethyl-N-((1S)-1-(5-(trifluormethyl)-2-pyridinyl)ethyl)-1H-pyrazolo[4,3-c]chinolin-8-carboxamid;
4-Amino-7-fluoro-N,1-dimethyl-N-((1R)-1-(5-(trifluormethyl)-2-pyridinyl)ethyl)-1H-pyrazolo[4,3-c]chinolin-8-carboxamid;
4-Amino-7-fluoro-N,1-dimethyl-N-((1S)-1-(5-(trifluormethyl)-2-pyridinyl)ethyl)-1H-pyrazolo[4,3-c]chinolin-8-carboxamid;
4-Amino-N-((1R)-1-(5-fluoro-2-pyridinyl)ethyl)-N,1-dimethyl-1H-pyrazolo[4,3-c]chinolin-8-carboxamid;
4-Amino-7-fluoro-N,1-dimethyl-N-((1R)-1-(6-(trifluormethyl)-3-pyridazinyl)ethyl)-1H-pyrazolo[4,3-c]chinolin-8-carboxamid;
4-Amino-N-((5-Cyano-2-pyridinyl)methyl)-N-((1R)-1-(2-pyrimidinyl)ethyl)-1,3-dihydrofuro[3,4-c][1,7]naphthyridin-8-carboxamid;
6-Amino-N-(2-methylpropyl)-N-((5-(trifluormethyl)-2-pyridinyl)methyl)-8,9-dihydro-7H-cyclopenta[c][1,8]naphthyridin-2-carboxamid;
4-Amino-N-(cyclopropylmethyl)-N-((5-(trifluormethyl)-2-pyridinyl)methyl)-1,3-dihydrofuro[3,4-c][1,8]naphthyridin-8-carboxamid;
4-Amino-N-((2R)-1-methoxy-2-propanyl)-N-((5-(trifluormethyl)-2-pyridinyl)methyl)-1,3-dihydrofuro[3,4-c][1,8]naphthyridin-8-carboxamid;
4-Amino-N-((1R)-1-(2-pyrimidinyl)ethyl)-N-((5-(trifluormethyl)-2-pyridinyl)methyl)-2,3-dihydro-1H-cyclopenta[c]chinolin-8-carboxamid;
4-Amino-N-(2-methylpropyl)-N-((5-(trifluormethyl)-2-pyridinyl)methyl)-2,3-dihydro-1H-cyclopenta[c]chinolin-8-carboxamid;
4-Amino-N-(2-methylpropyl)-N-((5-(trifluormethyl)-2-pyridinyl)methyl)-1,3-dihydrofuro[3,4-c][1,8]naphthyridin-8-carboxamid;
4-Amino-N-(2-methylpropyl)-N-((5-(trifluormethyl)-2-pyridinyl)methyl)-1,3-dihydrofuro[3,4-c]chinolin-8-carboxamid;
4-Amino-N-((1R)-1-(2-pyrimidinyl)ethyl)-N-((5-(trifluormethyl)-2-pyridinyl)methyl)-1,3-dihydrofuro[3,4-c][1,8]naphthyridin-8-carboxamid;
4-Amino-N-((5-Cyano-2-pyridinyl)methyl)-N-((1R)-1-(2-pyrimidinyl)ethyl)-2,3-dihydro-1H-cyclopenta[c]chinolin-8-carboxamid;
4-Amino-N-((5-Cyano-2-pyridinyl)methyl)-N-((1R)-1-(2-pyrimidinyl)ethyl)-1,3-dihydrofuro[3,4-c]chinolin-8-carboxamid;
4-Amino-N-((5-Cyano-2-pyridinyl)methyl)-N-((1S)-1-(2-pyrimidinyl)ethyl)-1,3-dihydrofuro[3,4-c]chinolin-8-carboxamid;
4-Amino-3-methyl-N-(2-methylpropyl)-N-((5-(trifluormethyl)-2-pyridinyl)methyl)[1,2]oxazolo[4,5-c]chinolin-8-carboxamid;
4-Amino-3-methyl-N-((1R)-1-(2-pyrimidinyl)ethyl)-N-((5-(trifluormethyl)-2-pyridinyl)methyl)-3H-pyrazolo[3,4-c]chinolin-8-carboxamid;
4-Amino-3-methyl-N-((1S)-1-(2-pyrimidinyl)ethyl)-N-((5-(trifluormethyl)-2-pyridinyl)methyl)-3H-pyrazolo[3,4-c]chinolin-8-carboxamid;
4-Amino-3-methyl-N-((1R)-1-(2-pyrimidinyl)ethyl)-N-((5-(trifluormethyl)-2-pyridinyl)methyl)[1,2]oxazolo[4,5-c]chinolin-8-carboxamid;
4-Amino-3-methyl-N-((1S)-1-(2-pyrimidinyl)ethyl)-N-((5-(trifluormethyl)-2-pyridinyl)methyl)[1,2]oxazolo[4,5-c]chinolin-8-carboxamid;
4-Amino-N-(2-methylpropyl)-N-((5-(trifluormethyl)-2-pyridinyl)methyl)-1,3-dihydrofuro[3,4-c][1,7]naphthyridin-8-carboxamid;
4-Amino-N-((1R)-1-(2-pyrimidinyl)ethyl)-N-((5-(trifluormethyl)-2-pyridinyl)methyl)-1,3-dihydrofuro[3,4-c][1,7]naphthyridin-8-carboxamid;
4-Amino-N-((5-chloro--2-pyridinyl)methyl)-N-((1R)-1-(2-pyrimidinyl)ethyl)-2,3-dihydro-1H-cyclopenta[c]chinolin-8-carboxamid;
4-Amino-N-((5-(Difluormethyl)-2-pyridinyl)methyl)-N-((1R)-1-(2-pyrimidinyl)ethyl)-2,3-dihydro-1H-cyclopenta[c]chinolin-8-carboxamid;
4-Amino-N-((6-bromo-3-pyridazinyl)methyl)-N-((1R)-1-(3-fluoro-2-pyridinyl)ethyl)-2,3-dihydro-1H-cyclopenta[c]chinolin-8-carboxamid;
6-Amino-N-((5-(difluormethyl)-2-pyridinyl)methyl)-N-((1R)-1-(2-pyrimidinyl)ethyl)-8,9-dihydro-7H-cyclopenta[c][1,8]naphthyridin-2-carboxamid;
6-Amino-N-((1R)-1-(2-Pyrimidinyl)ethyl)-N-((5-(Trifluormethyl)-2-pyridinyl)methyl)-8,9-dihydro-7H-cyclopenta[c][1,8]naphthyridin-2-carboxamid;
6-Amino-N-((5-chloro-2-pyridinyl)methyl)-N-((1R)-1-(2-pyrimidinyl)ethyl)-8,9-dihydro-7H-cyclopenta[c][1,8]naphthyridin-2-carboxamid;
6-Amino-N-((1R)-1-(3-fluoro-2-pyridinyl)ethyl)-N-((6-(Trifluormethyl)-3-pyridazinyl)methyl)-8,9-dihydro-7H-cyclopenta[c][1,8]naphthyridin-2-carboxamid;
6-Amino-N-((1R)-1-(2-Pyrimidinyl)ethyl)-N-((5-(Trifluormethyl)-2-pyridinyl)methyl)-2-phenanthridin-carboxamid;
6-Amino-N-((5-Cyano-2-pyridinyl)methyl)-N-((1R)-1-(2-pyrimidinyl)ethyl)-2-phenanthridincarboxamid;
5-Amino-N-(2-pyrimidinylmethyl)-N-((5-(trifluormethyl)-2-pyridinyl)methyl)benzo[c][2,6]naphthyridin-9-carboxamid;
5-Amino-N-(2-methylpropyl)-N-((5-(trifluormethyl)-2-pyridinyl)methyl)benzo[c][2,6]naphthyridin-9-carboxamid;
5-Amino-N-((1R)-1-(2-pyrimidinyl)ethyl)-N-((5-(trifluormethyl)-2-pyridinyl)methyl)benzo[c][2,6]naphthyridin-9-carboxamid;
4-Amino-N-((2R)-1-methoxy-2-propanyl)-3-methyl-N-((5-(trifluormethyl)-2-pyridinyl)methyl)[1,2]oxazolo[4,5-c]chinolin-8-carboxamid;
4-Amino-N-((2S)-1-methoxy-2-propanyl)-3-methyl-N-((5-(trifluormethyl)-2-pyridinyl)methyl)[1,2]oxazolo[4,5-c]chinolin-8-carboxamid;
4-Amino-N-((3-fluoro-2-pyridinyl)methyl)-N-((5-(trifluormethyl)-2-pyridinyl)methyl)thieno[2,3-c]chinolin-8-carboxamid;
4-Amino-N-(2-pyrimidinylmethyl)-N-((5-(trifluormethyl)-2-pyridinyl)methyl)thieno[2,3-c]chinolin-8-carboxamid;
4-Amino-N-(2-methylpropyl)-N-((5-(trifluormethyl)-2-pyridinyl)methyl)thieno[2,3-c]chinolin-8-carboxamid;
5-Amino-N-((1R)-1-(2-pyrimidinyl)ethyl)-N-((5-(trifluormethyl)-2-pyridinyl)methyl)pyrimido[4,5-c]chinolin-9-carboxamid;
4-Amino-N-((6-bromo-3-pyridazinyl)methyl)-N-((1R)-1-cyclopropyl-2-methoxyethyl)thieno[2,3-c]chinolin-8-carboxamid;
4-Amino-N-((6-bromo-3-pyridazinyl)methyl)-N-((1S)-1-cyclopropyl-2-methoxyethyl)thieno[2,3-c]chinolin-8-carboxamid;
4-Amino-N-((1R)-1-(2-Pyrimidinyl)ethyl)-N-((5-(Trifluormethyl)-2-pyridinyl)methyl)-1,3-dihydrofuro[3,4-c]chinolin-8-carboxamid;
4-Amino-N-((1S)-1-(2-pyrimidinyl)ethyl)-N-((5-(trifluormethyl)-2-pyridinyl)methyl)-1,3-dihydrofuro[3,4-c]chinolin-8-carboxamid;
4-Amino-N-((1S)-1-cyclopropyl-2-methoxyethyl)-N-((6-(4-morpholinyl)-3-pyridazinyl)methyl)-1,3-dihydrofuro[3,4-c]chinolin-8-carboxamid;
4-Amino-N-((6-ethoxy-3-pyridazinyl)methyl)-N-(2-methylpropyl)-1,3-dihydrofuro[3,4-c]chinolin-8-carboxamid;
4-Amino-N-((6-bromo-3-pyridazinyl)methyl)-N-((2R)-1-methoxy-2-propanyl)thieno[2,3-c]chinolin-8-carboxamid;
4-Amino-N-((6-bromo-3-pyridazinyl)methyl)-N-(1-methoxy-2-methyl-2-propanyl)-1,3-dihydrofuro[3,4-c]chinolin-8-carboxamid;
4-Amino-N-((6-bromo-3-pyridazinyl)methyl)-N-(1-methoxy-2-methyl-2-propanyl)-1,3-dihydrofuro[3,4-c][1,7]naphthyridin-8-carboxamid;
4-Amino-N-((6-methoxy-3-pyridazinyl)methyl)-N-((1R)-1-(2-pyrimidinyl)ethyl)-2,3-dihydro-1H-cyclopenta[c]chinolin-8-carboxamid;
4-Amino-N-((6-methoxy-3-pyridazinyl)methyl)-N-((1S)-1-(2-pyrimidinyl)ethyl)-2,3-dihydro-1H-cyclopenta[c]chinolin-8-carboxamid;
4-Amino-N-((1S)-1-(2-Pyrimidinyl)ethyl)-N-((6-(Trifluormethyl)-3-pyridazinyl)methyl)-2,3-dihydro-1H-cyclopenta[c]chinolin-8-carboxamid;
4-Amino-N-((1R)-1-(2-pyrimidinyl)ethyl)-N-((6-(Trifluormethyl)-3-pyridazinyl)methyl)-2,3-dihydro-1H-cyclopenta[c]chinolin-8-carboxamid;
4-Amino-N-((6-bromo-3-pyridazinyl)methyl)-N-((1S)-1-cyclopropyl-2-methoxyethyl)thieno[2,3-c]chinolin-8-carboxamid;
4-Amino-N-((6-bromo-3-pyridazinyl)methyl)-N-((1S)-1-cyclopropyl-2-methoxyethyl)-1,3-dihydrofuro[3,4-c][1,8]naphthyridin-8-carboxamid;
4-Amino-N-(cyclopropylmethyl)-N-((5-(trifluormethyl)-2-pyridinyl)methyl)-1,3-dihydrofuro[3,4-c][1,7]naphthyridin-8-carboxamid;
4-Amino-N-((1R)-1-(2-pyrimidinyl)ethyl)-N-((5-(Trifluormethyl)-2-pyridinyl)methyl)-1,3-dihydrofuro[3,4-c]chinolin-8-carboxamid;
6-Amino-N-((1R)-1-(2-pyrimidinyl)ethyl)-N-((5-(trifluormethyl)-2-pyridinyl)methyl)-7,8,9, 10-tetrahydro-2-phenanthridincarboxamid;
4-Amino-7-chloro-N-((1R)-1-(2-pyrimidinyl)ethyl)-N-((5-(trifluormethyl)-2-pyridinyl)methyl)-1,3-dihydrofuro[3,4-c]chinolin-8-carboxamid;
4-Amino-7-chloro-N-cyclopropyl-N-((5-(trifluormethyl)-2-pyridinyl)methyl)-1,3-dihydrofuro[3,4-c]chinolin-8-carboxamid;
4-Amino-N-((6-bromo-3-pyridazinyl)methyl)-N-((1R)-1-(2-pyrimidinyl)ethyl)-1,3-dihydrofuro[3,4-c]chinolin-8-carboxamid;
4-Amino-N-((5-bromo-2-pyridinyl)methyl)-N-((1R)-1-(2-pyrimidinyl)ethyl)-1,3-dihydrofuro[3,4-c]chinolin-8-carboxamid;
4-Amino-N-((5-Cyano-2-pyridinyl)methyl)-N-((1R)-1-(2-pyrimidinyl)ethyl)-1,3-dihydrofuro[3,4-c]chinolin-8-carboxamid;
4-Amino-N-(3,3-difluorcyclobutyl)-N-((6-(trifluormethyl)-3-pyridazinyl)methyl)-1,3-dihydrofuro[3,4-c]chinolin-8-carboxamid;
4-Amino-N-cyclopropyl-N-((6-(Trifluormethyl)-3-pyridazinyl)methyl)-1,3-dihydrofuro[3,4-c]chinolin-8-carboxamid;
4-Amino-N-((5-(Difluormethyl)-2-pyridinyl)methyl)-N-((1R)-1-(1-Methyl-1H-1,2,4-triazol-3-yl)ethyl)-1,3-dihydrofuro[3,4-c]chinolin-8-carboxamid;
4-Amino-N-(cyclopropylmethyl)-N-((5-(trifluormethoxy)-2-pyridinyl)methyl)-1,3-dihydrofuro[3,4-c]chinolin-8-carboxamid;
4-Amino-N-(2-methylpropyl)-N-((6-(trifluormethyl)-3-pyridazinyl)methyl)-1,3-dihydrofuro[3,4-c]chinolin-8-carboxamid;
4-Amino-N-((6-bromo-3-pyridazinyl)methyl)-N-(2-methylpropyl)-1,3-dihydrofuro[3,4-c]chinolin-8-carboxamid;
4-Amino-N-((5-Cyano-2-pyridinyl)methyl)-N-((1S)-1-(2-pyrimidinyl)ethyl)-1,3-dihydrofuro[3,4-c]chinolin-8-carboxamid;
4-Amino-N-((5-(Difluormethyl)-2-pyridinyl)methyl)-N-((1R)-1-(1-Methyl-1H-1,2,4-triazol-3-yl)ethyl)-1,3-dihydrofuro[3,4-c][1,7]naphthyridin-8-carboxamid;
4-Amino-N-(3,3-difluorcyclobutyl)-N-((6-(trifluormethyl)-3-pyridazinyl)methyl)-1,3-dihydrofuro[3,4-c][1,7]naphthyridin-8-carboxamid;
4-Amino-N-cyclobutyl-N-((6-(trifluormethyl)-3-pyridazinyl)methyl)-1,3-dihydrofuro[3,4-c]chinolin-8-carboxamid;
4-Amino-N-(2-methylpropyl)-N-((6-(trifluormethyl)-3-pyridazinyl)-methyl)-1,3-dihydrofuro[3,4-c][1,7]naphthyridin-8-carboxamid;
4-Amino-N-((6-bromo-3-pyridazinyl)methyl)-N-(cyclopropylmethyl)-1,3-dihydrofuro[3,4-c][1,7]naphthyridin-8-carboxamid;
4-Amino-N-((6-ethoxy-3-pyridazinyl)methyl)-N-(2-methylpropyl)-1,3-dihydrofuro[3,4-c][1,7]naphthyridin-8-carboxamid;
4-Amino-N-((6-bromo-3-pyridazinyl)methyl)-N-((1R)-1-(2-pyrimidinyl)ethyl)-1,3-dihydrofuro[3,4-c][1,7]naphthyridin-8-carboxamid;
4-Amino-N-((6-bromo-3-pyridazinyl)methyl)-N-(cyclopropylmethyl)-1,3-dihydrofuro[3,4-c]chinolin-8-carboxamid;
4-Amino-N-(2-propanyl)-N-((5-(trifluormethyl)-2-pyridinyl)methyl)-1,3-dihydrofuro[3,4-c][1,7]naphthyridin-8-carboxamid;
4-Amino-N-((6-chloro-3-pyridazinyl)methyl)-N-((1R)-1-(2-pyrimidinyl)ethyl)-1,3-dihydrofuro[3,4-c]chinolin-8-carboxamid;
4-Amino-N-((5-bromo-6-methyl-2-pyridinyl)methyl)-N-methyl-1,3-dihydrofuro[3,4-c]chinolin-8-carboxamid;
4-Amino-N-(2-propanyl)-N-((5-(trifluormethoxy)-2-pyridinyl)methyl)-1,3-dihydrofuro[3,4-c]chinolin-8-carboxamid;
4-Amino-N-((6-chloro-5-cyano-2-pyridinyl)methyl)-N-((1R)-1-(2-pyrimidinyl)ethyl)-1,3-dihydrofuro[3,4-c]chinolin-8-carboxamid;
4-Amino-N-((6-bromo-3-pyridazinyl)methyl)-N-((2R)-1-methoxy-2-propanyl)-1,3-dihydrofuro[3,4-c][1,7]naphthyridin-8-carboxamid;
4-Amino-N-((6-bromo-3-pyridazinyl)methyl)-N-((2R)-1-methoxy-2-propanyl)-1,3-dihydrofuro[3,4-c][1,8]naphthyridin-8-carboxamid;
4-Amino-N-((5-Cyano-2-pyridinyl)methyl)-N-(cyclopropylmethyl)-1,3-dihydrofuro[3,4-c][1,7]naphthyridin-8-carboxamid;
4-Amino-N-((6-chloro-5-methoxy-2-pyridinyl)methyl)-N-(2-propanyl)-1,3-dihydrofuro[3,4-c][1,7]naphthyridin-8-carboxamid;
4-Amino-N-((6-bromo-3-pyridazinyl)methyl)-N-(2-propanyl)-1,3-dihydrofuro[3,4-c][1,7]naphthyridin-8-carboxamid;
4-Amino-N-((5-chloro--6-methoxy-2-pyridinyl)methyl)-N-(2-propanyl)-1,3-dihydrofuro[3,4-c][1,7]naphthyridin-8-carboxamid;
4-amino-N-(3,4-dihydro-2H-pyrano[2,3-c]pyridin-6-ylmethyl)-N-((1R)-1-(2-pyrimidinyl)ethyl)-1,3-dihydrofuro[3,4-c][1,7]naphthyridine-8-carboxamide;
4-Amino-N-((5-Cyano-2-pyridinyl)methyl)-N-((2R)-1-fluoro-2-propanyl)-1,3-dihydrofuro[3,4-c][1,7]naphthyridin-8-carboxamid;
4-Amino-N-((5-Cyano-2-pyridinyl)methyl)-N-((2S)-1-fluoro-2-propanyl)-1,3-dihydrofuro[3,4-c][1,7]naphthyridin-8-carboxamid;
4-Amino-N-(cyclopropylmethyl)-N-((6-(trifluormethyl)-3-pyridazinyl)methyl)-1,3-dihydrofuro[3,4-c][1,7]naphthyridin-8-carboxamid;
4-Amino-N-(bicyclo[1.1.1]pentan-1-yl)-N-((5-(trifluormethyl)-2-pyridinyl)methyl)-1,3-dihydrofuro[3,4-c][1,7]naphthyridin-8-carboxamid;
4-Amino-N-(1-cyclopropyl-1H-pyrazol-4-yl)-N-((5-(trifluormethyl)-2-pyridinyl)methyl)-1,3-dihydrofuro[3,4-c][1,7]naphthyridin-8-carboxamid;
4-Amino-N-((5-(Difluormethyl)-2-pyridinyl)methyl)-N-(1-methyl-1H-pyrazol-4-yl)-1,3-dihydrofuro[3,4-c][1,7]naphthyridin-8-carboxamid;
4-Amino-N-methyl-N-(4-(trifluormethyl)benzyl)-1,3-dihydrofuro[3,4-c][1,7]naphthyridin-8-carboxamid;
4-Amino-N-methyl-N-((6-(Trifluormethyl)-3-pyridinyl)methyl)-1,3-dihydrofuro[3,4-c][1,7]naphthyridin-8-carboxamid;
4-Amino-N-((5-chloro-2-pyridinyl)methyl)-N-methyl-1,3-dihydrofuro[3,4-c][1,7]naphthyridin-8-carboxamid;
4-Amino-N-ethyl-N-((6-(Trifluormethyl)-3-pyridazinyl)methyl)-1,3-dihydrofuro[3,4-c]chinolin-8-carboxamid;
4-Amino-N-ethyl-N-((6-(Trifluormethyl)-3-pyridazinyl)methyl)-1,3-dihydrofuro[3,4-c][1,7]naphthyridin-8-carboxamid;
4-Amino-N-Methyl-N-((1R)-1-(5-(Trifluormethyl)-2-pyridinyl)ethyl)-1,3-dihydrofuro[3,4-c]chinolin-8-carboxamid;
4-Amino-N-methyl-N-((1S)-1-(5-(Trifluormethyl)-2-pyridinyl)ethyl)-1,3-dihydrofuro[3,4-c]chinolin-8-carboxamid;
4-Amino-N-((5-chloro--2-pyridinyl)-methyl)-N-ethyl-1,3-dihydrofuro[3,4-c][1,7]naphthyridin-8-carboxamid;
4-Amino-N-(1-methylcyclopropyl)-N-((5-(trifluormethyl)-2-pyridinyl)methyl)-1,3-dihydrofuro[3,4-c][1,7]naphthyridin-8-carboxamid;
4-Amino-N-Methyl-N-((1R)-1-(5-(Trifluormethyl)-2-pyridinyl)ethyl)-1,3-dihydrofuro[3,4-c][1,8]naphthyridin-8-carboxamid;
4-Amino-N-methyl-N-((1S)-1-(5-(Trifluormethyl)-2-pyridinyl)ethyl)-1,3-dihydrofuro[3,4-c][1,8]naphthyridin-8-carboxamid;
4-Amino-N-((5-chloro--2-pyridinyl)-methyl)-N-ethyl-1,3-dihydrofuro[3,4-c]chinolin-8-carboxamid;
4-Amino-N-((5-chloro--2-pyridinyl)methyl)-N-methyl-1,3-dihydrofuro[3,4-c]chinolin-8-carboxamid;
4-Amino-N-ethyl-N-((6-(Trifluormethyl)-3-pyridinyl)methyl)-1,3-dihydrofuro[3,4-c]chinolin-8-carboxamid;
4-Amino-N-ethyl-N-((6-(Trifluormethyl)-3-pyridinyl)methyl)-1,3-dihydrofuro[3,4-c][1,7]naphthyridin-8-carboxamid;
4-Amino-N-ethyl-N-((1R)-1-(5-(Trifluormethyl)-2-pyridinyl)ethyl)-1,3-dihydrofuro[3,4-c][1,8]naphthyridin-8-carboxamid;
4-Amino-N-ethyl-N-((1S)-1-(5-(Trifluormethyl)-2-pyridinyl)ethyl)-1,3-dihydrofuro[3,4-c][1,8]naphthyridin-8-carboxamid;
4-Amino-N-ethyl-N-(4-(Trifluormethyl)benzyl)-1,3-dihydrofuro[3,4-c]chinolin-8-carboxamid;
4-Amino-N-ethyl-N-(4-(Trifluormethyl)benzyl)-1,3-dihydrofuro[3,4-c][1,7]naphthyridin-8-carboxamid;
4-Amino-N-((6-bromo-3-pyridazinyl)methyl)-N-ethyl-1,3-dihydrofuro[3,4-c]chinolin-8-carboxamid;
4-Amino-N-Methyl-N-((1R)-1-(5-(Trifluormethyl)-2-pyridinyl)ethyl)-1,3-dihydrofuro[3,4-c][1,7]naphthyridin-8-carboxamid;
4-Amino-N-methyl-N-((1S)-1-(5-(Trifluormethyl)-2-pyridinyl)ethyl)-1,3-dihydrofuro[3,4-c][1,7]naphthyridin-8-carboxamid;
4-Amino-N-((6-bromo-3-pyridazinyl)methyl)-N-ethyl-1,3-dihydrofuro[3,4-c][1,7]naphthyridin-8-carboxamid;
4-Amino-N-(1-methyl-1H-pyrazol-4-yl)-N-((6-(trifluormethyl)-3-pyridazinyl)methyl)-1,3-dihydrofuro[3,4-c][1,7]naphthyridin-8-carboxamid;
4-Amino-N-(2,2,2-trifluorethyl)-N-((6-(trifluormethyl)-3-pyridazinyl)methyl)-1,3-dihydrofuro[3,4-c][1,7]naphthyridin-8-carboxamid;
4-Amino-N-(3-oxetanyl)-N-((6-(Trifluormethyl)-3-pyridazinyl)methyl)-1,3-dihydrofuro[3,4-c]chinolin-8-carboxamid;
4-Amino-7-fluoro-N-(1-methyl-1H-pyrazol-4-yl)-N-((5-(trifluormethyl)-2-pyridinyl)methyl)-1,3-dihydrofuro[3,4-c]chinolin-8-carboxamid;
4-Amino-7-chloro-N-(1-methyl-1H-pyrazol-4-yl)-N-((5-(trifluormethyl)-2-pyridinyl)methyl)-1,3-dihydrofuro[3,4-c]chinolin-8-carboxamid;
4-Amino-N,3-dimethyl-N-((1R)-1-(5-(trifluormethyl)-2-pyridinyl)ethyl)-3H-pyrazolo[3,4-c]chinolin-8-carboxamid;
4-Amino-N,3-dimethyl-N-((1S)-1-(5-(trifluormethyl)-2-pyridinyl)ethyl)-3H-pyrazolo[3,4-c]chinolin-8-carboxamid;
4-Amino-N-((5-chloro-2-pyridinyl)methyl)-N-(2-propanyl)-1,3-dihydrofuro[3,4-c]chinolin-8-carboxamid;
4-Amino-N-((5-chloro-2-pyridinyl)methyl)-N-(2-propanyl)-1,3-dihydrofuro[3,4-c][1,7]naphthyridin-8-carboxamid;
4-Amino-N-ethyl-7-fluoro-N-((1R)-1-(5-(Trifluormethyl)-2-pyridinyl)ethyl)-1,3-dihydrofuro[3,4-c]chinolin-8-carboxamid;
4-Amino-N-ethyl-7-fluoro-N-((1S)-1-(5-(trifluormethyl)-2-pyridinyl)ethyl)-1,3-dihydrofuro[3,4-c]chinolin-8-carboxamid;
(3R)-4-amino-N-ethyl-3-methyl-N-((1-methyl-1H-1,2,4-triazol-3-yl)methyl)-1,3-dihydrofuro[3,4-c]quinoline-8-carboxamide;
4-Amino-N-cyclobutyl-N-((6-(trifluormethyl)-3-pyridazinyl)methyl)-1,3-dihydrofuro[3,4-c][1,7]naphthyridin-8-carboxamid;
(3R)-4-Amino-N-cyclobutyl-3-methyl-N-((6-(trifluormethyl)-3-pyridazinyl)methyl)-1,3-dihydrofuro[3,4-c]chinolin-8-carboxamid;
4-Amino-N-((6-(Difluormethoxy)-3-pyridazinyl)methyl)-N-ethyl-1,3-dihydrofuro[3,4-c]chinolin-8-carboxamid;
4-Amino-N-((6-(Difluormethoxy)-3-pyridazinyl)methyl)-N-ethyl-7-fluoro-1,3-dihydrofuro[3,4-c]chinolin-8-carboxamid;
4-Amino-7-fluoro-N-((1R)-1-(5-fluoro-2-pyrimidinyl)ethyl)-N-(2-(trifluormethoxy)ethyl)-1,3-dihydrofuro[3,4-c]chinolin-8-carboxamid;
4-Amino-7-fluoro-N-((1S)-1-(5-fluoro-2-pyrimidinyl)ethyl)-N-(2-(trifluormethoxy)ethyl)-1,3-dihydrofuro[3,4-c]chinolin-8-carboxamid;
(3R)-4-Amino-N-((6-(Difluormethoxy)-3-pyridazinyl)methyl)-N-ethyl-3-methyl-1,3-dihydrofuro[3,4-c]chinolin-8-carboxamid;
4-Amino-N-((1R)-1-(6-chloro--3-pyridinyl)ethyl)-N-ethyl-1,3-dihydrofuro[3,4-c]chinolin-8-carboxamid;
4-Amino-N-((1S)-1-(6-chloro--3-pyridinyl)ethyl)-N-ethyl-1,3-dihydrofuro[3,4-c]chinolin-8-carboxamid;
4-Amino-N-((2R)-1-methoxy-2-propanyl)-N-((5-(Trifluormethyl)-2-pyridinyl)methyl)-1,3-dihydrofuro[3,4-c][1,7]naphthyridin-8-carboxamid;
4-Amino-7-fluoro-N-(1-(trifluormethyl)-1H-pyrazol-4-yl)-N-((5-(trifluormethyl)-2-pyridinyl)methyl)-1,3-dihydrofuro[3,4-c]chinolin-8-carboxamid;
4-Amino-N-(cyclopropylmethyl)-7-fluoro-N-((6-(trifluormethyl)-3-pyridazinyl)methyl)-1,3-dihydrofuro[3,4-c]chinolin-8-carboxamid;
4-Amino-N-(cyclopropylmethyl)-7-fluoro-3-methyl-N-((6-(trifluormethyl)-3-pyridazinyl)methyl)-3H-pyrazolo[3,4-c]chinolin-8-carboxamid;
4-Amino-N-(1-(Trifluormethyl)-1H-pyrazol-4-yl)-N-((5-(Trifluormethyl)-2-pyridinyl)methyl)-1,3-dihydrofuro[3,4-c][1,7]naphthyridin-8-carboxamid;
4-Amino-N-(2-propanyl)-N-(4-(trifluormethyl)benzyl)-1,3-dihydrofuro[3,4-c][1,7]naphthyridin-8-carboxamid;
4-Amino-N-cyclopropyl-N-(4-(trifluormethyl)benzyl)-1,3-dihydrofuro[3,4-c][1,7]naphthyridin-8-carboxamid;
4-Amino-N-cyclopropyl-N-((1S)-1-(5-(Trifluormethyl)-2-pyridinyl)ethyl)-1,3-dihydrofuro[3,4-c]chinolin-8-carboxamid;
4-Amino-N-cyclopropyl-N-((1R)-1-(5-(Trifluormethyl)-2-pyridinyl)ethyl)-1,3-dihydrofuro[3,4-c]chinolin-8-carboxamid;
4-Amino-7-chloro-N-((1R)-1-(6-chloro--3-pyridinyl)ethyl)-N-ethyl-1,3-dihydrofuro[3,4-c]chinolin-8-carboxamid;
4-Amino-7-chloro--N-((1S)-1-(6-chloro--3-pyridinyl)ethyl)-N-ethyl-1,3-dihydrofuro[3,4-c]chinolin-8-carboxamid;
4-Amino-7-chloro-N-ethyl-N-((1R)-1-(5-(Trifluormethyl)-2-pyridinyl)ethyl)-1,3-dihydrofuro[3,4-c]chinolin-8-carboxamid;
4-Amino-N-((1R)-1-(6-chloro--3-pyridinyl)ethyl)-N-ethyl-7-fluoro-1,3-dihydrofuro[3,4-c]chinolin-8-carboxamid;
4-Amino-N-((1S)-1-(6-chloro--3-pyridinyl)ethyl)-N-ethyl-7-fluoro-1,3-dihydrofuro[3,4-c]chinolin-8-carboxamid;
4-Amino-7-fluoro-1-methyl-N-(1-methyl-1H-pyrazol-4-yl)-N-((5-(trifluormethyl)-2-pyridinyl)methyl)-1H-pyrazolo[4,3-c]chinolin-8-carboxamid;
4-Amino-7-chloro-N-(cyclopropylmethyl)-N-((6-(trifluormethyl)-3-pyridazinyl)methyl)-1,3-dihydrofuro[3,4-c]chinolin-8-carboxamid;
4-Amino-N-ethyl-N-((1R)-1-(2-fluoro-4-(trifluormethyl)phenyl)ethyl)-1,3-dihydrofuro[3,4-c]chinolin-8-carboxamid;
4-Amino-N-ethyl-N-((1R)-1-(4-(Trifluormethyl)phenyl)ethyl)-1,3-dihydrofuro[3,4-c]chinolin-8-carboxamid;
4-Amino-7-chloro-N-cyclobutyl-N-((1R)-1-(6-(Trifluormethyl)-3-pyridazinyl)ethyl)-1,3-dihydrofuro[3,4-c]chinolin-8-carboxamid;
4-Amino-N-cyclobutyl-N-((1R)-1-(6-(Trifluormethyl)-3-pyridazinyl)ethyl)-1,3-dihydrofuro[3,4-c]chinolin-8-carboxamid;
(3R)-4-Amino-N-cyclopropyl-3-methyl-N-((1R)-1-(5-(Trifluormethyl)-2-pyridinyl)ethyl)-1,3-dihydrofuro[3,4-c]chinolin-8-carboxamid;
4-Amino-N-((6-ethoxy-3-pyridazinyl)methyl)-N-ethyl-7-fluoro-1,3-dihydrofuro[3,4-c]chinolin-8-carboxamid;
4-Amino-N-((S)-Cyclopropyl(5-(trifluormethyl)-2-pyridinyl)methyl)-N-ethyl-1,3-dihydrofuro[3,4-c]chinolin-8-carboxamid;
4-Amino-N-((6-ethoxy-3-pyridazinyl)methyl)-N-((1R)-1-(2-pyrimidinyl)ethyl)-1,3-dihydrofuro[3,4-c][1,7]naphthyridin-8-carboxamid;
4-Amino-7-fluoro-N-(1-methyl-1H-pyrazol-4-yl)-N-((6-(trifluormethyl)-3-pyridazinyl)methyl)-1,3-dihydrofuro[3,4-c]chinolin-8-carboxamid;
4-Amino-7-chloro-N-(1-methyl-1H-pyrazol-4-yl)-N-((6-(trifluormethyl)-3-pyridazinyl)methyl)-1,3-dihydrofuro[3,4-c]chinolin-8-carboxamid;
4-Amino-N-(cyclopropylmethyl)-N-((6-ethoxy-3-pyridazinyl)methyl)-7-fluoro-1,3-dihydrofuro[3,4-c]chinolin-8-carboxamid;
4-Amino-N-(cyclopropylmethyl)-N-((6-ethoxy-3-pyridazinyl)methyl)-1,3-dihydrofuro[3,4-c][1,7]naphthyridin-8-carboxamid;
5-Amino-N-(2-propanyl)-N-((5-(trifluormethyl)-2-pyridinyl)methyl)benzo[c][2,6]naphthyridin-9-carboxamid;
5-Amino-N-(2-propanyl)-N-((5-(trifluormethyl)-2-pyridinyl)methyl)pyrimido[4,5-c]chinolin-9-carboxamid;
5-Amino-N-(2-propanyl)-N-((5-(trifluormethyl)-2-pyridinyl)methyl)pyrido[4,3-c][1,7]naphthyridin-9-carboxamid;
4-Amino-1-methyl-N-(1-methyl-1H-pyrazol-4-yl)-N-((5-(Trifluormethyl)-2-pyridinyl)methyl)-1H-pyrazolo[4,3-c]chinolin-8-carboxamid;
4-Amino-N-((6-ethoxy-3-pyridazinyl)methyl)-3-methyl-N-((1R)-1-(2-pyrimidinyl)ethyl)-3H-pyrazolo[3,4-c]chinolin-8-carboxamid;
4-Amino-N-ethyl-N-(2-(4-(Trifluormethyl)phenyl)-2-propanyl)-1,3-dihydrofuro[3,4-c]chinolin-8-carboxamid;
5-Amino-N-(2-propanyl)-N-((5-(trifluormethyl)-2-pyridinyl)methyl)pyrimido[4,5-c][1,7]naphthyridin-9-carboxamid;
4-Amino-N-ethyl-N-((1R)-1-(5-(Trifluormethyl)-2-pyridinyl)propyl)-1,3-dihydrofuro[3,4-c][1,7]naphthyridin-8-carboxamid;
4-Amino-N-ethyl-7-fluoro-N-((1R)-1-(5-(Trifluormethyl)-2-pyridinyl)ethyl)-1,3-dihydrofuro[3,4-c]chinolin-8-carboxamid;
4-Amino-N-ethyl-N-((1R)-1-(6-(Trifluormethyl)-3-pyridazinyl)ethyl)-1,3-dihydrofuro[3,4-c][1,7]naphthyridin-8-carboxamid;
4-Amino-N-((R)-Cyclopropyl(5-(trifluormethyl)-2-pyridinyl)methyl)-N-methyl-1,3-dihydrofuro[3,4-c][1,7]naphthyridin-8-carboxamid;
4-Amino-N-((S)-Cyclopropyl(5-(trifluormethyl)-2-pyridinyl)methyl)-N-methyl-1,3-dihydrofuro[3,4-c][1,7]naphthyridin-8-carboxamid;
4-Amino-N-(cyclopropylmethyl)-N-((6-ethoxy-3-pyridazinyl)methyl)-7-fluoro-3-methyl-3H-pyrazolo[3,4-c]chinolin-8-carboxamid;
(3R)-4-Amino-N-(cyclopropylmethyl)-N-((6-ethoxy-3-pyridazinyl)methyl)-3-methyl-1,3-dihydrofuro[3,4-c]chinolin-8-carboxamid;
4-Amino-N-((R)-cyclopropyl(6-(trifluormethyl)-3-pyridazinyl)methyl)-N-ethyl-1,3-dihydrofuro[3,4-c][1,7]naphthyridin-8-carboxamid;
(3R)-4-Amino-3-methyl-N-((1R)-1-(5-(Trifluormethyl)-2-pyridinyl)ethyl)-1,3-dihydrofuro[3,4-c]chinolin-8-carboxamid;
4-Amino-7-fluoro-N, 1-dimethyl-N-((5-methyl-2-pyridinyl)methyl)-1H-pyrazolo[4,3-c]chinolin-8-carboxamid;
4-Amino-7-fluoro-N-((5-fluoro-2-pyridinyl)methyl)-N,1-dimethyl-1H-pyrazolo[4,3-c]chinolin-8-carboxamid;
4-Amino-N-((2,6-difluoro-3-pyridinyl)methyl)-7-fluoro-N, 1-dimethyl-1H-pyrazolo[4,3-c]chinolin-8-carboxamid;
4-Amino-7-chloro--N-((1R)-1-(5-fluoro-2-pyridinyl)ethyl)-N-methyl-1,3-dihydrofuro[3,4-c]chinolin-8-carboxamid;
4-Amino-7-chloro-N-((1S)-1-(5-fluoro-2-pyridinyl)ethyl)-N-methyl-1,3-dihydrofuro[3,4-c]chinolin-8-carboxamid;
4-Amino-N-((1R)-1-(5-(Difluormethyl)-2-pyridinyl)ethyl)-N-ethyl-1,3-dihydrofuro[3,4-c][1,7]naphthyridin-8-carboxamid;
4-Amino-N-methyl-N-((1R)-1-(5-(Trifluormethyl)-2-pyrazinyl)ethyl)-1,3-dihydrofuro[3,4-c]chinolin-8-carboxamid;
(3R)-4-Amino-N-((1R)-1-(5-(Difluormethyl)-2-pyridinyl)ethyl)-N-ethyl-3-methyl-1,3-dihydrofuro[3,4-c]chinolin-8-carboxamid;
4-Amino-N,1-dimethyl-N-((1R)-1-(5-(Trifluormethyl)-2-pyrazinyl)ethyl)-1H-pyrazolo[4,3-c]chinolin-8-carboxamid;
(3R)-4-Amino-N-ethyl-3-methyl-N-((1R)-1-(5-(Trifluormethyl)-2-pyridinyl)ethyl)-1,3-dihydrofuro[3,4-c]chinolin-8-carboxamid;
(3R)-4-Amino-N-ethyl-3-methyl-N-((1R)-1-(6-(Trifluormethyl)-3-pyridazinyl)ethyl)-1,3-dihydrofuro[3,4-c]chinolin-8-carboxamid;
4-Amino-7-fluoro-N,3-dimethyl-N-((1R)-1-(6-(trifluormethyl)-3-pyridazinyl)ethyl)-3H-pyrazolo[3,4-c]chinolin-8-carboxamid;
4-Amino-7-fluoro-N,1-dimethyl-N-((1R)-1-(5-(trifluormethyl)-2-pyrazinyl)ethyl)-1H-pyrazolo[4,3-c]chinolin-8-carboxamid;
4-Amino-N-ethyl-7-fluoro-N-((1R)-1-(6-(Trifluormethyl)-3-pyridazinyl)ethyl)-1,3-dihydrofuro[3,4-c]chinolin-8-carboxamid;
4-Amino-7-fluoro-N-((2-fluoro-6-(trifluormethyl)-3-pyridinyl)methyl)-N, 1-dimethyl-1H-pyrazolo[4,3-c]chinolin-8-carboxamid;
4-Amino-N-((R)-cyclopropyl(6-(trifluormethyl)-3-pyridazinyl)methyl)-N-ethyl-1,3-dihydrofuro[3,4-c]chinolin-8-carboxamid;
(3R)-4-Amino-N-ethyl-7-fluoro-3-methyl-N-((1R)-1-(6-(Trifluormethyl)-3-pyridazinyl)ethyl)-1,3-dihydrofuro[3,4-c]chinolin-8-carboxamid;
(3S)-4-Amino-N-ethyl-7-fluoro-3-methyl-N-((1R)-1-(6-(trifluormethyl)-3-pyridazinyl)ethyl)-1,3-dihydrofuro[3,4-c]chinolin-8-carboxamid;
4-Amino-N,3-dimethyl-N-((1R)-1-(4-(Pentafluorethyl)phenyl)ethyl)-3H-pyrazolo[3,4-c][1,7]naphthyridin-8-carboxamid;
4-Amino-7-fluoro-N,1-dimethyl-N-((1S)-1-(4-(pentafluorethyl)phenyl)ethyl)-1H-pyrazolo[4,3-c]chinolin-8-carboxamid;
4-Amino-7-fluoro-N,1-dimethyl-N-((1R)-1-(4-(pentafluorethyl)phenyl)ethyl)-1H-pyrazolo[4,3-c]chinolin-8-carboxamid;
4-Amino-7-fluoro-N,1-dimethyl-N-((5-(1-(trifluormethyl)-1H-pyrazol-4-yl)-2-pyridinyl)methyl)-1H-pyrazolo[4,3-c]chinolin-8-carboxamid;
4-Amino-N-cyclopropyl-1-methyl-N-((6-(trifluormethyl)-3-pyridazinyl)methyl)-1H-pyrazolo[4,3-c]chinolin-8-carboxamid;
4-Amino-7-chloro-N,1-dimethyl-N-((5-(trifluormethyl)-2-pyridinyl)methyl)-1H-pyrazolo[4,3-c]chinolin-8-carboxamid;
4-Amino-7-chloro-N,1-dimethyl-N-((6-(trifluormethyl)-3-pyridazinyl)methyl)-1H-pyrazolo[4,3-c]chinolin-8-carboxamid;
4-Amino-7-chloro-N-ethyl-1-methyl-N-((5-(trifluormethyl)-2-pyridinyl)methyl)-1H-pyrazolo[4,3-c]chinolin-8-carboxamid;
4-Amino-7-chloro-N-ethyl-1-methyl-N-((6-(trifluormethyl)-3-pyridazinyl)methyl)-1H-pyrazolo[4,3-c]chinolin-8-carboxamid;
4-Amino-7-chloro-N-cyclopropyl-1-methyl-N-((5-(trifluormethyl)-2-pyridinyl)methyl)-1H-pyrazolo[4,3-c]chinolin-8-carboxamid;
4-Amino-7-chloro-N-cyclopropyl-1-methyl-N-((6-(trifluormethyl)-3-pyridazinyl)methyl)-1H-pyrazolo[4,3-c]chinolin-8-carboxamid;
4-Amino-1-methyl-N-(2,2,2-trifluorethyl)-N-((5-(trifluormethyl)-2-pyridinyl)methyl)-1H-pyrazolo[4,3-c]chinolin-8-carboxamid;
4-Amino-7-chloro-N-cyclobutyl-1-methyl-N-((5-(trifluormethyl)-2-pyridinyl)methyl)-1H-pyrazolo[4,3-c]chinolin-8-carboxamid;
4-Amino-7-chloro-N-cyclobutyl-1-methyl-N-((6-(trifluormethyl)-3-pyridazinyl)methyl)-1H-pyrazolo[4,3-c]chinolin-8-carboxamid;
4-Amino-7-fluoro-1-methyl-N-(2,2,2-trifluorethyl)-N-((5-(trifluormethyl)-2-pyridinyl)methyl)-1H-pyrazolo[4,3-c]chinolin-8-carboxamid;
4-Amino-N-cyclobutyl-7-fluoro-1-methyl-N-(1,3-oxazol-4-ylmethyl)-1H-pyrazolo[4,3-c]chinolin-8-carboxamid;
4-Amino-7-fluoro-N, 1-dimethyl-N-((5-(trifluormethyl)-2-pyridinyl)methyl)-1H-pyrazolo[4,3-c]chinolin-8-carboxamid;
4-Amino-N-cyclopropyl-7-fluoro-1-methyl-N-((5-(trifluormethyl)-2-pyridinyl)methyl)-1H-pyrazolo[4,3-c]chinolin-8-carboxamid;
4-Amino-N-cyclopropyl-7-fluoro-1-methyl-N-((6-(trifluormethyl)-3-pyridazinyl)methyl)-1H-pyrazolo[4,3-c]chinolin-8-carboxamid;
4-Amino-7-fluoro-1-methyl-N-(2-propanyl)-N-((5-(trifluormethyl)-2-pyridinyl)methyl)-1H-pyrazolo[4,3-c]chinolin-8-carboxamid;
4-Amino-7-fluoro-N-((2-Methoxy-6-(trifluormethyl)-3-pyridinyl)methyl)-N, 1-dimethyl-1H-pyrazolo[4,3-c]chinolin-8-carboxamid;
4-Amino-N-cyclobutyl-7-fluoro-1-methyl-N-((5-(trifluormethyl)-2-pyridinyl)methyl)-1H-pyrazolo[4,3-c]chinolin-8-carboxamid;
4-Amino-7-chloro-N-cyclopropyl-3-methyl-N-((5-(trifluormethyl)-2-pyridinyl)methyl)-3H-pyrazolo[3,4-c]chinolin-8-carboxamid;
4-Amino-N-cyclopropyl-7-fluoro-N-((3-fluoro-5-(trifluormethyl)-2-pyridinyl)methyl)-1-methyl-1H-pyrazolo[4,3-c]chinolin-8-carboxamid;
4-Amino-N,1-dimethyl-7-(trifluormethyl)-N-((5-(trifluormethyl)-2-pyridinyl)methyl)-1H-pyrazolo[4,3-c]chinolin-8-carboxamid;
4-Amino-N,1-dimethyl-7-(trifluormethyl)-N-((6-(trifluormethyl)-3-pyridazinyl)methyl)-1H-pyrazolo[4,3-c]chinolin-8-carboxamid;
4-Amino-N-cyclopropyl-1-methyl-7-(trifluormethyl)-N-((5-(trifluormethyl)-2-pyridinyl)methyl)-1H-pyrazolo[4,3-c]chinolin-8-carboxamid;
4-Amino-N-cyclopropyl-1-methyl-7-(trifluormethyl)-N-((6-(trifluormethyl)-3-pyridazinyl)methyl)-1H-pyrazolo[4,3-c]chinolin-8-carboxamid;
4-Amino-N-((1R)-1-cyclopropylethyl)-7-fluoro-N-((6-(trifluormethyl)-3-pyridazinyl)methyl)-1,3-dihydrofuro[3,4-c]chinolin-8-carboxamid;
4-Amino-N-((1S)-1-cyclopropylethyl)-7-fluoro-N-((6-(trifluormethyl)-3-pyridazinyl)methyl)-1,3-dihydrofuro[3,4-c]chinolin-8-carboxamid;
4-Amino-N-(cyclopropylmethyl)-7-fluoro-N-((6-(2,2,2-Trifluorethoxy)-3-pyridazinyl)-methyl)-1,3-dihydrofuro[3,4-c]chinolin-8-carboxamid;
4-Amino-N-(cyclopropylmethyl)-7-fluoro-3-methyl-N-((6-(2,2,2-Trifluorethoxy)-3-pyridazinyl)methyl)-3H-pyrazolo[3,4-c]chinolin-8-carboxamid;
(3R)-4-Amino-N-(cyclopropylmethyl)-7-fluoro-3-methyl-N-((6-(2,2,2-Trifluorethoxy)-3-pyridazinyl)methyl)-1,3-dihydrofuro[3,4-c]chinolin-8-carboxamid;
4-Amino-N-ethyl-N-((1S)-1-(5-(Trifluormethyl)-2-pyridinyl)ethyl)-1,3-dihydrofuro[3,4-c]chinolin-8-carboxamid;
4-Amino-N-ethyl-N-((1R)-1-(5-(Trifluormethyl)-2-pyridinyl)ethyl)-1,3-dihydrofuro[3,4-c]chinolin-8-carboxamid;
4-Amino-N-ethyl-N-((1R)-1-(5-(Trifluormethyl)-2-pyridinyl)ethyl)-1,3-dihydrofuro[3,4-c][1,7]naphthyridin-8-carboxamid;
4-Amino-N-ethyl-N-((1S)-1-(5-(Trifluormethyl)-2-pyridinyl)ethyl)-1,3-dihydrofuro[3,4-c][1,7]naphthyridin-8-carboxamid;
4-Amino-N-(1-methyl-1H-pyrazol-4-yl)-N-((1R)-1-(5-(trifluormethyl)-2-pyridinyl)ethyl)-1,3-dihydrofuro[3,4-c][1,7]naphthyridin-8-carboxamid;
4-Amino-N-(1-methyl-1H-pyrazol-4-yl)-N-((1S)-1-(5-(trifluormethyl)-2-pyridinyl)ethyl)-1,3-dihydrofuro[3,4-c][1,7]naphthyridin-8-carboxamid;
(3S)-4-Amino-3-methyl-N-(1-methyl-1H-pyrazol-4-yl)-N-((5-(trifluormethyl)-2-pyridinyl)methyl)-1,3-dihydrofuro[3,4-c][1,7]naphthyridin-8-carboxamid;
(3R)-4-Amino-3-methyl-N-(1-methyl-1H-pyrazol-4-yl)-N-((5-(trifluormethyl)-2-pyridinyl)methyl)-1,3-dihydrofuro[3,4-c][1,7]naphthyridin-8-carboxamid;
(3S)-4-Amino-N-cyclobutyl-3-methyl-N-((6-(trifluormethyl)-3-pyridazinyl)methyl)-1,3-dihydrofuro[3,4-c][1,7]naphthyridin-8-carboxamid;
(3R)-4-Amino-N-cyclobutyl-3-methyl-N-((6-(trifluormethyl)-3-pyridazinyl)methyl)-1,3-dihydrofuro[3,4-c][1,7]naphthyridin-8-carboxamid;
4-Amino-N-(2-propanyl)-N-((1S)-1-(5-(Trifluormethyl)-2-pyridinyl)ethyl)-1,3-dihydrofuro[3,4-c]chinolin-8-carboxamid;
4-Amino-N-(2-propanyl)-N-((1R)-1-(5-(trifluormethyl)-2-pyridinyl)ethyl)-1,3-dihydrofuro[3,4-c]chinolin-8-carboxamid;
4-Amino-7-fluoro-N-((1S)-1-(5-fluoro-2-pyrimidinyl)ethyl)-N-(2-(trifluormethoxy)ethyl)-1,3-dihydrofuro[3,4-c]chinolin-8-carboxamid;
4-Amino-7-fluoro-N-((1R)-1-(5-fluoro-2-pyrimidinyl)ethyl)-N-(2-(trifluormethoxy)ethyl)-1,3-dihydrofuro[3,4-c]chinolin-8-carboxamid;
4-Amino-N-((1R)-1-(4-Cyanophenyl)ethyl)-N-ethyl-1,3-dihydrofuro[3,4-c]chinolin-8-carboxamid;
4-Amino-N-((1S)-1-(4-Cyanophenyl)ethyl)-N-ethyl-1,3-dihydrofuro[3,4-c]chinolin-8-carboxamid;
4-Amino-N-((1S)-1-(5-Cyano-2-pyridinyl)ethyl)-N-ethyl-1,3-dihydrofuro[3,4-c]chinolin-8-carboxamid;
4-Amino-N-((1R)-1-(5-Cyano-2-pyridinyl)ethyl)-N-ethyl-1,3-dihydrofuro[3,4-c]chinolin-8-carboxamid;
(3R)-4-Amino-3-methyl-N-(2-propanyl)-N-((1R)-1-(5-(trifluormethyl)-2-pyridinyl)ethyl)-1,3-dihydrofuro[3,4-c]chinolin-8-carboxamid;
(3R)-4-Amino-3-methyl-N-(2-propanyl)-N-((1S)-1-(5-(trifluormethyl)-2-pyridinyl)ethyl)-1,3-dihydrofuro[3,4-c]chinolin-8-carboxamid;
4-Amino-7-fluoro-N-(2-propanyl)-N-((1S)-1-(5-(trifluormethyl)-2-pyridinyl)ethyl)-1,3-dihydrofuro[3,4-c]chinolin-8-carboxamid;
4-Amino-7-fluoro-N-(2-propanyl)-N-((1R)-1-(5-(trifluormethyl)-2-pyridinyl)ethyl)-1,3-dihydrofuro[3,4-c]chinolin-8-carboxamid;
(3R)-4-Amino-N-((1S)-1-(5-cyano-2-pyridinyl)ethyl)-N-ethyl-3-methyl-1,3-dihydrofuro[3,4-c]chinolin-8-carboxamid;
(3R)-4-Amino-N-((1R)-1-(5-Cyano-2-pyridinyl)ethyl)-N-ethyl-3-methyl-1,3-dihydrofuro[3,4-c]chinolin-8-carboxamid;
4-Amino-7-chloro-N-(2-propanyl)-N-((1R)-1-(5-(trifluormethyl)-2-pyridinyl)ethyl)-1,3-dihydrofuro[3,4-c]chinolin-8-carboxamid;
4-Amino-7-chloro-N-(2-propanyl)-N-((1S)-1-(5-(trifluormethyl)-2-pyridinyl)ethyl)-1,3-dihydrofuro[3,4-c]chinolin-8-carboxamid;
4-Amino-N-(2-propanyl)-N-((1S)-1-(5-(trifluormethyl)-2-pyridinyl)ethyl)-1,3-dihydrofuro[3,4-c][1,7]naphthyridin-8-carboxamid;
4-Amino-N-(2-propanyl)-N-((1R)-1-(5-(Trifluormethyl)-2-pyridinyl)ethyl)-1,3-dihydrofuro[3,4-c][1,7]naphthyridin-8-carboxamid;
4-Amino-N-Methyl-N-((1R)-1-(5-(Trifluormethyl)-2-pyridinyl)propyl)-1,3-dihydrofuro[3,4-c][1,7]naphthyridin-8-carboxamid;
4-Amino-N-methyl-N-((1S)-1-(5-(Trifluormethyl)-2-pyridinyl)propyl)-1,3-dihydrofuro[3,4-c][1,7]naphthyridin-8-carboxamid;
(3R)-4-Amino-N,3-dimethyl-N-((1R)-1-(5-(Trifluormethyl)-2-pyridinyl)ethyl)-1,3-dihydrofuro[3,4-c]chinolin-8-carboxamid;
(3R)-4-Amino-N,3-dimethyl-N-((1S)-1-(5-(trifluormethyl)-2-pyridinyl)ethyl)-1,3-dihydrofuro[3,4-c]chinolin-8-carboxamid;
4-Amino-N-((1R)-1-(3,5-Difluoro-2-pyridinyl)ethyl)-7-fluoro-N,1-dimethyl-1H-pyrazolo[4,3-c]chinolin-8-carboxamid;
4-Amino-N-methyl-N-((1R)-1-(5-(Trifluormethyl)-2-pyridinyl)ethyl)-1,3-dihydrofuro[3,4-c][1,7]naphthyridin-8-carboxamid;
4-Amino-N,1-dimethyl-N-((1R)-1-(6-(Trifluormethyl)-3-pyridazinyl)ethyl)-1H-pyrazolo[4,3-c]chinolin-8-carboxamid;
(3R)-4-Amino-N,3-dimethyl-N-((1R)-1-(6-(trifluormethyl)-3-pyridazinyl)ethyl)-1,3-dihydrofuro[3,4-c]chinolin-8-carboxamid;
4-Amino-7-fluoro-N-methyl-N-((1R)-1-(4-(trifluormethyl)phenyl)ethyl)-1,3-dihydrofuro[3,4-c]chinolin-8-carboxamid;
4-Amino-7-fluoro-N-methyl-N-((1R)-1-(6-(Trifluormethyl)-3-pyridazinyl)ethyl)-1,3-dihydrofuro[3,4-c]chinolin-8-carboxamid;
(3R)-4-Amino-7-fluoro-N,3-dimethyl-N-((1S)-1-(4-(trifluormethyl)phenyl)ethyl)-1,3-dihydrofuro[3,4-c]chinolin-8-carboxamid;
(3R)-4-Amino-7-fluoro-N,3-dimethyl-N-((1R)-1-(4-(trifluormethyl)phenyl)ethyl)-1,3-dihydrofuro[3,4-c]chinolin-8-carboxamid;
4-Amino-N-((1R)-1-(3-fluoro-5-(trifluormethyl)-2-pyridinyl)ethyl)-N, 3-dimethyl-3H-pyrazolo[3,4-c][1,7]naphthyridin-8-carboxamid;
(3R)-4-Amino-7-fluoro-N,3-dimethyl-N-((1R)-1-(6-(trifluormethyl)-3-pyridazinyl)ethyl)-1,3-dihydrofuro[3,4-c]chinolin-8-carboxamid;
4-Amino-7-fluoro-N-((1R)-1-(3-fluoro-5-(trifluormethyl)-2-pyridinyl)ethyl)-N, 1-dimethyl-1H-pyrazolo[4,3-c]chinolin-8-carboxamid;
4-Amino-7-fluoro-N-((1S)-1-(3-fluoro-5-(trifluormethyl)-2-pyridinyl)ethyl)-N,1-dimethyl-1 H-pyrazolo[4,3-c]chinolin-8-carboxamid;
4-Amino-7-fluoro-N-((1R)-1-(3-fluoro-5-(trifluormethyl)-2-pyridinyl)ethyl)-N,3-dimethyl-3H-pyrazolo[3,4-c]chinolin-8-carboxamid;
4-Amino-7-fluoro-N-((1R)-1-(3-Methoxy-5-(trifluormethyl)-2-pyridinyl)ethyl)-N,3-dimethyl-3H-pyrazolo[3,4-c]chinolin-8-carboxamid;
4-Amino-7-fluoro-N-((1R)-1-(3-Methoxy-5-(trifluormethyl)-2-pyridinyl)ethyl)-N,1-dimethyl-1H-pyrazolo[4,3-c]chinolin-8-carboxamid;
4-Amino-N,3-dimethyl-N-((1R)-1-(4-(pentafluoro-lambda~6~-sulfanyl)phenyl)ethyl)-3H-pyrazolo[3,4-c][1,7]naphthyridin-8-carboxamid;
4-Amino-7-fluoro-N,1-dimethyl-N-((1S)-1-(4-(Pentafluoro-lambda~6~-sulfanyl)phenyl)ethyl)-1H-pyrazolo[4,3-c]chinolin-8-carboxamid;
4-Amino-7-fluoro-N,1-dimethyl-N-((1R)-1-(4-(Pentafluoro-lambda~6~-sulfanyl)phenyl)ethyl)-1H-pyrazolo[4,3-c]chinolin-8-carboxamid;
4-Amino-1-methyl-N-(4-(Pentafluoro-lambda~6~-sulfanyl)benzyl)-N-((1R)-1-(5-pyrimidinyl)propyl)-1H-pyrazolo[4,3-c][1,7]naphthyridin-8-carboxamid;
4-Amino-1-methyl-N-(4-(pentafluoro-lambda~6~-sulfanyl)benzyl)-N-((1S)-1-(5-pyrimidinyl)propyl)-1H-pyrazolo[4,3-c][1,7]naphthyridin-8-carboxamid;
4-Amino-7-fluoro-N-((1R)-1-(5-fluoro-2-pyridinyl)ethyl)-N,1-dimethyl-1H-pyrazolo[4,3-c]chinolin-8-carboxamid;
4-Amino-7-fluoro-N-((1S)-1-(5-fluoro-2-pyridinyl)ethyl)-N,1-dimethyl-1H-pyrazolo[4,3-c]chinolin-8-carboxamid;
4-Amino-N-ethyl-7-fluoro-N-((1R)-1-(5-fluoro-2-pyridinyl)ethyl)-1-methyl-1H-pyrazolo[4,3-c]chinolin-8-carboxamid;
4-Amino-N-ethyl-7-fluoro-N-((1S)-1-(5-fluoro-2-pyridinyl)ethyl)-1-methyl-1H-pyrazolo[4,3-c]chinolin-8-carboxamid;
4-Amino-N-cyclopropyl-7-fluoro-1-methyl-N-((1S)-1-(6-(trifluormethyl)-3-pyridazinyl)ethyl)-1H-pyrazolo[4,3-c]chinolin-8-carboxamid;
4-Amino-N-cyclopropyl-7-fluoro-1-methyl-N-((1R)-1-(6-(trifluormethyl)-3-pyridazinyl)ethyl)-1H-pyrazolo[4,3-c]chinolin-8-carboxamid;
4-Amino-N,1-dimethyl-7-(trifluormethyl)-N-((1R)-1-(6-(trifluormethyl)-3-pyridazinyl)ethyl)-1H-pyrazolo[4,3-c]chinolin-8-carboxamid;
4-Amino-7-chloro-N-cyclopropyl-1-methyl-N-((1S)-1-(6-(trifluormethyl)-3-pyridazinyl)ethyl)-1H-pyrazolo[4,3-c]chinolin-8-carboxamid;
4-Amino-7-chloro-N-cyclopropyl-1-methyl-N-((1R)-1-(6-(trifluormethyl)-3-pyridazinyl)ethyl)-1H-pyrazolo[4,3-c]chinolin-8-carboxamid;
4-Amino-N-(1-methyl-1H-pyrazol-4-yl)-N-(4-(trifluormethyl)benzyl)-1,3-dihydrofuro[3,4-c][1,7]naphthyridin-8-carboxamid;
4-Amino-N-(3-fluorphenyl)-N-((5-(trifluormethyl)-2-pyridinyl)methyl)-1,3-dihydrofuro[3,4-c]chinolin-8-carboxamid;
4-Amino-N-(1-methyl-1H-pyrazol-4-yl)-N-((5-(trifluormethyl)-2-pyridinyl)methyl)-1,3-dihydrofuro[3,4-c]chinolin-8-carboxamid;
4-Amino-N-(1-methyl-1H-pyrazol-4-yl)-N-((5-(trifluormethyl)-2-pyridinyl)methyl)-1,3-dihydrofuro[3,4-c][1,7]naphthyridin-8-carboxamid;
4-Amino-N-(1-methyl-1H-pyrazol-4-yl)-N-((5-(trifluormethyl)-2-pyridinyl)methyl)-1,3-dihydrofuro[3,4-c][1,8]naphthyridin-8-carboxamid;
5-Amino-N-((1R)-1-(2-pyrimidinyl)ethyl)-N-((5-(Trifluormethyl)-2-pyridinyl)methyl)-1,4-dihydro-2H-pyrano[3,4-c]chinolin-9-carboxamid;
4-Amino-N-((1R)-1-(2-pyrimidinyl)ethyl)-N-((5-(Trifluormethyl)-2-pyridinyl)methyl)-2,3-dihydrofuro[3,2-c]chinolin-8-carboxamid;
4-Amino-3,3-dimethyl-N-(2-methylpropyl)-N-((5-(trifluormethyl)-2-pyridinyl)methyl)-1,3-dihydrofuro[3,4-c]chinolin-8-carboxamid;
(3R)-4-Amino-N-((3-fluoro-2-pyridinyl)methyl)-3-methyl-N-((5-(trifluormethyl)-2-pyridinyl)methyl)-1,3-dihydrofuro[3,4-c]chinolin-8-carboxamid;
(3S)-4-Amino-N-((3-fluoro-2-pyridinyl)methyl)-3-methyl-N-((5-(trifluormethyl)-2-pyridinyl)methyl)-1,3-dihydrofuro[3,4-c]chinolin-8-carboxamid;
(3R)-4-Amino-3-methyl-N-((1R)-1-(tetrahydro-2H-pyran-4-yl)ethyl)-N-((5-(trifluormethyl)-2-pyridinyl)methyl)-1,3-dihydrofuro[3,4-c]chinolin-8-carboxamid;
(3R)-4-Amino-3-methyl-N-((1S)-1-(tetrahydro-2H-pyran-4-yl)ethyl)-N-((5-(trifluormethyl)-2-pyridinyl)methyl)-1,3-dihydrofuro[3,4-c]chinolin-8-carboxamid;
(3S)-4-Amino-3-methyl-N-((1R)-1-(tetrahydro-2H-pyran-4-yl)ethyl)-N-((5-(trifluormethyl)-2-pyridinyl)methyl)-1,3-dihydrofuro[3,4-c]chinolin-8-carboxamid;
(3S)-4-Amino-3-methyl-N-((1S)-1-(tetrahydro-2H-pyran-4-yl)ethyl)-N-((5-(trifluormethyl)-2-pyridinyl)methyl)-1,3-dihydrofuro[3,4-c]chinolin-8-carboxamid;
(3R)-4-Amino-N-((5-cyano-2-pyridinyl)methyl)-3-methyl-N-((1R)-1-(tetrahydro-2H-pyran-4-yl)ethyl)-1,3-dihydrofuro[3,4-c]chinolin-8-carboxamid;
(3R)-4-Amino-N-((5-cyano-2-pyridinyl)methyl)-3-methyl-N-((1S)-1-(tetrahydro-2H-pyran-4-yl)ethyl)-1,3-dihydrofuro[3,4-c]chinolin-8-carboxamid;
(3S)-4-Amino-N-((5-cyano-2-pyridinyl)methyl)-3-methyl-N-((1R)-1-(tetrahydro-2H-pyran-4-yl)ethyl)-1,3-dihydrofuro[3,4-c]chinolin-8-carboxamid;
(3S)-4-Amino-N-((5-cyano-2-pyridinyl)methyl)-3-methyl-N-((1S)-1-(tetrahydro-2H-pyran-4-yl)ethyl)-1,3-dihydrofuro[3,4-c]chinolin-8-carboxamid;
(3R)-4-Amino-N-((trans-4-hydroxycyclohexyl)methyl)-3-methyl-N-((5-(trifluormethyl)-2-pyridinyl)methyl)-1,3-dihydrofuro[3,4-c]chinolin-8-carboxamid;
(3S)-4-Amino-N-((trans-4-hydroxycyclohexyl)methyl)-3-methyl-N-((5-(trifluormethyl)-2-pyridinyl)methyl)-1,3-dihydrofuro[3,4-c]chinolin-8-carboxamid;
(3R)-4-Amino-N-((5-Cyano-2-pyridinyl)methyl)-N-((trans-4-hydroxycyclohexyl)methyl)-3-methyl-1,3-dihydrofuro[3,4-c]chinolin-8-carboxamid;
(3S)-4-Amino-N-((5-cyano-2-pyridinyl)methyl)-N-((trans-4-hydroxycyclohexyl)methyl)-3-methyl-1,3-dihydrofuro[3,4-c]chinolin-8-carboxamid;
(3R)-4-Amino-3-methyl-N-(tetrahydro-2H-pyran-4-ylmethyl)-N-((5-(trifluormethyl)-2-pyridinyl)methyl)-1,3-dihydrofuro[3,4-c]chinolin-8-carboxamid;
(3S)-4-Amino-3-methyl-N-(tetrahydro-2H-pyran-4-ylmethyl)-N-((5-(trifluormethyl)-2-pyridinyl)methyl)-1,3-dihydrofuro[3,4-c]chinolin-8-carboxamid;
(3R)-4-Amino-N-((5-Cyano-2-pyridinyl)methyl)-3-methyl-N-(tetrahydro-2H-pyran-4-ylmethyl)-1,3-dihydrofuro[3,4-c]chinolin-8-carboxamid;
(3S)-4-Amino-N-((5-Cyano-2-pyridinyl)methyl)-3-methyl-N-(tetrahydro-2H-pyran-4-ylmethyl)-1,3-dihydrofuro[3,4-c]chinolin-8-carboxamid;
(3R)-4-Amino-N-((1R,2R)-2-cyanocyclopentyl)-3-methyl-N-((5-(trifluormethyl)-2-pyridinyl)methyl)-1,3-dihydrofuro[3,4-c]chinolin-8-carboxamid;
(3R)-4-Amino-N-((1R,2S)-2-cyanocyclopentyl)-3-methyl-N-((5-(trifluormethyl)-2-pyridinyl)methyl)-1,3-dihydrofuro[3,4-c]chinolin-8-carboxamid;
(3R)-4-Amino-N-((1S,2R)-2-cyanocyclopentyl)-3-methyl-N-((5-(trifluormethyl)-2-pyridinyl)methyl)-1,3-dihydrofuro[3,4-c]chinolin-8-carboxamid;
(3R)-4-Amino-N-((1S,2S)-2-cyanocyclopentyl)-3-methyl-N-((5-(trifluormethyl)-2-pyridinyl)methyl)-1,3-dihydrofuro[3,4-c]chinolin-8-carboxamid;
(3S)-4-Amino-N-((1R,2R)-2-cyanocyclopentyl)-3-methyl-N-((5-(trifluormethyl)-2-pyridinyl)methyl)-1,3-dihydrofuro[3,4-c]chinolin-8-carboxamid;
(3S)-4-Amino-N-((1R,2S)-2-cyanocyclopentyl)-3-methyl-N-((5-(trifluormethyl)-2-pyridinyl)methyl)-1,3-dihydrofuro[3,4-c]chinolin-8-carboxamid;
(3S)-4-Amino-N-((1S,2R)-2-cyanocyclopentyl)-3-methyl-N-((5-(trifluormethyl)-2-pyridinyl)methyl)-1,3-dihydrofuro[3,4-c]chinolin-8-carboxamid;
(3S)-4-Amino-N-((1S,2S)-2-cyanocyclopentyl)-3-methyl-N-((5-(trifluormethyl)-2-pyridinyl)methyl)-1,3-dihydrofuro[3,4-c]chinolin-8-carboxamid;
(3R)-4-Amino-N-((1R,2R)-2-cyanocyclopentyl)-N-((5-cyano-2-pyridinyl)methyl)-3-methyl-1,3-dihydrofuro[3,4-c]chinolin-8-carboxamid;
(3R)-4-Amino-N-((1R,2S)-2-cyanocyclopentyl)-N-((5-Cyano-2-pyridinyl)methyl)-3-methyl-1,3-dihydrofuro[3,4-c]chinolin-8-carboxamid;
(3R)-4-Amino-N-((1S,2R)-2-cyanocyclopentyl)-N-((5-Cyano-2-pyridinyl)methyl)-3-methyl-1,3-dihydrofuro[3,4-c]chinolin-8-carboxamid;
(3R)-4-Amino-N-((1S,2S)-2-cyanocyclopentyl)-N-((5-Cyano-2-pyridinyl)methyl)-3-methyl-1,3-dihydrofuro[3,4-c]chinolin-8-carboxamid;
(3S)-4-Amino-N-((1R,2R)-2-cyanocyclopentyl)-N-((5-Cyano-2-pyridinyl)methyl)-3-methyl-1,3-dihydrofuro[3,4-c]chinolin-8-carboxamid;
(3S)-4-Amino-N-((1R,2S)-2-cyanocyclopentyl)-N-((5-Cyano-2-pyridinyl)methyl)-3-methyl-1,3-dihydrofuro[3,4-c]chinolin-8-carboxamid;
(3S)-4-Amino-N-((1S,2R)-2-cyanocyclopentyl)-N-((5-Cyano-2-pyridinyl)methyl)-3-methyl-1,3-dihydrofuro[3,4-c]chinolin-8-carboxamid;
(3S)-4-Amino-N-((1S,2S)-2-cyanocyclopentyl)-N-((5-Cyano-2-pyridinyl)methyl)-3-methyl-1,3-dihydrofuro[3,4-c]chinolin-8-carboxamid;
(3R)-4-Amino-3-methyl-N-((3R)-tetrahydro-3-furanyl)-N-((5-(trifluormethyl)-2-pyridinyl)methyl)-1,3-dihydrofuro[3,4-c]chinolin-8-carboxamid;
(3R)-4-Amino-3-methyl-N-((3S)-tetrahydro-3-furanyl)-N-((5-(trifluormethyl)-2-pyridinyl)methyl)-1,3-dihydrofuro[3,4-c]chinolin-8-carboxamid;
(3S)-4-Amino-3-methyl-N-((3R)-tetrahydro-3-furanyl)-N-((5-(trifluormethyl)-2-pyridinyl)methyl)-1,3-dihydrofuro[3,4-c]chinolin-8-carboxamid;
(3S)-4-Amino-3-methyl-N-((3S)-tetrahydro-3-furanyl)-N-((5-(trifluormethyl)-2-pyridinyl)methyl)-1,3-dihydrofuro[3,4-c]chinolin-8-carboxamid;
(3R)-4-Amino-3-methyl-N-((1R,5S,6r)-3-oxabicyclo[3.1.0]hexan-6-yl)-N-((5-(Trifluormethyl)-2-pyridinyl)methyl)-1,3-dihydrofuro[3,4-c]chinolin-8-carboxamid;
(3S)-4-Amino-3-methyl-N-((1R,5S,6r)-3-oxabicyclo[3.1.0]hexan-6-yl)-N-((5-(Trifluormethyl)-2-pyridinyl)methyl)-1,3-dihydrofuro[3,4-c]chinolin-8-carboxamid;
(3R)-4-Amino-3-methyl-N-((3R,4R)-3-methyltetrahydro-2H-pyran-4-yl)-N-((5-(trifluormethyl)-2-pyridinyl)methyl)-1,3-dihydrofuro[3,4-c]chinolin-8-carboxamid;
(3R)-4-Amino-3-methyl-N-((3R,4S)-3-methyltetrahydro-2H-pyran-4-yl)-N-((5-(Trifluormethyl)-2-pyridinyl)methyl)-1,3-dihydrofuro[3,4-c]chinolin-8-carboxamid;
(3R)-4-Amino-3-methyl-N-((3S,4R)-3-methyltetrahydro-2H-pyran-4-yl)-N-((5-(trifluormethyl)-2-pyridinyl)methyl)-1,3-dihydrofuro[3,4-c]chinolin-8-carboxamid;
(3R)-4-Amino-3-methyl-N-((3S,4S)-3-methyltetrahydro-2H-pyran-4-yl)-N-((5-(trifluormethyl)-2-pyridinyl)methyl)-1,3-dihydrofuro[3,4-c]chinolin-8-carboxamid;
(3S)-4-Amino-3-methyl-N-((3R,4R)-3-methyltetrahydro-2H-pyran-4-yl)-N-((5-(trifluormethyl)-2-pyridinyl)methyl)-1,3-dihydrofuro[3,4-c]chinolin-8-carboxamid;
(3S)-4-Amino-3-methyl-N-((3R,4S)-3-methyltetrahydro-2H-pyran-4-yl)-N-((5-(trifluormethyl)-2-pyridinyl)methyl)-1,3-dihydrofuro[3,4-c]chinolin-8-carboxamid;
(3S)-4-Amino-3-methyl-N-((3S,4R)-3-methyltetrahydro-2H-pyran-4-yl)-N-((5-(trifluormethyl)-2-pyridinyl)methyl)-1,3-dihydrofuro[3,4-c]chinolin-8-carboxamid;
(3S)-4-Amino-3-methyl-N-((3S,4S)-3-methyltetrahydro-2H-pyran-4-yl)-N-((5-(trifluormethyl)-2-pyridinyl)methyl)-1,3-dihydrofuro[3,4-c]chinolin-8-carboxamid;
(3R)-4-Amino-N-((3S,4R)-3-methoxytetrahydro-2H-pyran-4-yl)-3-methyl-N-((5-(trifluormethyl)-2-pyridinyl)methyl)-1,3-dihydrofuro[3,4-c]chinolin-8-carboxamid;
(3S)-4-Amino-N-((3S,4R)-3-methoxytetrahydro-2H-pyran-4-yl)-3-methyl-N-((5-(trifluormethyl)-2-pyridinyl)methyl)-1,3-dihydrofuro[3,4-c]chinolin-8-carboxamid;
(3R)-4-Amino-N-((3R,4S)-3-methoxytetrahydro-2H-pyran-4-yl)-3-methyl-N-((5-(trifluormethyl)-2-pyridinyl)methyl)-1,3-dihydrofuro[3,4-c]chinolin-8-carboxamid;
(3S)-4-Amino-N-((3R,4S)-3-methoxytetrahydro-2H-pyran-4-yl)-3-methyl-N-((5-(trifluormethyl)-2-pyridinyl)methyl)-1,3-dihydrofuro[3,4-c]chinolin-8-carboxamid;
(3R)-4-Amino-N-((1R,2R)-[1,1'-bi(cyclopropyl)]-2-yl)-3-methyl-N-((5-(trifluormethyl)-2-pyridinyl)methyl)-1,3-dihydrofuro[3,4-c]chinolin-8-carboxamid;
(3R)-4-Amino-N-((1R,2S)-[1,1'-bi(cyclopropyl)]-2-yl)-3-methyl-N-((5-(trifluormethyl)-2-pyridinyl)methyl)-1,3-dihydrofuro[3,4-c]chinolin-8-carboxamid;
(3R)-4-Amino-N-((1S,2R)-[1,1'-bi(cyclopropyl)]-2-yl)-3-methyl-N-((5-(trifluormethyl)-2-pyridinyl)methyl)-1,3-dihydrofuro[3,4-c]chinolin-8-carboxamid;
(3R)-4-Amino-N-((1S,2S)-[1,1'-bi(cyclopropyl)]-2-yl)-3-methyl-N-((5-(trifluormethyl)-2-pyridinyl)methyl)-1,3-dihydrofuro[3,4-c]chinolin-8-carboxamid;
(3S)-4-Amino-N-((1R,2R)-[1,1'-bi(cyclopropyl)]-2-yl)-3-methyl-N-((5-(trifluormethyl)-2-pyridinyl)methyl)-1,3-dihydrofuro[3,4-c]chinolin-8-carboxamid;
(3S)-4-Amino-N-((1R,2S)-[1,1'-bi(cyclopropyl)]-2-yl)-3-methyl-N-((5-(trifluormethyl)-2-pyridinyl)methyl)-1,3-dihydrofuro[3,4-c]chinolin-8-carboxamid;
(3S)-4-Amino-N-((1S,2R)-[1,1'-bi(cyclopropyl)]-2-yl)-3-methyl-N-((5-(trifluormethyl)-2-pyridinyl)methyl)-1,3-dihydrofuro[3,4-c]chinolin-8-carboxamid;
(3S)-4-Amino-N-((1S,2S)-[1,1'-bi(cyclopropyl)]-2-yl)-3-methyl-N-((5-(trifluormethyl)-2-pyridinyl)methyl)-1,3-dihydrofuro[3,4-c]chinolin-8-carboxamid;
(3R)-4-Amino-3-methyl-N-((1R)-spiro[2.5]octan-1-yl)-N-((5-(trifluormethyl)-2-pyridinyl)methyl)-1,3-dihydrofuro[3,4-c]chinolin-8-carboxamid;
(3R)-4-Amino-3-methyl-N-((1S)-spiro[2.5]octan-1-yl)-N-((5-(trifluormethyl)-2-pyridinyl)methyl)-1,3-dihydrofuro[3,4-c]chinolin-8-carboxamid;
(3S)-4-Amino-3-methyl-N-((1R)-spiro[2.5]octan-1-yl)-N-((5-(trifluormethyl)-2-pyridinyl)methyl)-1,3-dihydrofuro[3,4-c]chinolin-8-carboxamid;
(3S)-4-Amino-3-methyl-N-((1S)-spiro[2.5]octan-1-yl)-N-((5-(trifluormethyl)-2-pyridinyl)methyl)-1,3-dihydrofuro[3,4-c]chinolin-8-carboxamid;
(3R)-4-Amino-N-((1R,2R)-2-aminocyclopropyl)-3-methyl-N-((5-(trifluormethyl)-2-pyridinyl)methyl)-1,3-dihydrofuro[3,4-c]chinolin-8-carboxamid;
(3R)-4-Amino-N-((1R,2S)-2-aminocyclopropyl)-3-methyl-N-((5-(trifluormethyl)-2-pyridinyl)methyl)-1,3-dihydrofuro[3,4-c]chinolin-8-carboxamid;
(3R)-4-Amino-N-((1S,2R)-2-aminocyclopropyl)-3-methyl-N-((5-(trifluormethyl)-2-pyridinyl)methyl)-1,3-dihydrofuro[3,4-c]chinolin-8-carboxamid;
(3R)-4-Amino-N-((1S,2S)-2-aminocyclopropyl)-3-methyl-N-((5-(trifluormethyl)-2-pyridinyl)methyl)-1,3-dihydrofuro[3,4-c]chinolin-8-carboxamid;
(3S)-4-Amino-N-((1R,2R)-2-aminocyclopropyl)-3-methyl-N-((5-(trifluormethyl)-2-pyridinyl)methyl)-1,3-dihydrofuro[3,4-c]chinolin-8-carboxamid;
(3S)-4-Amino-N-((1R,2S)-2-aminocyclopropyl)-3-methyl-N-((5-(trifluormethyl)-2-pyridinyl)methyl)-1,3-dihydrofuro[3,4-c]chinolin-8-carboxamid;
(3S)-4-Amino-N-((1S,2R)-2-aminocyclopropyl)-3-methyl-N-((5-(trifluormethyl)-2-pyridinyl)methyl)-1,3-dihydrofuro[3,4-c]chinolin-8-carboxamid;
(3S)-4-Amino-N-((1S,2S)-2-aminocyclopropyl)-3-methyl-N-((5-(trifluormethyl)-2-pyridinyl)methyl)-1,3-dihydrofuro[3,4-c]chinolin-8-carboxamid;
(3R)-4-Amino-N-((1R,2R)-2-ethoxycyclopropyl)-3-methyl-N-((5-(trifluormethyl)-2-pyridinyl)methyl)-1,3-dihydrofuro[3,4-c]chinolin-8-carboxamid;
(3R)-4-Amino-N-((1R,2S)-2-ethoxycyclopropyl)-3-methyl-N-((5-(trifluormethyl)-2-pyridinyl)methyl)-1,3-dihydrofuro[3,4-c]chinolin-8-carboxamid;
(3R)-4-Amino-N-((1S,2R)-2-ethoxycyclopropyl)-3-methyl-N-((5-(trifluormethyl)-2-pyridinyl)methyl)-1,3-dihydrofuro[3,4-c]chinolin-8-carboxamid;
(3R)-4-Amino-N-((1S,2S)-2-ethoxycyclopropyl)-3-methyl-N-((5-(trifluormethyl)-2-pyridinyl)methyl)-1,3-dihydrofuro[3,4-c]chinolin-8-carboxamid;
(3S)-4-Amino-N-((1R,2R)-2-ethoxycyclopropyl)-3-methyl-N-((5-(trifluormethyl)-2-pyridinyl)methyl)-1,3-dihydrofuro[3,4-c]chinolin-8-carboxamid;
(3S)-4-Amino-N-((1R,2S)-2-ethoxycyclopropyl)-3-methyl-N-((5-(trifluormethyl)-2-pyridinyl)methyl)-1,3-dihydrofuro[3,4-c]chinolin-8-carboxamid;
(3S)-4-Amino-N-((1S,2R)-2-ethoxycyclopropyl)-3-methyl-N-((5-(trifluormethyl)-2-pyridinyl)methyl)-1,3-dihydrofuro[3,4-c]chinolin-8-carboxamid;
(3S)-4-Amino-N-((1S,2S)-2-ethoxycyclopropyl)-3-methyl-N-((5-(trifluormethyl)-2-pyridinyl)methyl)-1,3-dihydrofuro[3,4-c]chinolin-8-carboxamid;
(3R)-4-Amino-N-((1R)-2,2-dimethylcyclopropyl)-3-methyl-N-((5-(trifluormethyl)-2-pyridinyl)methyl)-1,3-dihydrofuro[3,4-c]chinolin-8-carboxamid;
(3R)-4-Amino-N-((1S)-2,2-dimethylcyclopropyl)-3-methyl-N-((5-(trifluormethyl)-2-pyridinyl)methyl)-1,3-dihydrofuro[3,4-c]chinolin-8-carboxamid;
(3S)-4-Amino-N-((1R)-2,2-dimethylcyclopropyl)-3-methyl-N-((5-(trifluormethyl)-2-pyridinyl)methyl)-1,3-dihydrofuro[3,4-c]chinolin-8-carboxamid;
(3S)-4-Amino-N-((1S)-2,2-dimethylcyclopropyl)-3-methyl-N-((5-(trifluormethyl)-2-pyridinyl)methyl)-1,3-dihydrofuro[3,4-c]chinolin-8-carboxamid;
(3R)-4-Amino-3-methyl-N-((1R)-spiro[2.4]heptan-1-yl)-N-((5-(trifluormethyl)-2-pyridinyl)methyl)-1,3-dihydrofuro[3,4-c]chinolin-8-carboxamid;
(3R)-4-Amino-3-methyl-N-((1S)-spiro[2.4]heptan-1-yl)-N-((5-(trifluormethyl)-2-pyridinyl)methyl)-1,3-dihydrofuro[3,4-c]chinolin-8-carboxamid;
(3S)-4-Amino-3-methyl-N-((1R)-spiro[2.4]heptan-1-yl)-N-((5-(trifluormethyl)-2-pyridinyl)methyl)-1,3-dihydrofuro[3,4-c]chinolin-8-carboxamid;
(3S)-4-Amino-3-methyl-N-((1S)-spiro[2.4]heptan-1-yl)-N-((5-(trifluormethyl)-2-pyridinyl)methyl)-1,3-dihydrofuro[3,4-c]chinolin-8-carboxamid;
(3R)-4-Amino-3-methyl-N-((1R,2R)-2-(trifluormethyl)cyclopropyl)-N-((5-(trifluormethyl)-2-pyridinyl)methyl)-1,3-dihydrofuro[3,4-c]chinolin-8-carboxamid;
(3R)-4-Amino-3-methyl-N-((1S,2S)-2-(trifluormethyl)cyclopropyl)-N-((5-(trifluormethyl)-2-pyridinyl)methyl)-1,3-dihydrofuro[3,4-c]chinolin-8-carboxamid;
(3S)-4-Amino-3-methyl-N-((1R,2R)-2-(trifluormethyl)cyclopropyl)-N-((5-(trifluormethyl)-2-pyridinyl)methyl)-1,3-dihydrofuro[3,4-c]chinolin-8-carboxamid;
(3S)-4-Amino-3-methyl-N-((1S,2S)-2-(trifluormethyl)cyclopropyl)-N-((5-(trifluormethyl)-2-pyridinyl)methyl)-1,3-dihydrofuro[3,4-c]chinolin-8-carboxamid;
(3R)-4-Amino-3-methyl-N-((1R)-1-(2-pyrimidinyl)ethyl)-N-((5-(trifluormethyl)-2-pyridinyl)methyl)-1,3-dihydrofuro[3,4-c]chinolin-8-carboxamid;
(3S)-4-Amino-3-methyl-N-((1R)-1-(2-pyrimidinyl)ethyl)-N-((5-(trifluormethyl)-2-pyridinyl)methyl)-1,3-dihydrofuro[3,4-c]chinolin-8-carboxamid;
(3R)-4-Amino-N-((5-Cyano-2-pyridinyl)methyl)-N-(5,6-dihydro-2H-pyran-3-ylmethyl)-3-methyl-1,3-dihydrofuro[3,4-c]chinolin-8-carboxamid;
(3S)-4-Amino-N-((5-Cyano-2-pyridinyl)methyl)-N-(5,6-dihydro-2H-pyran-3-ylmethyl)-3-methyl-1,3-dihydrofuro[3,4-c]chinolin-8-carboxamid;
(3R)-4-Amino-N-((1-Cyanocyclopropyl)methyl)-N-((5-Cyano-2-pyridinyl)methyl)-3-methyl-1,3-dihydrofuro[3,4-c]chinolin-8-carboxamid;
(3S)-4-Amino-N-((1-Cyanocyclopropyl)methyl)-N-((5-Cyano-2-pyridinyl)methyl)-3-methyl-1,3-dihydrofuro[3,4-c]chinolin-8-carboxamid;
(3R)-4-Amino-N-((2-Amino-1,3-thiazol-5-yl)methyl)-N-((5-Cyano-2-pyridinyl)methyl)-3-methyl-1,3-dihydrofuro[3,4-c]chinolin-8-carboxamid;
(3S)-4-Amino-N-((2-Amino-1,3-thiazol-5-yl)methyl)-N-((5-Cyano-2-pyridinyl)methyl)-3-methyl-1,3-dihydrofuro[3,4-c]chinolin-8-carboxamid;
(3R)-4-Amino-N-(5,6-dihydro-2H-pyran-3-ylmethyl)-3-methyl-N-((5-(trifluormethyl)-2-pyridinyl)methyl)-1,3-dihydrofuro[3,4-c]chinolin-8-carboxamid;
(3S)-4-Amino-N-(5,6-dihydro-2H-pyran-3-ylmethyl)-3-methyl-N-((5-(trifluormethyl)-2-pyridinyl)methyl)-1,3-dihydrofuro[3,4-c]chinolin-8-carboxamid;
(3R)-4-Amino-3-methyl-N-(1H-pyrrolo[2,3-b]pyridin-4-ylmethyl)-N-((5-(trifluormethyl)-2-pyridinyl)methyl)-1,3-dihydrofuro[3,4-c]chinolin-8-carboxamid;
(3S)-4-Amino-3-methyl-N-(1H-pyrrolo[2,3-b]pyridin-4-ylmethyl)-N-((5-(trifluormethyl)-2-pyridinyl)methyl)-1,3-dihydrofuro[3,4-c]chinolin-8-carboxamid;
(3R)-4-Amino-N-((1-Cyanocyclopropyl)methyl)-3-methyl-N-((5-(Trifluormethyl)-2-pyridinyl)methyl)-1,3-dihydrofuro[3,4-c]chinolin-8-carboxamid;
(3S)-4-Amino-N-((1-Cyanocyclopropyl)methyl)-3-methyl-N-((5-(Trifluormethyl)-2-pyridinyl)methyl)-1,3-dihydrofuro[3,4-c]chinolin-8-carboxamid;
(3R)-4-Amino-N-((2-Amino-1,3-thiazol-5-yl)methyl)-3-methyl-N-((5-(trifluormethyl)-2-pyridinyl)methyl)-1,3-dihydrofuro[3,4-c]chinolin-8-carboxamid;
(3S)-4-Amino-N-((2-Amino-1,3-thiazol-5-yl)methyl)-3-methyl-N-((5-(trifluormethyl)-2-pyridinyl)methyl)-1,3-dihydrofuro[3,4-c]chinolin-8-carboxamid;
(3R)-4-Amino-3-methyl-N-(2-methylpropyl)-N-((5-(trifluormethyl)-2-pyridinyl)methyl)-1,3-dihydrofuro[3,4-c]chinolin-8-carboxamid;
(3S)-4-Amino-3-methyl-N-(2-methylpropyl)-N-((5-(trifluormethyl)-2-pyridinyl)methyl)-1,3-dihydrofuro[3,4-c]chinolin-8-carboxamid;
(3R)-4-Amino-N-((2R,3R)-3-hydroxy-2-butanyl)-3-methyl-N-((5-(trifluormethyl)-2-pyridinyl)methyl)-1,3-dihydrofuro[3,4-c]chinolin-8-carboxamid;
(3R)-4-Amino-N-((2R,3S)-3-hydroxy-2-butanyl)-3-methyl-N-((5-(trifluormethyl)-2-pyridinyl)methyl)-1,3-dihydrofuro[3,4-c]chinolin-8-carboxamid;
(3R)-4-Amino-N-((2S,3R)-3-hydroxy-2-butanyl)-3-methyl-N-((5-(trifluormethyl)-2-pyridinyl)methyl)-1,3-dihydrofuro[3,4-c]chinolin-8-carboxamid;
(3R)-4-Amino-N-((2S,3S)-3-hydroxy-2-butanyl)-3-methyl-N-((5-(trifluormethyl)-2-pyridinyl)methyl)-1,3-dihydrofuro[3,4-c]chinolin-8-carboxamid;
(3S)-4-Amino-N-((2R,3R)-3-hydroxy-2-butanyl)-3-methyl-N-((5-(trifluormethyl)-2-pyridinyl)methyl)-1,3-dihydrofuro[3,4-c]chinolin-8-carboxamid;
(3S)-4-Amino-N-((2R,3S)-3-hydroxy-2-butanyl)-3-methyl-N-((5-(trifluormethyl)-2-pyridinyl)methyl)-1,3-dihydrofuro[3,4-c]chinolin-8-carboxamid;
(3S)-4-Amino-N-((2S,3R)-3-hydroxy-2-butanyl)-3-methyl-N-((5-(trifluormethyl)-2-pyridinyl)methyl)-1,3-dihydrofuro[3,4-c]chinolin-8-carboxamid;
(3S)-4-Amino-N-((2S,3S)-3-hydroxy-2-butanyl)-3-methyl-N-((5-(trifluormethyl)-2-pyridinyl)methyl)-1,3-dihydrofuro[3,4-c]chinolin-8-carboxamid;
(3R)-4-Amino-N-((1R,3R)-3-hydroxycyclohexyl)-3-methyl-N-((5-(trifluormethyl)-2-pyridinyl)methyl)-1,3-dihydrofuro[3,4-c]chinolin-8-carboxamid;
(3R)-4-Amino-N-((1R,3S)-3-hydroxycyclohexyl)-3-methyl-N-((5-(trifluormethyl)-2-pyridinyl)methyl)-1,3-dihydrofuro[3,4-c]chinolin-8-carboxamid;
(3R)-4-Amino-N-((1S,3R)-3-hydroxycyclohexyl)-3-methyl-N-((5-(trifluormethyl)-2-pyridinyl)methyl)-1,3-dihydrofuro[3,4-c]chinolin-8-carboxamid;
(3R)-4-Amino-N-((1S,3S)-3-hydroxycyclohexyl)-3-methyl-N-((5-(trifluormethyl)-2-pyridinyl)methyl)-1,3-dihydrofuro[3,4-c]chinolin-8-carboxamid;
(3S)-4-Amino-N-((1R,3R)-3-hydroxycyclohexyl)-3-methyl-N-((5-(trifluormethyl)-2-pyridinyl)methyl)-1,3-dihydrofuro[3,4-c]chinolin-8-carboxamid;
(3S)-4-Amino-N-((1R,3S)-3-hydroxycyclohexyl)-3-methyl-N-((5-(trifluormethyl)-2-pyridinyl)methyl)-1,3-dihydrofuro[3,4-c]chinolin-8-carboxamid;
(3S)-4-Amino-N-((1S,3R)-3-hydroxycyclohexyl)-3-methyl-N-((5-(trifluormethyl)-2-pyridinyl)methyl)-1,3-dihydrofuro[3,4-c]chinolin-8-carboxamid;
(3S)-4-Amino-N-((1S,3S)-3-hydroxycyclohexyl)-3-methyl-N-((5-(trifluormethyl)-2-pyridinyl)methyl)-1,3-dihydrofuro[3,4-c]chinolin-8-carboxamid;
(3R)-4-Amino-N-(2-(cis-3-hydroxycyclobutyl)ethyl)-3-methyl-N-((5-(trifluormethyl)-2-pyridinyl)methyl)-1,3-dihydrofuro[3,4-c]chinolin-8-carboxamid;
(3R)-4-Amino-N-(2-(trans-3-hydroxycyclobutyl)ethyl)-3-methyl-N-((5-(trifluormethyl)-2-pyridinyl)methyl)-1,3-dihydrofuro[3,4-c]chinolin-8-carboxamid;
(3S)-4-Amino-N-(2-(cis-3-hydroxycyclobutyl)ethyl)-3-methyl-N-((5-(trifluormethyl)-2-pyridinyl)methyl)-1,3-dihydrofuro[3,4-c]chinolin-8-carboxamid;
(3S)-4-Amino-N-(2-(trans-3-hydroxycyclobutyl)ethyl)-3-methyl-N-((5-(trifluormethyl)-2-pyridinyl)methyl)-1,3-dihydrofuro[3,4-c]chinolin-8-carboxamid;
(3R)-4-Amino-N-((2R)-2-hydroxypropyl)-3-methyl-N-((5-(trifluormethyl)-2-pyridinyl)methyl)-1,3-dihydrofuro[3,4-c]chinolin-8-carboxamid;
(3R)-4-Amino-N-((2S)-2-hydroxypropyl)-3-methyl-N-((5-(trifluormethyl)-2-pyridinyl)methyl)-1,3-dihydrofuro[3,4-c]chinolin-8-carboxamid;
(3S)-4-Amino-N-((2R)-2-hydroxypropyl)-3-methyl-N-((5-(trifluormethyl)-2-pyridinyl)methyl)-1,3-dihydrofuro[3,4-c]chinolin-8-carboxamid;
(3S)-4-Amino-N-((2S)-2-hydroxypropyl)-3-methyl-N-((5-(trifluormethyl)-2-pyridinyl)methyl)-1,3-dihydrofuro[3,4-c]chinolin-8-carboxamid;
(3R)-4-Amino-N-((2R)-3-hydroxy-2-methylpropyl)-3-methyl-N-((5-(trifluormethyl)-2-pyridinyl)methyl)-1,3-dihydrofuro[3,4-c]chinolin-8-carboxamid;
(3R)-4-Amino-N-((2S)-3-hydroxy-2-methylpropyl)-3-methyl-N-((5-(trifluormethyl)-2-pyridinyl)methyl)-1,3-dihydrofuro[3,4-c]chinolin-8-carboxamid;
(3S)-4-Amino-N-((2R)-3-hydroxy-2-methylpropyl)-3-methyl-N-((5-(trifluormethyl)-2-pyridinyl)methyl)-1,3-dihydrofuro[3,4-c]chinolin-8-carboxamid;
(3S)-4-Amino-N-((2S)-3-hydroxy-2-methylpropyl)-3-methyl-N-((5-(trifluormethyl)-2-pyridinyl)methyl)-1,3-dihydrofuro[3,4-c]chinolin-8-carboxamid;
(3R)-4-Amino-3-methyl-N-((2R)-3,3,3-Trifluoro-2-hydroxypropyl)-N-((5-(trifluormethyl)-2-pyridinyl)methyl)-1,3-dihydrofuro[3,4-c]chinolin-8-carboxamid;
(3R)-4-Amino-3-methyl-N-((2S)-3,3,3-Trifluoro-2-hydroxypropyl)-N-((5-(trifluormethyl)-2-pyridinyl)methyl)-1,3-dihydrofuro[3,4-c]chinolin-8-carboxamid;
(3S)-4-Amino-3-methyl-N-((2R)-3,3,3-Trifluoro-2-hydroxypropyl)-N-((5-(trifluormethyl)-2-pyridinyl)methyl)-1,3-dihydrofuro[3,4-c]chinolin-8-carboxamid;
(3S)-4-Amino-3-methyl-N-((2S)-3,3,3-Trifluoro-2-hydroxypropyl)-N-((5-(trifluormethyl)-2-pyridinyl)methyl)-1,3-dihydrofuro[3,4-c]chinolin-8-carboxamid;
(3R)-4-Amino-3-methyl-N-((1-methyl-1H-1,2,4-triazol-3-yl)methyl)-N-((5-(trifluormethyl)-2-pyridinyl)methyl)-1,3-dihydrofuro[3,4-c]chinolin-8-carboxamid;
(3S)-4-Amino-3-methyl-N-((1-methyl-1H-1,2,4-triazol-3-yl) methyl)-N-((5-(trifluormethyl)-2-pyridinyl)methyl)-1,3-dihydrofuro[3,4-c]chinolin-8-carboxamid;
(3R)-4-Amino-3-methyl-N-((3R)-tetrahydro-3-furanylmethyl)-N-((5-(trifluormethyl)-2-pyridinyl)methyl)-1,3-dihydrofuro[3,4-c]chinolin-8-carboxamid;
(3R)-4-Amino-3-methyl-N-((3S)-tetrahydro-3-furanylmethyl)-N-((5-(trifluormethyl)-2-pyridinyl)methyl)-1,3-dihydrofuro[3,4-c]chinolin-8-carboxamid;
(3S)-4-Amino-3-methyl-N-((3R)-tetrahydro-3-furanylmethyl)-N-((5-(trifluormethyl)-2-pyridinyl)methyl)-1,3-dihydrofuro[3,4-c]chinolin-8-carboxamid;
(3S)-4-Amino-3-methyl-N-((3S)-tetrahydro-3-furanylmethyl)-N-((5-(trifluormethyl)-2-pyridinyl)methyl)-1,3-dihydrofuro[3,4-c]chinolin-8-carboxamid;
(3R)-4-Amino-N-((2R)-2-cyanopropyl)-3-methyl-N-((5-(trifluormethyl)-2-pyridinyl)methyl)-1,3-dihydrofuro[3,4-c]chinolin-8-carboxamid;
(3R)-4-Amino-N-((2S)-2-cyanopropyl)-3-methyl-N-((5-(trifluormethyl)-2-pyridinyl)methyl)-1,3-dihydrofuro[3,4-c]chinolin-8-carboxamid;
(3S)-4-Amino-N-((2R)-2-cyanopropyl)-3-methyl-N-((5-(trifluormethyl)-2-pyridinyl)methyl)-1,3-dihydrofuro[3,4-c]chinolin-8-carboxamid;
(3S)-4-Amino-N-((2S)-2-cyanopropyl)-3-methyl-N-((5-(trifluormethyl)-2-pyridinyl)methyl)-1,3-dihydrofuro[3,4-c]chinolin-8-carboxamid;
(3R)-4-Amino-N,3-dimethyl-N-((5-(trifluormethyl)-2-pyridinyl)methyl)-1,3-dihydrofuro[3,4-c]chinolin-8-carboxamid;
(3S)-4-Amino-N,3-dimethyl-N-((5-(trifluormethyl)-2-pyridinyl)methyl)-1,3-dihydrofuro[3,4-c]chinolin-8-carboxamid;
(3R)-4-Amino-N-ethyl-3-methyl-N-((5-(trifluormethyl)-2-pyridinyl)methyl)-1,3-dihydrofuro[3,4-c]chinolin-8-carboxamid;
(3S)-4-Amino-N-ethyl-3-methyl-N-((5-(trifluormethyl)-2-pyridinyl)methyl)-1,3-dihydrofuro[3,4-c]chinolin-8-carboxamid;
(3R)-4-Amino-3-methyl-N-(2-propanyl)-N-((5-(trifluormethyl)-2-pyridinyl)methyl)-1,3-dihydrofuro[3,4-c]chinolin-8-carboxamid;
(3S)-4-Amino-3-methyl-N-(2-propanyl)-N-((5-(trifluormethyl)-2-pyridinyl)methyl)-1,3-dihydrofuro[3,4-c]chinolin-8-carboxamid;
4-Amino-N,3,3-trimethyl-N-((5-(trifluormethyl)-2-pyridinyl)methyl)-1,3-dihydrofuro[3,4-c]chinolin-8-carboxamid;
4-Amino-N-ethyl-3,3-dimethyl-N-((5-(trifluormethyl)-2-pyridinyl)methyl)-1,3-dihydrofuro[3,4-c]chinolin-8-carboxamid;
4-Amino-3,3-dimethyl-N-(2-propanyl)-N-((5-(trifluormethyl)-2-pyridinyl)methyl)-1,3-dihydrofuro[3,4-c]chinolin-8-carboxamid;
4-Amino-N-cyclopropyl-3,3-dimethyl-N-((5-(trifluormethyl)-2-pyridinyl)methyl)-1,3-dihydrofuro[3,4-c]chinolin-8-carboxamid; und
4-Amino-N-((1R)-1-(3-Cyano-5-(trifluormethyl)-2-pyridinyl)ethyl)-7-fluoro-N,1-dimethyl-1H-pyrazolo[4,3-c]chinolin-8-carboxamid.

15. Die Verbindung nach Anspruch 14, das Tautomer davon, das Stereoisomer davon oder das pharmazeutisch verträgliche Salz einer der vorgenannten, wobei die Verbindung ausgewählt ist aus:
(3R)-4-Amino-N-ethyl-7-fluoro-3-methyl-N-((1R)-1-(6-(trifluormethyl)-3-pyridazinyl)ethyl)-1,3-dihydrofuro[3,4-c]chinolin-8-carboxamid;
4-Amino-N,1-dimethyl-N-((5-(trifluormethyl)-2-pyridinyl)methyl)-1H-pyrazolo[4,3-c]chinolin-8-carboxamid;
4-Amino-7-fluoro-N-((1R)-1-(3-fluoro-5-(trifluormethyl)-2-pyridinyl)ethyl)-N,1-dimethyl-1H-pyrazolo[4,3-c]chinolin-8-carboxamid;
4-Amino-N,1-dimethyl-N-(4-(pentafluoro-lambda-6~-sulfanyl)benzyl)-1H-pyrazolo[4,3-c][1,7]naphthyridin-8-carboxamid;
4-Amino-7-fluoro-N,1-dimethyl-N-((1R)-1-(6-(trifluormethyl)-3-pyridazinyl)ethyl)-1H-pyrazolo[4,3-c]chinolin-8-carboxamid;
(3R)-4-Amino-7-fluoro-N,3-dimethyl-N-((1R)-1-(6-(trifluormethyl)-3-pyridazinyl)ethyl)-1,3-dihydrofuro[3,4-c]chinolin-8-carboxamid;
4-Amino-7-fluoro-N-methyl-N-((1R)-1-(6-(trifluormethyl)-3-pyridazinyl)ethyl)-1,3-dihydrofuro[3,4-c]chinolin-8-carboxamid;
5-Amino-N-(2-propanyl)-N-((5-(trifluormethyl)-2-pyridinyl)methyl)pyrido[4,3-c][1,7]naphthyridin-9-carboxamid;
4-Amino-N-ethyl-7-fluoro-1-methyl-N-((5-(trifluormethyl)-2-pyridinyl)methyl)-1H-pyrazolo[4,3-c]chinolin-8-carboxamid;
4-Amino-7-chloro-N-((1R)-1-(5-fluoro-2-pyridinyl)ethyl)-N,1-dimethyl-1H-pyrazolo[4,3-c]chinolin-8-carboxamid;
4-Amino-N-cyclobutyl-N-((6-(Trifluormethyl)-3-pyridazinyl)methyl)-1,3-dihydrofuro[3,4-c]chinolin-8-carboxamid;
4-Amino-N-methyl-N-((5-(trifluormethyl)-2-pyridinyl)methyl)-1,3-dihydrofuro[3,4-c]chinolin-8-carboxamid;
4-Amino-N-cyclopropyl-1-methyl-N-((5-(trifluormethyl)-2-pyridinyl)methyl)-1H-pyrazolo[4,3-c]chinolin-8-carboxamid;
4-Amino-N-ethyl-7-fluoro-1-methyl-N-((6-(trifluormethyl)-3-pyridazinyl)methyl)-1H-pyrazolo[4,3-c]chinolin-8-carboxamid;
4-Amino-N-cyclopropyl-7-fluoro-1-methyl-N-((6-(trifluormethyl)-3-pyridazinyl)methyl)-1H-pyrazolo[4,3-c]chinolin-8-carboxamid;
4-Amino-7-fluoro-N,1-dimethyl-N-((1R)-1-(5-(trifluormethyl)-2-pyridinyl)ethyl)-1H-pyrazolo[4,3-c]chinolin-8-carboxamid;
(3R)-4-Amino-N-ethyl-3-methyl-N-((1R)-1-(6-(Trifluormethyl)-3-pyridazinyl)ethyl)-1,3-dihydrofuro[3,4-c]chinolin-8-carboxamid;
4-Amino-N-cyclopropyl-7-fluoro-1-methyl-N-((5-(trifluormethyl)-2-pyridinyl)methyl)-1H-pyrazolo[4,3-c]chinolin-8-carboxamid;
4-Amino-7-fluoro-N,1-dimethyl-N-((5-(trifluormethyl)-2-pyridinyl)methyl)-1H-pyrazolo[4,3-c]chinolin-8-carboxamid und
4-Amino-N-ethyl-1-methyl-N-((5-(trifluormethyl)-2-pyridinyl)methyl)-1H-pyrazolo[4,3-c]chinolin-8-carboxamid.

16. Eine pharmazeutische Zusammensetzung, umfassend die Verbindung nach einem der Ansprüche 1, 14 oder 15, das Tautomer davon, das Stereoisomer davon oder das pharmazeutisch verträgliche Salz einer der vorstehenden Verbindungen oder ein pharmazeutisch verträgliches Salz davon, und mindestens einen pharmazeutisch verträglichen Hilfsstoff.

17. Die Verbindung nach einem der Ansprüche 1, 14 oder 15, das Tautomer davon, das Stereoisomer davon oder das pharmazeutisch verträgliche Salz einer der vorgenannten zur Verwendung bei der Behandlung von Krebs, wobei der Krebs vorzugsweise ausgewählt ist aus Eierstock-, Lungen-, Lymphoid-, Glioblastom-, Dickdarm-, Melanom-, Magen-, Bauchspeicheldrüsen- oder Blasenkrebs.

## Revendications

1. Composé de Formule I 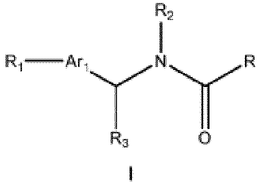 tautomère de celui-ci, stéréoisomère de celui-ci, ou sel pharmaceutiquement acceptable de l'un quelconque des précédents, dans lequel :
R est un cycle triple choisi indépendamment parmi les formules IA et IB : 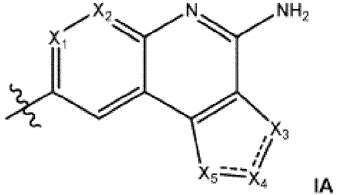 et 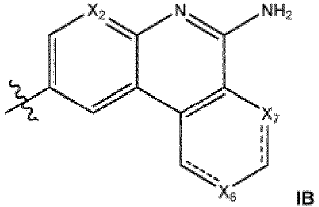
dans lequel 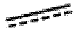 est une liaison simple ou double,
X¹, X², X⁶ et X⁷ sont, à chaque occurrence, N ou C, dans lequel X¹ et X² ne peuvent pas être tous deux N en même temps, et dans lequel, si X¹ est C, il peut être éventuellement substitué par un halogène ;
X³, X⁴ et X⁵ sont, à chaque occurrence, choisis indépendamment parmi un atome de C, O, N et S éventuellement substitué ; dans lequel les substituants sont choisis indépendamment parmi un alkyle en C₁₋₃, un alkyle en C₁₋₃(OH), dans lequel l'alkyle peut être éventuellement substitué par un halogène ;
R³, à chaque occurrence, est choisi indépendamment parmi H ou un alkyle en C₁₋₃ ;
Ar¹ est un aryle ou un hétéroaryle à six chaînons éventuellement substitué choisi indépendamment parmi : 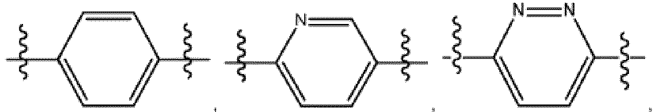 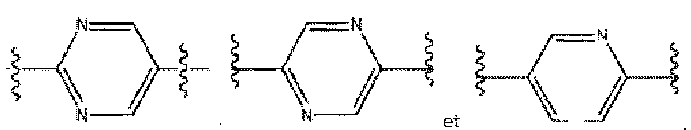
dans lequel les substituants sont choisis indépendamment parmi un alkyle en C₁₋₃, un -O-alkyle en C₁₋₃ ou un halogène ;
R¹, à chaque occurrence, est choisi indépendamment parmi H, un halogène, un alkyle en C₁₋₃ éventuellement substitué, dans lequel les substituants sont choisis parmi un halogène ; -CN, -O-alkyle en C₁₋₃ éventuellement substitué, dans lequel les substituants sont choisis parmi un halogène ; -C(O)O-alkyle en C₁₋₃, dans lequel l'alkyle en C₁₋₃ éventuellement substitué par un halogène, et un morpholinyle ; et
R², à chaque occurrence, est choisi indépendamment parmi un alkyle en C₁₋₈ éventuellement substitué, dans lequel les substituants sont choisis parmi un halogène, un hydroxy, un amino, -O-alkyle en C₁₋₃ ou -CN ; un cycle ou un hétérocycle de 5 ou 6 chaînons, éventuellement substitué par un hydroxy, un amino, un alkyle en C₁₋₆ éventuellement substitué, dans lequel les substituants sont choisis parmi un halogène ; un alkyle en C₁₋₆-O-alkyle en C₁₋₃ éventuellement substitué, dans lequel les substituants sont choisis parmi un halogène ; un 5,6,7,8-tétrahydro-[1,2,4]triazolo[1,5-a]pyridynyle ; un alkyle en C₁₋₃-hétéro-cyclyle, dans lequel l'hétérocyclyle est choisi parmi un 3,4-dihydro-2H-pyrano[2,3-c]py-ridynyle ; un pyradazinyle, un triazolyle, un pyrimidinyle, un tétrahydrofuranyle, un 1H-pyr-rolo[2,3-b]pyridinyle, un cyclohexyle éventuellement substitués ; dans lequel les substituants sont choisis parmi un alkyle en C₁₋₃, -CN et un halogène, ou un alkyle en C₁₋₆-O-alkyle en C₁₋₃ éventuellement substitué, dans lequel les substituants sont choisis parmi un halogène ; un phényle éventuellement substitué, dans lequel les substituants sont choisis parmi un halogène ou un alkyle en C₁₋₃.

2. Composé selon la revendication 1, tautomère de celui-ci, stéréoisomère de celui-ci, ou sel pharmaceutiquement acceptable de l'un quelconque des précédents, dans lequel R¹ est un cycle triple de formule IA.

3. Composé selon la revendication 1, tautomère de celui-ci, stéréoisomère de celui-ci, ou sel pharmaceutiquement acceptable de l'un quelconque des précédents, dans lequel R¹ est un cycle triple de formule IB.

4. Composé selon la revendication 1, tautomère de celui-ci, stéréoisomère de celui-ci, ou sel pharmaceutiquement acceptable de l'un quelconque des précédents, dans lequel R est un cycle triple de formule IA et X¹ et X² sont tous les deux C.

5. Composé selon la revendication 4, tautomère de celui-ci, stéréoisomère de celui-ci, ou sel pharmaceutiquement acceptable de l'un quelconque des précédents, dans lequel X¹ est non substitué ou substitué par un halogène.

6. Composé selon la revendication 1, tautomère de celui-ci, stéréoisomère de celui-ci, ou sel pharmaceutiquement acceptable de l'un quelconque des précédents, dans lequel R est un cycle triple de formule IB et X² est C ou N.

7. Composé selon la revendication 4, tautomère de celui-ci, stéréoisomère de celui-ci, ou sel pharmaceutiquement acceptable de l'un quelconque des précédents, dans lequel R est un cycle triple de formule IA1 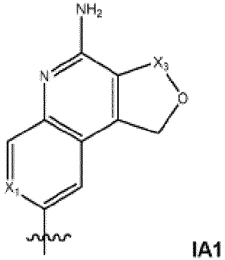 ou R est un cycle triple de formule IA2

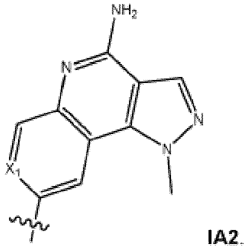

8. Composé selon la revendication 7, tautomère de celui-ci, stéréoisomère de celui-ci, ou sel pharmaceutiquement acceptable de l'un quelconque des précédents, dans lequel X³ est C, non substitué ou substitué par un méthyle.

9. Composé selon la revendication 1, tautomère de celui-ci, stéréoisomère de celui-ci, ou sel pharmaceutiquement acceptable de l'un quelconque des précédents, dans lequel R³ est H ou un méthyle.

10. Composé selon la revendication 7, tautomère de celui-ci, stéréoisomère de celui-ci, ou sel pharmaceutiquement acceptable de l'un quelconque des précédents, dans lequel R² est un alkyle en C₁₋₈ éventuellement substitué, de préférence un méthyle, éthyle, isopropyle ou cycloalkyle en C₁₋₆ éventuellement substitué.

11. Composé selon la revendication 10, tautomère de celui-ci, stéréoisomère de celui-ci, ou sel pharmaceutiquement acceptable de l'un quelconque des précédents, dans lequel Ar¹ est

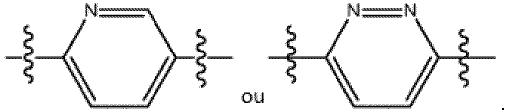

12. Composé selon la revendication 11, dans lequel Ar¹ est 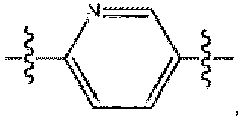 tautomère de celui-ci, stéréoisomère de celui-ci, ou sel pharmaceutiquement acceptable de l'un quelconque des précédents, dans lequel R¹ est un halogène, ou un alkyle en C₁₋₃ éventuellement substitué ou -CN.

13. Composé selon la revendication 1, tautomère de celui-ci, stéréoisomère de celui-ci, ou sel pharmaceutiquement acceptable de l'un quelconque des précédents, dans lequel R est un cycle triple de formule IB et R¹ est un halogène, un alkyle en C₁₋₃ éventuellement substitué ou -CN.

14. Composé, tautomère de celui-ci, stéréoisomère de celui-ci, ou sel pharmaceutiquement acceptable de l'un quelconque des précédents, dans lequel le composé est choisi parmi :
4-amino-N-(cyclopropylméthyl)-N-((5-(trifluorométhyl)-2-pyridinyl)méthyl)-1,3-dihydro-furo[3,4-c]quinoline-8-carboxamide ;
4-amino-N-((5-cyano-2-pyridinyl)méthyl)-N-(2-propanyl)-1,3-dihydrofuro[3,4-c]quinoline-8-carboxamide ;
4-amino-N-((6-cyclopropyl-3-pyridinyl)méthyl)-N-(2-propanyl)-1,3-dihydrofuro[3,4-c]quino-line-8-carboxamide ;
4-amino-N-((2R)-1-méthoxy-2-propanyl)-N-((6-(trifluorométhyle)-3-pyridazinyl)méthyl)-1,3-dihydrofuro[3,4-c]quinoline-8-carboxamide ;
4-amino-N-((2S)-1-méthoxy-2-propanyl)-N-((6-(trifluorométhyl)-3-pyridazinyl)méthyl)-1,3-dihy-drofuro[3,4-c]quinoline-8-carboxamide ;
(3R)-4-amino-N-((2R)-1-méthoxy-2-propanyl)-3-méthyl-N-((6-(trifluorométhyl)-3-pyrida-zinyl)méthyl)-1,3-dihydrofuro[3,4-c]quinoline-8-carboxamide ;
(3R)-4-amino-N-((2S)-1-méthoxy-2-propanyl)-3-méthyl-N-((6-(trifluorométhyl)-3-pyrida-zinyl)méthyl)-1,3-dihydrofuro[3,4-c]quinoline-8-carboxamide ;
4-amino-N-((6-chloro-3-pyridazinyl)méthyl)-N-((2R)-1-méthoxy-2-propanyl)-1,3-dihydro-furo[3,4-c]quinoline-8-carboxamide ;
4-amino-N-((6-chloro-3-pyridazinyl)méthyl)-N-((2R)-1-méthoxy-2-propanyl)-1,3-dihydro-furo[3,4-c][1,7]naphtyridine-8-carboxamide ;
(3R)-4-amino-N-((6-chloro-3-pyridazinyl)méthyl)-N-((2R)-1-méthoxy-2-propanyl)-3-méthyl-1,3-dihydrofuro[3,4-c]quinoline-8-carboxamide ;
(3R)-4-amino-N-((6-chloro-3-pyridazinyl)méthyl)-N-((2R)-1-méthoxy-2-propanyl)-3-méthyl-1,3-dihydrofuro[3,4-c][1,7]naphtyridine-8-carboxamide ;
(3S)-4-amino-N-(bicyclo[1.1.1]pentan-1-yl)-3-méthyl-N-((5-(trifluorométhyl)-2-pyridinyl)méthyl)-1,3-dihydrofuro[3,4-c][1,7]naphtyridine-8-carboxamide ;
(3R)-4-amino-3-méthyl-N-(2-propanyl)-N-((5-(trifluorométhyl)-2-pyridinyl)méthyl)-1,3-dihydro-furo[3,4-c][1,7]naphtyridine-8-carboxamide ;
(3R)-4-amino-N-(bicyclo[1.1.1]pentan-1-yl)-3-méthyl-N-((5-(trifluorométhyl)-2-pyridinyl)méthyl)-1,3-dihydrofuro[3,4-c][1,7]naphtyridine-8-carboxamide ;
(3R)-4-amino-N-((5-cyano-2-pyridinyl)méthyl)-N,3-diméthyl-1,3-dihydrofuro[3,4-c][1,7]naphtyri-dine-8-carboxamide ;
(3R)-4-amino-3-méthyl-N-(2-propanyl)-N-((6-(trifluorométhyl)-3-pyridazinyl)méthyl)-1,3-dihy-drofuro[3,4-c][1,7]naphtyridine-8-carboxamide ;
(3R)-4-amino-N-((2R)-1-méthoxy-2-propanyl)-3-méthyl-N-((6-(trifluorométhyl)-3-pyrida-zinyl)méthyl)-1,3-dihydrofuro[3,4-c][1,7]naphtyridine-8-carboxamide ;
(3R)-4-amino-N-((2S)-1-méthoxy-2-propanyl)-3-méthyl-N-((6-(trifluorométhyl)-3-pyrida-zinyl)méthyl)-1,3-dihydrofuro[3,4-c][1,7]naphtyridine-8-carboxamide ;
(3R)-4-amino-N-cyclopropyl-3-méthyl-N-((5-(trifluorométhyl)-2-pyridinyl)méthyl)-1,3-dihydro-furo[3,4-c][1,7]naphtyridine-8-carboxamide ;
(3R)-4-amino-N-((5-cyano-2-pyridinyl)méthyl)-N-cyclopropyl-3-méthyl-1,3-dihydrofuro[3,4-c][1,7]naphtyridine-8-carboxamide ;
(3R)-4-amino-N,3-diméthyl-N-((6-(trifluorométhyl)-3-pyridazinyl)méthyl)-1,3-dihydrofuro[3,4-c][1,7]naphtyridine-8-carboxamide ;
(3S)-4-amino-N-cyclopropyl-3-méthyl-N-((5-(trifluorométhyl)-2-pyridinyl)méthyl)-1,3-dihydro-furo[3,4-c][1,7]naphtyridine-8-carboxamide ;
(3S)-4-amino-N,3-diméthyl-N-((6-(trifluorométhyl)-3-pyridazinyl)méthyl)-1,3-dihydrofuro[3,4-c][1,7]naphtyridine-8-carboxamide ;
(3R)-4-amino-3-méthyl-N-(2,2,2-trifluoroéthyl)-N-((5-(trifluorométhyl)-2-pyridinyl)méthyl)-1,3-dihydrofuro[3,4-c][1,7]naphtyridine-8-carboxamide ;
4-amino-N-(cyclopropylméthyl)-N-((5-(trifluorométhyl)-2-pyridinyl)méthyl)-1,3-dihydrofuro[3,4-c][1,8]naphtyridine-8-carboxamide ;
4-amino-N-((2R)-1-méthoxy-2-propanyl)-N-((5-(trifluorométhyl)-2-pyridinyl)méthyl)-1,3-dihydro-furo[3,4-c][1,8]naphtyridine-8-carboxamide ;
4-amino-N-((5-cyclopropyl-2-pyridinyl)méthyl)-N-méthyl-1,3-dihydrofuro[3,4-c]quinoline-8-car-boxamide ;
4-amino-N-((5-cyano-2-pyridinyl)méthyl)-N-méthyl-1,3-dihydrofuro[3,4-c]quinoline-8-carboxa-mide ;
4-amino-N-((5-cyano-2-pyridinyl)méthyl)-N-(cyclopropylméthyl)-1,3-dihydrofuro[3,4-c]quino-line-8-carboxamide ;
(3R)-4-amino-N-((6-bromo-3-pyridazinyl)méthyl)-N-(cyclopropylméthyl)-3-méthyl-1,3-dihydro-furo[3,4-c]quinoline-8-carboxamide ;
4-amino-N-((6-bromo-3-pyridazinyl)méthyl)-N-méthyl-1,3-dihydrofuro[3,4-c]quinoline-8-carboxamide ;
(3R)-4-amino-N-((5-bromo-2-pyridinyl)méthyl)-N,3-diméthyl-1,3-dihydrofuro[3,4-c]quinoline-8-carboxamide ;
(3R)-4-amino-N-((6-cyclopropyl-3-pyridazinyl)méthyl)-3-méthyl-N-((1R)-1-(2-pyrimidinyl)éthyl)-1,3-dihydrofuro[3,4-c]quinoline-8-carboxamide ;
4-amino-N-((5-bromo-2-pyridinyl)méthyl)-N-méthyl-1,3-dihydrofuro[3,4-c]quinoline-8-carboxa-mide ;
(3R)-4-amino-N-((5-cyano-2-pyridinyl)méthyl)-N-(cyclopropylméthyl)-3-méthyl-1,3-dihydro-furo[3,4-c]quinoline-8-carboxamide ;
(3R)-4-amino-N-((6-bromo-3-pyridazinyl)méthyl)-3-méthyl-N-(2-propanyl)-1,3-dihydrofuro[3,4-c]quinoline-8-carboxamide ;
(3R)-4-amino-N-(cyclopropylméthyl)-3-méthyl-N-((6-(trifluorométhyl)-3-pyridazinyl)méthyl)-1,3-dihydrofuro[3,4-c]quinoline-8-carboxamide ;
(3R)-4-amino-N-(cyclopropylméthyl)-3-méthyl-N-((5-(trifluorométhyl)-2-pyridinyl)méthyl)-1,3-dihydrofuro[3,4-c]quinoline-8-carboxamide ;
4-amino-N-((6-bromo-3-pyridazinyl)méthyl)-N-(2-propanyl)-1,3-dihydrofuro[3,4-c]quinoline-8-carboxamide ;
4-amino-N-(cyclopropylméthyl)-N-((6-(trifluorométhyl)-3-pyridazinyl)méthyl)-1,3-dihydro-furo[3,4-c]quinoline-8-carboxamide ;
4-amino-N-((1-méthylcyclopropyl)méthyl)-N-((5-(trifluorométhyl)-2-pyridinyl)méthyl)-1,3-dihy-drofuro[3,4-c]quinoline-8-carboxamide ;
(3R)-4-amino-N-(2,2-diméthylpropyl)-3-méthyl-N-((5-(trifluorométhyl)-2-pyridinyl)méthyl)-1,3-dihydrofuro[3,4-c]quinoline-8-carboxamide ;
(3R)-4-amino-3-méthyl-N-((1-méthylcyclopropyl)méthyl)-N-((5-(trifluorométhyl)-2-pyridinyl)mé-thyl)-1,3-dihydrofuro[3,4-c]quinoline-8-carboxamide ;
4-amino-N-(2,2-diméthylpropyl)-3,3-diméthyl-N-((5-(trifluorométhyl)-2-pyridinyl)méthyl)-1,3-di-hydrofuro[3,4-c]quinoline-8-carboxamide ;
4-amino-3,3-diméthyl-N-((1-méthylcyclopropyl)méthyl)-N-((5-(trifluorométhyl)-2-pyridinyl)mé-thyl)-1,3-dihydrofuro[3,4-c]quinoline-8-carboxamide ;
4-amino-N-(2,2-diméthylpropyl)-N-((5-(trifluorométhyl)-2-pyridinyl)méthyl)-1,3-dihydrofuro[3,4-c]quinoline-8-carboxamide ;
4-amino-N-cyclopropyl-N-((5-cyclopropyl-2-pyridinyl)méthyl)-1,3-dihydrofuro[3,4-c]quinoline-8-carboxamide ;
4-amino-N-méthyl-N-((5-(trifluorométhyl)-2-pyridinyl)méthyl)-1,3-dihydrofuro[3,4-c][1,7]naphty-ridine-8-carboxamide ;
4-amino-7-fluoro-N-(2-propanyl)-N-((5-(trifluorométhyl)-2-pyridinyl)méthyl)-1,3-dihydrofuro[3,4-c]quinoline-8-carboxamide ;
4-amino-N-((5-cyano-2-pyridinyl)méthyl)-N-(2-propanyl)-1,3-dihydrofuro[3,4-c][1,7]naphtyri-dine-8-carboxamide ;
4-amino-N-((5-cyano-2-pyridinyl)méthyl)-N-cyclopropyl-1,3-dihydrofuro[3,4-c][1,7]naphtyridine-8-carboxamide ;
4-amino-N-cyclopropyl-N-((5-cyclopropyl-2-pyridinyl)méthyl)-1,3-dihydrofuro[3,4-c][1,7]naphty-ridine-8-carboxamide ;
4-amino-N-((5-cyclopropyl-2-pyridinyl)méthyl)-N-méthyl-1,3-dihydrofuro[3,4-c][1,7]naphtyri-dine-8-carboxamide ;
(3R)-4-amino-N-((6-cyclopropyl-3-pyridinyl)méthyl)-3-méthyl-N-(2-propanyl)-1,3-dihydro-furo[3,4-c]quinoline-8-carboxamide ;
(3R)-4-amino-N-cyclopropyl-3-méthyl-N-((5-(trifluorométhyl)-2-pyridinyl)méthyl)-1,3-dihydro-furo[3,4-c]quinoline-8-carboxamide ;
4-amino-N-méthyl-N-((6-(trifluorométhyl)-3-pyridazinyl)méthyl)-1,3-dihydrofuro[3,4-c]quinoline-8-carboxamide ;
(3R)-4-amino-N-cyclopropyl-3-méthyl-N-((6-(trifluorométhyl)-3-pyridazinyl)méthyl)-1,3-dihydro-furo[3,4-c]quinoline-8-carboxamide ;
(3R)-4-amino-N-((5-cyano-2-pyridinyl)méthyl)-3-méthyl-N-(2-propanyl)-1,3-dihydrofuro[3,4-c]quinoline-8-carboxamide ;
(3R)-4-amino-N-((5-cyano-2-pyridinyl)méthyl)-N-cyclopropyl-3-méthyl-1,3-dihydrofuro[3,4-c]quinoline-8-carboxamide ;
(3R)-4-amino-N-((5-cyclopropyl-2-pyridinyl)méthyl)-N,3-diméthyl-1,3-dihydrofuro[3,4-c]quino-line-8-carboxamide ;
4-amino-N-cyclopropyl-7-fluoro-N-((6-(trifluorométhyl)-3-pyridazinyl)méthyl)-1,3-dihydro-furo[3,4-c]quinoline-8-carboxamide ;
4-amino-7-fluoro-N-(2-propanyl)-N-((6-(trifluorométhyl)-3-pyridazinyl)méthyl)-1,3-dihydro-furo[3,4-c]quinoline-8-carboxamide ;
4-amino-7-fluoro-N-méthyl-N-((6-(trifluorométhyl)-3-pyridazinyl)méthyl)-1,3-dihydrofuro[3,4-c]quinoline-8-carboxamide ;
(3R)-4-amino-N-éthyl-3-méthyl-N-((5-(trifluorométhyl)-2-pyridinyl)méthyl)-1,3-dihydrofuro[3,4-c]quinoline-8-carboxamide ;
4-amino-N-éthyl-N-((5-(trifluorométhyl)-2-pyridinyl)méthyl)-1,3-dihydrofuro[3,4-c][1,7]naphtyri-dine-8-carboxamide ;
4-amino-N-éthyl-N-((5-(trifluorométhyl)-2-pyridinyl)méthyl)-1,3-dihydrofuro[3,4-c]quinoline-8-carboxamide ;
4-amino-N-((6-cyclopropyl-3-pyridinyl)méthyl)-7-fluoro-N-(2-propanyl)-1,3-dihydrofuro[3,4-c]quinoline-8-carboxamide ;
4-amino-N-((5-cyclopropyl-2-pyridinyl)méthyl)-N-(2-propanyl)-1,3-dihydrofuro[3,4-c]quinoline-8-carboxamide ;
4-amino-N-cyclopropyl-N-((5-(trifluorométhyl)-2-pyridinyl)méthyl)-1,3-dihydrofuro[3,4-c][1,7]naphtyridine-8-carboxamide ;
4-amino-N-(2-propanyl)-N-((6-(trifluorométhyl)-3-pyridazinyl)méthyl)-1,3-dihydrofuro[3,4-c][1,7]naphtyridine-8-carboxamide ;
4-amino-N-((5-cyano-2-pyridinyl)méthyl)-N-méthyl-1,3-dihydrofuro[3,4-c][1,7]naphtyridine-8-carboxamide ;
(3R)-4-amino-N-cyclopropyl-N-((5-cyclopropyl-2-pyridinyl)méthyl)-3-méthyl-1,3-dihydro-furo[3,4-c]quinoline-8-carboxamide ;
4-amino-N-cyclopropyl-7-fluoro-N-((5-(trifluorométhyl)-2-pyridinyl)méthyl)-1,3-dihydrofuro[3,4-c]quinoline-8-carboxamide ;
4-amino-N-cyclopropyl-N-((5-cyclopropyl-2-pyridinyl)méthyl)-7-fluoro-1,3-dihydrofuro[3,4-c]qui-noline-8-carboxamide ;
4-amino-N-((5-cyclopropyl-2-pyridinyl)méthyl)-7-fluoro-N-(2-propanyl)-1,3-dihydrofuro[3,4-c]quinoline-8-carboxamide ;
4-amino-N-((5-cyclopropyl-2-pyridinyl)méthyl)-7-fluoro-N-méthyl-1,3-dihydrofuro[3,4-c]quino-line-8-carboxamide ;
4-amino-N-((6-éthoxy-3-pyridazinyl)méthyl)-N-((2R)-1-méthoxy-2-propanyl)-1,3-dihydro-furo[3,4-c]quinoline-8-carboxamide ;
(3R)-4-amino-N-((6-éthoxy-3-pyridazinyl)méthyl)-N-((2R)-1-méthoxy-2-propanyl)-3-méthyl-1,3-dihydrofuro[3,4-c]quinoline-8-carboxamide ;
4-amino-N-méthyl-N-((6-(trifluorométhyl)-3-pyridazinyl)méthyl)-1,3-dihydrofuro[3,4-c][1,7]naph-tyridine-8-carboxamide ;
(3R)-4-amino-N,3-diméthyl-N-((5-(trifluorométhyl)-2-pyridinyl)méthyl)-1,3-dihydrofuro[3,4-c]qui-noline-8-carboxamide ;
(3R)-4-amino-N-((5-cyclopropyl-2-pyridinyl)méthyl)-3-méthyl-N-(2-propanyl)-1,3-dihydro-furo[3,4-c]quinoline-8-carboxamide ;
4-amino-N-(bicyclo[1.1.1]pentan-1-yl)-N-((6-éthoxy-3-pyridazinyl)méthyl)-1,3-dihydrofuro[3,4-c][1,7]naphtyridine-8-carboxamide ;
4-amino-7-chloro-N-((5-cyclopropyl-2-pyridinyl)méthyl)-N-(2-propanyl)-1,3-dihydrofuro[3,4-c]quinoline-8-carboxamide ;
(3R)-4-amino-N-(bicyclo[1.1.1]pentan-1-yl)-3-méthyl-N-((5-(trifluorométhyl)-2-pyridinyl)méthyl)-1,3-dihydrofuro[3,4-c]quinoline-8-carboxamide ;
4-amino-N-(bicyclo[1.1.1]pentan-1-yl)-7-fluoro-N-((5-(trifluorométhyl)-2-pyridinyl)méthyl)-1,3-dihydrofuro[3,4-c]quinoline-8-carboxamide ;
4-amino-N-cyclopropyl-N-((6-(trifluorométhyl)-3-pyridazinyl)méthyl)-1,3-dihydrofuro[3,4-c][1,7]naphtyridine-8-carboxamide ;
4-amino-N-((6-cyclopropyl-3-pyridinyl)méthyl)-N-(2-propanyl)-1,3-dihydrofuro[3,4-c][1,7]naph-tyridine-8-carboxamide ;
(3R)-4-amino-3-méthyl-N-(2-propanyl)-N-((5-(trifluorométhyl)-2-pyridinyl)méthyl)-1,3-dihydro-furo[3,4-c]quinoline-8-carboxamide ;
4-amino-N-((5-cyclopropyl-2-pyridinyl)méthyl)-N-(2-propanyl)-1,3-dihydrofuro[3,4-c][1,7]naph-tyridine-8-carboxamide ;
(3R)-4-amino-N-((5-cyano-2-pyridinyl)méthyl)-N,3-diméthyl-1,3-dihydrofuro[3,4-c]quinoline-8-carboxamide ;
4-amino-N-(bicyclo[1.1.1]pentan-1-yl)-N-((6-éthoxy-3-pyridazinyl)méthyl)-1,3-dihydrofuro[3,4-c]quinoline-8-carboxamide ;
4-amino-N-((6-éthoxy-3-pyridazinyl)méthyl)-N-((2R)-1-méthoxy-2-propanyl)-1,3-dihydro-furo[3,4-c][1,7]naphtyridine-8-carboxamide ;
4-amino-7-chloro-N-méthyl-N-((6-(trifluorométhyl)-3-pyridazinyl)méthyl)-1,3-dihydrofuro[3,4-c]quinoline-8-carboxamide ;
4-amino-N-((2R)-1-méthoxy-2-propanyl)-N-((6-(trifluorométhyl)-3-pyridazinyl)méthyl)-1,3-dihy-drofuro[3,4-c][1,7]naphtyridine-8-carboxamide ;
4-amino-N-((2S)-1-méthoxy-2-propanyl)-N-((6-(trifluorométhyl)-3-pyridazinyl)méthyl)-1,3-dihy-drofuro[3,4-c][1,7]naphtyridine-8-carboxamide ;
4-amino-N-(2-propanyl)-N-((6-(trifluorométhyl)-3-pyridazinyl)méthyl)-1,3-dihydrofuro[3,4-c]qui-noline-8-carboxamide ;
4-amino-7-chloro-N-((5-cyano-2-pyridinyl)méthyl)-N-(2-propanyl)-1,3-dihydrofuro[3,4-c]quino-line-8-carboxamide ;
(3R)-4-amino-3-méthyl-N-(2-propanyl)-N-((6-(trifluorométhyl)-3-pyridazinyl) méthyl)-1,3-dihy-drofuro[3,4-c]quinoline-8-carboxamide ;
4-amino-7-chloro-N-(2-propanyl)-N-((6-(trifluorométhyl)-3-pyridazinyl)méthyl)-1,3-dihydro-furo[3,4-c]quinoline-8-carboxamide ;
4-amino-N-(bicyclo[1.1.1]pentan-1-yl)-7-chloro-N-((5-(trifluorométhyl)-2-pyridinyl)méthyl)-1,3-dihydrofuro[3,4-c]quinoline-8-carboxamide ;
(3R)-4-amino-N-(cyclopropylméthyl)-3-méthyl-N-((5-(trifluorométhyl)-2-pyridinyl)méthyl)-1,3-dihydrofuro[3,4-c][1,7]naphtyridine-8-carboxamide ;
(3R)-4-amino-N-((6-éthoxy-3-pyridazinyl)méthyl)-N-((2R)-1-méthoxy-2-propanyl)-3-méthyl-1,3-dihydrofuro[3,4-c][1,7]naphtyridine-8-carboxamide ;
4-amino-N-((1-méthylcyclopropyl)méthyl)-N-((5-(trifluorométhyl)-2-pyridinyl)méthyl)-1,3-dihy-drofuro[3,4-c][1,7]naphtyridine-8-carboxamide ;
4-amino-N-méthyl-N-(4-(trifluorométhyl)benzyl)-1,3-dihydrofuro[3,4-c]quinoline-8-carboxa-mide ;
4-amino-N-méthyl-N-((6-(trifluorométhyl)-3-pyridinyl)méthyl)-1,3-dihydrofuro[3,4-c]quinoline-8-carboxamide ;
4-amino-7-chloro-N-((6-cyclopropyl-3-pyridinyl)méthyl)-N-(2-propanyl)-1,3-dihydrofuro[3,4-c]quinoline-8-carboxamide ;
(3R)-4-amino-N-(cyclopropylméthyl)-3-méthyl-N-((6-(trifluorométhyl)-3-pyridazinyl)méthyl)-1,3-dihydrofuro[3,4-c][1,7]naphtyridine-8-carboxamide ;
(3S)-4-amino-N-((6-cyclopropyl-3-pyridinyl)méthyl)-3-méthyl-N-(2-propanyl)-1,3-dihydro-furo[3,4-c][1,7]naphtyridine-8-carboxamide ;
(3R)-4-amino-N-((5-cyano-2-pyridinyl)méthyl)-3-méthyl-N-(2-propanyl)-1,3-dihydrofuro[3,4-c][1,7]naphtyridine-8-carboxamide ;
(3R)-4-amino-N-cyclopropyl-3-méthyl-N-((6-(trifluorométhyl)-3-pyridazinyl)méthyl)-1,3-dihydro-furo[3,4-c][1,7]naphtyridine-8-carboxamide ;
(3R)-4-amino-N-((5-cyclopropyl-2-pyridinyl)méthyl)-3-méthyl-N-(2-propanyl)-1,3-dihydro-furo[3,4-c][1,7]naphtyridine-8-carboxamide ;
(3S)-4-amino-N-((5-cyclopropyl-2-pyridinyl)méthyl)-3-méthyl-N-(2-propanyl)-1,3-dihydro-furo[3,4-c][1,7]naphtyridine-8-carboxamide ;
(3R)-4-amino-N-éthyl-3-méthyl-N-((5-(trifluorométhyl)-2-pyridinyl)méthyl)-1,3-dihydrofuro[3,4-c][1,7]naphtyridine-8-carboxamide ;
(3R)-4-amino-N-(bicyclo[1.1.1]pentan-1-yl)-N-((6-éthoxy-3-pyridazinyl)méthyl)-3-méthyl-1,3-dihydrofuro[3,4-c][1,7 naphtyridine-8-carboxamide ;
(3R)-4-amino-N-((6-bromo-3-pyridazinyl)méthyl)-N-((2R)-1-méthoxy-2-propanyl)-3-méthyl-1,3-dihydrofuro[3,4-c][1,7]naphtyridine-8-carboxamide ;
(3R)-4-amino-N,3-diméthyl-N-((5-(trifluorométhyl)-2-pyridinyl)méthyl)-1,3-dihydrofuro[3,4-c][1,7]naphtyridine-8-carboxamide ;
(3R)-4-amino-N-((6-cyclopropyl-3-pyridinyl)méthyl)-3-méthyl-N-(2-propanyl)-1,3-dihydro-furo[3,4-c][1,7]naphtyridine-8-carboxamide ;
4-(6-((((4-amino-1,3-dihydrofuro[3,4-c]quinolin-8-yl)carbonyl)(méthyl)amino)méthyl)-3-pyri-dinyl)-1-piperazinecarboxylate de méthyle ;
(3S)-4-amino-N-((5-cyclopropyl-2-pyridinyl)méthyl)-N,3-diméthyl-1,3-dihydrofuro[3,4-c][1,7]naphtyridine-8-carboxamide ;
(3R)-4-amino-N-((5-cyclopropyl-2-pyridinyl)méthyl)-N,3-diméthyl-1,3-dihydrofuro[3,4-c][1,7]naphtyridine-8-carboxamide ;
(3R)-4-amino-N-cyclopropyl-N-((5-cyclopropyl-2-pyridinyl)méthyl)-3-méthyl-1,3-dihydro-furo[3,4-c][1,7]naphtyridine-8-carboxamide ;
(3S)-4-amino-N-cyclopropyl-N-((5-cyclopropyl-2-pyridinyl)méthyl)-3-méthyl-1,3-dihydro-furo[3,4-c][1,7]naphtyridine-8-carboxamide ;
4-amino-N-(cyclopropylméthyl)-N-((6-(difluorométhoxy)-3-pyridazinyl)méthyl)-1,3-dihydro-furo[3,4-c][1,7]naphtyridine-8-carboxamide ;
4-amino-N-éthyl-7-fluoro-N-((5-(trifluorométhyl)-2-pyridinyl)méthyl)-1,3-dihydrofuro[3,4-c]qui-noline-8-carboxamide ;
4-amino-7-chloro-N-éthyl-N-((5-(trifluorométhyl)-2-pyridinyl)méthyl)-1,3-dihydrofuro[3,4-c]qui-noline-8-carboxamide ;
(3R)-4-amino-N-((5-chloro-2-pyridinyl)méthyl)-N-éthyl-3-méthyl-1,3-dihydrofuro[3,4-c]quino-line-8-carboxamide ;
4-amino-N-éthyl-3-méthyl-N-((5-(trifluorométhyl)-2-pyridinyl)méthyl)-3H-pyrazolo[3,4-c]quino-line-8-carboxamide ;
(3R)-4-amino-N-((5-chloro-2-pyridinyl)méthyl)-3-méthyl-N-(2-propanyl)-1,3-dihydrofuro[3,4-c]quinoline-8-carboxamide ;
(3R)-4-amino-N-(cyclopropylméthyl)-N-((6-(difluorométhoxy)-3-pyridazinyl)méthyl)-3-méthyl-1,3-dihydrofuro[3,4-c][1,7]naphtyridine-8-carboxamide ;
(3S)-4-amino-N-(cyclopropylméthyl)-N-((6-(difluorométhoxy)-3-pyridazinyl)méthyl)-3-méthyl-1,3-dihydrofuro[3,4-c][1,7]naphtyridine-8-carboxamide ;
4-amino-N-(cyclopropylméthyl)-N-((6-(difluorométhoxy)-3-pyridazinyl)méthyl)-1,3-dihydro-furo[3,4-c]quinoline-8-carboxamide ;
4-amino-N-éthyl-7-fluoro-3-méthyl-N-((5-(trifluorométhyl)-2-pyridinyl)méthyl)-3H-pyrazolo[3,4-c]quinoline-8-carboxamide ;
5-amino-N-éthyl-N-((5-(trifluorométhyl)-2-pyridinyl)méthyl)benzo[c][2,6]naphtyridine-9-carboxa-mide ;
4-amino-N-((5-(3,6-dihydro-2H-pyran-4-yl)-2-pyridinyl)méthyl)-N-méthyl-1,3-dihydrofuro[3,4-c]quinoline-8-carboxamide ;
6-((((4-amino-1,3-dihydrofuro[3,4-c]quinolin-8-yl)carbonyl)(méthyl)amino)méthyl)-3',6'-dihy-dro[3,4'-bipyridine]-1'(2'H)-carboxylate de méthyle ;
5-oxo-N-(2-propanyl)-N-((5-(trifluorométhyl)-2-pyridinyl)méthyl)-5,6-dihydropyrazolo[1,5-c]qui-nazoline-9-carboxamide ;
4-amino-1,3-diméthyl-N-(2-propanyl)-N-((5-(trifluorométhyl)-2-pyridinyl)méthyl)-1H-pyra-zolo[4,3-c]quinoline-8-carboxamide ;
(3R)-4-amino-N-((5-bromo-2-pyridinyl)méthyl)-N,3-diméthyl-1,3-dihydrofuro[3,4-c][1,7]naphtyri-dine-8-carboxamide ;
(3S)-4-amino-N-((5-bromo-2-pyridinyl)méthyl)-N,3-diméthyl-1,3-dihydrofuro[3,4-c][1,7]naphtyri-dine-8-carboxamide ;
(3R)-4-amino-3-méthyl-N-(2-propanyl)-N-((5-(trifluorométhyl)-2-pyrazinyl)méthyl)-1,3-dihydro-furo[3,4-c]quinoline-8-carboxamide ;
4-amino-7-fluoro-N-((1R)-1-(2-pyrimidinyl)éthyl)-N-((5-(trifluorométhyl)-2-pyridinyl)méthyl)-1,3-dihydrofuro[3,4-c]quinoline-8-carboxamide ;
4-amino-N-((5-cyano-2-pyridinyl)méthyl)-7-fluoro-N-((1R)-1-(2-pyrimidinyl)éthyl)-1,3-dihydro-furo[3,4-c]quinoline-8-carboxamide ;
4-amino-N-((6-bromo-3-pyridazinyl)méthyl)-7-fluoro-N-((1R)-1-(3-fluoro-2-pyridinyl)éthyl)-1,3-dihydrofuro[3,4-c]quinoline-8-carboxamide ;
4-amino-N-((5-(difluorométhyl)-2-pyridinyl)méthyl)-N-((1R)-1-(2-pyrimidinyl)éthyl)-1,3-dihydro-furo[3,4-c]quinoline-8-carboxamide ;
4-amino-N-((5-chloro-2-pyridinyl)méthyl)-N-((1R)-1-(2-pyrimidinyl)éthyl)-1,3-dihydrofuro[3,4-c]quinoline-8-carboxamide ;
4-amino-N-((1S)-1-(2-pyrimidinyl)éthyl)-N-((6-(trifluorométhyl)-3-pyridazinyl)méthyl)-1,3-dihy-drofuro[3,4-c]quinoline-8-carboxamide ;
4-amino-N-((1R)-1-(2-pyrimidinyl)éthyl)-N-((6-(trifluorométhyl)-3-pyridazinyl)méthyl)-1,3-dihy-drofuro[3,4-c]quinoline-8-carboxamide ;
4-amino-N-((1R)-1-(3-fluoro-2-pyridinyl)éthyl)-N-((6-(trifluorométhyl)-3-pyridazinyl)méthyl)-1,3-dihydrofuro[3,4-c]quinoline-8-carboxamide ;
4-amino-N-cyclopropyl-N-((5-(trifluorométhyl)-2-pyridinyl)méthyl)-1,3-dihydrofuro[3,4-c]quino-line-8-carboxamide ;
4-amino-N-(1,3-diméthoxy-2-propanyl)-N-((5-(trifluorométhyl)-2-pyridinyl)méthyl)-1,3-dihydro-furo[3,4-c]quinoline-8-carboxamide ;
4-amino-N-((2R)-1-méthoxy-2-propanyl)-N-((5-(trifluorométhyl)-2-pyridinyl)méthyl)-1,3-dihydro-furo[3,4-c]quinoline-8-carboxamide ;
4-amino-N-((6-méthoxy-3-pyridazinyl)méthyl)-N-((1R)-1-(2-pyrimidinyl)éthyl)-1,3-dihydro-furo[3,4-c]quinoline-8-carboxamide ;
4-amino-N-((8R)-5,6,7,8-tétrahydro[1,2,4]triazolo[1,5-a]pyridin-8-yl)-N-((5-(trifluorométhyl)-2-pyridinyl)méthyl)-1,3-dihydrofuro[3,4-c]quinoline-8-carboxamide ;
4-amino-N-((8S)-5,6,7,8-tétrahydro[1,2,4]triazolo[1,5-a]pyridin-8-yl)-N-((5-(trifluorométhyl)-2-pyridinyl)méthyl)-1,3-dihydrofuro[3,4-c]quinoline-8-carboxamide ;
4-amino-N-((5-cyano-2-pyridinyl)méthyl)-N-((8R)-5,6,7,8-tétrahydro[1,2,4]triazolo[1,5-a]pyridin-8-yl)-1,3-dihydrofuro[3,4-c]quinoline-8-carboxamide ;
4-amino-N-((5-cyano-2-pyridinyl)méthyl)-N-((8S)-5,6,7,8-tétrahydro[1,2,4]triazolo[1,5-a]pyridin-8-yl)-1,3-dihydrofuro[3,4-c]quinoline-8-carboxamide ;
4-amino-N-(4-(3-oxétanyl)benzyl)-N-((1R)-1-(2-pyrimidinyl)éthyl)-1,3-dihydrofuro[3,4-c]quino-line-8-carboxamide ;
4-amino-N-(4-(3-oxétanyl)benzyl)-N-((1S)-1-(2-pyrimidinyl)éthyl)-1,3-dihydrofuro[3,4-c]quino-line-8-carboxamide ;
4-amino-N-méthyl-N-((5-(trifluorométhyl)-2-pyridinyl)méthyl)-1,3-dihydrofuro[3,4-c]quinoline-8-carboxamide ;
4-amino-N-(2-propanyl)-N-((5-(trifluorométhyl)-2-pyridinyl)méthyl)-1,3-dihydrofuro[3,4-c]quino-line-8-carboxamide ;
4-amino-N-(cyclopropylméthyl)-N-((6-(4-morpholinyl)-3-pyridazinyl)méthyl)-1,3-dihydro-furo[3,4-c]quinoline-8-carboxamide ;
4-amino-N-((1R)-1-(2-pyrimidinyl)éthyl)-N-((6-(2,2,2-trifluoroéthoxy)-3-pyridazinyl)méthyl)-1,3-dihydrofuro[3,4-c]quinoline-8-carboxamide ;
4-amino-N-((5-cyano-2-pyridinyl)méthyl)-N-cyclopropyl-1,3-dihydrofuro[3,4-c]quinoline-8-car-boxamide ;
4-amino-N-((5-bromo-2-pyridinyl)méthyl)-N-cyclopropyl-1,3-dihydrofuro[3,4-c]quinoline-8-car-boxamide ;
4-amino-N-cyclopropyl-N-((5-(difluorométhyl)-2-pyridinyl)méthyl)-1,3-dihydrofuro[3,4-c]quino-line-8-carboxamide ;
4-amino-N-((1-cyanocyclopropyl)méthyl)-N-((5-cyano-2-pyridinyl)méthyl)-1,3-dihydrofuro[3,4-c]quinoline-8-carboxamide ;
4-amino-N-((6-cyclopropyl-3-pyridazinyl)méthyl)-N-((1R)-1-(2-pyrimidinyl)éthyl)-1,3-dihydro-furo[3,4-c]quinoline-8-carboxamide ;
4-amino-N-((3R,4S)-3-méthoxytétrahydro-2H-pyran-4-yl)-N-((5-(trifluorométhyl)-2-pyridinyl)méthyl)-1,3-dihydrofuro[3,4-c]quinoline-8-carboxamide ;
4-amino-N-((3S,4R)-3-méthoxytétrahydro-2H-pyran-4-yl)-N-((5-(trifluorométhyl)-2-pyridinyl)méthyl)-1,3-dihydrofuro[3,4-c]quinoline-8-carboxamide ;
4-amino-N-((3R,4R)-4-méthoxytétrahydro-2H-pyran-3-yl)-N-((5-(trifluorométhyl)-2-pyridinyl)méthyl)-1,3-dihydrofuro[3,4-c]quinoline-8-carboxamide ;
4-amino-N-((3S,4S)-4-méthoxytétrahydro-2H-pyran-3-yl)-N-((5-(trifluorométhyl)-2-pyridinyl)méthyl)-1,3-dihydrofuro[3,4-c]quinoline-8-carboxamide ;
4-amino-7-chloro-N-((5-cyano-2-pyridinyl)méthyl)-N-cyclopropyl-1,3-dihydrofuro[3,4-c]quinoline-8-carboxamide ;
4-amino-N-((6-chloro-5-méthoxy-2-pyridinyl)méthyl)-N-(2-propanyl)-1,3-dihydrofuro[3,4-c]quinoline-8-carboxamide ;
4-amino-N-((6-bromo-3-pyridazinyl)méthyl)-N-((2R)-1-méthoxy-2-propanyl)-1,3-dihydrofuro[3,4-c]quinoline-8-carboxamide ;
4-amino-7-chloro-N-(cyclopropylméthyl)-N-((5-(trifluorométhyl)-2-pyridinyl)méthyl)-1,3-dihydrofuro[3,4-c]quinoline-8-carboxamide ;
(3R)-4-amino-3-méthyl-N-(2-propanyl)-N-((5-(trifluorométhoxy)-2-pyridinyl)méthyl)-1,3-dihydrofuro[3,4-c]quinoline-8-carboxamide ;
(3R)-4-amino-N-((5-(difluorométhyl)-2-pyridinyl)méthyl)-3-méthyl-N-((1R)-1-(1-méthyl-1H-1,2,4-triazol-3-yl)éthyl)-1,3-dihydrofuro[3,4-c]quinoline-8-carboxamide ;
(3R)-4-amino-3-méthyl-N-(1-méthyl-1H-pyrazol-4-yl)-N-((5-(trifluorométhyl)-2-pyridinyl)méthyl)-1,3-dihydrofuro[3,4-c]quinoline-8-carboxamide ;
(3R)-4-amino-N-((6-bromo-3-pyridazinyl)méthyl)-N-((2R)-1-méthoxy-2-propanyl)-3-méthyl-1,3-dihydrofuro[3,4-c]quinoline-8-carboxamide ;
4-amino-N-(3,4-dihydro-2H-pyrano[2,3-c]pyridin-6-ylméthyl)-N-((1R)-1-(2-pyrimidinyl)éthyl)-1,3-dihydrofuro[3,4-c]quinoline-8-carboxamide ;
4-amino-7-chloro-N-méthyl-N-((5-(trifluorométhyl)-2-pyridinyl)méthyl)-1,3-dihydrofuro[3,4-c]quinoline-8-carboxamide ;
4-amino-N-(bicyclo[1.1.1]pentan-1-yl)-N-((5-(trifluorométhyl)-2-pyridinyl)méthyl)-1,3-dihydrofuro[3,4-c]quinoline-8-carboxamide ;
4-amino-7-chloro-N-((5-cyano-2-pyridinyl)méthyl)-N-méthyl-1,3-dihydrofuro[3,4-c]quinoline-8-carboxamide ;
4-amino-N-((5-cyano-2-pyridinyl)méthyl)-N-((2R)-1-fluoro-2-propanyl)-1,3-dihydrofuro[3,4-c]quinoline-8-carboxamide ;
4-amino-N-((5-cyano-2-pyridinyl)méthyl)-N-((2S)-1-fluoro-2-propanyl)-1,3-dihydrofuro[3,4-c]quinoline-8-carboxamide ;
4-amino-7-chloro-N-((5-cyano-2-pyridinyl)méthyl)-N-(cyclopropylméthyl)-1,3-dihydrofuro[3,4-c]quinoline-8-carboxamide ;
4-amino-7-fluoro-N-méthyl-N-((5-(trifluorométhyl)-2-pyridinyl)méthyl)-1,3-dihydrofuro[3,4-c]quinoline-8-carboxamide ;
4-amino-N-((5-cyano-2-pyridinyl)méthyl)-N-cyclopropyl-7-fluoro-1,3-dihydrofuro[3,4-c]quinoline-8-carboxamide ;
4-amino-N-((5-cyano-2-pyridinyl)méthyl)-7-fluoro-N-(2-propanyl)-1,3-dihydrofuro[3,4-c]quinoline-8-carboxamide ;
4-amino-7-chloro-N-((5-cyclopropyl-2-pyridinyl)méthyl)-N-méthyl-1,3-dihydrofuro[3,4-c]quino-line-8-carboxamide ;
4-amino-N-((5-cyano-2-pyridinyl)méthyl)-7-fluoro-N-méthyl-1,3-dihydrofuro[3,4-c]quinoline-8-carboxamide ;
4-amino-7-chloro-N-cyclopropyl-N-((5-cyclopropyl-2-pyridinyl)méthyl)-1,3-dihydrofuro[3,4-c]quinoline-8-carboxamide ;
(3R)-4-amino-3-méthyl-N-(2,2,2-trifluoroéthyl)-N-((5-(trifluorométhyl)-2-pyridinyl)méthyl)-1,3-dihydrofuro[3,4-c]quinoline-8-carboxamide ;
(3R)-4-amino-N-(bicyclo[1.1.1]pentan-1-yl)-N-((6-éthoxy-3-pyridazinyl)méthyl)-3-méthyl-1,3-dihydrofuro[3,4-c]quinoline-8-carboxamide ;
4-amino-N-(2,2,2-trifluoroéthyl)-N-((5-(trifluorométhyl)-2-pyridinyl)méthyl)-1,3-dihydrofuro[3,4-c]quinoline-8-carboxamide ;
4-amino-7-chloro-N-(2-propanyl)-N-((5-(trifluorométhyl)-2-pyridinyl)méthyl)-1,3-dihydrofuro[3,4-c]quinoline-8-carboxamide ;
(3R)-4-amino-N,3-diméthyl-N-((6-(trifluorométhyl)-3-pyridazinyl)méthyl)-1,3-dihydrofuro[3,4-c]quinoline-8-carboxamide ;
4-amino-N-(2,2,2-trifluoroéthyl)-N-((5-(trifluorométhyl)-2-pyridinyl)méthyl)-1,3-dihydrofuro[3,4-c][1,7]naphtyridine-8-carboxamide ;
4-amino-7-chloro-N-((2R)-1-méthoxy-2-propanyl)-N-((6-(trifluorométhyl)-3-pyridazinyl)méthyl)-1,3-dihydrofuro[3,4-c]quinoline-8-carboxamide ;
4-amino-7-chloro-N-((2S)-1-méthoxy-2-propanyl)-N-((6-(trifluorométhyl)-3-pyridazinyl)méthyl)-1,3-dihydrofuro[3,4-c]quinoline-8-carboxamide ;
4-amino-7-fluoro-N-((2R)-1-méthoxy-2-propanyl)-N-((6-(trifluorométhyl)-3-pyridazinyl)méthyl)-1,3-dihydrofuro[3,4-c]quinoline-8-carboxamide ;
4-amino-7-fluoro-N-((2S)-1-méthoxy-2-propanyl)-N-((6-(trifluorométhyl)-3-pyridazinyl)méthyl)-1,3-dihydrofuro[3,4-c]quinoline-8-carboxamide ;
(3R)-4-amino-3-méthyl-N-(tétrahydro-2H-pyran-4-yl)-N-((5-(trifluorométhyl)-2-pyridinyl)méthyl)-1,3-dihydrofuro[3,4-c]quinoline-8-carboxamide ;
4-amino-7-chloro-N-cyclopropyl-N-((6-(trifluorométhyl)-3-pyridazinyl)méthyl)-1,3-dihydro-furo[3,4-c]quinoline-8-carboxamide ;
6-amino-N-(1-méthyl-1H-pyrazol-4-yl)-N-((5-(trifluorométhyl)-2-pyridinyl) méthyl)-8,9-dihydro-7H-cyclopenta[c][1,7]naphtyridine-2-carboxamide ;
6-amino-N-((5-(difluorométhyl)-2-pyridinyl)méthyl)-N-(1-méthyl-1H-pyrazol-4-yl)-8,9-dihydro-7H-cyclopenta[c][1,7]naphtyridine-2-carboxamide ;
4-amino-3-méthyl-N-(1-méthylcyclopropyl)-N-((5-(trifluorométhyl)-2-pyridinyl)méthyl)-3H-pyra-zolo[3,4-c]quinoline-8-carboxamide ;
4-amino-3-méthyl-N-(1-méthyl-1H-pyrazol-4-yl)-N-((5-(trifluorométhyl)-2-pyridinyl)méthyl)-3H-pyrazolo[3,4-c]quinoline-8-carboxamide ;
4-amino-7-fluoro-N-(2-propanyl)-N-((5-(trifluorométhyl)-2-pyrazinyl)méthyl)-1,3-dihydro-furo[3,4-c]quinoline-8-carboxamide ;
4-amino-N-éthyl-7-fluoro-N-((5-(trifluorométhyl)-2-pyrazinyl)méthyl)-1,3-dihydrofuro[3,4-c]qui-noline-8-carboxamide ;
4-amino-7-chloro-N-éthyl-N-((5-(trifluorométhyl)-2-pyrazinyl)méthyl)-1,3-dihydrofuro[3,4-c]qui-noline-8-carboxamide ;
4-amino-7-chloro-N-(2-propanyl)-N-((5-(trifluorométhyl)-2-pyrazinyl)méthyl)-1,3-dihydro-furo[3,4-c]quinoline-8-carboxamide ;
(3R)-4-amino-N-éthyl-3-méthyl-N-((5-(trifluorométhyl)-2-pyrazinyl)méthyl)-1,3-dihydrofuro[3,4-c]quinoline-8-carboxamide ;
4-amino-N-cyclobutyl-7-fluoro-N-((6-(trifluorométhyl)-3-pyridazinyl)méthyl)-1,3-dihydrofuro[3,4-c]quinoline-8-carboxamide ;
4-amino-7-méthoxy-N-méthyl-N-((5-(trifluorométhyl)-2-pyridinyl)méthyl)-1,3-dihydrofuro[3,4-c]quinoline-8-carboxamide ;
4-amino-N,7-diméthyl-N-((5-(trifluorométhyl)-2-pyridinyl)méthyl)-1,3-dihydrofuro[3,4-c]quino-line-8-carboxamide ;
4-amino-N-cyclobutyl-7-fluoro-3-méthyl-N-((6-(trifluorométhyl)-3-pyridazinyl)méthyl)-3H-pyra-zolo[3,4-c]quinoline-8-carboxamide ;
4-amino-7-chloro-N-cyclobutyl-N-((6-(trifluorométhyl)-3-pyridazinyl)méthyl)-1,3-dihydro-furo[3,4-c]quinoline-8-carboxamide ;
4-amino-7-fluoro-3-méthyl-N-(1-méthyl-1H-pyrazol-4-yl)-N-((5-(trifluorométhyl)-2-pyridinyl)méthyl)-3H-pyrazolo[3,4-c]quinoline-8-carboxamide ;
4-amino-1-méthyl-N-(1-méthyl-1H-pyrazol-4-yl)-N-((5-(trifluorométhyl)-2-pyridinyl)méthyl)-1H-pyrazolo[4,3-c][1,7]naphtyridine-8-carboxamide ;
4-amino-N-cyclopropyl-7-fluoro-3-méthyl-N-((1R)-1-(5-(trifluorométhyl)-2-pyridinyl)éthyl)-3H-pyrazolo[3,4-c]quinoline-8-carboxamide ;
4-amino-N-cyclobutyl-N-((6-(difluorométhoxy)-3-pyridazinyl)méthyl)-7-fluoro-1,3-dihydro-furo[3,4-c]quinoline-8-carboxamide ;
4-amino-N-méthyl-N-(4-(pentafluoro-lambda~6~-sulfanyl)benzyl)-1,3-dihydrofuro[3,4-c]quino-line-8-carboxamide ;
4-amino-N-méthyl-N-(4-(pentafluoro-lambda~6~-sulfanyl)benzyl)-1,3-dihydrofuro[3,4-c][1,7]naphtyridine-8-carboxamide ;
4-amino-7-fluoro-N-méthyl-N-(4-(pentafluoro-lambda~6~-sulfanyl)benzyl)-1,3-dihydrofuro[3,4-c]quinoline-8-carboxamide ;
4-amino-7-chloro-N-méthyl-N-(4-(pentafluoro-lambda~6 --sulfanyl)benzyl)-1,3-dihydrofuro[3,4-c]quinoline-8-carboxamide ;
(3R)-4-amino-N,3-diméthyl-N-(4-(pentafluoro-lambda~6~-sulfanyl)benzyl)-1,3-dihydrofuro[3,4-c]quinoline-8-carboxamide ;
4-amino-7-fluoro-N,3-diméthyl-N-(4-(pentafluoro-lambda~6~-sulfanyl)benzyl)-3H-pyrazolo[3,4-c]quinoline-8-carboxamide ;
4-amino-N-((5-fluoro-2-pyridinyl)méthyl)-N,1,7-triméthyl-1H-pyrazolo[4,3-c]quinoline-8-car-boxamide ;
4-amino-N-((6-cyclopropyl-3-pyridazinyl)méthyl)-7-fluoro-N,3-diméthyl-3H-pyrazolo[3,4-c]qui-noline-8-carboxamide ;
4-amino-N-((6-cyclopropyl-3-pyridazinyl)méthyl)-7-fluoro-N,1-diméthyl-1H-pyrazolo[4,3-c]qui-noline-8-carboxamide ;
4-amino-7-chloro-N-((6-cyclopropyl-3-pyridazinyl)méthyl)-N-méthyl-1,3-dihydrofuro[3,4-c]qui-noline-8-carboxamide ;
4-amino-N,3,7-triméthyl-N-((5-(trifluorométhyl)-2-pyridinyl)méthyl)-3H-pyrazolo[3,4-c]quinoline-8-carboxamide ;
4-amino-N,1,7-triméthyl-N-((5-(trifluorométhyl)-2-pyridinyl)méthyl)-1H-pyrazolo[4,3-c]quinoline-8-carboxamide ;
4-amino-N-((5-cyclopropyl-2-pyridinyl)méthyl)-7-fluoro-3-méthyl-N-(2-propanyl)-3H-pyra-zolo[3,4-c]quinoline-8-carboxamide ;
4-amino-N-((5-chloro-2-pyridinyl)méthyl)-N,1-diméthyl-1H-pyrazolo[4,3-c]quinoline-8-carboxa-mide ;
4-amino-N-((5-(difluorométhyl)-2-pyridinyl)méthyl)-N,1-diméthyl-1H-pyrazolo[4,3-c]quinoline-8-carboxamide ;
4-amino-N-((5-(difluorométhyl)-2-pyridinyl)méthyl)-7-fluoro-N,1-diméthyl-1H-pyrazolo[4,3-c]qui-noline-8-carboxamide ;
4-amino-N-(3-fluoro-4-(trifluorométhyl)benzyl)-N,1-diméthyl-1H-pyrazolo[4,3-c]quinoline-8-car-boxamide ;
4-amino-N-((3-fluoro-5-(trifluorométhyl)-2-pyridinyl)méthyl)-N,1-diméthyl-1H-pyrazolo[4,3-c]quinoline-8-carboxamide ;
4-amino-7-fluoro-N-((3-fluoro-5-(trifluorométhyl)-2-pyridinyl)méthyl)-N-méthyl-1,3-dihydro-furo[3,4-c]quinoline-8-carboxamide ;
4-amino-N,1-diméthyl-N-((6-(1-(trifluorométhyl)-1H-pyrazol-4-yl)-3-pyridazinyl)méthyl)-1H-py-razolo[4,3-c]quinoline-8-carboxamide ;
4-amino-N,1-diméthyl-N-((6-(4-(trifluorométhyl)phényl)-3-pyridazinyl)méthyl)-1H-pyrazolo[4,3-c]quinoline-8-carboxamide ;
4-amino-N,1-diméthyl-N-((1R)-1-(4'-(trifluorométhyl)[biphényl]-4-yl)éthyl)-1H-pyrazolo[4,3-c]quinoline-8-carboxamide ;
4-amino-N,1-diméthyl-N-((1R)-1-(6-(4-(trifluorométhyl)phényl)-3-pyridazinyl)éthyl)-1H-pyra-zolo[4,3-c]quinoline-8-carboxamide ;
4-amino-N,1-diméthyl-N-((1R)-1-(4'-(pentafluoro-lambda~6~-sulfanyl)[biphényl]-4-yl)éthyl)-1H-pyrazolo[4,3-c]quinoline-8-carboxamide ;
4-amino-N-(2-propanyl)-N-((5-(trifluorométhyl)-2-pyridinyl)méthyl)-2,3-dihydrofuro[3,2-c][1,7]naphtyridine-8-carboxamide ;
4-amino-N-(2-(5-chloro-2-pyridinyl)-2,2-difluoroéthyl)-N,1-diméthyl-1H-pyrazolo[4,3-c]quino-line-8-carboxamide ;
4-amino-7-fluoro-N-(2-propanyl)-N-((5-(trifluorométhyl)-2-pyridinyl)méthyl)-2,3-dihydrofuro[3,2-c]quinoline-8-carboxamide ;
4-amino-7-chloro-N-éthyl-N-((5-(trifluorométhyl)-2-pyridinyl)méthyl)-1,3-dihydrofuro[3,4-c][1,8]naphtyridine-8-carboxamide ;
4-amino-7-fluoro-1,3-diméthyl-N-(2-propanyl)-N-((5-(trifluorométhyl)-2-pyridinyl)méthyl)-1H-pyrazolo[4,3-c]quinoline-8-carboxamide ;
4-amino-N-((5-cyclopropyl-2-pyridinyl)méthyl)-N-éthyl-1,3-dihydrofuro[3,4-c]quinoline-8-car-boxamide ;
4-amino-N-((5-cyclopropyl-2-pyridinyl)méthyl)-N-éthyl-1-méthyl-1H-pyrazolo[4,3-c]quinoline-8-carboxamide ;
4-amino-N-((5-cyclopropyl-2-pyridinyl)méthyl)-N-éthyl-1-méthyl-1H-pyrazolo[4,3-c][1,7]naphty-ridine-8-carboxamide ;
(3R)-4-amino-N-((5-cyclopropyl-2-pyridinyl)méthyl)-N-éthyl-3-méthyl-1,3-dihydrofuro[3,4-c]qui-noline-8-carboxamide ;
4-amino-N,1-diméthyl-N-(4-(trifluorométhyl)benzyl)-1H-pyrazolo[4,3-c]quinoline-8-carboxa-mide ;
4-amino-N,1-diméthyl-N-(4-(trifluorométhyl)benzyl)-1H-pyrazolo[4,3-c][1,7]naphtyridine-8-car-boxamide ;
4-amino-N-((5-cyclopropyl-2-pyridinyl)méthyl)-N-éthyl-7-fluoro-1-méthyl-1H-pyrazolo[4,3-c]qui-noline-8-carboxamide ;
4-amino-N-éthyl-1-méthyl-N-((5-(trifluorométhyl)-2-pyridinyl)méthyl)-1H-pyrazolo[4,3-c]quino-line-8-carboxamide ;
4-amino-N-éthyl-3-méthyl-N-((5-(trifluorométhyl)-2-pyridinyl)méthyl)[1,2]oxazolo[4,5-c]quino-line-8-carboxamide ;
4-amino-N-éthyl-1-méthyl-N-((6-(trifluorométhyl)-3-pyridazinyl)méthyl)-1H-pyrazolo[4,3-c]qui-noline-8-carboxamide ;
4-amino-N-éthyl-1-méthyl-N-((5-(trifluorométhyl)-2-pyridinyl)méthyl)-1H-pyrazolo[4,3-c][1,7]naphtyridine-8-carboxamide ;
4-amino-N-éthyl-1-méthyl-N-((6-(trifluorométhyl)-3-pyridazinyl)méthyl)-1H-pyrazolo[4,3-c][1,7]naphtyridine-8-carboxamide ;
(3R)-4-amino-N-éthyl-3-méthyl-N-((6-(trifluorométhyl)-3-pyridazinyl)méthyl)-1,3-dihydro-furo[3,4-c]quinoline-8-carboxamide ;
4-amino-7-fluoro-N,1-diméthyl-N-(4-(trifluorométhyl)benzyl)-1H-pyrazolo[4,3-c]quinoline-8-car-boxamide ;
4-amino-N-éthyl-1,3-diméthyl-N-((5-(trifluorométhyl)-2-pyridinyl)méthyl)-1H-pyrazolo[4,3-c][1,7]naphtyridine-8-carboxamide ;
4-amino-N-éthyl-7-fluoro-1-méthyl-N-((5-(trifluorométhyl)-2-pyridinyl)méthyl)-1H-pyrazolo[4,3-c]quinoline-8-carboxamide ;
4-amino-N-éthyl-7-fluoro-1-méthyl-N-((6-trifluorométhyl)-3-pyridazinyl)méthyl)-1H-pyra-zolo[4,3-c]quinoline-8-carboxamide ;
(3R)-4-amino-N-éthyl-7-fluoro-3-méthyl-N-((5-(trifluorométhyl)-2-pyridinyl)méthyl)-1,3-dihydro-furo[3,4-c]quinoline-8-carboxamide ;
(3S)-4-amino-N-éthyl-7-fluoro-3-méthyl-N-((5-(trifluorométhyl)-2-pyridinyl)méthyl)-1,3-dihydro-furo[3,4-c]quinoline-8-carboxamide ;
4-amino-7-chloro-N,1-diméthyl-N-(4-(trifluorométhyl)benzyl)-1H-pyrazolo[4,3-c]quinoline-8-car-boxamide ;
(3R)-4-amino-N-éthyl-7-fluoro-3-méthyl-N-((6-(trifluorométhyl)-3-pyridazinyl)méthyl)-1,3-dihy-drofuro[3,4-c]quinoline-8-carboxamide ;
4-amino-N-éthyl-7-fluoro-1,3-diméthyl-N-((5-(trifluorométhyl)-2-pyridinyl)méthyl)-1H-pyra-zolo[4,3-c]quinoline-8-carboxamide ;
4-amino-N,1-diméthyl-N-(4-(pentafluoroéthyl)benzyl)-1H-pyrazolo[4,3-c]quinoline-8-carboxa-mide ;
4-amino-N,1-diméthyl-N-(4-(pentafluoroéthyl)benzyl)-1H-pyrazolo[4,3-c][1,7]naphtyridine-8-carboxamide ;
4-amino-7-fluoro-N,1-diméthyl-N-(4-(pentafluoroéthyl)benzyl)-1H-pyrazolo[4,3-c]quinoline-8-carboxamide ;
4-amino-N-éthyl-1-méthyl-N-(4-(pentafluoro-lambda~6~-sulfanyl)benzyl)-1H-pyrazolo[4,3-c]quinoline-8-carboxamide ;
4-amino-N-éthyl-1-méthyl-N-(4-(pentafluoro-lambda~6~-sulfanyl)benzyl)-1H-pyrazolo[4,3-c][1,7]naphtyridine-8-carboxamide ;
4-amino-N-éthyl-7-fluoro-1-méthyl-N-(4-(pentafluoro-lambda~6~-sulfanyl)benzyl)-1H-pyra-zolo[4,3-c]quinoline-8-carboxamide ;
4-amino-N,1-diméthyl-N-((5-(trifluorométhyl)-2-pyridinyl)méthyl)-1H-pyrazolo[4,3-c]quinoline-8-carboxamide ;
4-amino-N,1-diméthyl-N-((5-(trifluorométhyl)-2-pyridinyl)méthyl)-1H-pyrazolo[4,3-c][1,7]naphty-ridine-8-carboxamide ;
4-amino-N-cyclopropyl-1-méthyl-N-((5-(trifluorométhyl)-2-pyridinyl)méthyl)-1H-pyrazolo[4,3-c]quinoline-8-carboxamide ;
4-amino-N-cyclopropyl-7-fluoro-1-(2,2,2-trifluoroéthyl)-N-((5-(trifluorométhyl)-2-pyridinyl)mé-thyl)-1H-pyrazolo[4,3-c]quinoline-8-carboxamide ;
4-amino-7-fluoro-N,1-diméthyl-N-((6-(trifluorométhyl)-3-pyridazinyl)méthyl)-1H-pyrazolo[4,3-c]quinoline-8-carboxamide ;
4-amino-N-méthyl-7-(trifluorométhyl)-N-((5-(trifluorométhyl)-2-pyridinyl)méthyl)-1,3-dihydro-furo[3,4-c]quinoline-8-carboxamide ;
4-amino-N,1-diméthyl-N-(4-(pentafluoro-lambda~6~-sulfanyl)benzyl)-1H-pyrazolo[4,3-c]quino-line-8-carboxamide ;
4-amino-N,1-diméthyl-N-(4-(pentafluoro-lambda~6~-sulfanyl)benzyl)-1H-pyrazolo[4,3-c][1,7]naphtyridine-8-carboxamide ;
4-amino-7-fluoro-N,1-diméthyl-N-(4-(pentafluoro-lambda~6~-sulfanyl)benzyl)-1H-pyrazolo[4,3-c]quinoline-8-carboxamide ;
4-amino-7-fluoro-N-(2-hydroxy-4-(pentafluoro-lambda~6~-sulfanyl)benzyl)-N-méthyl-1,3-dihy-drofuro[3,4-c]quinoline-8-carboxamide ;
4-amino-N-((5-cyano-2-pyridinyl)méthyl)-N-((2R)-1-fluoro-2-propanyl)-1,3-dihydrofuro[3,4-c]quinoline-8-carboxamide ;
4-amino-N-((5-cyano-2-pyridinyl)méthyl)-N-((2S)-1-fluoro-2-propanyl)-1,3-dihydrofuro[3,4-c]quinoline-8-carboxamide ;
(3R)-4-amino-3-(hydroxyméthyl)-N-(2-méthylpropyl)-N-((5-(trifluorométhyl)-2-pyridinyl)méthyl)-1,3-dihydrofuro[3,4-c]quinoline-8-carboxamide ;
(3S)-4-amino-3-(hydroxyméthyl)-N-(2-méthylpropyl)-N-((5-(trifluorométhyl)-2-pyridinyl)méthyl)-1,3-dihydrofuro[3,4-c]quinoline-8-carboxamide ;
4-amino-N-((1R)-2-cyano-1-cyclopropyléthyl)-N-((5-cyano-2-pyridinyl)méthyl)-1,3-dihydro-furo[3,4-c]quinoline-8-carboxamide ;
4-amino-N-((1S)-2-cyano-1-cyclopropyléthyl)-N-((5-cyano-2-pyridinyl)méthyl)-1,3-dihydro-furo[3,4-c]quinoline-8-carboxamide ;
4-amino-N-((5-cyano-6-méthyl-2-pyridinyl)méthyl)-N-((3R,4R)-4-méthoxytétrahydro-2H-pyran-3-yl)-1,3-dihydrofuro[3,4-c]quinoline-8-carboxamide ;
4-amino-N-((5-cyano-6-méthyl-2-pyridinyl)méthyl)-N-((3S,4S)-4-méthoxytétrahydro-2H-pyran-3-yl)-1,3-dihydrofuro[3,4-c]quinoline-8-carboxamide ;
4-amino-N-((3S,4R)-3-méthoxytétrahydro-2H-pyran-4-yl)-N-((5-(trifluorométhyl)-2-pyridinyl)mé-thyl)-1,3-dihydrofuro[3,4-c]quinoline-8-carboxamide ;
4-amino-N-((3R,4S)-3-méthoxytétrahydro-2H-pyran-4-yl)-N-((5-(trifluorométhyl)-2-pyridinyl)mé-thyl)-1,3-dihydrofuro[3,4-c]quinoline-8-carboxamide ;
4-amino-N-((3R,4R)-4-méthoxytétrahydro-2H-pyran-3-yl)-N-((5-(trifluorométhyl)-2-pyri-dinyl)méthyl)-1,3-dihydrofuro[3,4-c]quinoline-8-carboxamide ;
4-amino-N-((3S,4S)-4-méthoxytétrahydro-2H-pyran-3-yl)-N-((5-(trifluorométhyl)-2-pyridinyl)mé-thyl)-1,3-dihydrofuro[3,4-c]quinoline-8-carboxamide ;
4-amino-N-((5-cyano-6-méthyl-2-pyridinyl)méthyl)-N-((3R,4R)-4-méthoxytétrahydro-2H-pyran-3-yl)-1,3-dihydrofuro[3,4-c]quinoline-8-carboxamide ;
(3R)-4-amino-N-cyclopropyl-3-méthyl-N-((1R)-1-(5-(trifluorométhyl)-2-pyridinyl)éthyl)-1,3-dihy-drofuro[3,4-c][1,7]naphtyridine-8-carboxamide ;
(35S)-4-amino-N-cyclopropyl-3-méthyl-N-((1R)-1-(5-(trifluorométhyl)-2-pyridinyl)éthyl)-1,3-dihy-drofuro[3,4-c][1,7]naphtyridine-8-carboxamide ;
4-amino-N-((1S)-1-(5-fluoro-2-pyridinyl)éthyl)-N,1,7-triméthyl-1H-pyrazolo[4,3-c]quinoline-8-carboxamide ;
4-amino-N-((1R)-1-(5-fluoro-2-pyridinyl)éthyl)-N,1,7-triméthyl-1H-pyrazolo[4,3-c]quinoline-8-carboxamide ;
4-amino-7-chloro-N-((1R)-1-(5-fluoro-2-pyridinyl)éthyl)-N,1-diméthyl-1H-pyrazolo[4,3-c]quino-line-8-carboxamide ;
4-amino-7-chloro-N-((1S)-1-(5-fluoro-2-pyridinyl)éthyl)-N,1-diméthyl-1H-pyrazolo[4,3-c]quino-line-8-carboxamide ;
4-amino-7-fluoro-N-((1R)-1-(3-fluoro-5-(trifluorométhyl)-2-pyridinyl)éthyl)-N-méthyl-1,3-dihydro-furo[3,4-c]quinoline-8-carboxamide ;
(3R)-4-amino-N-éthyl-3-(fluorométhyl)-N-((5-(trifluorométhyl)-2-pyridinyl)méthyl)-1,3-dihydro-furo[3,4-c]quinoline-8-carboxamide ;
(3R)-4-amino-N-((5-bromo-2-pyridinyl)méthyl)-N,3-diméthyl-1,3-dihydrofuro[3,4-c][1,7]naphtyri-dine-8-carboxamide ;
4-amino-N,1-diméthyl-N-((1R)-1-(4-(trifluorométhyl)phényl)éthyl)-1H-pyrazolo[4,3-c][1,7]naph-tyridine-8-carboxamide ;
4-amino-N,1-diméthyl-N-((1S)-1-(4-(trifluorométhyl)phényl)éthyl)-1H-pyrazolo[4,3-c][1,7]naph-tyridine-8-carboxamide ;
4-amino-N,1-diméthyl-N-((1R)-1-(5-(trifluorométhyl)-2-pyridinyl)éthyl)-1H-pyrazolo[4,3-c]quino-line-8-carboxamide ;
4-amino-N,1-diméthyl-N-((1S)-1-(5-(trifluorométhyl)-2-pyridinyl)éthyl)-1H-pyrazolo[4,3-c]quino-line-8-carboxamide ;
4-amino-7-fluoro-N,1-diméthyl-N-((1R)-1-(5-(trifluorométhyl)-2-pyridinyl)éthyl)-1H-pyrazolo[4,3-c]quinoline-8-carboxamide ;
4-amino-7-fluoro-N,1-diméthyl-N-((1S)-1-(5-(trifluorométhyl)-2-pyridinyl)éthyl)-1H-pyrazolo[4,3-c]quinoline-8-carboxamide ;
4-amino-N-((1R)-1-(5-fluoro-2-pyridinyl)éthyl)-N,1-diméthyl-1H-pyrazolo[4,3-c]quinoline-8-car-boxamide ;
4-amino-7-fluoro-N,1-diméthyl-N-((1R)-1-(6-(trifluorométhyl)-3-pyridazinyl)éthyl)-1H-pyra-zolo[4,3-c]quinoline-8-carboxamide ;
4-amino-N-((5-cyano-2-pyridinyl)méthyl)-N-((1R)-1-(2-pyrimidinyl)éthyl)-1,3-dihydrofuro[3,4-c][1,7]naphtyridine-8-carboxamide ;
6-amino-N-(2-méthylpropyl)-N-((5-(trifluorométhyl)-2-pyridinyl)méthyl)-8,9-dihydro-7H-cyclo-penta[c][1,8]naphtyridine-2-carboxamide ;
4-amino-N-(cyclopropyiméthyl)-N-((5-(trifluorométhyl)-2-pyridinyl)méthyl)-1,3-dihydrofuro[3,4-c][1,8]naphtyridine-8-carboxamide ;
4-amino-N-((2R)-1-méthoxy-2-propanyl)-N-((5-(trifluorométhyl)-2-pyridinyl)méthyl)-1,3-dihydro-furo[3,4-c][1,8]naphtyridine-8-carboxamide ;
4-amino-N-((1R)-1-(2-pyrimidinyl)éthyl)-N-((5-(trifluorométhyl)-2-pyridinyl)méthyl)-2,3-dihydro-1H-cyclopenta[c)quinoline-8-carboxamide ;
4-amino-N-(2-méthylpropyl)-N-((5-(trifluorométhyl)-2-pyridinyl)méthyl)-2,3-dihydro-1H-cyclo-penta[c]quinoline-8-carboxamide ;
4-amino-N-(2-méthylpropyl)-N-((5-(trifluorométhyl)-2-pyridinyl)méthyl)-1,3-dihydrofuro[3,4-c][1,8]naphtyridine-8-carboxamide ;
4-amino-N-(2-méthylpropyl)-N-((5-(trifluorométhyl)-2-pyridinyl)méthyl)-1,3-dihydrofuro[3,4-c]quinoline-8-carboxamide ;
4-amino-N-((1R)-1-(2-pyrimidinyl)éthyl)-N-((5-(trifluorométhyl)-2-pyridinyl)méthyl)-1,3-dihydro-furo[3,4-c][1,8]naphtyridine-8-carboxamide ;
4-amino-N-((5-cyano-2-pyridinyl)méthyl)-N-((1R)-1-(2-pyrimidinyl)éthyl)-2,3-dihydro-1H-cyclo-penta[c]quinoline-8-carboxamide ;
4-amino-N-((5-cyano-2-pyridinyl)méthyl)-N-((1R)-1-(2-pyrimidinyl)éthyl)-1,3-dihydrofuro[3,4-c]quinoline-8-carboxamide ;
4-amino-N-((5-cyano-2-pyridinyl)méthyl)-N-((1S)-1-(2-pyrimidinyl)éthyl)-1,3-dihydrofuro[3,4-c]quinoline-8-carboxamide ;
4-amino-3-méthyl-N-(2-méthylpropyl)-N-((5-(trifluorométhyl)-2-pyridinyl)méthyl)[1,2]oxa-zolo[4,5-c]quinoline-8-carboxamide ;
4-amino-3-méthyl-N-((1R)-1-(2-pyrimidinyl)éthyl)-N-((5-(trifluorométhyl)-2-pyridinyl)méthyl)-3H-pyrazolo[3,4-c]quinoline-8-carboxamide ;
4-amino-3-méthyl-N-((1S)-1-(2-pyrimidinyl)éthyl)-N-((5-(trifluorométhyl)-2-pyridinyl)méthyl)-3H-pyrazolo[3,4-c]quinoline-8-carboxamide ;
4-amino-3-méthyl-N-((1R)-1-(2-pyrimidinyl)éthyl)-N-((5-(trifluorométhyl)-2-pyridinyl)mé-thyl)[1,2]oxazolo[4,5-c]quinoline-8-carboxamide ;
4-amino-3-méthyl-N-((1S)-1-(2-pyrimidinyl)éthyl)-N-((5-(trifluorométhyl)-2-pyridinyl)méthyl) [1,2]oxazolo[4,5-c]quinoline-8-carboxamide ;
4-amino-N-(2-méthylpropyl)-N-((5-(trifluorométhyl)-2-pyridinyl)méthyl)-1,3-dihydrofuro[3,4-c][1,7]naphtyridine-8-carboxamide ;
4-amino-N-((1R)-1-(2-pyrimidinyl)éthyl)-N-((5-(trifluorométhyl)-2-pyridinyl)méthyl)-1,3-dihydro-furo[3,4-c][1,7]naphtyridine-8-carboxamide ;
4-amino-N-((5-chloro-2-pyridinyl)méthyl)-N-((1R)-1-(2-pyrimidinyl)éthyl)-2,3-dihydro-1H-cyclo-penta[c]quinoline-8-carboxamide ;
4-amino-N-((5-(difluorométhyl)-2-pyridinyl)méthyl)-N-((1R)-1-(2-pyrimidinyl)éthyl)-2,3-dihydro-1H-cyclopenta[c]quinoline-8-carboxamide ;
4-amino-N-((6-bromo-3-pyridazinyl)méthyl)-N-((1R)-1-(3-fluoro-2-pyridinyl)éthyl)-2,3-dihydro-1H-cyclopenta[c]quinoline-8-carboxamide ;
6-amino-N-((5-(difluorométhyl)-2-pyridinyl)méthyl)-N-((1R)-1-(2-pyrimidinyl)éthyl)-8,9-dihydro-7H-cyclopenta[c][1,8]naphtyridine-2-carboxamide ;
6-amino-N-((1R)-1-(2-pyrimidinyl)éthyl)-N-((5-(trifluorométhyl)-2-pyridinyl)méthyl)-8,9-dihydro-7H-cyclopenta[c][1,8]naphtyridine-2-carboxamide ;
6-amino-N-((5-chloro-2-pyridinyl)méthyl)-N-((1R)-1-(2-pyrimidinyl)éthyl)-8,9-dihydro-7H-cyclo-penta[c][1,8]naphtyridine-2-carboxamide ;
6-amino-N-((1R)-1-(3-fluoro-2-pyridinyl)éthyl)-N-((6-(trifluorométhyl)-3-pyridazinyl)méthyl)-8,9-dihydro-7H-cyclopenta[c][1,8]naphtyridine-2-carboxamide ;
6-amino-N-((1R)-1-(2-pyrimidinyl)éthyl)-N-((5-(trifluorométhyl)-2-pyridinyl)méthyl)-2-phénan-thridinecarboxamide ;
6-amino-N-((5-cyano-2-pyridinyl)méthyl)-N-((1R)-1-(2-pyrimidinyl)éthyl)-2-phénanthridinecar-boxamide ;
5-amino-N-(2-pyrimidinylméthyl)-N-((5-(trifluorométhyl)-2-pyridinyl)méthyl)benzo[c][2,6]naphty-ridine-9-carboxamide ;
5-amino-N-(2-méthylpropyl)-N-((5-(trifluorométhyl)-2-pyridinyl)méthyl)benzo[c][2,6]naphtyri-dine-9-carboxamide ;
5-amino-N-((1R)-1-(2-pyrimidinyl)éthyl)-N-((5-(trifluorométhyl)-2-pyridinyl)mé-thyl)benzo[c][2,6]naphtyridine-9-carboxamide ;
4-amino-N-((2R)-1-méthoxy-2-propanyl)-3-méthyl-N-((5-(trifluorométhyl)-2-pyridinyl)mé-thyl)[1,2]oxazolo[4,5-c]quinoline-8-carboxamide ;
4-amino-N-((2S)-1-méthoxy-2-propanyl)-3-méthyl-N-((5-(trifluorométhyl)-2-pyridinyl)mé-thyl)[1,2]oxazolo[4,5-c]quinoline-8-carboxamide ;
4-amino-N-((3-fluoro-2-pyridinyl)méthyl)-N-((5-(trifluorométhyl)-2-pyridinyl)méthyl)thiéno[2,3-c]quinoline-8-carboxamide ;
4-amino-N-(2-pyrimidinylméthyl)-N-((5-(trifluorométhyl)-2-pyridinyl)méthyl)thiéno[2,3-c]quino-line-8-carboxamide ;
4-amino-N-(2-méthylpropyl)-N-((5-(trifluorométhyl)-2-pyridinyl)méthyl)thiéno[2,3-c]quinoline-8-carboxamide ;
5-amino-N-((1R)-1-(2-pyrimidinyl)éthyl)-N-((5-(trifluorométhyl)-2-pyridinyl)méthyl)pyrimido[4,5-c]quinoline-9-carboxamide ;
4-amino-N-((6-bromo-3-pyridazinyl)méthyl)-N-((1R)-1-cyclopropyl-2-méthoxyéthyl)thiéno[2,3-c]quinoline-8-carboxamide ;
4-amino-N-((6-bromo-3-pyridazinyl)méthyl)-N-((1S)-1-cyclopropyl-2-méthoxyéthyl)thiéno[2,3-c]quinoline-8-carboxamide ;
4-amino-N-((1R)-1-(2-pyrimidinyl)éthyl)-N-((5-(trifluorométhyl)-2-pyridinyl)méthyl)-1,3-dihydro-furo[3,4-c]quinoline-8-carboxamide ;
4-amino-N-((1S)-1-(2-pyrimidinyl)éthyl)-N-((5-(trifluorométhyl)-2-pyridinyl)méthyl)-1,3-dihydro-furo 3,4-c]quinoline-8-carboxamide ;
4-amino-N-((1S)-1-cyclopropyl-2-méthoxyéthyl)-N-((6-(4-morpholinyl)-3-pyridazinyl)méthyl)-1,3-dihydrofuro[3,4-c]quinoline-8-carboxamide ;
4-amino-N-((6-éthoxy-3-pyridazinyl)méthyl)-N-(2-méthylpropyl)-1,3-dihydrofuro[3,4-c]quino-line-8-carboxamide ;
4-amino-N-((6-bromo-3-pyridazinyl)méthyl)-N-((2R)-1-méthoxy-2-propanyl)thiéno[2,3-c]quino-line-8-carboxamide ;
4-amino-N-((6-bromo-3-pyridazinyl)méthyl)-N-(1-méthoxy-2-méthyl-2-propanyl)-1,3-dihydro-furo[3,4-c]quinoline-8-carboxamide ;
4-amino-N-((6-bromo-3-pyridazinyl)méthyl)-N-(1-méthoxy-2-méthyl-2-propanyl)-1,3-dihydro-furo[3,4-c][1,7]naphtyridine-8-carboxamide ;
4-amino-N-((6-méthoxy-3-pyridazinyl)méthyl)-N-((1R)-1-(2-pyrimidinyl)éthyl)-2,3-dihydro-1H-cyclopenta[c]quinoline-8-carboxamide ;
4-amino-N-((6-méthoxy-3-pyridazinyl)méthyl)-N-((1S)-1-(2-pyrimidinyl)éthyl)-2,3-dihydro-1H-cyclopenta[c]quinoline-8-carboxamide ;
4-amino-N-((1S)-1-(2-pyrimidinyl)éthyl)-N-((6-(trifluorométhyl)-3-pyridazinyl)méthyl)-2,3-dihy-dro-1H-cyclopenta[c]quinoline-8-carboxamide ;
4-amino-N-((1R)-1-(2-pyrimidinyl)éthyl)-N-((6-(trifluorométhyl)-3-pyridazinyl)méthyl)-2,3-dihy-dro-1H-cyclopenta[c]quinoline-8-carboxamide ;
4-amino-N-((6-bromo-3-pyridazinyl)méthyl)-N-((1S)-1-cyclopropyl-2-méthoxyéthyl)thiéno[2,3-c]quinoline-8-carboxamide ;
4-amino-N-((6-bromo-3-pyridazinyl)méthyl)-N-((1S)-1-cyclopropyl-2-méthoxyéthyl)-1,3-dihy-drofuro[3,4-c][1,8]naphtyridine-8-carboxamide ;
4-amino-N-(cyclopropylméthyl)-N-((5-(trifluorométhyl)-2-pyridinyl)méthyl)-1,3-dihydrofuro[3,4-c][1,7]naphtyridine-8-carboxamide ;
4-amino-N-((1R)-1-(2-pyrimidinyl)éthyl)-N-((5-(trifluorométhyl)-2-pyridinyl)méthyl)-1,3-dihydro-furo[3,4-c]quinoline-8-carboxamide ;
6-amino-N-((1R)-1-(2-pyrimidinyl)éthyl)-N-((5-(trifluorométhyl)-2-pyridinyl)méthyl)-7,8,9,10-té-trahydro-2-phénanthridinecarboxamide ;
4-amino-7-chloro-N-((1R)-1-(2-pyrimidinyl)éthyl)-N-((5-(trifluorométhyl)-2-pyridinyl)méthyl)-1,3-dihydrofuro[3,4-c]quinoline-8-carboxamide ;
4-amino-7-chloro-N-cyclopropyl-N-((5-(trifluorométhyl)-2-pyridinyl)méthyl)-1,3-dihydrofuro[3,4-c]quinoline-8-carboxamide ;
4-amino-N-((6-bromo-3-pyridazinyl)méthyl)-N-((1R)-1-(2-pyrimidinyl)éthyl)-1,3-dihydrofuro[3,4-c]quinoline-8-carboxamide ;
4-amino-N-((5-bromo-2-pyridinyl)méthyl)-N-((1R)-1-(2-pyrimidinyl)éthyl)-1,3-dihydrofuro[3,4-c]quinoline-8-carboxamide ;
4-amino-N-((5-cyano-2-pyridinyl)méthyl)-N-((1R)-1-(2-pyrimidinyl)éthyl)-1,3-dihydrofuro[3,4-c]quinoline-8-carboxamide ;
4-amino-N-(3,3-difluorocyclobutyl)-N-((6-(trifluorométhyl)-3-pyridazinyl)méthyl)-1,3-dihydro-furo[3,4-c]quinoline-8-carboxamide ;
4-amino-N-cyclopropyl-N-((6-(trifluorométhyl)-3-pyridazinyl)méthyl)-1,3-dihydrofuro[3,4-c]qui-noline-8-carboxamide ;
4-amino-N-((5-(difluorométhyl)-2-pyridinyl)méthyl)-N-((1R)-1-(1-méthyl-1H-1,2,4-triazol-3-yl)éthyl)-1,3-dihydrofuro[3,4-c]quinoline-8-carboxamide ;
4-amino-N-(cyclopropylméthyl)-N-((5-(trifluorométhoxy)-2-pyridinyl)méthyl)-1,3-dihydro-furo[3,4-c]quinoline-8-carboxamide ;
4-amino-N-(2-méthylpropyl)-N-((6-(trifluorométhyl)-3-pyridazinyl)méthyl)-1,3-dihydrofuro[3,4-c]quinoline-8-carboxamide ;
4-amino-N-((6-bromo-3-pyridazinyl)méthyl)-N-(2-méthylpropyl)-1,3-dihydrofuro[3,4-c]quinoline-8-carboxamide ;
4-amino-N-((5-cyano-2-pyridinyl)méthyl)-N-((1S)-1-(2-pyrimidinyl)éthyl)-1,3-dihydrofuro[3,4-c]quinoline-8-carboxamide ;
4-amino-N-((5-(difluorométhyl)-2-pyridinyl)méthyl)-N-((1R)-1-(1-méthyl-1H-1,2,4-triazol-3-yl)éthyl)-1,3-dihydrofuro[3,4-c][1,7]naphtyridine-8-carboxamide ;
4-amino-N-(3,3-difluorocyclobutyl)-N-((6-(trifluorométhyl)-3-pyridazinyl)méthyl)-1,3-dihydro-furo[3,4-c][1,7]naphtyridine-8-carboxamide ;
4-amino-N-cyclobutyl-N-((6-(trifluorométhyl)-3-pyridazinyl)méthyl)-1,3-dihydrofuro[3,4-c]quino-line-8-carboxamide ;
4-amino-N-(2-méthylpropyl)-N-((6-(trifluorométhyl)-3-pyridazinyl)méthyl)-1,3-dihydrofuro[3,4-c][1,7]naphtyridine-8-carboxamide ;
4-amino-N-((6-bromo-3-pyridazinyl)méthyl)-N-(cyclopropylméthyl)-1,3-dihydrofuro[3,4-c][1,7]naphtyridine-8-carboxamide ;
4-amino-N-((6-éthoxy-3-pyridazinyl)méthyl)-N-(2-méthylpropyl)-1,3-dihydrofuro[3,4-c][1,7]naphtyridine-8-carboxamide ;
4-amino-N-((6-bromo-3-pyridazinyl)méthyl)-N-((1R)-1-(2-pyrimidinyl)éthyl)-1,3-dihydrofuro[3,4-c][1,7]naphtyridine-8-carboxamide ;
4-amino-N-((6-bromo-3-pyridazinyl)méthyl)-N-(cyclopropylméthyl)-1,3-dihydrofuro[3,4-c]quino-line-8-carboxamide ;
4-amino-N-(2-propanyl)-N-((5-(trifluorométhyl)-2-pyridinyl)méthyl)-1,3-dihydrofuro[3,4-c][1,7]naphtyridine-8-carboxamide ;
4-amino-N-((6-chloro-3-pyridazinyl)méthyl)-N-((1R)-1-(2-pyrimidinyl)éthyl)-1,3-dihydrofuro[3,4-c]quinoline-8-carboxamide ;
4-amino-N-((5-bromo-6-méthyl-2-pyridinyl)méthyl)-N-méthyl-1,3-dihydrofuro[3,4-c]quinoline-8-carboxamide ;
4-amino-N-(2-propanyl)-N-((5-(trifluorométhoxy)-2-pyridinyl)méthyl)-1,3-dihydrofuro[3,4-c]qui-noline-8-carboxamide ;
4-amino-N-((6-chloro-5-cyano-2-pyridinyl)méthyl)-N-((1R)-1-(2-pyrimidinyl)éthyl)-1,3-dihydro-furo[3,4-c]quinoline-8-carboxamide ;
4-amino-N-((6-bromo-3-pyridazinyl)méthyl)-N-((2R)-1-méthoxy-2-propanyl)-1,3-dihydro-furo[3,4-c][1,7]naphtyridine-8-carboxamide ;
4-amino-N-((6-bromo-3-pyridazinyl)méthyl)-N-((2R)-1-méthoxy-2-propanyl)-1,3-dihydro-furo[3,4-c][1,8]naphtyridine-8-carboxamide ;
4-amino-N-((5-cyano-2-pyridinyl)méthyl)-N-(cyclopropylméthyl)-1,3-dihydrofuro[3,4-c][1,7]naphtyridine-8-carboxamide ;
4-amino-N-((6-chloro-5-méthoxy-2-pyridinyl)méthyl)-N-(2-propanyl)-1,3-dihydrofuro[3,4-c][1,7]naphtyridine-8-carboxamide ;
4-amino-N-((6-bromo-3-pyridazinyl)méthyl)-N-(2-propanyl)-1,3-dihydrofuro[3,4-c][1,7]naphtyri-dine-8-carboxamide ;
4-amino-N-((5-chloro-6-méthoxy-2-pyridinyl)méthyl)-N-(2-propanyl)-1,3-dihydrofuro[3,4-c][1,7]naphtyridine-8-carboxamide ;
4-amino-N-(3,4-dihydro-2H-pyrano[2,3-c]pyridin-6-ylméthyl)-N-((1R)-1-(2-pyrimidinyl)éthyl)-1,3-dihydrofuro)[3,4-c][1,7]naphtyridine-8-carboxamide ;
4-amino-N-((5-cyano-2-pyridinyl)méthyl)-N-((2R)-1-fluoro-2-propanyl)-1,3-dihydrofuro[3,4-c][1,7]naphtyridine-8-carboxamide ;
4-amino-N-((5-cyano-2-pyridinyl)méthyl)-N-((2S)-1-fluoro-2-propanyl)-1,3-dihydrofuro[3,4-c][1,7]naphtyridine-8-carboxamide ;
4-amino-N-(cyclopropylméthyl)-N-((6-(trifluorométhyl)-3-pyridazinyl)méthyl)-1,3-dihydro-furo[3,4-c][1,7]naphtyridine-8-carboxamide ;
4-amino-N-(bicyclo[1.1.1]pentan-1-yl)-N-((5-(trifluorométhyl)-2-pyridinyl)méthyl)-1,3-dihydro-furo[3,4-c][1,7]naphtyridine-8-carboxamide ;
4-amino-N-(1-cyclopropyl-1H-pyrazol-4-yl)-N-((5-(trifluorométhyl)-2-pyridinyl)méthyl)-1,3-dihy-drofuro[3,4-c][1,7]naphtyridine-8-carboxamide ;
4-amino-N-((5-(difluorométhyl)-2-pyridinyl)méthyl)-N-(1-méthyl-1H-pyrazol-4-yl)-1,3-dihydro-furo[3,4-c][1,7]naphtyridine-8-carboxamide ;
4-amino-N-méthyl-N-(4-(trifluorométhyl)benzyl)-1,3-dihydrofuro[3,4-c][1,7]naphtyridine-8-car-boxamide ;
4-amino-N-méthyl-N-((6-(trifluorométhyl)-3-pyridinyl)méthyl)-1,3-dihydrofuro[3,4-c][1,7]naphty-ridine-8-carboxamide ;
4-amino-N-((5-chloro-2-pyridinyl)méthyl)-N-méthyl-1,3-dihydrofuro[3,4-c][1,7]naphtyridine-8-carboxamide ;
4-amino-N-éthyl-N-((6-(trifluorométhyl)-3-pyridazinyl)méthyl)-1,3-dihydrofuro[3,4-c]quinoline-8-carboxamide ;
4-amino-N-éthyl-N-((6-(trifluorométhyl)-3-pyridazinyl)méthyl)-1,3-dihydrofuro[3,4-c][1,7]naphty-ridine-8-carboxamide ;
4-amino-N-méthyl-N-((1R)-1-(5-(trifluorométhyl)-2-pyridinyl)éthyl)-1,3-dihydrofuro[3,4-c]quino-line-8-carboxamide ;
4-amino-N-méthyl-N-((1S)- 1-(5-(trifluorométhyl)-2-pyridinyl)éthyl)-1,3-dihydrofuro[3,4-c]quino-line-8-carboxamide ;
4-amino-N-((5-chloro-2-pyridinyl)méthyl)-N-éthyl-1,3-dihydrofuro[3,4-c][1,7]naphtyridine-8-car-boxamide ;
4-amino-N-(1-méthylcyclopropyl)-N-((5-(trifluorométhyl)-2-pyridinyl)méthyl)-1,3-dihydro-furo[3,4-c][1,7]naphtyridine-8-carboxamide ;
4-amino-N-méthyl-N-((1R)-1-(5-(trifluorométhyl)-2-pyridinyl)éthyl)-1,3-dihydrofuro[3,4-c][1,8]naphtyridine-8-carboxamide ;
4-amino-N-méthyl-N-((1S)-1-(5-(trifluorométhyl)-2-pyridinyl)éthyl)-1,3-dihydrofuro[3,4-c][1,8]naphtyridine-8-carboxamide ;
4-amino-N-((5-chloro-2-pyridinyl)méthyl)-N-éthyl-1,3-dihydrofuro[3,4-c]quinoline-8-carboxa-mide ;
4-amino-N-((5-chloro-2-pyridinyl)méthyl)-N-méthyl-1,3-dihydrofuro[3,4-c]quinoline-8-carboxa-mide ;
4-amino-N-éthyl-N-((6-(trifluorométhyl)-3-pyridinyl)méthyl)-1,3-dihydrofuro[3,4-c]quinoline-8-carboxamide ;
4-amino-N-éthyl-N-((6-(trifluorométhyl)-3-pyridinyl)méthyl)-1,3-dihydrofuro[3,4-c][1,7]naphtyri-dine-8-carboxamide ;
4-amino-N-éthyl-N-((1R)-1-(5-(trifluorométhyl)-2-pyridinyl)éthyl)-1,3-dihydrofuro[3,4-c][1,8]naphtyridine-8-carboxamide ;
4-amino-N-éthyl-N-((1S)-1-(5-(trifluorométhyl)-2-pyridinyl)éthyl)-1,3-dihydrofuro[3,4-c][1,8]naphtyridine-8-carboxamide ;
4-amino-N-éthyl-N-(4-trifluorométhyl)benzyl)-1,3-dihydrofuro[3,4-c]quinoline-8-carboxamide ;
4-amino-N-éthyl-N-(4-(trifluorométhyl)benzyl)-1,3-dihydrofuro[3,4-c][1,7]naphtyridine-8-car-boxamide ;
4-amino-N-((6-bromo-3-pyridazinyl)méthyl)-N-éthyl-1,3-dihydrofuro[3,4-c]quinoline-8-carboxa-mide ;
4-amino-N-méthyl-N-((1R)-1-(5-(trifluorométhyl)-2-pyridinyl)éthyl)-1,3-dihydrofuro[3,4-c][1,7]naphtyridine-8-carboxamide ;
4-amino-N-méthyl-N-((1S)-1-(5-(trifluorométhyl)-2-pyridinyl)éthyl)-1,3-dihydrofuro[3,4-c][1,7]naphtyridine-8-carboxamide ;
4-amino-N-((6-bromo-3-pyridazinyl)méthyl)-N-éthyl-1,3-dihydrofuro[3,4-c][1,7]naphtyridine-8-carboxamide ;
4-amino-N-(1-méthyl-1H-pyrazol-4-yl)-N-((6-(trifluorométhyl)-3-pyridazinyl)méthyl)-1,3-dihydro-furo[3,4-c][1,7]naphtyridine-8-carboxamide ;
4-amino-N-(2,2,2-trifluoroéthyl)-N- ((6-(trifluorométhyl)-3-pyridazinyl)méthyl)-1,3-dihydro-furo[3,4-c][1,7]naphtyridine-8-carboxamide ;
4-amino-N-(3-oxétanyl)-N-((6-(trifluorométhyl)-3-pyridazinyl)méthyl)-1,3-dihydrofuro[3,4-c]qui-noline-8-carboxamide ;
4-amino-7-fluoro-N-(1-méthyl-1H-pyrazol-4-yl)-N-((5-(trifluorométhyl)-2-pyridinyl)méthyl)-1,3-dihydrofuro 3,4-c]quinoline-8-carboxamide ;
4-amino-7-chloro-N-(1-méthyl-1H-pyrazol-4-yl)-N-((5-(trifluorométhyl)-2-pyridinyl)méthyl)-1,3-dihydrofuro[3,4-c]quinoline-8-carboxamide ;
4-amino-N,3-diméthyl-N-((1R)-1-(5-(trifluorométhyl)-2-pyridinyl)éthyl)-3H-pyrazolo[3,4-c]quino-line-8-carboxamide ;
4-amino-N,3-diméthyl-N-((1S)-1-(5-(trifluorométhyl)-2-pyridinyl)éthyl)-3H-pyrazolo[3,4-c]quino-line-8-carboxamide ;
4-amino-N-((5-chloro-2-pyridinyl)méthyl)-N-(2-propanyl)-1,3-dihydrofuro[3,4-c]quinoline-8-car-boxamide ;
4-amino-N-((5-chloro-2-pyridinyl)méthyl)-N-(2-propanyl)-1,3-dihydrofuro[3,4-c][1,7]naphtyri-dine-8-carboxamide ;
4-amino-N-éthyl-7-fluoro-N-((1R)-1-(5-(trifluorométhyl)-2-pyridinyl)éthyl)-1,3-dihydrofuro[3,4-c]quinoline-8-carboxamide ;
4-amino-N-éthyl-7-fluoro-N-((1S)-1-(5-(trifluorométhyl)-2-pyridinyl)éthyl)-1,3-dihydrofuro[3,4-c]quinoline-8-carboxamide ;
(3R)-4-amino-N-éthyl-3-méthyl-N-((1-méthyl-1H-1,2,4-triazol-3-yl)méthyl)-1,3-dihydrofuro[3,4-c]quinoline-8-carboxamide ;
4-amino-N-cyclobutyl-N-((6-(trifluorométhyl)-3-pyridazinyl)méthyl)-1,3-dihydrofuro[3,4-c][1,7]naphtyridine-8-carboxamide ;
(3R)-4-amino-N-cyclobutyl-3-méthyl-N-((6-(trifluorométhyl)-3-pyridazinyl)méthyl)-1,3-dihydro-furo[3,4-c]quinoline-8-carboxamide ;
4-amino-N-((6-(difluorométhoxy)-3-pyridazinyl)méthyl)-N-éthyl-1,3-dihydrofuro[3,4-c]quinoline-8-carboxamide ;
4-amino-N-((6-(difluorométhoxy)-3-pyridazinyl)méthyl)-N-éthyl-7-fluoro-1,3-dihydrofuro[3,4-c]quinoline-8-carboxamide ;
4-amino-7-fluoro-N-((1R)-1-(5-fluoro-2-pyrimidinyl)éthyl)-N-(2-(trifluorométhoxy)éthyl)-1,3-dihy-drofuro[3,4-c]quinoline-8-carboxamide ;
4-amino-7-fluoro-N-((1S)-1-(5-fluoro-2-pyrimidinyl)éthyl)-N-(2-(trifluorométhoxy)éthyl)-1,3-dihy-drofuro[3,4-c]quinoline-8-carboxamide ;
(3R)-4-amino-N-((6-(difluorométhoxy)-3-pyridazinyl)méthyl)-N-éthyl-3-méthyl-1,3-dihydro-furo[3,4-c]quinoline-8-carboxamide ;
4-amino-N-((1R)-1-(6-chloro-3-pyridinyl)éthyl)-N-éthyl-1,3-dihydrofuro[3,4-c]quinoline-8-car-boxamide ;
4-amino-N-((1S)-1-(6-chloro-3-pyridinyl)éthyl)-N-éthyl-1,3-dihydrofuro[3,4-c]quinoline-8-car-boxamide ;
4-amino-N-((2R)-1-méthoxy-2-propanyl)-N-((5-(trifluorométhyl)-2-pyridinyl)méthyl)-1,3-dihydro-furo [3,4-c][1,7]naphtyridine-8-carboxamide ;
4-amino-7-fluoro-N-(1-(trifluorométhyl)-1H-pyrazol-4-yl)-N-((5-(trifluorométhyl)-2-pyridinyl)mé-thyl)-1,3-dihydrofuro[3,4-c]quinoline-8-carboxamide ;
4-amino-N-(cyclopropylméthyl)-7-fluoro-N-((6-(trifluorométhyl)-3-pyridazinyl)méthyl)-1,3-dihy-drofuro[3,4-c]quinoline-8-carboxamide ;
4-amino-N-(cyclopropylméthyl)-7-fluoro-3-méthyl-N-((6-(trifluorométhyl)-3-pyridazinyl)méthyl)-3H-pyrazolo[3,4-c]quinoline-8-carboxamide ;
4-amino-N-(1-(trifluorométhyl)-1 H-pyrazol-4-yl)-N-((5-(trifluorométhyl)-2-pyridinyl)méthyl)-1,3-dihydrofuro[3,4-c][1,7]naphtyridine-8-carboxamide ;
4-amino-N-(2-propanyl)-N-(4-(trifluorométhyl)benzyl)-1,3-dihydrofuro[3,4-c][1,7]naphtyridine-8-carboxamide ;
4-amino-N-cyclopropyl-N-(4-(trifluorométhyl)benzyl)-1,3-dihydrofuro[3,4-c][1,7]naphtyridine-8-carboxamide ;
4-amino-N-cyclopropyl-N-((1S)-1-(5-(trifluorométhyl)-2-pyridinyl)éthyl)-1,3-dihydrofuro[3,4-c]quinoline-8-carboxamide ;
4-amino-N-cyclopropyl-N-((1R)-1-(5-(trifluorométhyl)-2-pyridinyl)éthyl)-1,3-dihydrofuro[3,4-c]quinoline-8-carboxamide ;
4-amino-7-chloro-N-((1R)-1-(6-chloro-3-pyridinyl)éthyl)-N-éthyl-1,3-dihydrofuro[3,4-c]quinoline-8-carboxamide ;
4-amino-7-chloro-N-((1S)-1-(6-chloro-3-pyridinyl)éthyl)-N-éthyl-1,3-dihydrofuro[3,4-c]quinoline-8-carboxamide ;
4-amino-7-chloro-N-éthyl-N-((1R)-1-(5-(trifluorométhyl)-2-pyridinyl)éthyl)-1,3-dihydrofuro[3,4-c]quinoline-8-carboxamide ;
4-amino-N-((1R)-1-(6-chloro-3-pyridinyl)éthyl)-N-éthyl-7-fluoro-1,3-dihydrofuro[3,4-c]quinoline-8-carboxamide ;
4-amino-N-((1S)-1-(6-chloro-3-pyridinyl)éthyl)-N-éthyl-7-fluoro-1,3-dihydrofuro[3,4-c]quinoline-8-carboxamide ;
4-amino-7-fluoro-1-méthyl-N-(1-méthyl-1H-pyrazol-4-yl)-N-((5-(trifluorométhyl)-2-pyridinyl)mé-thyl)-1H-pyrazolo[4,3-c]quinoline-8-carboxamide ;
4-amino-7-chloro-N-(cyclopropylméthyl)-N-((6-(trifluorométhyl)-3-pyridazinyl)méthyl)-1,3-dihy-drofuro[3,4-c]quinoline-8-carboxamide ;
4-amino-N-éthyl-N-((1R)-1-(2-fluoro-4-(trifluorométhyl)phényl)éthyl)-1,3-dihydrofuro[3,4-c]qui-noline-8-carboxamide ;
4-amino-N-éthyl-N-((1R)-1-(4-(trifluorométhyl)phényl)éthyl)-1,3-dihydrofuro[3,4-c]quinoline-8-carboxamide ;
4-amino-7-chloro-N-cyclobutyl-N-((1R)-1-(6-(trifluorométhyl)-3-pyridazinyl)éthyl)-1,3-dihydro-furo[3,4-c]quinoline-8-carboxamide ;
4-amino-N-cyclobutyl-N-((1R)-1-(6-(trifluorométhyl)-3-pyridazinyl)éthyl)-1,3-dihydrofuro[3,4-c]quinoline-8-carboxamide ;
(3R)-4-amino-N-cyclopropyl-3-méthyl-N-((1R)-1-(5-(trifluorométhyl)-2-pyridinyl)éthyl)-1,3-dihy-drofuro[3,4-c]quinoline-8-carboxamide ;
4-amino-N-((6-éthoxy-3-pyridazinyl)méthyl)-N-éthyl-7-fluoro-1,3-dihydrofuro[3,4-c]quinoline-8-carboxamide ;
4-amino-N-((S)-cyclopropyl(5-(trifluorométhyl)-2-pyridinyl)méthyl)-N-éthyl-1,3-dihydrofuro[3,4-c]quinoline-8-carboxamide ;
4-amino-N-((6-éthoxy-3-pyridazinyl)méthyl)-N-((1 R)-1-(2-pyrimidinyl)éthyl)-1,3-dihydrofuro[3,4-c][1,7]naphtyridine-8-carboxamide ;
4-amino-7-fluoro-N-(1-méthyl-1H-pyrazol-4-yl)-N-((6-(trifluorométhyl)-3-pyridazinyl)méthyl)-1,3-dihydrofuro[3,4-c]quinoline-8-carboxamide ;
4-amino-7-chloro-N-(1-méthyl-1H-pyrazol-4-yl)-N-((6-(trifluorométhyl)-3-pyridazinyl)méthyl)-1,3-dihydrofuro[3,4-c]quinoline-8-carboxamide ;
4-amino-N-(cyclopropylméthyl)-N-((6-éthoxy-3-pyridazinyl)méthyl)-7-fluoro-1,3-dihydro-furo[3,4-c]quinoline-8-carboxamide ;
4-amino-N-(cyclopropylméthyl)-N-((6-éthoxy-3-pyridazinyl)méthyl)-1,3-dihydrofuro[3,4-c][1,7]naphtyridine-8-carboxamide ;
5-amino-N-(2-propanyl)-N-((5-(trifluorométhyl)-2-pyridinyl)méthyl)benzo[c][2,6]naphtyridine-9-carboxamide ;
5-amino-N-(2-propanyl)-N-((5-(trifluorométhyl)-2-pyridinyl)méthyl)pyrimido[4,5-c]quinoline-9-carboxamide ;
5-amino-N-(2-propanyl)-N-((5-(trifluorométhyl)-2-pyridinyl)méthyl)pyrido[4,3-c][1,7]naphtyri-dine-9-carboxamide ;
4-amino-1-méthyl-N-(1-méthyl-1H-pyrazol-4-yl)-N-((5-(trifluorométhyl)-2-pyridinyl)méthyl)-1H-pyrazolo[4,3-c]quinoline-8-carboxamide ;
4-amino-N-((6-éthoxy-3-pyridazinyl)méthyl)-3-méthyl-N-((1R)-1-(2-pyrimidinyl)éthyl)-3H-pyra-zolo[3,4-c]quinoline-8-carboxamide ;
4-amino-N-éthyl-N-(2-(4-(trifluorométhyl)phényl)-2-propanyl)-1,3-dihydrofuro[3,4-c]quinoline-8-carboxamide ;
5-amino-N-(2-propanyl)-N-((5-(trifluorométhyl)-2-pyridinyl)méthyl)pyrimido[4,5-c][1,7]naphtyri-dine-9-carboxamide ;
4-amino-N-éthyl-N-((1R)-1-(5-(trifluorométhyl)-2-pyridinyl)propyl)-1,3-dihydrofuro[3,4-c][1,7]naphtyridine-8-carboxamide ;
4-amino-N-éthyl-7-fluoro-N-((1R)-1-(5-(trifluorométhyl)-2-pyridinyl)éthyl)-1,3-dihydrofuro[3,4-c]quinoline-8-carboxamide ;
4-amino-N-éthyl-N-((1R)-1-(6-(trifluorométhyl)-3-pyridazinyl)éthyl)-1,3-dihydrofuro[3,4-c][1,7]naphtyridine-8-carboxamide ;
4-amino-N-((R)-cyclopropyl(5-(trifluorométhyl)-2-pyridinyl)méthyl)-N-méthyl-1,3-dihydro-furo[3,4-c][1,7]naphtyridine-8-carboxamide ;
4-amino-N-((S)-cyclopropyl(5-(trifluorométhyl)-2-pyridinyl)méthyl)-N-méthyl-1,3-dihydro-furo[3,4-c][1,7]naphtyridine-8-carboxamide ;
4-amino-N-(cyclopropylméthyl)-N-((6-éthoxy-3-pyridazinyl)méthyl)-7-fluoro-3-méthyl-3H-pyra-zolo[3,4-c]quinoline-8-carboxamide ;
(3R)-4-amino-N-(cyclopropylméthyl)-N-((6-éthoxy-3-pyridazinyl)méthyl)-3-méthyl-1,3-dihydro-furo[3,4-c]quinoline-8-carboxamide ;
4-amino-N-((R)-cyclopropyl(6-(trifluorométhyl)-3-pyridazinyl)méthyl)-N-éthyl-1,3-dihydro-furo[3,4-c][1,7]naphtyridine-8-carboxamide ;
(3R)-4-amino-3-méthyl-N-((1R)-1-(5-(trifluorométhyl)-2-pyridinyl)éthyl)-1,3-dihydrofuro[3,4-c]quinoline-8-carboxamide ;
4-amino-7-fluoro-N,1-diméthyl-N-((5-méthyl-2-pyridinyl)méthyl)-1H-pyrazolo[4,3-c]quinoline-8-carboxamide ;
4-amino-7-fluoro-N-((5-fluoro-2-pyridinyl)méthyl)-N,1-diméthyl-1H-pyrazolo[4,3-c]quinoline-8-carboxamide ;
4-amino-N-((2,6-difluoro-3-pyridinyl) méthyl)-7-fluoro-N,1-diméthyl-1H-pyrazolo[4,3-c]quino-line-8-carboxamide ;
4-amino-7-chloro-N-((1R)-1-(5-fluoro-2-pyridinyl)éthyl)-N-méthyl-1,3-dihydrofuro[3,4-c]quino-line-8-carboxamide ;
4-amino-7-chloro-N-((1S)-1-(5-fluoro-2-pyridinyl)éthyl)-N-méthyl-1,3-dihydrofuro[3,4-c]quino-line-8-carboxamide ;
4-amino-N-((1R)-1-(5-(difluorométhyl)-2-pyridinyl)éthyl)-N-éthyl-1,3-dihydrofuro[3,4-c][1,7]naphtyridine-8-carboxamide ;
4-amino-N-méthyl-N-((1R)-1-(5-(trifluorométhyl)-2-pyrazinyl)éthyl)-1,3-dihydrofuro[3,4-c]quino-line-8-carboxamide ;
(3R)-4-amino-N-((1R)-1-(5-(difluorométhyl)-2-pyridinyl)éthyl)-N-éthyl-3-méthyl-1,3-dihydro-furo[3,4-c]quinoline-8-carboxamide ;
4-amino-N,1-diméthyl-N-((1R)-1-(5-(trifluorométhyl)-2-pyrazinyl)éthyl)-1H-pyrazolo[4,3-c]qui-noline-8-carboxamide ;
(3R)-4-amino-N-éthyl-3-méthyl-N-((1R)-1-(5-(trifluorométhyl)-2-pyridinyl)éthyl)-1,3-dihydro-furo[3,4-c]quinoline-8-carboxamide ;
(3R)-4-amino-N-éthyl-3-méthyl-N-((1R)-1-(6-(trifluorométhyl)-3-pyridazinyl)éthyl)-1,3-dihydro-furo[3,4-c]quinoline-8-carboxamide ;
4-amino-7-fluoro-N,3-diméthyl-N-((1R)-1-(6-(trifluorométhyl)-3-pyridazinyl)éthyl)-3H-pyra-zolo[3,4-c]quinoline-8-carboxamide ;
4-amino-7-fluoro-N,1-diméthyl-N-((1R)-1-(5-(trifluorométhyl)-2-pyrazinyl)éthyl)-1H-pyra-zolo[4,3-c]quinoline-8-carboxamide ;
4-amino-N-éthyl-7-fluoro-N-((1R)-1-(6-(trifluorométhyl)-3-pyridazinyl)éthyl)-1,3-dihydrofuro[3,4-c]quinoline-8-carboxamide ;
4-amino-7-fluoro-N-((2-fluoro-6-(trifluorométhyl)-3-pyridinyl)méthyl)-N,1-diméthyl-1H-pyra-zolo[4,3-c]quinoline-8-carboxamide ;
4-amino-N-((R)-cyclopropyl(6-(trifluorométhyl)-3-pyridazinyl)méthyl)-N-éthyl-1,3-dihydro-furo[3,4-c]quinoline-8-carboxamide ;
(3R)-4-amino-N-éthyl-7-fluoro-3-méthyl-N-((1R)-1-(6-(trifluorométhyl)-3-pyridazinyl)éthyl)-1,3-dihydrofuro[3,4-c]quinoline-8-carboxamide ;
(3S)-4-amino-N-éthyl-7-fluoro-3-méthyl-N-((1R)-1-(6-(trifluorométhyl)-3-pyridazinyl)éthyl)-1,3-dihydrofuro[3,4-c]quinoline-8-carboxamide ;
4-amino-N,3-diméthyl-N-((1R)-1-(4-(pentafluoroéthyl)phényl)éthyl)-3H-pyrazolo[3,4-c][1,7]naphtyridine-8-carboxamide ;
4-amino-7-fluoro-N,1-diméthyl-N-((1S)-1-(4-(pentafluoroéthyl)phényl)éthyl)-1H-pyrazolo[4,3-c]quinoline-8-carboxamide ;
4-amino-7-fluoro-N,1-diméthyl-N-((1R)-1-(4-(pentafluoroéthyl)phényl)éthyl)-1H-pyrazolo[4,3-c]quinoline-8-carboxamide ;
4-amino-7-fluoro-N,1-diméthyl-N-((5-(1-(trifluorométhyl)-1H-pyrazol-4-yl)-2-pyridinyl)méthyl)-1H-pyrazolo[4,3-c]quinoline-8-carboxamide ;
4-amino-N-cyclopropyl-1-méthyl-N-((6-(trifluorométhyl)-3-pyridazinyl)méthyl)-1H-pyrazolo[4,3-c]quinoline-8-carboxamide ;
4-amino-7-chloro-N,1-diméthyl-N-((5-(trifluorométhyl)-2-pyridinyl)méthyl)-1H-pyrazolo[4,3-c]quinoline-8-carboxamide ;
4-amino-7-chloro-N,1-diméthyl-N-((6-(trifluorométhyl)-3-pyridazinyl)méthyl)-1H-pyrazolo[4,3-c]quinoline-8-carboxamide ;
4-amino-7-chloro-N-éthyl-1-méthyl-N-((5-(trifluorométhyl)-2-pyridinyl)méthyl)-1H-pyrazolo[4,3-c]quinoline-8-carboxamide ;
4-amino-7-chloro-N-éthyl-1-méthyl-N-((6-(trifluorométhyl)-3-pyridazinyl)méthyl)-1H-pyra-zolo[4,3-c]quinoline-8-carboxamide ;
4-amino-7-chloro-N-cyclopropyl-1-méthyl-N-((5-(trifluorométhyl)-2-pyridinyl)méthyl)-1H-pyra-zolo[4,3-c]quinoline-8-carboxamide ;
4-amino-7-chloro-N-cyclopropyl-1-méthyl-N-((6-(trifluorométhyl)-3-pyridazinyl)méthyl)-1H-pyra-zolo[4,3-c]quinoline-8-carboxamide ;
4-amino-1-méthyl-N-(2,2,2-trifluoroéthyl)-N-((5-(trifluorométhyl)-2-pyridinyl)méthyl)-1H-pyra-zolo[4,3-c]quinoline-8-carboxamide ;
4-amino-7-chloro-N-cyclobutyl-1-méthyl-N-((5-(trifluorométhyl)-2-pyridinyl)méthyl)-1H-pyra-zolo[4,3-c]quinoline-8-carboxamide ;
4-amino-7-chloro-N-cyclobutyl-1-méthyl-N-((6-(trifluorométhyl)-3-pyridazinyl)méthyl)-1H-pyra-zolo[4,3-c]quinoline-8-carboxamide ;
4-amino-7-fluoro-1-méthyl-N-(2,2,2-trifluoroéthyl)-N-((5-(trifluorométhyl)-2-pyridinyl)méthyl)-1H-pyrazolo[4,3-c]quinoline-8-carboxamide ;
4-amino-N-cyclobutyl-7-fluoro-1-méthyl-N-(1,3-oxazol-4-ylméthyl)-1H-pyrazolo[4,3-c]quinoline-8-carboxamide ;
4-amino-7-fluoro-N,1-diméthyl-N-((5-(trifluorométhyl)-2-pyridinyl)méthyl)-1H-pyrazolo[4,3-c]quinoline-8-carboxamide ;
4-amino-N-cyclopropyl-7-fluoro-1-méthyl-N-((5-(trifluorométhyl)-2-pyridinyl)méthyl)-1H-pyra-zolo[4,3-c]quinoline-8-carboxamide ;
4-amino-N-cyclopropyl-7-fluoro-1-méthyl-N-((6-(trifluorométhyl)-3-pyridazinyl)méthyl)-1H-pyra-zolo[4,3-c]quinoline-8-carboxamide ;
4-amino-7-fluoro-1-méthyl-N-(2-propanyl)-N-((5-(trifluorométhyl)-2-pyridinyl)méthyl)-1H-pyra-zolo[4,3-c]quinoline-8-carboxamide ;
4-amino-7-fluoro-N-((2-méthoxy-6-(trifluorométhyl)-3-pyridinyl)méthyl)-N,1-diméthyl-1H-pyra-zolo[4,3-c]quinoline-8-carboxamide ;
4-amino-N-cyclobutyl-7-fluoro-1-méthyl-N-((5-(trifluorométhyl)-2-pyridinyl)méthyl)-1H-pyra-zolo[4,3-c]quinoline-8-carboxamide ;
4-amino-7-chloro-N-cyclopropyl-3-méthyl-N-((5-(trifluorométhyl)-2-pyridinyl)méthyl)-3H-pyra-zolo[3,4-c]quinoline-8-carboxamide ;
4-amino-N-cyclopropyl-7-fluoro-N-((3-fluoro-5-(trifluorométhyl)-2-pyridinyl)méthyl)-1-méthyl-1H-pyrazolo[4,3-c]quinoline-8-carboxamide ;
4-amino-N,1-diméthyl-7-(trifluorométhyl)-N-((5-(trifluorométhyl)-2-pyridinyl)méthyl)-1H-pyra-zolo[4,3-c]quinoline-8-carboxamide ;
4-amino-N,1-diméthyl-7-(trifluorométhyl)-N-((6-(trifluorométhyl)-3-pyridazinyl)méthyl)-1H-pyra-zolo[4,3-c]quinoline-8-carboxamide ;
4-amino-N-cyclopropyl-1-méthyl-7-(trifluorométhyl)-N-((5-(trifluorométhyl)-2-pyridinyl)méthyl)-1H-pyrazolo[4,3-c]quinoline-8-carboxamide ;
4-amino-N-cyclopropyl-1-méthyl-7-(trifluorométhyl)-N-((6-(trifluorométhyl)-3-pyridazinyl)mé-thyl)-1H-pyrazolo[4,3-c]quinoline-8-carboxamide ;
4-amino-N-((1R)-1-cyclopropyléthyl)-7-fluoro-N-((6-(trifluorométhyl)-3-pyridazinyl)méthyl)-1,3-dihydrofuro[3,4-c]quinoline-8-carboxamide ;
4-amino-N-((1S)-1-cyclopropyléthyl)-7-fluoro-N-((6-(trifluorométhyl)-3-pyridazinyl)méthyl)-1,3-dihydrofuro[3,4-c]quinoline-8-carboxamide ;
4-amino-N-(cyclopropylméthyl)-7-fluoro-N-((6-(2,2,2-trifluoroéthoxy)-3-pyridazinyl)méthyl)-1,3-dihydrofuro[3,4-c]quinoline-8-carboxamide ;
4-amino-N-(cyclopropylméthyl)-7-fluoro-3-méthyl-N-((6-(2,2,2-trifluoroéthoxy)-3-pyrida-zinyl)méthyl)-3H-pyrazolo[3,4-c]quinoline-8-carboxamide ;
(3R)-4-amino-N-(cyclopropylméthyl)-7-fluoro-3-méthyl-N-((6-(2,2,2-trifluoroéthoxy)-3-pyrida-zinyl)méthyl)-1,3-dihydrofuro[3,4-c]quinoline-8-carboxamide ;
4-amino-N-éthyl-N-((1S)-1-(5-(trifluorométhyl)-2-pyridinyl)éthyl)-1,3-dihydrofuro[3,4-c]quino-line-8-carboxamide ;
4-amino-N-éthyl-N-((1R)-1-(5-(trifluorométhyl)-2-pyridinyl)éthyl)-1,3-dihydrofuro[3,4-c]quino-line-8-carboxamide ;
4-amino-N-éthyl-N-((1R)-1-(5-(trifluorométhyl)-2-pyridinyl)éthyl)-1,3-dihydrofuro[3,4-c][1,7]naphtyridine-8-carboxamide ;
4-amino-N-éthyl-N-((1S)-1-(5-(trifluorométhyl)-2-pyridinyl)éthyl)-1,3-dihydrofuro[3,4-c][1,7]naphtyridine-8-carboxamide ;
4-amino-N-(1-méthyl-1H-pyrazol-4-yl)-N-((1R)-1-(5-(trifluorométhyl)-2-pyridinyl)éthyl)-1,3-dihy-drofuro[3,4-c][1,7]naphtyridine-8-carboxamide ;
4-amino-N-(1-méthyl-1H-pyrazol-4-yl)-N-((1S)-1-(5-(trifluorométhyl)-2-pyridinyl)éthyl)-1,3-dihy-drofuro[3,4-c][1,7]naphtyridine-8-carboxamide ;
(3S)-4-amino-3-méthyl-N-(1-méthyl-1H-pyrazol-4-yl)-N-((5-(trifluorométhyl)-2-pyridinyl)méthyl)-1,3-dihydrofuro[3,4-c][1,7]naphtyridine-8-carboxamide ;
(3R)-4-amino-3-méthyl-N-(1-méthyl-1H-pyrazol-4-yl)-N-((5-(trifluorométhyl)-2-pyridinyl)méthyl)-1,3-dihydrofuro[3,4-c][1,7]naphtyridine-8-carboxamide ;
(3S)-4-amino-N-cyclobutyl-3-méthyl-N-((6-(trifluorométhyl)-3-pyridazinyl)méthyl)-1,3-dihydro-furo[3,4-c][1,7]naphtyridine-8-carboxamide ;
(3R)-4-amino-N-cyclobutyl-3-méthyl-N-((6-(trifluorométhyl)-3-pyridazinyl)méthyl)-1,3-dihydro-furo[3,4-c][1,7]naphtyridine-8-carboxamide ;
4-amino-N-(2-propanyl)-N-((1S)-1-(5-(trifluorométhyl)-2-pyridinyl)éthyl)-1,3-dihydrofuro[3,4-c]quinoline-8-carboxamide ;
4-amino-N-(2-propanyl)-N-((1R)-1-(5-(trifluorométhyl)-2-pyridinyl)éthyl)-1,3-dihydrofuro[3,4-c]quinoline-8-carboxamide ;
4-amino-7-fluoro-N-((1S)-1-(5-fluoro-2-pyrimidinyl)éthyl)-N-(2-(trifluorométhoxy)éthyl)-1,3-dihy-drofuro[3,4-c]quinoline-8-carboxamide ;
4-amino-7-fluoro-N-((1R)-1-(5-fluoro-2-pyrimidinyl)éthyl)-N-(2-(trifluorométhoxy)éthyl)-1,3-dihy-drofuro[3,4-c]quinoline-8-carboxamide ;
4-amino-N-((1R)-1-(4-cyanophényl)éthyl)-N-éthyl-1,3-dihydrofuro[3,4-c]quinoline-8-carboxa-mide ;
4-amino-N-((1S)-1-(4-cyanophényl)éthyl)-N-éthyl-1,3-dihydrofuro[3,4-c]quinoline-8-carboxa-mide ;
4-amino-N-((1S)-1-(5-cyano-2-pyridinyl)éthyl)-N-éthyl-1,3-dihydrofuro[3,4-c]quinoline-8-car-boxamide ;
4-amino-N-((1R)-1-(5-cyano-2-pyridinyl)éthyl)-N-éthyl-1,3-dihydrofuro[3,4-c]quinoline-8-car-boxamide ;
(3R)-4-amino-3-méthyl-N-(2-propanyl)-N-((1R)-1-(5-(trifluorométhyl)-2-pyridinyl)éthyl)-1,3-dihy-drofuro[3,4-c]quinoline-8-carboxamide ;
(3R)-4-amino-3-méthyl-N-(2-propanyl)-N-((1S)-1-(5-(trifluorométhyl)-2-pyridinyl)éthyl)-1,3-dihy-drofuro(3,4-c)quinoline-8-carboxamide ;
4-amino-7-fluoro-N-(2-propanyl)-N-((1S)-1-(5-(trifluorométhyl)-2-pyridinyl)éthyl)-1,3-dihydro-furo[3,4-c]quinoline-8-carboxamide ;
4-amino-7-fluoro-N-(2-propanyl)-N-((1R)-1-(5-(trifluorométhyl)-2-pyridinyl)éthyl)-1,3-dihydro-furo[3,4-c]quinoline-8-carboxamide ;
(3R)-4-amino-N-((1S)-1-(5-cyano-2-pyridinyl)éthyl)-N-éthyl-3-méthyl-1,3-dihydrofuro[3,4-c]qui-noline-8-carboxamide ;
(3R)-4-amino-N-((1R)-1-(5-cyano-2-pyridinyl)éthyl)-N-éthyl-3-méthyl-1,3-dihydrofuro[3,4-c]qui-noline-8-carboxamide ;
4-amino-7-chloro-N-(2-propanyl)-N-((1R)-1-(5-(trifluorométhyl)-2-pyridinyl)éthyl)-1,3-dihydro-furo[3,4-c]quinoline-8-carboxamide ;
4-amino-7-chloro-N-(2-propanyl)-N-((1S)-1-(5-(trifluorométhyl)-2-pyridinyl)éthyl)-1,3-dihydro-furo[3,4-c]quinoline-8-carboxamide ;
4-amino-N-(2-propanyl)-N-((1S)-1-(5-(trifluorométhyl)-2-pyridinyl)éthyl)-1,3-dihydrofuro[3,4-c][1,7]naphtyridine-8-carboxamide ;
4-amino-N-(2-propanyl)-N-((1R)-1-(5-(trifluorométhyl)-2-pyridinyl)éthyl)-1,3-dihydrofuro[3,4-c][1,7]naphtyridine-8-carboxamide ;
4-amino-N-méthyl-N-((1R)-1-(5-(trifluorométhyl)-2-pyridinyl)propyl)-1,3-dihydrofuro[3,4-c][1,7]naphtyridine-8-carboxamide ;
4-amino-N-méthyl-N-((1S)-1-(5-(trifluorométhyl)-2-pyridinyl)propyl)-1,3-dihydrofuro[3,4-c][1,7]naphtyridine-8-carboxamide ;
(3R)-4-amino-N,3-diméthyl-N-((1R)-1-(5-(trifluorométhyl)-2-pyridinyl)éthyl)-1,3-dihydrofuro[3,4-c]quinoline-8-carboxamide ;
(3R)-4-amino-N,3-diméthyl-N-((1S)-1-(5-(trifluorométhyl)-2-pyridinyl)éthyl)-1,3-dihydrofuro[3,4-c]quinoline-8-carboxamide ;
4-amino-N-((1R)-1-(3,5-difluoro-2-pyridinyl)éthyl)-7-fluoro-N,1-diméthyl-1H-pyrazolo[4,3-c]qui-noline-8-carboxamide ;
4-amino-N-méthyl-N-((1R)-1-(5-(trifluorométhyl)-2-pyridinyl)éthyl)-1,3-dihydrofuro[3,4-c][1,7]naphtyridine-8-carboxamide ;
4-amino-N,1-diméthyl-N-((1R)-1-(6-(trifluorométhyl)-3-pyridazinyl)éthyl)-1H-pyrazolo[4,3-c]qui-noline-8-carboxamide ;
(3R)-4-amino-N,3-diméthyl-N-((1R)-1-(6-(trifluorométhyl)-3-pyridazinyl)éthyl)-1,3-dihydro-furo[3,4-c]quinoline-8-carboxamide ;
4-amino-7-fluoro-N-méthyl-N-((1R)-1-(4-(trifluorométhyl)phényl)éthyl)-1,3-dihydrofuro[3,4-c]quinoline-8-carboxamide ;
4-amino-7-fluoro-N-méthyl-N-((1R)-1-(6-(trifluorométhyl)-3-pyridazinyl)éthyl)-1,3-dihydro-furo[3,4-c]quinoline-8-carboxamide ;
(3R)-4-amino-7-fluoro-N,3-diméthyl-N-((1S)-1-(4-(trifluorométhyl)phényl)éthyl)-1,3-dihydro-furo[3,4-c]quinoline-8-carboxamide ;
(3R)-4-amino-7-fluoro-N,3-diméthyl-N-((1R)-1-(4-(trifluorométhyl)phényl)éthyl)-1,3-dihydro-furo[3,4-c]quinoline-8-carboxamide ;
4-amino-N-((1R)-1-(3-fluoro-5-(trifluorométhyl)-2-pyridinyl)éthyl)-N,3-diméthyl-3H-pyrazolo[3,4-c][1,7]naphtyridine-8-carboxamide ;
(3R)-4-amino-7-fluoro-N,3-diméthyl-N-((1R)-1-(6-(trifluorométhyl)-3-pyridazinyl)éthyl)-1,3-dihy-drofuro[3,4-c]quinoline-8-carboxamide ;
4-amino-7-fluoro-N-((1R)-1-(3-fluoro-5-(trifluorométhyl)-2-pyridinyl)éthyl)-N,1-diméthyl-1H-py-razolo[4,3-c]quinoline-8-carboxamide ;
4-amino-7-fluoro-N-((1S)-1-(3-fluoro-5-(trifluorométhyl)-2-pyridinyl)éthyl)-N,1-diméthyl-1H-py-razolo[4,3-c]quinoline-8-carboxamide ;
4-amino-7-fluoro-N-((1R)-1-(3-fluoro-5-(trifluorométhyl)-2-pyridinyl)éthyl)-N,3-diméthyl-3H-py-razolo[3,4-c]quinoline-8-carboxamide ;
4-amino-7-fluoro-N-((1R)-1-(3-méthoxy-5-(trifluorométhyl)-2-pyridinyl)éthyl)-N,3-diméthyl-3H-pyrazolo[3,4-c]quinoline-8-carboxamide ;
4-amino-7-fluoro-N-((1R)-1-(3-méthoxy-5-(trifluorométhyl)-2-pyridinyl)éthyl)-N,1-diméthyl-1H-pyrazolo[4,3-c]quinoline-8-carboxamide ;
4-amino-N,3-diméthyl-N-((1R)-1-(4-(pentafluoro-lambda~6~-sulfanyl)phényl)éthyl)-3H-pyra-zolo[3,4-c][1,7]naphtyridine-8-carboxamide ;
4-amino-7-fluoro-N,1-diméthyl-N-((1S)-1-(4-(pentafluoro-lambda-6~-sulfanyl)phényl)éthyl)-1H-pyrazolo[4,3-c]quinoline-8-carboxamide ;
4-amino-7-fluoro-N,1-diméthyl-N-((1R)-1-(4-(pentafluoro-lambda-6--sulfanyl)phényl)éthyl)-1H-pyrazolo [4,3-c]quinoline-8-carboxamide ;
4-amino-1-méthyl-N-(4-(pentafluoro-lambda~6~-sulfanyl)benzyl)-N-((1R)-1-(5-pyrimidinyl)pro-pyl)-1H-pyrazolo[4,3-c][1,7]naphtyridine-8-carboxamide ;
4-amino-1-méthyl-N-(4-(pentafluoro-lambda~6~-sulfanyl)benzyl)-N-((1S)-1-(5-pyrimidinyl)pro-pyl)-1H-pyrazolo(4,3-c][1,7]naphtyridine-8-carboxamide ;
4-amino-7-fluoro-N-((1R)-1-(5-fluoro-2-pyridinyl)éthyl)-N,1-diméthyl-1H-pyrazolo[4,3-c]quino-line-8-carboxamide ;
4-amino-7-fluoro-N-((1S)-1-(5-fluoro-2-pyridinyl)éthyl)-N,1-diméthyl-1H-pyrazolo[4,3-c]quino-line-8-carboxamide ;
4-amino-N-éthyl-7-fluoro-N-((1R)-1-(5-fluoro-2-pyridinyl)éthyl)-1-méthyl-1H-pyrazolo[4,3-c]qui-noline-8-carboxamide ;
4-amino-N-éthyl-7-fluoro-N-((1S)-1-(5-fluoro-2-pyridinyl)éthyl)-1-méthyl-1H-pyrazolo[4,3-c]qui-noline-8-carboxamide ;
4-amino-N-cyclopropyl-7-fluoro-1-méthyl-N-((1S)-1-(6-(trifluorométhyl)-3-pyridazinyl)éthyl)-1H-pyrazolo[4,3-c]quinoline-8-carboxamide ;
4-amino-N-cyclopropyl-7-fluoro-1-méthyl-N-((1R)-1-(6-(trifluorométhyl)-3-pyridazinyl)éthyl)-1H-pyrazolo[4,3-c]quinoline-8-carboxamide ;
4-amino-N,1-diméthyl-7-(trifluorométhyl)-N-((1R)-1-(6-(trifluorométhyl)-3-pyridazinyl)éthyl)-1H-pyrazolo[4,3-c]quinoline-8-carboxamide ;
4-amino-7-chloro-N-cyclopropyl-1-méthyl-N-((1S)-1-(6-(trifluorométhyl)-3-pyridazinyl)éthyl)-1H-pyrazolo[4,3-c]quinoline-8-carboxamide ;
4-amino-7-chloro-N-cyclopropyl-1-méthyl-N-((1R)-1-(6-(trifluorométhyl)-3-pyridazinyl)éthyl)-1H-pyrazolo[4,3-c]quinoline-8-carboxamide ;
4-amino-N-(1-méthyl-1H-pyrazol-4-yl)-N-(4-(trifluorométhyl)benzyl)-1,3-dihydrofuro[3,4-c][1,7]naphtyridine-8-carboxamide ;
4-amino-N-(3-fluorophényl)-N-((5-(trifluorométhyl)-2-pyridinyl)méthyl)-1,3-dihydrofuro[3,4-c]quinoline-8-carboxamide ;
4-amino-N-(1-méthyl-1H-pyrazol-4-yl)-N-((5-(trifluorométhyl)-2-pyridinyl)méthyl)-1,3-dihydro-furo[3,4-c]quinoline-8-carboxamide ;
4-amino-N-(1-méthyl-1H-pyrazol-4-yl)-N-((5-(trifluorométhyl)-2-pyridinyl)méthyl)-1,3-dihydro-furo[3,4-c][1,7]naphtyridine-8-carboxamide ;
4-amino-N-(1-méthyl-1H-pyrazol-4-yl)-N-((5-(trifluorométhyl)-2-pyridinyl)méthyl)-1,3-dihydro-furo[3,4-c][1,8]naphtyridine-8-carboxamide ;
5-amino-N-((1R)-1-(2-pyrimidinyl)éthyl)-N-((5-(trifluorométhyl)-2-pyridinyl)méthyl)-1,4-dihydro-2H-pyrano[3,4-c]quinoline-9-carboxamide ;
4-amino-N-((1R)-1-(2-pyrimidinyl)éthyl)-N-((5-(trifluorométhyl)-2-pyridinyl)méthyl)-2,3-dihydro-furo[3,2-c]quinoline-8-carboxamide ;
4-amino-3,3-diméthyl-N-(2-méthylpropyl)-N-((5-(trifluorométhyl)-2-pyridinyl)méthyl)-1,3-dihy-drofuro[3,4-c]quinoline-8-carboxamide ;
(3R)-4-amino-N-((3-fluoro-2-pyridinyl)méthyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((3-fluoro-2-pyridinyl)méthyl)-3-méthyl-N-((5-(trifluorométhyl)-2-pyridinyl)mé-thyl)-1,3-dihydrofuro[3,4-c]quinoline-8-carboxamide ;
(3R)-4-amino-3-méthyl-N-((1R)-1-(tétrahydro-2H-pyran-4-yl)éthyl)-N-((5-(trifluorométhyl)-2-py-ridinyl)méthyl)-1,3-dihydrofuro[3,4-c]quinoline-8-carboxamide ;
(3R)-4-amino-3-méthyl-N-((1S)-1-(tétrahydro-2H-pyran-4-yl)éthyl)-N-((5-(trifluorométhyl)-2-py-ridinyl)méthyl)-1,3-dihydrofuro[3,4-c]quinoline-8-carboxamide ;
(3S)-4-amino-3-méthyl-N-((1R)-1-(tétrahydro-2H-pyran-4-yl)éthyl)-N-((5-(trifluorométhyl)-2-py-ridinyl)méthyl)-1,3-dihydrofuro[3,4-c]quinoline-8-carboxamide ;
(3S)-4-amino-3-méthyl-N-((1S)-1-(tétrahydro-2H-pyran-4-yl)éthyl)-N-((5-(trifluorométhyl)-2-py-ridinyl)méthyl)-1,3-dihydrofuro[3,4-c]quinoline-8-carboxamide ;
(3R)-4-amino-N-((5-cyano-2-pyridinyl)méthyl)-3-méthyl-N-((1R)-1-(tétrahydro-2H-pyran-4-yl)éthyl)-1,3-dihydrofuro[3,4-c]quinoline-8-carboxamide ;
(3R)-4-amino-N-((5-cyano-2-pyridinyl)méthyl)-3-méthyl-N-((1S)-1-(tétrahydro-2H-pyran-4-yl)éthyl)-1,3-dihydrofuro[3,4-c]quinoline-8-carboxamide ;
(3S)-4-amino-N-((5-cyano-2-pyridinyl)méthyl)-3-méthyl-N-((1R)-1-(tétrahydro-2H-pyran-4-yl)éthyl)-1,3-dihydrofuro[3,4-c]quinoline-8-carboxamide ;
(3S)-4-amino-N-((5-cyano-2-pyridinyl)méthyl)-3-méthyl-N-((1S)-1-(tétrahydro-2H-pyran-4-yl)éthyl)-1,3-dihydrofuro[3,4-c]quinoline-8-carboxamide ;
(3R)-4-amino-N-((trans-4-hydroxycyclohexyl)méthyl)-3-méthyl-N-((5-(trifluorométhyl)-2-pyri-dinyl) méthyl)-1,3-dihydrofuro[3,4-c]quinoline-8-carboxamide ;
(3S)-4-amino-N-((trans-4-hydroxycyclohexyl)méthyl)-3-méthyl-N-((5-(trifluorométhyl)-2-pyri-dinyl)méthyl)-1,3-dihydrofuro[3,4-c]quinoline-8-carboxamide ;
(3R)-4-amino-N-((5-cyano-2-pyridinyl)méthyl)-N-((trans-4-hydroxycyclohexyl)méthyl)-3-méthyl-1,3-dihydrofuro[3,4-c]quinoline-8-carboxamide ;
(3S)-4-amino-N-((5-cyano-2-pyridinyl)méthyl)-N-((trans-4-hydroxycyclohexyl)méthyl)-3-méthyl-1,3-dihydrofuro[3,4-c]quinoline-8-carboxamide ;
(3R)-4-amino-3-méthyl-N-(tétrahydro-2H-pyran-4-ylméthyl)-N-((5-(trifluorométhyl)-2-pyri-dinyl)méthyl)-1,3-dihydrofuro[3,4-c]quinoline-8-carboxamide ;
(3S)-4-amino-3-méthyl-N-(tétrahydro-2H-pyran-4-ylméthyl)-N-((5-(trifluorométhyl)-2-pyri-dinyl)méthyl)-1,3-dihydrofuro[3,4-c]quinoline-8-carboxamide ;
(3R)-4-amino-N-((5-cyano-2-pyridinyl)méthyl)-3-méthyl-N-(tétrahydro-2H-pyran-4-ylméthyl)-1,3-dihydrofuro[3,4-c]quinoline-8-carboxamide ;
(3S)-4-amino-N-((5-cyano-2-pyridinyl)méthyl)-3-méthyl-N-(tétrahydro-2H-pyran-4-ylméthyl)-1,3-dihydrofuro[3,4-c]quinoline-8-carboxamide ;
(3R)-4-amino-N-((1R,2R)-2-cyanocyclopentyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1R,2S)-2-cyanocyclopentyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1S,2R)-2-cyanocyclopentyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1S,2S)-2-cyanocyclopentyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1R,2R)-2-cyanocyclopentyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1R,2S)-2-cyanocyclopentyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1S,2R)-2-cyanocyclopentyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1S,2S)-2-cyanocyclopentyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1R,2R)-2-cyanocyclopentyl)-N-((5-cyano-2-pyridinyl)méthyl)-3-méthyl-1,3-dihydrofuro[3,4-c]quinoline-8-carboxamide ;
(3R)-4-amino-N-((1R,2S)-2-cyanocyclopentyl)-N-((5-cyano-2-pyridinyl)méthyl)-3-méthyl-1,3-dihydrofuro[3,4-c]quinoline-8-carboxamide ;
(3R)-4-amino-N-((1S,2R)-2-cyanocyclopentyl)-N-((5-cyano-2-pyridinyl)méthyl)-3-méthyl-1,3-dihydrofuro[3,4-c]quinoline-8-carboxamide ;
(3R)-4-amino-N-((1S,2S)-2-cyanocyclopentyl)-N-((5-cyano-2-pyridinyl)méthyl)-3-méthyl-1,3-dihydrofuro[3,4-c]quinoline-8-carboxamide ;
(3S)-4-amino-N-((1R,2R)-2-cyanocyclopentyl)-N-((5-cyano-2-pyridinyl)méthyl)-3-méthyl-1,3-dihydrofuro[3,4-c]quinoline-8-carboxamide ;
(3S)-4-amino-N-((1R,2S)-2-cyanocyclopentyl)-N-((5-cyano-2-pyridinyl) méthyl)-3-méthyl-1,3-dihydrofuro[3,4-c]quinoline-8-carboxamide ;
(3S)-4-amino-N-((1S,2R)-2-cyanocyclopentyl)-N-((5-cyano-2-pyridinyl) méthyl)-3-méthyl-1,3-dihydrofuro[3,4-c]quinoline-8-carboxamide ;
(3S)-4-amino-N-((1S,2S)-2-cyanocyclopentyl)-N-((5-cyano-2-pyridinyl)méthyl)-3-méthyl-1,3-dihydrofuro[3,4-c]quinoline-8-carboxamide ;
(3R)-4-amino-3-méthyl-N-((3R)-tétrahydro-3-furanyl)-N-((5-(trifluorométhyl)-2-pyridinyl)méthyl)-1,3-dihydrofuro[3,4-c]quinoline-8-carboxamide ;
(3R)-4-amino-3-méthyl-N-((3S)-tétrahydro-3-furanyl)-N-((5-(trifluorométhyl)-2-pyridinyl)méthyl)-1,3-dihydrofuro[3,4-c]quinoline-8-carboxamide ;
(3S)-4-amino-3-méthyl-N-((3R)-tétrahydro-3-furanyl)-N-((5-(trifluorométhyl)-2-pyridinyl)méthyl)-1,3-dihydrofuro[3,4-c]quinoline-8-carboxamide ;
(3S)-4-amino-3-méthyl-N-((3S)-tétrahydro-3-furanyl)-N-((5-(trifluorométhyl)-2-pyridinyl)méthyl)-1,3-dihydrofuro[3,4-c]quinoline-8-carboxamide ;
(3R)-4-amino-3-méthyl-N-((1R,5S,6r)-3-oxabicyclo[3.1.0]hexan-6-yl)-N-((5-(trifluorométhyl)-2-pyridinyl)méthyl)-1,3-dihydrofuro[3,4-c]quinoline-8-carboxamide ;
(3S)-4-amino-3-méthyl-N-((1R,5S,6r)-3-oxabicyclo[3.1.0]hexan-6-yl)-N-((5-(trifluorométhyl)-2-pyridinyl)méthyl)-1,3-dihydrofuro[3,4-c]quinoline-8-carboxamide ;
(3R)-4-amino-3-méthyl-N-((3R,4R)-3-méthyltétrahydro-2H-pyran-4-yl)-N-((5-(trifluorométhyl)-2-pyridinyl)méthyl)-1,3-dihydrofuro[3,4-c]quinoline-8-carboxamide ;
(3R)-4-amino-3-méthyl-N-((3R,4S)-3-méthyltétrahydro-2H-pyran-4-yl)-N-((5-(trifluorométhyl)-2-pyridinyl)méthyl)-1,3-dihydrofuro[3,4-c]quinoline-8-carboxamide ;
(3R)-4-amino-3-méthyl-N-((3S,4R)-3-méthyltétrahydro-2H-pyran-4-yl)-N-((5-(trifluorométhyl)-2-pyridinyl)méthyl)-1,3-dihydrofuro[3,4-c]quinoline-8-carboxamide ;
(3R)-4-amino-3-méthyl-N-((3S,4S)-3-méthyltétrahydro-2H-pyran-4-yl)-N-((5-(trifluorométhyl)-2-pyridinyl)méthyl)-1,3-dihydrofuro[3,4-c]quinoline-8-carboxamide ;
(3S)-4-amino-3-méthyl-N-((3R,4R)-3-méthyltétrahydro-2H-pyran-4-yl)-N-((5-(trifluorométhyl)-2-pyridinyl)méthyl)-1,3-dihydrofuro[3,4-c]quinoline-8-carboxamide ;
(3S)-4-amino-3-méthyl-N-((3R,4S)-3-méthyltétrahydro-2H-pyran-4-yl)-N-((5-(trifluorométhyl)-2-pyridinyl)méthyl)-1,3-dihydrofuro[3,4-c]quinoline-8-carboxamide ;
(3S)-4-amino-3-méthyl-N-((3S,4R)-3-méthyltétrahydro-2H-pyran-4-yl)-N-((5-(trifluorométhyl)-2-pyridinyl)méthyl)-1,3-dihydrofuro[3,4-c]quinoline-8-carboxamide ;
(3S)-4-amino-3-méthyl-N-((3S,4S)-3-méthyltétrahydro-2H-pyran-4-yl)-N-((5-(trifluorométhyl)-2-pyridinyl)méthyl)-1,3-dihydrofuro[3,4-c]quinoline-8-carboxamide ;
(3R)-4-amino-N-((3S,4R)-3-méthoxytétrahydro-2H-pyran-4-yl)-3-méthyl-N-((5-(trifluorométhyl)-2-pyridinyl)méthyl)-1,3-dihydrofuro[3,4-c]quinoline-8-carboxamide ;
(3S)-4-amino-N-((3S,4R)-3-méthoxytétrahydro-2H-pyran-4-yl)-3-méthyl-N-((5-(trifluorométhyl)-2-pyridinyl)méthyl)-1,3-dihydrofuro[3,4-c]quinoline-8-carboxamide ;
(3R)-4-amino-N-((3R,4S)-3-méthoxytétrahydro-2H-pyran-4-yl)-3-méthyl-N-((5-(trifluorométhyl)-2-pyridinyl)méthyl)-1,3-dihydrofuro[3,4-c]quinoline-8-carboxamide ;
(3S)-4-amino-N-((3R,4S)-3-méthoxytétrahydro-2H-pyran-4-yl)-3-méthyl-N-((5-(trifluorométhyl)-2-pyridinyl)méthyl)-1,3-dihydrofuro[3,4-c]quinoline-8-carboxamide ;
(3R)-4-amino-N-((1R,2R)-[1,1'-bi(cyclopropyl)]-2-yl)-3-méthyl-N-((5-(trifluorométhyl)-2-pyri-dinyl)méthyl)-1,3-dihydrofuro[3,4-c]quinoline-8-carboxamide ;
(3R)-4-amino-N-((1R,2S)-[1,1'-bi(cyclopropyl)]-2-yl)-3-méthyl-N-((5-(trifluorométhyl)-2-pyri-dinyl)méthyl)-1,3-dihydrofuro[3,4-c]quinoline-8-carboxamide ;
(3R)-4-amino-N-((1S,2R)-[1,1'-bi(cyclopropyl)]-2-yl)-3-méthyl-N-((5-(trifluorométhyl)-2-pyri-dinyl)méthyl)-1,3-dihydrofuro[3,4-c]quinoline-8-carboxamide ;
(3R)-4-amino-N-((1S,2S)-[1,1'-bi(cyclopropyl)]-2-yl)-3-méthyl-N-((5-(trifluorométhyl)-2-pyri-dinyl)méthyl)-1,3-dihydrofuro[3,4-c]quinoline-8-carboxamide ;
(3S)-4-amino-N-((1R,2R)-[1,1'-bi(cyclopropyl)]-2-yl)-3-méthyl-N-((5-(trifluorométhyl)-2-pyri-dinyl)méthyl)-1,3-dihydrofuro[3,4-c]quinoline-8-carboxamide ;
(3S)-4-amino-N-((1R,2S)-[1,1'-bi(cyclopropyl)]-2-yl)-3-méthyl-N-((5-(trifluorométhyl)-2-pyri-dinyl)méthyl)-1,3-dihydrofuro[3,4-c]quinoline-8-carboxamide ;
(3S)-4-amino-N-((1S,2R)-[1,1'-bi(cyclopropyl)]-2-yl)-3-méthyl-N-((5-(trifluorométhyl)-2-pyri-dinyl)méthyl)-1,3-dihydrofuro[3,4-c]quinoline-8-carboxamide ;
(3S)-4-amino-N-((1S,2S)-[1,1'-bi(cyclopropyl)]-2-yl)-3-méthyl-N-((5-(trifluorométhyl)-2-pyri-dinyl)méthyl)-1,3-dihydrofuro[3,4-c]quinoline-8-carboxamide ;
(3R)-4-amino-3-méthyl-N-((1R)-spiro[2.5]octan-1-yl)-N-((5-(trifluorométhyl)-2-pyridinyl)méthyl)-1,3-dihydrofuro[3,4-c]quinoline-8-carboxamide ;
(3R)-4-amino-3-méthyl-N-((1S)-spiro[2.5]octan-1-yl)-N-((5-(trifliuorométhyl)-2-pyridinyl)méthyl)-1,3-dihydrofuro[3,4-c]quinoline-8-carboxamide ;
(3S)-4-amino-3-méthyl-N-((1R)-spiro[2.5]octan-1-yl)-N-((5-(trifluorométhyl)-2-pyridinyl)méthyl)-1,3-dihydrofuro[3,4-c]quinoline-8-carboxamide ;
(3S)-4-amino-3-méthyl-N-((1S)-spiro[2.5]octan-1-yl)-N-((5-(trifluorométhyl)-2-pyridinyl)méthyl)-1,3-dihydrofuro[3,4-c]quinoline-8-carboxamide ;
(3R)-4-amino-N-((1R,2R)-2-aminocyclopropyl)-3-méthyl-N-((5-(trifluorométhyl)-2-pyridinyl) mé-thyl)-1,3-dihydrofuro[3,4-c]quinoline-8-carboxamide ;
(3R)-4-amino-N-((1R,2S)-2-aminocyclopropyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1S,2R)-2-aminocyclopropyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1S,2S)-2-aminocyclopropyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1R,2R)-2-aminocyclopropyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1R,2S)-2-aminocyclopropyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1S,2R)-2-aminocyclopropyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1S,2S)-2-aminocyclopropyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1R,2R)-2-éthoxycyclopropyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1R,2S)-2-éthoxycyclopropyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1S,2R)-2-éthoxycyclopropyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1S,2S)-2-éthoxycyclopropyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1R,2R)-2-éthoxycyclopropyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1R,2S)-2-éthoxycyclopropyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1S,2R)-2-éthoxycyclopropyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1S,2S)-2-éthoxycyclopropyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1R)-2,2-diméthylcyclopropyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1S)-2,2-diméthylcyclopropyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1R)-2,2-diméthylcyclopropyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1S)-2,2-diméthylcyclopropyl)-3-méthyl-N-((5-(trifluorométhyl)-2-pyridinyl)mé-thyl)-1,3-dihydrofuro[3,4-c]quinoline-8-carboxamide ;
(3R)-4-amino-3-méthyl-N-((1R)-spiro[2.4]heptan-1-yl)-N-((5-(trifluorométhyl)-2-pyridinyl)mé-thyl)-1,3-dihydrofuro[3,4-c]quinoline-8-carboxamide ;
(3R)-4-amino-3-méthyl-N-((1S)-spiro[2.4]heptan-1-yl)-N-((5-(trifluorométhyl)-2-pyridinyl)mé-thyl)-1,3-dihydrofuro[3,4-c]quinoline-8-carboxamide ;
(3S)-4-amino-3-méthyl-N-((1R)-spiro[2.4]heptan-1-yl)-N-((5-(trifluorométhyl)-2-pyridinyl)mé-thyl)-1,3-dihydrofuro[3,4-c]quinoline-8-carboxamide ;
(3S)-4-amino-3-méthyl-N-((1S)-spiro[2.4]heptan-1-yl)-N-((5-(trifluorométhyl)-2-pyridinyl)mé-thyl)-1,3-dihydrofuro[3,4-c]quinoline-8-carboxamide ;
(3R)-4-amino-3-méthyl-N-((1R,2R)-2-(trifluorométhyl)cyclopropyl)-N-((5-(trifluorométhyl)-2-pyri-dinyl)méthyl)-1,3-dihydrofuro[3,4-c]quinoline-8-carboxamide ;
(3R)-4-amino-3-méthyl-N-((1S,2S)-2-(trifluorométhyl) cyclopropyl)-N-((5-(trifluorométhyl)-2-py-ridinyl)méthyl)-1,3-dihydrofuro[3,4-c]quinoline-8-carboxamide ;
(3S)-4-amino-3-méthyl-N-((1R,2R)-2-(trifluorométhyl) cyclopropyl)-N-((5-(trifluorométhyl)-2-py-ridinyl)méthyl)-1,3-dihydrofuro[3,4-c]quinoline-8-carboxamide ;
(3S)-4-amino-3-méthyl-N-((1S,2S)-2-(trifluorométhyl)cyclopropyl)-N-((5-(trifluorométhyl)-2-pyri-dinyl)méthyl)-1,3-dihydrofuro[3,4-c]quinoline-8-carboxamide ;
(3R)-4-amino-3-méthyl-N-((1R)-1-(2-pyrimidinyl)éthyl)-N-((5-(trifluorométhyl)-2-pyridinyl)mé-thyl)-1,3-dihydrofuro[3,4-c]quinoline-8-carboxamide ;
(3S)-4-amino-3-méthyl-N-((1R)-1-(2-pyrimidinyl)éthyl)-N-((5-(trifluorométhyl)-2-pyridinyl)mé-thyl)-1,3-dihydrofuro[3,4-c]quinoline-8-carboxamide ;
(3R)-4-amino-N-((5-cyano-2-pyridinyl)méthyl)-N-(5,6-dihydro-2H-pyran-3-ylméthyl)-3-méthyl-1,3-dihydrofuro[3,4-c]quinoline-8-carboxamide ;
(3S)-4-amino-N-((5-cyano-2-pyridinyl)méthyl)-N-(5,6-dihydro-2H-pyran-3-ylméthyl)-3-méthyl-1,3-dihydrofuro[3,4-c]quinoline-8-carboxamide ;
(3R)-4-amino-N-((1-cyanocyclopropyl)méthyl)-N-((5-cyano-2-pyridinyl)méthyl)-3-méthyl-1,3-dihydrofuro[3,4-c]quinoline-8-carboxamide ;
(3S)-4-amino-N-((1-cyanocyclopropyl)méthyl)-N-((5-cyano-2-pyridinyl)méthyl)-3-méthyl-1,3-dihydrofuro[3,4-c]quinoline-8-carboxamide ;
(3R)-4-amino-N-((2-amino-1,3-thiazol-5-yl)méthyl)-N-((5-cyano-2-pyridinyl) méthyl)-3-méthyl-1,3-dihydrofuro[3,4-c]quinoline-8-carboxamide ;
(3S)-4-amino-N-((2-amino-1,3-thiazol-5-yl)méthyl)-N-((5-cyano-2-pyridinyl)méthyl)-3-méthyl-1,3-dihydrofuro[3,4-c]quinoline-8-carboxamide ;
(3R)-4-amino-N-(5,6-dihydro-2H-pyran-3-ylméthyl)-3-méthyl-N-((5-(trifluorométhyl)-2-pyri-dinyl)méthyl)-1,3-dihydrofuro[3,4-c]quinoline-8-carboxamide ;
(3S)-4-amino-N-(5,6-dihydro-2H-pyran-3-ylméthyl)-3-méthyl-N-((5-(trifluorométhyl)-2-pyri-dinyl)méthyl)-1,3-dihydrofuro[3,4-c]quinoline-8-carboxamide ;
(3R)-4-amino-3-méthyl-N-(1H-pyrrolo[2,3-b]pyridin-4-ylméthyl)-N-((5-(trifluorométhyl)-2-pyri-dinyl)méthyl)-1,3-dihydrofuro[3,4-c]quinoline-8-carboxamide ;
(3S)-4-amino-3-méthyl-N-(1H-pyrrolo[2,3-b]pyridin-4-ylméthyl)-N-((5-(trifluorométhyl)-2-pyri-dinyl)méthyl)-1,3-dihydrofuro[3,4-c]quinoline-8-carboxamide ;
(3R)-4-amino-N-((1-cyanocyclopropyl)méthyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1-cyanocyclopropyl)méthyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((2-amino-1,3-thiazol-5-yl)méthyl)-3-méthyl-N-((5-(trifluorométhyl)-2-pyri-dinyl)méthyl)-1,3-dihydrofuro[3,4-c]quinoline-8-carboxamide ;
(3S)-4-amino-N-((2-amino-1,3-thiazol-5-yl)méthyl)-3-méthyl-N-((5-(trifluorométhyl)-2-pyri-dinyl)méthyl)-1,3-dihydrofuro[3,4-c]quinoline-8-carboxamide ;
(3R)-4-amino-3-méthyl-N-(2-méthylpropyl)-N-((5-(trifluorométhyl)-2-pyridinyl)méthyl)-1,3-dihy-drofuro[3,4-c]quinoline-8-carboxamide ;
(3S)-4-amino-3-méthyl-N-(2-méthylpropyl)-N-((5-(trifluorométhyl)-2-pyridinyl)méthyl)-1,3-dihy-drofuro[3,4-c]quinoline-8-carboxamide ;
(3R)-4-amino-N-((2R,3R)-3-hydroxy-2-butanyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((2R,3S)-3-hydroxy-2-butanyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((2S,3R)-3-hydroxy-2-butanyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((2S,3S)-3-hydroxy-2-butanyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((2R,3R)-3-hydroxy-2-butanyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((2R,3S)-3-hydroxy-2-butanyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((2S,3R)-3-hydroxy-2-butanyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((2S,3S)-3-hydroxy-2-butanyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1R,3R)-3-hydroxycyclohexyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1R,3S)-3-hydroxycyclohexyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1S,3R)-3-hydroxycyclohexyl)-3-méthyl-N-((5-(trifluorométhyl)-2-pyridinyl)mé-thyl)-1,3-dihydrofuro[3,4-c]quinoline-8-carboxamide ;
(3R)-4-amino-N-((1S,3S)-3-hydroxycyclohexyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1R,3R)-3-hydroxycyclohexyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1R,3S)-3-hydroxycyclohexyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1S,3R)-3-hydroxycyclohexyl)-3-méthyl-N-((5-(trifluorométhyl)-2-pyridinyl)mé-thyl)-1,3-dihydrofuro[3,4-c]quinoline-8-carboxamide ;
(3S)-4-amino-N-((1S,3S)-3-hydroxycyclohexyl)-3-méthyl-N-((5-(trifluorométhyl)-2-pyridinyl)mé-thyl)-1,3-dihydrofuro[3,4-c]quinoline-8-carboxamide ;
(3R)-4-amino-N-(2-(cis-3-hydroxycyclobutyl)éthyl)-3-méthyl-N-((5-(trifluorométhyl)-2-pyri-dinyl)méthyl)-1,3-dihydrofuro[3,4-c]quinoline-8-carboxamide ;
(3R)-4-amino-N-(2-(trans-3-hydroxycyclobutyl)éthyl)-3-méthyl-N-((5-(trifluorométhyl)-2-pyridinyl)méthyl)-1,3-dihydrofuro[3,4-c]quinoline-8-carboxamide ;
(3S)-4-amino-N-(2-(cis-3-hydroxycyclobutyl)éthyl)-3-méthyl-N-((5-(trifluorométhyl)-2-pyridinyl)méthyl)-1,3-dihydrofuro[3,4-c]quinoline-8-carboxamide ;
(3S)-4-amino-N-(2-(trans-3-hydroxycyclobutyl)éthyl)-3-méthyl-N-((5-(trifluorométhyl)-2-pyridinyl)méthyl)-1,3-dihydrofuro[3,4-c]quinoline-8-carboxamide ;
(3R)-4-amino-N-((2R)-2-hydroxypropyl)-3-méthyl-N-((5-(trifluorométhyl)-2-pyridinyl)méthyl)-1,3-dihydrofuro[3,4-c]quinoline-8-carboxamide ;
(3R)-4-amino-N-((2S)-2-hydroxypropyl)-3-méthyl-N-((5-(trifluorométhyl)-2-pyridinyl)méthyl)-1,3-dihydrofuro[3,4-c]quinoline-8-carboxamide ;
(3S)-4-amino-N-((2R)-2-hydroxypropyl)-3-méthyl-N-((5-(trifluorométhyl)-2-pyridinyl)méthyl)-1,3-dihydrofuro[3,4-c]quinoline-8-carboxamide ;
(3S)-4-amino-N-((2S)-2-hydroxypropyl)-3-méthyl-N-((5-(trifluorométhyl)-2-pyridinyl)méthyl)-1,3-dihydrofuro[3,4-c]quinoline-8-carboxamide ;
(3R)-4-amino-N-((2R)-3-hydroxy-2-méthylpropyl)-3-méthyl-N-((5-(trifluorométhyl)-2-pyri-dinyl)méthyl)-1,3-dihydrofuro[3,4-c]quinoline-8-carboxamide ;
(3R)-4-amino-N-((2S)-3-hydroxy-2-méthylpropyl)-3-méthyl-N-((5-(trifluorométhyl)-2-pyri-dinyl)méthyl)-1,3-dihydrofuro[3,4-c]quinoline-8-carboxamide ;
(3S)-4-amino-N-((2R)-3-hydroxy-2-méthylpropyl)-3-méthyl-N-((5-(trifluorométhyl)-2-pyri-dinyl)méthyl)-1,3-dihydrofuro [3,4-c]quinoline-8-carboxamide ;
(3S)-4-amino-N-((2S)-3-hydroxy-2-méthylpropyl)-3-méthyl-N-((5-(trifluorométhyl)-2-pyri-dinyl)méthyl)-1,3-dihydrofuro[3,4-c]quinoline-8-carboxamide ;
(3R)-4-amino-3-méthyl-N-((2R)-3,3,3-trifluoro-2-hydroxypropyl)-N-((5-(trifluorométhyl)-2-pyri-dinyl)méthyl)-1,3-dihydrofuro[3,4-c]quinoline-8-carboxamide ;
(3R)-4-amino-3-méthyl-N-((2S)-3,3,3-trifluoro-2-hydroxypropyl)-N-((5-(trifluorométhyl)-2-pyri-dinyl)méthyl)-1,3-dihydrofuro[3,4-c]quinoline-8-carboxamide ;
(3S)-4-amino-3-méthyl-N-((2R)-3,3,3-trifluoro-2-hydroxypropyl)-N-((5-(trifluorométhyl)-2-pyri-dinyl)méthyl)-1,3-dihydrofuro[3,4-c]quinoline-8-carboxamide ;
(3S)-4-amino-3-méthyl-N-((2S)-3,3,3-trifluoro-2-hydroxypropyl)-N-((5-(trifluorométhyl)-2-pyri-dinyl)méthyl)-1,3-dihydrofuro[3,4-c]quinoline-8-carboxamide ;
(3R)-4-amino-3-méthyl-N-((1-méthyl-1H-1,2,4-triazol-3-yl)méthyl)-N-((5-(trifluorométhyl)-2-pyri-dinyl)méthyl)-1,3-dihydrofuro[3,4-c]quinoline-8-carboxamide ;
(3S)-4-amino-3-méthyl-N-((1-méthyl-1H-1,2,4-triazol-3-yl)méthyl)-N-((5-(trifluorométhyl)-2-pyri-dinyl)méthyl)-1,3-dihydrofuro[3,4-c]quinoline-8-carboxamide ;
(3R)-4-amino-3-méthyl-N-((3R)-tétrahydro-3-furanylméthyl)-N-((5-(trifluorométhyl)-2-pyri-dinyl)méthyl)-1,3-dihydrofuro[3,4-c]quinoline-8-carboxamide ;
(3R)-4-amino-3-méthyl-N-((3S)-tétrahydro-3-furanylméthyl)-N-((5-(trifluorométhyl)-2-pyri-dinyl)méthyl)-1,3-dihydrofuro[3,4-c]quinoline-8-carboxamide ;
(3S)-4-amino-3-méthyl-N-((3R)-tétrahydro-3-furanylméthyl)-N-((5-(trifluorométhyl)-2-pyri-dinyl)méthyl)-1,3-dihydrofuro[3,4-c]quinoline-8-carboxamide ;
(3S)-4-amino-3-méthyl-N-((3S)-tétrahydro-3-furanylméthyl)-N-((5-(trifluorométhyl)-2-pyri-dinyl)méthyl)-1,3-dihydrofuro[3,4-c]quinoline-8-carboxamide ;
(3R)-4-amino-N-((2R)-2-cyanopropyl)-3-méthyl-N-((5-(trifluorométhyl)-2-pyridinyl)méthyl)-1,3-dihydrofuro 3,4-c)quinoline-8-carboxamide ;
(3R)-4-amino-N-((2S)-2-cyanopropyl)-3-méthyl-N-((5-(trifluorométhyl)-2-pyridinyl)méthyl)-1,3-dihydrofuro[3,4-c]quinoline-8-carboxamide ;
(3S)-4-amino-N-((2R)-2-cyanopropyl)-3-méthyl-N-((5-(trifluorométhyl)-2-pyridinyl)méthyl)-1,3-dihydrofuro[3,4-c]quinoline-8-carboxamide ;
(3S)-4-amino-N-((2S)-2-cyanopropyl)-3-méthyl-N-((5-(trifluorométhyl)-2-pyridinyl)méthyl)-1,3-dihydrofuro[3,4-c]quinoline-8-carboxamide ;
(3R)-4-amino-N,3-diméthyl-N-((5-(trifluorométhyl)-2-pyridinyl)méthyl)-1,3-dihydrofuro[3,4-c]qui-noline-8-carboxamide ;
(3S)-4-amino-N,3-diméthyl-N-((5-(trifluorométhyl)-2-pyridinyl)méthyl)-1,3-dihydrofuro[3,4-c]qui-noline-8-carboxamide ;
(3R)-4-amino-N-éthyl-3-méthyl-N-((5-(trifluorométhyl)-2-pyridinyl)méthyl)-1,3-dihydrofuro[3,4-c]quinoline-8-carboxamide ;
(3S)-4-amino-N-éthyl-3-méthyl-N-((5-(trifluorométhyl)-2-pyridinyl)méthyl)-1,3-dihydrofuro[3,4-c]quinoline-8-carboxamide ;
(3R)-4-amino-3-méthyl-N-(2-propanyl)-N-((5-(trifluorométhyl)-2-pyridinyl)méthyl)-1,3-dihydro-furo[3,4-c]quinoline-8-carboxamide ;
(3S)-4-amino-3-méthyl-N-(2-propanyl)-N-((5-(trifluorométhyl)-2-pyridinyl)méthyl)-1,3-dihydro-furo[3,4-c]quinoline-8-carboxamide ;
4-amino-N,3,3-triméthyl-N-((5-(trifluorométhyl)-2-pyridinyl)méthyl)-1,3-dihydrofuro[3,4-c]quino-line-8-carboxamide ;
4-amino-N-éthyl-3,3-diméthyl-N-((5-(trifluorométhyl)-2-pyridinyl)méthyl)-1,3-dihydrofuro[3,4-c]quinoline-8-carboxamide ;
4-amino-3,3-diméthyl-N-(2-propanyl)-N-((5-(trifluorométhyl)-2-pyridinyl)méthyl)-1,3-dihy-drofuro[3,4-c]quinoline-8-carboxamide ;
4-amino-N-cyclopropyl-3,3-diméthyl-N-((5-(trifluorométhyl)-2-pyridinyl)méthyl)-1,3-dihydro-furo[3,4-c]quinoline-8-carboxamide ; et
4-amino-N-((1R)-1-(3-cyano-5-(trifluorométhyl)-2-pyridinyl)éthyl)-7-fluoro-N,1-diméthyl-1H-pyrazolo[4,3-c]quinoline-8-carboxamide.

15. Composé selon la revendication 14, tautomère de celui-ci, stéréoisomère de celui-ci, ou sel pharmaceutiquement acceptable de l'un quelconque des précédents, dans lequel le composé est choisi parmi :
(3R)-4-amino-N-éthyl-7-fluoro-3-méthyl-N-((1R)-1-(6-(trifluorométhyl)-3-pyridazinyl)éthyl)-1,3-dihydrofuro[3,4-c]quinoline-8-carboxamide ;
4-amino-N,1-diméthyl-N-((5-(trifluorométhyl)-2-pyridinyl)méthyl)-1H-pyrazolo[4,3-c]quino-line-8-carboxamide ;
4-amino-7-fluoro-N-((1R)-1-(3-fluoro-5-(trifluorométhyl)-2-pyridinyl)éthyl)-N,1-diméthyl-1H-pyrazolo[4,3-c]quinoline-8-carboxamide ;
4-amino-N,1-diméthyl-N-(4-(pentafluoro-lambda~6~-sulfanyl)benzyl)-1H-pyrazolo[4,3-c][1,7]naphtyridine-8-carboxamide ;
4-amino-7-fluoro-N,1-diméthyl-N-((1R)-1-(6-(trifluorométhyl)-3-pyridazinyl)éthyl)-1H-pyrazolo[4,3-c]quinoline-8-carboxamide ;
(3R)-4-amino-7-fluoro-N,3-diméthyl-N-((1R)-1-(6-(trifluorométhyl)-3-pyridazinyl)éthyl)-1,3-dihydrofuro[3,4-c]quinoline-8-carboxamide ;
4-amino-7-fluoro-N-méthyl-N-((1R)-1-(6-(trifluorométhyl)-3-pyridazinyl)éthyl)-1,3-dihydrofuro[3,4-c]quinoline-8-carboxamide ;
5-amino-N-(2-propanyl)-N-((5-(trifluorométhyl)-2-pyridinyl)méthyl)pyrido[4,3-c][1,7]naphtyridine-9-carboxamide ;
4-amino-N-éthyl-7-fluoro-1-méthyl-N-((5-(trifluorométhyl)-2-pyridinyl)méthyl)-1H-pyrazolo[4,3-c]quinoline-8-carboxamide ;
4-amino-7-chloro-N-((1R)-1-(5-fluoro-2-pyridinyl)éthyl)-N,1-diméthyl-1H-pyrazolo[4,3-c]quinoline-8-carboxamide ;
4-amino-N-cyclobutyl-N-((6-(trifluorométhyl)-3-pyridazinyl)méthyl)-1,3-dihydrofuro[3,4-c]quinoline-8-carboxamide ;
4-amino-N-méthyl-N-((5-(trifluorométhyl)-2-pyridinyl)méthyl)-1,3-dihydrofuro[3,4-c]quinoline-8-carboxamide ;
4-amino-N-cyclopropyl-1-méthyl-N-((5-(trifluorométhyl)-2-pyridinyl)méthyl)-1H-pyrazolo[4,3-c]quinoline-8-carboxamide ;
4-amino-N-éthyl-7-fluoro-1-méthyl-N-((6-(trifluorométhyl)-3-pyridazinyl)méthyl)-1H-pyrazolo[4,3-c]quinoline-8-carboxamide ;
4-amino-N-cyclopropyl-7-fluoro-1-méthyl-N-((6-(trifluorométhyl)-3-pyridazinyl)méthyl)-1H-pyrazolo[4,3-c]quinoline-8-carboxamide ;
4-amino-7-fluoro-N,1-diméthyl-N-((1R)-1-(5-(trifluorométhyl)-2-pyridinyl)éthyl)-1H-pyrazolo[4,3-c]quinoline-8-carboxamide ;
(3R)-4-amino-N-éthyl-3-méthyl-N-((1R)-1-(6-(trifluorométhyl)-3-pyridazinyl)éthyl)-1,3-dihydrofuro[3,4-c]quinoline-8-carboxamide ;
4-amino-N-cyclopropyl-7-fluoro-1-méthyl-N-((5-(trifluorométhyl)-2-pyridinyl)méthyl)-1H-pyrazolo[4,3-c]quinoline-8-carboxamide ;
4-amino-7-fluoro-N,1-diméthyl-N-((5-(trifluorométhyl)-2-pyridinyl)méthyl)-1H-pyrazolo[4,3-c]quinoline-8-carboxamide et
4-amino-N-éthyl-1-méthyl-N-((5-(trifluorométhyl)-2-pyridinyl)méthyl)-1H-pyrazolo[4,3-c]quinoline-8-carboxamide.

16. Composition pharmaceutique, comprenant le composé selon l'une quelconque des revendications 1, 14 ou 15, le tautomère de celui-ci, le stéréoisomère de celui-ci ou le sel pharmaceutiquement acceptable de l'un quelconque des précédents, ou un sel pharmaceutiquement acceptable de ceux-ci, et au moins un excipient pharmaceutiquement acceptable.

17. Composé selon l'une quelconque des revendications 1, 14 ou 15, tautomère de celui-ci, stéréoisomère de celui-ci ou sel pharmaceutiquement acceptable de l'un quelconque des précédents destinés à être utilisés dans le traitement d'un cancer, dans lequel le cancer est choisi de préférence parmi un cancer de l'ovaire, du poumon, du système lymphatique, un glioblastome, un cancer du côlon, un mélanome, un cancer de l'estomac, un cancer du pancréas ou un cancer de la vessie.
